(19) Europäisches Patentamt
European Patent Office
Office européen des brevets

(11) EP 0 450 097 B1

(12) **EUROPEAN PATENT SPECIFICATION**

(45) Date of publication and mention of the grant of the patent: **24.04.1996 Bulletin 1996/17**

(21) Application number: **90915185.4**

(22) Date of filing: **18.10.1990**

(51) Int. Cl.$^6$: **A61K 31/00**, C07D 215/08, C07D 215/12, C07D 223/16, C07D 225/06, C07D 241/42, C07D 243/14, C07D 265/36, C07D 267/14

(86) International application number: **PCT/JP90/01340**

(87) International publication number: **WO 91/05549 (02.05.1991 Gazette 1991/10)**

(54) **BENZOHETEROCYCLIC COMPOUNDS**
BENZOHETEROZYKLISCHE VERBINDUNGEN
COMPOSES BENZOHETEROCYCLIQUES

(84) Designated Contracting States: **CH DE DK ES FR GB IT LI NL SE**

(30) Priority: **20.10.1989 JP 274338/89**
**15.03.1990 JP 66063/90**
**20.04.1990 JP 105580/90**
**09.07.1990 JP 181858/90**

(43) Date of publication of application: **09.10.1991 Bulletin 1991/41**

(73) Proprietor: **OTSUKA PHARMACEUTICAL CO., LTD.**
**Chiyoda-ku Tokyo 101 (JP)**

(72) Inventors:
- **OGAWA, Hidenori**
  **Tokushima-shi Tokushima 771-11 (JP)**
- **MIYAMOTO, Hisashi**
  **Tokushima-shi Tokushima 771-11 (JP)**
- **KONDO, Kazumi**
  **Naruto-shi Tokushima 772 (JP)**
- **YAMASHITA, Hiroshi**
  **Itano-gun Tokushima 771-02 (JP)**
- **NAKAYA, Kenji**
  **Tokushima-shi Tokushima 771-01 (JP)**
- **KOMATSU, Hajime**
  **Tokushima-shi Tokushima 771-01 (JP)**
- **TANAKA, Michinori**
  **Tokushima-shi Tokushima 771-01 (JP)**
- **KORA, Shinya**
  **Tokushima-shi Tokushima 771-01 (JP)**
- **TOMINAGA, Michiaki**
  **Itano-gun Tokushima 771-13 (JP)**
- **YABUUCHI, Yoichi**
  **Tokushima-shi Tokushima 771-01 (JP)**

(74) Representative: **von Kreisler, Alek, Dipl.-Chem. et al**
**Patentanwälte**
**von Kreisler-Selting-Werner**
**Postfach 10 22 41**
**D-50462 Köln (DE)**

(56) References cited:

| | |
|---|---|
| **EP-A- 0 151 539** | **EP-A- 0 187 322** |
| **DE-A- 1 595 863** | **DE-A- 1 906 593** |
| **DE-A- 2 314 392** | **FR-A- 1 405 271** |
| **US-A- 3 035 047** | **US-A- 3 182 070** |
| **US-A- 3 458 498** | **US-A- 4 335 123** |

- **Chemical Abstracts, volume 102, no. 25, 24 June 1985, (Columbus, Ohio, US), see page 582, abstract 220763r, & JP, A, 6004170**

Remarks:
The file contains technical information submitted after the application was filed and not included in this specification

EP 0 450 097 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

Technical Field

This invention relates to novel benzoheterocyclic compounds which have excellent vasopressin antagonistic activities and are useful as vasodilator, hypotensive agent, water diuretics, platelet aggregation inhibitor.

DE-A-1 595 863 discloses N-aminoacyl-3,4-dihydro-(2H)-1,4-benzoxazines of the formula

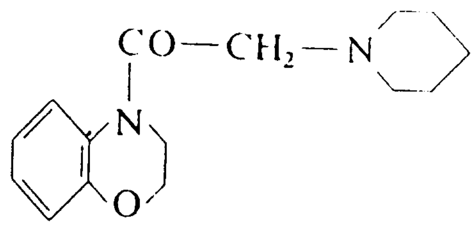

(I)

as anti-inflammatory and analgesic agents.

FR-A-1 405 271 discloses benzoxazepine derivatives of the formula

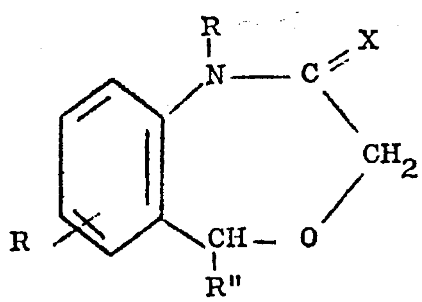

(A)

wherein R represents hydrogen or halogen, R' and R" represent a hydrogen, alkyl-, aryl- or aralkyl radical, X represents oxygen or two hydrogen atoms and its seclative, tranquillizing and hypnotic effect.

US-A-3 458 498 discloses 7-alkoxy-1-carboxamido-2,3,4,5-tetrahydro-1H-1-benzazepines which are pharmaceutically efficacious as diuretic, hypoglycemic, antibacterial and anticonvulsant agents.

EP-A-0 187 322 discloses carbostyril derivatives of the formula

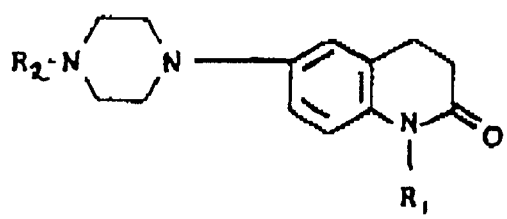

and their pharmaceutically acceptable salts.

These compounds are indicated to exhibit a positive inotropic effect. They are useful for treating heart diseases such as congestive heart failure.

EP-A-0 151 539 discloses benzobicyclic lactam acids of the formula

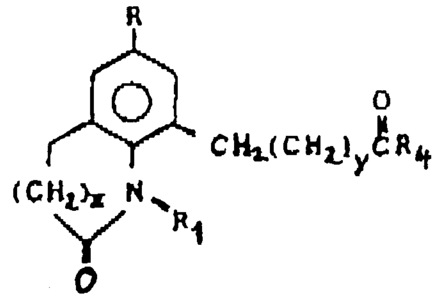

These compounds are used for treating semility or for reversing amnesia.

US-A-3 035 047 discloses N-substituted-phthalamoyl derivatives of 6-aminopencillanic acid which show an ehemotherapeutic effect.

US-A-3 182 070 discloses compounds having vasopressin antagonistic activity and which are structurally quite different from the compounds of the present invention.

Disclosure of the Invention

The benzoheterocyclic compounds of this invention have the following formula:

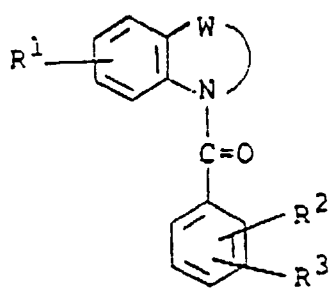

(1)

wherein $R^1$ is hydrogen atom, a halogen atom, a $C_1$-$C_6$ alkyl, an amino having optionally a $C_1$-$C_6$ alkyl substituent, or a $C_1$-$C_6$ alkoxy,

$R^2$ is hydrogen atom, a halogen atom, a $C_1$-$C_6$ alkoxy, a phenyl($C_1$-$C_6$)alkoxy, hydroxy, a $C_1$-$C_6$ alkyl, an amino having optionally a $C_1$-$C_6$ alkyl substituent, a carbamoyl-substituted $C_1$-$C_6$ alkoxy, an amino-substituted $C_1$-$C_6$ alkoxy having optionally a $C_1$-$C_6$ alkyl substituent, or a benzoyloxy which has optionally a halogen substituent on the phenyl ring,

$R^3$ is a group of the formula:

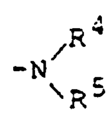

or a group of the formula:

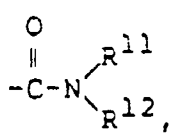

$R^4$ is hydrogen atom, a benzoyl which has optionally a halogen substituent on tee phenyl ring, or a $C_1$-$C_6$ alkyl,
$R^5$ is (a) a group of the fromula:

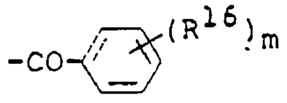

[wherein $R^{16}$ is (i) a halogen atom; (ii) a $C_1$-$C_6$ alkyl which has optionally a substituent selected from a halogen atom and hydroxy; (iii) hydroxy; (iv) a $C_1$-$C_6$ alkoxy; (v) a $C_1$-$C_6$ alkanoyloxy; (vi) a $C_1$-$C_6$ alkylthio; (vii) a $C_1$-$C_6$ alkanoyl; (viii) carboxy; (ix) a $C_1$-$C_6$ alkoxycarbonyl; (x) cyano; (xi) nitro; (xii) an amino which has optionally a substituent selected from a $C_1$-$C_6$ alkyl and a $C_1$-$C_6$ alkanoyl; (xiii) phenyl; (xiv) a $C_3$-$C_8$ cycloalkyl; (xv) a $C_2$-$C_6$ alkanoyloxy-substituted $C_1$-$C_6$ alkoxy; (xvi) a carboxy-substituted $C_1$-$C_6$ alkoxy; (xvii) a halogen-substituted $C_1$-$C_6$ alkoxy; (xviii) a carbamoyl-substituted $C_1$-$C_6$ alkoxy; (xix) a hydroxy-substituted $C_1$-$C_6$ alkoxy; (xx) a $C_1$-$C_6$ alkoxycarbonyl-substituted $C_1$-$C_6$ alkoxy; (xxi) a phthalimido-substituted $C_1$-$C_6$ alkoxy; (xxii) an aminocarbonyl-$C_1$-$C_6$ alkoxy having a $C_1$-$C_6$ alkyl substituent; or (xxiii) a group of the formula;

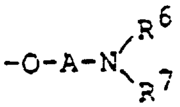

(A is alkylene with up to 6 carbon atoms, and

$R^6$ and $R^7$ are the same or different and are each hydrogen atom, a $C_1$-$C_6$ alkyl having optionally a hydroxy substituent, a $C_1$-$C_6$ alkanoyl, or benzoyl, or

$R^6$ and $R^7$ may bind together with nitrogen atom to which they bond to form a 5- or 6-membered saturated heterocyclic group with or without being intervened with nitrogen or oxygen atom wherein the heterocyclic group has optionally a substituent selected from piperidinyl and a $C_1$-$C_6$ alkyl);
and m is an integer of 0 to 3],

(b) a phenyl-$C_1$-$C_6$ alkoxycarbonyl,
(c) a $C_1$-$C_6$ alkanoyl,
(d) a phenyl-$C_2$-$C_6$ alkanoyl,
(e) a $C_3$-$C_8$ cycloalkyl-$C_2$-$C_6$ alkanoyl,
(f) a $C_3$-$C_8$ cycloalkylcarbonyl,
(g) tricyclo[3.3.1.1]decanylcarbonyl,
(h) naphthylcarbonyl,
(i) pyridylcarbonyl
(j) furoyl,
(k) thenoyl,
(l) a phenoxy-$C_2$-$C_6$ alkanoyl which phenyl ring has optionally 1 to 3 substituents selected from a $C_1$-$C_6$ alkyl, a $C_1$-$C_6$ alkoxy and an amino having optionally a $C_1$-$C_6$ alkanoyl substituent,
(m) a phthalimido-substituted $C_2$-$c_6$ alkanoyl,
(o) a $C_1$-$C_6$ alkoxycarbonyl-$C_2$-$C_6$ alkanoyl,
(p) a carboxy-$C_2$-$C_6$ alkanoyl,
(q) a naphthyloxy-$C_2$-$C_6$ alkanoyl,
(r) a halogen-substituted $C_1$-$C_6$ alkanoyl,
(s) a group of the formula:

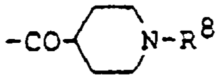

(wherein $R^8$ is (i) hydrogen atom, (ii) a $C_1$-$C_6$ alkyl, (iii) a phenyl-$C_1$-$C_6$ alkoxycarbonyl, (iv) a carbamoyl-$C_1$-$C_6$ alkyl, (v) an amino-$C_2$-$C_6$ alkanoyl having optionally a $C_1$-$C_6$ alkyl substituent, or (vi) a $C_1$-$C_6$ alkanoyl),
(t) an anilinocarbonyl which has optionally a $C_1$-$C_6$ alkyl substituent on the phenyl ring,
(u) phenoxycarbonyl,

(v) a phenylsulfonyl which has optionally a substituent selected from a halogen atom and a $C_1$-$C_6$ alkyl on the phenyl ring,

(w) quinolylsulfonyl, or

(x) a group of the formula:

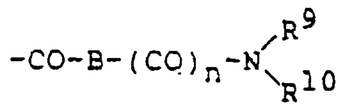

wherein B is a $C_1$-$C_6$ alkylene, n is an integer of 0 or 1, and $R^9$ and $R^{10}$ are the same or different and are each (i) hydrogen atom, (ii) alkyl with up to 6 carbon atoms having optionally a hydroxy substituent, (iii) a $C_3$-$C_8$ cycloalkyl, (iv) a phenyl-$C_1$-$C_6$ alkyl, (v) a $C_1$-$C_6$ alkanoyl, (vi) alkenyl having up to 6 carbon atoms, (vii) a phenoxy-$C_1$-$C_6$ alkyl, (viii) a phenyl which has optionally 1 to 3 substituents selected from an amino-$C_1$-$C_6$ alkyl having optionally a $C_1$-$C_6$ alkanoyl substituent, a $C_1$-$C_6$ alkyl, a $C_1$-$C_6$ alkoxy and a halogen atom, (ix) a phthalimido-substituted $C_1$-$C_6$ alkyl, (x) an amino-$C_1$-$C_6$ alkyl having optionally a $C_1$-$C_6$ alkanoyl substituent, (xi) alkynyl having up to 6 carbon atoms, or (xii) an amino-$C_1$-$C_6$ alkyl having optionally a $C_1$-$C_6$ alkyl substituent, or $R^9$ and $R^{10}$ may bind together with nitrogen atom to which they bond to form a 5- or 6-membered saturated heterocyclic group with or without being intervened with nitrogen or oxygen atom wherein the heterocyclic group has optionally a substituent selected from a $C_1$-$C_6$ alkyl, a $C_1$-$C_6$ alkoxycarbonyl and piperidinyl),

$R^{11}$ is hydrogen atom or a $C_1$-$C_6$ alkyl,

$R^{12}$ is a $C_3$-$C_8$ cycloalkyl, or a phenyl which has optionally 1 to 3 substituents selected from a $C_1$-$C_6$ alkoxy, a $C_1$-$C_6$ alkyl and a halogen atom,

W is a group of the formula: $-(CH_2)_p-$ (p is an integer of 3 to 5), or a group of the formula: $-CH=CH-(CH_2)_q-$(q is an integer of 1 to 3), or wherein the heterocyclic group of the formula:

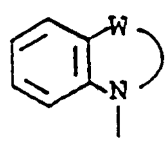

is 2,3,4,5-tetrahydro-1H-1,4-benzodiazepine

and further said $-(CH_2)_p-$ group having optionally 1 to 3 substituents selected from (i) a $C_1$-$C_6$ alkyl having optionally a hydroxy substituent, (ii) a $C_1$-$C_6$ alkoxycarbonyl, (iii) carboxy, (iv) hydroxy, (v) oxo, (vi) a $C_1$-$C_6$ alkanoyloxy having optionally a halogen substituent, (vii) an amino-$C_1$-$C_6$ alkyl having optionally a substituent selected from a $C_1$-$C_6$ alkyl and a $C_1$-$C_6$ alkanoyl, (viii) a $C_2$-$C_6$ alkanoyloxy-substituted $C_1$-$C_6$ alkyl, (ix) a $C_1$-$C_6$ alkylsulfonyloxy-$C_1$-$C_6$ alkyl, (x) an azido-$C_1$-$C_6$ alkyl, (xi) a group of the formula: $-O-CH_2-$ (which is bound to the group $-(CH_2)_p-$ or $-CH=CH-(CH_2)_q-$ so as to be in the spiro form), (xii) an aminocarbonyloxy having optionally a $C_1$-$C_6$ alkyl substituent, (xiii) a $C_1$-$C_6$ alkoxy, (xiv) a $C_1$-$C_6$ alkoxycarbonyl-substituted $C_1$-$C_6$ alkoxy, (xv) a carboxy-substituted $C_1$-$C_6$ alkoxy, (xvi) an aminocarbonyl-$C_1$-$C_6$ alkoxy having optionally a $C_1$-$C_6$ alkyl substituent, (xvii) an amino-$C_1$-$C_6$ alkoxy having optionally a substituent selected from a $C_1$-$C_6$ alkyl and a $C_1$-$C_6$ alkanoyl, (xviii) a phthalimido-substituted $C_1$-$C_6$ alkoxy, (xix) hydroxyimino, (xx) a $C_1$-$C_6$ alkanoyloxyimino, (xxi) a $C_1$-$C_6$ alkylidene, (xxii) a halogen atom, (xxiii) azido, (xxvi) hydrazino, (xxvii) pyrrolyl, (xxviii) an amino-$C_1$-$C_6$ alkanoyloxy having optionally a $C_1$-$C_6$ alkyl substituent, (xxix) a group of the formula:

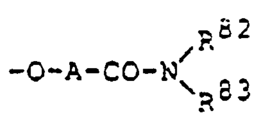

(A is as defined above,

and $R^{82}$ and $R^{83}$ are the same or different and are each hydrogen atom, a $C_1$-$C_6$ alkyl, a carbamoyl-substituted $C_1$-$C_6$ alkyl, a hydroxy-substituted $C_1$-$C_6$ alkyl, or a pyridyl-$C_1$-$C_6$ alkyl, or $R^{82}$ and $R^{83}$ may bind together with nitrogen atom to which they bond to form a 5- or 6-membered saturated heterocyclic group with or without being intervened with nitrogen, oxygen or sulfur atom wherein the heterocyclic group has optionally a substituent selected from oxo, a $C_1$-$C_6$ alkyl, a $C_1$-$C_6$ alkanoyl, and carbamoyl), and

(xxx) a group of the formula:

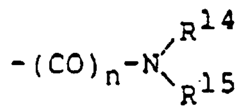

(wherein n is as defined above, and $R^{14}$ and $R^{15}$ are the same or different and are each (i) hydrogen atom, (ii) a $C_1$-$C_6$ alkyl, (iii) alkenyl having up to 6 carbon atoms, (iv) a $C_1$-$C_6$ alkanoyl, (v) a $C_3$-$C_8$ cycloalkyl, (vi) an oxiranyl-substituted $C_1$-$C_6$ alkyl, (vii) a $C_1$-$C_6$ alkyl having 1 to 2 substituents selected from a $C_1$-$C_6$ alkoxy, hydroxy and an amino having optionally a $C_1$-$C_6$ alkyl substituent, (viii) a phenyl-$C_1$-$C_6$ alkyl, (ix) a pyridyl-$C_1$-$C_6$ alkyl, (x) a $C_1$-$C_6$ alkylsulfonyl, (xi) benzoyl, (xii) a $C_1$-$C_6$ alkoxycarbonyl, (xiii) anilinocarbonyl, (xiv) an aminocarbonyl having optionally a $C_1$-$C_6$ alkyl substituent, (xv) a cyano-substituted $C_1$-$C_6$ alkyl, (xvi) a $C_1$-$C_6$ alkoxycarbonyl-substituted $C_1$-$C_6$ alkyl, (xvii) a carbamoyl-substituted $C_1$-$C_6$ alkyl, (xviii) a carboxy-substituted $C_1$-$C_6$ alkyl, (xix) a tetrahydropyranyloxy-substituted $C_1$-$C_6$ alkyl, (xx) a $C_2$-$C_6$ alkanoyloxy-substituted $C_1$-$C_6$ alkyl, (xxi) a piperidinyl having optionally a phenyl-$C_1$-$C_6$ alkyl substituent on the piperidinyl ring, (xxii) a halogen-substituted $C_2$-$C_6$ alkanoyl, (xxiii) an imidazolyl-substituted $C_2$-$C_6$ alkanoyl, (xxiv) an amino-$C_2$-$C_6$ alkanoyl having optionally a substituent selected from a $C_1$-$C_6$ alkyl and a $C_1$-$C_6$ alkoxycarbonyl, (xxv) an aminocarbonyl-$C_1$-$C_6$ alkyl having optionally a $C_1$-$C_6$ alkyl substituent, or (xxvi) a phenyl-$C_1$-$C_6$ alkoxycarbonyl, or $R^{14}$ and $R^{15}$ may bind together with nitrogen atom to which they bond to form a 5- or 6-membered saturated heterocyclic group with or without being intervened with nitrogen or oxygen, wherein the heterocyclic group may optionally have a substituent selected from a $C_1$-$C_6$ alkyl, a phenyl-$C_1$-$C_6$ alkyl or a $C_1$-$C_6$ alkanoyl), and further said $-CH=CH-(CH_2)_q-$ group having optionally 1 to 3 substituents selected from

(i) A $C_1$-$C_6$-alkyl having optionally a hydroxy substituent and
(ii) an amino-$C_1$-$C_6$-alkyl having optionally a substituent selected from $C_1$-$C_6$ alkyl and a $C_1$-$C_6$ alkanoyl,

and a salt thereof.

The benzoheterocyclic compounds of the formula (1) and their salts have excellent vasopressin antagonistic activities and vasodilating activity, hypotensive activity, activity for inhibiting saccharide release in liver, activity for inhibiting growth of mesangium cells, water diuretic activity, platelet agglutination inhibitory activity and are useful as vasodilator, hypotensive agent, water diuretics, platelet agglutination inhibitor and are used for the prophylaxis and treatment of hypertension, edema, ascites, heart failure, renal function disorder, vasopressin parasecretion syndrome (SIADH), hepatocirrhosis, hyponatremia, hypokaliemia, diabetic and circulation disorder.

Each group in the above formula (1) includes specifically the following groups.

The "lower alkoxy" includes a straight chain or branched chain alkoxy group having 1 to 6 carbon atoms, for example, methoxy, ethoxy, propoxy, isopropoxy, butoxy, tert-butoxy, pentyloxy and hexyloxy.

The "lower alkyl" includes a straight chain or branched chain alkyl group having 1 to 6 carbon atoms, for example, methyl, ethyl, propyl, isopropyl, butyl, tert-butyl, pentyl and hexyl.

The "halogen atom" includes fluorine atom, chlorine atom, bromine atom and iodine atome.

The "amino having optionally a lower alkyl substituent" includes an amino having optionally one or two substituents selected from a straight chain or branched chain alkyl group having 1 to 6 carbon atoms, for example, amino, methylamino, ethylamino, propylamino, isopropylamino, butylamino, tert-butylamino, pentylamino, hexylamino, dimethylamino, diethylamino, dipropylamino, dibutylamino, dipentylamino, dihexylamino, N-methyl-N-ethylamino, N-ethyl-N-propylamino, N-methyl-N-bintylamino and N-methyl-N-hexylamino.

The "lower alkenyl" includes a straight chain or branched chain alkenyl group having 2 to 6 carbon atoms, for example, vinyl, allyl, 2-butenyl, 3-butenyl, 1-methylallyl, 2-pentenyl and 2-hexenyl.

The "lower alkyl which has optionally a substituent selected from a halogen atom and hydroxy" includes a straight chain or branched chain alkyl group having 1 to 6 carbon atoms which may optionally have 1 to 3 substituents selected from a halogen atom and hydroxy, for example, in addition to the above-mentioned lower alkyl groups, hydroxymethyl, 2-hydroxyethyl, 1-hydroxyethyl, 3-hydroxypropyl, 2,3-dihyroxypropyl, 4-hydroxybutyl, 1,1-dimethyl-2-hydroxyethyl, 5,5,4-trihydroxypentyl, 5-hydroxypentyl, 6-hydroxyhexyl, 1-hydroxyisopropyl, 2-methyl-3-hydroxypropyl, trifluoromethyl, trichloromethyl, chloromethyl, bromomethyl, fluoromethyl, iodomethyl, difluoromethyl, dibromomethyl, 2-chloroethyl, 2,2,2-trifluoroethyl, 2,2,2-trichloroethyl, 3-chloropropyl, 2,3-dichloropropyl, 4,4,4-trichlorobutyl, 4-fluorobutyl, 5-chloropentyl, 3-chloro-2-methylpropyl, 5-bromohexyl and 5,6-dichlorohexyl.

The "lower alkylene" includes a straight chain or branched chain alkylene group having 1 to 6 carbon atoms, for example, methylene, ethylene, trimethylene, 2-methyltrimethylene, 2,2-dimethyltrimethylene, 1-methyltrimethylene, methylmethylene, ethylmethylene, tetramethylene, pentamethylene and hexamethylene.

The "lower alkanoyloxy" includes a straight chain or branched chain alkanoyloxy group having 1 to 6 carbon atoms, for example, formyloxy, acetyloxy, propionyloxy, butyryloxy, isobutyryloxy, pentanoyloxy, tert-butylcarbonyloxy and hexanoyloxy.

The "lower alkylthio" includes a straight chain or branched chain alkylthio group having 1 to 6 carbon atoms, for example, methylthio, ethylthio, propylthio, isopropylthio, butylthio, tert-butylthio, pentylthio and hexylthio.

The "lower alkanoyl" includes a straight chain or branched chain alkanoyl group having 1 to 6 carbon atoms, for example, formyl, acetyl, propionyl, butyryl, isobutyryl, pentanoyl, tert-butylcarbonyl and hexanolyl.

The "lower alkoxycarbonyl" includes a straight chain or branched chain alkoxycarbonyl group having 1 to 6 carbon atoms in the alkoxy moiety, for example, methoxycarbonyl, ethoxycarbonyl, propoxycarbonyl, isopropoxycarbonyl, butoxycarbonyl, tert-butoxycarbonyl, pentyloxycarbonyl and hexyloxycarbonyl.

The "amino having optionally a substituent selected from a lower alkyl and a lower alkanoyl" includes an amino having optionally one or two substituents selected from a straight chain or branched chain alkyl group having 1 to 6 carbon atoms and a straight chain or branched chain alkanoyl group having 1 to 6 carbon atoms, for example, amino, methylamino, ethylamino, propylamino, isopropylamino, butylamino, tert-butylamino, pentylamino, hexylamino, dimethylamino, diethylamino, dipropylamino, dibutylamino, dipentylamino, dihexylamino, N-methyl-N-ethylamino, N-ethyl-N-propylamino, N-methyl-N-butylamino, N-methyl-N-hexylamino, N-methyl-N-acetylamino, N-acetylamino, N-formylamino, N-propionylamino, N-butyrylamino, N-isobutyrylamino, N-pentanoylamino, N-tert-butylcarbonylamino, N-hexanoylamino and N-ethyl-N-acetylamino.

The "cycloalkyl" includes a cycloalkyl having 3 to 8 carbon atoms, for example, cyclopropyl, cyclobutyl, cyclopentyl, cyclohexyl, cycloheptyl and cyclooctyl.

The "lower alkanoyloxy-substituted lower alkoxy" includes a straight chain or branched chain alkoxy group having 1 to 6 carbon atoms which is substituted by a straight chain or branched chain alkanoyloxy group having 2 to 6 carbon atoms, for example, acetyloxymethoxy, 2-propionyloxyethoxy, 1-butyryloxyethoxy, 3-acetyloxypropoxy, 4-acetyloxybutoxy, 4-isobutyryloxybutoxy, 5-pentanoyloxypentyloxy, 6-acetyloxyhexyloxy, 6-tert-butylcarbonyloxyhexyloxy, 1,1-dimethyl-2-hexanoyloxyethoxy and 2-methyl-3-acetyloxypropoxy.

The "carbamoyl-substituted lower alkoxy" includes a carbamoyl-substituted alkoxy group wherein the alkoxy moiety is a straight chain or branched chain alkoxy group having 1 to 6 carbon atoms, for example, carbamoylmethoxy, 2-carbamoylethoxy, 1-carbamoylethoxy, 3-carbamoylpropoxy, 4-carbamoylbutoxy, 5-carbamoylpentyloxy, 6-carbamoylhexyloxy, 1,1-dimethyl-2-carbamoylethoxy and 2-methyl-3-carbamoylpropoxy.

The "hydroxy-substituted lower alkoxy" includes a straight chain or branched chain alkoxy group having 1 to 6 carbon atoms and having 1 to 3 hydroxy substitutents, for example, hydroxymethoxy, 2-hydroxyethoxy, 1-hydroxyethoxy, 3-hydroxypropoxy, 2,3-dihydroxypropoxy, 4-hydroxybutoxy, 3,4-dihydroxybutoxy, 1,1-dimethyl-2-hydroxyethoxy, 5-hydroxypentyloxy, 6-hydroxyhexyloxy, 2-metnyl-3-hydroxypropoxy and 2,3,4-trihydroxybutoxy.

The "lower alkoxycarbonyl-substituted lower alkoxy" includes an alkoxycarbonyl-substituted straight chain or branched chain alkoxy group having 1 to 6 carbon atoms wherein the alkoxycarbonyl moiety is a straight chain or branched chain alkoxycarbonyl group having 1 to 6 carbon atoms, for example, methoxycarbonylmethoxy, 3-methoxycarbonylpropoxy, ethoxycarboxymethoxy, 3-ethoxycarbonylpropoxy, 4-ethoxycarbonylbutoxy, 5-isopropoxycarbonylpentyloxy, 6-propoxycarbonylhexyloxy, 1,1-dimethyl-2-butoxycarbonylethoxy, 2-methyl-3-tert-butoxycarbonylpropoxy, 2-pentyloxycarbonylethoxy and hexyloxycarbonylmethoxy.

The "carboxy-substituted lower alkoxy" includes a carboxy-substituted alkoxy group wherein the alkoxy moiety is a straight chain or branched chain alkoxy group having 1 to 6 carbon atoms, for example, carboxymethoxy, 2-carboxyethoxy, 1-carboxyethoxy, 3-carboxypropoxy, 4-carboxybutoxy, 5-carboxypentyloxy, 6-carboxyhexyloxy, 1,1-dimethyl-2-carboxyethoxy and 2-methyl-3-carboxypropoxy.

The "phthalimido-substituted lower alkoxy" includes a straight chain or branched chain alkoxy group having 1 to 6 carbon atoms which is substituted by phthalimido group, for example, phthalimidomethoxy, 2-phthalimidoethoxy, 1-phthalimidoethoxy, 3-phthalimidopropoxy, 4-phthalimidobutoxy, 5-phthalimidopentyloxy, 6-phthalimidohexyloxy, 1,1-dimethyl-2-phthalimidoethoxy and 2-methyl-3-phthalimidopropoxy.

The "5- or 6-membered saturated heterocyclic group which is formed by binding the groups $R^6$ and $R^7$ together with the nitrogen atom to which they bond with or without being intervened with nitrogen or oxygen atom" includes, for example, pyrrolidinyl, piperidinyl, piperazinyl and morpholino.

The "heterocyclic group having a substituent selected from piperidinyl and a lower alkyl" includes a heterocyclic group having 1 to 3 substituents selected from piperidinyl and a straight chain or branched chain alkyl group having 1 to 6 carbon atoms, for example, 4-methylpiperiazinyl, 3,4-dimethylpiperazinyl, 3-ethylpyrrolidinyl, 2-propylpyrrolidinyl, 3,4,5-trimethylpiperidinyl, 4-butylpiperidinyl, 3-pentylmorpholino, 4-hexylpiperazinyl, 4-(1-piperidinyl)piperidinyl, 3-(1-piperidinyl)pyrrolidinyl, 3-(1-piperidinyl)-4-methylpiperazinyl and 3-(1-piperidinyl)morpholino.

The "phenyl(lower)alkanoyl" includes a phenylalkanoyl wherein the alkanoyl moiety is a straight chain or branched chain alkanoyl group having 2 to 6 carbon atoms, for example, phenylacetyl, 3-phenylpropionyl, 2-phenylpropionyl, 4-phenylbutyryl, 2,2-dimethyl-3-phenylpropionyl, 5-phenylpentanoyl and 6-phenylhexanoyl.

The "cycloalkyl-lower alkanoyl" includes $C_3$-$C_8$ cycloalkyl-alkanoyl group wherein the alkanoyl moiety is a straight chain or branched chain alkanoyl having 2 to 6 carbon atoms, for example, cyclohexylacetyl, 3-cyclopropylpropionyl, 2-cyclopentylpropionyl, 4-cyclohexylbutyryl, 2,2-dimethyl-3-cycloheptylpropionyl, 5-cyclooctylpentanoyl and 6-cyclohexylhexanoyl.

The "cycloalkylcarbonyl" includes a cycloalkylcarbonyl having 3 to 8 carbon atoms, for example, cyclopropylcarbonyl, cyclobutylcarbonyl, cyclopentylcarbonyl, cyclohexylcarbonyl, cycloheptylcarbonyl and cyclooctylcarbonyl.

The "amino having optionally a lower alkanoyl substituent" includes an amino having optionally a straight chain or branched chain alkanoyl group having 1 to 6 carbon atoms, for example, amino, formylamino, acetylamino, propionylamino, butyrylamino, isobutyrylamino, pentanoylamino, tert-butylcarbonylamino and hexanoylamino.

The "phenoxy-lower alkanoyl which phenyl ring has optionally 1 to 3 substituents selected from a lower alkyl, a lower alkoxy and an amino having optonally a lower alkanoyl substituent" includes a phenoxyalkanoyl group wherein the alkanoyl moiety is a straight chain or branched chain alkanoyl having 2 to 6 carbon atoms and the phenyl ring has optionally 1 to 3 substituents selected from a straight chain or branched chain alkyl having 1 to 6 carbon atoms, a straight chain or branched chain alkoxy having 1 to 6 carbon atoms and an amino having optionally a straight chain or branched chain alkanoyl having 1 to 6 carbon atoms, for example, phenoxyacetyl, 3-phenoxypropionyl, 2-phenoxypropionyl, 4-phenoxybutyryl, 2,2-dimethyl-3-phenoxypropionyl, 5-phenoxypentanoyl, 6-phenoxyhexanoyl, (2-aminophenoxy)acetyl, 3-(4-aminophenoxy)propionyl, (2-methylphenoxy)acetyl, (4-methylphenoxy)acetyl, (3-methylphenoxy)acetyl, (3-methoxyphenoxy)acetyl, (3-acetylaminophenoxy)acetyl, 4-(2-propionylaminophenoxy)butyryl, 2,2-dimethyl-3-(4-butyrylaminophenoxy)propionyl, 5-(2-pentanoylaminophenoxy)pentanoyl, 6-(4-hexanoylaminophenoxy)hexanoyl, 3-(2-ethylphenoxy)propionyl, 2-(4-propylphenoxy)propionyl, 4-(4-butylphenoxy)butyryl, 5-(3-pentylphenoxy)pentanoyl, 6-(4-hexylphenoxy)hexanoyl, (2,3-dimethylphenoxy)acetyl, (2,5-dimethylphenoxy)acetyl, (3,4-dimethylphenoxy)acetyl, (3,4,5-trimethylphenoxy)acetyl, 3-(4-ethoxyphenoxy)propionyl, 2-(2-propoxyphenoxy)propionyl, 4-(3-butoxyphenoxy)butyryl, 5-(4-pentyloxyphenoxy)pentanoyl, 6-(4-hexyloxyphenoxy)hexanoyl, (3,4-dimethoxyphenoxy)acetyl, (3,5-dimethoxyphenoxy)acetyl, (2,4-dimethoxyphenoxy)acetyl, (3,4,5-trimethoxyphenoxy)acetyl, (2-acetylamino-4-methylphenoxy)acetyl and (4-acetylamino-3-methoxyphenoxy)acetyl.

The "phthalimido-substituted lower alkanoyl" includes a straight chain or branched chain alkanoyl group having 2 to 6 carbon atoms which is substituted by phthalimido group, for example, 2-phthalimidoacetyl, 3-phthalimidopropionyl, 2-phthalimidopropionyl, 4-phthalimidobutyryl, 2,2-dimethyl-3-phthalimidopropionyl, 5-phthalimidopentanoyl, 6-phthalimidohexanoyl and 3-methyl-4-phthalimidobutyryl.

The "lower alkoxycarbonyl-lower alkanoyl" includes an alkoxycarbonyl-alkanoyl group wherein the alkoxy moiety is a straight chain or branched chain alkoxy having 1 to 6 carbon atoms and the alkanoyl moiety is a straight chain or branched chain alkanoyl having 2 to 6 carbon atoms, for example, methoxycarbonylacetyl, 3-methoxycarbonylpropionyl, ethoxycarbonylacetyl, 3-ethoxycarbonylpropionyl, 4-ethoxycarbonylbutyryl, 3-propoxycarbonylpropionyl, 2-methoxycarbonylpropionyl, 6-propoxycarbonylhexanoyl, 5-isopropoxycarbonylpentanoyl, 2,2-dimethyl-3-butoxycarbonylpropionyl, 2-methyl-3-tert-butoxycarbonylpropionyl, pentyloxycarbonylacetyl and hexyloxycarbonylacetyl.

The "carboxy-lower alkanoyl" includes a carboxy-alkanoyl group wherein the alkanoyl moiety is a straight chain or branched chain alkanoyl having 2 to 6 carbon atoms, for example, carboxyacetyl, 3-carboxypropionyl, 2-carboxypropionyl, 4-carboxybutyryl, 2,2-dimethyl-3-carboxypropionyl, 5-carboxypentanoyl and 6-carboxyhexanoyl.

The "naphthyloxy-lower alkanoyl" includes a naphthyloxy-alkanoyl group wherein the alkanoyl moiety is a straight chain or branched chain alkanoyl having 2 to 6 carbon atoms, for example, naphtyloxyacetyl, 3-naphtyloxypropionyl, 2-naphtyloxypropionyl, 4-naphthyloxybutyryl, 2,2-dimethyl-3-naphthyloxypropionyl, 5-naphthyloxypentanoyl and 6-naphthyloxyhexanoyl.

The "phenyl-lower alkoxycarbonyl" includes a phenylalkoxycarbonyl wherein the alkoxycarbonyl moiety is a straight chain or branched chain alkoxycarbonyl group having 1 to 6 carbon atoms, for example, benzyloxycarbonyl, 2-phenylethoxycarbonyl, 1-phenylethoxycarbonyl, 3-phenylpropoxycarbonyl, 4-phenylbutoxycarbonyl, 5-phenylpentyloxycarbonyl, 6-phenylhexyloxycarbonyl, 1,1-dimethyl-2-phenylethoxycarbonyl and 2-methyl-3-phenylpropoxycarbonyl.

The "lower alkyl having optionally a hydroxy substituent" includes a straight chain or branched chain alkyl having 1 to 6 carbon atoms and having optionally 1 to 3 hydroxy substituents, for example, hydroxymethyl, 2-hydroxyethyl, 1-hydroxyethyl, 3-hydroxypropyl, 2,3-dihydroxyethyl, 4-hydroxybutyl, 3,4-dihydroxybutyl, 1,1-dimethyl-2-hydroxyethyl, 5-hydroxypentyl, 6-hydroxyhexyl, 2-methyl-3-hydroxypropyl and 2,3,4-trihydroxybutyl.

The "phenyl-lower alkyl" includes a phenylalkyl group wherein the alkyl moiety is a straight chain or branched chain alkyl group having 1 to 6 carbon atoms, for example, benzyl, 2-phenylethyl, 1-phenylethyl, 3-phenylpropyl, 4-phenylbutyl, 5-phenylpentyl, 6-phenylhexyl, 1,1-dimethyl-2-phenylethyl and 2-methyl-3-phenylpropyl.

The "phenoxy-lower alkyl" includes a phenoxyalkyl group wherein the alkyl moiety is a straight chain or branched chain alkyl group having 1 to 6 carbon atoms, for example, phenoxymethyl, 1-phenoxyethyl, 2-phenoxyethyl, 3-phenoxypropyl, 4-phenoxybutyl, 5-phenoxypentyl, 6-phenoxyhexyl, 1,1-dimethyl-2-phenoxyethyl and 2-methyl-3-phenoxypropyl.

The "phenyl which has optionally 1 to 3 substituents selected from a lower alkyl, a lower alkoxy and a halogen atom" includes a phenyl group which has optionally 1 to 3 substituents selected from a straight chain or branched chain alkyl

group having 1 to 6 carbon atoms, a straight chain or branched chain alkoxy group having 1 to 6 carbon atoms and a halogen atom, for example, phenyl, 2-methoxyphenyl, 3-methoxyphenyl, 4-methoxyphenyl, 2-ethoxyphenyl, 3-ethoxyphenyl, 4-ethoxyphenyl, 4-isopropoxyphenyl, 4-pentyloxyphenyl, 2,4-dimethoxyphenyl, 4-hexyloxyphenyl, 3,4-dimethoxyphenyl, 3-ethoxy-4-methoxyphenyl, 2,3-dimethoxyphenyl, 3,4-diethoxyphenyl, 2,5-dimethoxyphenyl, 2,6-dimethoxyphenyl, 3,5-dimethoxyphenyl, 3,4-dipentyloxyphenyl, 3,4,5-trimethoxyphenyl, 2-chlorophenyl, 3-chlorophenyl, 4-chlorophenyl, 2-fluorophenyl, 3-fluorophenyl, 4-fluorophenyl, 2-bromophenyl, 3-bromophenyl, 4-bromophenyl, 2-iodophenyl, 3-iodophenyl, 4-iodophenyl, 3,4-dichlorophenyl, 3,5-dichlorophenyl, 2,6-dichlorophenyl, 2,3-dichlorophenyl, 2,4-dichlorophenyl, 3,4-difluorophenyl, 3,5-dibromophenyl, 3,4,5-trichlorophenyl, 2-methoxy-3-chlorophenyl, 2-methylphenyl, 3-methylphenyl, 4-methylphenyl, 2-ethylphenyl, 3-ethylphenyl, 4-ethylphenyl, 4-isopropylphenyl, 3-butylphenyl, 4-pentylphenyl, 4-hexylphenyl, 3,4-dimethylphenyl, 3,4-diethylphenyl, 2,4-dimethylphenyl, 2,5-dimethylphenyl, 2,6-dimethylphenyl, 3,4,5-trimethylphenyl, 3-chloro-4-methylphenyl, 3-methoxy-4-methyl-5-iodophenyl, 3,4-dimethoxy-5-bromophenyl and 3,5-diiodo-4-methoxyphenyl.

The "amino-lower alkyl having optionally a lower alkyl substituent" includes a straight chain or branched chain alkyl group having 1 to 6 carbon atoms which is substituted by an amino group having optionally 1 to 2 substituents of a straight chain or branched chain alkyl group having 1 to 6 carbon atoms, for example, aminomethyl, 2-aminoethyl, 1-aminoethyl, 3-aminopropyl, 4-aminobutyl, 5-aminopentyl, 6-aminohexyl, 1,1-dimethyl-2-aminoethyl, 2-methyl-3-aminopropyl, methylaminomethyl, 1-ethylaminoethyl, 2-propylaminoethyl, 3-isopropylaminopropyl, 4-butylaminobutyl, 5-pentylaminopentyl, 6-hexylaminohexyl, dimethylaminomethyl, (N-ethyl-N-propylamino)methyl and 2-(N-methyl-N-hexylamino)ethyl.

The "5- or 6-membered saturated heterocyclic group which is formed by binding the groups $R^9$ and $R^{10}$ together with the nitrogen atom to which they bond with or without being intervened with nitrogen or oxygen atom" includes, for example, pyrrolidinyl, piperidinyl, piperazinyl and morpholino.

The "heterocyclic group having a substituent selected from a lower alkyl, a lower alkoxycarbonyl and piperidinyl" includes a heterocyclic group having 1 to 3 substituents selected from a straight chain or branched chain alkyl group having 1 to 6 carbon atoms, a straight chain or branched chain alkoxycarbonyl having 1 to 6 carbon atoms and piperidinyl, for example, in addition to the above-mentioned heterocyclic groups having a substituent of a lower alkyl and piperidinyl, 4-methoxycarbonylpiperazinyl, 4-ethoxycarbonylpiperidinyl, 3-propoxycarbonylpyrrolidinyl, 2-pentyloxycarbonylmorpholino, 4-hexyloxycarbonylpiperidinyl, 4-ethoxycarbonyl-3-methylpiperidinyl and 3-methyl-4-ethoxycarbonylpiperazinyl.

The "5- or 6-membered saturated heterocyclic group which is formed by binding the groups $R^{14}$ and $R^{15}$ together with the nitrogen atom to which they bond with or without being intervened with nitrogen or oxygen atom" includes, for example, pyrrolidinyl, piperidinyl, piperazinyl and morpholino.

The "heterocyclic group having a lower alkyl substituent" includes a heterocyclic group having 1 to 3 substituents of a straight chain or branched chain alkyl group having 1 to 6 carbon atoms, for example, 4-methylpiperazinyl, 3-4-dimethylpiperazinyl, 3-ethylpyrrolidinyl, 2-propylpyrrolidinyl, 3,4,5-trimethylpiperidinyl, 4-butylpiperidinyl, 3-pentylmorpholino and 4-hexylpiperazinyl.

The heterocyclic ring in the formula (1) includes tetrahydroquinolyl, 2,3,4,5-tetrahydro-1H-benzazepinyl, 1,2,3,4,5,6-hexahydrobenzazocinyl, 1,2-dihydroquinolyl, 2,3-dihydro-1H-benzazepinyl and 1,2,3,4-tetrahydrobenzazocinyl.

The heterocyclic ring in the formula (1) includes 3,4-dihydro-2H-1,4-benzoxazinyl, 1,2,3,5-tetrahydro-4,1-benzoxazepinyl, 1,2,3,4-tetrahydroquinoxalinyl, 1,2,3,4,5,6-hexahydro-1,5-benzodiazocinyl, 5-methyl-1,2,3,4,5,6-hexahydro-1,5-benzodiazocinyl, 4-methyl-1,2,3,4-tetrahydroquinoxalinyl, 1,2,3,4-tetrahydro-5,1-benzoxazepinyl, 3,4-dihydro-2H-1,4-benzothiazinyl, 2,3,4,5-tetrahydro-1,5-benzothiazepinyl, 1,2,3,5-tetrahydro-4,1-benzothiazepinyl, 4-ethyl-1,2,3,4-tetrahydroquinoxalinyl, 4-propyl-1,2,3,4-tetrahydroquinoxalinyl, 4-butyl-1,2,3,4-tetrahydroquinoxalinyl, 4-pentyl-1,2,3,4-tetrahydroquinoxalinyl, 4-hexyl-1,2,3,4-tetrahydroquinoxalinyl, 2,3,4,5-tetrahydro-1H-1,4-benzodiazepinyl, 4-methyl-2,3,4,5-tetrahydro-1H-1,4-benzodiazepinyl, 4-ethyl-2,3,4,5-tetrahydro-1H-1,4-benzodiazepinyl, 4-propyl-2,3,4,5-tetrahydro-1H-1,4-benzodiazepinyl, 4-butyl-2,3,4,5-tetrahydro-1H-1,4-benzodiazepinyl, 4-pentyl-2,3,4,5-tetrahydro-1H-1,4-benzodiazepinyl, 4-hexyl-2,3,4,5-tetrahydro-1H-1,4-benzodiazepinyl, 2,3,4,5-tetrahydro-1H-1,5-benzodiazepinyl, 5-methyl-2,3,4,5-tetrahydro-1H-1,5-benzodiazepinyl, 5-ethyl-2,3,4,5-tetrahydro-1H-1,5-benzodiazepinyl, 5-propyl-2,3,4,5-tetrahydro-1H-1,5-benzodiazepinyl, 5-butyl-2,3,4,5-tetrahydro-1H-1,5-benzodiazepinyl, 5-pentyl-2,3,4,5-tetrahydro-1H-1,5-benzodiazepinyl, 5-hexyl-2,3,4,5-tetrahydro-1H-1,5-benzodiazepinyl, 3,4-dihydro-1-oxo-2H-1,4-benzothiazepinyl, 3,4-dihydro-1,1-dioxo-2H-1,4-benzothiazepinyl, 1-oxo-2,3,4,5-tetrahydro-1,5-benzothiazepinyl, 1,1-dioxo-2,3,4,5-tetrahydro-1,5-benzothiazepinyl, 4-oxo-1,2,3,5-tetrahydro-4,1-benzothiazepinyl and 4,4-dioxo-1,2,3,5-tetrahydro-4,1-benzothiazepinyl.

The "halogen-substituted lower alkoxy" includes a straight chain or branched chain alkoxy group having 1 to 6 carbon atoms which has 1 to 3 substituents of a halogen atom, for example, trifluoromethoxy, trichloromethoxy, chloromethoxy, bromomethoxy, fluoromethoxy, iodomethoxy, difluoromethoxy, dibromomethoxy, 2-chloroethoxy, 2,2,2-trifluoroethoxy, 2,2,2-trichloroethoxy, 3-chloropropoxy, 2,3-dichloropropoxy, 4,4,4-trichlorobutoxy, 4-fluorobutoxy, 5-chloropentyloxy, 3-chloro-2-methylpropoxy, 6-bromohexyloxy and 5,6-dichlorohexyloxy.

The "halogen-substituted lower alkanoyl" includes a straight chain or branched chain alkanoyl group having 1 to 6 carbon atoms which has 1 to 3 substituents of a halogen atom, for example, 2,2,2-trifluoroacetyl, 2,2,2-trichloroacetyl, 2-chloroacetyl, 2-bromoacetyl, 2-fluoroacetyl, 2-iodoacetyl, 2,2-difluoroacetyl, 2,2-dibromoacetyl, 3,3,3-trifluoropropionyl, 3,3,3-trichloropropionyl, 3-chloropropionyl, 2,3-dichloropropionyl, 4,4,4-trichlorobutyryl, 4-fluorobutyryl, 5-chloropentanoyl, 3-chloro-2-methylpropionyl, 6-bromohexanoyl and 5,6-dibromohexanoyl.

The "aminocarbonyl-lower alkoxy having a lower alkyl substituent" includes a straight chain or branched chain alkoxy group having 1 to 6 carbon atoms which is substituted by an aminocarbonyl group having 1 to 2 substituents of a straight chain or branched chain alkyl group having 1 to 6 carbon atoms, for example, methylaminocarbonylmethoxy, 1-ethylaminocarbonylethoxy, 2-propylaminocarbonylethoxy, 3-isopropylaminocarbonylpropoxy, 4-butylaminocarbonylbutoxy, 5-pentylaminocarbonylpentyloxy, 6-hexylaminocarbonylhexyloxy, dimethylaminocarbonylmethoxy, 3-diethylaminocarbonylpropoxy, diethylaminocarbonylmethoxy, (N-ethyl-N-propylamino)carbonylmethoxy and 2-(N-methyl-N-hexylamino)carbonylethoxy.

The "carbamoyl-lower alkyl" includes a carbamoyl-substituted alkyl group wherein the alkyl moiety is a straight chain or branched chain alkyl group having 1 to 6 carbon atoms, for example, carbamoylmethyl, 2-carbamoylethyl, 1-carbamoylethyl, 3-carbamoylpropyl, 4-carbamoylbutyl, 5-carbamoylpentyl, 6-carbamoylhexyl, 1,1-dimethyl-2-carbamoylethy and 2-methyl-3-carbamoylpropyl.

The "amino-lower alkanoyl having optionally a lower alkyl substituent" includes a straight chain or branched chain alkanoyl having 2 to 6 carbon atoms which is substituted by an amino group having optionally 1 to 2 substituents of a straight chain or branched chain alkyl group having 1 to 6 carbon atoms, for example, 2-aminoacetyl, 3-aminopropionyl, 2-aminopropionyl, 4-aminobutyryl, 5-aminopentanoyl, 6-aminohexanoyl, 2,2-dimethyl-3-aminopropionyl, 2-methyl-3-aminopropionyl, 2-methylaminoacetyl, 2-ethylaminopropionyl, 3-propylaminopropionyl, 3-isopropylaminopropionyl, 4-butylaminobutyryl, 5-pentylaminopentanoyl, 6-hexylaminohexanoyl, 2-dimethylaminoacetyl, 2-diethylaminoacetyl, 2-(N-ethyl-N-propylamino)acetyl and 3-(N-methyl-N-hexylamino)propionyl.

The "amino-lower alkyl having optionally a lower alkanoyl substituent" includes a straight chain or branched chain alkyl having 1 to 6 carbon atoms which is substituted by an amino group having optionally a substituent of a straight chain or branched chain alkanoyl group having 1 to 6 carbon atoms, for example, aminomethyl, 2-aminoethyl, 1-aminoethyl, 3-aminopropyl, 4-aminobutyl, 5-aminopenty, 6-aminohexyl, 1,1-dimethyl-2-aminoethyl, 2-methyl-3-aminopropyl, acetylaminomethyl, 1-acetylaminoethyl, 2-propionylaminoethyl, 3-isopropionylaminopropyl, 4-butyrylaminobutyl, 5-pentanoylaminopentyl, 6-hexanoylaminohexyl and formylaminomethyl.

The "anilinocarbonyl having optionally a lower alkyl substituent on the phenyl ring" includes an anilinocarbonyl group having optionally 1 to 3 substituents of a straight chain or branched chain alkyl group having 1 to 6 carbon atoms on the phenyl ring, for example, anilinocarbonyl, 2-methylanilinocarbonyl, 3-methylanilinocarbonyl, 4-methylanilinocarbonyl, 2-ethylanilinocarbonyl, 3-ethylanilinocarbonyl, 4-ethylanilinocarbonyl, 4-isopropylanilinocarbonyl, 3-butylanilinocarbonyl, 4-pentylanilinocarbonyl, 4-hexylanilinocarbonyl, 3,4-dimethylanilinocarbonyl, 3,4-diethylanilinocarbonyl, 2,4-dimethylanilinocarbonyl, 2,5-dimethylanilinocarbonyl, 2,6-dimethylanilinocarbonyl and 3,4,5-trimethylanilinocarbonyl.

The "phenylsulfonyl which has optionally a substituent selected from a halogen and a lower alkyl on the phenyl ring" includes a phenylsulfonyl group which has optionally 1 to 3 substitutents selected from a straight chain or branched chain alkyl group having 1 to 6 carbon atoms and a halogen atom, for example, phenylsulfonyl, 2-chlorophenylsulfonyl, 3-chlorophenylsulfonyl, 4-chlorophenylsulfonyl, 2-fluorophenylsulfonyl, 3-fluorophenylsulfonyl, 4-fluorophenylsulfonyl, 2-bromophenylsulfonyl, 3-bromophenylsulfonyl, 4-bromophenylsulfonyl, 2-iodophenylsulfonyl, 3-iodophenylsulfonyl, 4-iodophenylsulfonyl, 3,4-dichlorophenylsulfonyl, 3,5-dichlorophenylsulfonyl, 2,6-dichlorophenylsulfonyl, 2,3-dichlorophenylsulfonyl, 2,4-dichlorophenylsulfonyl, 3,4-difluorophenylsulfonyl, 3,5-dibromophenylsulfonyl, 3,4,5-trichlorophenylsulfonyl, 2-ethyl-3-chlorophenylsulfonyl, 2-methylphenylsulfonyl, 3-methylphenylsulfonyl, 4-methylphenylsulfonyl, 2-ethylphenylsulfonyl, 3-ethylphenylsulfonyl, 4-ethylphenylsulfonyl, 4-isopropylphenylsulfonyl, 3-butylphenylsulfonyl, 4-pentylphenylsulfonyl, 4-hexylphenylsulfonyl, 3,4-dimethylphenylsulfonyl, 3,4-diethylphenylsulfonyl, 2,4-dimethylphenylsulfonyl, 2,5-dimethylphenylsulfonyl, 2,6-dimethylphenylsulfonyl, 3,4,6-trimethylphenylsulfonyl, 3,4,5-trimethylphenylsulfonyl, 3-chloro-4-methylphenylsulfonyl, 4-methyl-5-iodophenylsulfonyl, 3,4-dimethyl-5-bromophenylsulfonyl and 3,5-diiodo-4-methylphenylsulfonyl.

The "phthalimido-substituted lower alkyl" includes a straight chain or branched chain alkyl group having 1 to 6 carbon atoms which is substituted by phthalimido group, for example, phthalimidomethyl, 2-phthalimidoethyl, 1-phthalimidoethyl, 3-phthalimidopropyl, 4-phthalimidobutyl, 5-phthalimidopentyl, 6-phthalimidohexyl, 1,1-dimethyl-2-phthalimidoethyl and 2-methyl-3-phthalimidopropyl.

The "lower alkynyl" includes a straight chain or branched chain alkynyl having 2 to 6 carbon atoms, for example, ethynyl, 2-propynyl, 2-butynyl, 3-butynyl, 1-methyl-2-propynyl, 2-pentynyl and 2-hexynyl.

The "benzoyl which has optionally a halogen substituent on the phenyl ring" includes a benzoyl group which has optionally 1 to 3 substituents of a halogen atom on the phenyl ring, for example, benzoyl, 2-chlorobenzoyl, 3-chlorobenzoyl, 4-chlorobenzoyl, 2-fluorobenzoyl, 3-fluorobenzoyl, 4-fluorobenzoy1, 2-bromobenzoyl, 3-bromobenzoyl, 4-bromobenzoyl, 2-iodobenzoyl, 3-iodobenzoyl, 4-iodobenzoyl, 3,4-dichlorobenzoyl, 3,5-dichlorobenzoyl, 2,6-dichlorobenzoyl, 2,3-dichlorobenzoyl, 2,4-dichlorobenzoyl, 3,4-difluorobenzoyl, 3,5-dibromobenzoyl and 3,4,5-trichlorobenzoyl.

The "phenyl-lower alkoxy" includes a phenylalkoxy group wherein the alkoxy moiety is a straight chain or branched chain alkoxy group having 1 to 6 carbon atoms, for example, benzyloxy, 2-phenylethoxy, 1-phenylethoxy, 3-phenylpropoxy, 4-phenylbutoxy, 5-phenylpentyloxy, 6-phenylhexyloxy, 1,1-dimethyl-2-phenylethoxy and 2-methyl-3-phenylpropoxy.

The "amino-lower alkoxy having optionally a substituent selected from a lower alkyl and a lower alkanoyl" include a straight chain or branched chain alkoxy group having 1 to 6 carbon atoms which is substituted by an amino group having optionally 1 to 2 substituents selected from a straight chain or branched chain alkyl group having 1 to 6 carbon atoms and a straight chain or branched chain alkanoyl group having 1 to 6 carbon atoms, for example, aminomethoxy, 2-aminoethoxy, 1-aminoethoxy, 3-aminopropoxy, 4-aminobutoxy, 5-aminopentyloxy, 6-aminohexyloxy, 1,1-dimethyl-2-aminoethoxy, 2-methyl-3-aminopropoxy, acetylaminomethoxy, 1-acetylaminoethoxy, 2-propionylaminoethoxy, 3-isopropionylaminopropoxy, 4-butyrylaminobutoxy, 5-pentanoylaminopentyloxy, 6-hexanoylaminohexyloxy, formylaminomethoxy, methylaminomethoxy, 1-ethylaminoethoxy, 2-propylaminoethoxy, 3-isopropylaminopropoxy, 4-butylaminobutoxy, 5-pentylaminopentyloxy, 6-hexylaminohexyloxy, dimethylaminomethoxy, (N-ethyl-N-propylamino)methoxy and 2-(N-methyl-N-hexylamino)ethoxy.

The "benzoyloxy which has optionally a halogen substituent on the phenyl ring" includes a benzoyloxy group which has optionally 1 to 3 substituents of a halogen atom on the phenyl ring, for example, benzoyloxy, 2-chlorobenzoyloxy, 3-chlorobenzoyloxy, 4-chlorobenzoyloxy, 2-fluorobenzoyloxy, 3-fluorobenzoyloxy, 4-fluorobenzoyloxy, 2-bromobenzoyloxy, 3-bromobenzoyloxy, 4-bromobenzoyloxy, 2-iodobenzoyloxy, 3-iodobenzoyloxy, 4-iodobenzoyloxy, 3,4-dichlorobenzoyloxy, 3,5-dichlorobenzoyloxy, 2,6-dichlorobenzoyloxy, 2,3-dichlorobenzoyloxy, 2,4-dichlorobenzoyloxy, 3,4-difluorobenzoyloxy, 3,5-dibromobenzoyloxy and 3,4,5-trichlorobenzoyloxy.

The "lower alkanoyloxy-substituted lower alkyl" includes a straight chain or branched chain alkyl group having 1 to 6 carbon atoms which is substituted by a straight chain or branched chain alkanoyloxy group having 2 to 6 carbon atoms, for example, acetyloxymethyl, 2-propionyloxyethyl, 1-butyryloxyethyl, 3-acetyloxypropyl, 4-acetyloxybutyl, 4-isobutyryloxybutyl, 5-pentanoyloxypentyl, 6-acetyloxyhexyl, 6-tert-butylcarbonyloxyhexyl, 1,1-dimethyl-2-hexanoyloxyethyl and 2-methyl-3-acetyloxypropyl.

The "lower alkylsulfonyloxy-lower alkyl" includes a straight chain or branched chain alkyl group having 1 to 6 carbon atoms which is substituted by a straight chain or branched chain alkylsulfonyloxy group having 1 to 6 carbon atoms, for example, methylsulfonyloxymethyl, 1-ethylsulfonyloxyethyl, 2-propylsulfonyloxyethyl, 3-isopropylsulfonyloxypropyl, 4-butylsulfonyloxybutyl, 5-pentylsulfoyloxypentyl, 6-hexylsulfonyloxyhexyi, 1,1-dimethyl-2-methylsulfoyloxyethyl and 2-methyl-3-ethylsulfonyloxypropyl.

The "azido-lower alkyl" includes a straight chain or branched chain alkyl group having 1 to 6 carbon atoms which is substituted by an azido group, for example, azidomethyl, 1-azidoethyl, 2-azidoethyl, 3-azidopropyl, 4-azidobutyl, 5-azidopentyl, 6-azidohexyl, 1,1-dimethyl-2-azidoethyl and 2-methyl-3-azidopropyl.

The "lower alkanoyloxyimino" includes a straight chain or branched chain alkanoyloxyimino group having 1 to 6 carbon atoms, for example, formyloxyimino, acetyloxyimino, propionyloxyimino, butyryloxyimino, isobutyryloxyimino, pentanoyloxyimino, tert-butylcarbonyloxyimino and hexanoyloxyimino.

The "lower alkylidene" includes a straight chain or branched chain alkylidene group having 1 to 6 carbon atoms, for example, methylidene, ethylidene, propylidene, isopropylidene, butylidene, pentylidene and hexylidene.

The "oxiranyl-substituted lower alkyl" includes a straight chain or branched chain alkyl group having 1 to 6 carbon atoms which is substituted by oxiranyl group, for example, oxiranylmethyl, 1-oxiranylethyl, 2-oxiranylethyl, 3-oxiranylpropyl, 4-oxiranylbutyl, 5-oxiranylpentyl, 6-oxiranylhexyl, 1,1-dimethyl-2-oxiranylethyl and 2-methyl-3-oxiranylpropyl.

The "lower alkyl having 1 to 2 substituents selected from a lower alkoxy, hydroxy and an amino having optionally a lower alkyl substituent" includes a straight chain or branched chain alkyl group having 1 to 6 carbon atoms and having 1 to 2 substituents selected from a straight chain or branched chain alkoxy group having 1 to 6 carbon atoms, hydroxy and an amino having optionally a straight chain or branched chain alkyl group having 1 to 6 carbon atoms, for example, methoxymethyl, 1-ethoxyethyl, 2-propoxyethyl, 3-isopropoxypropyl, 4-butoxybutyl, 5-pentyloxypentyl, 6-hexyloxyhexyl, 1,1-dimethyl-2-methoxyethyl, 2-methyl-3-ethoxypropyl, 3-methoxy-2-hydroxypropyl, hydroxymethyl, 2-hydroxyethyl, 1-hydroxyethyl, 3-hydroxypropyl, 2,3-dihydroxyethyl, 4-hydroxybutyl, 3,4-dihydroxybutyl, 1,1-dimethyl-2-hydroxyethyl, 5,6-dihydroxyhexyl, 5-hydroxypentyl, 6-hydroxyhexyl, 6-(N-ethyl-N-methylamino)-5-methoxyhexyl, 2-methyl-3-hydroxypropyl, aminomethyl, 1-aminoethyl, 2-aminoethyl, 3-aminopropyl, 4-aminobutyl, 5-aminopentyl, 6-aminohexyl, 1,1-dimethyl-2-aminoethyl, 2-methyl-3-aminopropyl, methylaminomethyl, ethylaminomethy, propylaminomethyl, isopropylaminomethyl, butylaminomethyl, tert-butylaminomethyl, pentylaminomethyl, hexylaminomethyl, dimethylaminomethyl, diethylaminomethyl, dipropylaminomethyl, dibutylaminomethyl, dipentylaminomethyl, dihexylaminomethyl, N-methyl-N-ethylaminomethyl, N-methyl-N-propylaminomethyl, N-methyl-N-butylaminomethyl, N-methyl-N-hexylaminomethyl, 1-methylaminoethyl, 2-ethylaminoethyl, 3-propylaminopropyl, 4-butylaminobutyl, 1,1-dimethyl-2-pentylaminoethyl, 5-hexylaminopentyl, 6-dimethylaminohexyl, 4-dimethylaminobutyl, 2-diethylaminoethyl, 1-(N-methyl-N-hexylamino)ethyl, 3-dihexylaminopropyl, 6-diethylaminohexyl, 4-dibutylaminobutyl, 2-(N-methyl-N-pentylamino)ethyl, 2-hydroxy-3-diethylaminopropyl, 3-hydroxy-4-methylaminobutyl, 5-hydroxy-6-diethylaminohexyl, 4-hydroxy-5-dimethylaminopentyl, 4-hydroxy-5-methylaminopentyl, 4-hydroxy-5-diethylaminopentyl, 5-hydroxy-6-ethylaminohexyl, 5-hydroxy-6-isopropylaminohexyl and 5-hydroxy-6-aminohexyl.

The "aminocarbonyloxy having optionally a lower alkyl substituent" includes an aminocarbonyloxy group having optionally 1 to 2 substituents of a straight chain or branched chain alkyl group having 1 to 6 carbon atoms, for example, aminocarbonyloxy, methylaminocarbonyloxy, ethylaminocarbonyloxy, propylaminocarbonyloxy, isopropylaminocarbonyloxy, butylaminocarbonyloxy, tert-butylaminocarbonyloxy, pentylaminocarbonyloxy, hexylaminocarbonyloxy, dimethylaminocarbonyloxy, diethylaminocarbonyloxy, dipropylaminocarbonyloxy, dibutylaminocarbonyloxy, dipentylaminocarbonyloxy, dihexylaminocarbonyloxy, N-methyl-N-ethylaminocarbonyloxy, N-ethyl-N-propylaminocarbonyloxy, N-methyl-N-butylaminocarbonyloxy and N-methyl-N-hexylaminocarbonyloxy.

The "lower alkanoyloxy having optionally a halogen substituent" includes a straight chain or branched chain alkanoyloxy group having 1 to 6 carbon atoms which has optionally 1 to 3 substituents a halogen atom, for example, in addition to the above lower alkanoyl group, 2,2,2-trifluoroacetyloxy, 2,2,2-trichloroacetyloxy, 2-chloroacetyloxy, 2-bromoacetyloxy, 2-fluoroacetyloxy, 2-iodoacetyloxy, 2,2-difluoroacetyloxy, 2,2-dibromoacetyloxy, 3,3,3-trifluoropropionyloxy, 3,3,3-trichloropropionyloxy, 3-chloropropionyloxy, 2,3-dichloropropionyloxy, 4,4,4-trichlorobutyryloxy, 4-fluorobutyryloxy, 5-chloropentanoyloxy, 3-chloro-2-methylpropionyloxy, 6-bromohexanoyloxy and 5,6-dibromohexanoyloxy.

The "amino-lower alkyl having optionally a substituent selected from a lower alkyl and a lower alkanoyl" include a straight chain or branched chain alkyl group having 1 to 6 carbon atoms which is substituted by an amino group having optionally 1 to 2 substituents selected from a straight chain or branched chain alkyl group having 1 to 6 carbon atoms and a straight chain or branched chain alkanoyl group having 1 to 6 carbon atoms, for example, aminomethyl, 2-aminoethyl, 1-aminoethyl, 3-aminopropyl, 4-aminobutyl, 5-aminopentyl, 6-aminohexyl, 1,1-dimethyl-2-aminoethyl, 2-methyl-3-aminopropyl, acetylaminomethyl, 1-acetylaminoethyl, 2-propionylaminoethyl, 3-isopropionylaminopropyl, 4-butyrylaminobutyl, 5-pentanoylaminopentyl, 6-hexanoylaminohexyl, formylaminomethyl, methylaminomethyl, 1-ethylaminoethyl, 2-propylaminoethyl, 3-isopropylaminopropyl, 4-butylaminobutyl, 5-pentylaminopentyl, 6-hexylaminohexyl, dimethylaminomethyl, (N-ethyl-N-propylamino)methyl and 2-(N-methyl-N-hexylamino)ethyl.

The "amino-lower alkanoyloxy having optionally a lower alkyl substituent" includes a straight chain or branched chain alkanoyloxy having 2 to 6 carbon atoms which is substituted by an amino group having optionally 1 to 2 substituents of a straight chain or branched chain alkyl group having 1 to 6 carbon atoms, for example, 2-aminoacetyloxy, 3-aminopropionyloxy, 2-aminopropionyloxy, 4-aminobutyryloxy, 5-aminopentanoyloxy, 6-aminohexanoyloxy, 2,2-dimethyl-3-aminopropionyloxy, 2-methyl-3-aminopropionyloxy, 2-methylaminoacetyloxy, 2-thylaminopropionyloxy, 3-propylaminopropionyloxy, 3-isopropylaminopropionyloxy, 4-butylaminobutyryloxy, 5-pentylaminopentanoyloxy, 6-hexylaminohexanoyloxy, 2-dimethylaminoacetyloxy, 2-diethylaminoacetyloxy, 2-(N-ethyl-N-propylamino)acetyloxy and 3-(N-methyl-N-hexylamino)propionyloxy.

The "pyridyl-lower alkyl" include a pyridylalkyl group wherein the alkyl moiety is a straight chain or branched chain alkyl group having 1 to 6 carbon atoms, for example, (4-pyridyl)methyl, 1-(3-pyridyl)ethyl, 2-(2-pyridyl)ethyl, 3-(2-pyridyl)propyl, 4-(3-pyridyl)butyl, 5-(4-pyridyl)pentyl, 6-(2-pyridyl)hexyl, 1,1-dimethyl-2-(3-pyridyl)ethyl and 2-methyl-3-(4-pyridyl)propyl.

The "5- or 6-membered saturated heterocyclic group which is formed by binding the groups $R^{82}$ and $R^{83}$ together with the nitrogen atom to which they bond with or without being intervened with nitrogen, oxygen or sulfur atom" includes, for example, pyrrolidinyl, piperidinyl, piperazinyl, morpholino and thiomorpholino.

The above heterocyclic group which has a substituent selected from oxo, a lower alkyl, a lower alkanoyl and carbamoyl includes the above heterocyclic groups which have 1 to 3 substituents selected from oxo, a straight chain or branched chain alkyl group having 1 to 6 carbon atoms, a straight chain or branched chain alkanoyl group having 1 to 6 carbon atoms, and carbamoyl group, for example, 4-methylpiperazinyl, 3,4-dimethylpiperazinyl, 3-ethylpyrrolidinyl, 2-propylpyrrolidinyl, 3,4,5-trimethylpiperidinyl, 4-butylpiperidinyl, 3-pentylmorpholino, 4-hexylpiperazinyl, 2-methylthiomorpholino, 4-acetylpiperazinyl, 2-propionylmorpholino, 3-butyrylthiomorpholino, 3-pentanoylpyrrolidinyl, 4-hexanoylpiperidinyl, 3-methyl-4-acetylpiperazinyl, 2-carbamoylpyrrolidinyl, 4-carbamoylpiperazinyl, 3-carbamoylthiomorpholino, 2-carbamoylmorpholino, 3-carbamoylpiperidinyl, 1-oxo-thiomorpholino and 1,1-dioxothiomorpholino.

The "lower alkylsulfonyl" includes a straight chain or branched chain alkylsulfonyl group having 1 to 6 carbon atoms, for example, methylsulfonyl, ethylsulfonyl, propylsulfonyl, isopropylsulfonyl, butylsulfonyl, tert-butylsulfonyl, pentylsulfonyl and hexylsulfonyl.

The "aminocarbonyl having optionally a lower alkyl substituent" includes an aminocarbonyl group having optionally 1 to 2 substituents of a straight chain or branched chain alkyl group having 1 to 6 carbon atoms, for example, aminocarbonyl, methylaminocarbonyl, ethylaminocarbonyl, propylaminocarbonyl, isopropylaminocarbonyl, butylaminocarbonyl, tert-butylaminocarbonyl, pentylaminocarbonyl, hexylaminocarbonyl, dimethylaminocarbonyl, diethylaminocarbonyl, dipropylaminocarbonyl, dibutylaminocarbonyl, dipentylaminocarbonyl, dihexylaminocarbonyl, N-methyl-N-ethylaminocarbonyl, N-ethyl-N-propylaminocarbonyl, N-methyl-N-butylaminocarbonyl and N-methyl-N-hexylaminocarbonyl.

The "cyano-substituted lower alkyl" includes a straight chain or branched chain alkyl group having 1 to 6 carbon atoms which is substituted by cyano group, for example, cyanomethyl, 2-cyanoethyl, 1-cyanoethyl, 3-cyanopropyl, 4-cyanobutyl, 5-cyanopentyl, 6-cyanohexyl, 1,1-dimethyl-2-caynoethyl and 2-methyl-3-cyanopropyl.

The "lower alkoxycarbonyl-substituted lower alkyl" includes an alkoxycarbonyl-substituted straight chain or branched chain alkyl group having 1 to 6 carbon atoms wherein the alkoxycarbonyl moiety is a straight chain or branched chain alkoxycarbonyl group having 1 to 6 carbon atoms, for example, methoxycarbonylmethyl, 3-methoxycarbonylpropyl, ethoxycarboxymethyl, 3-ethoxycarbonylpropyl, 4-ethoxycarbonylbutyl, 5-isopropoxycarbonylpentyl, 6-propoxycarbonylhexyl, 1,1-dimethyl-2-butoxycarbonylethyl, 2-methyl-3-tert-butoxycarbonylpropyl, 2-pentyloxycarbonylethyl and hexyloxycarbonylmethyl.

The "carboxy-substituted lower alkyl" includes a carboxy-substituted alkyl group wherein the alkyl moiety is a straight chain or branched chain alkyl group having 1 to 6 carbon atoms, for example, carboxymethyl, 2-carboxyethyl, 1-carboxyethyl, 3-carboxypropyl, 4-carboxybutyl, 5-carboxypentyl, 6-carboxyhexyl, 1,1-dimethyl-2-carboxyethyl and 2-methyl-3-carboxypropyl.

The "tetrahydropyranyloxy-substituted lower alkyl" includes a tetrahydropyranyloxy-substituted straight chain or branched chain alkyl group having 1 to 6 carbon atoms, for example, (2-tetrahydropyranyloxy)methyl, 2-(3-tetrahydropyranyloxy)ethyl, 1-(4-tetrahydropyranyloxy)ethyl, 3-(2-tetrahydropyranyloxy)propyl, 4-(3-tetrahydropyranyloxy)butyl, 5-(4-tetrahydropyranyloxy)pentyl, 6-(2-tetrahydropyranyloxy)hexyl, 1,1-dimethyl-2-(3-tetrahydropyranyloxy)ethyl and 2-methyl-3-(4-tetrahydropyranyloxy)propyl.

The "piperidinyl having optionally a phenyl-lower alkyl substituent" includes a piperidinyl which has optionally a substituent of a phenylalkyl group wherein the alkyl moiety is a straight chain or branched chain alkyl group having 1 to 6 carbon atoms, for example, piperidinyl, 1-benzyl-4-piperidinyl, 1-(2-phenylethyl)-3-piperidinyl, 1-(1-phenylethyl)-2-piperidinyl, 1-(3-phenylpropyl)-4-piperidinyl, 1-(4-phenylbutyl)-4-piperidinyl, 1-(5-phenylpentyl)-4-piperidinyl, 1-(6-phenylhexyl)-4-piperidinyl, 1-(1,1-dimethyl-2-phenylethyl)-3-piperidinyl and 1-(2-methyl-3-phenylpropyl)-2-piperidinyl.

The "imidazolyl-substituted lower alkanoyl" includes an imidazolyl-substituted alkanoyl group wherein the alkanoyl moiety is a straight chain or branched chain alkanoyl group having 2 to 6 carbon atoms, for example, (1-imidazolyl)acetyl, 3-(2-imidazolyl)propionyl, 2-(4-imidazolyl)propionyl, 4-(1-imidazolyl)butyryl, 2,2-dimethyl-3-(2-imidazolyl)propionyl, 5-(4-imidazolyl)pentanoyl and 6-(1-imidazolyl)hexanoyl.

The "amino-lower alkanoyl having optionally a substituent selected from a lower alkyl and a lower alkoxycarbonyl" includes a straight chain or branched chain alkanoyl having 2 to 6 carbon atoms which is substituted by an amino group having optionally 1 to 2 substituents selected from a straight chain or branched chain alkyl group having 1 to 6 carbon atoms and a straight chain or branched chain alkoxycarbonyl group having 1 to 6 carbon atoms, for example, 2-aminoacetyl, 3-aminopropionyl, 2-aminopropionyl, 4-aminobutyryl, 5-aminopentanoyl, 6-aminohexanoyl, 2,2-dimethyl-3-aminopropionyl, 2-methyl-3-aminopropionyl, 2-methylaminoacetyl, 2-ethylaminopropionyl, 3-propylaminopropionyl, 3-isopropylaminopropionyl, 4-butylaminobutyryl, 5-pentylaminopentanoyl, 6-hexylaminohexanoyl, 2-dimethylaminoacetyl, 2-diethylaminoacetyl, 2-(N-ethyl-N-propylamino)acetyl, 3-(N-methyl-N-hexylamino)propionyl, 2-methoxycarbonylaminoacetyl, 2-ethoxycarbonylaminoacetyl, 3-propoxycarbonylaminopropionyl, 4-butoxycarbonylaminobutyryl, 2-tert-butoxycarbonylaminoacetyl, 5-pentyloxycarbonylaminopentanoyl, 6-hexyloxycarbonylaminohexanoyl and 2-(N-methyl-N-tert-butoxycarbonylamino)acetyl.

The "aminocarbonyl-lower alkyl having a lower alkyl substituent" includes a straight chain or branched chain alkyl group having 1 to 6 carbon atoms which is substituted by an aminocarbonyl group having 1 to 2 substituents of a straight chain or branched chain alkyl group having 1 to 6 carbon atoms, for example, methylaminocarbonylmethyl, 1-ethylaminocarbonylethyl, 2-propylaminocarbonylethyl, 3-isopropylaminocarbonylpropyl, 4-butylaminocarbonylbutyl, 5-pentylaminocarbonylpentyl, 6-hexylaminocarbonylhexyl, dimethylaminocarbonylmethyl, 3-diethylaminocarbonylpropyl, diethylaminocarbonylmethyl, (N-ethyl-N-propylamino)carbonylmethyl and 2-(N-methyl-N-hexylamino)carbonylethyl.

The "amino-substituted lower alkoxy having optionally a lower alkyl substituent" includes an amino-substituted straight chain or branched chain alkoxy having 1 to 6 carbon atoms which has optionally 1 to 2 substituents of a straight chain or branched chain alkyl having 1 to 6 carbon atoms, such as aminomethoxy, 2-aminoethoxy, 1-aminoethoxy, 3-aminopropoxy, 4-aminobutoxy, 5-aminopentyloxy, 6-aminohexyloxy, 1,1-dimethyl-2-aminoethoxy, 2-methyl-3-aminopropoxy, methylaminomethoxy, 1-ethylaminoethoxy, 2-propylaminoethoxy, 3-isopropylaminopropoxy, 4-butylaminobutoxy, 5-pentylaminopentyloxy, 6-hexylaminohexyloxy, dimethylaminomethoxy, (N-ethyl-N-propylamino)methoxy and 2-(N-methyl-N-hexylamino)ethoxy.

The compounds of the present invention can be prepared by various processes, for example, by the processes shown in the following reaction schemes.

[Reaction Scheme-1]

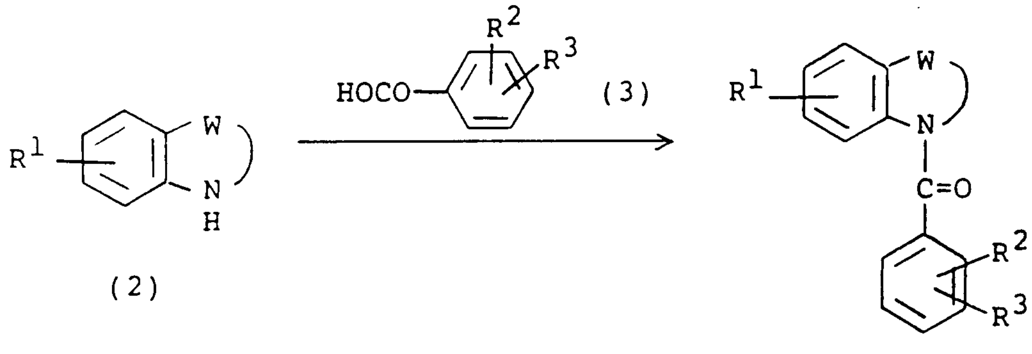

(3)

(2)

(1)

wherein $R^1$, $R^2$, $R^3$, and W are the same as defined above.

The process of Reaction Scheme-1 is carried out by reacting a benzoheterocyclic compound of the formula (2) and a carboxylic acid compound of the formula (3) by a conventional amido bond forming reaction. The amido bond forming reaction can be carried out under the conditions for the conventional amido bond forming reaction, for example,

(a) a mixed acid anhydride process, i.e. a process of reacting the carboxylic acid compound (3) with an alkylhalocarboxylic acid to form a mixed acid anhydride and reacting the resultant with the amine compound (2),
(b) an activated ester process, i.e. a process of converting the carboxylic acid compound (3) into an activated ester, such as p-nitrophenyl ester, N-hydroxysuccinimide ester, 1-hydroxybenzotriazole ester, and reacting the resultant with the amine compound (2),
(c) a carbodiimide process, i.e. a process of condensing the carboxylic acid compound (3) and the amine compound (2) in the presence of an activating agent such as dicyclohexylcarbodiimide, carbonyldiimidazole,
(d) other processes, i.e. a process of converting the carboxylic acid compound (3) into a carboxylic anhydride by treatment with a dehydrating agent such as acetic anhydride, and reacting the resultant with the amine compound (2); a process of reacting an ester of the carboxylic acid compound (3) with a lower alcohol and the amine compound (2) at a high temperature under high pressure; a process of reacting an acid halide compound of the carboxylic acid compound (3), i.e. a carboxylic acid halide, with the amine compound (2).

The mixed acid anhydride used in the above mixed acid anhydride process (a) is obtained by the known Schötten-Baumann reaction, and the reaction product is used without isolation from the reaction mixture for the reaction with the amine compound (2) to give the desired compound of the formula (1). The Schötten-Baumann reaction is usually carried out in the presence of a basic compound. The basic compound is any conventional compounds used for the Schötten-Baumann reaction and includes, for example, organic basic compounds such as triethylamine, trimethylamine, pyridine, dimethylaniline, N-methylmorpholine, 1,5-diazabicyclo[4.3.0]nonene-5 (DBN), 1,8-diazabicyclo[5.4.0]undecene-7 (DBU), 1,4-diazabicyclo[2.2.2]octane (DABCO), and inorganic basic compounds such as potassium carbonate, sodium carbonate, potassium hydrogen carbonate, sodium hydrogen carbonate. The reaction is usually carried out at a temperature of from -20°C to 100°C, preferably from 0°C to 50°C, for 5 minutes to about 10 hours, preferably 5 minutes to 2 hours.

The reaction of the thus obtained mixed acid anhydride with the amine compound (2) is usually carried out at a temperature of from -20°C to 150°C, preferably 10°C to 50°C, for 5 minutes to 10 hours, preferably 5 minutes to 5 hours. The mixed acid anhydride process is usually carried out in an appropriate solvent. The solvent is any conventional solvents which are usually used in the mixed acid anhydride process and includes, for example, halogenated hydrocarbons (e.g. chloroform, dichloromethane dichloroethane), aromatic hydrocarbons (e.g. benzene, toluene, xylene), ethers (e.g. diethyl ether, diisopropyl ether, tetrahydrofuran, dimethoxyethane), esters (e.g. methyl acetate, ethyl acetate), aprotic polar solvents (e.g. N,N-dimethylformamide, dimethylsulfoxide, hexamethylphosphoric triamide), or a mixture of these solvents. The alkylhalocarboxylic acid used in the mixed acid anhydride process includes, for example, methyl chloroformate, methyl bromoformate, ethyl chloroformate, ethyl bromoformate and isobutyl chloroformate. In said process, the carboxylic acid compound (3), the alkylhalocarboxylic acid and the amine (2) are usually used in each equimolar amount, but preferably, the alkylhalocarboxylic acid and the carboxylic acid compound (3) are used each in an amount of 1 to 1.5 mole to 1 mole of the amine (2).

Among the above other processes (d), in case of the process of reacting the carboxylic acid halide with the amine compound (2), the reaction is usually carried out in the presence of a basic compound in an appropriate solvent. The basic compound is any conventional compounds and includes, in addition to the basic compounds used for the above-mentioned Schötten-Baumann reaction, sodium hydroxide, potassium hydroxide, sodium hydride, potassium hydride. etc. The solvent includes, in addition to the solvents used for the above-mentioned mixed acid anhydride process, alcohols (e.g. methanol, ethanol, propanol, butanol, 3-methoxy-1-butanol, ethylcellosolve, methylcellosolve), acetonitrile, pyridine, acetone and water. The amount of the amine compound (2) and the carboxylic acid halide is not critical, but the carboxylic acid halide is usually used at least in equimolar amount, preferably 1 to 5 moles to 1 mole of the amine compound (2). The reaction is usually carried out at a temperature of from -20°C to 180°C, preferably from 0°C to 150°C, for 5 minutes to 30 hours.

The amido bond forming reaction in the above Reaction Scheme-1 may also be carried out by reacting the carboxylic acid compound (3) and the amine (2) in the presence of a condensation agent, i.e. phosphoric compounds such as triphenylphosphine, diphenylphosphinyl chloride, phenyl-N-phenylphosphoramide chloridate, diethyl chlorophosphate, diethyl phosphorocyanidate, diphenylphosphoric azide, bis(2-oxo-3-oxazolidinyl)phosphinic chloride. The reaction is usually carried out in the presence of the solvent and basic compound as used in the above reaction of the carboxylic acid halide and the amine (2) at a temperature of from -20°C to 150°C, preferably 0°C to 100°C, for 5 minutes to 30 hours. The condensation agent and the carboxylic acid compound (3) are used at least in equimolar amount, preferably about 1 to 2 moles, to 1 mole of the amine (2).

[Reaction Scheme-2]

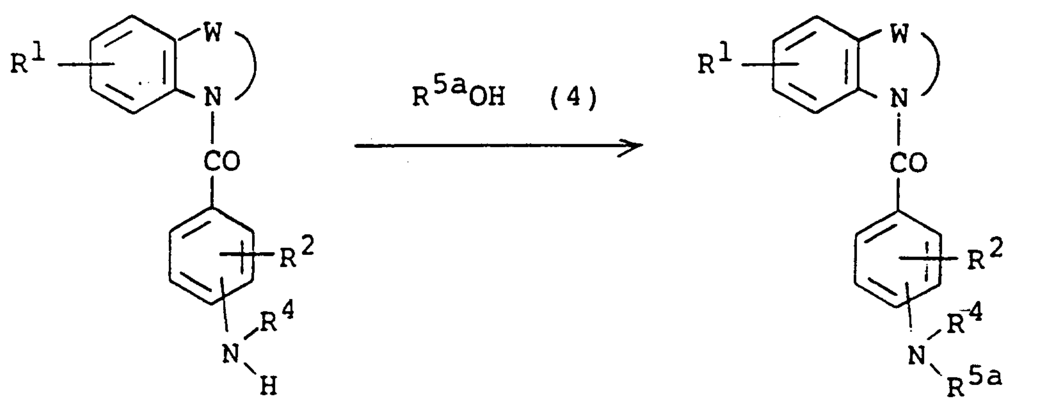


(2b) (1b)

wherein $R^1$, $R^2$, $R^4$ and W are as defined above, $R^{5a}$ is the same as $R^5$ as defined above except excluding an anilinocarbonyl having optionally a lower alkyl substituent on the phenyl ring, a phenylsulfonyl having optionally a substituent selected from a halogen atom and a lower alkyl on the phenyl ring and quinolylsulfonyl.

The reaction of the compound (2b) and the compound (4) is carried out in the same manner as in the reaction of the compound (2) and the compound (3) in the above Reaction Scheme-1.

[Reaction Scheme-3]

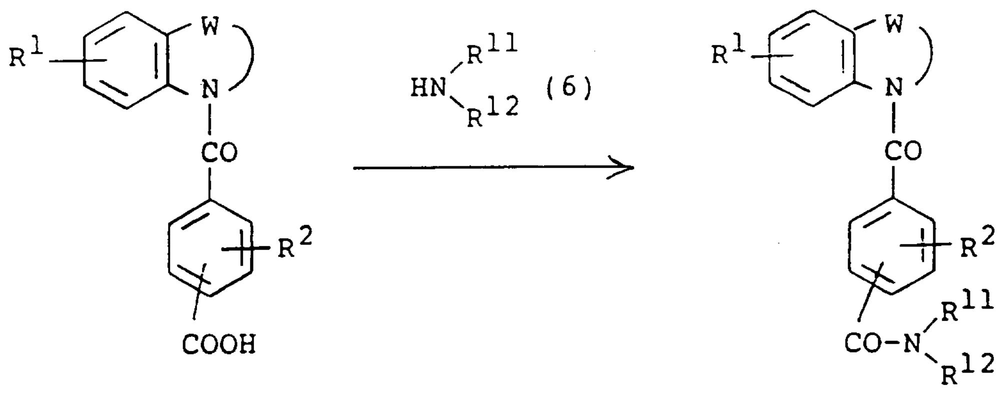


(5) (1c)

wherein $R^1$, $R^2$, $R^{11}$, $R^{12}$ and W are as defined above.

The reaction of the compound (5) and the compound (6) is carried out under the same conditions as used in the reaction of the compound (2) and the compound (3) in the above Reaction Scheme-1.

[Reaction Scheme-4]

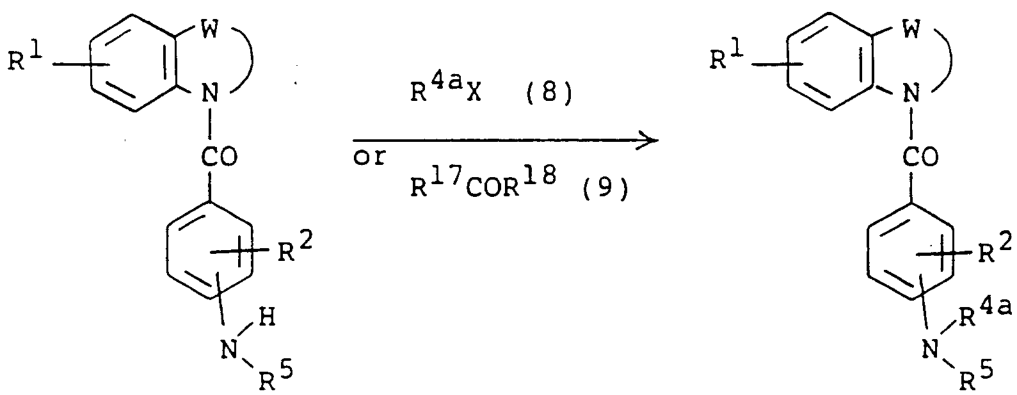


(7) (1d)

wherein $R^1$, $R^2$, $R^5$ and W are as defined above, and $R^{4a}$ is a lower alkyl, $R^{17}$ and $R^{18}$ are each hydrogen atom or a lower alkyl, and X is a halogen atom.

The reaction of the compound (7) and the compound (8) is usually carried out in an inert solvent in the presence or absence of a basic compound. The inert solvent includes, for example, aromatic hydrocarbons (e.g. benzene, toluene, xylene), ethers (e.g. tetrahydrofuran, dioxane, diethylene glycol dimethyl ether), halogenated hydrocarbons (e.g. dichloromethane, chloroform, carbon tetrachloride), lower alcohols (e.g. methanol, ethanol, isopropanol, butanol, tert-butanol), acetic acid, ethyl acetate, acetone, acetonitrile, pyridine, dimethylsulfoxide, dimethylformamide, hexamethylphosphoric triamide, or a mixture of these solvents. The basic compound includes, for example, carbonates (e.g. sodium carbonate, potassium carbonate, sodium hydrogen carbonate, potassium hydrogen carbonate), metal hydroxides (e.g. sodium hydroxide, potassium hydroxide), sodium hydride, potassium, sodium, sodium amide, metal alcoholates (e.g. sodium methoxide, sodium ethoxide), and organic basic compounds (e.g. pyridine, N-ethyldiisopropylamine, dimethylaminopyridine, triethylamine, 1,5-diazabicyclo[4.3.0]nonene-(5) (DBN), 1,8-diazabicyclo[5.4.0]undecene-7 (DBU), 1,4-diazabicyclo[2.2.2]octane (DABCO)). The amount of the compound (7) and the compound (8) is not critical, but the compound (8) is usually used at least in equivalent amount, preferably 1 to 10 moles, to 1 mole of the compound (7). The reaction

is usually carried out at a temperature of from 0°C to 200°C, preferably from 0°C to 170°C, for 30 minutes to 30 hours. In the reaction, an alkali metal halide (e.g. sodium iodide, potassium iodide) may be added to the reaction system.

The reaction of the compound (7) and the compound (9) is carried out in an appropriate solvent or without solvent in the presence of a reducing agent. The solvent includes, for example, water, alcohols (e.g. methanol, ethanol, isopropanol), acetonitrile, formic acid, acetic acid, ethers (e.g. dioxane, diethyl ether, diglyme, tetrahydrofuran), aromatic hydrocarbons (e.g. benzene, toluene, xylene), or a mixture of these solvents. The reducing agent includes, for example, formic acid, fatty acid alkali metal salts (e.g. sodium formate), hydrogenating reducing agents (e.g. sodium boro hydride, sodium cyanoboro hydride, lithium aluminum hydride), catalystic reducing agents (e.g. palladium black, palladium-carbon, platinum oxide, platinum black, Raney nickel).

When formic acid is used as the reducing agent, the reaction is usually carried out at a temperature of from room temperature to 200°C, preferably 50°C to 150°C, for 1 to 10 hours. The formic acid is usually used in a large excess amount to the compound (7).

When a hydrogenating reducing agent is used, the reaction is usually carried out at a temperature of -30°C to 100°C, preferably 0°C to 70°C, for 30 minutes to 12 hours. The reducing agent is usually used in an amount of 1 to 20 moles, preferably 1 to 6 moles, to 1 mole of the compound (7). When lithiun aluminum hydride is used as the reducing agent, it is preferable to use a solvent selected from ethers (e.g. diethyl ether, dioxane, tetrahydrofuran, diglyme) and aromatic hydrocarbons (e.g. benzene, toluene, xylene).

When a catalytic reducing agent is used, the reaction is usually carried out under atmospheric pressure to about 20 atm., preferably atmospheric pressure to about 10 atm. under hydrogen atmosphere or in the presence of a hydrogen donor (e.g. formic acid, ammonium formate, cyclohexene, hydrazine hydrate) at a temperature of -30°C to 100°C, preferably 0°C to 60°C, for 1 to 12 hours. The catalytic reducing agent is usually used in an amount of 0.1 to 40 % by weight, preferably 1 to 20 % by weight, of the amount of the compound (7). The compound (9) is usually used at least in equivalent amount, preferably equivalent to a large excess amount, to the compound (7).

[Reaction Scheme-5A]

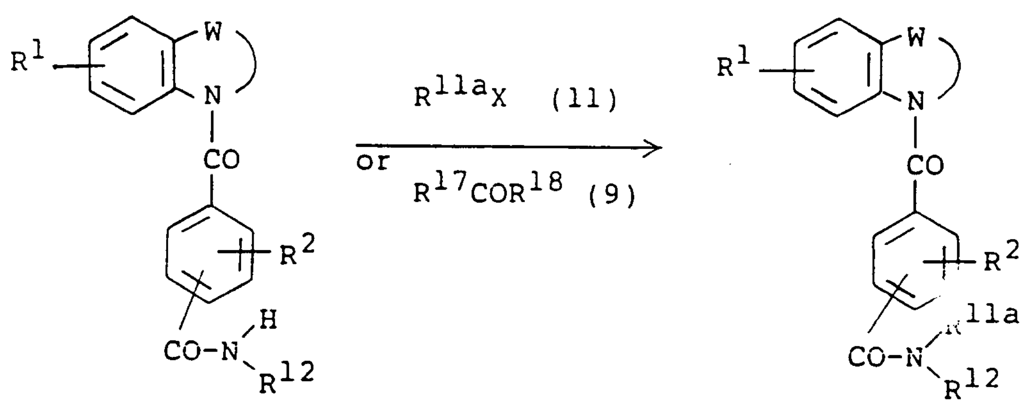


(10) (1e)

wherein $R^1$, $R^2$, $R^{12}$, $R^{17}$, $R^{18}$, X and W are as defined above, and $R^{11a}$ is a lower alkyl.

[Reaction Scheme-5B]

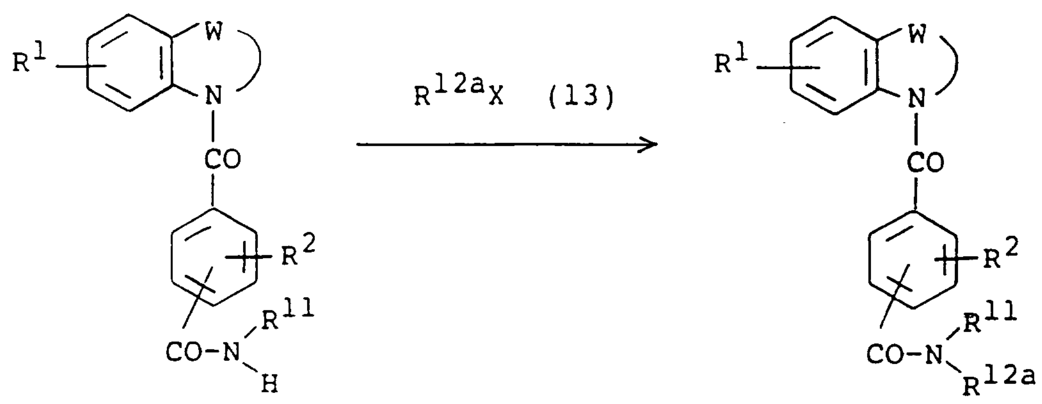


(12) (1f)

wherein $R^1$, $R^2$, $R^{11}$, X and W are as defined above, and $R^{12a}$ is a cycloalkyl.

The reaction of the compound (10) and the compound (11) in the Reaction Scheme-5A and the reaction of the compound (12) and the compound (13) in the Reaction Scheme-5B are carried out in the same manner as in the reaction of the compound (7) and the compound (8) in the above Reaction Scheme-4.

Besides, the reaction of the compound (10) and the compound (9) in the Reaction Scheme-5A is carried out in the same manner as in the reaction of the compound (7) and the compound (9) in the above Reaction Scheme-4.

Reaction Scheme-6A]

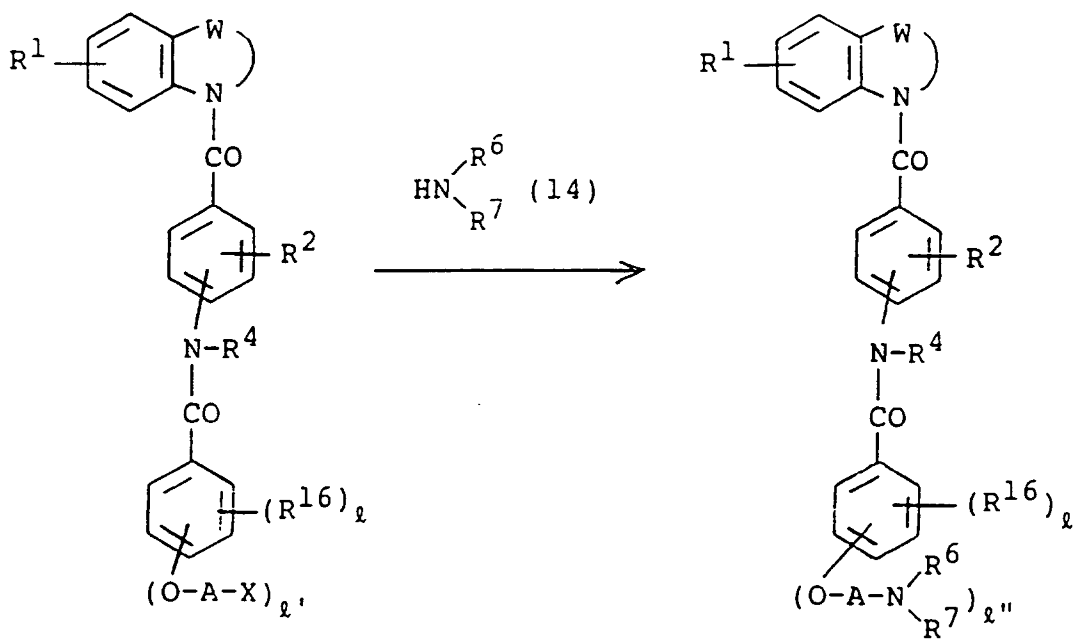


(1g) (1h)

wherein $R^1$, $R^2$, $R^4$, $R^{16}$, $R^6$, $R^7$, X, W, and A are as defined above, $\ell$ is 0 or an integer of 1 to 3, $\ell'$ and $\ell''$ are each an integer of 1 to 3, provided that $\ell + \ell'$ and $\ell + \ell''$ are each an integer not more than 3.

[Reaction Scheme-6B]

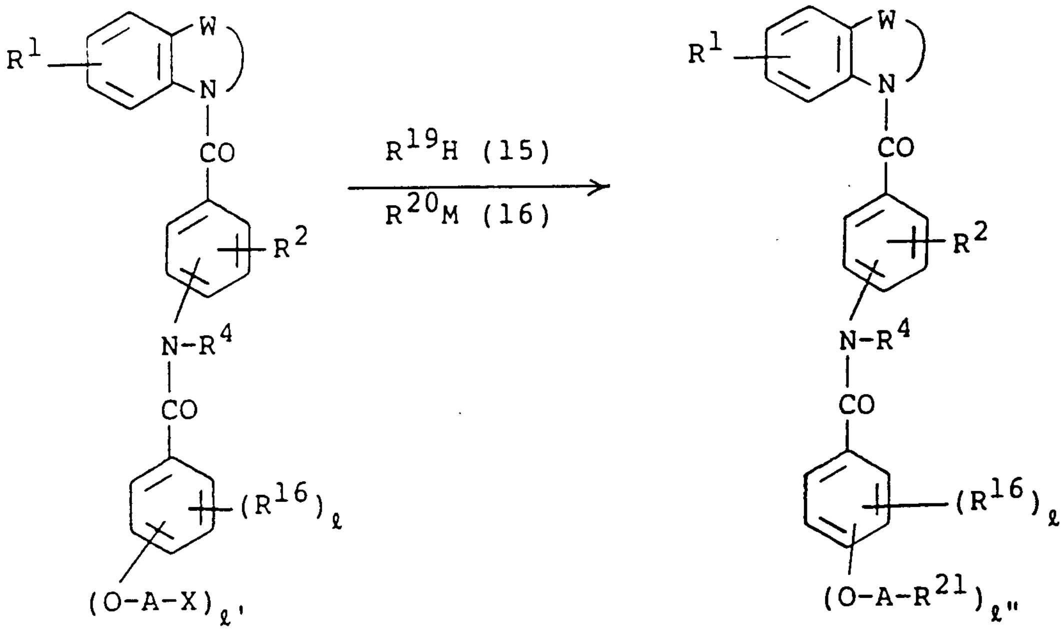


(1g)

(1i)

wherein $R^1$, $R^2$, $R^4$, $R^{16}$, X, W, A, $\ell$, $\ell'$, and $\ell''$ are as defined above, and $R^{19}$ is a lower alkanoyloxy, $R^{20}$ is a lower alkanoyloxy, hydroxy or phthalimido, $R^{21}$ is the same as as $R^{19}$ and $R^{20}$, and M is an alkali metal (e.g. potassium, sodium).

The reaction of the compound (1g) and the compound (14) in the Reaction Scheme-6A and the reaction of the compound (1g) and the compound (15) or (16) in the Reaction Scheme-6B can be carried out under the same conditions as in the reaction of the compound (7) and the compound (8) in the above Reaction Scheme-4. In the reaction, an alkali

metal halide (e.g. sodium iodide, potassium iodide) may be added to the reaction system.

[Reaction Scheme-7]

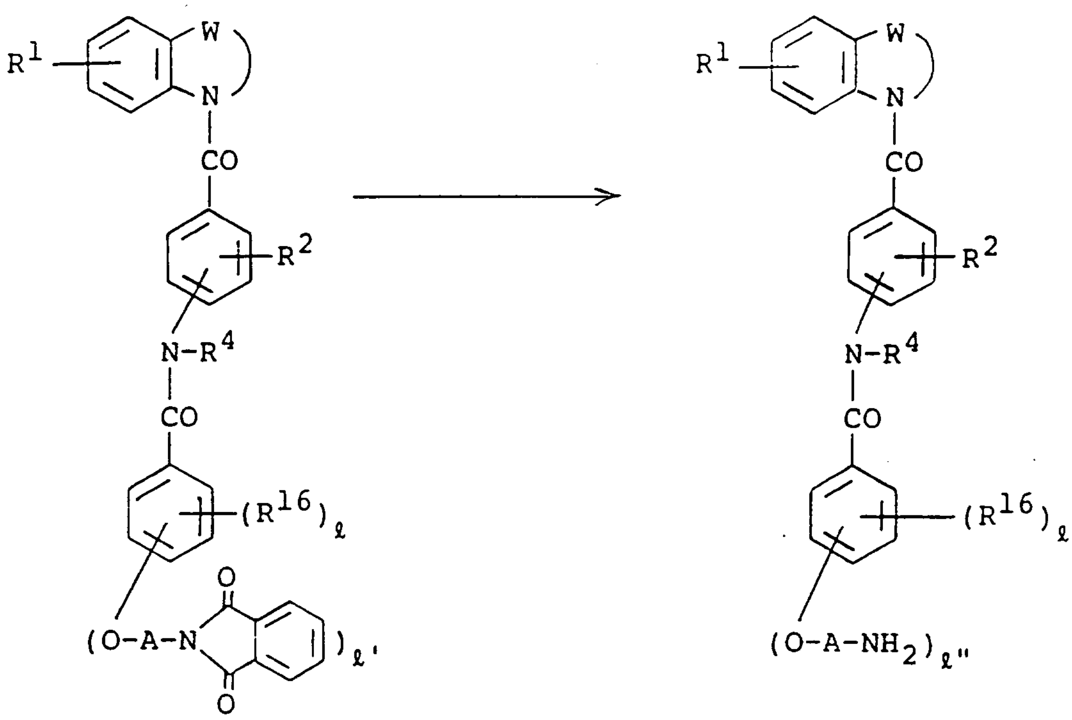

(1j)

(1k)

wherein $R^1$, $R^2$, $R^4$, $R^{16}$, W, $\ell$, $\ell'$, $\ell''$ and A are as defined above.

The reaction of converting the compound (1j) into the compound (1k) can be carried out by reacting the compound (1j) with hydrazine in an appropriate solvent or by hydrolyzing the compound (1j). The solvent used in the reaction with hydrazine includes water and further the same solvent as used in the reaction of the compound (2b) and the compound (4) in the above Reaction Scheme-2. The reaction is usually carried out at a temperature of from room temperature to 120°C, preferably 0°C to 100°C, for 0.5 to 5 hours. Hydrazine is usually used in an amount of at least 1 mole, preferably about 1 to 5 moles, to 1 mole of the compound (1j).

The hydrolysis can be carried out in an appropriate solvent or without solvent in the presence of an acid or a basic compound. The solvent includes, for example, water, lower alcohols (e.g. methanol, ethanol, isopropanol), ketones (e.g. acetone, methyl ethyl ketone), ethers (e.g. dioxane, tetrahydrofuran, ethylene glycol dimethyl ether), fatty acids (e.g. acetic acid, formic acid), or a mixture of these solvents. The acid includes, for example, mineral acids (e.g. hydrochloric acid, sulfuric acid, hydrobromic acid) and organic acids (e.g. formic acid, acetic acid, aromatic sulfonic acids). The basic compound includes, for example, metal carbonates (e.g. sodium carbonate, potassium carbonate) and metal hydroxides (e.g. sodium hydroxide, potassium hydroxide, calcium hydroxide). The reaction is usually carried out at a temperature

of from room temperature to 200°C, preferably from room temperature to 150°C, for 10 minutes to 25 hours.

[Reaction Scheme-8]

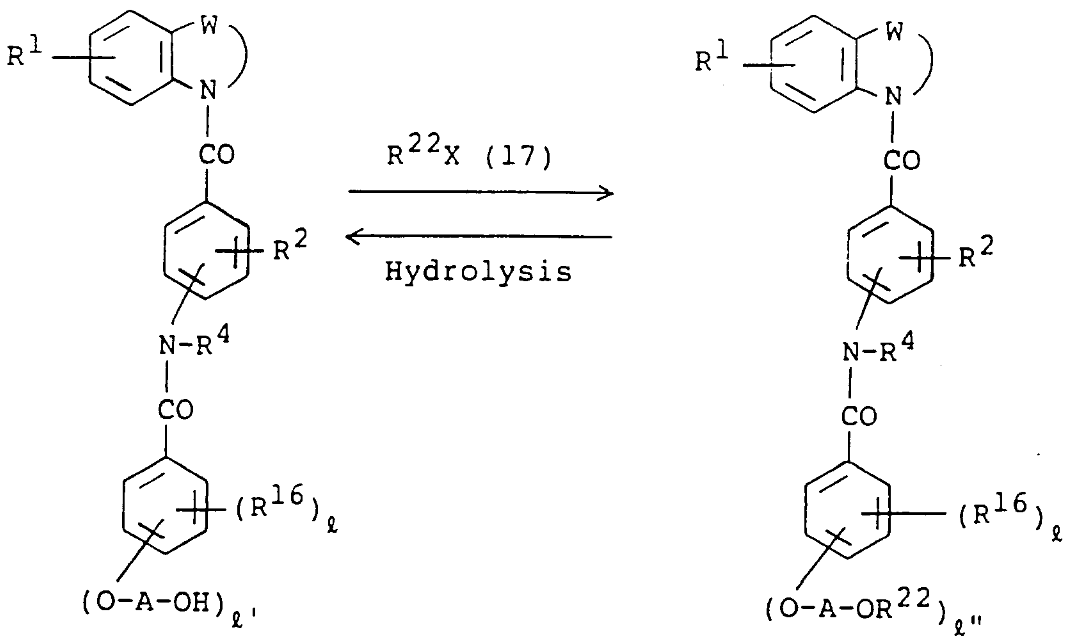


(1ℓ) (1m)

wherein $R^1$, $R^2$, $R^4$, W, $R^{16}$, ℓ, ℓ', ℓ'', X, and A are as defined above, and $R^{22}$ is a lower alkanoyl.

The reaction of the compound (1ℓ) and the compound (17) is carried out under the same conditions as in the reaction of the compound (7) and the compound (8) in the Reaction Scheme-4. In the reaction, an alkali metal halide (e.g. sodium iodide, potassium iodide) may be added to the reaction system.

The reaction of converting the compound (1m) into the compound (1ℓ) can be carried out under the same condition as in the hydrolysis of the compound (1j) in the Reaction Scheme-7.

[Reaction Scheme-9]

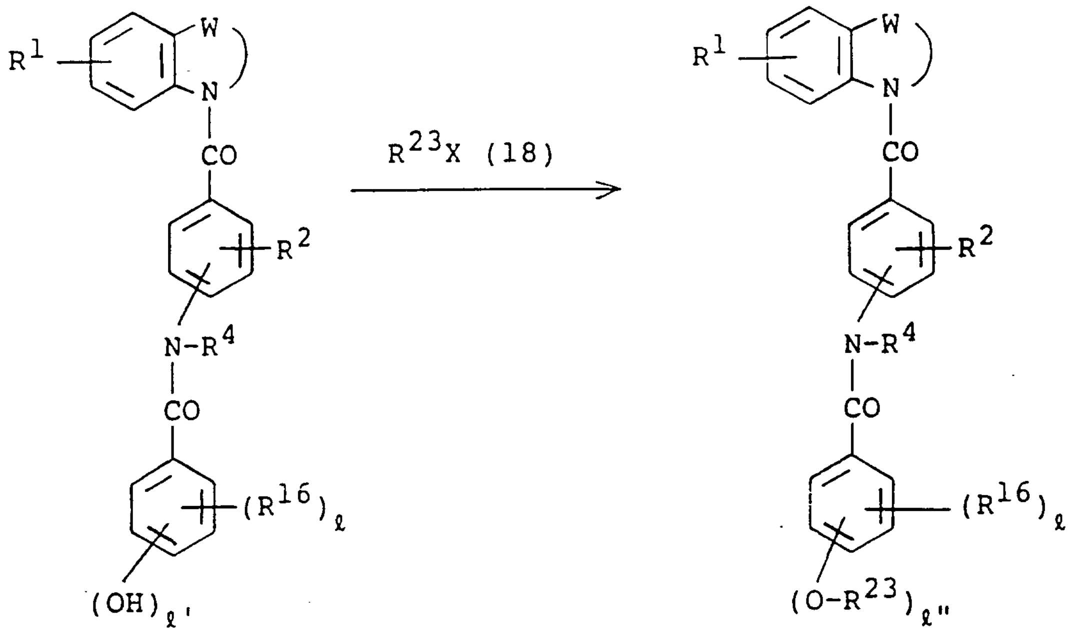


(1n) (1o)

wherein $R^1$, $R^2$, $R^4$, W, $R^{16}$, ℓ, ℓ', ℓ", and X are as defined above, and $R^{23}$ is a lower alkyl, a lower alkanoyloxy-substituted lower alkyl, a halogen-substituted lower alkyl, a carboxy-substituted lower alkyl, a carbamoyl-substituted lower alkyl, a hydroxy-substituted lower alkyl, a lower alkoxycarbonyl-substituted lower alkyl, a phthalimido-substituted lower alkyl, an aminocarbonyl-lower alkyl having optionally a lower alkyl substituent, or a group of the formula:

$$-A-N\begin{matrix} R^6 \\ R^7 \end{matrix}$$

(A, $R^6$ and $R^7$ are as defined above).

The reaction of the compound (1n) and the compound (18) can be carried out under the same conditions as in the reaction or the compound (7) and the compound (8) in the above Reaction Scheme-4. In the reaction, an alkali metal halide (e.g. sodium iodide, potassium iodide) may be added to the reaction system.

[Reaction Scheme-10]

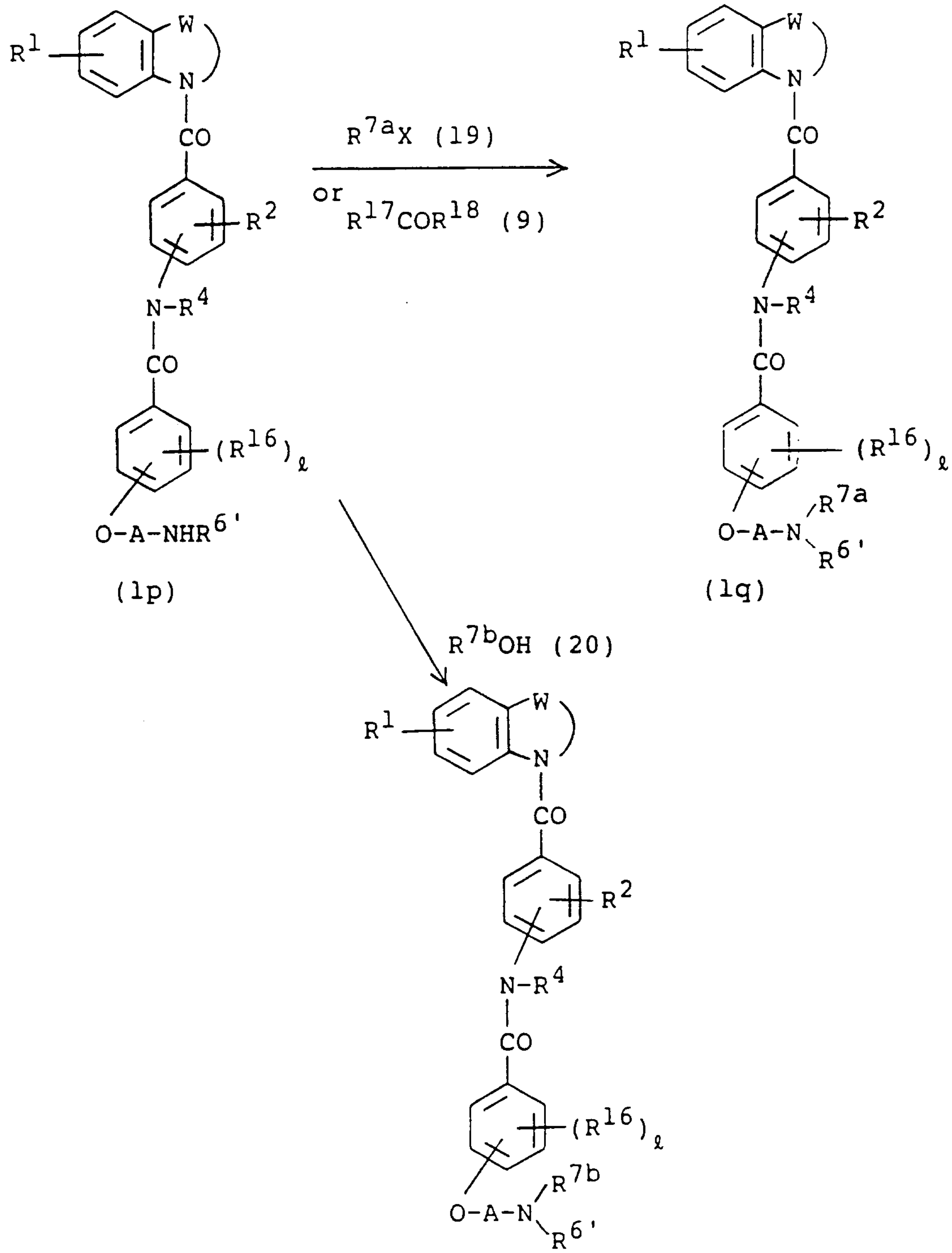


(1p)

(1q)

(1r)

wherein $R^1$, $R^2$, $R^4$, W, $R^{16}$, $R^{17}$, $R^{18}$, $\ell$, X, and A are as defined above, and $R^{6'}$ is hydrogen atom, a lower alkyl having optionally a hydroxy substituent, a lower alkanoyl, or benzoyl, $R^{7a}$ is a lower alkyl having optionally a hydroxy substituent, and $R^{7b}$ is a lower alkanoyl or benzoyl.

The reaction of the compound (1p) and the compound (19) or the compound (9) can be carried out under the same conditions as in the reaction of the compound (7) and the compound (8) or the compound (9) in the above Reaction Scheme-4.

The reaction of, the compound (1p) and the compound (20) can be carried out under the same conditions as in the reaction of the compound (2) and the compound (3) in the Reaction Scheme-1.

Besides, the compound (1r) can also be obtained by reacting the compound (1p) with a compound of the formula: $(R^{7b})_2O$ ($R^{7b}$ is as defined above). The reaction can be carried out in an appropriate solvent or without solvent in the presence or absence, peferably presence, of a basic compound. The solvent includes, for example, the above-mentioned aromatic hydrocarbons, lower alcohols (e.g. methanol, ethanol, propanol), dimethylformamide, dimethylsulfoxide, and further halogenated hydrocarbons (e.g. chloroform, methylene chloride), acetone, pyridine). The basic compound includes, for example, tertiary amines (e.g. triethylamine, pyridine), sodium hydroxide, potassium hydroxide and sodium hydride. The above reaction can also be carried out in a solvent such as acetic acid or benzoic acid in the presence of a mineral acid (e.g. sulfuric acid). The acid anhydride is usually used in an equimolar amount or more, preferably 1 to 10 moles, to 1 mole of the starting compound, and the reaction is usually carried out at a temperature of 0°C to 200°C, preferably from 0°C to 150°C, for 0.5 to 15 hours.

[Reaction Scheme-11]

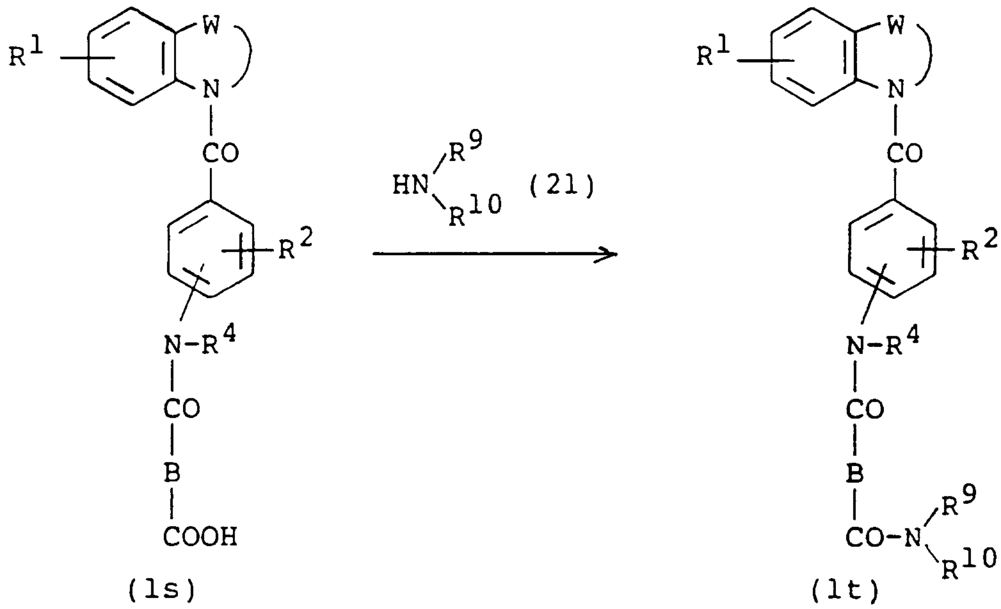


(1s) (1t)

wherein $R^1$, $R^2$, $R^4$, $R^9$, $R^{10}$, W, and B are as defined above.

The reaction of the compound (1s) and the compound (21) can be carried out under the same conditions as in the reaction of the compound (2) and the compound (3) in the above Reaction Scheme-1.

[Reaction Scheme-12]

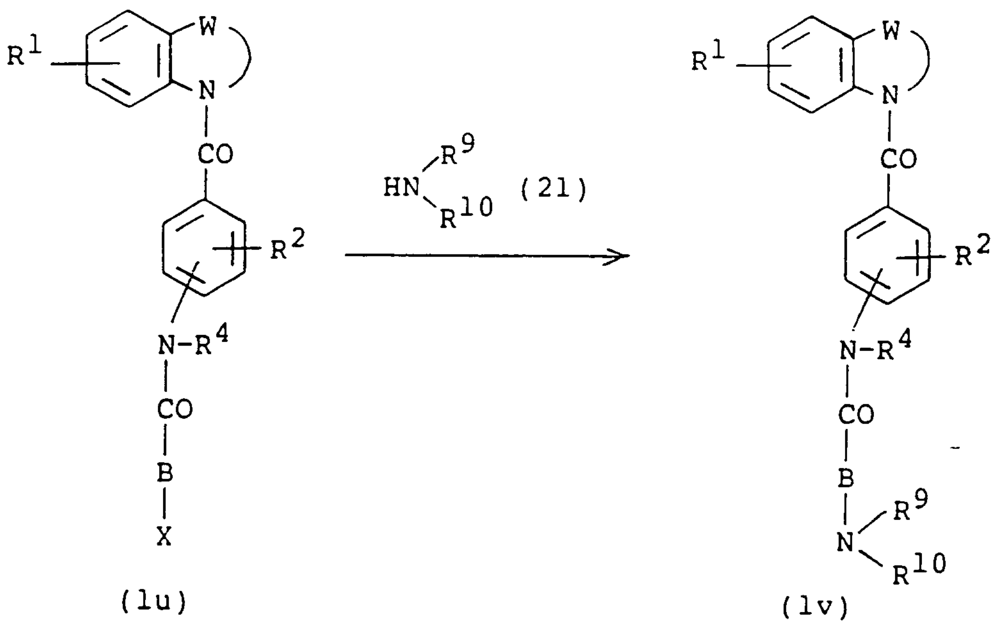


(1u)

(1v)

wherein $R^1$, $R^2$, $R^4$, W, $R^9$, $R^{10}$, X, and B are as defined above.

The reaction of the compound (1u) and the compound (21) can be carried out under the same conditions as in the reaction of the compound (7) and the compound (8) in the above Reaction Scheme-4. In the reaction, an alkali metal

halide (e.g. sodium iodide, potassium iodide) may be added to the reaction system.

[Reaction Scheme-13]

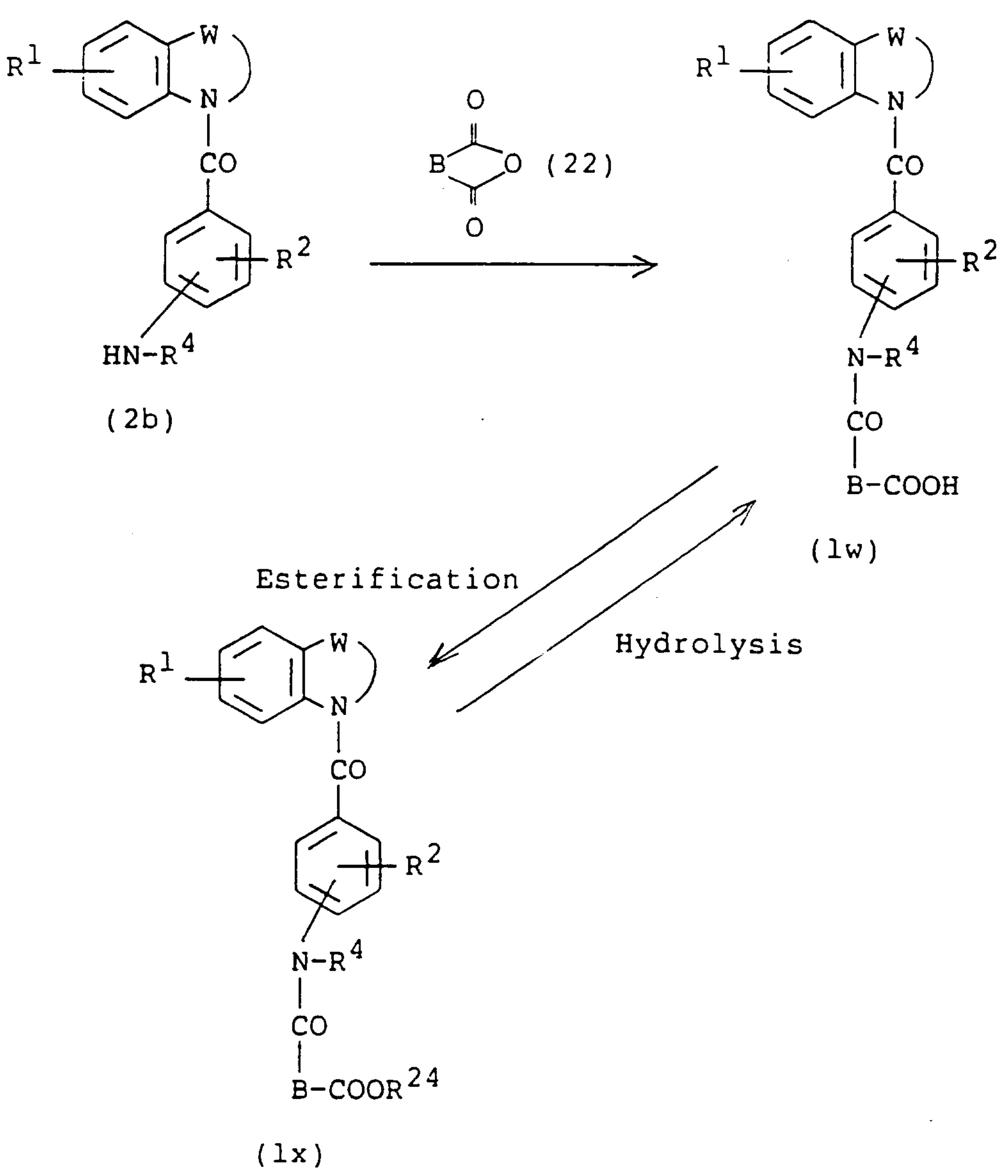


wherein $R^1$, $R^2$, $R^4$, W, and B are as defined above, and $R^{24}$ is a lower alkyl.

The reaction of the compound (2b) and the compound (22) can be carried out in an appropriate inert solvent. The inert solvent includes, for example, aromatic hydrocarbons (e.g. benzene, toluene, xylene), ethers (e.g. tetrahydrofuran, dioxane, diethylene glycol dimethyl ether), lower alcohols (e.g. methanol, ethanol, isopropanol, butanol), halogenated hydrocarbons (e.g. dichloromethane, chloroform, carbon tetrachloride), acetic acid, ethyl acetate, acetonitrile, dimethylsulfoxide, dimethylformamide and hexamethylphosphoric triamide. The amount of the compound (2b) and the compound (22) is not critical, but the compound (22) is usually used in an amount of at least one mole, preferably 1 to 2 moles, to 1 mole of the compound (2b). The reaction is usually carried out at a temperature of from 0°C to 150°C, preferably from 0°C to 100°C, for 30 minutes to 10 hours.

The esterification of the compound (1w) is usually carried out by reacting the starting compound with an alcohol (e.g. methanol, ethanol, isopropanol) in the presence of a mineral acid (e.g. hydrochloric acid, sulfuric acid) and a halogenating agent (e.g. thionyl chloride, phosphorus oxychloride, phosphorus pentachloride, phosphorus trichloride) at a temperature of 0°c to 150°C, preferably 50°C to 100°C, for 1 to 10 hours.

The hydrolysis of the compound (1x) can be carried out under the same conditions as in the hydrolysis of the compound (1j) in the Reaction Scheme-7.

[Reac'ion Scheme-14]

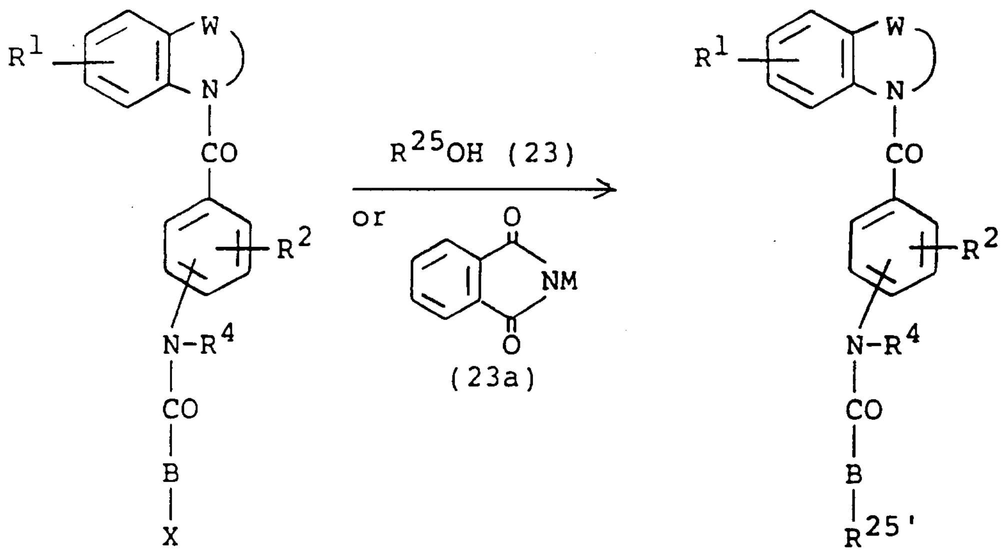


(1u) (1y)

wherein $R^1$, $R^2$, $R^4$, W, B, M, and X are as defined above, and $R^{25}$ is a phenyl which has optionally 1 to 3 substituents selected from a lower alkyl, a lower alkoxy and an amino having optionally a lower alkanoyl substituent, or naphthyl, and $R^{25'}$ is a phenoxy which has optionally 1 to 3 substituents selected from a lower alkyl, a lower alkoxy and an amino having optionally a lower alkanoyl substituent, naphthyloxy or phthalimido.

The reaction of the compound (1u) and the compound (23) or (23a) can be carried out under the same conditions as in the reaction of the compound (7) and the compound (8) in the above Reaction Scheme-4.

The compound (1y) wherein $R^{25'}$ is phthalimido can be converted into the compound (1y) wherein $R^{25'}$ is amino under the same conditions as in the reaction of converting the compound (1j) into the compound (1k) in the above

Reaction Scheme-7.

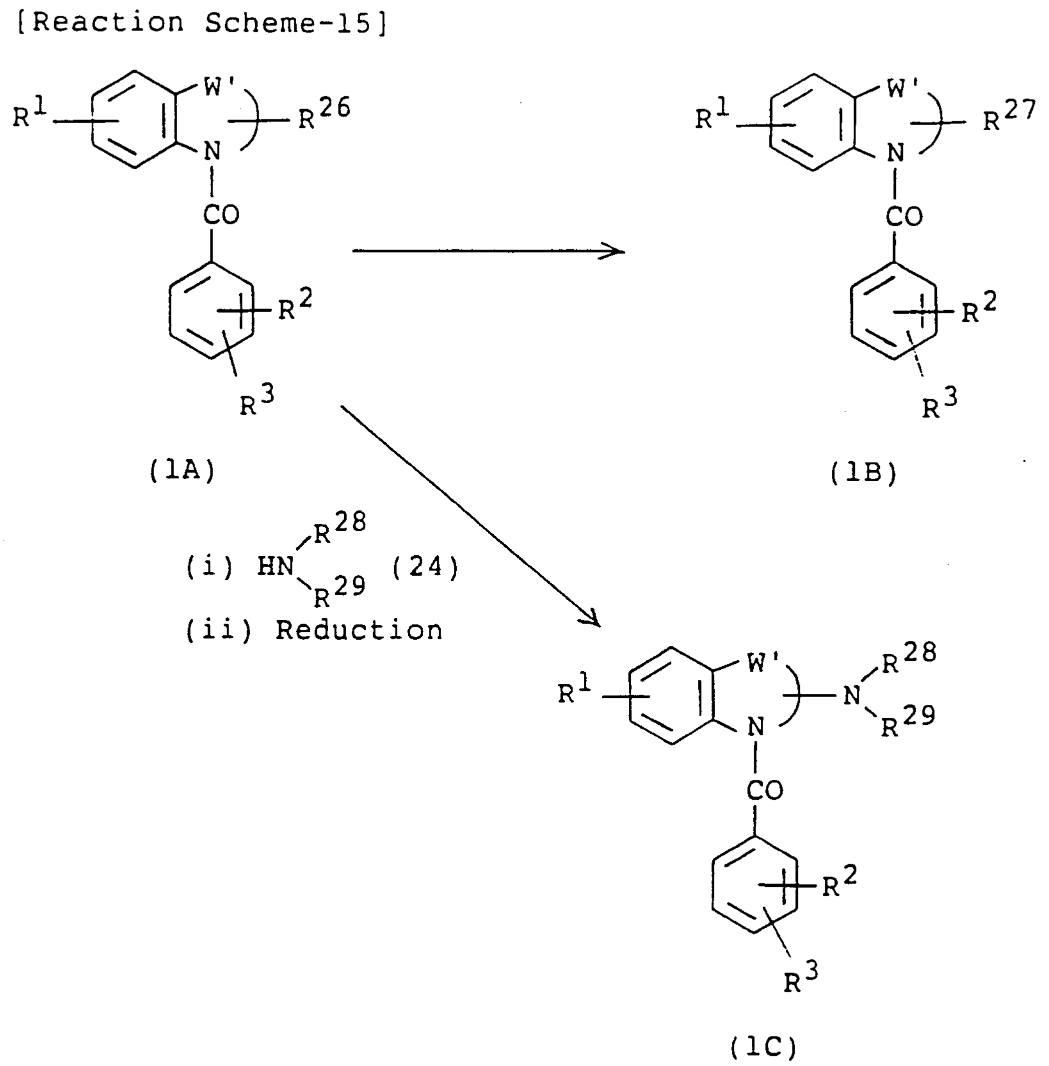


wherein $R^1$, $R^2$ and $R^3$ are as defined above, and $R^{26}$ is oxo, $R^{27}$ is hydroxy, and W' is the same as W, provided that the substituents on the group $-(CH_2)_p-$ or $-CH=CH-(CH_2)_q-$ are 0 to 2, and $R^{28}$ and $R^{29}$ are the same or different and are each hydrogen atom, a lower alkenyl, a cycloalkyl, an oxiranyl-substituted lower alkyl, a lower alkyl having 1 to 2 substituents selected from a lower alkoxy, hydroxy and an amino having optionally a lower alkyl substituent, a phenyl-lower alkyl, a pyridyl-lower alkyl, a cyano-substituted lower alkyl, a lower alkoxycarbonyl-substituted lower alkyl, a carbamoyl-substituted lower alkyl, a carboxy-substituted lower alkyl, a tetrahydropyranyloxy-substituted lower alkyl, a lower alkanoyloxy-substituted lower alkyl, a piperidinyl which has optionally a phenyl-lower alkyl substituent, an aminocarbonyl-lower alkyl having optionally a lower alkyl substituent, or a lower alkyl, or $R^{28}$ and $R^{29}$ may bind together with the nitrogen atom to which they bond to form a 5- or 6-membered saturated heterocyclic group with or without being intervened with nitrogen or oxygen atom, which heterocyclic ring may optionally have a substituent selected from a lower alkyl, a phenyl-lower alkyl, or a lower alkanoyl.

The conversion of the compound (1A) into the compound (1B) is carried out by reduction thereof. The reducing reaction is preferably carried out by using a hydrogenating reducing agent (e.g. lithium aluminum hydride, sodium boro hydride, diborane). The reducing agent is usually used in an amount of at least one mole, preferably 1 to 15 moles, to 1 mole of the starting compound. The reducing reaction is usually carried out in an appropriate solvent, for example, water, alcohols (e.g. methanol, ethanol, isopropanol), ethers (e.g. tetrahydrofuran, diethyl ether, diisopropyl ether, digyme), or a mixture of these solvents, at a temperature of from -60°C to 150°C, peferably -30°C to 100°C, for 10 minutes to 15 hours. When lithium aluminum hydride or diborane is used as the reducing agent, it is preferable to use an anhydrous solvent such as tetrahydrofuran, diethyl ether, diisopropyl ether and diglyme.

The reaction of converting the compound (1A) into the compound (1C) is usually carried out in an appropriate solvent or without solvent in the presence or absence of a dehydrating agent. The solvent includes, for example, lower alcohols (e.g. methanol, ethanol, isopropanol), aromatic hydrocarbons (e.g. benzene, toluene, xylene), halogenated hydrocarbons (e.g. dichloromethane, dichloroethane, chloroform, carbon tetrachloride), aprotic polar solents (e.g. dimethylformamide, dimethylacetamide, N-methylpyrrolidone), or a mixture of these solvents. The dehydrating agent includes, for example, conventional drying agent used for dehydrating solvents (e.g. molecular sieves), mineral acids (e.g. hydrochloric acid, sulfuric acid, borone trifluoride), and organic acids (e.g. p-toluenesulfonic acid, etc.). The reaction is usually carried out at a temperature of from room temperature to 250°C, preferably from 50°C to 200°C, for 1 to 48 hours. The amount of the compound (24) is not critical, but it is usually used at least in an equivalent amount, preferably equimolar to largely excess to the amount of the compound (1A). The dehydrating agent is preferably used in a largely excess amount in case of the drying agent and in a catalytic amount in case of the acid.

The subsequent reducing reaction can be carried out by various methods, for example by catalytically hydrogenating the compound in an appropriate solvent in the presence of a catalyst. The solvent includes, for example, water, acetic acid, alcohols (e.g. methanol, ethanol, isopropanol), hydrocarbons (e.g. hexane, cyclohexane), ethers (e.g. diethylene glycol dimethyl ether, dioxane, tetrahydrofuran, diethyl ether), esters (e.g. ethyl acetate, methyl acetate), aprotic polar solvents (e.g. dimethylformamide), or a mixture of these solvents. The catalyst includes, for example, palladium, palladium black, palladium-carbon, platinum, platinum oxide, copper chromite and Raney nickel. The catalyst is usually used in an amount of 0.02 to 1 part by weight to 1 part by weight of the starting compound. The reaction is usually carried out at a temperature of from -20°C to 100°C, peferably 0°C to 70°C, under a hydrogen atmospheric pressure of 101.3 kPa to 1.013 MPa (1 to 10 atm) for 0.5 to 20 hours.

Although the reducting reaction can be carried out under the above conditions, it is preferably carried out by using a hydrogenating reducing agent. The hydrogenating reducing agent includes, for example, lithium aluminum hydride, sodium borohydride, diborane, etc., and it is usually used in an amount of at least one mole, preferably 1 to 10 moles, to 1 mole of the compound (1A). The reaction is usually carried out in an appropriate solvent, such as water, lower alcohols (e.g. methanol, ethanol, isopropanol), ethers (e.g. tetrahydrofuran, diethyl ether, diglyme), dimethylformamide, or a mixture of these solvents, at a temperature of -60°C to 50°C, preferably -30°C to room temperature, for 10 minutes to 5 hours. When lithium aluminum hydride or diborane is used as the reducing agent, it is preferable to use an anhydrous solvent such as diethyl ether, tetrahydrofuran, diglyme.

The compound (1C) wherein at least one of $R^{28}$ and $R^{29}$ is hydrogen atom can be converted into the compound (1C) wherein at least one of $R^{28}$ and $R^{29}$ is a lower alkyl by reacting the compound (1C) with the compound (8) or the compound (9) under the same conditions as in the reaction of the compound (7) and the compound (8) or (9) in the

above Reaction Scheme-4.

[Reaction Scheme-16]

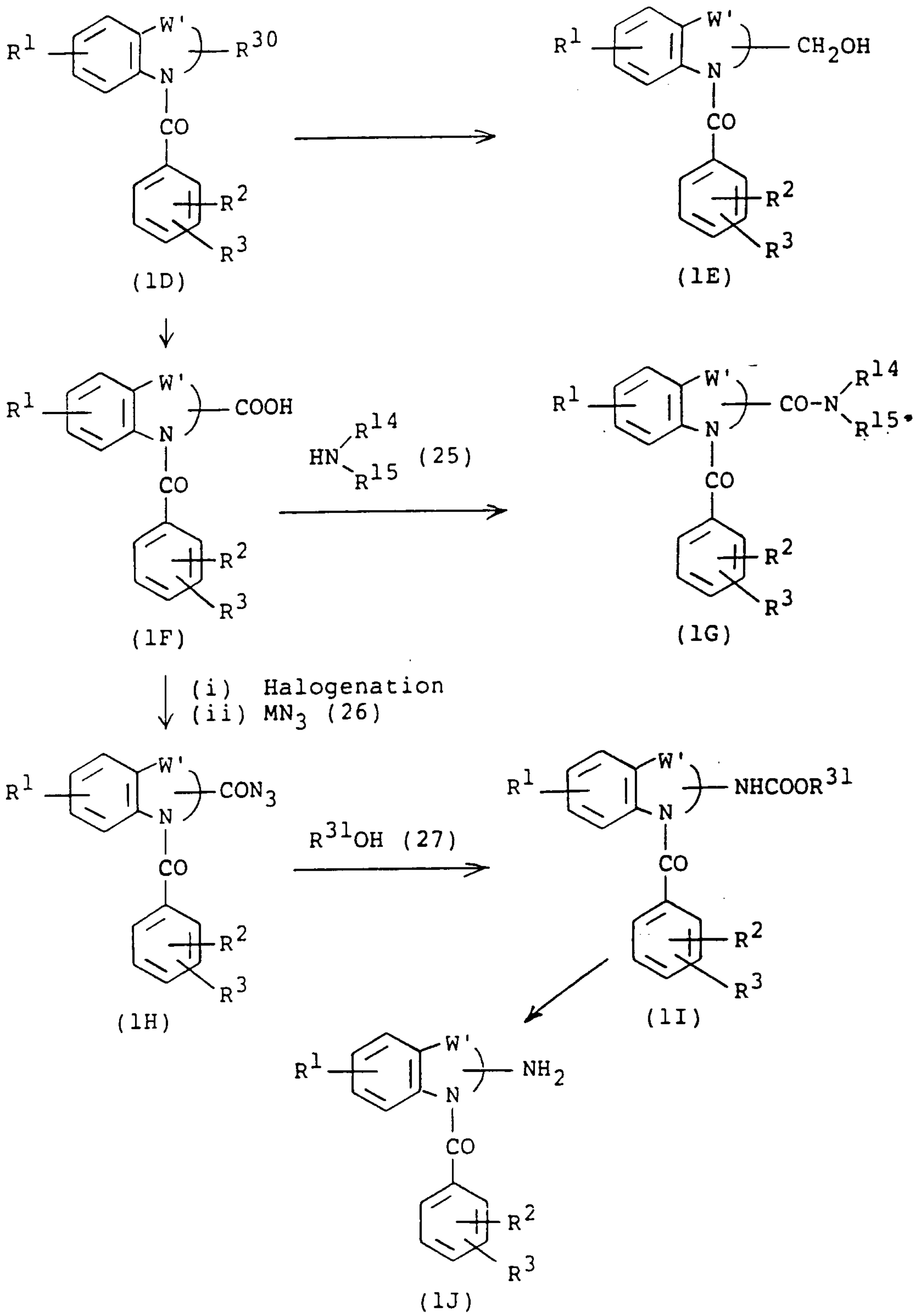


wherein $R^1$, $R^2$, $R^3$, $R^{14}$, $R^{15}$, W', and M are as defined above, and $R^{31}$ is a phenyl-lower alkyl, and $R^{30}$ is a lower alkoxycarbonyl.

The reaction of converting the compound (1D) into the compound (1E) can be carried out under the same conditions as in the reaction of converting the compound (1A) into the compound (1B) in the above Reaction Scheme-15.

The reaction of converting the compound (1D) into the compound (1F) can be carried out under the same conditions as in the hydrolysis reaction of the compound (1j) in the above Reaction Scheme-7.

The reaction of the compound (1F) and the compound (25) can be carried out under the same conditions as in the reaction of the compound (2) and the compound (3) in the above Reaction Scheme-1.

The halogenation of the compound (1F) can be carried out under a conventional condition for halogenation of a carboxylic acid. The reaction of the thus-obtained carboxylic acid halide of the compound (1F) with the compound (26) is carried out in an appropriate solvent in the presence or absence of a basic compound. The solvent includes, for example, halogenated hydrocarbons (e.g. methylene chloride, chloroform), aromatic hydrocarbons (e.g. benzene, toluene, xylene), ethers (e.g. diethyl ether, tetrahydrofuran, dimethoxyethane), esters (e.g. methyl acetate, ethyl acetate), aprotic polar solvents (e.g. N,N-dimethylformamide, dimethylsulfoxide, hexamethylphosphoric triamide), alcohols (e.g. methanol, ethanol, propanol, butanol, 3-methoxy-1-butanol, ethyl cellosolve, methyl cellosolve), pyridine, acetone, acetonitrile, water, or a mixture of these solvents. The basic compound includes, for example, organic bases such as triethylamine, trimethylamine, pyridine, dimethylaniline, N-methylmorpholine, DBN, DBU, DABCO, inorganic bases such as potassium carbonate, sodium carbonate, potassium hydroxide, sodium hydroxide, potassium hydride, sodium hydride, silver carbonate and alcoholates (e.g. sodium methylate, sodium ethylate). The compound (26) is usually used in an amount of at least 1 mole, preferably 1 to 1.5 mole, to 1 mole of the carboxylic acid halide of the compound (1F). The reaction is usually carried out at a temperature of from -30°C to 180°C, preferably from 0°C to 150°C, for 5 minutes to 30 hours.

The reaction of the compound (1H) and the compound (27) is carried out in an appropriate solvent or without solvent at a temperature of from 0°C to 200°C, preferably from room temperature to 150°C. The solvent includes the same solvents as used in the above reaction of the carboxylic acid halide of the compound (1F) and the compound (26). The compound (27) is preferably used in an amount largely excess to the the compound (1H). The reaction is usually completed in a reaction time of 1 to 5 hours.

The reaction of converting the compound (1I) into the compound (1J) can be carried out by reducing the compound. The reducing reaction is usually carried out by catalytically hydrogenating the compound in an appropriate solvent in the presence of a catalyst. The solvent includes, for example, water, acetic acid, alcohols (e.g. methanol, ethanol, isopropanol), hydrocarbons (e.g. hexane, cyclohexane), ethers (e.g. dioxane, tetrahydrofuran, diethyl ether, diethylene glycol dimethyl ether), esters (e.g. ethyl acetate, methyl acetate), aprotic polar solvents (e.g. N,N-dimethylformamide), acetic acid, or a mixture of these solvents. The catalyst includes, for example, palladium, palladium black, palladium-carbon, platinum, platinum oxide, copper chromite, and Raney nickel. The catalyst is usually used in an amount of 0.02 to 1 part by weight to 1 part by weight of the starting compound. The reaction is usually carried out at a temperature of from -20°C to 100°C, peferably 0°C to 80°C, under a hydrogen atmospheric pressure of 101.3 kPa to 1.013 MPa (1 to

10 atm) for 0.5 to 20 hours.

[Reaction Scheme-17]

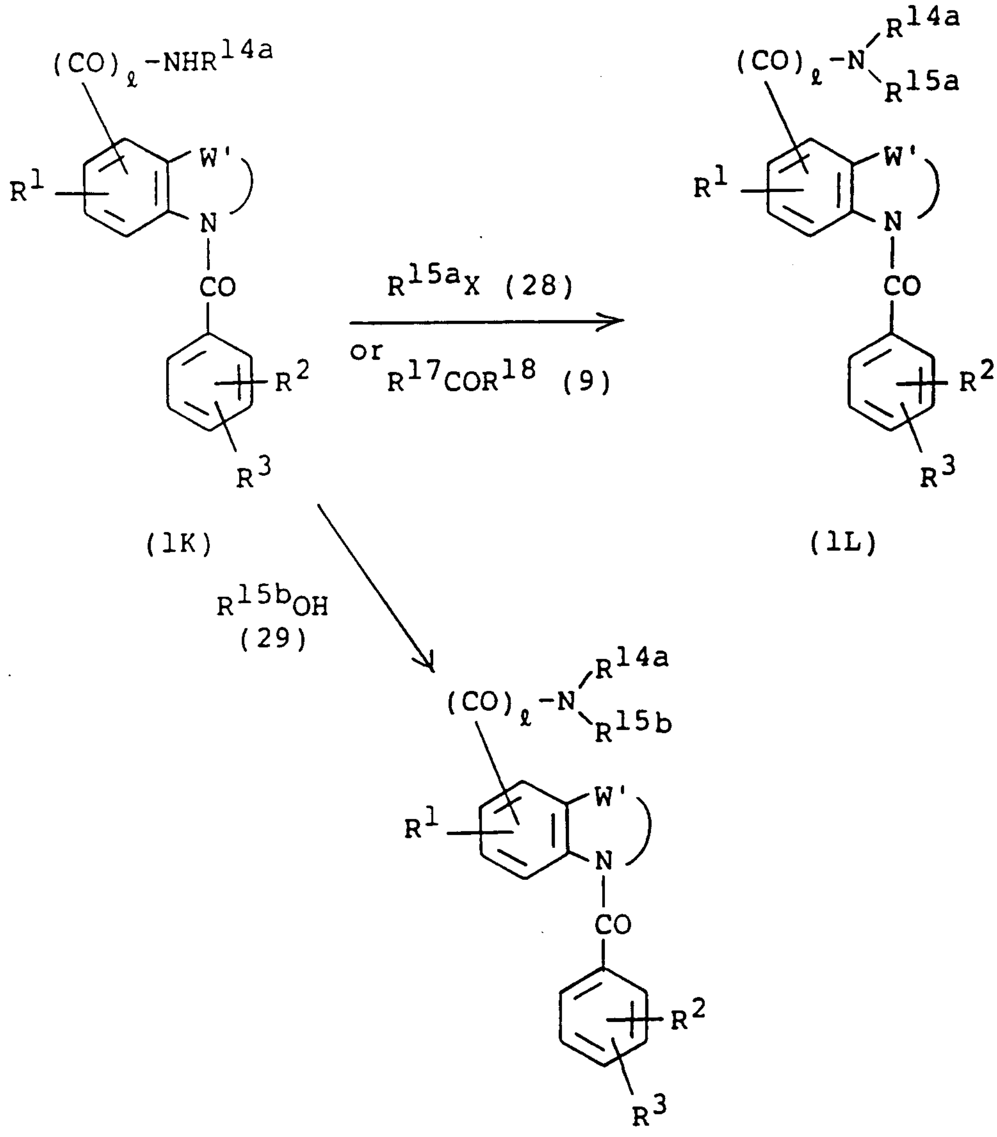


(1K) (1L)

(1M)

wherein $R^1$, $R^2$, $R^3$, W', $\ell$, $R^{17}$, $R^{18}$, and X are as defined above, and $R^{14a}$ is hydrogen atom, a lower alkyl, a lower alkanoyl, a lower alkenyl, a cycloalkyl, an oxiranyl-substituted lower alkyl, a lower alkyl having 1 to 2 substituents selected from a lower alkoxy, hydroxy and an amino having optionally a lower alkyl substituent, a phenyl-lower alkyl, a pyridyl-lower alkyl, a lower alkylsulfonyl, benzoyl, a lower alkoxycarbonyl, anilinocarbonyl, an aminocarbonyl having optionally a lower alkyl substituent, a cyano-substituted lower alkyl, a lower alkoxycarbonyl-substituted lower alkyl, a carbamoyl-substituted lower alkyl, a carboxy-substituted lower alkyl, a tetrahydropyranyloxy-substituted lower alkyl, a lower alkanoyloxy-substituted lower alkyl, a piperidinyl having optionally a phenyl-lower alkyl substituent, a halogen-substituted lower alkanoyl, an imiazolyl-substituted lower alkanoyl, an amino-lower alkanoyl having optionally a substituent selected from a lower alkyl and a lower alkoxycarbonyl, an aminocarbonyl-lower alkyl having optionally a lower alkyl substituent, or a phenyl-lower alkoxycarbonyl, $R^{15a}$ is a lower alkyl, a cycloalkyl, an oxiranyl-substituted lower alkyl, a lower alkyl having 1 to 2 substituents selected from a lower alkoxy, hydroxy and an amino having optionally a lower alkyl substituent, a phenyl-lower alkyl, a pyridyl-lower alkyl, a lower alkylsulfonyl, a cyano-substituted lower alkyl, a lower alkoxycarbonyl-substituted lower alkyl, a carbamoyl-substituted lower alkyl, a carboxy-substituted lower alkyl, a tetrahydropyranyloxy-substituted lower alkyl, a lower alkanoyloxy-substituted lower alkyl, a piperidinyl having optionally a phenyl-lower alkyl

substituent, an aminocarbonyl-lower alkyl having optionally a lower alkyl substituent, or a lower alkenyl, and $R^{15b}$ is a lower alkanoyl, a phenyl-lower alkoxycarbonyl, benzoyl, a lower alkoxycarbonyl, a halogen-substituted lower alkanoyl, an imidazolyl-substituted lower alkanoyl, or an amino-lower alkanoyl having optionally a substituent selected from a lower alkyl and a lower alkoxycarbonyl.

The reaction of the compound (1K) and the compound (28) or the compound (9) can be carried out under the same conditions as in the reaction of the compound (7) and the compound (8) or the compound (9) in the above Reaction Scheme-4.

The reaction of the compound (1K) and the compound (29) can be carried out under the same conditions as in the reaction of the compound (2) and the compound (3) in the above Reaction Scheme-1. The compound (1M) can also be obtained by reacting the compound (1K) with a compound of the formula $(R^{15b})_2O$ (wherein $R^{15b}$ is as defined above). The reaction can be carried out under the same conditions as in the reaction of the compound (1p) and the compound of the formula: $(R^{7b})_2O$ as described hereinbefore.

The compound (1M) wherein $R^{15b}$ is formyl can also be prepared by reacting the compound (1K) with a formate of the formula: $HCOOR^{82}$ ($R^{82}$ is a lower alkyl). The reaction is usually carried out in the solvent as used in the reaction of the compound (7) and the compound (8) in the above Reaction Scheme-4 or without solvent, at a temperature of 0°C to 200°C, preferably 0°C to 170°C, for 30 minutes to 30 hours. The formate is preferably used in a largely excess amount

to the compound (1K).

[Reaction Scheme-18]

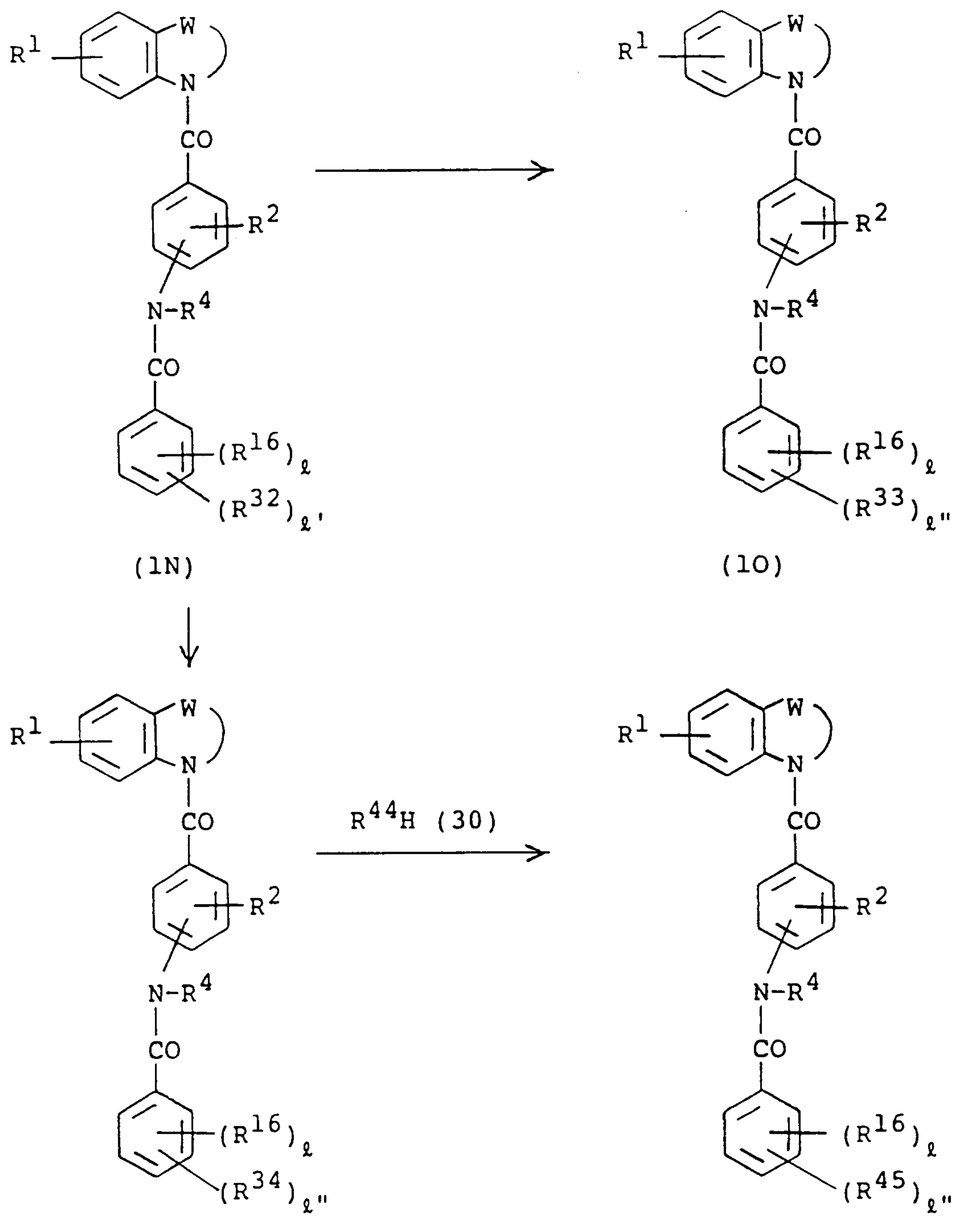


(1N) (1O)

(1P) (1P')

wherein $R^1$, $R^2$, $R^4$, $R^{16}$, W, $\ell$, $\ell'$ and $\ell''$ are as defined above, and $R^{32}$ is a lower alkoxycarbonyl-substituted lower alkoxy, $R^{33}$ is a carbamoyl-substituted lower alkoxy, $R^{34}$ is a carboxy-substituted lower alkoxy, $R^{44}$ is an amino having optionally a lower alkyl substituent, and $R^{45}$ is an aminocarbonyl-lower alkoxy having optionally a lower alkyl substituent.

The conversion of the compound (1N) into the compound (1O) can be carried out by reacting the compound with aqueous ammonia in an appropriate solvent in an autoclave. The solvent includes the same solvents as used in the reaction of the carboxylic acid halide and the amine (2) in the above Reaction Scheme-1. The aqueous ammonia is used in a largely excess amount to the compound (1N). The reaction proceeds advantageously by adding an ammonium

halide (e.g. ammonium chloride) to the reaction system. The reaction is usually carried out at a temperature of from room temperature to 200°C, preferably from room temperature to 150°C, for 1 to 10 hours.

The reaction of converting the compound (1N) into the compound (1P) can be carried out under the same conditions as in the hydrolysis of the compound (1j) in the above Reaction Scheme-7.

The reaction of the compound (1P) and the compound (30) can be carried out under the same conditions as in the reaction of the compound (2) and the compound (3) in the above Reaction Scheme-1.

[Reaction Scheme-19]

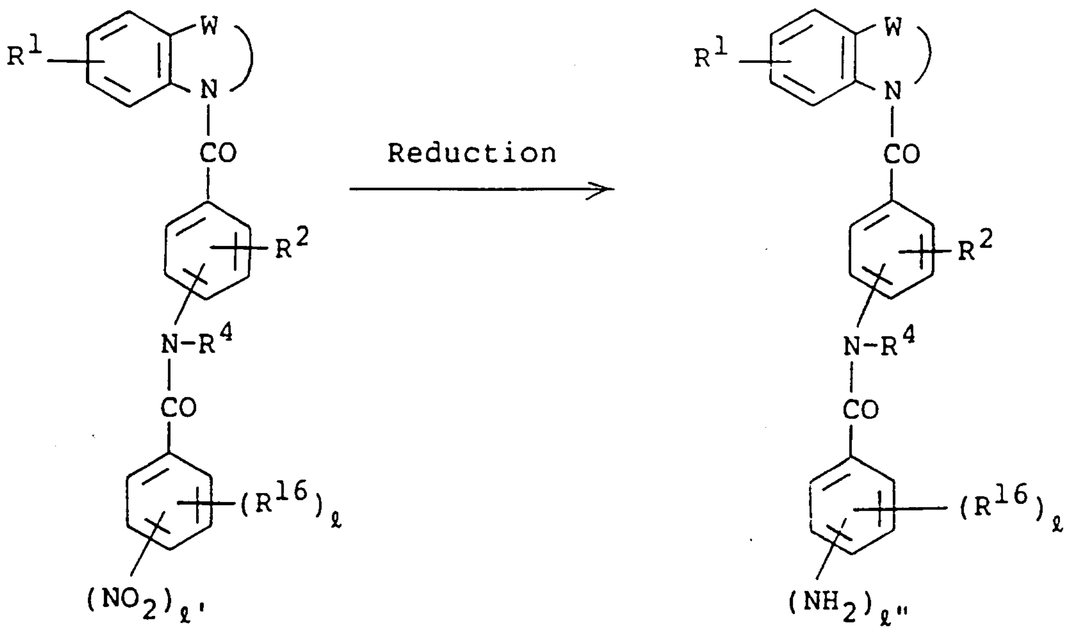


(1Q)

(1R)

wherein $R^1$, $R^2$, $R^4$, $R^{16}$, W, $\ell$, $\ell'$ and $\ell''$ are as defined above.

The reducing reaction in the above reaction scheme is usually carried out, for example, (i) with a reducing catalyst in an appropriate solvent or (ii) with a reducing agent such as a mixture of a metal or metal salt with an acid, or a mixture of a metal or metal salt with an alkali metal hydroxide, a sulfide or an ammonium salt in an appropriate inert solvent.

In case of using a reducing catalyst, the solvent includes, for example, water, acetic acid, alcohols (e.g. methanol, ethanol, isopropanol), hydrocarbons (e.g. hexane, cyclohexane), ethers (e.g. dioxane, tetrahydrofuran, diethyl ether, diethylene glycol dimethyl ether), esters (e.g. ethyl acetate, methyl acetate), aprotic polar solvents (e.g. N,N-dimethylformamide), or a mixture of these solvents. The catalyst includes, for example, palladium, palladium black, palladium-carbon, platinum, platinum oxide, copper chromite and Raney nickel. The catalyst is usually used in an amount of 0.02 to 1 part by weight to 1 part by weight of the starting compound. The reaction is usually carried out at a temperature of from -20°C to 150°C, peferably 0°C to 100°C, under a hydrogen pressure of 101.3 kPa to 1.013 MPa (1 to 10 atm.) for 0.5 to 10 hours. In the reaction, an acid such as hydrochloric acid may optionally added to the reaction system.

In case of the above method (ii), the reducting agent includes a mixture of iron, zinc, tin or stannous chloride and a mineral acid (e.g. hydrochloric acid, sulfuric acid), or a mixture of iron, ferrous sulfate, zinc or tin and an alkali metal hydroxide (e.g. sodium hydroxide), a sulfide (e.g. ammonium sulfide), aqueous ammonia, or an ammonium salt (e.g. ammonium chloride). The inert solvent includes, for example water, acetic acid, methanol, ethanol and dioxane. The reducing reaction conditions are determined depending on the kinds of the reducting agent, but in case of using a reducing agent comprising stannous chloride and hydrochloric acid, for example, it is preferably carried out at a temperature of 0°C to room temperature for 0.5 to 10 hours. The reducing agent is usually used in an amount of at least

one mole, preferably 1 to 5 moles, to 1 mole of the starting compound.

[Reaction Scheme-20]

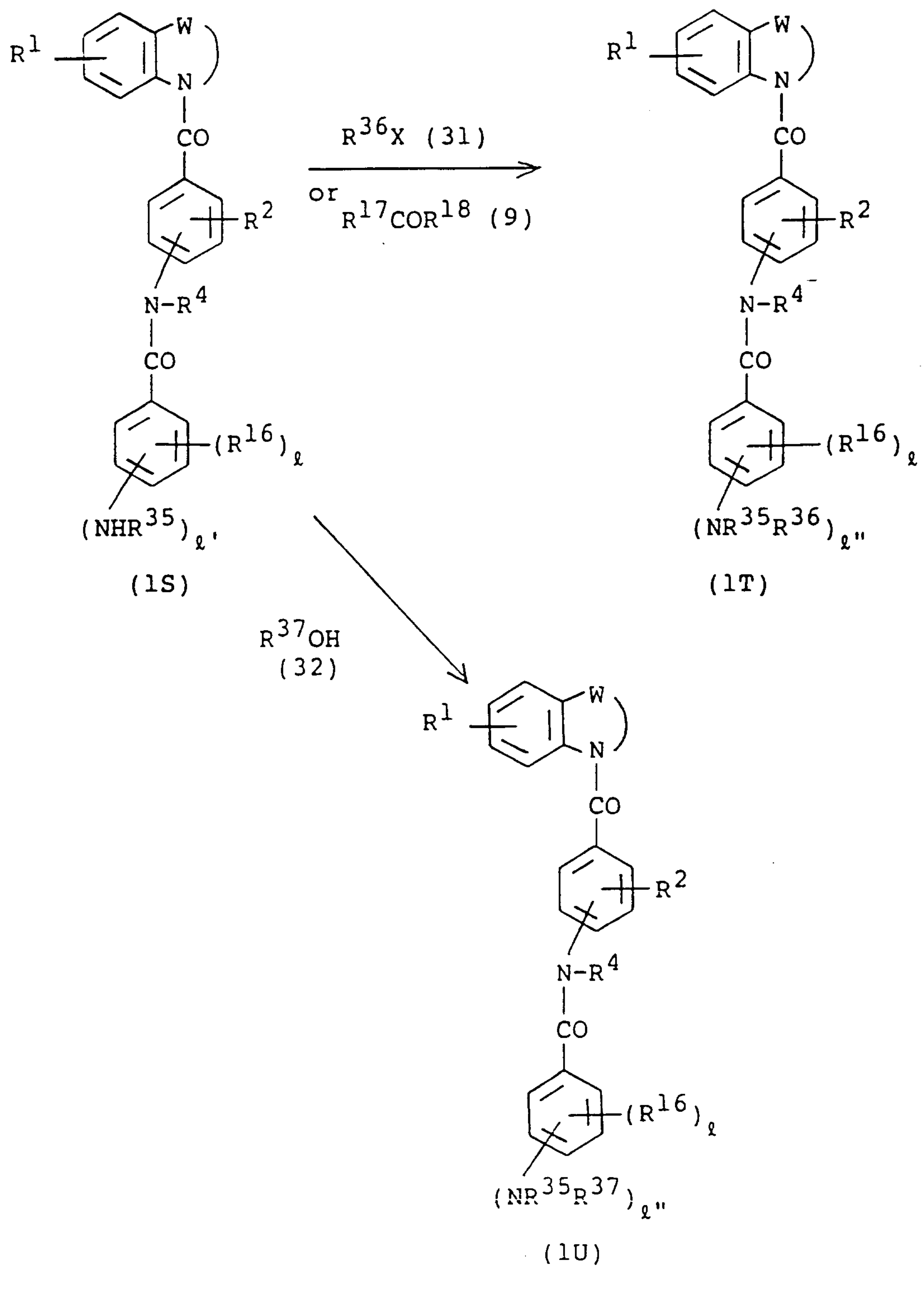


wherein $R^1$, $R^2$, $R^4$, $R^{16}$, $R^{17}$, $R^{18}$, $\ell$, $\ell'$, $\ell''$ and W are as defined above, and $R^{36}$ is a lower alkyl, $R^{37}$ is a lower alkanoyl, and $R^{35}$ is hydrogen atom, a lower alkyl or a lower alkanoyl.

The reaction of the compound (1S) and tee compound (31) or the compound (9) can be carried out under the same conditions as in the reaction of the compound (7) and the compound (8) or the compound (9) in the above Reaction Scheme-4.

The reaction of the compound (1S) and the compound (32) can be carried out under the same conditions as in the reaction of the compound (2) and the compound (3) in the above Reaction Scheme-1. Besides, the compound (1U) can also be obtained by reacting the compound (1S) with a compound of the formula: $(R^{37})_2O$ ($R^{37}$ is as defined above). The reaction is carried out under the same conditions as in the above reaction of the compound (1p) and a compound of the formula: $(R^{7b})_2O$.

The compound (1) wherein $R^8$ is a phenyl-lower alkoxycarbonyl can be converted into the compound (1) wherein $R^8$ is hydrogen atom in the same manner as in the reaction of converting the compound (1l) into the compound (1J) in the above Reaction Scheme-16.

Other derivatives of the starting compound (2) can be prepared, for example, by the process shown in the following reaction scheme.

[Reaction Scheme-21]

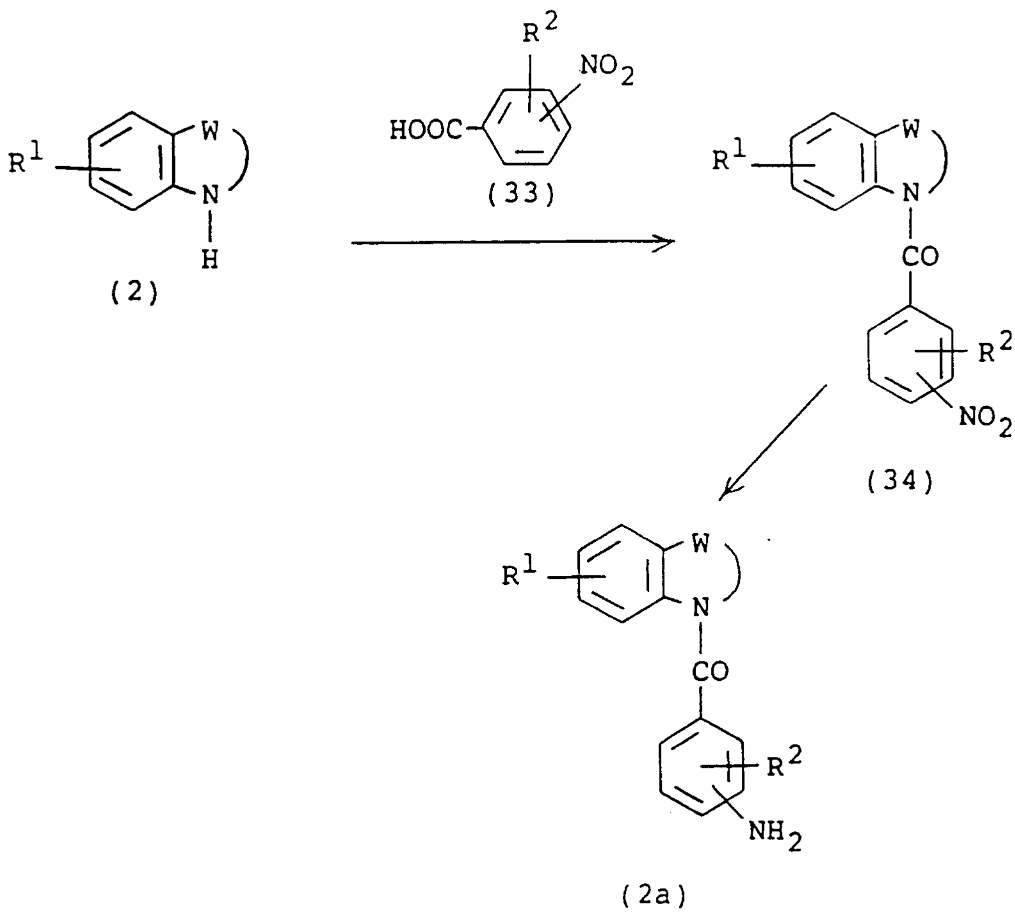

wherein $R^1$, $R^2$, and W are as defined above.

The reaction of the compound (2) and the compound (33) can be carried out under the same conditions as in the reaction of the compound (2) and the compound (3) in the above Reaction Scheme-1.

The reaction of converting the compound (34) into the compound (2a) can be carried out under the same conditions as in the reducing reaction in the above Reaction Scheme-19.

The starting compound (5) can be prepared, for example, by the process of the following reaction scheme.

[Reaction Scheme-22]

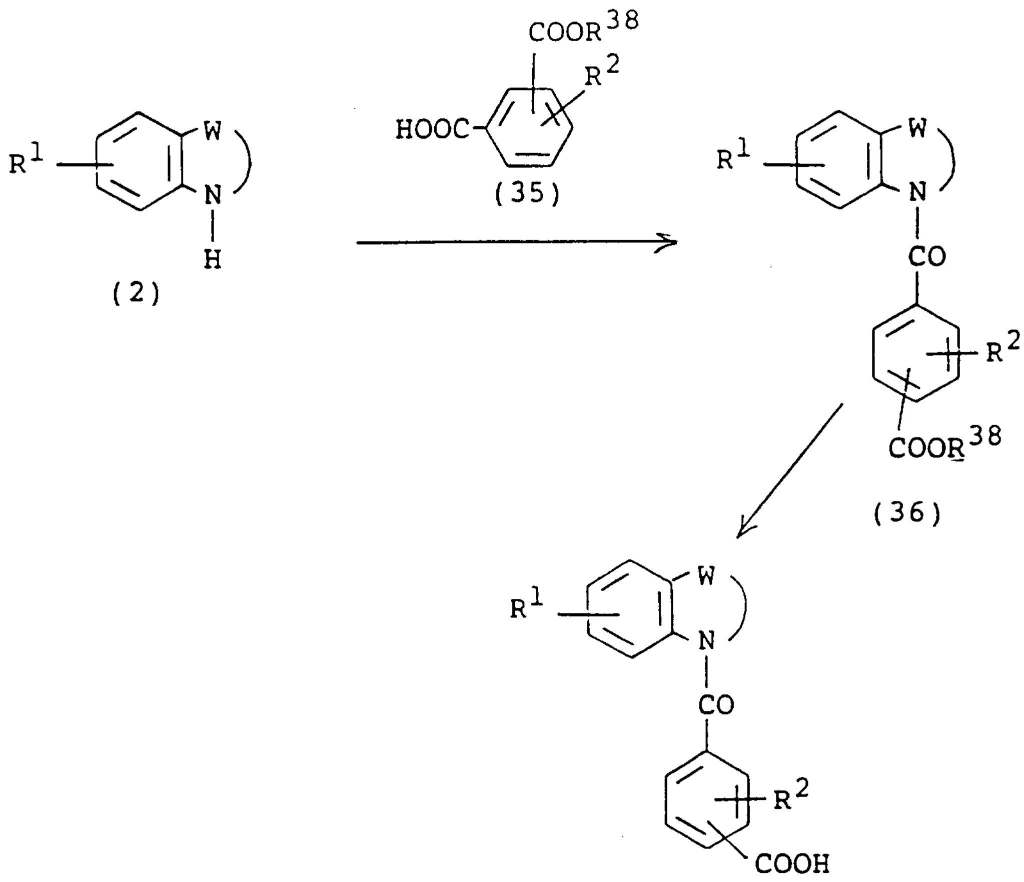

(2) (35) (36)

(5)

wherein $R^1$, $R^2$, and W are as defined above, and $R^{38}$ is a lower alkyl.

The reaction of the compound (2) and the compound (35) can be carried out under the same conditions as in the reaction of the compound (2) and the compound (3) in the above Reaction Scheme-1.

The reaction of converting the compound (36) into the compound (5) can be carried out under the same conditions as in the hydrolysis reaction in the above Reaction Scheme-7.

[Reaction Scheme-23]

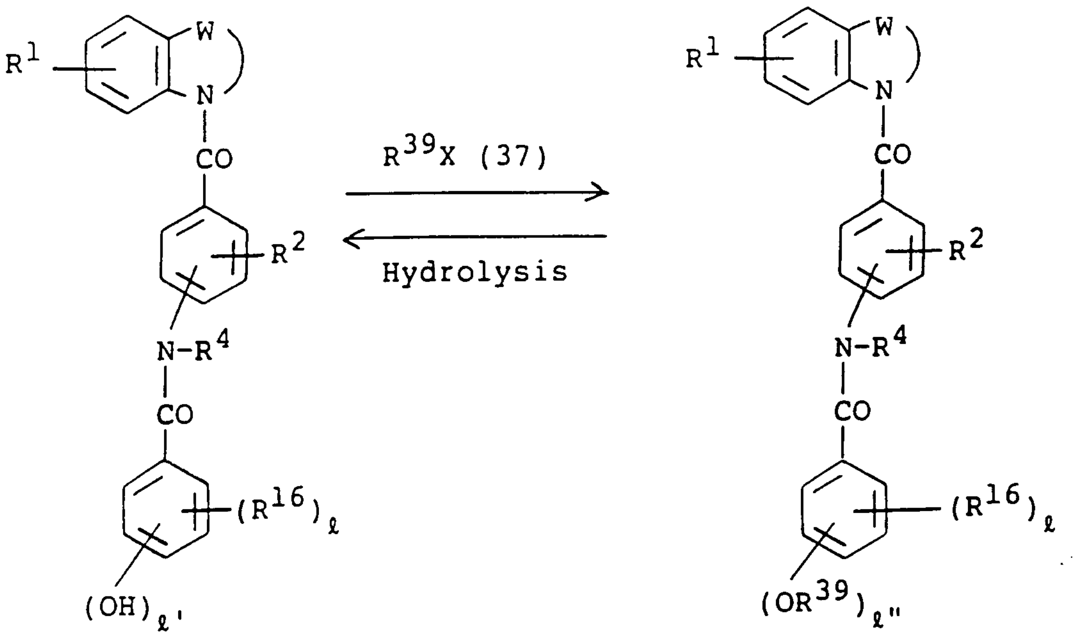


(1W)

(1X)

wherein $R^1$, $R^2$, $R^4$, $R^{16}$, ℓ, ℓ', ℓ", X, and W are as defined above, and $R^{39}$ is a lower alkanoyl.

The reaction of the compound (1W) and the compound (37) can be carried out under the same conditions as in the reaction of the compound (1n) and the compound (18) in the above Reaction Scheme-9.

The hydrolysis reaction of the compound (1X) can be carried out under the same conditions as in the hydrolysis of the compound (1j) in the above Reaction Scheme-7.

[Reaction Scheme-24]

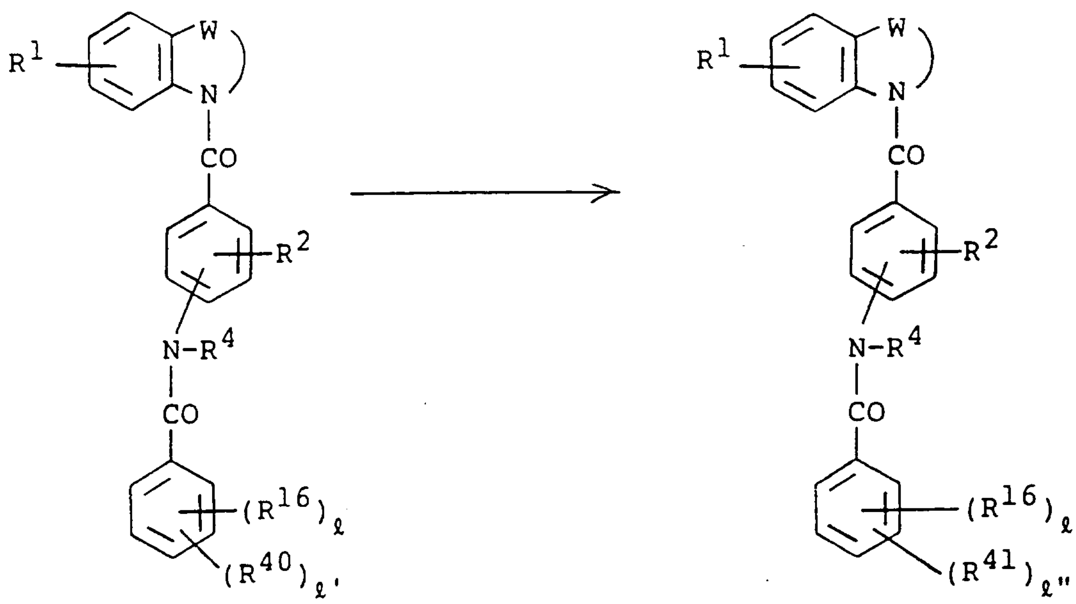

(1Y) (1Z)

wherein $R^1$, $R^2$, $R^4$, $R^{16}$, $\ell$, $\ell'$, $\ell''$, and W are as defined above, $R^{40}$ is a lower alkanoyl, and $R^{41}$ is a hydroxy-substituted lower alkyl.

The reaction of converting the compound (1Y) into the compound (1Z) can be carried out under the same conditions as in the reaction of converting the compound (1A) into the compound (1B) in the above Reaction Scheme-15.

[Reaction Scheme-25]

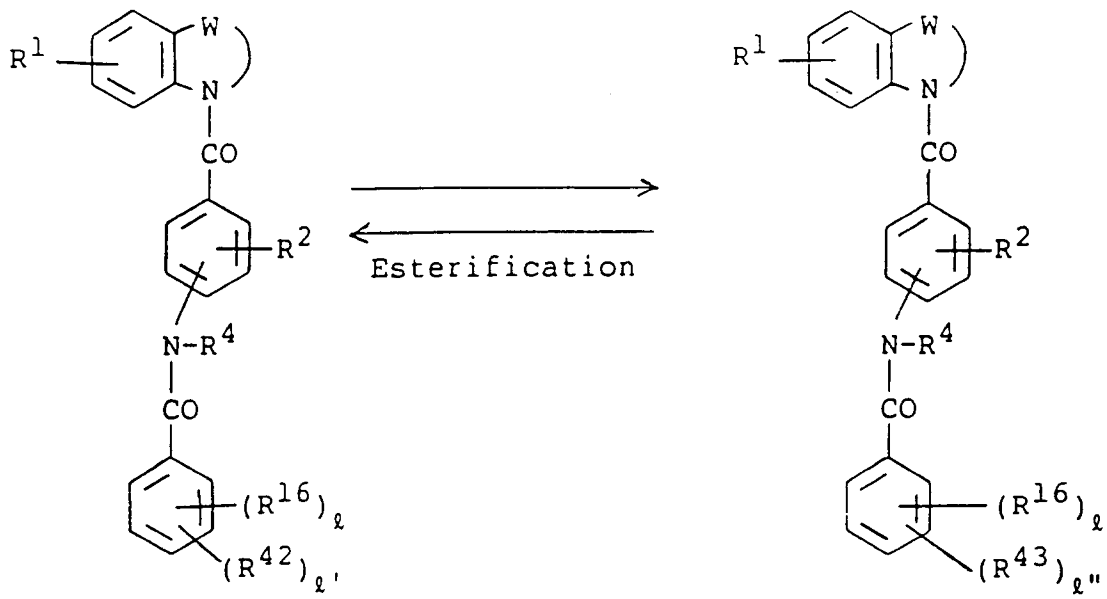


(1aa) (1bb)

wherein $R^1$, $R^2$, $R^4$, $R^{16}$, $\ell$, $\ell'$, $\ell''$, and W are as defined above, $R^{42}$ is a lower alkoxycarbonyl and $R^{43}$ is carboxyl.

The reaction of converting the compound (1aa) into the compound (1bb) can be carried out under the same conditions as in the hydrolysis of the compound (1j) in the above Reaction Scheme-7.

The esterification reaction of the compound (1bb) can be carried out under the same conditions as in the esterification of the compound (1w) in the above Reaction Scheme-13.

[Reaction Scheme-26]

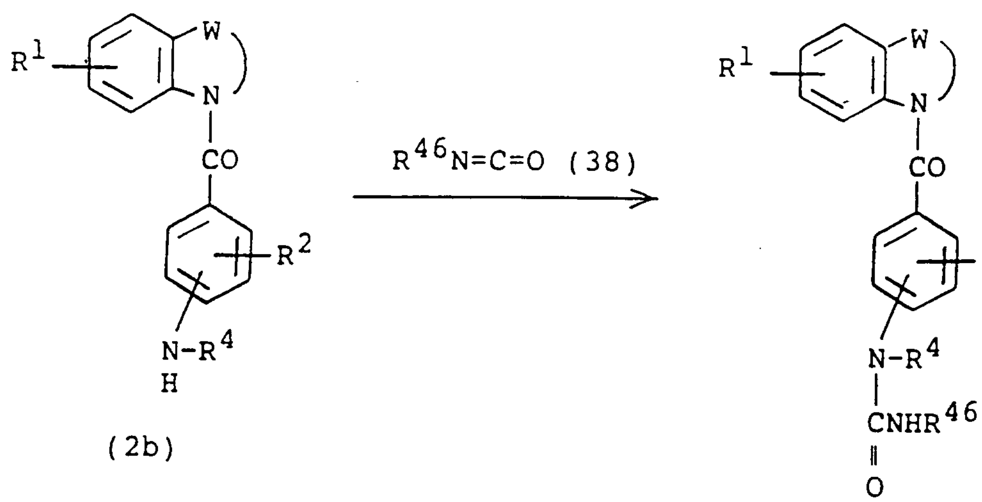


(2b)

(1cc)

wherein $R^1$, $R^2$, $R^4$, and W are as defined above, and $R^{46}$ is a phenyl having optionally a lower alkyl substituent.

The reaction of the compound (2b) and the compound (38) is usually carried out in an appropriate solvent or without solvent in the presence or absence, preferably in the absence, of a basic compound. The solvent and basic compound are the same as those used in the reaction of the carboxylic acid halide and the amine (2) in the above Reaction Scheme-1.

The compound (38) is usually used in an amount of 1 to 5 moles, preferably 1 to 3 moles, to 1 mole of the compound (2b). The reaction is usually carried out at a temperature of from 0°C to 200°C, preferably from room temperature to 150°C, for 5 minutes to 30 hours. In the reaction, a boron compound (e.g. boron trifluoride etherate, etc.) may be added to the reaction system.

[Reaction Scheme-27]

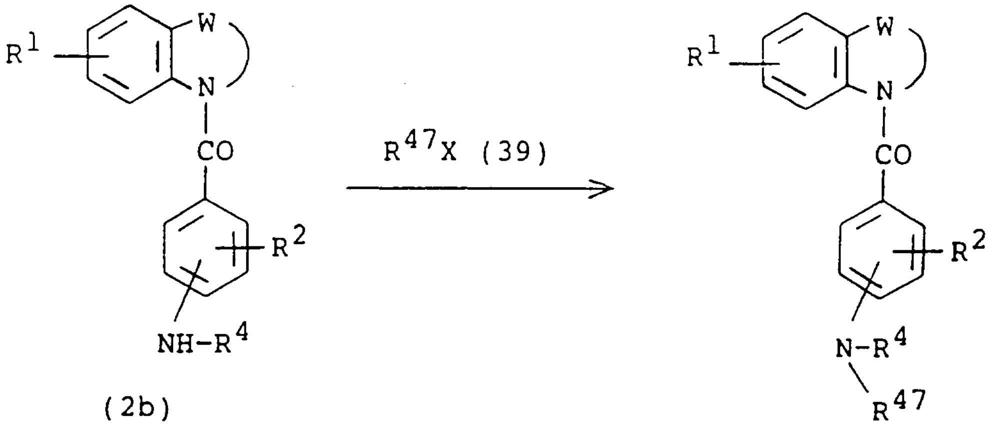


(2b)

(1dd)

wherein $R^1$, $R^2$, $R^4$, W, and X are as defined above, and $R^{47}$ is a phenylsulfonyl which has optionally a substituent selected from a halogen atom and a lower alkyl on the phenyl ring, or quinolylsulfonyl.

The reaction of the compound (2b) and the compound (39) can be carried out under the same conditions as in the reaction of the compound (7) and the compound (8) in the above Reaction Scheme-4.

[Reaction Scheme-28]

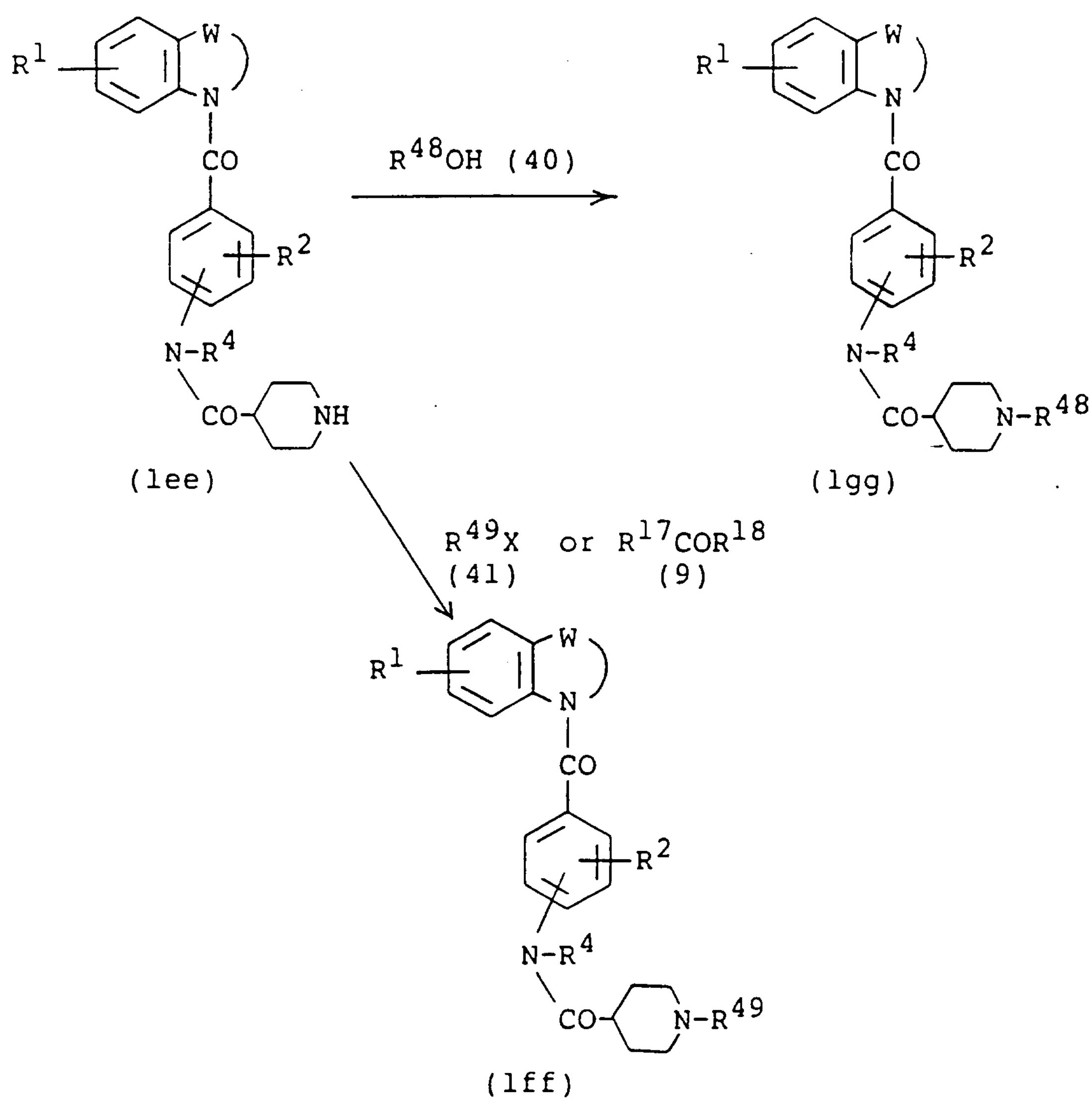


(1ee) (1gg) (1ff)

wherein $R^1$, $R^2$, $R^4$, W, $R^{17}$, $R^{18}$, and X are as defined above, $R^{48}$ is a phenyl-lower alkoxycarbonyl, a lower alkanoyl, an amino-lower alkanoyl having optionally a lower alkyl substituent, and $R^{49}$ is a lower alkyl or a carbamoyl-lower alkyl.

The reaction of the compound (1ee) and the compound (40) can be carried out under the same conditions as in the reaction of the compound (2) and the compound (3) in the above Reaction Scheme-1.

The reaction of the compound (1ee) and the compound (41) or the compound (9) can be carried out under the same conditions as in the reaction of the compound (7) and the compound (8) or the compound (9) in the above Reaction Scheme-4, provided that in the reaction product (1ff) produced by the reaction of the compound (1ee) and the compound

(9), the group $R^{49}$ is a lower alkyl.

[Reaction Scheme-29]

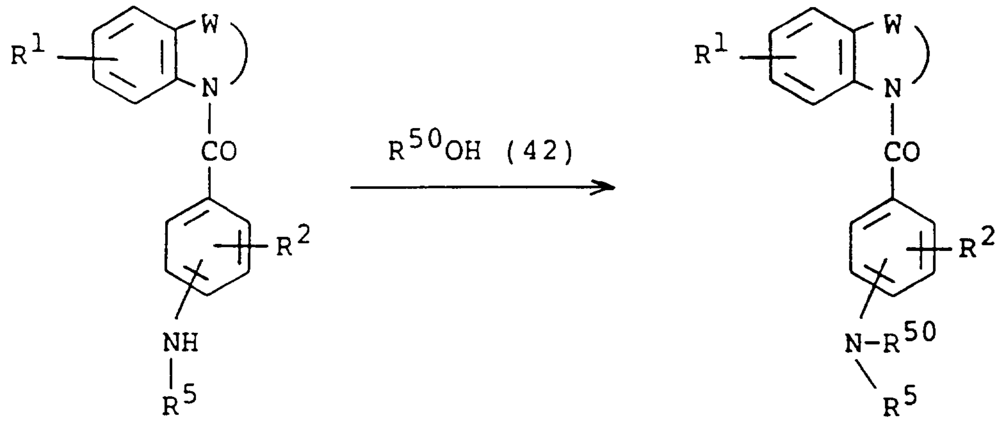


(7) (1hh)

wherein $R^1$, $R^2$, $R^5$, and W are as defined above, and $R^{50}$ is a benzoyl having optionally a halogen substituent on the phenyl ring.

The reaction of the compound (7) and the compound (42) can be carried out under the same conditions as in the reaction of the compound (2) and the compound (3) in the above Reaction Scheme-1.

[Reaction Scheme-30]

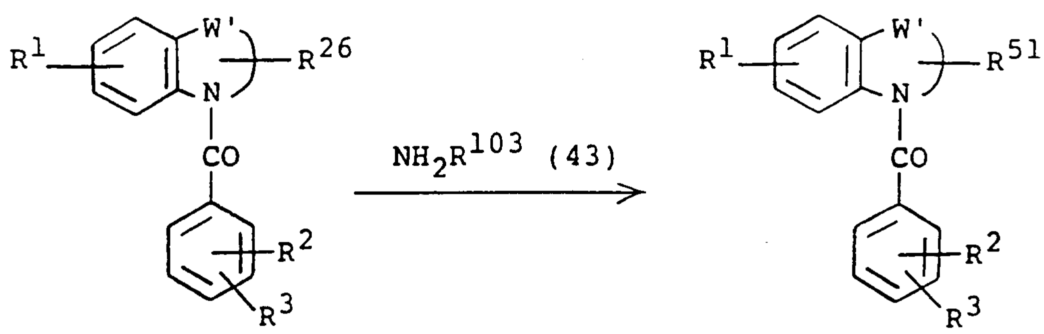


(1A) (1ii)

wherein $R^1$, W', $R^{26}$, $R^2$, and $R^3$ are as defined above, $R^{103}$ is hydroxy or sulfoxy, and $R^{51}$ is hydroxyimino or sulfoxyimino.

The reaction of the compound (1A) and the compound (43) is usually carried out in an appropriate inert solvent in the presence or absence of a basic compound. The basic compound includes, for example, inorganic basic compounds such as sodium hydroxide, potassium hydroxide, sodium carbonate, potassium carbonate, and organic basic compounds such as piperidine, pyridine, triethylamine, 1,5-diazabicyclo[4.3.0]nonene-5 (DBN), 1,8-diazabicyclo[5.4.0]undecene-7 (DBU), 1,4-diazabicyclo[2.2.2]octane (DABCO). The inert solvent includes, for example, lower alcohols (e.g. methanol, ethanol, isopropanol), ethers (e.g. dioxane, tetrahydrofuran, diethyl ether, ethylene glycol monomethyl ether), aromatic hydrocarbons (e.g. benzene, toluene, xylene), halogenated hydrocarbons (e.g. dichloromethane, dichloroethane, chloroform, carbon tetrachloride), pyridine, dimethylformamide, dimethylsulfoxide, hexamethylphosphoric triamide, or a mixture of these solvents. The compound (43) is usually used at least in equivalent amount, preferably 1 to 5 moles, to 1 mole of the compound (1A). The reaction is usually carried out at a temperature of from room temperature to 200°C,

preferably from 50°C to 150°C, for 1 to 10 hours.

[Reaction Scheme-31]

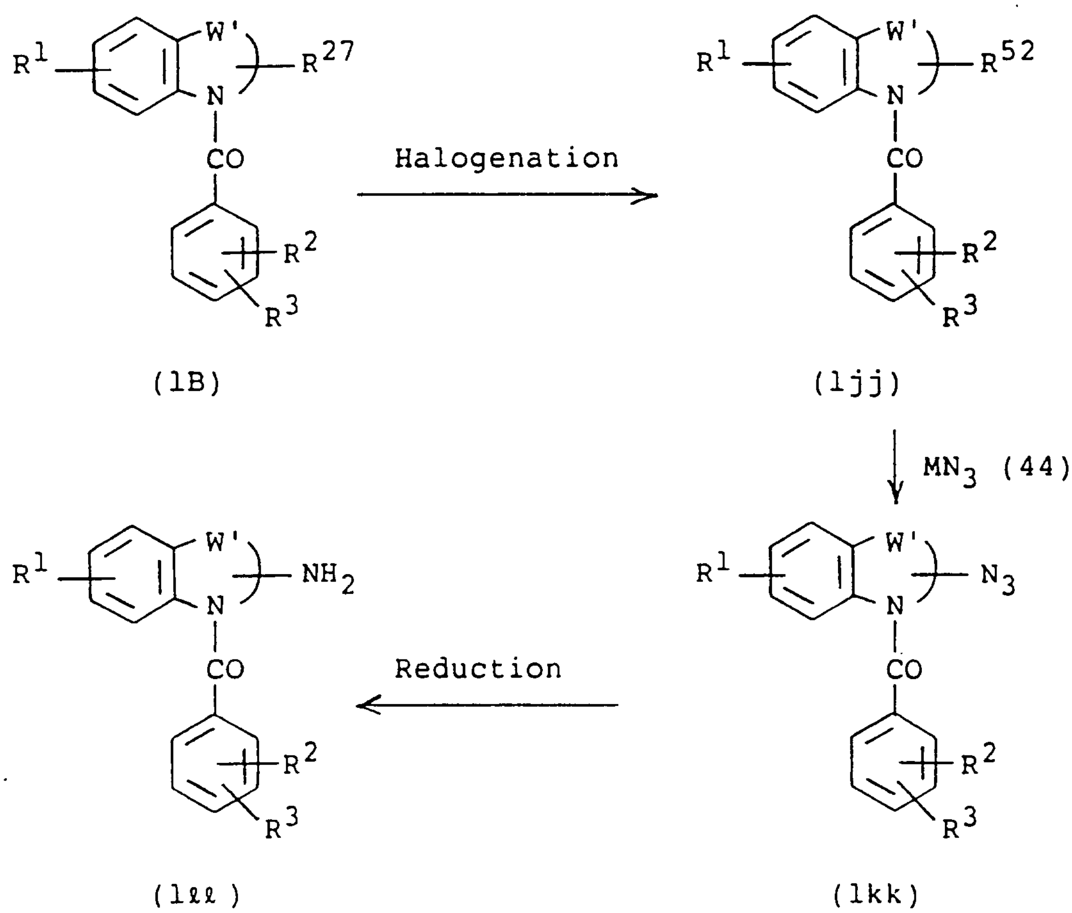


(1B) (1jj) (1kk) (1ℓℓ)

wherein $R^1$, W', $R^{27}$, $R^2$, M, and $R^3$ are as defined above, and $R^{52}$ is a halogen atom.

The halogenation of the compound (1B) is usually carried out in an appropriate solvent or without solvent by reacting the compound (1B) with a halogenating agent.

The halogenating agent includes mineral acids (e.g. hydrochloric acid, hydrobromic acid), N,N-diethyl-1,2,2-trichlorovinylamide, phosphorus pentachloride, phosphorus pentabromide, phosphorus oxychloride, thionyl chloride, methanesulfonyl chloride, or a combination of p-toluenesulfonyl chloride, and a basic compound. The basic compound includes the same compounds as used in the reaction of the compound (1A) and the compound (43) in the above Reaction Scheme-30. The solvent includes, for example, ethers (e.g. dioxane, tetrahydrofuran) and halogenated hydrocarbons (e.g. chloroform, methylene chloride, carbon tetrachloride). The amount of the halogenating agent may vary depending on the kinds of the halogenating agents, and in case of a combination of p-toluenesulfonyl chloride) and a basic compound, it is used in an amount of at least 1 mole, preferably 1 to 2 moles, to 1 mole of the compound (1B), and in case of other halogenating agents, it is used at least in an equimolar amount, usually in a largely excess amount, to the compound (1B). The reaction is usually carried out at a temperature of from room temperature to 150°C, preferably from room temperature to 80°C, for 1 to 80 hours.

The reaction of the compound (1jj) and the compound (44) can be carried out under the same conditions as in the reaction of the compound (7) and the compound (8) in the above Reaction Scheme-4.

The reducing reaction of the compound (1kk) can be carried out under the same conditions as in the reducing reaction using a reducing catalyst for converting the compound (1A) into the compound (1C) in the above Reaction

Scheme-15.

[Reaction Scheme-32A]

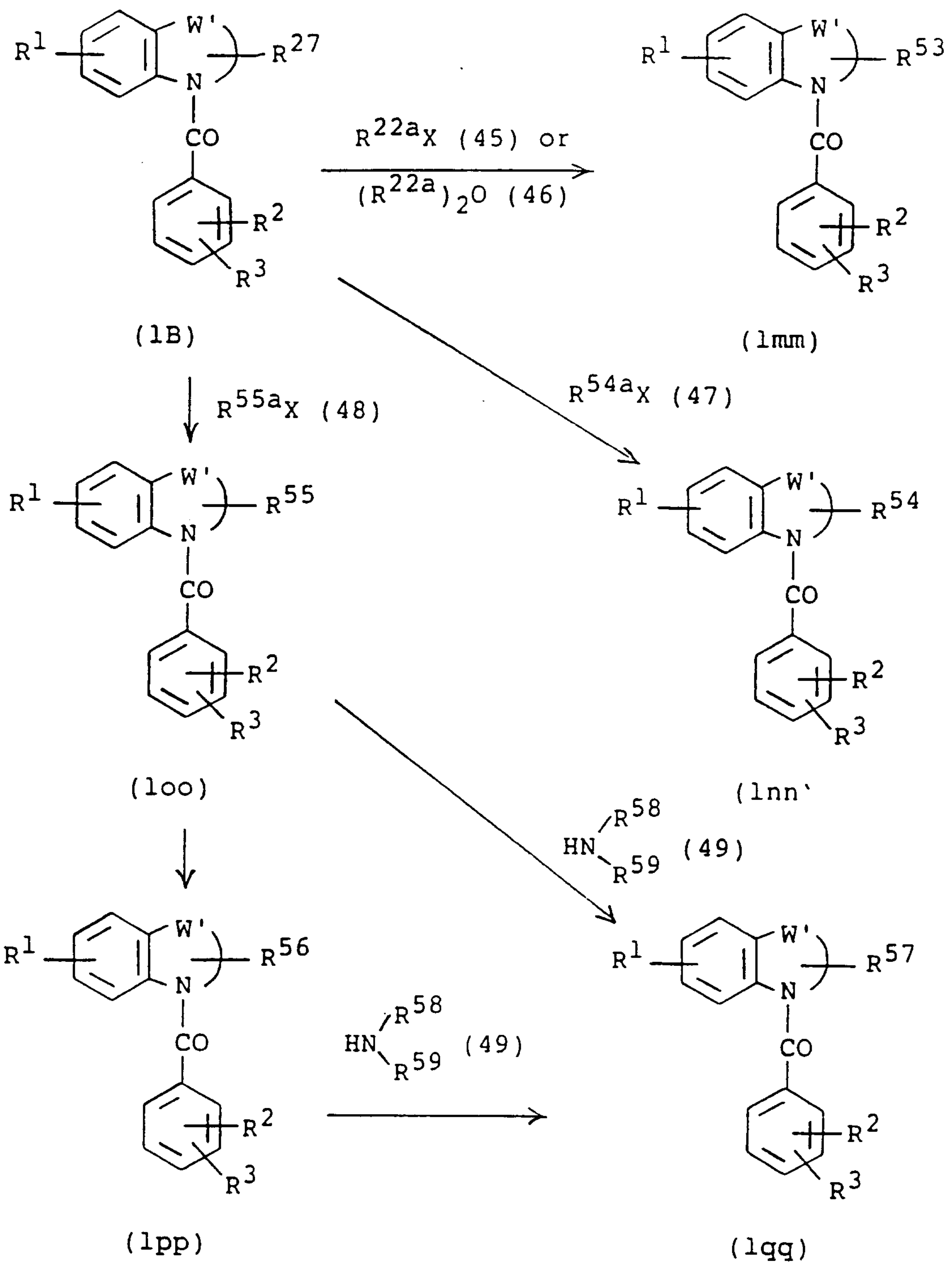


(1B) (1mm) (1oo) (1nn) (1pp) (1qq)

wherein $R^1$, W', $R^2$, $R^3$, $R^{27}$, X, and A are as defined above, $R^{53}$ is a lower alkanoyloxy having optionally a halogen substituent, $R^{54}$ is a lower alkoxy, an amino-lower alkanoyloxy having optionally a lower alkyl substituent, or a group of

the formula:

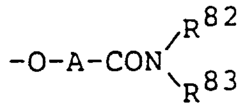

(A, $R^{82}$ and $R^{83}$ are as defined above), $R^{55}$ is a lower alkoxycarbonyl-substituted lower alkoxy, $R^{56}$ is a carboxy-substituted lower alkoxy, $R^{57}$ is an aminocarbonyl-lower alkoxy having optionally a lower alkyl substituent, $R^{54a}$ is a lower alkyl, an amino-lower alkanoyl having optionally a lower alkyl substituent, or a group of the formula:

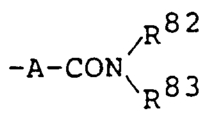

(A, $R^{82}$ and $R^{83}$ are as defined above), $R^{55a}$ is a lower alkoxycarbonyl-substituted lower alkyl, $R^{58}$ and $R^{59}$ are the same or different and are each hydrogen atom or a lower alkyl, and $R^{22a}$ is a lower alkanoyl having optionally a halogen substituent.

[Reaction Scheme-32B]

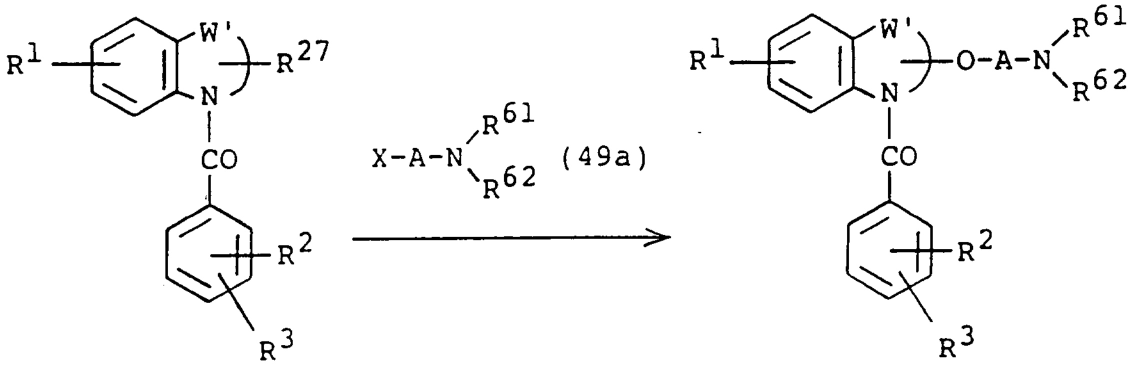


(1B) (1qq')

wherein $R^1$, W', $R^2$, $R^3$, X, $R^{27}$, and A are as defined above, and $R^{61}$ and $R^{62}$ are the same or different and are each hydrogen atom, a lower alkyl or a lower alkanoyl.

The reaction of the compound (1B) and the compound (45) or the compound (46) in the Reaction Scheme-32A can be carried out under the same conditions as in the reaction of the compound (1n) and the compound (18) in the above Reaction Scheme-9.

The reaction of the compound (1B) and the compound (47) and the reaction of the compound (1B) and the compound (48) can be carried out under the same conditions as in the reaction of the compound (1n) and the compound (18) in the above Reaction Scheme-9.

The reaction of converting the compound (1oo) into the compound (1pp) can be carried out under the same conditions as in the hydrolysis reaction of the compound (1j) in the above Reaction Scheme-7.

The reaction of the compound (1oo) and the compound (49) and the reaction of the compound (1pp) and the compound (49) can be carried out under the same conditions as in the reaction of the compound (2) and the compound (3) in the above Reaction Scheme-1.

The reaction of the compound (1B) and the compound (49a) in the Reaction Scheme-32B can be carried out under the same conditions as in the reaction of the compound (1n) and the compound (18) in the above Reaction Scheme-9.

[Reaction Scheme-33]

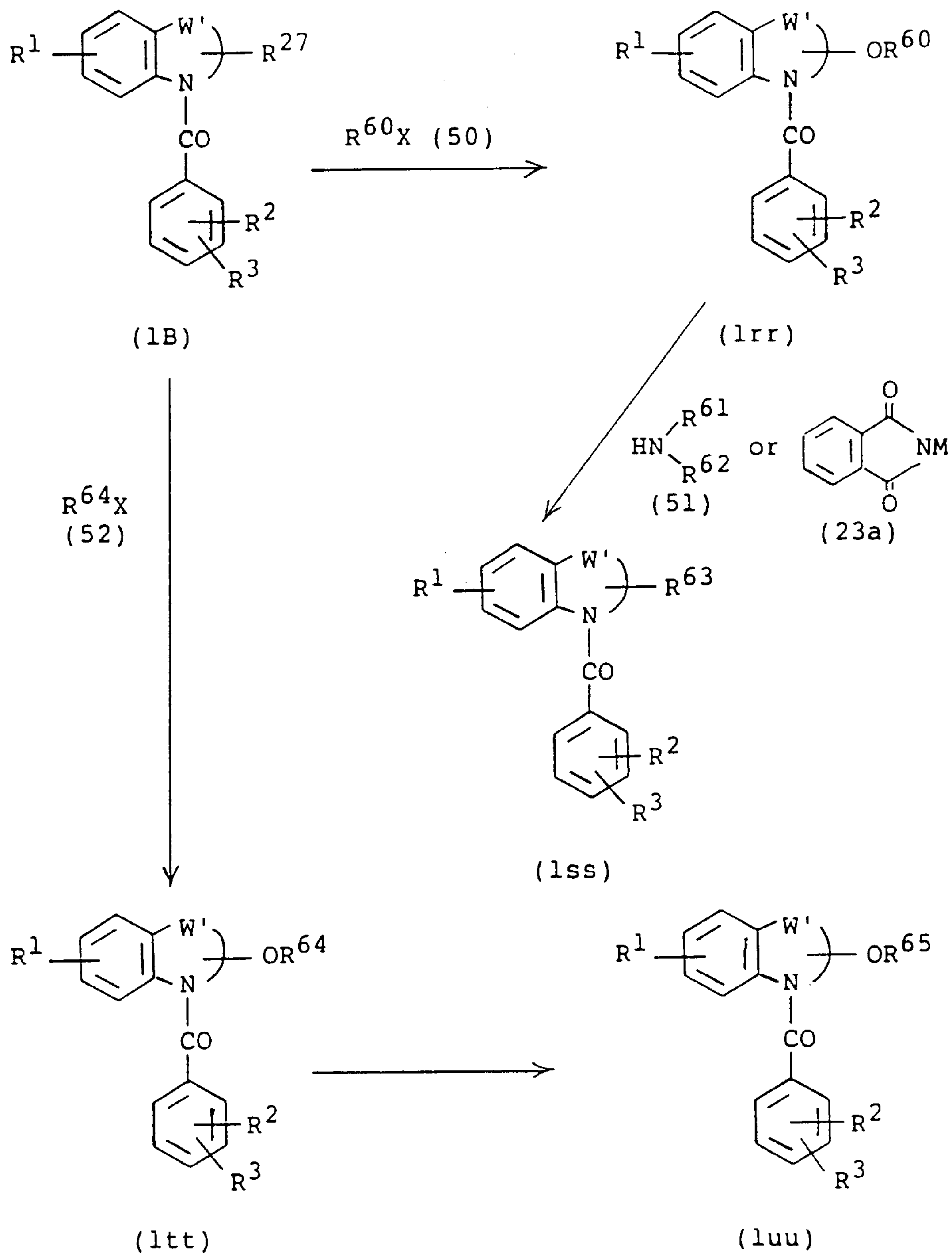


(1B) (1rr) (51) (23a) (1ss) (1tt) (1uu)

wherein $R^1$, W', $R^2$, $R^3$, $R^{27}$, $R^{61}$, $R^{62}$, M, and X are as defined above, $R^{60}$ is a halogen-substituted lower alkyl, $R^{64}$ is a phthalimido-substituted lower alkyl, $R^{63}$ is an amino-lower alkoxy having optionally a substituent selected from a lower alkyl and a lower alkanoyl, or a phthalimido-substituted lower alkoxy, and $R^{65}$ is an amino-substituted lower alkyl.

The reaction of the compound (1B) and the compound (50) and the reaction of the compound (1B) and the compound (52) can be carried out under the same conditions as in the reaction of the compound (1n) and the compound (18) in the above Reaction Scheme-9.

The reaction of the compound (1rr) and the compound (5l) or the compound (23a) can be carried out under the same conditions as in the reaction of the compound (1g) and the compound (14) in the above Reaction Scheme-6.

The reaction of converting the compound (1tt) into the compound (1uu) can be carried out under the same conditions as in the reaction of converting the compound (1j) into the compound (1k) in the above Reaction Scheme-7.

[Reaction Scheme-34]

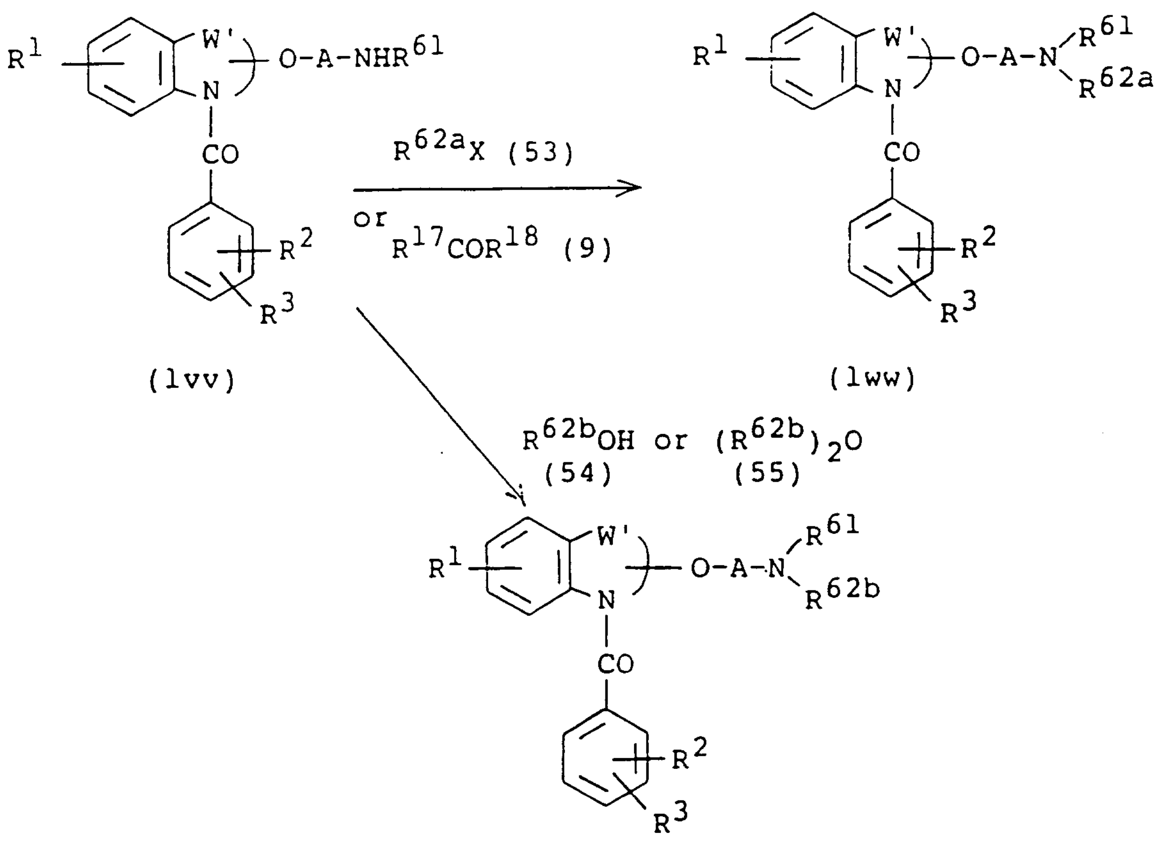


(1vv) (1ww)

(1xx)

wherein $R^1$, $R^2$, $R^3$, $R^{61}$, W', A, $R^{17}$, $R^{18}$, and X are as defined above, $R^{62a}$ is a lower alkyl, and $R^{62b}$ is a lower alkanoyl.

The reaction of the compound (1vv) and the compound (53) or the compound (9) can be carried out under the same conditions as in the reaction of the compound (7) and the compound (8) or the compound (9) in the above Reaction Scheme-4.

The reaction of the compound (1vv) and the compound (54) can be carried out under the same conditions as in the reaction of the compound (2) and the compound (3) in the above Reaction Scheme-1.

The reaction of the compound (1vv) and the compound (55) can be carried out under the same conditions as in the reaction of the compound (1p) and the compound of the formula: $(R^{7b})_2O$ in the above Reaction Scheme-10.

[Reaction Scheme-35]

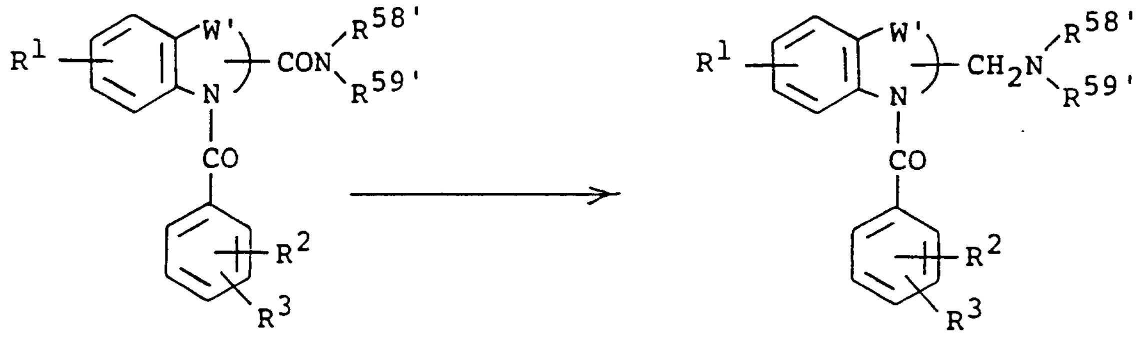

(1yy) (1zz)

wherein $R^1$, $R^2$, $R^3$, and W' are as defined above, $R^{58'}$ and $R^{59'}$ are the same or different and are each hydrogen atom, a lower alkyl, or a lower alkanoyl.

The reaction of converting the compound (1yy) into the compound (1zz) is usually carried out by reducing the compound (1yy).

The reducing reaction is preferably carried out by using a hydrogenating reducing agent. The hydrogenating reducing agent includes, for example, lithium aluminum hydride, sodium boro hydride, diborane. The reducing agent is usually used in an amount of at least one mole, preferably 1 to 15 moles, to 1 mole of the starting compound. The reducing reaction is usually carried out in an appropriate solvent, such as water, lower alcohols (e.g. methanol, ethanol, isopropanol), ethers (e.g. tetrahydrofuran, diethyl ether, diisopropyl ether, diglyme), or a mixture of these soslvents, at a temperature of -60°C to 150°C, preferably -30°C to 100°C, for 10 minutes to 5 hours. When lithium aluminum hydride or diborane is used as the reducing agent, it is preferable to use an anhydrous solvent such as diethyl ether, tetrahydrofuran, diglyme.

[Reaction Scheme-36]

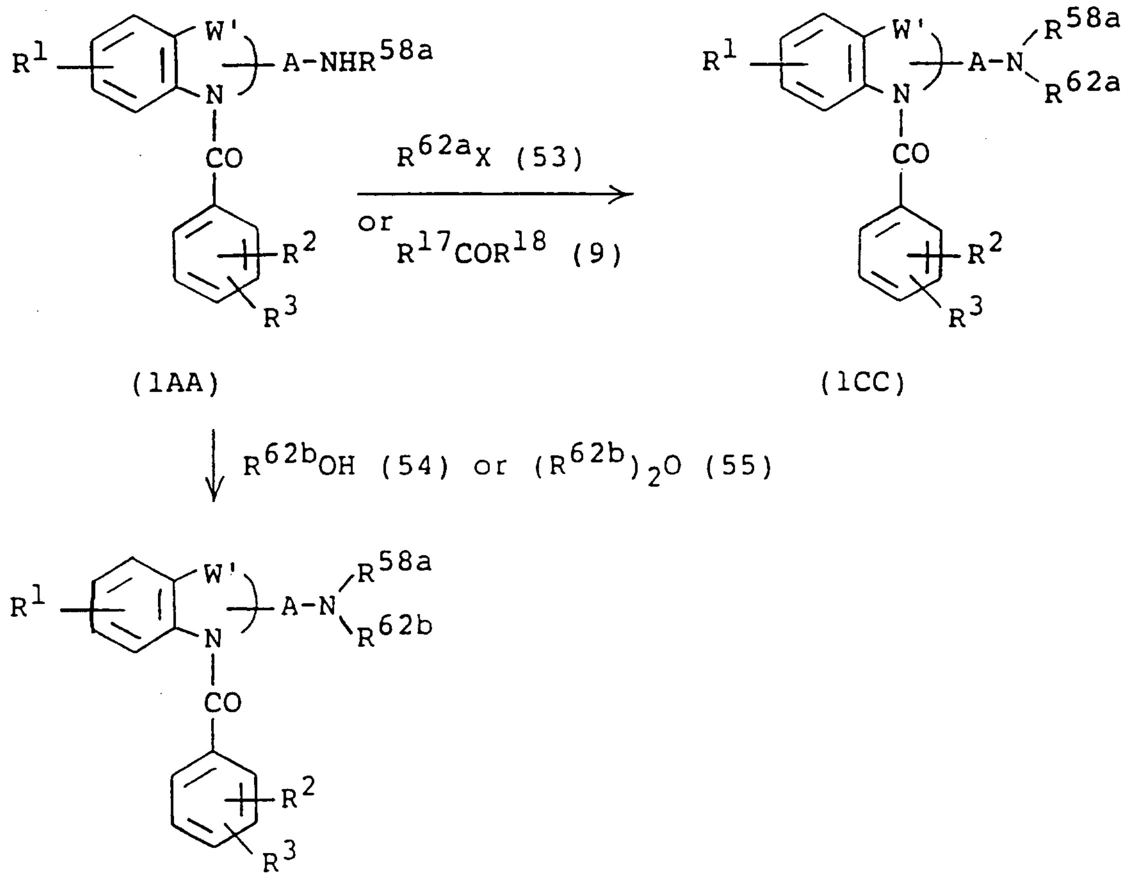


(1AA)

(1CC)

(1BB)

wherein $R^1$, W', $R^2$, $R^3$, $R^{62a}$, $R^{62b}$, X, $R^{17}$, $R^{18}$, and A are as defined above, $R^{58a}$ is hydrogen atom, a lower alkyl or a lower alkanoyl.

The reaction of the compound (1AA) and the compound (53) or the compound (9) can be carried out under the same conditions as in the reaction of the compound (7) and the compound (8) or the compound (9) in the above Reaction Scheme-4.

The reaction of the compound (1AA) and the compound (54) can be carried out under the same conditions as in the reaction of the compound (2) and the compound (3) in the above Reaction Scheme-1.

The reaction of the compound (1AA) and the compound (55) can be carried out under the same conditions as in the reaction of the compound (1p) and the compound of the formula: $(R^{7b})_2O$ in the above Reaction Scheme-10.

The compound (1BB) wherein $R^{62b}$ is formyl can also be prepared by reacting the compound (1AA) with a formate of the formula: $HCOOR^{82}$ under the same conditions as in the reaction of the compound (1K) and the compound of the formula: $HCOOR^{82}$ as described hereinbefore.

The compound (1) wherein $R^{16}$ or $R^2$ is a lower alkoxy can be converted into the correspond compound (1) wherein $R^{16}$ or $R^2$ is hydroxy by heating the compound in a mixture of an acid (e.g. hydrobromic acid, hydrochloric acid, etc.) and a solvent (e.g. water, methanol, ethanol, isopropyl alcohol) at 30 to 150°C, preferably at 50 to 120°C.

Besides, the compound (1) wherein $R^{16}$ or $R^2$ is hydroxy can also be prepared by hydrolysis of the above compound (1) wherein $R^{16}$ or $R^2$ is a lower alkoxy. The hydrolysis can be carried out in an appropriate solvent in the presence of an acid. The solvent includes, for example, water, lower alcohols (e.g. methanol, ethanol, isopropyl alcohol), ethers (e.g. dioxane, tetrahydrofuran), halogenated hydrocarbons (e.g. dichloromethane, chloroform, carbon tetrachloride), polar solvents (e.g. acetonitrile), or a mixture of these solvents. The acid includes, for example, mineral acids (e.g. hydrochloric acid, hydrobromic acid), Lewis acids (e.g. boron trifluoride, aluminum chloride, boron tribromide), iodides (e.g. sodium iodide, potassium iodide), or a mixture of the above Lewis acid and iodide. The reaction is usually carried out at a temperature of from room temperature to 150°C, preferably from room temperature to 100C, for 0.5 to 30 hours.

[Reaction Scheme-37]

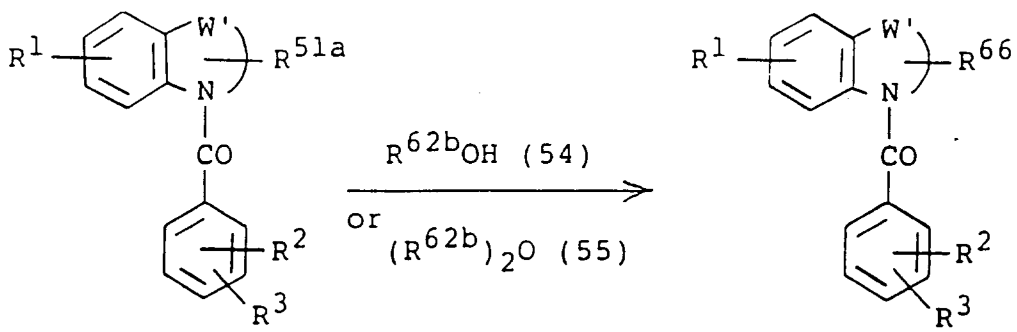


(1ii') (1DD)

wherein $R^1$, $R^2$, $R^3$, $R^{62b}$, and W' are as defined above, $R^{51a}$ is hydroxyimino, and $R^{66}$ is a lower alkanoyloxyimino.

The reaction of the compound (1ii') and the compound (54) can be carried out under the same conditions as in the reaction of the compound (2) and the compound (3) in the above Reaction Scheme-1.

The reaction of the compound (1ii') and the compound (55) can be carried out under the same conditions as in the reaction of the compound (1p) and the compound of the formula: $(R^{7b})_2O$ in the above Reaction Scheme-10.

[Reaction Scheme-38A]

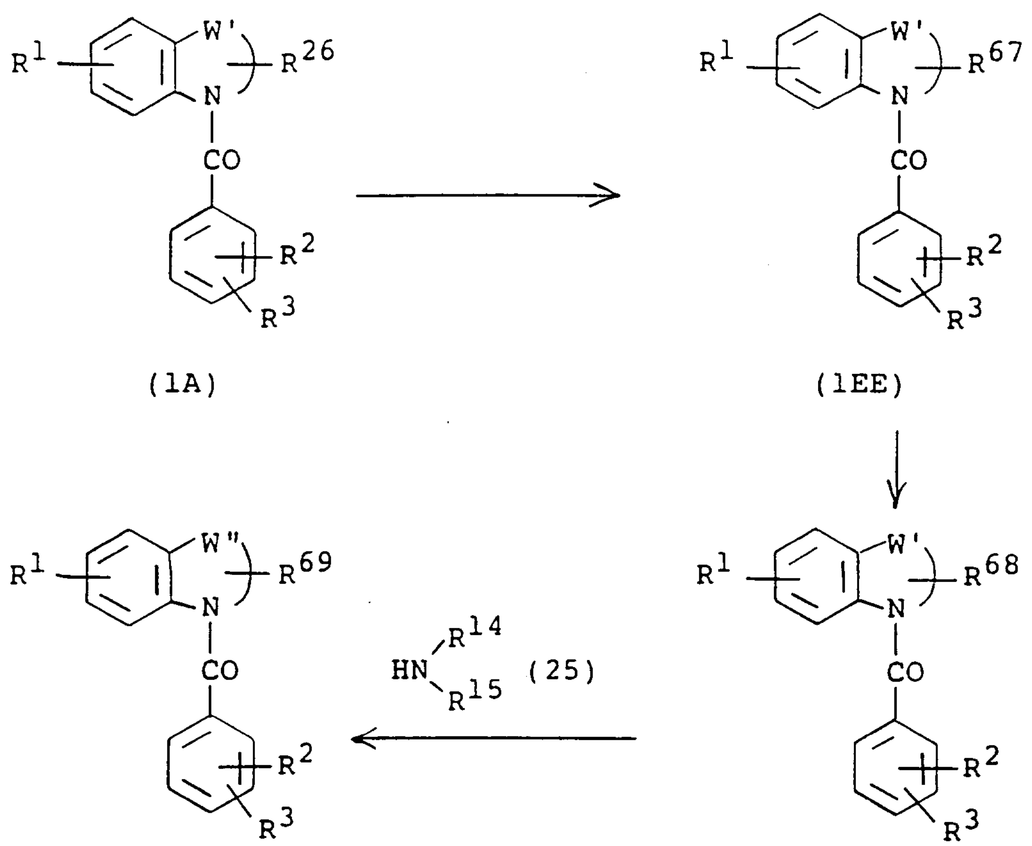


(1A) (1EE)

(1GG) (1FF)

[Reaction Scheme-38B]

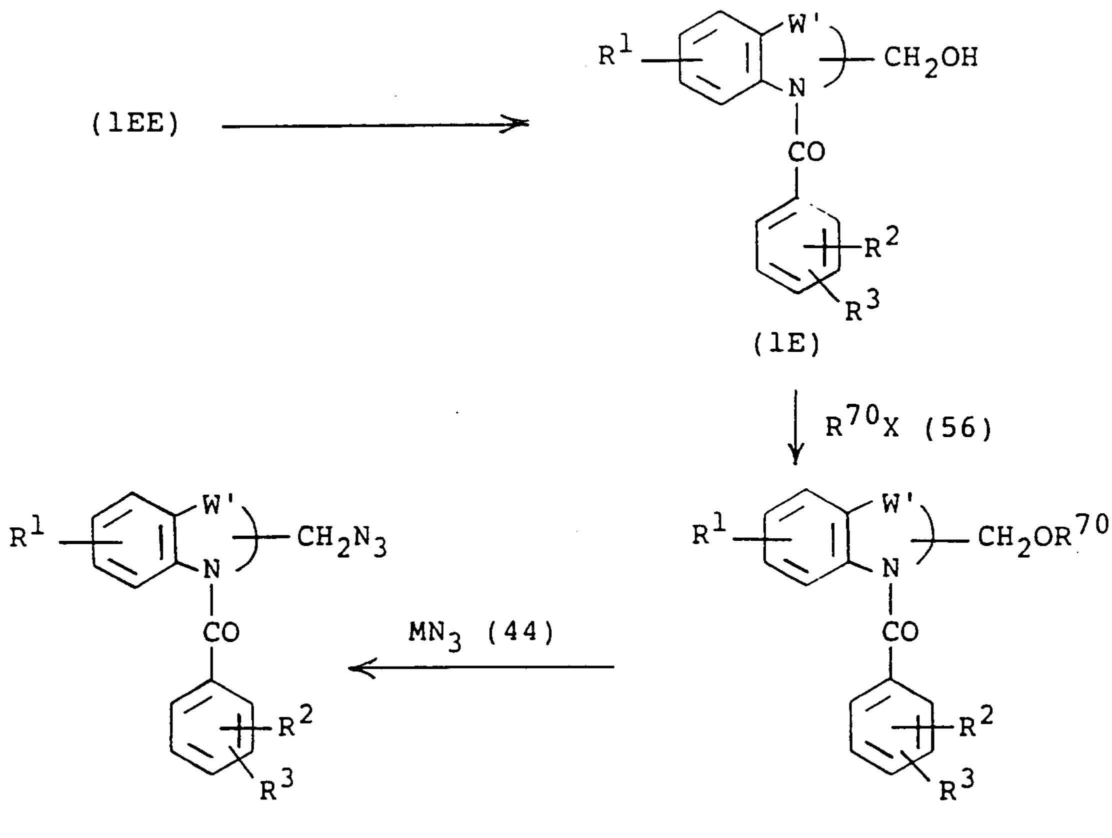


(1EE)

(1E)

(1JJ)

(1HH)

[Reaction Scheme-38C]

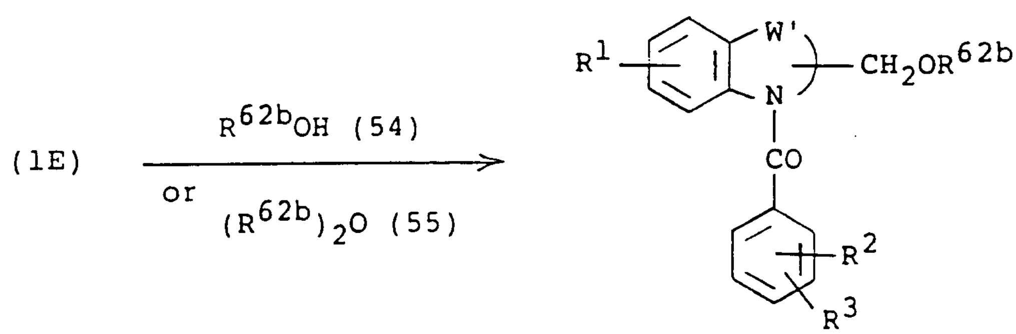


(1E)

(1II)

[Reaction Scheme-38D]

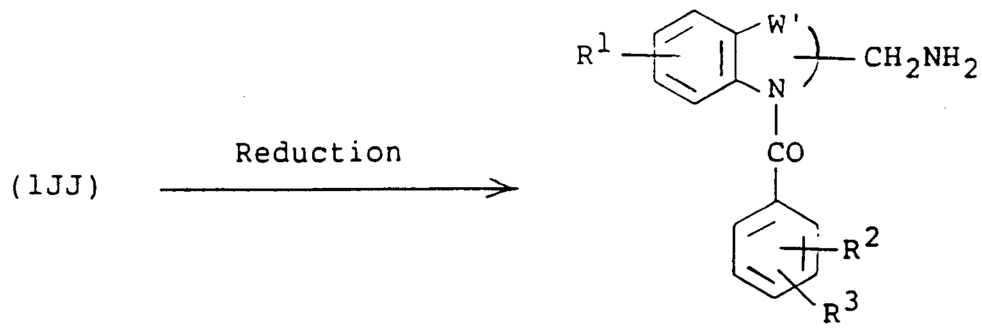


(1KK)

[Reaction Scheme-38E]

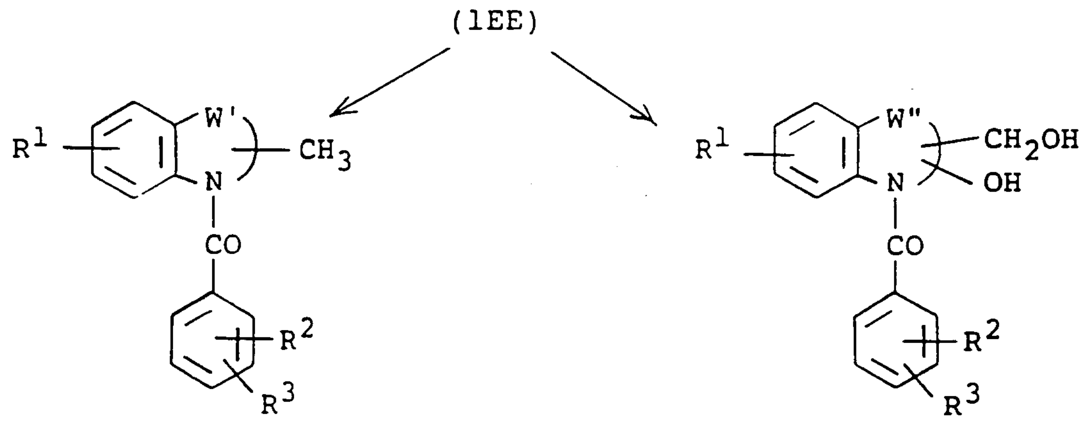


(1LL) (1MM)

wherein $R^1$, $R^2$, $R^3$, W', $R^{26}$, $R^{14}$, $R^{15}$, $R^{62b}$, X and M are as defined above, $R^{67}$ is methylidene, $R^{68}$ is a group of the formula:

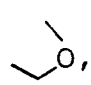

and $R^{69}$ is a group of the formula:

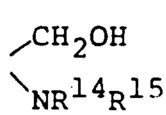

($R^{14}$ and $R^{15}$ are as defined above), or

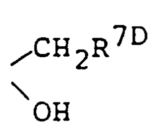

($R^{7D}$ is an amino having optionally a substituent selected from a lower alkyl and a lower alkanoyl, $R^{70}$ is a lower alkylsulfonyl, and W" is the same as the above W, provided that the number of the substituent in the groups $-(CH_2)_p-$ and $-CH=CH-(CH_2)_q-$is 0 or 1.

The reaction of converting the compound (1A) into the compound (1EE) is carried out in an appropriate solvent in the presence of a Wittig reagent and a basic compound. The Wittig reagent includes, for example, a phosphoric compound of the formula:

$$[(R^{71})_3P^{+}\text{-}CH_2\text{-}R^{72}]X^{-} \quad (A)$$

wherein $R^{71}$ is phenyl, $R^{72}$ is hydrogen atom or a lower alkyl, and X is a halogen atom. The basic compound includes inorganic bases (e.g. metallic sodium, metallic potassium, sodium hydride, sodium amide, sodium hydroxide, potassium hydroxide, sodium carbonate, potassium carbonate, sodium hydrogen carbonate), metal alcoholates (e.g. sodium methylate, sodium ethylate, potassium t-butoxide), alkyl or aryl lithiums or lithium amides (e.g. methyl lithium, n-butyl lithium, phenyl lithium, lithium diisopropylamide), organic bases (e.g. pyridine, piperidine, quinoline, triethylamine, N,N-dimethylaniline). The solvent includes any solvent which does not affect on the reaction, for example, ethers (e.g. diethyl ether, dioxane, tetrahydrofuran, monoglyme, diglyme), aromatic hydrocarbons (e.g. benzene, toluene, xylene), aliphatic hydrocarbons (e.g. n-hexane, heptane, cyclohexane), amines (e.g. pyridine, N,N-dimethylaniline), aprotic polar solvents (e.g. N,N-dimethylformamide, dimethylsulfoxide, hexamethylphosphoric triamide) and alcohols (e.g. methanol, ethanol, isopropanol). The reaction is usually carried out at a temperature of -80°C to 150°C, preferably -80°C to 120°C, for 0.5 to 15 hours.

The reaction of converting the compound (1EE) into the compound (1LL) can be carried out under the same conditions as in the catalytically hydrogenation reaction for converting the compound (1A) into the compound (1C) in the above Reaction Scheme-15.

The reaction of converting the compound (1EE) into the compound (1FF) is carried out in an appropriate solvent in the presence of an oxidizing agent. The solvent includes, for example, water, organic acids (e.g. formic acid, acetic acid, trifluoroacetic acid), alcohols (e.g. methanol, ethanol, etc.), halogenated hydrocarbons (e.g. chloroform, dichloromethane), or a mixture of these solvents. The oxidizing agent includes, for example, peracids (e.g. performic acid, peracetic acid, trifluoro-peracetic acid, perbenzoic acid, m-chloroperbenzoic acid, o-carboxy-perbenzoic acid), hydrogen peroxide, sodium metaperiodate, dichromic acid, dichromates (e.g. sodium dichromate, potassium dichromate), permanganic acid, permanganates (e.g. potassium permanganate, sodium permanganate) and lead salts (e.g. lead tetraacetate, etc.). The oxidizing agent is usually used in an amount of at least 1 mole, preferably 1 to 2 moles, to 1 mole of the starting compound. The oxidizing agent is usually used at least 2 moles, preferably 2 to 4 moles, to 1 mole of the starting compound. The above reaction is usually carried out at a temperature of -10°C to 40°C, preferably from -10°C to room temperature, for 1 to 100 hours.

The reaction of the compound (1FF) and the compound in be carried out under the same conditions as in the reaction of the compound (7) and the compound (8) in the above Reaction Scheme-4.

The reaction of converting the compound (1EE) into the compound (1E) can be carried out by firstly subjecting it to hydroboration reaction and then to oxidation.

The hydroboration reaction is carried out in a solvent such as ethers (e.g. diethyl ether, tetrahydrofuran, dioxane) in the presence of a hydroborating agent at a temperature of from 0°C to 50°C, preferaly 0°C to room temperature, for 1 to 10 hours. The hydroborating agent includes boron hydride compounds, for example, $BH_3$.tetrahydrofuran, $BH_3.S(CH_3)_2$, $BH_2Cl$, $(CH_3)_2CHC(CH_3)_2BH_2$, $(CH_3)_2CHCH(CH_3)BH$,

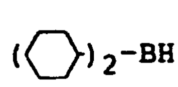, 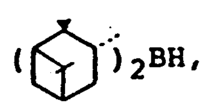,

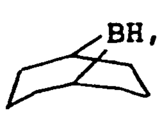, 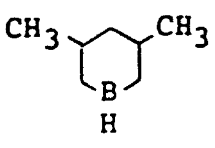, $BHCl_2$ and 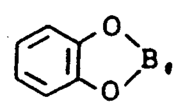,

The subsequent oxidation is carried out in water in the presence of an oxidizing agent. The oxidizing agent includes, for example, alkaline hydrogen peroxides (e.g. hydrogen peroxide - sodium hydroxide), and air oxidation is also used. The reaction is usually carried out at a temperature of from room temperature to 150°C, preferably from room temperature to 100°C, for 0.5 to 7 hours.

The hydroborating agent and the oxidizing agent are each used in an amount of at least 1 mole, preferably 1 to 2 mole, to 1 mole of the compound (1EE).

The reaction of the compound (1E) and the compound (54) can be carried out under the same conditions as in the reaction of the compound (2) and the compound (3) in the above Reaction Scheme-1.

The reaction of the compound (1E) and the compound (55) can be carried out under the same conditions as in the reaction of the compound (1p) and the compound of the formula: $(R^{7b})_2O$ in the above Reaction Scheme-10.

The reaction of the compound (1E) and the compound (56) can be carried out under the same conditions as in the reaction of the compound (7) and the compound (8) in the above Reaction Scheme-4.

The reaction of the compound (1HH) and the compound (44) can be carried out under the same conditions as in the reaction of the compound (7) and the compound (8) in the above Reaction Scheme-4.

The reducing reaction of the compound (1JJ) can be carried out under the same conditions as in the catalytic hydrogenation reaction for converting the compound (1A) into the compound (1C) in the above Reaction Scheme-15.

The reaction of converting the compound (1EE) into the compound (1MM) can be carried out by reacting with an oxidizing agent in an appropriate solvent in the presence of a co-oxidizing agent.

The solvent used for the reaction with an oxidizing agent includes, for example, pyridine, ethers (e.g. dioxane, tetrahydrofuran, diethyl ether), aromatic hydrocarbons (e.g. benzene, toluene, xylene), halogenated hydrocarbons (e.g. dichloromethane, dichloroethane, chloroform, carbon tetrachloride), esters (e.g. ethyl acetate), water, alcohols (e.g. methanol, ethanol, isopropanol, t-butanole), or a mixture of these solvents. The co-oxidizing agent includes, for example, organic amine N-oxides (e g. pyridine N-oxide, N-ethyldiisopropylamine N-oxide, N-methylmorpholine N-oxide, trimethylamine N-oxide, triethylamine N-oxide). The oxidizing agent includes, for example, osmium tetraoxide. The oxidizing agent is usually used in an amount of at least 1 mole, preferably 1 to 5 moles, to 1 mole of the starting compound. The reaction is usually carried out at a temperature of from -20°C to 150°C, preferably from room temperature to 100°C, for 1 to 10 hours.

[Reaction Scheme-39]

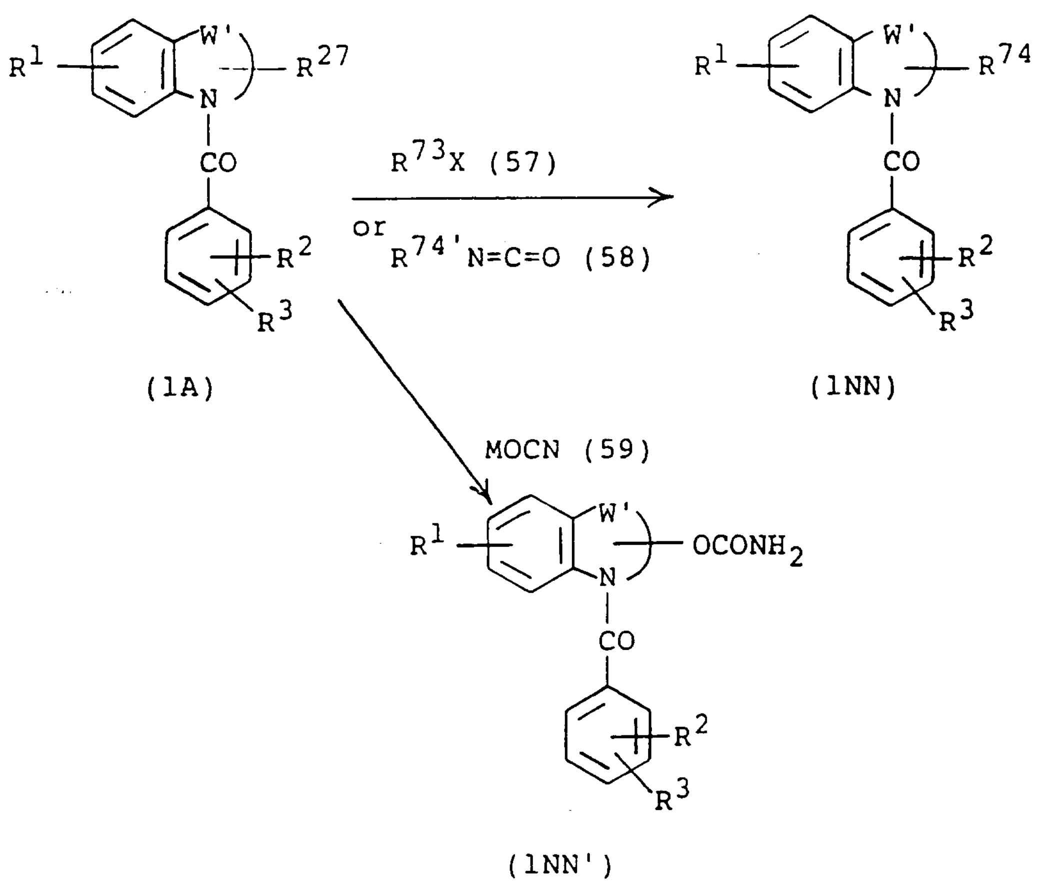


wherein $R^1$, $R^2$, $R^3$, $R^{27}$, W', M, and X are as defined above, $R^{73}$ is an aminocarbonyl having optionally a lower alkyl substituent, $R^{74}$ is an aminocarbonyloxy having optionally a lower alkyl substituent, $R^{74'}$ is a lower alkyl.

The reaction of the compound (1A) and the compound (57) can be carried out under the same conditions as in the reaction of the compound (7) and the compound (8) in the above Reaction Scheme-4.

The reaction of the compound (1A) and the compound (59) is carried out in an appropriate solvent in the presence of an acid. The solvent includes the same solvent as used in the reaction or the compound (7) and the compound (8) in the above Reaction Scheme-4. The acid includes, for example, mineral acids (e.g. hydrochloride acid, sulfuric acid), sulfonic acids (e.g. methanesulfonic acid, p-toluenesulfonic acid) and alkanoic acids (e.g. trifluoroacetic acid). The compound (59) is used in an amount of at least 1 mole, preferably 1 to 5 moles, to 1 mole of the compound (1A). The reaction is usually carried out at a temperature of from room temperature to 150°C, preferably from room temperature to 100°C, for 1 to 7 hours.

The reaction of the compound (1A) and the compound (58) can be carried out under the same conditions as in the reaction of the compound (2b) and the compound (38) in the above Reaction Scheme-26.

[Reaction Scheme-40]

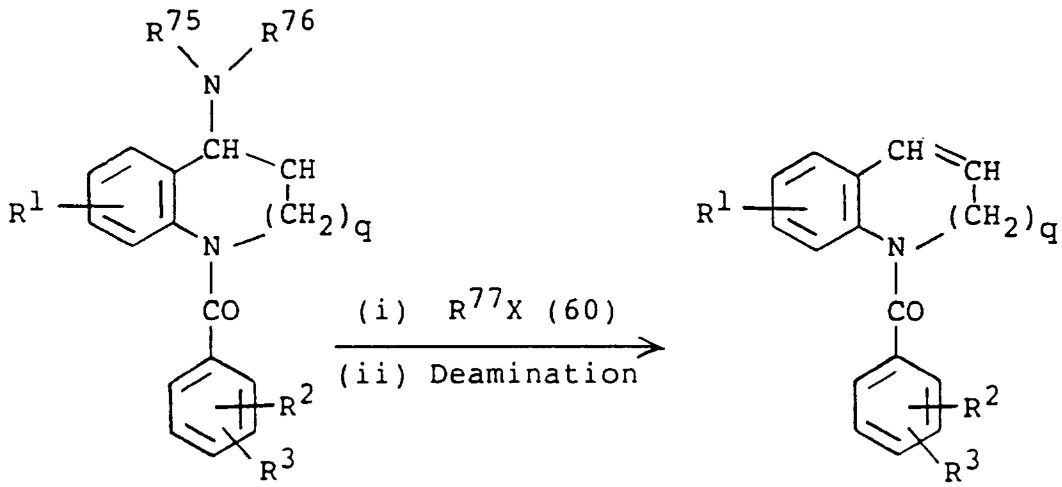


(100) (1PP)

wherein $R^1$, $R^2$, $R^3$, X, and q are as defined above, and $R^{75}$, $R^{76}$ and $R^{77}$ are each a lower alkyl, and further the group: $-(CH_2)_q-$ may optionally have 1 to 3 substituents selected from a lower alkyl having optionally a hydroxy substituent, a lower alkoxycarbonyl, carboxyl, hydroxy, oxo, a lower alkanoyloxy having optionally a halogen substituent, an amino-lower alkyl having optionally a substituent selected from a lower alkyl and a lower alkanoyl, a lower alkanoyloxy-substituted lower alkyl, a lower alkylsulfonyloxy-lower alkyl, an azido-lower alkyl, a group of the formula:

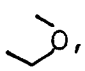

an aminocarbonyloxy having optionally a lower alkyl substituent, a lower alkoxy, a lower alkoxycarbonyl-substituted lower alkoxy, a carboxy-substituted lower alkoxy, an aminocarbonyl-lower alkoxy having optionally a lower alkyl substituent, an amino-lower alkoxy having optionally a substituent selected from a lower alkyl and a lower alkanoyl, a phthalimido-substituted lower alkoxy, hydroxyimino, a lower alkanoyloxyimino, a lower alkylidene, a halogen atom, azido, sulfoxyimino, a group of the formula:

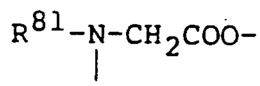

($R^{81}$ is hydrogen atom or a lower alkyl), hydrazino, pyrrolyl, an amino-lower alkanoyloxy having optionally a lower alkyl substituent, a group of the formula:

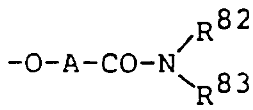

(A is as defined above, and $R^{82}$ and $R^{83}$ are the same or different and are each hydrogen atom, a lower alkyl, a carbamoyl-substituted lower alkyl, a hydroxy-substituted lower alkyl, or a pyridyl-lower alkyl, or $R^{82}$ and $R^{83}$ may bind together with nitrogen atom to which they bond to form a 5- or 6-membered saturated heterocyclic group with or without being intervened with nitrogen, oxygen or sulfur atom wherein the heterocyclic group has optionally a substituent selected from oxo, a lower alkyl, a lower alkanoyl, and carbamoyl), and a group of the formula:

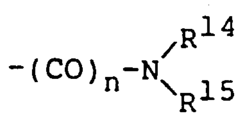

(n is as defined above, and $R^{14}$ and $R^{15}$ are the same or different and are each hydrogen atom, a lower alkyl, a lower alkenyl, a lower alkanoyl, a cycloalkyl, an oxiranyl-substituted lower alkyl, a lower alkyl having 1 to 2 substituents selected from a lower alkoxy, hydroxy and an amino having optionally a lower alkyl substituent, a phenyl-lower alkyl, a pyridyl-lower alkyl, a lower alkylsulfonyl, benzoyl, a lower alkoxycarbonyl, anilinocarbonyl, an aminocarbonyl having optionally a lower alkyl substituent, a cyano-substituted lower alkyl, a lower alkoxycarbonyl-substituted lower alkyl, a carbamoyl-substituted lower alkyl, a carboxy-substituted lower alkyl, a tetrahydropyranyloxy-substituted lower alkyl, a lower alkanoyloxy-substituted lower alkyl, a piperidinyl having optionally a phenyl-lower alkyl substituent on the piperidinyl ring, a halogen-substituted lower alkanoyl, an imidazolyl-substituted lower alkanoyl, an amino-lower alkanoyl having optionally a substituent selected from a lower alkyl and a lower alkoxycarbonyl, an aminocarbonyl-lower alkyl having optionally a lower alkyl substituent, or a phenyl-lower alkoxycarbonyl, or $R^{14}$ and $R^{15}$ may bind together with the nitrogen atom to which they bond to form a 5- or 6-membered saturated heterocyclic group with or without being intervened with nitrogen or oxygen atom, which heterocyclic group may optionally have a substituent selected from a lower alkyl, a phenyl-lower alkyl and a lower alkanoyl

The reaction of the compound (1OO) and the compound (60) is carried out in an appropriate solvent in an autoclave. The solvent includes any solvent as used in the reaction of the compound (7) and the compound (8) in the above Reaction Scheme-4. The reaction is usually carried out at a temperature of from room temperature to 200°C, preferably from room temperature to 150°C, for 1 to 7 hours.

The subsequent deamination reaction is carried out in an appropriate solvent in the presence of a basic compound. The solvent includes the same solvent as used in the above reaction of the compound (1OO) and the compound (60). The basic compound includes any basic compound as used in the reaction of converting the compound (1A) into the compound (1EE) in the above Reaction Scheme-38. The reaction is usually carried out at a temperature of from room

temperature to 150°C, preferably from room temperature to 100°C, for 1 to 10 hours.

[Reaction Scheme-41]

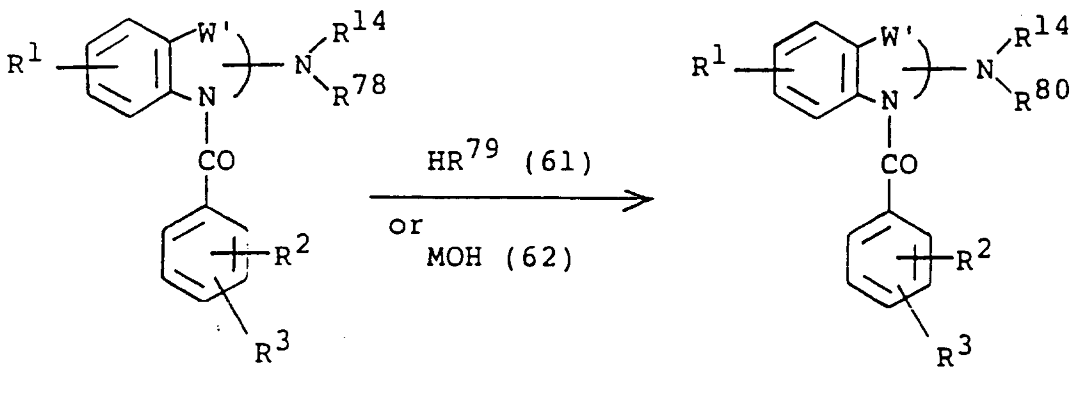


(1QQ) (1RR)

wherein $R^1$, $R^2$, $R^3$, $R^{14}$, M, and W' are as defined above, $R^{78}$ is an oxiranyl-substituted lower alkyl, $R^{79}$ is a lower alkoxy, or an amino having optionally a lower alkyl substituent, and $R^{80}$ is a lower alkyl having 2 substituents selected from hydroxy, a lower alkoxy, and an amino having optionally a lower alkyl substituent.

The reaction of the compound (1QQ) and the compound (61) can be carried out under the same conditions as in the reaction of the compound (7) and the compound (8) in the above Reaction Scheme-4.

The reaction of the compound (1QQ) and the compound (62) can be carried out by firstly reacting them in trifluoroacetic acid at a temperature of 0°C to 100°C, preferably 0°C to 50°C, for 1 to 7 hours, followed by hydrolysis of the resultant.

The hydrolysis is carried out in an appropriate solvent or without solvent in the presence of an acid or a basic compound. The solvent includes, for example, water, lower alcohols (e.g. methanol, ethanol, isopropanol), ketones (e.g. acetone, methyl ethyl ketone), ethers (e.g. dioxane, tetrahydrofuran, ethylene glycol dimethyl ether), fatty acids (e.g. acetic acid, formic acid), or a mixture of these solvents. The acid includes, for example, mineral acids (e.g. hydrochloric acid, sulfuric acid, drobromic acid) and organic acids (e.g. formic acid, acetic acid, aromatic sulfonic acid). The basic compound includes, for example, metal carbonates (e.g. sodium carbonate, potassium carbonate), metal hydroxides (e.g. sodium hydroxide, potassium hydroxide, calcium hydroxide). The reaction is usually carried out at a temperature of from room temperature to 200°C, preferably from room temperature to 150°C, for 0.5 to 25 hours.

[Reaction Scheme-42]

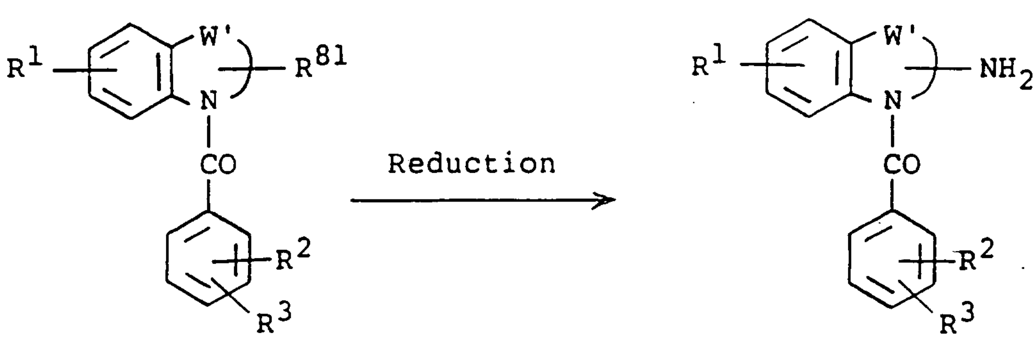


(1SS) (1ℓℓ)

wherein $R^1$, $R^2$, $R^3$, and W' are as defined above, and $R^{81}$ is hydroxyimino or a lower alkanoyloxyimino.

The reaction of converting the compound (1SS) into the compound (1ℓℓ) is carried out by catalytically hydrogenating the compound (1SS) in an appropriate solvent in the presence of a catalyst. The solvent includes, for example, water,

acetic acid, alcohols (e.g. methanol, ethanol, isopropanol), hydrocarbons (e.g. hexane, cyclohexane), ethers (e.g. diethylene glycol dimethyl ether, dioxane, tetrahydrofuran, diethyl ether), esters (e.g. ethyl acetate, methyl acetate), aprotic polar solvents (e.g. dimethylformamide), or a mixture of these solvents. The catalyst includes, for example, palladium, palladium black, palladium-carbon, platinum, platinum oxide, copper chromate and Raney nickel. The catalyst is usually used in an amount of 0.02 to 1 part by weight to 1 part by weight of the compound (1SS). The reaction is usually carried out at a temperature of from -20°C to 100°C, peferably 0°C to 70°C, under a hydrogen atmospheric pressure of 101.3 kPa to 1.013 MPa (1 to 10 atm) for 0.5 to 20 hours.

Alternatively, the reducing reaction can also be carried out by using a hydrogenating reducing agent. The hydrogenating reducing agent includes, for example, lithium aluminum hydride, sodium boro hydride, diborane. The reducing agent is usually used in an amount of at least one mole, preferably 1 to 10 moles, to 1 mole of the compound (1SS). The reaction is usually carried out in an appropriate solvent, such as water, lower alcohols (e.g. methanol, ethanol, isopropanol), ethers (e.g. tetrahydrofuran, diethyl ether, diglyme), acetic acid, and the like, at a temperature of 0°C to 200°C, preferably 0°C to 170°C, for 10 minutes to 10 hours. When lithium aluminum hydride or diborane is used as the reducing agent, it is preferable to use an anhydrous solvent such as diethyl ether, tetrahydrofuran, diglyme.

[Reaction Scheme-43]

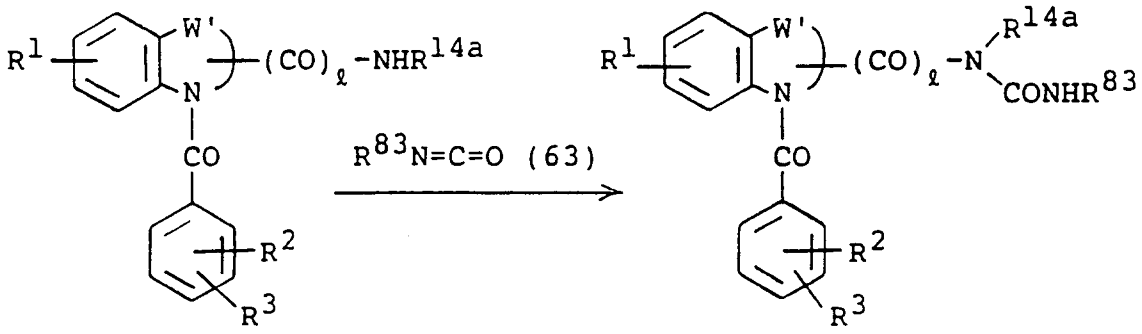


(1K) (1TT)

wherein $R^1$, $R^2$, $R^3$, W', $\ell$, $R^{14a}$ are as defined above, and $R^{83}$ is phenyl or a lower alkyl.

The reaction of the compound (1K) and the compound (63) can be carried out under the same conditions as in the reaction of the compound (2b) and the compound (38) in the above Reaction Scheme-26.

[Reaction Scheme-44]

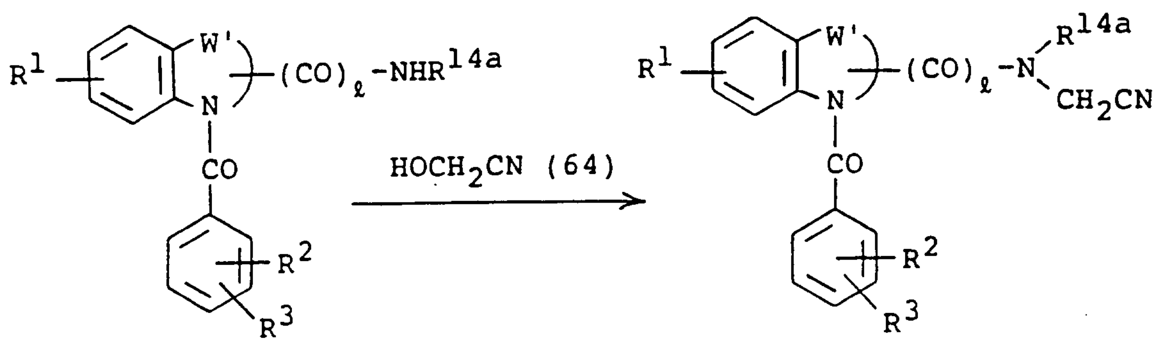


(1K) (1UU)

wherein $R^1$, $R^2$, $R^3$, W', $\ell$, $R^{14a}$ are as defined above.

The reaction of the compound (1K) and the glyconitrile (64) can be carried out in an appropriate solvent. The solvent includes the same solvent as used in the reaction of the compound (7) and the compound (8) in the above Reaction Scheme-4. The reaction is usually carried out at a temperature of from 0°C to 150°C, preferably 0°C to 100°C, for 1 to

10 hours. The glyconitrile (64) is used in an amount of at least 1 mole, preferably 1 to 2 moles, to 1 mole of the compound (1K).

[Reaction Scheme-45]

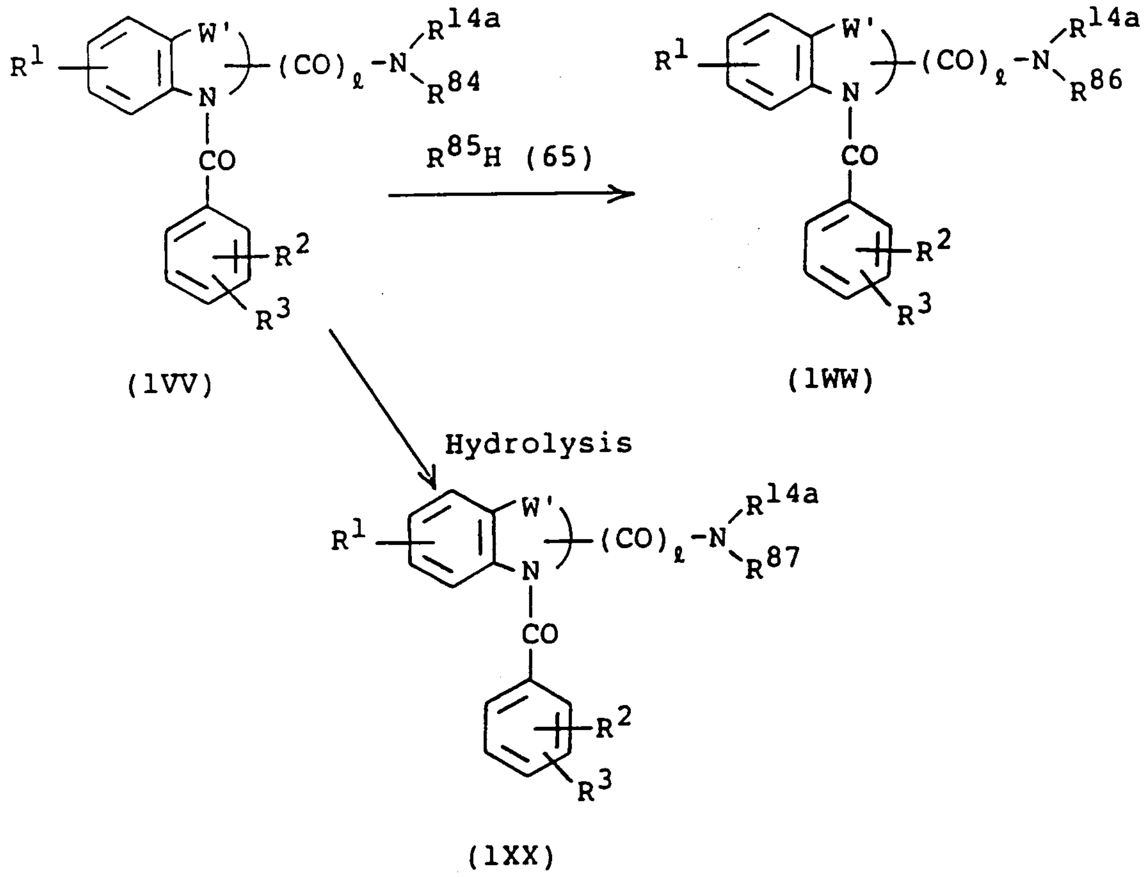


(1VV)

(1WW)

(1XX)

wherein $R^1$, $R^2$, $R^3$, W', ℓ, $R^{14a}$ are as defined above, $R^{84}$ is a lower alkoxycarbonyl-substituted lower alkyl, $R^{85}$ is an amino having optionally a lower alkyl substituent, $R^{86}$ is an aminocarbonyl-lower alkyl having optionally a lower alkyl substituent, and $R^{87}$ is a carboxy-substituted lower alkyl.

The reaction of the compound (1VV) and the compound (65) can be carried out under the same conditions as in the reaction of the compound (2) and the compound (3) in the above Reaction Scheme-1.

The hydrolysis reaction of the compound (1VV) can be carried out under the same conditions as in the hydrolysis reaction of the compound (1QQ) and the compound (62) in the above Reaction Scheme-41.

[Reaction Scheme-46]

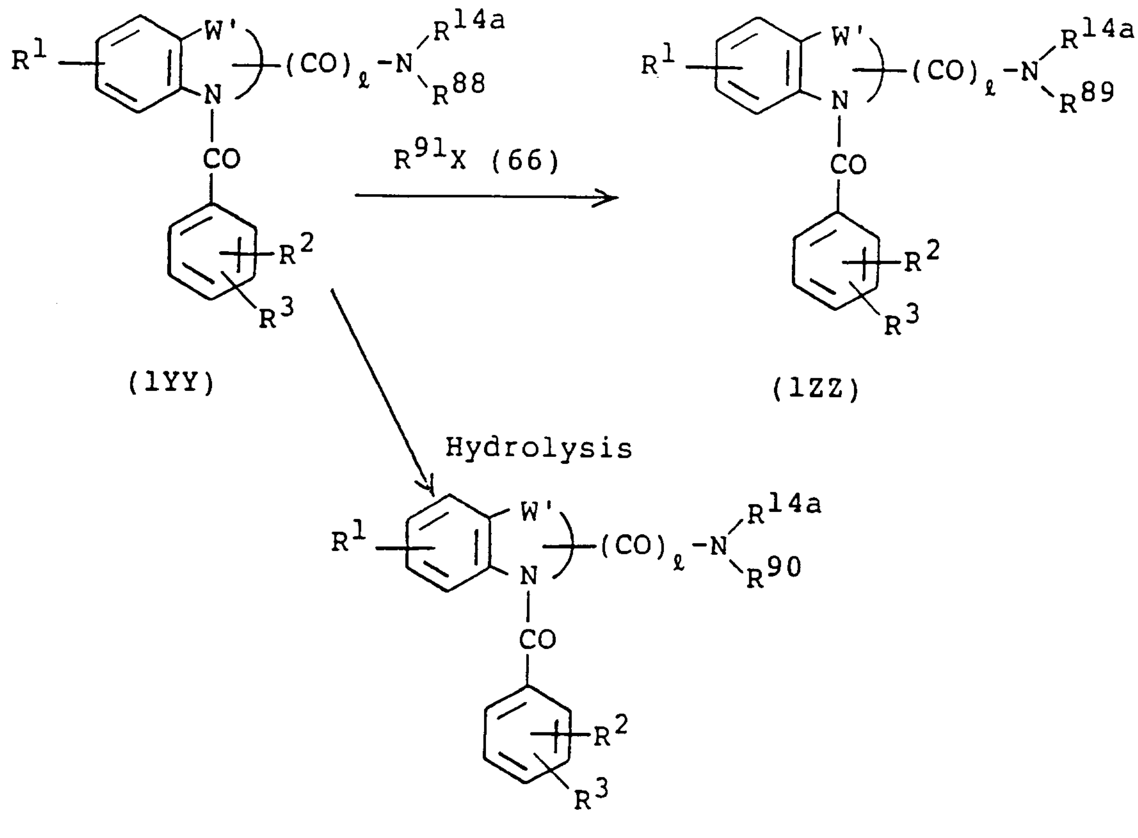


(1YY)

(1ZZ)

(1aaa)

wherein $R^1$, $R^2$, $R^3$, W', $\ell$, X, and $R^{14a}$ are as defined above, $R^{88}$ is a tetrahydropyranyloxy-substituted lower alkyl, $R^{89}$ is a lower alkanoyloxy-substituted lower alkyl, $R^{90}$ is a hydroxy-substituted lower alkyl, and $R^{91}$ is a lower alkanoyl.

The reaction of the compound (1YY) and the compound (66) can be carried out in a solvent such as acetic acid at a temperature of 0°C to 200°C, preferably 0°C to 150°C, for 0.5 to 15 hours.

The hydrolysis reaction of the compound (1YY) can be carried out under the same conditions as in the hydrolysis reaction of the compound (1QQ) and the compound (62) in the above Reaction Scheme-41, wherein a pyridinium salt

(e.g. pyridinium p-toluenesulfonate) may be used as the acid.

[Reaction Scheme-47]

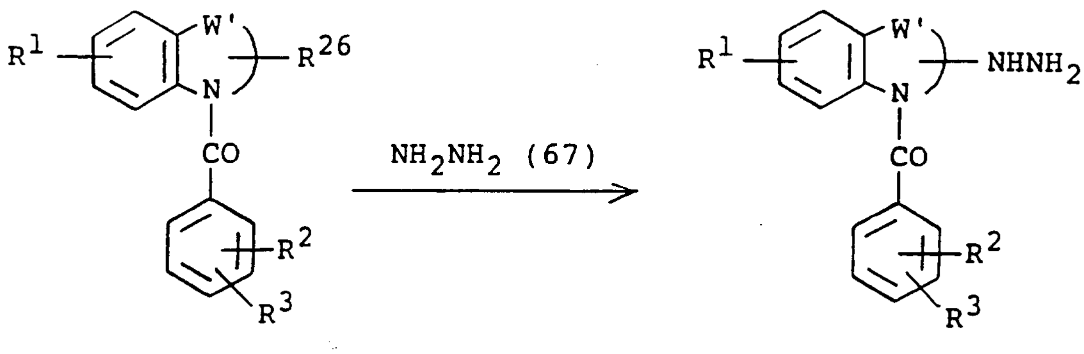


(1A) (1bbb)

wherein $R^1$, $R^2$, $R^3$, W', and $R^{26}$ are as defined above.

The reaction of converting the compound (1A) into the compound (1bbb) can be carried out under the same conditions as in the reaction of converting the compound (1A) into the compound (1C) in the above Reaction Scheme-15.

[Reaction Scheme-48]

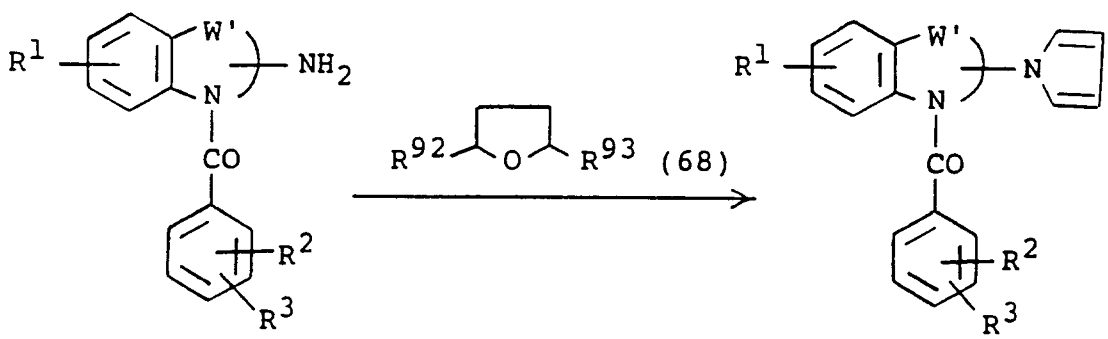


(1ℓℓ) (1ccc)

wherein $R^1$, $R^2$, $R^3$ and W' are as defined above, $R^{92}$ and $R^{93}$ are each a lower alkoxy.

The reaction of the compound (1ℓℓ) and the compound (68) is carried out in an appropriate solvent in the presence of an acid. The solvent includes, for example, water, alcohols (e.g. methanol, ethanol, isopropanol), ketones (e.g. acetone, methyl ethyl ketone), ethers (e.g. dioxane, tetrahydrofuran, ethylene glycol dimethyl ether), fatty acids (e.g. acetic acid, formic acid), or a mixture of these solvents. The acid includes, for example, mineral acids (e.g. hydrochloric acid, sulfuric acid, hydrobromic acid), organic acids (e.g. formic acid, acetic acid, aromatic sulfonic acids). The reaction is usually carried out at a temperature of from room temperature to 200°C, preferably from room temperature to 150°C, for 0.5 to 5 hours. The compound (68) is usually used in an amount of at least 1 mole, preferably 1 to 2 moles, to 1 mole

of the compound (1ℓℓ).

[Reaction Scheme-49]

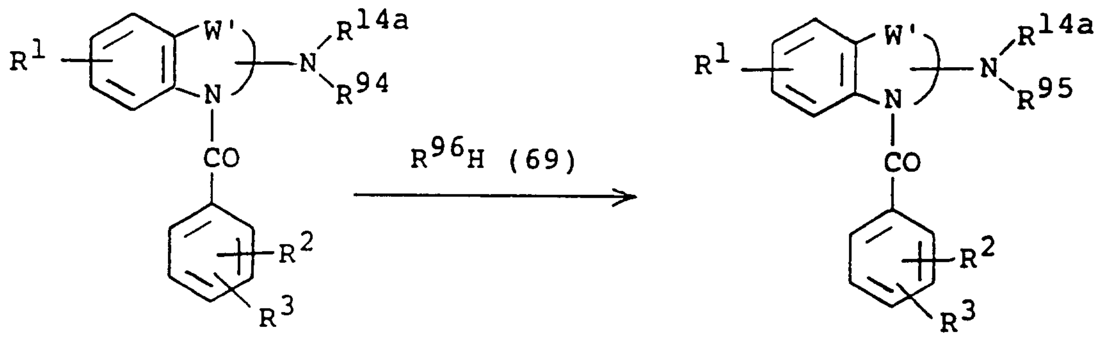


(1ddd) (1eee)

wherein $R^1$, $R^2$, $R^3$, W', and $R^{14a}$ are as defined above, $R^{94}$ is a halogen-substituted lower alkanoyl, $R^{95}$ is an imidazolyl-substituted lower alkanoyl or an amino-lower alkanoyl having optionally a substituent selected from a lower alkyl and a lower alkoxycarbonyl, and $R^{96}$ is imidazolyl, or an amino having optionally a substituent selected from a lower alkyl and a lower alkoxycarbonyl.

The reaction of the compound (1ddd) and the compound (69) can be carried out under the same conditions as in the reaction of the compound (7) and the compound (8) in the above Reaction Scheme-4.

[Reaction Scheme-50]

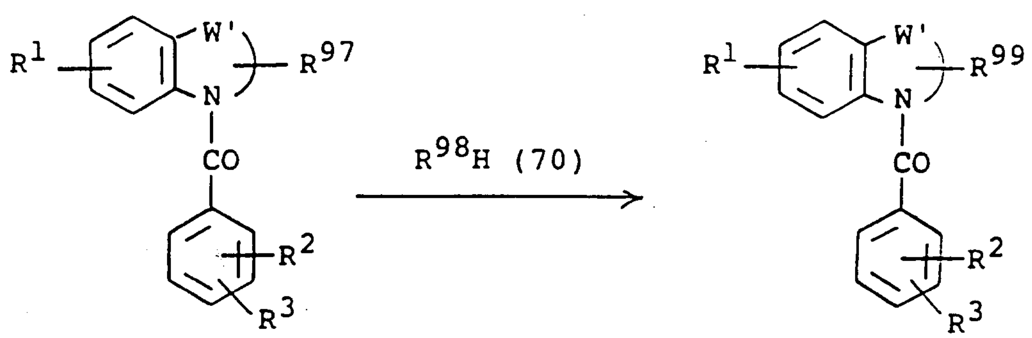


(1fff) (1ggg)

wherein $R^1$, $R^2$, $R^3$, and W' are as defined above, $R^{97}$ is a lower alkanoyloxy having a halogen substituent, $R^{98}$ is an amino having optionally a lower alkyl substituent, and $R^{99}$ is an amino-lower alkanoyloxy having optionally a lower alkyl substituent.

The reaction of the compound (1fff) and the compound (70) can be carried out under the same conditions as in the reaction of the compound (7) and the compound (8) in the above Reaction Scheme-4.

[Reaction Scheme-51]

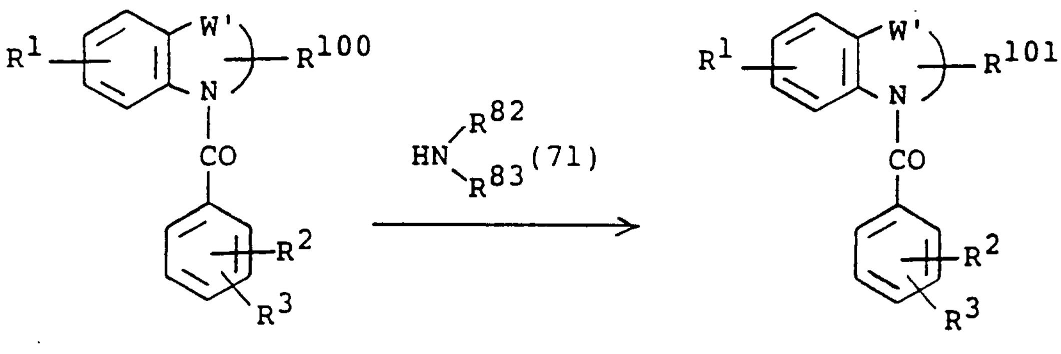


(1hhh) (1iii)

wherein $R^1$, $R^2$, $R^3$, W', $R^{82}$, and $R^{83}$ are as defined above, $R^{100}$ is a carboxy-substituted lower alkoxy, and $R^{101}$ is a group of the formula:

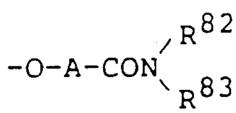

(A, $R^{82}$ and $R^{83}$ are as defined above).

The reaction of the compound (1hhh) and the compound (71) can be carried out under the same conditions as in the reaction of the compound (2) and the compound (3) in the above Reaction Scheme-1.

[Reaction Scheme-52]

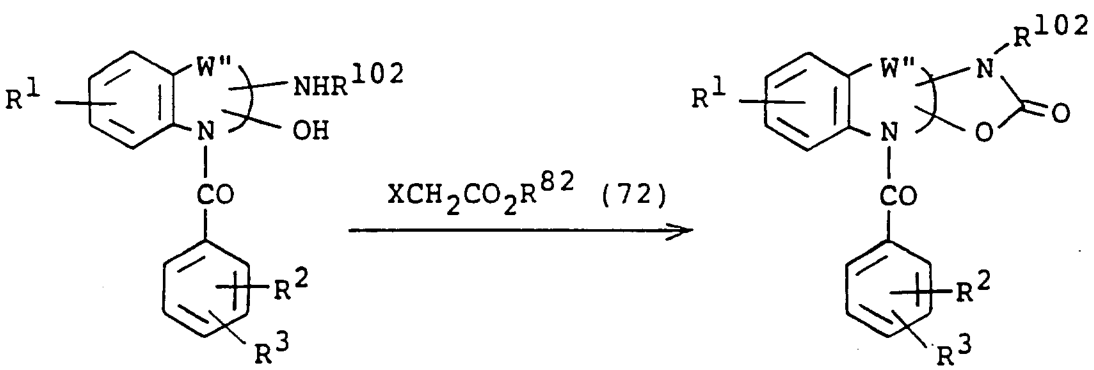


(1jjj) (1kkk)

wherein $R^1$, $R^2$, $R^3$, W", X, and $R^{82}$ are as defined above, and $R^{102}$ is hydrogen atom or a lower alkyl, provided that in the compound (1jjj), the groups of the formulae: -NH-$R^{102}$ and -OH are substituted at the positions adjacent each other.

The reaction of the compound (1jjj) and the compound (72) can be carried out under the same conditions as in the reaction of the compound (7) and the compound (8) in the above Reaction Scheme-4.

[Reaction Scheme-53]

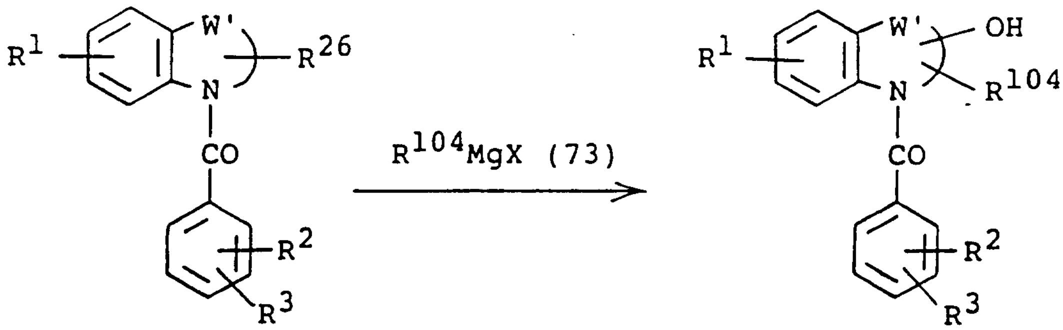


(1A) (1ℓℓℓ)

wherein $R^1$, $R^2$, $R^3$, W', $R^{26}$ and X are as defined above, and $R^{104}$ is a lower alkyl.

The reaction of the compound (1A) and the compound (73) can be carried out in an appropriate solvent. The solvent includes, for example, ethers (diethyl ether, dioxane, tetrahydrofuran), aromatic hydrocarbons (e.g. benzene, toluene, xylene), saturated hydrocarbons (e.g. pentane, hexane, heptane, cyclohexane), or a mixture of these solvents. The reaction is usually carried out at a temperature of from -70°C to 50°C, preferably from -30°C to room temperature, for 1 to 6 hours. The compound (73) is used in an amount of at least 1 mole, preferably 1 to 5 moles, to 1 mole of the compound (1A).

[Reaction Scheme-54]

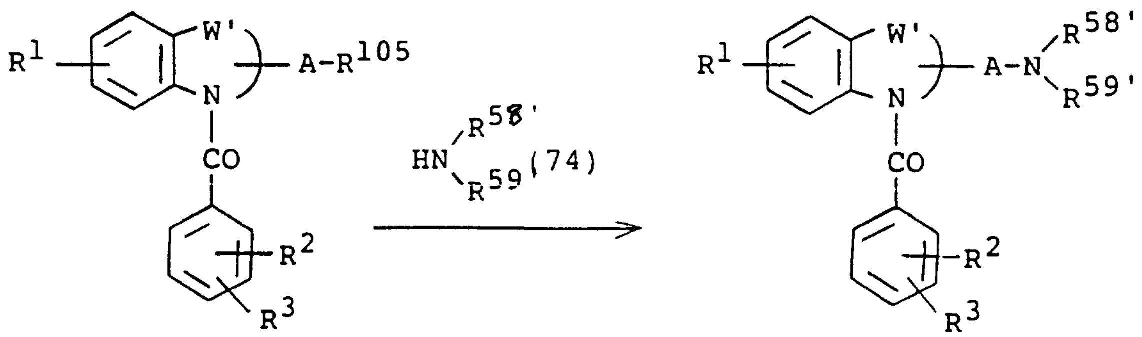


(1mmm) (1nnn)

wherein $R^1$, $R^2$, $R^3$, W', $R^{58'}$, $R^{59'}$, and A are as defined above, and $R^{105}$ is a lower alkylsulfonyloxy.

The reaction of the compound (1mmm) and the compound (74) can be carried out under the same conditions as in the reaction of the compound (7) and the compound (8) in the above Reaction Scheme-4.

Among the active compounds (1) of this invention, the compounds having an acidic group can easily be converted into salts by treating with a pharmaceutically acceptable basic compound. The basic compound includes, for example, metal hydroxides such as sodium hydroxide, potassium hydroxide, lithium hydroxide, calcium hydroxide, alkali metal carbonates or hydrogen carbonates such as sodium carbonate, sodium hydrogen carbonate, alkali metal alcoholates such as sodium methylate, potassium ethylate. Besides, among the active compounds (1) of this invention, the compounds having a basic group can easily be converted into acid addition salts thereof by treating with a pharmaceutically acceptable acid. The acid includes, for example, inorganic acids such as sulfuric acid, nitric acid, hydrochloric acid, hydrobromic acid, and organic acids such as acetic acid, p-toluenesulfonic acid, ethanesulfonic acid, oxalic acid, maleic

acid, fumaric acid, citric acid, succinic acid, benzoic acid. These salts are useful as an active ingredient as like as the compounds (1) in the free form.

In addition, the compounds (1) of this invention include stereoisomers and optical isomers, and these isomers are also useful as the active ingredient in this invention.

The compounds of this invention thus obtained can easily be isolated and purified by conventional isolation methods. The isolation methods are, for example, distillation method, recrystallization method, column chromatography, ion exchange chromatography, gel chromatography, affinity chromtography, preparative thin layer chromatography and extraction with a solvent.

The compounds and their salts of this invention are useful as a vasopressin antagonist and are used in the form of a conventional pharmaceutical preparation. The preparation is prepared by using conventional dilutents or carriers such as fillers, thickening agents, binders, wetting agents, disintegrators, surfactants and lubricants. The pharmaceutical preparations may be selected from various forms in accordance with the desired utilities, and the representative forms are tablets, pills, powders, solutions, suspensions, emulsions, granules, capsules, suppositorles and injections (solutions, suspensions,). In order to form in tablets, there are used carriers such as vehicles (e.g. lactose, white sugar, sodium chloride, glucose, urea, starches, calcium carbonate, kaolin, crystalline cellulose, silicic acid), binders (e.g. water, ethanol, propanol, simple syrup, glucose solution, starch solution, gelatin solution, carboxymethyl cellulose, shellac, methyl cellulose, potassium phosphate, polyvinylpyrrolidone), disintegrators (e.g. dry starch, sodium arginate, agar powder, laminaran powder, sodium hydrogen carbonate, calcium carbonate, polyoxyethylene sorbitan fatty acid esters, sodium laurylsulfate, stearic monoglyceride, starches, lactose), disintegration inhibitors (e.g. white sugar, stearin, cacao butter, hydrogenated oils), absorption promoters (e.g. quaternary ammonium base, sodium laurylsulfate), wetting agents (e.g. glycerin, starches), adsorbents (e.g. starches, lactose, kaolin, bentonite, colloidal silicates), and lubricants (e.g. purified talc, stearates, boric acid powder, polyethylene glycol, etc.). Moreover, the tablets may also be in the form of a conventional coated tablet, such as sugar-coated tablets, gelatin-coated tablets, enteric coated tablets, film coating tablets, or double or multiple layer tablets. In the preparation of pills, the carriers include vehicles (e.g. glucose, lactose, starches, cacao butter, hydrogenated vegetable oils, kaolin, talc), binders (e.g. gum arabic powder, tragacanth powder, gelatin, ethanol), disintegrators (e.g. laminaran, agar), and the like. In the preparation of suppositories, the carriers include, for example, polyethylene glycol, cacao butter, higher alcohols, higher alcohol esters, gelatin and semi-synthetic glycerides. Capsules can be prepared by charging a mixture of the compound of this invention with the above carriers into hard gelatin capsules or soft capsules in a usual manner. In the preparation of injections, the solutions, emulsions or suspendions are sterilized and are preferably made isotonic with the blood. In the preparation of these solutions, emulsions and suspensions, there are used conventional diluents, such as water, ethyl alcohol, macrogol (propylene glycol), ethoxylated isostearyl alcohol, polyoxylated isostearyl alcohol and polyoxyethylene sorbitan fatty acid esters. In this case, the pharmaceutical preparations may also be incorporated with sodium chloride, glucose, or glycerin in an amount sufficient to make them isotonic, and may also be incorporated with conventional solubilizers, buffers, anesthetizing agents. Besides, the pharmaceutical preparations may optionally be incorporated with coloring agents, preservatives, perfumes, flavors, sweeting agents, and other medicaments, if required.

The amount of the active compound of this invention (active ingredient) to be incorporated into the anti-vasopressin preparations is not specified but may be selected from a broad range, but usually, it is preferably in the range of 1 to 70 % by weight, more preferably 5 to 50 % by weight.

The anti-vasopressin preparation of this invention may be administered in any method, and suitable method for administration may be determined in accordance with various forms of preparation, ages, sexes and other conditions of the patients and the degree of severity of diseases. For instance, tablets, pills, solutions, suspensions, emulsions, granules and capsules are administered orally. The injections are intraveneously administered alone or together with a conventional auxiliary liquid (e.g. glucose, amino acid solutions), and further are optionally administered alone in intramuscular, intracutaneous, subcutaneous, or intraperitoneal route, if required. Suppositories are administered in intrarectal route.

The dosage of the anti-vasopressin agent of this invention may be selected in accordance with the usage, ages, sexes and other conditions of the patients and the degree of severity of the diseases, but is usually in the range of 0.6 to 50 mg of the active compound of this invention per 1 kg of body weight of the patient per day. The active compound is preferably contained in an amount of 10 to 1000 mg per the dosage unit.

Brief Description of Drawing

Fig. 1 to Fig. 4 show a chart of NMR ($CDCl_3$) of the compounds in Examples 978 and 979.

Best Mode for Carrying Out the Invention

The present invention is illustrated by the following Preparations of anti-vasopressin agent, Reference Examples of processes for preparing the starting compounds to be used for preparing the active compounds, Examples of processes for preparing the active compounds, and Experiments of the activities of the active compounds of this invention.

Preparation 1

Film coated tablets are prepared from the following components.

| Components | Amount |
|---|---|
| 4-Methylamino-1-[4-(3,5-dichlorobenzoylamino)benzoyl]-1,2,3,4-tetrahydroquinoline | 150 g |
| Avicel (tradename of microcrystalline cellulose, manufactured by Asahi Chemical Industry Co., Ltd., Japan) | 40 g |
| Corn starch | 30 g |
| Magnesium stearate | 2 g |
| Hydroxypropyl methylcellulose | 10 g |
| Polyethylene glycol-6000 | 3 g |
| Castor oil | 40 g |
| Ethanol | 40 g |

The active component of this invention, Avicel, corn starch and magnesium stearate are mixed and kneaded and the mixture is tabletted using a conventional pounder (R 10 mm) for sugar coating. The tablets thus obtained are coated with a film coating agent consisting of hydroxypropyl methylcellulose, polyethylene glycol-6000, castor oil and ethanol to give film coated tablets.

Preparation 2

Tablets are prepared from the following components.

| Components | Amount |
|---|---|
| 1-[4-(N-Butylanilinoacetylamino)benzoyl]-2,3,4,5-tetrahydroy-1H-benzazepine | 150 g |
| Citric acid | 1.0 g |
| Lactose | 33.5 g |
| Dicalcium phosphate | 70.0 g |
| Pullonic F-68 | 30.0 g |
| Sodium laurylsulfate | 15.0 g |
| Polyvinylpyrrolidone | 15.0 g |
| Polyethylene glycol (Carbowax 1500) | 4.5 g |
| Polyethylene glycol (Carbowax 6000) | 45.0 g |
| Corn starch | 30.0 g |
| Dry sodium stearate | 3.0 g |
| Dry magnesium stearate | 3.0 g |
| Ethanol | q.s. |

The active compound of this invention, citric acid, lactose, dicalcium phosphate, Pullonic F-68 and sodium laurylstearate are mixed. The mixture is screened with No. 60 screen and is granulated with an alcohol solution containing polyvinylpyrrolidone, carbowax 1500 and 6000. If required, an alcohol is added thereto so that the powder mixture is made a paste-like mass. Corn starch is added to the mixture and the mixture is continuously mixed to form uniform particles. The resulting particles are passed through No. 10 screen and entered into a tray and then dried in an oven at 100°C for 12 to 14 hours. The dried particles are screened with No. 16 screen and thereto are added dry sodium laurylsulfate and dry magnesium stearate, and the mixture is tabletted to form the desired shape.

The core tablets thus prepared are vanished and dusted with talc in order to guard from wetting. Undercoating is applied to the core tablets. In order to administer the tablets orally, the core tablets are vanished several times. In order to give round shape and smooth surface to the tablets, further undercoating and coating with lubricant are applied thereto. The tablets are further coated with a coloring coating material until the desired colored tablets are obtained. After drying, the coated tablets are polished to obtain the desired tablets having uniform gloss.

Preparation 3

An injection preparation is prepared from the following components.

| Components | Amount |
|---|---|
| 4-Methyl-1-[4-(2,3-dimethylbenzoylamino)benzoyl]-2,3,4,5-tetrahydro-1H-1,4-benzodiazepine | 5 g |
| Polyethylene glycol (molecular weight: 4000) | 0.3 g |
| Sodium chloride | 0.9 g |
| Polyoxyethylene sorbitan monooleate | 0.4 g |
| Sodium metabisulfite | 0.1 g |
| Methyl-paraben | 0.18 g |
| Propyl-paraben | 0.02 g |
| Distilled water for injection | 10.0 ml |

The above parabens, sodium metabisulfite and sodium chloride are dissolved in distilled water of half volume of the above with stirring at 80°C. The solution thus obtained is cooled to 40°C, and the active compound of this invention and further polyethylene glycol and polyoxyethylene sorbitan monooleate are dissolved in the above solution. To the solution is added distilled water for injection to adjust to the desired volume, and the solution is sterilized by filtering with an appropriate filter paper to give an injection preparation.

Reference Example 1

To a solution of 1,2,3,4-tetrahydroquinoline (28.7 g) in acetone (400 ml) and water (200 ml) is added potassium carbonate (38.8 g), and thereto is added p-nitrobenzoyl chloride (40 g) under ice-cooling and the mixture is stirred at room temperature overnight. To the reaction mixture is added a suitable amount of water. The precipitated crystal is collected by filtration and dried to give 1-(4-nitrobenzoyl)-1,2,3,4-tetrahydroquinoline (40.8 g) as white powder, m.p. 86 - 88°C.

Reference Example 2

To a solution of 10 % Pd-C (5 g) in ethanol (500 ml) is added 1-(4-nitrobenzoyl)-1,2,3,4-tetrahydroquinoline (53.4 g) and the mixture is subjected to catalytic reduction at ordinary temperature under atmospheric pressure of hydrogen. After the reduction, 10 % Pd-C is removed by filtration, and the filtrate is concentrated under reduced pressure to give 1-(4-aminobenzoyl)-1,2,3,4-tetrahydroquinoline (46.7 g) as yellow powder, m.p. 185 - 188°C.

Reference Example 3

Using the suitable starting materials, the following compounds are obtained in the same manner as in Reference Example 1.

1-(3-Nitrobenzoyl)-1,2,3,4-tetrahydroquinoline, white powder, m.p. 134 - 136°C

1-(2-Nitrobenzoyl)-1,2,3,4-tetrahydroquinoline, yellow powder, m.p. 152 - 154°C

3-Methyl-1-(4-nitrobenzoyl)-1,2,3,4-tetrahydroquinoline, yellow powder, m.p. 109 - 110°C

4-Methyl-1-(4-nitrobenzoyl)-1,2,3,4-tetrahydroquinoline, yellow powder, m.p. 134 - 136°C

2-Methyl-1-(4-nitrobenzoyl)-1,2,3,4-tetrahydroquinoline, yellow powder, m.p. 143 - 145°C

1-(4-Nitrobenzoyl)-2,3,4,5-tetrahydro-1H-benzazepine, yellow powder, m.p. 143 - 145°C

1-(3-Methyl-4-nitrobenzoyl)-2,3,4,5-tetrahydro-1H-benzazepine, white powder, m.p. 100 - 102°C

1-(3-Methoxy-4-nitrobenzoyl)-2,3,4,5-tetrahydro-1H-benzazepine, yellow powder, m.p. 146 - 148°C

1-(4-Nitrobenzoyl)-1,2,3,4,5,6-hexahydrobenzazocine, white powder, m.p. 83 - 85°C

1-(4-Nitrobenzoyl)-3,4-dihydro-2H-1,4-benzoxazine, yellow powder, m.p. 167 - 169°C

1-(4-Nitrobenzoyl)-1,2,3,5-tetrahydro-4,1-benzoxazepine, yellow powder, m.p. 196 - 198°C

1-(4-Nitrobenzoyl)-4-methyl-1,2,3,4-tetrahydroquinoxaline, brown powder

$^1$H-NMR ($CDCl_3$) δ : 3.03 (3H, s), 3.54 (2H, t, J=5.7 Hz), 4.06 (2H, t, J=5.7 Hz), 6.2-6.5 (2H, m), 6.70 (1H, d, J=8.2 Hz), 6.9-7.1 (1H, m), 7.54 (2H, d, J=8.8 Hz), 8.13 (2H, d, J = 8.8 Hz)

1-(4-Nitrobenzoyl)-5-methyl-2,3,4,5-tetrahydro-1H-1,5-benzodiazepine, yellow oil

$^1$H-NMR ($CDCl_3$) δ : 1.7-2.0 (1H, m), 2.0-2.3 (1H, m), 2.8-3.0 (1H, m), 2.98 (3H, s), 3.0-3.2 (1H, m), 3.4-3.6 (1H, m), 4.6-4.8 (1H, m), 6.5-6.7 (2H, m), 6.94 (1H, d, J=8.1 Hz), 7.1-7.2 (1H, m), 7.33 (2H, d, J=8.9 Hz), 7.97 (2H, d, J=8.9 Hz)

1-(4-Nitrobenzoyl)-4-methyl-2,3,4,5-tetrahydro-1H-1,4-benzodiazepine, brown oil

$^1$H-NMR ($CDCl_3$) δ : 2.44 (3H, s), 3.0-3.3 (3H, m), 3.77 (1H, d, J=13.7 Hz), 4.06 (1H, d, J=13.6 Hz), 4.9-5.1 (1H, m), 6.59 (1H, d, J=7.7 Hz), 6.97 (1H, t, J=7.6 Hz), 7.15 (1H, t, J=7.4 Hz), 7.2-7.5 (3H, m), 8.03 (2H, d, J=8.8 Hz)

1-(3-Methoxy-4-nitrobenzoyl)-4-methyl-2,3,4,5-retrahydro-1H-1,4-benzodiazepine, yellow powder, m.p. 146 - 148°C

1-(4-Nitrobenzoyl)-4-n-propyl-2,3,4,5-tetrahydro-1H-1,4-benzodiazepine, yellow powder, m.p. 131 - 133°C

1-(4-Nitrobenzoyl)-5-chloro-1,2,3,4-tetrahydroquinoline, white powder, m.p. 134 - 136°C

1-(4-Nitrobenzoyl)-6-methoxy-1,2,3,4-tetrahydroquinoline, yellow powder, m.p. 149 - 151°C

1-(4-Nitrobenzoyl)-6-methyl-1,2,3,4-tetrahydroquinoline, yellow powder, m.p. 109 - 110°C

1-(4-Nitrobenzoyl)-7-methoxy-1,2,3,4-tetrahydroquinoline, yellow powder, m.p. 139 - 141°C

1-(4-Nitrobenzoyl)-3-(4-methyl-1-piperazinyl)-1,2,3,4-tetrahydroquinoline, yellow amorphous

$^1$H-NMR ($CDCl_3$) δ : 2.29 (3H, s), 2.35-3.20 (11H, m), 3.86-4.15 (2H, m), 6.48-6.63 (1H, m), 6.89 (1H, t, J=7.4 Hz), 7.05 (1H, t, J=7.4 Hz), 7.22 (1H, d, J=7.4 Hz), 7.52 (2H, d, J=8.8 Hz), 8.11 (2H, d, J=8.8 Hz)

1-(4-Nitrobenzoyl)-3-(1-pyrrolidinyl)-1,2,3,4-tetrahydroquinoline, yellow amorphous

$^1$H-NMR ($CDCl_3$) δ : 1.70-1.95 (4H, m), 2.52-3.30 (7H, m), 3.80-4.22 (2H, m), 6.52 (1H, brs), 6.88 (1H, t, J=7.6 Hz), 6.96-7.11 (1H, m), 7.20 (2H, d, J=7.6 Hz), 7.54 (2H, d, J=8.8 Hz), 8.12 (2H, d, J=8.8 Hz)

1-(4-Nitrobenzoyl)-4-oxo-1,2,3,4-tetrahydroquinoline, yellow powder, m.p. 189 - 190°C

1-(4-Nitrobenzoyl)-3-hydroxymethyl-1,2,3,4-tetrahydroquinoline, yellow powder, m.p. 97 - 100°C

1-(4-Nitrobenzoyl)-3-ethoxycarbonyl-1,2,3,4-tetrahydroquinoline, pale yellow powder, m.p. 162 - 163°C

1-(4-Nitrobenzoyl)-4-dimethylamino-1,2,3,4-tetrahydroquinoline, light brown oil

$^1$H-NMR ($CDCl_3$) δ : 1.80-2.02 (1H, m), 2.20-2.50 (7H, m), 3.47 (1H, t, J=4.9 Hz), 3.70-3.88 (1H, m), 4.06-4.25 (1H, m), 6.46 (1H, d, J=7.5 Hz), 6.89 (1H, t, J=7.5 Hz), 7.05 (1H, t, J=7.5 Hz), 7.34 (1H, d, J=7.5 Hz), 7.50 (2H, d, J=7.0 Hz), 8.10 (2H, d, J=7.0 Hz)

Reference Example 4

Using the suitable starting materials, the following compounds are obtained in the same manner as in Reference Example 2.

1-(3-Aminobenzoyl)-1,2,3,4-tetrahydroquinoline, white powder, m.p. 128 - 130°C

1-(2-Aminobenzoyl)-1,2,3,4-tetrahydroquinoline, yellow powder

$^1$H-NMR ($CDCl_3$) δ : 2.01 (2H, quint, J=6.6 Hz), 2.81 (2H, t, J=6.6 Hz), 3.86 (2H, t, J=6.4 Hz), 4.6-4.8 (2H, m), 6.43 (1H, t, J=7 Hz), 6.66 (1H, d, J=8 Hz), 6.79 (1H, dd, J=1.4 Hz, J=7.6 Hz), 6.8-7.2 (5H, m)

3-Methyl-1-(4-aminobenzoyl)-1,2,3,4-tetrahydroquinoline, yellow powder, m.p. 197 - 200°C

4-Methyl-1-(4-aminobenzoyl)-1,2,3,4-tetrahydroquinoline, yellow powder, m.p. 197 - 199°C

2-Methyl-1-(4-aminobenzoyl)-1,2,3,4-tetrahydroquinoline, yellow powder, m.p. 204 - 206°C

1-(4-Aminobenzoyl)-2,3,4,5-tetrahydro-1H-benzazepine, yellow powder, m.p. 172 - 174°C

1-(3-Methyl-4-aminobenzoyl)-2,3,4,5-tetrahydro-1H-benzazepine, white powder, m.p. 156 - 158°C

1-(3-Methoxy-4-aminobenzoyl)-2,3,4,5-tetrahydro-1H-benzazepine, white powder, m.p. 165 - 167°C

1-(4-Aminobenzoyl)-1,2,3,4,5,6-hexahydrobenzazocine, white powder, m.p. 177 - 179°C

1-(4-Aminobenzoyl)-3,4-dihydro-2H-1,4-benzoxazine, white powder, m.p. 192 - 194°C

1-(4-Aminobenzoyl)-1,2,3,5-tetrahydro-4,1-benzoxazepine, yellow powder, m.p. 196 - 198°C

1-(4-Aminobenzoyl)-4-methyl-1,2,3,4-tetrahydroquinoxaline, yellow powder, m.p. 210 - 212°C

1-(4-Aminobenzoyl)-5-methyl-2,3,4,5-tetrahydro-1H-1,5-benzodiazepine, white powder, m.p. 159 - 161°C

1-(4-Aminobenzoyl)-4-methyl-2,3,4,5-tetrahydro-1H-1,4-benzodiazepine, brown powder, m.p. 169 - 171°C

1-(3-Methoxy-4-aminobenzoyl)-4-methyl-2,3,4,5-tetrahydro-1H-1,4-benzodiazepine, yellow oil

$^1$H-NMR ($CDCl_3$) δ : 2.41 (3H, s), 2.9-3.2 (3H, m), 3.61 (3H, s), 3.6-4.2 (4H, m), 4.8-5.2 (1H, m), 6.38 (1H, d, J=8.1 Hz), 6.6-6.8 (3H, m), 6.9-7.2 (2H, m), 7.2-7.4 (1H, m)

1-(4-Aminobenzoyl)-4-n-propyl-2,3,4,5-tetrahydro-1H-1,4-benzazepine, brown powder, m.p. 151 - 153°C

1-(4-Aminobenzoyl)-5-chloro-1,2,3,4-tetrahydroquinoline, white powder, m.p. 174 - 175°C

1-(4-Aminobenzoyl)-6-methoxy-1,2,3,4-tetrahydroquinoline, pale yellow powder, m.p. 159 - 160°C

1-(4-Aminobenzoyl)-6-methyl-1,2,3,4-tetrahydroquinoline, white powder, m.p. 145 - 146°C

1-(4-Aminobenzoyl)-7-methoxy-1,2,3,4-tetrahydroquinoline, pale yellow powder, m.p. 150 - 152 °C

1-(4-Aminobenzoyl)-3-(4-methyl-1-piperazinyl)-1,2,3,4-tetrahydroquinoline, light beige powder, m.p. 157 - 159°C

1-(4-Aminobenzoyl)-3-(1-pyrrolidinyl)-1,2,3,4-tetrahydroquinoline, pale yellow powder, m.p. 173 - 174.5°C

1-(4-Aminobenzoyl)-2,3-dihydro-4(1H)-quinolinone, pale yellow powder, m.p. 178 - 180°C

1-(4-Aminobenzoyl)-3-hydroxymethyl-1,2,3,4-tetrahydroquinoline, white powder, m.p. 179 - 181°C

1-(4-Aminobenzoyl)-3-ethoxycarbonyl-1,2,3,4-tetrahydroquinoline, pale yellow amorphous

$^1$H-NMR ($CDCl_3$) δ : 1.21 (3H, t, J=7.1 Hz), 3.00-3.24 (3H, m), 3.70-4.30 (6H, m), 6.48 (2H, d, J=8.5 Hz), 6.69 (1H, d, J=7.9 Hz), 6.77-7.30 (5H, m)

1-(4-Aminobenzoyl)-4-dimethylamino-1,2,3,4-tetrahydroquinoline, brown oil

$^1$H-NMR ($CDCl_3$) δ : 1.83-2.05 (1H, m), 2.13-2.30 (1H, m), 2.34 (6H, m), 3.55-3.83 (2H, m), 3.89 (1H, brs), 3.97-4.18 (1H, m), 6.47 (2H, d, J=7.0 Hz), 6.68 (1H, d, J=7.9 Hz), 6.85-7.05 (2H, m), 7.20 (2H, d, J=7.0 Hz), 7.37 (1H, d, J=7.4 Hz)

Reference Example 5

To terephthalic acid monomethyl ester (15 g) is added thionyl chloride (100 ml) and the mixture is refluxed for 2 hours. The thionyl chloride is distilled off under reduced pressure to give terephthalic acid chloride monomethyl ester. Separately, to in solution of 1,2,3,4-tetrahydroquinoline (14.4 g) in dichloromethane (200 ml) is added triethylamine (16.9 g) and further thereto is added slowly terephthalic acid chloride monomethyl ester obtained above under ice-cooling. Then, the mixture is stirred at room temperature for 1 hour. After completion of the reaction, water is added to the reaction mixture. The mixture is extracted with dichloromethane and dried over magnesium sulfate. The solvent is distilled off under reduced pressure and the resulting residue is purified by silica gel column chromatography (eluent; dichloromethane) to give 1-(4-methoxycarbonylbenzoyl)-1,2,3,4-tetrahydroquinoline (22.7 g) as white powder, m.p. 72 - 74°C.

Reference Example 6

To a solution of 1-(4-methoxycarbonylbenzoyl)-1,2,3,4-tetrahydroquinoline (22.7 g) in methanol (300 ml) is added 5 % aqueous sodium hydroxide solution (150 ml) and the mixture is refluxed for 2 hours. Methanol is distilled off under reduced pressure and the resulting residue is acidified with diluted hydrochloric acid, extracted with diethyl ether, and dried over magnesium sulfate. The solvent is distilled off under reduced pressure and the resulting crystal is collected by filtration to give 1-(4-carboxybenzoyl)-1,2,3,4-tetrahydroquinoline (13.2 g) as white powder, m.p. 181 - 183°C.

Reference Example 7

Using the suitable starting materials, the following compounds are obtained in the same manner as in Reference Example 1.

5-Dimethylamino-1-(4-nitrobenzoyl)-2,3,4,5-tetrahydro-1H-benzazepine, pale yellow powder, m.p. 139 - 142°C

5-Dimethylamino-1-(3-methoxy-4-nitrobenzoyl)-2,3,4,5-tetrahydro-1H-benzazepine, white powder, m.p. 139 - 141°C

4-(N-Methyl-N-ethylamino)-1-(4-nitrobenzoyl)-1,2,3,4-tetrahydroquinoline, pale yellow oil

$^1$H-NMR ($CDCl_3$) δ : 1.11 (3H, t, J=7.1 Hz), 1.90-2.25 (2H, m), 2.30 (3H, s), 2.57 (2H, q, J=7.1 Hz), 3.55-3.85 (2H, m), 4.00-4.21 (1H, m), 6.35-6.60 (1H, m), 6.80-6.98 (1H, t, J=7.9 Hz), 7.00-7.15 (1H, m), 7.33-7.60 (3H, m), 8.10 (2H, d, J=8.8 Hz)

4-Dimethylamino-1-(3-methoxy-4-nitrobenzoyl)-1,2,3,4-tetrahydroquinoline, brown oil

$^1$H-NMR ($CDCl_3$) δ : 1.80-2.05 (1H, m), 2.33 (6H, s), 2.30-2.50 (1H, m), 3.40-3.52 (1H, m), 3.78 (3H, s), 3.70-3.88 (1H, m), 4.04-4.24 (1H, m), 6.52 (1H, d, J=8.2 Hz), 6.85-7.13 (4H, m), 7.28-7.38 (1H, m), 7.71 (1H, d, J=8.2 Hz)

1-(4-Nitrobenzoyl)-4-ethyl-2,3,4,5-tetrahydro-1H-1,4-benzodiazepine, yellow oil

$^1$H-NMR ($CDCl_3$) δ : 1.16 (3H, t, J=7.1 Hz), 2.5-2.7 (2H, m), 3.0-3.3 (3H, m), 3.98 (2H, q, J=14 Hz), 4.8-5.0 (1H, m), 6.59 (1H, d, J=7.7 Hz), 6.96 (1H, t, J=7.7 Hz), 7.14 (1H, t, J=7.4 Hz), 7.2-7.4 (3H, m), 8.02 (2H, d, J=8.8 Hz)

1-(4-Nitrobenzoyl)-4-isopropyl-2,3,4,5-tetrahydro-1H-1,4-benzodiazepine, yellow powder, m.p. 222 - 223°C

1-(4-Nitrobenzoyl)-4-cyclohexyl-2,3,4,5-tetrahydro-1H-1,4-benzodiazepine, brown oil

$^1H$-NMR ($CDCl_3$) δ : 1.0-1.5 (5H, m), 1.5-2.1 (5H, m), 2.4-2.7 (1H, m), 2.9-3.3 (3H, m), 3.94 (2H, s), 4.9-5.1 (1H, m), 6.57 (1H, d, J=7.7 Hz), 6.8-7.0 (1H, m), 7.0-7.2 (1H, m), 7.2-7.4 (3H, m), 8.01 (2H, d, J=8.8 Hz)

1-(4-Nitrobenzoyl)-5-methyl-1,2,3,4,5,6-hexahydro-1,5-benzodiazocine, yellow oil

$^1H$-NMR ($CDCl_3$) δ : 1.5-2.1 (2H, m), 2.40 (3H, s), 2.3-2.6 (1H, m), 2.8-3.2 (2H, m), 3.50 (1H, d, J=13.4 Hz), 3.84 (1H, d, J=13.4 Hz), 4.8-5.0 (1H, m), 7.0-7.3 (4H, m), 7.41 (2H, d, J=8.9 Hz), 8.00 (2H, d, J=8.9 Hz)

1-(4-Nitrobenzoyl)-1,2,3,4-tetrahydro-5,1-benzoxazepine, white powder, m.p. 144.5 - 145.5°C

1-(2-Nitrobenzoyl)-4-methyl-2,3,4,5-tetrahydro-1H-1,4-benzodiazepine, yellow powder, m.p. 177 - 180°C

1-(3-Nitrobenzoyl)-4-methyl-2,3,4,5-tetrahydro-1H-1,4-benzodiazepine, yellow powder, m.p. 145 - 146°C

6-Fluoro-1-(4-nitrobenzoyl)-1,2,3,4-tetrahydroquinoline, yellow needles, m.p. 145 - 146°C

Reference Example 8

Using the suitable starting materials, the following compounds are obtained in the same manner as in Reference Example 2.

5-Dimethylamino-1-(4-aminobenzoyl)-2,3,4,5-tetrahydro-1H-benzazepine, white powder, m.p. 120 - 122°C

5-Dimethylamino-1-(3-methoxy-4-amino)-2,3,4,5-tetrahydro-1H-benzazepine, white powder, m.p. 121 - 123°C

4-(N-Methy-N-ethylamino)-1-(4-aminobenzoyl)-1,2,3,4-tetrahydroquinoline, orange amorphous

$^1H$-NMR ($CDCl_3$) δ : 1.11 (3H, t, J=7.1 Hz), 1.90-2.20 (2H, m), 2.28 (3H, s), 2.26 (2H, q, J=7.1 Hz), 3.60-4.25 (5H, m), 6.48 (2H, d, J=8.5 Hz), 6.69 (1H, d, J=7.9 Hz), 6.80-7.05 (2H, m), 7.24 (2H, d, J=8.5 Hz), 7.46 (1H, d, J=6.2 Hz)

4-Dimethylamino-1-(3-methoxy-4-aminobenzoyl)-1,2,3,4-tetrahydroquinoline, pale yellow amorphous

$^1H$-NMR ($CDCl_3$) δ : 1.83-2.04 (1H, m), 2.15-2.32 (1H, m), 2.33 (6H, s), 3.50-3.82 (2H, m), 3.64 (3H, s), 3.95-4.18 (3H, m), 6.50 (1H, d, J=7.9 Hz), 6.65 (1H, dd, J=7.9 Hz, 1.1 Hz), 6.78-7.03 (4H, m), 7.34 (1H, dd, J=7.5 Hz, 1.5 Hz)

1-(4-Aminobenzoyl)-4-ethyl-2,3,4,5-tetrahydro-1H-1,4-benzodiazepine, white powder, m.p. 186 - 188°C

1-(4-Aminobenzoyl)-4-isopropyl-2,3,4,5-tetrahydro-1H-1,4-benzodiazepine, white powder, m.p. 191 - 192°C

1-(4-Aminobenzoyl)-4-cyclohexyl-2,3,4,5-tetrahydro-1H-1,4-benzodiazepine, white powder, m.p. 149.5 - 150.5°C

1-(4-Aminobenzoyl)-5-methyl-1,2,3,4,5,6-hexahydro-1,5-benzodiazocine, yellow powder, m.p. 143 - 145°C

1-(4-Aminobenzoyl)-1,2,3,4-tetrahydro-5,1-benzoxazepine, yellow powder, m.p. 163.5 - 164.5°C

1-(2-Aminobenzoyl)-4-methyl-2,3,4,5-tetrahydro-1H-1,4-benzodiazepine, yellow powder, m.p. 144 - 146°C

1-(3-Aminobenzoyl)-4-methyl-2,3,4,5-tetrahydro-1H-1,4-benzodiazepine, white powder, mp. 153 - 155°C

6-Fluoro-1-(4-aminobenzoyl)-1,2,3,4-tetrahydroquinoline, white powder, m.p. 160.5 - 161.5°C

Reference Example 9

Using the suitable starting materials, the following compounds are obtained in the same manner as in Reference Example 1.

1-(2-Chloro-4-nitrobenzoyl)-4-methyl-2,3,4,5-tetrahydro-1H-1,4-benzodiazepine

$^1H$-NMR ($CDCl_3$) δ : 2.40 (3H, s), 2.96-3.33 (3H, m), 3.60-3.79 (1H, m), 3.96-4.23 (1H, m), 4.70-4.91 (1H, m), 6.80-7.43 (5H, m), 7.80-7.99 (1H, m), 8.08-8.21 (1H, m)

1-(3-Methyl-4-nitrobenzoyl)-4-methyl-2,3,4,5-tetrahydro-1H-1,4-benzodiazepine

$^1H$-NMR ($CDCl_3$) δ : 2.43 (3H, s), 2.48 (3H, s), 2.92-3.28 (3H, m), 3.91 (2H, AB-q, J=13.9 Hz, 45.5 Hz), 4.77-5.01 (1H, m), 6.54-6.70 (1H, m), 6.88-7.37 (5H, m), 7.62-7.78 (1H, m)

5-Dimethylamino-1-(2-chloro-4-nitrobenzoyl)-2,3,4,5-tetrahydro-1H-benzazepine

$^1H$-NMR ($CDCl_3$) δ : 1.23-2.57 (10H, m), 2.68-5.15 (3H, m), 6.79-7.45 (4H, m), 7.49-8.39 (3H, m)

5-Oxo-1-(4-nitrobenzoyl)-2,3,4,5-tetrahydro-1H-benzazepine, white powder (ethyl acetate/n-hexane), m.p. 147 - 148°C

5-Hydroxy-1-(4-nitrobenzoyl)-2,3,4,5-tetrahydro-1H-benzazepine, white powder (ethyl acetate/n-hexane), m.p. 148 - 150°C

5-Methoxy-1-(4-nitrobenzoyl)-2,3,4,5-tetrahydro-1H-benzazepine, colorless amorphous

$^1H$-NMR ($CDCl_3$) δ : 1.47-2.48 (4H, m), 2.70-3.10 (1H, m), 3.26-3.64 (3H, m), 4.29-5.12 (2H, m), 6.60 (1H, d, J=7.7 Hz), 6.88-7.67 (5H, m), 7.92-8.12 (2H, m)

5-Ethoxycarbonylmethoxy-1-(4-nitrobenzoyl)-2,3,4,5-tetrahydro-1H-benzazepine, white powder, m.p. 107 - 108°C (recrystallized from ethyl acetate/n-hexane)

5-(4-Bromobutoxy)-1-(4-nitrobenzoyl)-2,3,4,5-tetrahydro-1H-benzazepine, colorless oil

$^1H$-NMR ($CDCl_3$) δ : 1.49-2.55 (8H, m), 2.72-3.07 (1H, m), 3.24-3.77 (4H, m), 4.40-5.15 (2H, m), 6.53-6.66 (1H, m), 6.91-7.06 (1H, m), 7.07-7.80 (4H, m), 7.94-8.13 (2H, m)

5-(4-Dimethylaminobutoxy)-1-(4-nitrobenzoyl)-2,3,4,5-tetrahydro-1H-benzazepine, colorless oil

$^1H$-NMR ($CDCl_3$) δ : 1.51-1.88 (6H, m), 2.23-2.61 (4H, m), 2.27 (3H, s), 2.35 (3H, s), 2.74-3.14 (1H, m), 3.55-3.77 (2H, m), 4.48-5.11 (2H, m), 6.54-6.66 (1H, m), 6.91-7.04 (1H, m), 7.06-7.80 (4H, m), 7.93-8.11 (2H, m)

5-[3-(Phthalimid-1-yl)propoxy-1-(4-nitrobenzoyl)-2,3,4,5-tetrahydro-1H-benzazepine, colorless amorphous

$^1$H-NMR ($CDCl_3$) δ : 1.48-2.56 (6H, m), 2.71-3.05 (1H, m), 3.40-4.05 (4H, m), 4.47-5.11 (2H, m), 6.50-6.64 (1H, m), 6.84-7.03 (1H, m), 7.03-7.20 (1H, m), 7.20-7.57 (2H, m), 7.57-7.93 (5H, m), 7.97-8.20 (2H, m)

5-Chloro-1-(4-nitrobenzoyl)-2,3,4,5-tetrahydro-1H-benzazepine, light brown powder

$^1$H-NMR ($CDCl_3$) δ : 1.75-3.3 (4H, m), 4.6-6.25 (3H, m), 6.45-6.7 (1H, m), 6.8-7.5 (4H, m), 7.55-7.7 (1H, m), 7.9-8.1 (2H, m)

5-Oxo-1-(2-chloro-4-nitorobenzoyl)-2,3,4,5-tetrahydro-1H-benzazepine, pale yellow amorphous

$^1$H-NMR ($CDCl_3$) δ : 1.95-2.45 (2H, m), 2.94 (1H, t, J=6 Hz), 3.05-5.3 (2H, m), 6.96-7.1 (1H, m), 7.12-7.5 (3H, m), 7.75-7.85 (1H, m), 7.95-8.1 (1H, m), 8.14 (1H, s)

4-Dimethylaminomethyl-1-(4-nitrobenzoyl)-1,2,3,4-tetrahydroquinoline, white powder, m.p. 117 - 119°C

3-Dimethylamino-1-(4-nitrobenzoyl)-2,3,4,5-tetrahydro-1H-benzazepine, yellow oil

$^1$H-NMR ($CDCl_3$) δ : 1.5-1.7 (1H, m), 2.1-2.4 (1H, m), 2.42 (6H, s), 2.6-2.7 (1H, m), 2.8-3.0 (3H, m), 5.1-5.3 (1H, m), 6.62 (1H, d, J=7.8 Hz), 6.95 (1H, t, J=7.7 Hz), 7.14 (1H, t, J=7.5 Hz), 7.2-7.4 (3H, m), 8.00 (2H, d, J=8.9 Hz)

3-Dimethylamino-1-(3-methoxy-4-nitrobenzoyl)-2,3,4,5-tetrahydro-1H-benzazepine, yellow oil

$^1$H-NMR ($CDCl_3$) δ : 1.5-1.7 (1H, m), 2.0-2.3 (1H, m), 2.41 (6H, s), 2.5-2.8 (1H, m), 2.8-3.0 (3H, m), 3.75 (3H, s), 5.1-5.3 (1H, m), 6.6-6.8 (2H, m), 6.9-7.3 (4H, m), 7.59 (1H, d, J=8.3 Hz)

4-(4-Nitrobenzoyl)-3,4-dihydro-2H-1,4-benzothiazine, yellow powder, m.p. 180 - 182°C

5-(4-Nitrobenzoyl)-2,3,4,5-tetrahydro-1,5-benzothiazepine, yellow powder, m.p. 162 - 163°C

Reference Example 10

Using the suitable starting materials, the following compounds are obtained in the same manner as in Reference Example 2.

1-(2-Cloro-4-aminobenzoyl)-4-methyl-2,3,4,5-tetrahydro-1H-1,4-benzodiazepine, white powder (recrystallized from methanol/diethyl ether), m.p. 194.5 - 195.5°C

1-(3-Methyl-4-aminobenzoyl)-4-methyl-2,3,4,5-tetrahydro-1H-1,4-benzodiazepine

$^1$H-NMR ($CDCl_3$) δ : 2.01 (3H, s), 2.41 (3H, s), 2.82-3.21 (3H, m), 3.50-4.21 (4H, m), 4.78-5.14 (1H, m), 6.24-6.40 (1H, m), 6.59-6.82 (2H, m), 6.90-7.18 (3H, m), 7.19-7.34 (1H, m)

5-Dimethylamino-1-(2-chloro-4-aminobenzoyl)-2,3,4,5-tetrahydro-1H-benzazepine, white powder (recrystallized from dichloromethane/diethyl ether), m.p. 162 - 164°C

5-Dimethylamino-1-(2-methoxy-4-aminobenzoyl)-2-3-4-5-tetrahydro-1H-benzazepine (recrystallized from methanol/diethyl ether)

$^1$H-NMR ($CDCl_3$) δ : 1.23-2.80 (11H, m), 2.90-3.38 (1H, m), 3.50-5.19 (6H, m), 5.87-6.41 (2H, m), 6.65-7.56 (5H, m)

5-Methoxy-1-(4-aminobenzoyl)-2,3,4,5-tetrahydro-1H-benzazepine, white powder (recrystallized from ethyl acetate/n-hexane), m.p. 154 - 155°C

5-Ethoxycarbonylmethoxy-1-(4-aminobenzoyl)-2,3,4,5-tetrahydro-1H-benzazepine, white powder (recrystallized from ethyl acetate/n-hexane), m.p. 231 - 232°C

5-(4-Dimethylaminobutoxy)-1-(4-aminobenzoyl)-2,3,4,5-tetrahydro-1H-benzazepine, colorless oil

$^1$H-NMR ($CDCl_3$) δ : 1.47-1.83 (6H, m), 1.83-2.54 (4H, m), 2.29 (6H, s), 2.61-3.00 (1H, m), 3.36-3.76 (2H, m), 4.35-5.20 (2H, m), 6.27-6.48 (2H, m), 6.57-6.76 (1H, m), 6.90-7.61 (5H, m)

5-[3-(Phthalimid-1-yl)propoxy]-1-(4-aminobenzoyl)-2,3,4,5-tetrahydro-1H-benzazepine, colorless amorphous

$^1$H-NMR ($CDCl_3$) δ : 1.30-2.47 (6H, m), 2.57-3.01 (1H, m), 3.30-4.06 (4H, m), 4.34-5.20 (2H, m), 6.30-6.53 (2H, m), 6.57-6.78 (1H, m), 6.87-7.57 (5H, m), 7.62-7.76 (2H, m), 7.76-7.97 (2H, m)

5-Chloro-1-(4-aminobenzoyl)-2,3,4,5-tetrahydro-1H-benzazepine, pale yellow amorphous

$^1$H-NMR ($CDCl_3$) δ : 1.35-4.3 (7H, m), 4.55-6.7 (2H, m), 6.3-6.55 (2H, m), 6.6-6.8 (1H, m), 6.85-7.45 (5H, m)

5-Oxo-1-(4-aminobenzoyl)-2,3,4,5-tetrahydro-1H-benzazepine, pale yellow amorphous

$^1$H-NMR ($CDCl_3$) δ : 1.95-2.35 (2H, m), 2.89 (2H, t, J=6.3 Hz), 3.0-5.3 (4H, m), 6.35-6.47 (2H, m), 6.72-6.83 (1H, m), 7.0-7.15 (2H, m), 7.18-7.32 (2H, m), 7.81-7.93 (1H, m)

5-Oxo-1-(2-chloro-4-aminobenzoyl)-2,3,4,5-tetrahydro-1H-benzazepine, white powder

$^1$H-NMR ($CDCl_3$) δ : 1.85-2.3 (2H, m), 2.87 (2H, t, J=6.2 Hz), 3.1-4.75 (4H, m), 6.15-7.5 (6H, m), 7.65-7.9 (1H, m)

4-Dimethylaminomethyl-1-(4-aminobenzoyl)-1,2,3,4-tetrahydroquinoline, white powder, m.p. 123 - 125°C

3-Dimethylamino-1-(4-aminobenzoyl)-2,3,4,5-tetrahydro-1H-benzazepine, white powder, m.p. 175 - 177°C

3-Dimethylamino-1-(3-methoxy-4-aminobenzoyl)-2,3,4,5-tetrahydro-1H-benzazepine, yellow oil

$^1$H-NMR ($CDCl_3$) δ : 1.5-1.7 (1H, m), 2.1-2.3 (1H, m), 2.3-2.6 (1H, m), 2.40 (6H, s), 2.7-3.0 (3H, m), 3.60 (3H, s), 3.8-4.0 (2H, br), 5.2-5.4 (1H, m), 6.37 (1H, d, J=8.2 Hz), 6.5-6.8 (3H, m), 6.9-7.4 (3H, m)

4-(4-Aminobenzoyl)-3,4-dihydro-2H-1,4-benzothiazine, yellow powder, m.p. 207 - 210°C

5-(4-Aminobenzoyl)-2,3,4,5-tetrahydro-1,5-benzothiazepine, yellow powder, m.p. 193 - 195°C

Reference Example 11

Using the suitable starting materials, the following compounds are obtained in the same manner as in Reference Example 1.

5-Carbamoyloxy-1-(4-nitrobenzoyl)-2,3,4,5-tetrahydro-1H-benzazepine, white powder, m.p. 243 - 244°C (recrystallized from ethyl acetate/diisopropyl ether)

5-Methylaminocarbonyloxy-1-(4-nitrobenzoyl)-2,3,4,5-tetrahydro-1H-benzazepine, white powder, m.p. 207 - 208°C (recrystallized from ethyl acetate/n-hexane)

5-Dimethylaminocarbonyloxy-1-(4-nitrobenzoyl)-2,3,4,5-tetrahydro-1H-benzazepine, white powder, m.p. 155 - 156°C (recrystallized from ethyl acetate/diisopropyl ether/n-hexane)

5-Methylidenyl-1-(4-nitrobenzoyl)-2,3,4,5-tetrahydro-1H-benzazepine, colorless prisms, m.p. 133.5 - 134°C (recrystallized from ethyl acetate/diisopropyl ether)

5-Oxo-6-methyl-1-(2-chloro-4-nitrobenzoyl)-2,3,4,5-tetrahydro-1H-benzazepine, colorless prisms, m.p. 90 - 92°C (recrystallized from ethanol)

1-(4-Nitrobenzoyl)-1,2,3,5-tetrahydro-4,1-benzothiazepine, yellow powder, m.p. 185 - 187°C (recrystallized from dichloromethane/diethyl ether)

5-Dimethylamino-1-(2-dimethylamino-4-nitrobenzoyl)-2,3,4,5-tetrahydro-1H-benzazepine, yellow powder, m.p. 123 - 125°C (recrystallized from diethyl ether/dichloromethane)

5-Oxo-1-(4-nitrobenzoyl)-2,3,4,5-tetrahydro-1H-1,4-benzodiazepine, white powder, m.p. 201.5 - 202.5°C (recrystallized from diethyl ether/dichloromethane)

5-Oxo-4-methyl-1-(4-nitrobenzoyl)-2,3,4,5-tetrahydro-1H-1,4-benzodiazepine, white powder, m.p. 136 - 138°C (recrystallized from diethyl ether/dichloromethane)

5-Dimethylamino-1-(3-methyl-4-nitrobenzoyl)-2,3,4,5-tetrahydro-1H-benzazepine, yellow oil

$^1$H-NMR ($CDCl_3$) δ : 1.16-3.18 (11H, m), 2.18 (3H, s), 3.40-5.15 (2H, m), 6.50-7.68 (6H, m), 7.70-7.84 (1H, m)

5-Dimethylamino-1-(2-methyl-4-nitrobenzoyl)-2,3,4,5-tetrahydro-1H-benzazepine, colorless amorphous

$^1$H-NMR ($CDCl_3$) δ : 1.19-2.86 (11H, m), 2.20 (3H, s), 2.94-3.24 (1H, m), 3.36-5.18 (1H, m), 6.49-8.20 (7H, m)

5-Dimethylamino-1-(2-fluoro-4-nitrobenzoyl)-2,3,4,5-tetrahydro-1H-benzazepine, yellow oil

$^1$H-NMR ($CDCl_3$) δ : 1.21-2.66 (10H, m), 2.66-5.11 (3H, m), 6.63-8.25 (7H, m)

5-Dimethylamino-1-(3-fluoro-4-nitrobenzoyl)-2,3,4,5-tetrahydro-1H-benzazepine, white powder, m.p. 152 - 152.5°C (recrystallized from chloroform/diethyl ether)

Reference Example 12

Using the suitable starting materials, the following compounds are obtained in the same manner as in Reference Example 2.

5-Carbamoyloxy-1-(4-aminobenzoyl)-2,3,4,5-tetrahydro-1H-benzazepine, white powder, m.p. 215 - 216°C (recrystallized from ethyl acetate/n-hexane)

5-Methylaminocarbonyloxy-1-(4-aminobenzoyl)-2,3,4,5-tetrahydro-1H-benzazepine, white powder, m.p. 192 - 195°C (recrystallized from ethyl acetate/n-hexane)

5-Dimethylaminocarbonyloxy-1-(4-aminobenzoyl)-2,3,4,5-tetrahydro-1H-benzazepine, white powder, m.p. 228 - 230°C (recrystallized from ethyl acetate/diisopropyl ether)

5-Methyl-1-(4-aminobenzoyl)-2,3,4,5-tetrahydro-1H-benzazepine, white powder, m.p. 155 - 156°C (recrystallized from ethyl acetate/n-hexane)

5-Oxo-6-methyl-1-(2-chloro-4-aminobenzoyl)-2,3,4,5-tetrahydro-1H-benzazepine, white powder, m.p. 193 - 195°C (recrystallized from ethanol)

1-(4-Aminobenzoyl)-1,2,3,5-tetrahydro-4,1-benzothiazepine, white powder, m.p. 179 - 180°C (recrystallized from dichloromethane/diethyl ether)

5-Dimethylamino-1-(2-dimethylamino-4-aminobenzoyl)-2,3,4,5-tetrahydro-1H-benzazepine, white powder, m.p. 163 - 165°C (recrystallized from diethyl ether/dichloromethane)

5-Oxo-1-(4-aminobenzoyl)-2,3,4,5-tetrahydro-1H-benzazepine, yellow powder, m.p. 195 - 197°C (recrystallized from diethyl ether/dichloromethane)

5-Oxo-4-methyl-1-(4-aminobenzoyl)-2,3,4,5-tetrahydro-1H-1,4-benzazepine, yellow powder, m.p. 190 - 192°C (recrystallized from diethyl ether/dichloromethane)

5-Dimethylamino-1-(2-ethoxy-4-aminobenzoyl)-2,3,4,5-tetrahydro-1H-benzazepine, white powder, m.p. 111 - 114°C (recrystallized from diethyl ether)

5-Dimethylamino-1-(3-methyl-4-aminobenzoyl)-2,3,4,5-tetrahydro-1H-benzazepine, yellow oil

$^1$H-NMR ($CDCl_3$) δ : 0.66-2.56 (14H, m), 2.93-5.22 (4H, m), 6.23-7.80 (7H, m)

5-Dimethylamino-1-(2-methyl-4-aminobenzoyl)-2,3,4,5-tetrahydro-1H-benzazepine, white powder, m.p. 154 - 156°C (recrystallized from methanol/diethyl ether)

5-Dimethylamino-1-(2-fluoro-4-aminobenzoyl)-2,3,4,5-tetrahydro-1H-benzazepine, white powder, m.p. 161 - 163°C (recrystallized from dichloromethane/diethyl ether)

5-Dimethylamino-1-(3-fluoro-4-aminobenzoyl)-2,3,4,5-tetrahydro-1H-benzazepine, white powder, m.p. 156 - 157°C (recrystallized from methanol/diethyl ether)

5-Oxo-1-(2-methoxy-4-aminobenzoyl)-2,3,4,5-tetrahydro-1H-benzazepine, colorless prisms, m.p. 160 - 160.5°C (recrystallized from methanol/diethyl ether)

Example 1

To a solution of 1,2,3,4-tetrahydroquinoline (28.7 g) in acetone (400 ml) and water (200 ml) is added potassium carbonate (38.8 g) and further thereto is added 4-benzoylaminobenzoyl chloride (56 g) under ice-cooling. The mixture is stirred at room temperature overnight. Water is added to the reaction mixture, and the mixture is extracted with dichloromethane. The extract is dried over magnesium sulfate, and the solvent is distilled off under reduced pressure. The resulting residue is purified by silica gel column chromatography and recrystallized from methanol to give 1-[4-(benzoylamino)benzoyl]-1,2,3,4-tetrahydroquinoline (57 g) as white powder, m.p. 202.5 - 203.5°C.

Using the suitable starting materials, the compounds as shown in the following Table 1 are obtained in the same manner as in Example 1.

Table 1

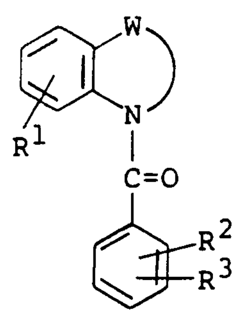

Example 2

Structure

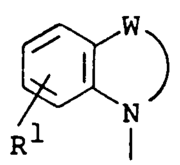 : 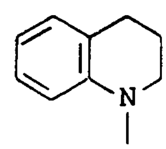

$R^2$: H

$R^3$: 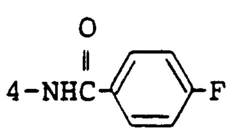

Crystalline form: Light yellow powder

Recrystallization solvent: Methanol

Melting Point: 198.5 - 199.5°C

Form: Free

Example 3

Structure

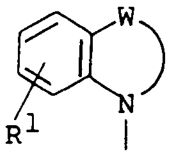 : 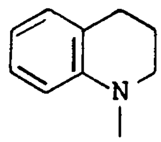

$R^2$: H

$R^3$: 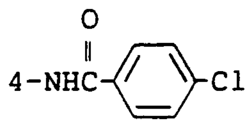

Crystalline form: White powder

Recrystallization solvent: Methanol

Melting Point: 200.5 - 201.5°C

Form: Free

Example 4

Structure

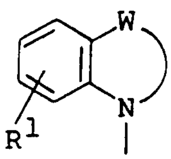 : 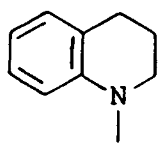

$R^2$: H

$R^3$: 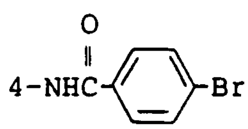

Crystalline form: Yellow powder

Recrystallization solvent: Methanol

Melting Point: 206 - 207°C

Form: Free

Example 5

Structure

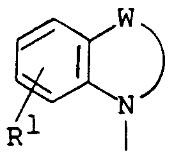 : 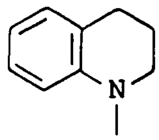

$R^2$: H

$R^3$: 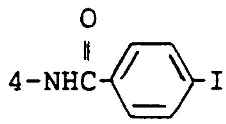

Crystalline form: Yellow powder

Recrystallization solvent: Methanol

Melting Point: 216 - 217°C

Form: Free

Example 6

Structure

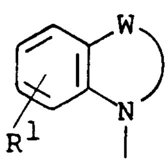 : 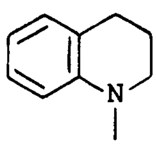

$R^2$: H

$R^3$: 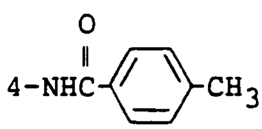

Crystalline form: White powder

Recrystallization solvent: Methanol

Melting Point: 202 - 203°C

Form: Free

Example 7

Structure

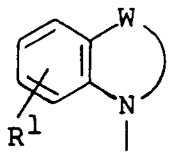 : 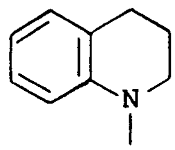 $R^2$: H

$R^3$: 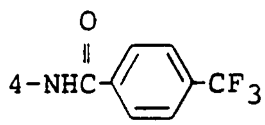

Crystalline form: White powder

Recrystallization solvent: Methanol

Melting Point: 212 - 213°C

Form: Free

Example 8

Structure

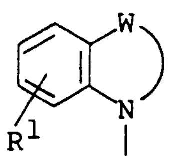 : 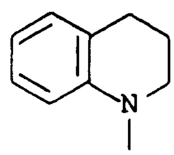 $R^2$: H

$R^3$: 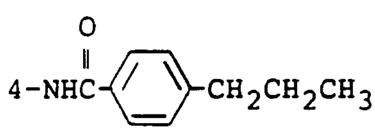

Crystalline form: White powder

Recrystallization solvent: Methanol

Melting Point: 167.5 - 168.5°C

Form: Free

Example 9

Structure

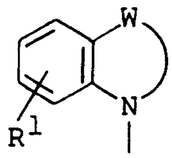 : 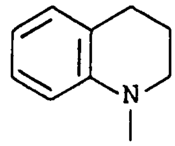

$R^2$: H

$R^3$: 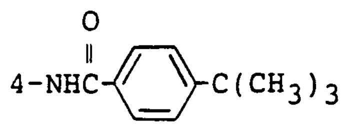

Crystalline form: White powder

Recrystallization solvent: Methanol

Melting Point: 205 - 206°C

Form: Free

Example 10

Structure

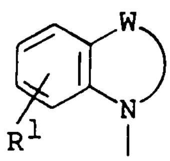 : 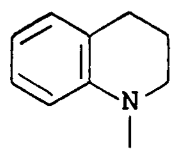

$R^2$: H

$R^3$: 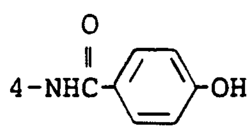

Crystalline form: White powder

Recrystallization solvent: Methanol

Melting Point: >300°C

NMR analysis: 1)

Form: Free

Example 11

Structure

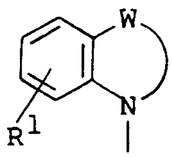 : 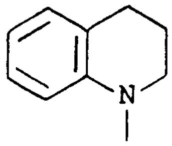

$R^2$: H

$R^3$: 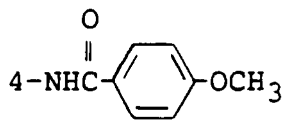

Crystalline form: Yellow powder

Recrystallization solvent: Methanol

Melting Point: 176 - 177°C

Form: Free

Example 12

Structure

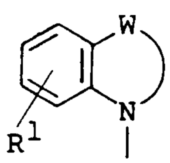 : 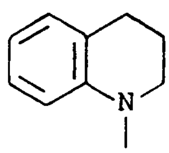

$R^2$: H

$R^3$: 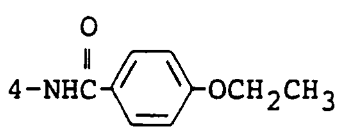

Crystalline form: White powder

Recrystallization solvent: Methanol

Melting Point: 219 - 220°C

Form: Free

Example 13

Structure

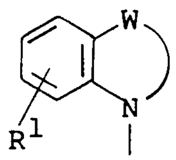 : 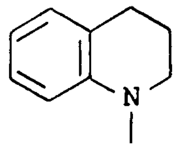 $R^2$: H

$R^3$: 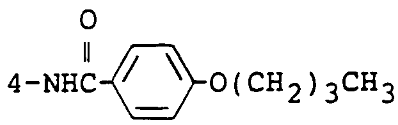

Crystalline form: White powder

Recrystallization solvent: Methanol

Melting Point: 193 - 194°C

Form: Free

Example 14

Structure

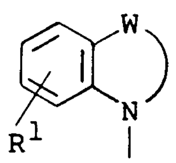 : 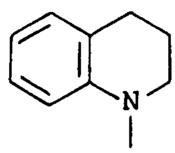 $R^2$: H

$R^3$: 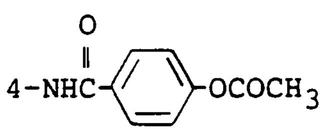

Crystalline form: White powder

Recrystallization solvent: Methanol

Melting Point: 232 - 233°C

Form: Free

Example 15

Structure

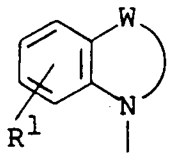

:

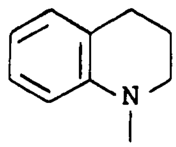

$R^2$: H

$R^3$: 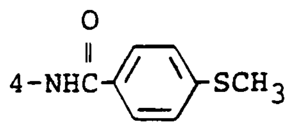

Crystalline form: White powder

Recrystallization solvent: Methanol

Melting Point: 209 - 210°C

Form: Free

Example 16

Structure

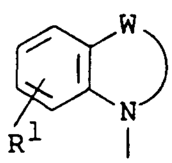

:

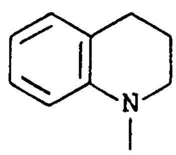

$R^2$: H

$R^3$: 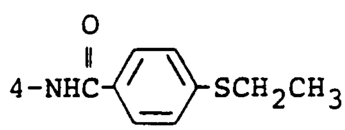

Crystalline form: White powder

Recrystallization solvent: Methanol

Melting Point: 184.5 - 185.5°C

Form: Free

Example 17

Structure

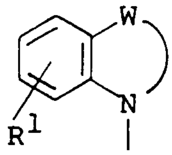 : 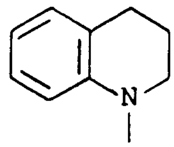

$R^2$: H

$R^3$: 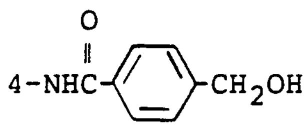

Crystalline form: White powder

Recrystallization solvent: Methanol

Melting Point: 224.5 - 225.5°C

Form: Free

Example 18

Structure

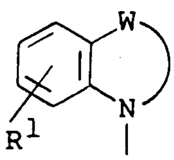 : 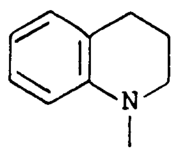

$R^2$: H

$R^3$: 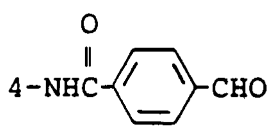

Crystalline form: White powder

Recrystallization solvent: Methanol

Melting Point: 220.5 - 221.5°C

Form: Free

Example 19

Structure

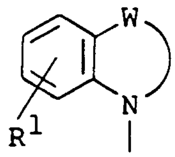 : 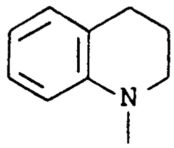

$R^2$: H

$R^3$: 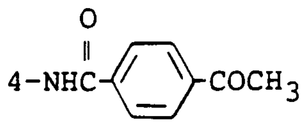

Crystalline form: Yellow powder

Recrystallization solvent: Methanol

Melting Point: 231 - 232°C

Form: Free

Example 20

Structure

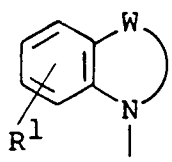 : 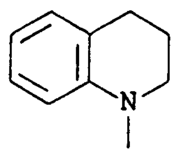

$R^2$: H

$R^3$: 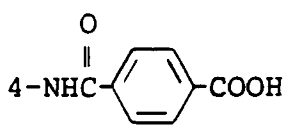

Crystalline form: White powder

Recrystallization solvent: Methanol

Melting Point: >300°C

NMR analysis: 2)

Form: Free

Example 21

Structure

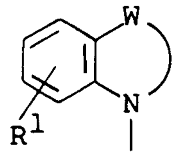 : 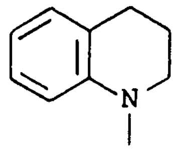

$R^2$: H

$R^3$: 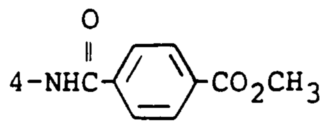

Crystalline form: White powder

Recrystallization solvent: Methanol

Melting Point: 208 - 209°C

Form: Free

Example 22

Structure

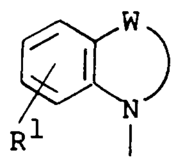 : 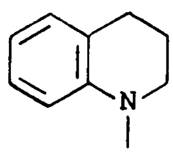

$R^2$: H

$R^3$: 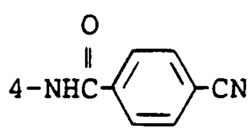

Crystalline form: White powder

Recrystallization solvent: Methanol

Melting Point: 234.5 - 235.5°C

Form: Free

Example 23

Structure

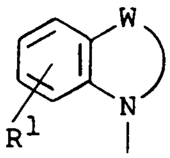 : 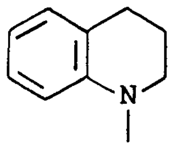

$R^2$: H

$R^3$: 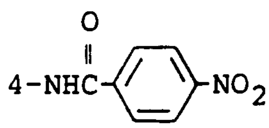

Crystalline form: Yellow powder

Recrystallization solvent: Methanol

Melting Point: 263.5 - 264.5°C

Form: Free

Example 24

Structure

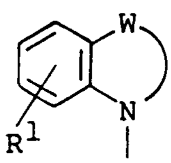 : 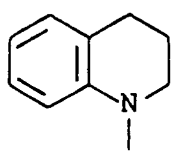

$R^2$: H

$R^3$: 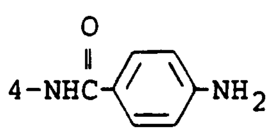

Crystalline form: White powder

Recrystallization solvent: Methanol

Melting Point: 237 - 238°C

Form: Free

Example 25

Structure

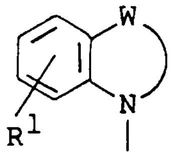 : 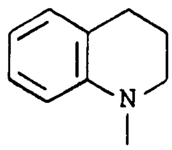

$R^2$: H

$R^3$: 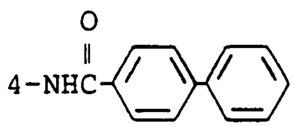

Crystalline form: White powder

Recrystallization solvent: Methanol

Melting Point: 234 - 235°C

Form: Free

Example 26

Structure

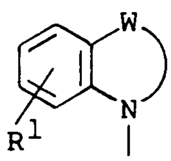 : 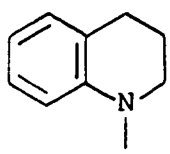

$R^2$: H

$R^3$: 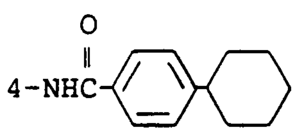

Crystalline form: White powder

Recrystallization solvent: Methanol

Melting Point: 236.5 - 237.5°C

Form: Free

Example 27

Structure

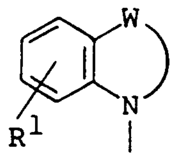 : 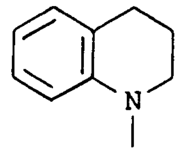

$R^2$: H

$R^3$: 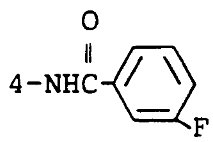

Crystalline form: White powder

Recrystallization solvent: Methanol

Melting Point: 206.5 - 207.5°C

Form: Free

Example 28

Structure

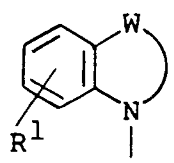 : 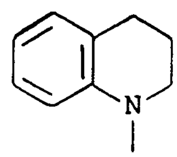

$R^2$: H

$R^3$: 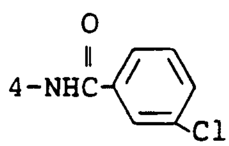

Crystalline form: White powder

Recrystallization solvent: Methanol

Melting Point: 210 - 211°C

Form: Free

Example 29

Structure

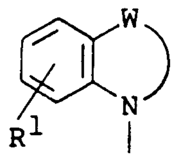 : 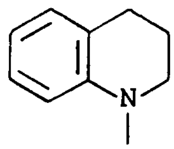

$R^2$: H

$R^3$: 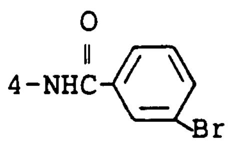

Crystalline form: White powder

Recrystallization solvent: Methanol

Melting Point: 210.5 - 211.5°C

Form: Free

Example 30

Structure

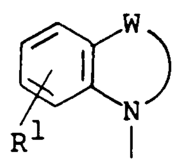 : 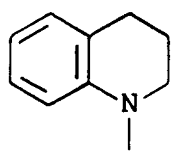

$R^2$: H

$R^3$: 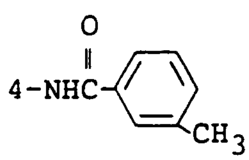

Crystalline form: White powder

Recrystallization solvent: Methanol

Melting Point: 178 - 179°C

Form: Free

Example 31

Structure

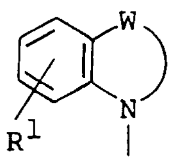 : 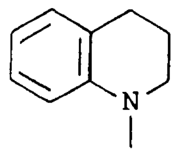

$R^2$: H

$R^3$: 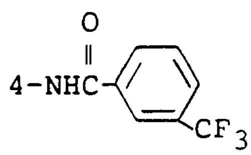

Crystalline form: White powder

Recrystallization solvent: Methanol

Melting Point: 192 - 193°C

Form: Free

---

Example 32

Structure

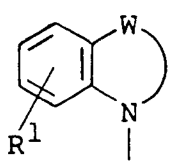 : 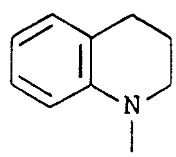

$R^2$: H

$R^3$: 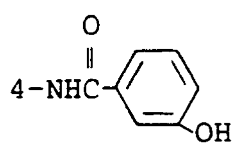

Crystalline form: White powder

Recrystallization solvent: Methanol

Melting Point: 217 - 218°C

Form: Free

Example 33

Structure

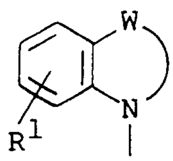 : 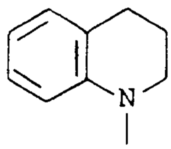

$R^2$: H

$R^3$: 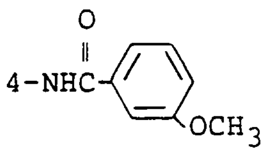

Crystalline form: White powder

Recrystallization solvent: Methanol

Melting Point: 143 - 144°C

Form: Free

Example 34

Structure

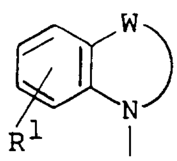 : 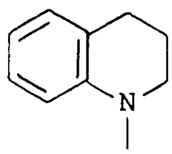

$R^2$: H

$R^3$: 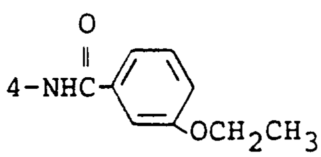

Crystalline form: White powder

Recrystallization solvent: Methanol

Melting Point: 170.5 - 171.5°C

Form: Free

Example 35

Structure

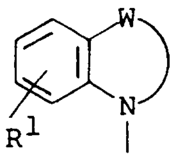 : 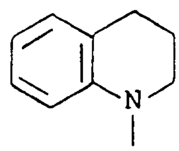

$R^2$: H

$R^3$: 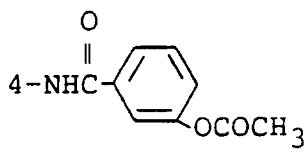

Crystalline form: White powder

Recrystallization solvent: Methanol

Melting Point: 169.5 - 170.5°C

Form: Free

Example 36

Structure

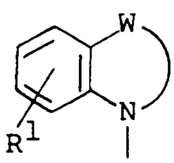 : 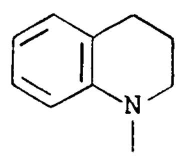

$R^2$: H

$R^3$: 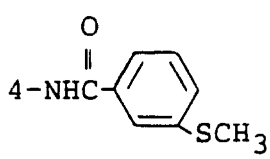

Crystalline form: White powder

Recrystallization solvent: Methanol

Melting Point: 174.5 - 175.5°C

Form: Free

Example 37

Structure

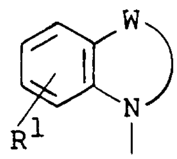 : 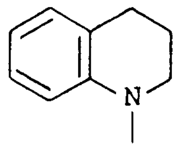 $R^2$: H

$R^3$: 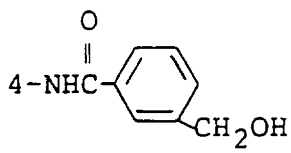

Crystalline form: White powder

Recrystallization solvent: Methanol

Melting Point: 148.5 - 149.5°C

Form: Free

Example 38

Structure

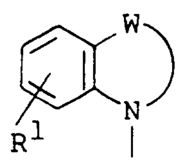 : 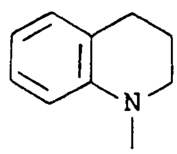 $R^2$: H

$R^3$: 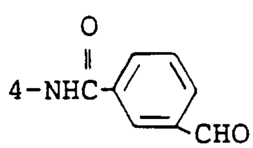

Crystalline form: White powder

Recrystallization solvent: Methanol

Melting Point: 165 - 166°C

Form: Free

Example 39

Structure

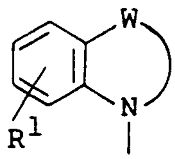 : 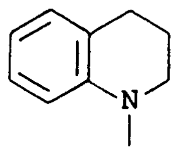

$R^2$: H

$R^3$: 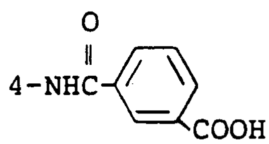

Crystalline form: White powder

Recrystallization solvent: Methanol

Melting Point: 243 - 244°C

Form: Free

Example 40

Structure

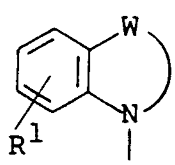 : 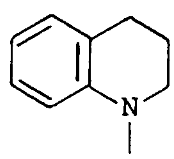

$R^2$: H

$R^3$: 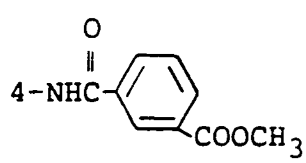

Crystalline form: White powder

Recrystallization solvent: Methanol

Melting Point: 199 - 200°C

Form: Free

Example 41

Structure

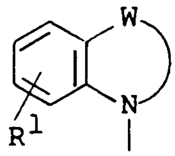

:

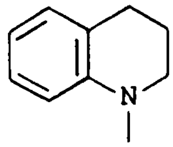

$R^2$: H

$R^3$: 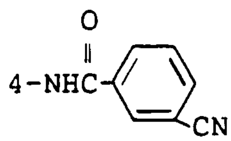

Crystalline form: White powder

Recrystallization solvent: Methanol

Melting Point: 232.5 - 233.5°C

Form: Free

Example 42

Structure

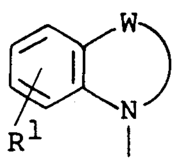

:

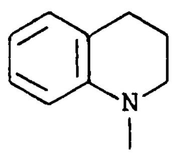

$R^2$: H

$R^3$: 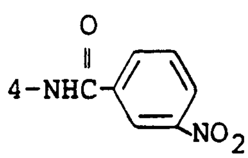

Crystalline form: White powder

Recrystallization solvent: Methanol

Melting Point: 178.5 - 179.5°C

Form: Free

Example 43

Structure

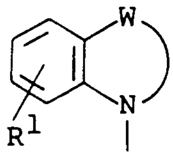
:
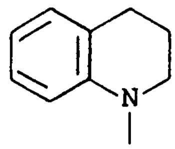

$R^2$: H

$R^3$: 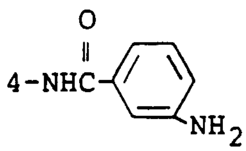

Crystalline form: White powder

Recrystallization solvent: Methanol

Melting Point: 205.5 - 206.5°C

Form: Free

Example 44

Structure

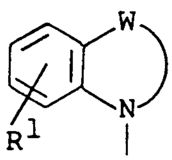
:
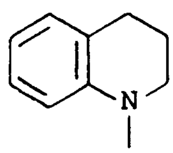

$R^2$: H

$R^3$: 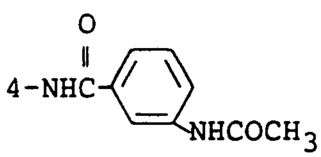

Crystalline form: White powder

Recrystallization solvent: Methanol

Melting Point: 234 - 235°C

Form: Free

Example 45

Structure

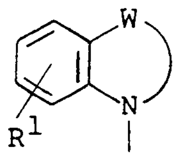 : 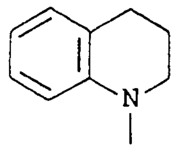

$R^2$: H

$R^3$: 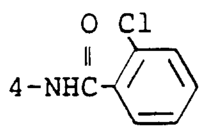

Crystalline form: White powder

Recrystallization solvent: Methanol

Melting Point: 225 - 226°C

Form: Free

Example 46

Structure

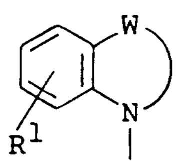 : 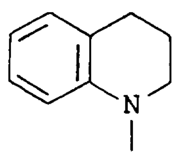

$R^2$: H

$R^3$: 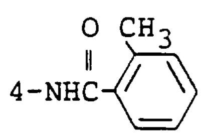

Crystalline form: White powder

Recrystallization solvent: Methanol

Melting Point: 224 - 225°C

Form: Free

Example 47

Structure

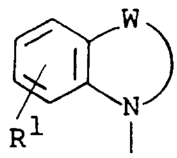 : 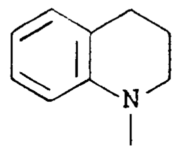

$R^2$: H

$R^3$: 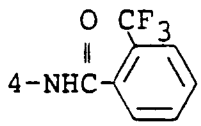

Crystalline form: White powder

Recrystallization solvent: Methanol

Melting Point: 236 - 237°C

Form: Free

Example 48

Structure

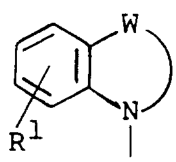 : 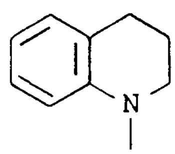

$R^2$: H

$R^3$: 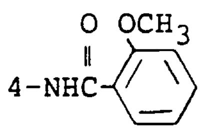

Crystalline form: Yellow powder

Recrystallization solvent: Methanol

Melting Point: 175.5 - 176.5°C

Form: Free

Example 49

Structure

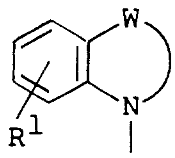 : 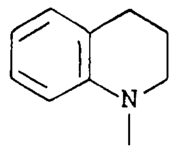 $R^2$: H

$R^3$: 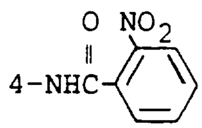

Crystalline form: White powder

Recrystallization solvent: Methanol

Melting Point: 231 - 232°C

Form: Free

Example 50

Structure

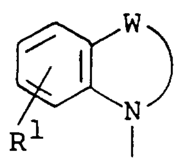 : 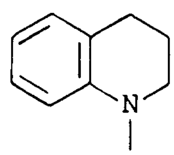 $R^2$: H

$R^3$: 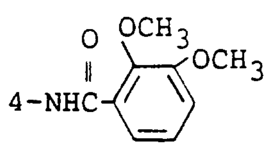

Crystalline form: Yellow powder

Recrystallization solvent: Methanol

Melting Point: 204 - 205°C

Form: Free

Example 51

Structure

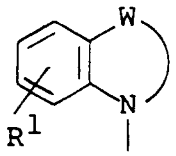 : 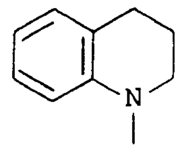

$R^2$: H

$R^3$: 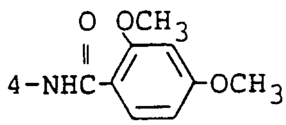

Crystalline form: White powder

Recrystallization solvent: Methanol

Melting Point: 190 - 191°C

Form: Free

Example 52

Structure

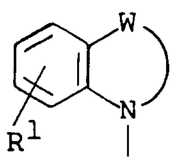 : 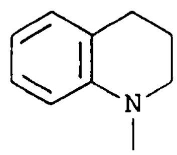

$R^2$: H

$R^3$: 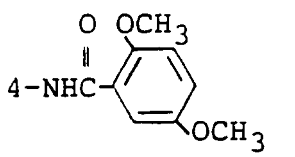

Crystalline form: White powder

Recrystallization solvent: Methanol

Melting Point: 156 - 157°C

Form: Free

Example 53

Structure

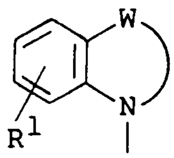 : 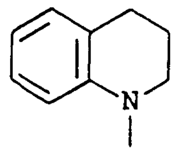 $R^2$: H

$R^3$: 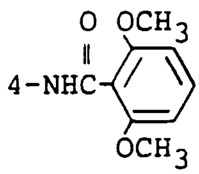

Crystalline form: White powder

Recrystallization solvent: Methanol

Melting Point: 200 - 201°C

Form: Free

Example 54

Structure

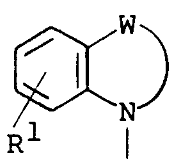 : 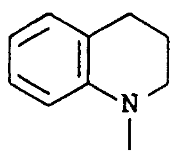 $R^2$: H

$R^3$: 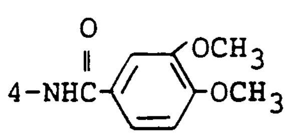

Crystalline form: White powder

Recrystallization solvent: Methanol

Melting Point: 206 - 207°C

Form: Free

Example 55

Structure

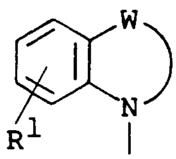 : 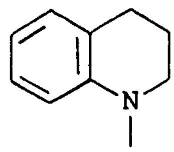

$R^2$: H

$R^3$: 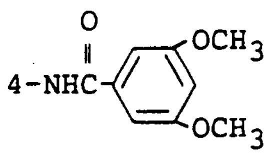

Crystalline form: Colorless amorphous

NMR analysis: 3)

Form: Free

Example 56

Structure

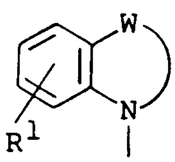 : 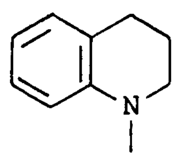

$R^2$: H

$R^3$: 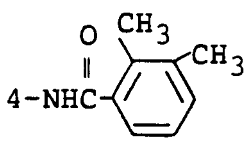

Crystalline form: White powder

Recrystallization solvent: Methanol

Melting Point: 215.5 - 216.5°C

Form: Free

Example 57

Structure

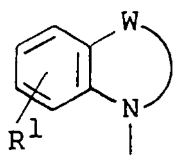 : 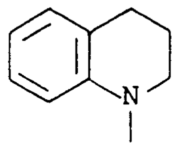

$R^2$: H

$R^3$: 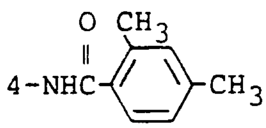

Crystalline form: White powder

Recrystallization solvent: Methanol

Melting Point: 189 - 190°C

Form: Free

Example 58

Structure

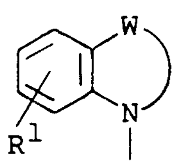 : 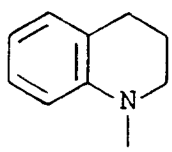

$R^2$: H

$R^3$: 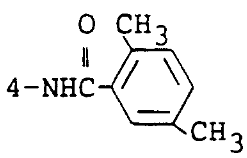

Crystalline form: White powder

Recrystallization solvent: Methanol

Melting Point: 203.5 - 204.5°C

Form: Free

Example 59

Structure

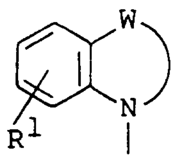 : 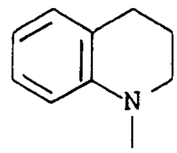

$R^2$: H

$R^3$: 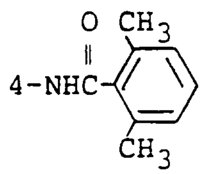

Crystalline form: Yellow powder

Recrystallization solvent: Methanol

Melting Point: 254.5 - 255.5°C

Form: Free

Example 60

Structure

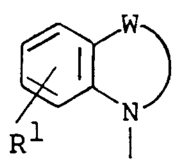 : 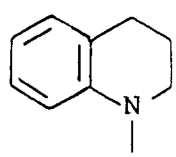

$R^2$: H

$R^3$: 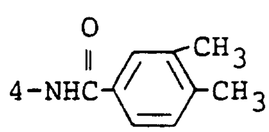

Crystalline form: Brown powder

Recrystallization solvent: Methanol

Melting Point: 182.5 - 183.5°C

Form: Free

Example 61

Structure

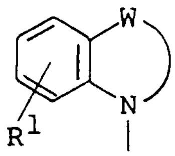 : 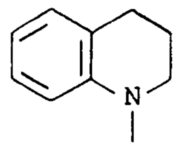 $R^2$: H

$R^3$: 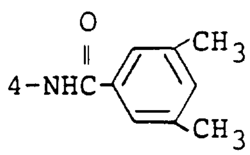

Crystalline form: Colorless amorphous

NMR analysis: 4)

Form: Free

Example 62

Structure

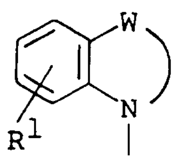 : 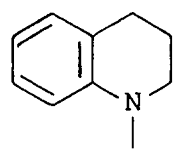 $R^2$: H

$R^3$: 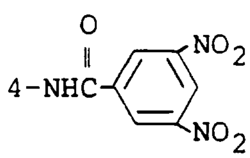

Crystalline form: White powder

Recrystallization solvent: Methanol

Melting Point: 263 - 264°C

Form: Free

Example 63

Structure

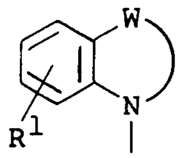 : 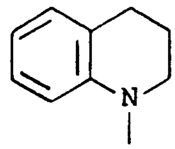 $R^2$: H

$R^3$: 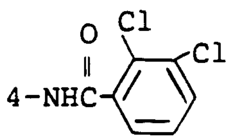

Crystalline form: White powder

Recrystallization solvent: Dichloromethane/ethanol

Melting Point: 217 - 218°C

Form: Free

Example 64

Structure

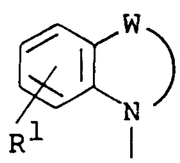 : 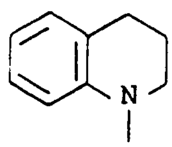 $R^2$: H

$R^3$: 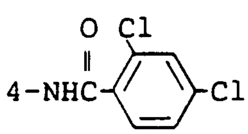

Crystalline form: White powder

Recrystallization solvent: Dichloromethane/ethanol

Melting Point: 183 - 184°C

Form: Free

Example 65

Structure

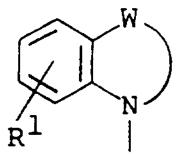 : 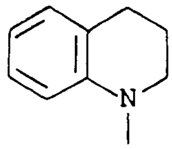

$R^2$: H

$R^3$: 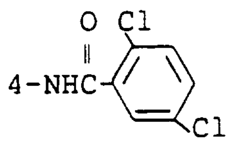

Crystalline form: Yellow powder

Recrystallization solvent: Dichloromethane/ethanol

Melting Point: 207.5 - 208.5°C

Form: Free

Example 66

Structure

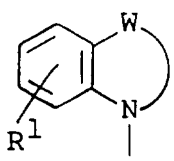 : 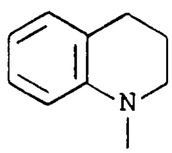

$R^2$: H

$R^3$: 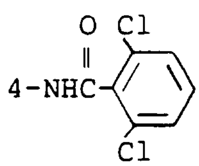

Crystalline form: Yellow powder

Recrystallization solvent: Dichloromethane/ethanol

Melting Point: 251 - 252°C

Form: Free

Example 67

Structure

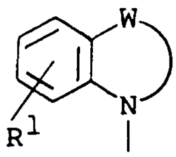 : 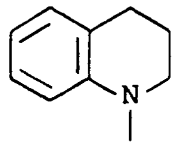

$R^2$: H

$R^3$: 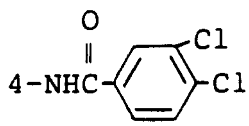

Crystalline form: White powder

Recrystallization solvent: Dichloromethane/ethanol

Melting Point: 208.5 - 209.5°C

Form: Free

Example 68

Structure

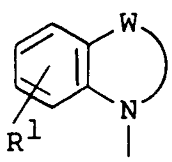 : 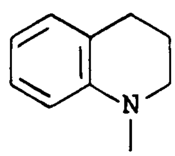

$R^2$: H

$R^3$: 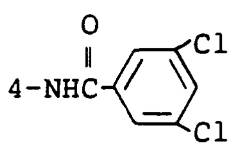

Crystalline form: White powder

Recrystallization solvent: Dichloromethane/ethanol

Melting Point: 231 - 232°C

Form: Free

Example 69

Structure

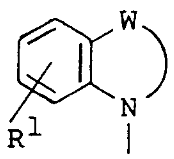 : 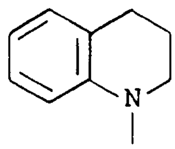

$R^2$: H

O
‖
$R^3$: 4-$NHCCH_3$

Crystalline form: Colorless amorphous

NMR analysis: 5)

Form: Free

Example 70

Structure

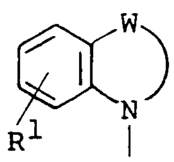 : 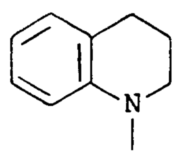

$R^2$: H

O
‖
$R^3$: 4-$NHC(CH_2)_2CH_3$

Crystalline form: White powder

Recrystallization solvent: Methanol

Melting Point: 134 - 135°C

Form: Free

Example 71

Structure

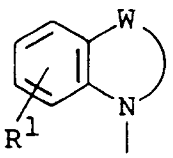 : 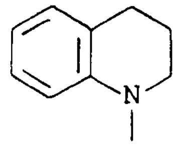 $R^2$: H

O
‖
$R^3$: 4-NHC$(CH_2)_4CH_3$

Crystalline form: Yellow powder

Recrystallization solvent: Methanol

Melting Point: 115 - 116°C

Form: Free

Example 72

Structure

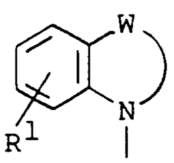 : 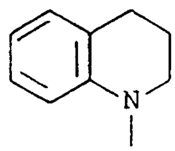 $R^2$: H

O
‖
$R^3$: 4-NHC$CH_2C(CH_3)_3$

Crystalline form: White powder

Recrystallization solvent: Methanol

Melting Point: 178.5 - 179.5°C

Form: Free

Example 73

Structure

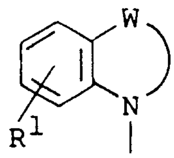 : 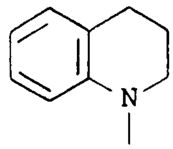

$R^2$: H

O
‖
$R^3$: 4-NHCCH$(CH_3)_2$

Crystalline form: White powder

Recrystallization solvent: Methanol

Melting Point: 182.5 - 183.5°C

Form: Free

Example 74

Structure

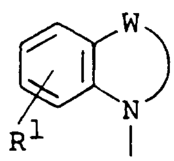 : 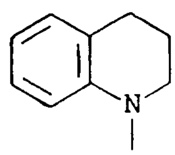

$R^2$: H

O
‖
$R^3$: 4-NHCC$(CH_3)_3$

Crystalline form: White powder

Recrystallization solvent: Methanol

Melting Point: 164 - 165°C

Form: Free

Example 75

Structure

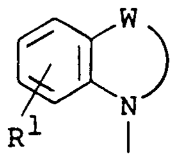 : 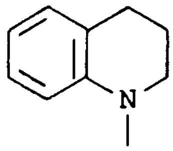

$R^2$: H

$R^3$: 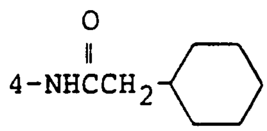

Crystalline form: Colorless amorphous

NMR analysis: 6)

Form: Free

Example 76

Structure

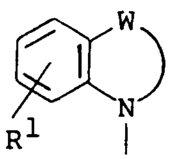 : 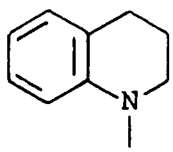

$R^2$: H

$R^3$: 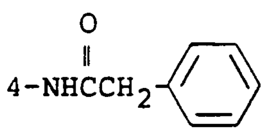

Crystalline form: Yellow amorphous

NMR analysis: 7)

Form: Free

Example 77

Structure

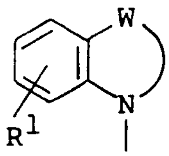 : 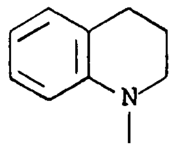

$R^2$: H

$R^3$: 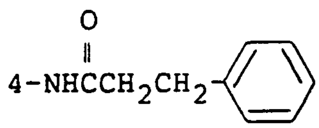

Crystalline form: White powder

Recrystallization solvent: Methanol

Melting Point: 155 - 156°C

Form: Free

Example 78

Structure

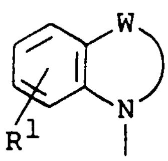 : 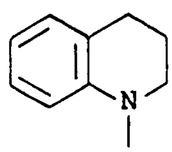

$R^2$: H

$R^3$: 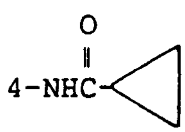

Crystalline form: White powder

Recrystallization solvent: Methanol

Melting Point: 182.5 - 183.5°C

Form: Free

Example 79

Structure

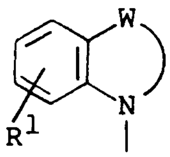 : 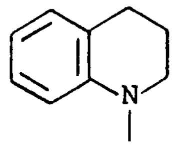 $R^2$: H

$R^3$: 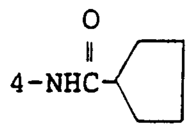

Crystalline form: White powder

Recrystallization solvent: Methanol

Melting Point: 164.5 - 165.5°C

Form: Free

Example 80

Structure

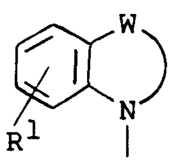 : 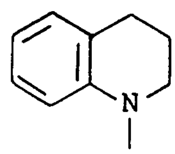 $R^2$: H

$R^3$: 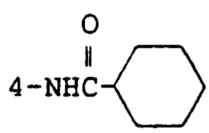

Crystalline form: White powder

Recrystallization solvent: Methanol

Melting Point: 165 - 167°C

Form: Free

Example 81

Structure

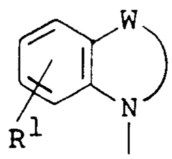 : 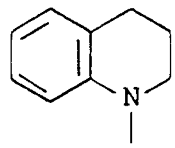 $R^2$: H

$R^3$: 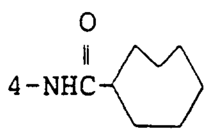

Crystalline form: White powder

Recrystallization solvent: Methanol

Melting Point: 124 - 125°C

Form: Free

Example 82

Structure

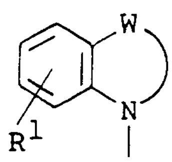 : 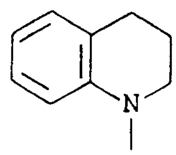 $R^2$: H

$R^3$: 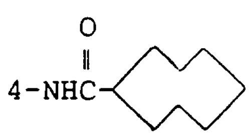

Crystalline form: White powder

Recrystallization solvent: Methanol

Melting Point: 140.5 - 141.5°C

Form: Free

Example 83

Structure

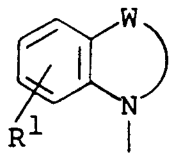 : 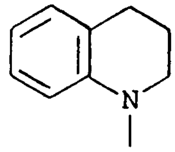

R$^2$: H

R$^3$: 4-NHC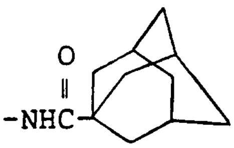

Crystalline form: Colorless amorphous

NMR analysis: 8)

Form: Free

Example 84

Structure

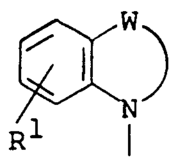 : 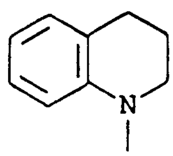

R$^2$: H

R$^3$: 4-NHC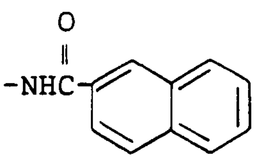

Crystalline form: White powder

Recrystallization solvent: Methanol

Melting Point: 211 - 212°C

Form: Free

Example 85

Structure

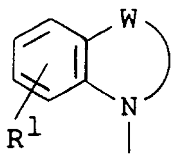 : 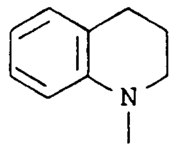 $R^2$: H

$R^3$: 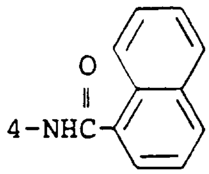

Crystalline form: White powder

Recrystallization solvent: Methanol

Melting Point: 178 - 179°C

Form: Free

Example 86

Structure

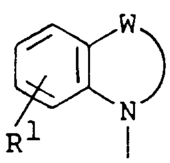 : 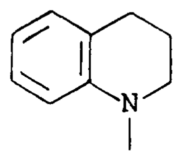 $R^2$: H

$R^3$: 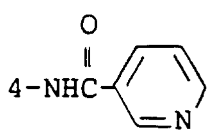

Crystalline form: White powder

Recrystallization solvent: Methanol

Melting Point: 212.5 - 213.5°C

Form: Free

Example 87

Structure

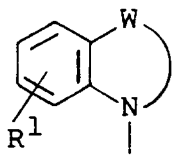 : 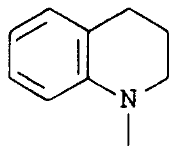

$R^2$: H

$R^3$: 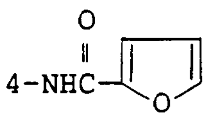

Crystalline form: White powder

Recrystallization solvent: Methanol

Melting Point: 193 - 194°C

Form: Free

Example 88

Structure

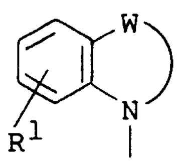 : 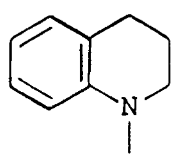

$R^2$: H

$R^3$: 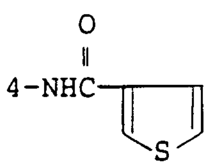

Crystalline form: White powder

Recrystallization solvent: Methanol

Melting Point: 203 - 204°C

Form: Free

Example 89

Structure

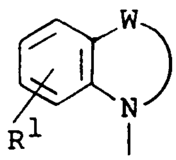 : 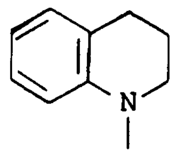

$R^2$: H

$R^3$: 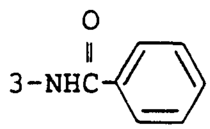

Crystalline form: Colorless amorphous

NMR analysis: 9)

Form: Free

Example 90

Structure

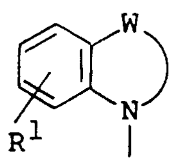 : 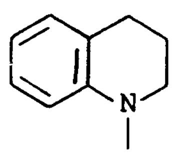

$R^2$: H

$R^3$: 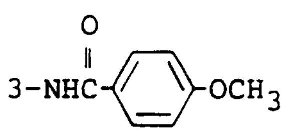

Crystalline form: Colorless amorphous

NMR analysis: 10)

Form: Free

Example 91

Structure

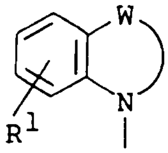 : 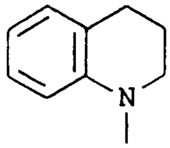

$R^2$: H

$R^3$: 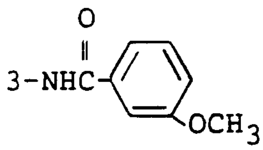

Crystalline form: Colorless amorphous

NMR analysis: 11)

Form: Free

Example 92

Structure

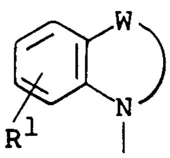 : 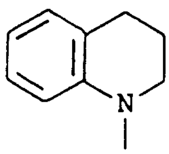

$R^2$: H

$R^3$: 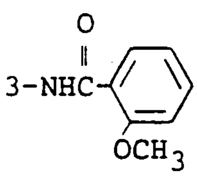

Crystalline form: White powder

Recrystallization solvent: Methanol

Melting Point: 156.5 - 157.5°C

Form: Free

Example 93

Structure

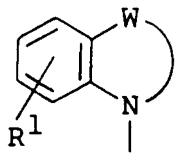 : 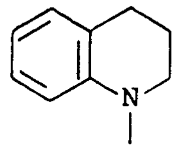 $R^2$: H

$R^3$: 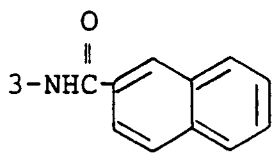

Crystalline form: Colorless amorphous

NMR analysis: 12)

Form: Free

Example 94

Structure

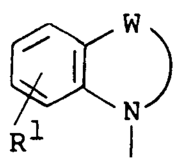 : 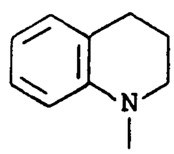 $R^2$: H

$R^3$: 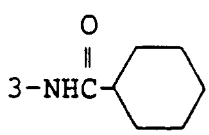

Crystalline form: White powder

Recrystallization solvent: Methanol

Melting Point: 203.5 - 204.5°C

Form: Free

Example 95

Structure

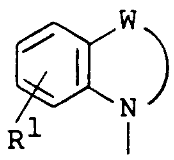 : 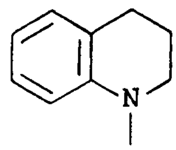 $R^2$: H

$R^3$: 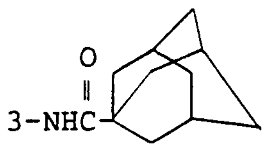

Crystalline form: Colorless amorphous

NMR analysis: 13)

Form: Free

Example 96

Structure

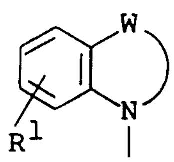 : 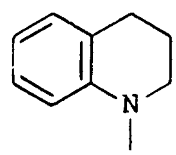 $R^2$: H

$R^3$: 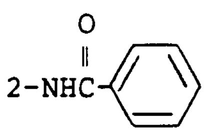

Crystalline form: Yellow powder

Recrystallization solvent: Methanol

Melting Point: 126 - 127°C

Form: Free

Example 97

Structure

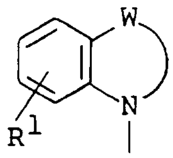 : 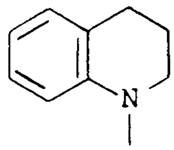 $R^2$: H

$R^3$: 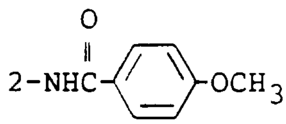

Crystalline form: White powder

Recrystallization solvent: Methanol

Melting Point: 158.5 - 159.5°C

Form: Free

Example 98

Structure

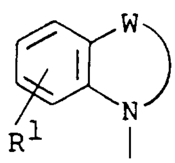 : 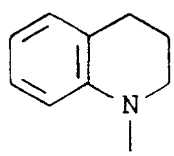 $R^2$: H

$R^3$: 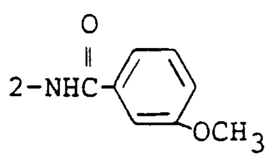

Crystalline form: White powder

Recrystallization solvent: Methanol

Melting Point: 129 - 130°C

Form: Free

| | |
|---|---|
| Example 99 | |
| Structure | |
| 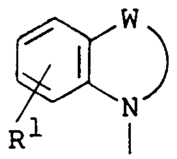 : 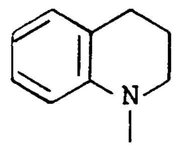 | $R^2$: H |
| $R^3$: 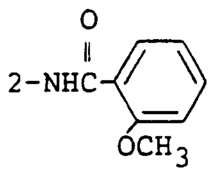 | |
| Crystalline form: White powder | |
| Recrystallization solvent: Methanol | |
| Melting Point: 131.5 - 132.5°C | |
| Form: Free | |
| Example 100 | |
| Structure | |
| 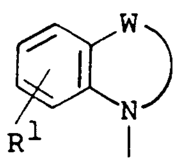 : 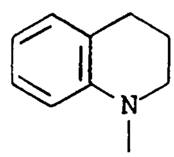 | $R^2$: H |
| $R^3$: 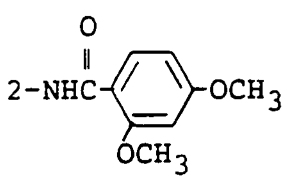 | |
| Crystalline form: White powder | |
| Recrystallization solvent: Methanol | |
| Melting Point: 140 - 141°C | |
| Form: Free | |

Example 101

Structure

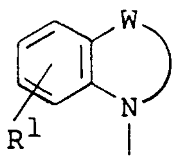 : 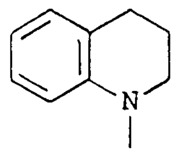

$R^2$: H

$R^3$: 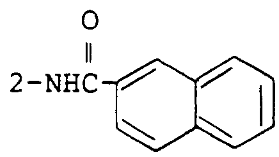

Crystalline form: White powder

Recrystallization solvent: Methanol

Melting Point: 138.5 - 139.5°C

Form: Free

Example 102

Structure

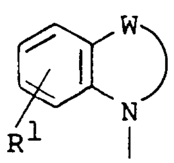 : 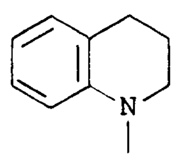

$R^2$: H

$R^3$: 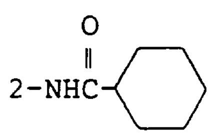

Crystalline form: White powder

Recrystallization solvent: Methanol

Melting Point: 128 - 129°C

Form: Free

Example 103

Structure

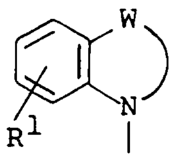 : 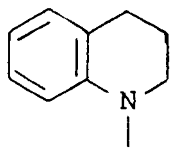 $R^2$: H

$R^3$: 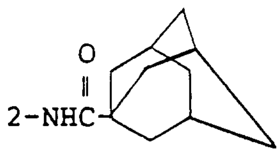

Crystalline form: White powder

Recrystallization solvent: Methanol

Melting Point: 160 - 161°C

Form: Free

Example 104

Structure

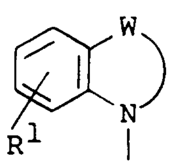 : 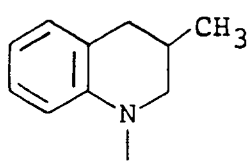 $R^2$: H

$R^3$: 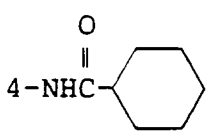

Crystalline form: White powder

Recrystallization solvent: Methanol

Melting Point: 175 - 176°C

Form: Free

Example 105

Structure

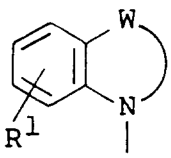 : 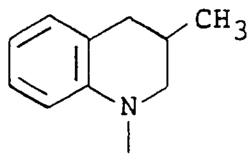

$R^2$: H

$R^3$: 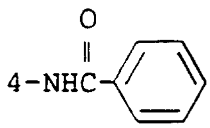

Crystalline form: White powder

Recrystallization solvent: Methanol

Melting Point: 197 - 198°C

Form: Free

Example 106

Structure

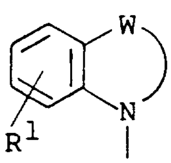 : 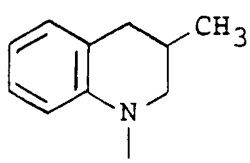

$R^2$: H

$R^3$: 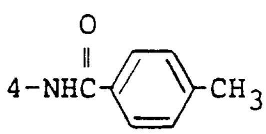

Crystalline form: White powder

Recrystallization solvent: Methanol

Melting Point: 204 - 205°C

Form: Free

Example 107

Structure

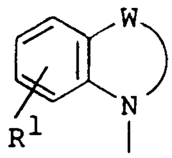 : 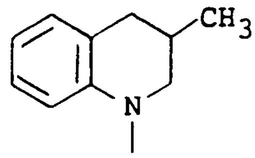

$R^2$: H

$R^3$: 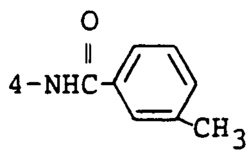

Crystalline form: White powder

Recrystallization solvent: Methanol

Melting Point: 174 - 175°C

Form: Free

Example 108

Structure

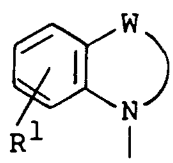 : 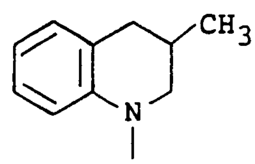

$R^2$: H

$R^3$: 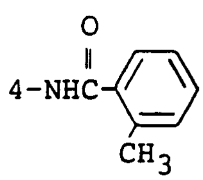

Crystalline form: Yellow powder

Recrystallization solvent: Methanol

Melting Point: 202 - 203°C

Form: Free

Example 109

Structure

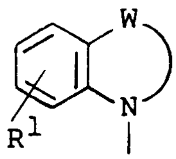 : 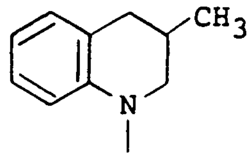

$R^2$: H

$R^3$: 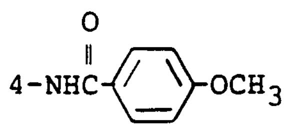

Crystalline form: White powder

Recrystallization solvent: Methanol

Melting Point: 203 - 204°C

Form: Free

Example 110

Structure

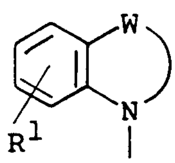 : 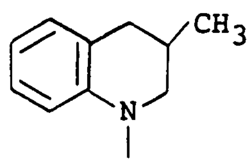

$R^2$: H

$R^3$: 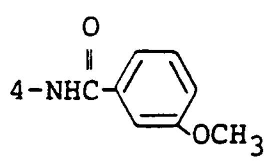

Crystalline form: White powder

Recrystallization solvent: Methanol

Melting Point: 170.5 - 171.5°C

Form: Free

Example 111

Structure

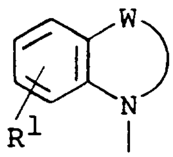 : 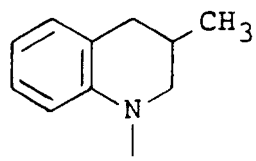  $R^2$: H

$R^3$: 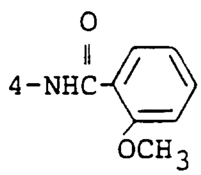

Crystalline form: White powder

Recrystallization solvent: Methanol

Melting Point: 149 - 150°C

Form: Free

Example 112

Structure

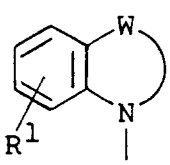 : 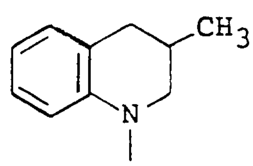  $R^2$: H

$R^3$: 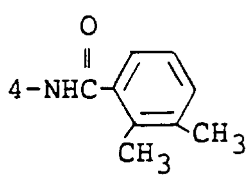

Crystalline form: White powder

Recrystallization solvent: Methanol

Melting Point: 185 - 186°C

Form: Free

Example 113

Structure

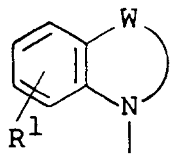 : 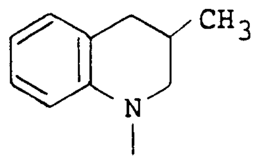

$R^2$: H

$R^3$: 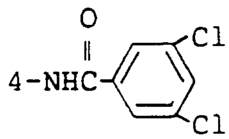

Crystalline form: White powder

Recrystallization solvent: Dichloromethane/ethanol

Melting Point: 225 - 226°C

Form: Free

Example 114

Structure

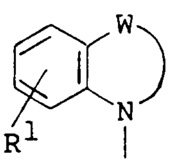 : 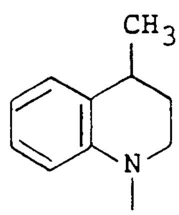

$R^2$: H

$R^3$: 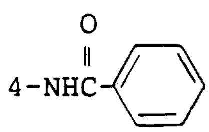

Crystalline form: White powder

Recrystallization solvent: Methanol

Melting Point: 234 - 235°C

Form: Free

Example 115

Structure

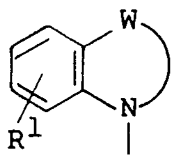

:

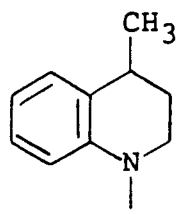

$R^2$: H

$R^3$: 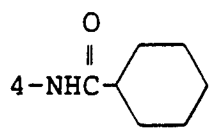

Crystalline form: White powder

Recrystallization solvent: Methanol

Melting Point: 149.5 - 150.5°C

Form: Free

Example 116

Structure

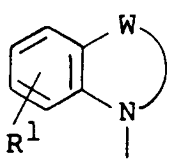

:

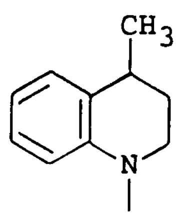

$R^2$: H

$R^3$: 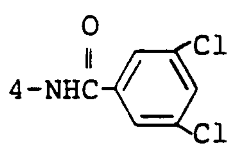

Crystalline form: White powder

Recrystallization solvent: Dichloromethane/ethanol

Melting Point: 197 - 198°C

Form: Free

Example 117

Structure

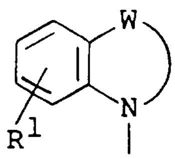 : 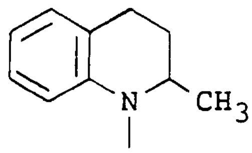

$R^2$: H

$R^3$: 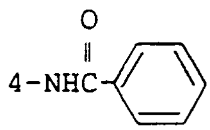

Crystalline form: White powder

Recrystallization solvent: Methanol

Melting Point: 204 - 205°C

Form: Free

---

Example 118

Structure

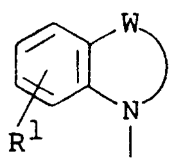 : 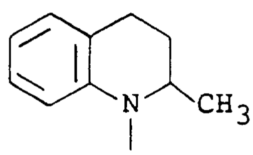

$R^2$: H

$R^3$: 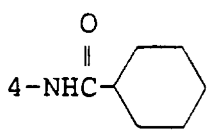

Crystalline form: White powder

Recrystallization solvent: Methanol

Melting Point: 224.5 - 225.5°C

Form: Free

Example 119

Structure

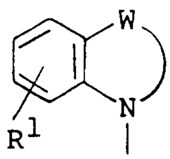 : 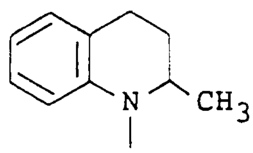

$R^2$: H

$R^3$: 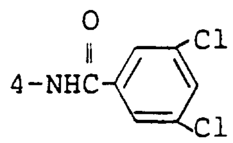

Crystalline form: White powder

Recrystallization solvent: Dichloromethane/ethanol

Melting Point: 189.5 - 190.5°C

Form: Free

Example 120

Structure

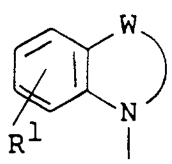 : 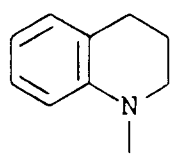

$R^2$: H

$R^3$: 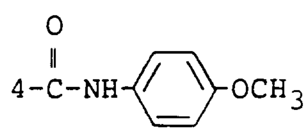

Crystalline form: White powder

Recrystallization solvent: Methanol

Melting Point: 221.5 - 222.5°C

Form: Free

Example 121

Structure

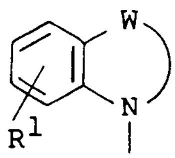 : 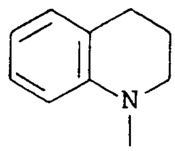 $R^2$: H

$R^3$: 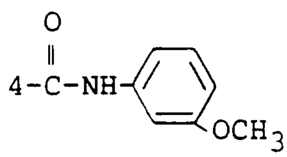

Crystalline form: Colorless needles

Recrystallization solvent: Methanol

Melting Point: 154 - 155°C

Form: Free

Example 122

Structure

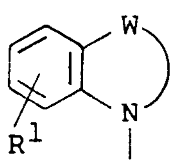 : 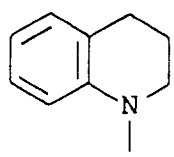 $R^2$: H

$R^3$: 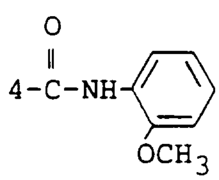

Crystalline form: White powder

Recrystallization solvent: Methanol

Melting Point: 165 - 166°C

Form: Free

Example 123

Structure

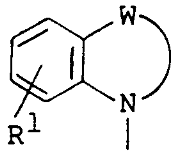 : 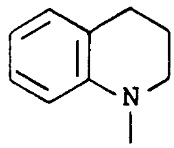

$R^2$: H

$R^3$: 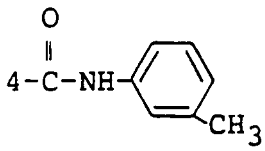

Crystalline form: Colorless needles

Recrystallization solvent: Methanol

Melting Point: 141 - 142°C

Form: Free

Example 124

Structure

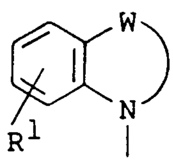 : 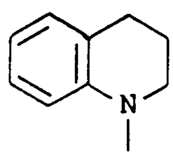

$R^2$: H

$R^3$: 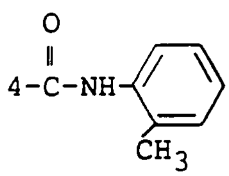

Crystalline form: White powder

Recrystallization solvent: Methanol

Melting Point: 165.5 - 166.5°C

Form: Free

Example 125

Structure

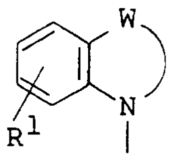 : 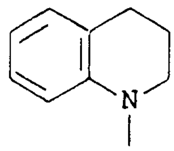

$R^2$: H

$R^3$: 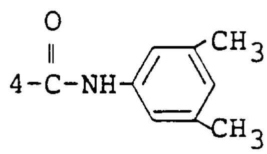

Crystalline form: Colorless needles

Recrystallization solvent: Methanol

Melting Point: 164 - 165°C

Form: Free

---

Example 126

Structure

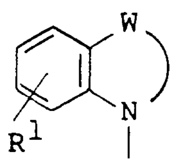 : 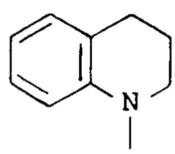

$R^2$: H

$R^3$: 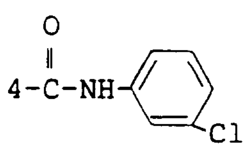

Crystalline form: White powder

Recrystallization solvent: Methanol

Melting Point: 203.5 - 204.5°C

Form: Free

Example 127

Structure

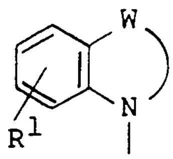 : 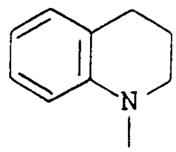

$R^2$: H

$R^3$: 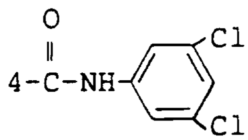

Crystalline form: White powder

Recrystallization solvent: Dichloromethane/ethanol

Melting Point: 236.5 - 237.5°C

Form: Free

Example 128

Structure

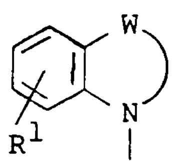 : 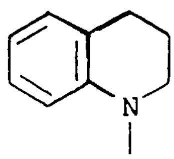

$R^2$: H

$R^3$: 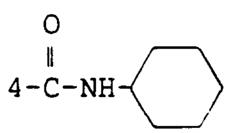

Crystalline form: White powder

Recrystallization solvent: Methanol

Melting Point: 206.5 - 207.5°C

Form: Free

Example 129

Structure

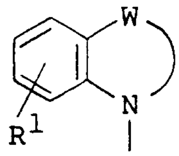

:

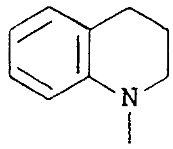

$R^2$: H

$R^3$:

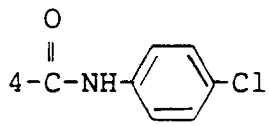

Crystalline form: White powder

Recrystallization solvent: Methanol

Melting Point: 271 - 272°C

Form: Free

Example 130

Structure

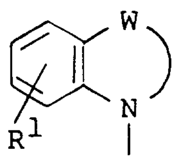

:

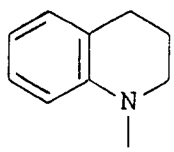

$R^2$: H

$R^3$:

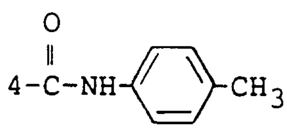

Crystalline form: White powder

Recrystallization solvent: Methanol

Melting Point: 246 - 247°C

Form: Free

Example 131

Structure

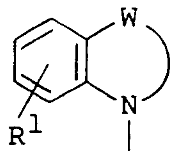 : 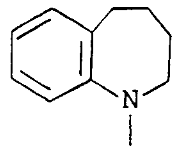

$R^2$: H

$R^3$: 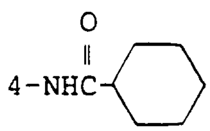

Crystalline form: White powder

Recrystallization solvent: Ethanol/diethyl ether

Melting Point: 210 - 211°C

Form: Free

Example 132

Structure

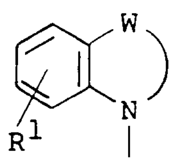 : 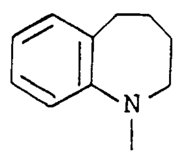

$R^2$: H

$R^3$: 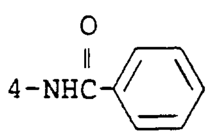

Crystalline form: White powder

Recrystallization solvent: Ethanol/diethyl ether

Melting Point: 230.5 - 231.5°C

Form: Free

Example 133

Structure

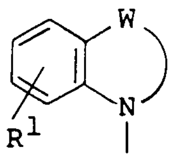 : 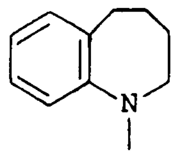

$R^2$: H

$R^3$: 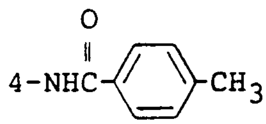

Crystalline form: White powder

Recrystallization solvent: Ethanol/diethyl ether

Melting Point: 203 - 204°C

Form: Free

Example 134

Structure

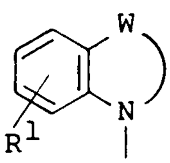 : 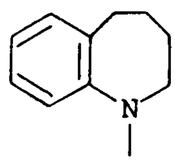

$R^2$: H

$R^3$: 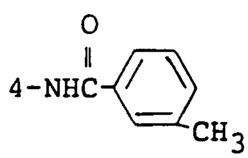

Crystalline form: White powder

Recrystallization solvent: Ethanol/diethyl ether

Melting Point: 170 - 171°C

Form: Free

Example 135

Structure

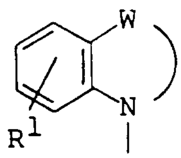 : 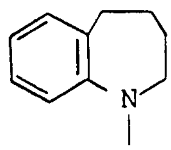

$R^2$: H

$R^3$: 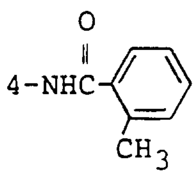

Crystalline form: White powder

Recrystallization solvent: Ethanol/diethyl ether

Melting Point: 225.5 - 226.5°C

Form: Free

Example 136

Structure

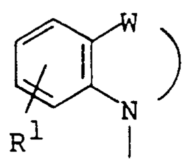 : 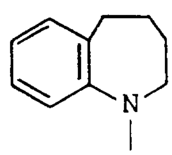

$R^2$: H

$R^3$: 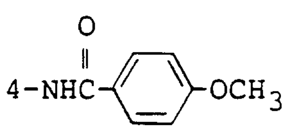

Crystalline form: White powder

Recrystallization solvent: Ethanol/diethyl ether

Melting Point: 210.5 - 211.5°C

Form: Free

Example 137

Structure

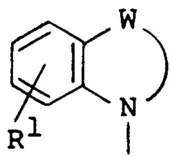 : 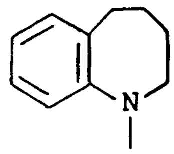

$R^2$: H

$R^3$: 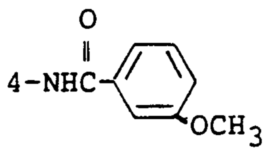

Crystalline form: White powder

Recrystallization solvent: Ethanol/diethyl ether

Melting Point: 183 - 184°C

Form: Free

Example 138

Structure

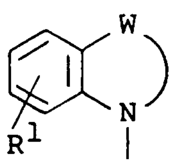 : 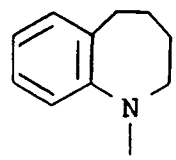

$R^2$: H

$R^3$: 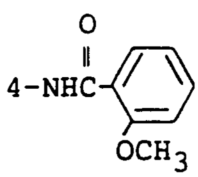

Crystalline form: White powder

Recrystallization solvent: Ethanol/diethyl ether

Melting Point: 191.5 - 192.5°C

Form: Free

Example 139

Structure

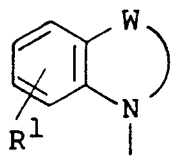 : 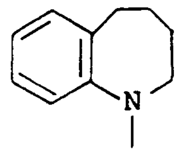

$R^2$: H

$R^3$: 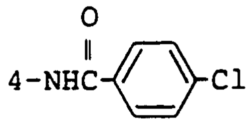

Crystalline form: White powder

Recrystallization solvent: Ethanol/diethyl ether

Melting Point: 203.5 - 204.5°C

Form: Free

Example 140

Structure

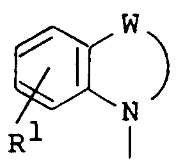 : 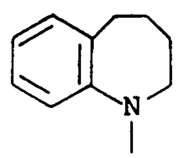

$R^2$: H

$R^3$: 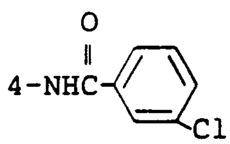

Crystalline form: White powder

Recrystallization solvent: Ethanol/diethyl ether

Melting Point: 215.5 - 216.5°C

Form: Free

Example 141

Structure

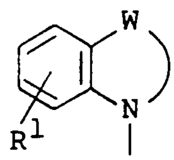 : 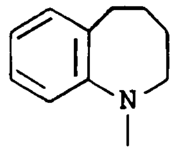

$R^2$: H

$R^3$: 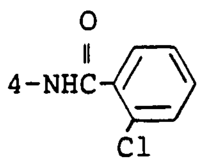

Crystalline form: White powder

Recrystallization solvent: Ethanol/diethyl ether

Melting Point: 211.5 - 212.5°C

Form: Free

Example 142

Structure

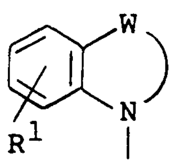 : 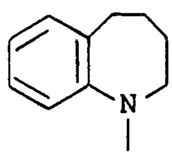

$R^2$: H

$R^3$: 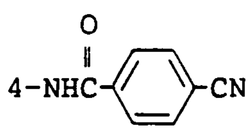

Crystalline form: White powder

Recrystallization solvent: Ethanol/diethyl ether

Melting Point: 280.5 - 281.5°C

Form: Free

Example 143

Structure

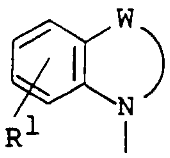 : 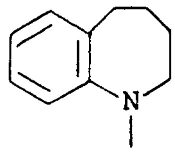

$R^2$: H

$R^3$: 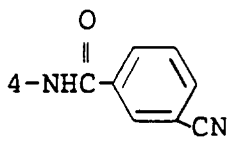

Crystalline form: White powder

Recrystallization solvent: Ethanol/diethyl ether

Melting Point: 235.5 - 236.5°C

Form: Free

Example 144

Structure

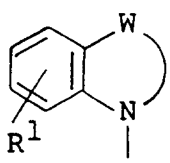 : 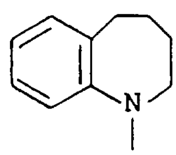

$R^2$: H

$R^3$: 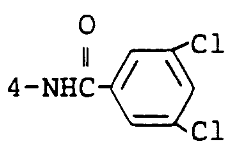

Crystalline form: White powder

Recrystallization solvent: Ethanol/dichloromethane

Melting Point: 249.5 - 250.5°C

Form: Free

Example 145

Structure

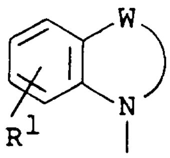 : 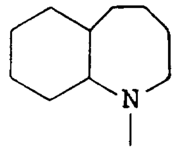 $R^2$: H

$R^3$: 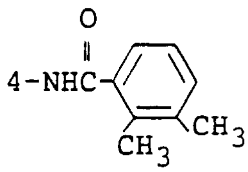

Crystalline form: White powder

Recrystallization solvent: Ethanol/diethyl ether

Melting Point: 217 - 218°C

Form: Free

Example 146

Structure

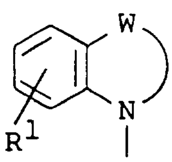 : 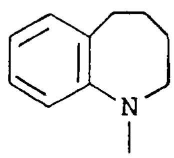 $R^2$: 3-$CH_3$

$R^3$: 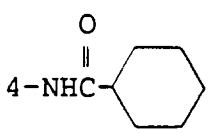

Crystalline form: White powder

Recrystallization solvent: Ethanol/diethyl ether

Melting Point: 201.5 - 203°C

Form: Free

Example 147

Structure

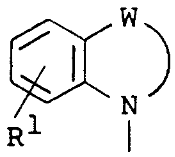 : 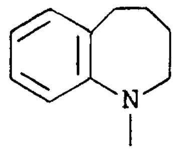

$R^2$: 3-$CH_3$

$R^3$: 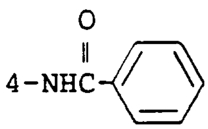

Crystalline form: White powder

Recrystallization solvent: Ethanol/diethyl ether

Melting Point: 221 - 222°C

Form: Free

Example 148

Structure

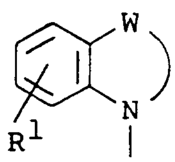 : 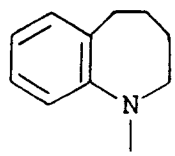

$R^2$: 3-$CH_3$

$R^3$: 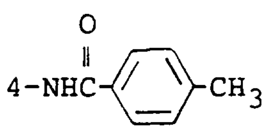

Crystalline form: White powder

Recrystallization solvent: Ethanol/diethyl ether

Melting Point: 193 - 194°C

Form: Free

Example 149

Structure

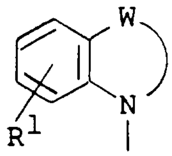 : 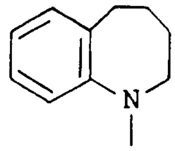

$R^2$: 3-$CH_3$

$R^3$: 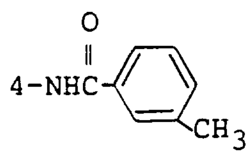

Crystalline form: White powder

Recrystallization solvent: Ethanol/diethyl ether

Melting Point: 176 - 177°C

Form: Free

Example 150

Structure

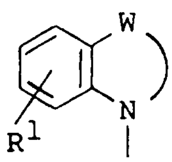 : 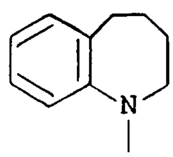

$R^2$: 3-$CH_3$

$R^3$: 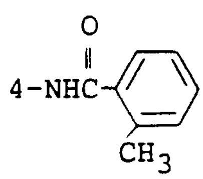

Crystalline form: White powder

Recrystallization solvent: Ethanol/diethyl ether

Melting Point: 188 - 189.5°C

Form: Free

Example 151

Structure

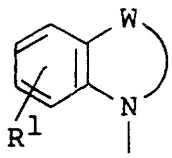 : 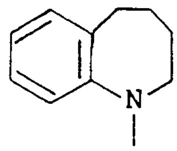

$R^2$: 3-$CH_3$

$R^3$: 4-NHC- 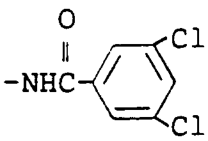

Crystalline form: White powder

Recrystallization solvent: Ethanol/diethyl ether

Melting Point: 227 - 228°C

Form: Free

Example 152

Structure

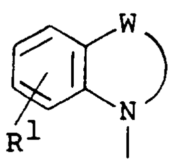 : 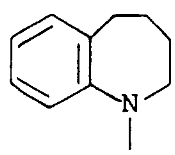

$R^2$: 3-$CH_3$

$R^3$: 4-NHC- 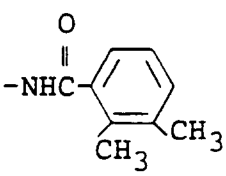

Crystalline form: White powder

Recrystallization solvent: Ethanol/diethyl ether

Melting Point: 186 - 187°C

Form: Free

Example 153

Structure

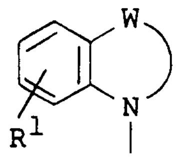 : 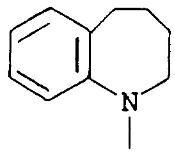

$R^2$: 3-$OCH_3$

$R^3$: 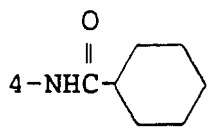

Crystalline form: White powder

Recrystallization solvent: Ethanol/diethyl ether

Melting Point: 135 - 136°C

Form: Free

Example 154

Structure

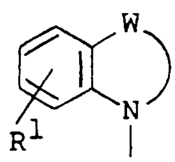 : 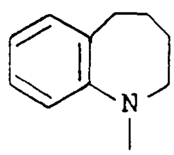

$R^2$: 3-$OCH_3$

$R^3$: 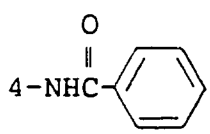

Crystalline form: White powder

Recrystallization solvent: Ethanol/diethyl ether

Melting Point: 173 - 174°C

Form: Free

Example 155

Structure

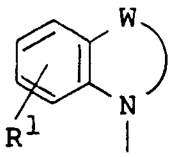 : 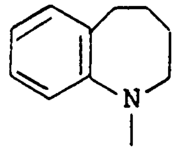 $R^2$: 3-$OCH_3$

$R^3$: 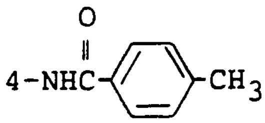

Crystalline form: White powder

Recrystallization solvent: Ethanol/diethyl ether

Melting Point: 174.5 - 175.5°C

Form: Free

Example 156

Structure

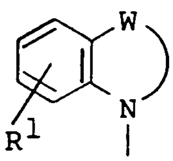 : 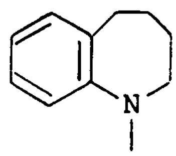 $R^2$: 3-$OCH_3$

$R^3$: 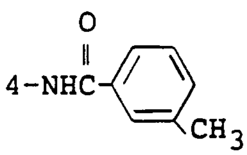

Crystalline form: White powder

Recrystallization solvent: Ethanol/diethyl ether

Melting Point: 156 - 157°C

Form: Free

Example 157

Structure

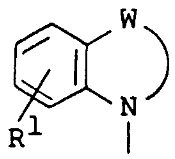 : 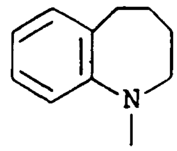

$R^2$: 3-$OCH_3$

$R^3$: 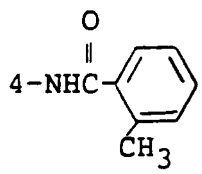

Crystalline form: White powder

Recrystallization solvent: Ethanol/diethyl ether

Melting Point: 153 - 154°C

Form: Free

Example 158

Structure

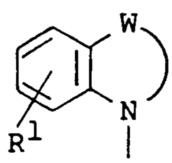 : 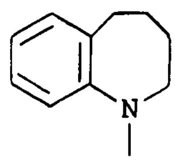

$R^2$: 3-$OCH_3$

$R^3$: 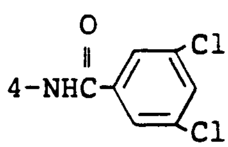

Crystalline form: White powder

Recrystallization solvent: Ethanol/diethyl ether

Melting Point: 169 - 170°C

Form: Free

Example 159

Structure

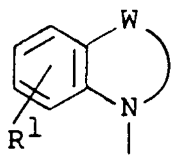 : 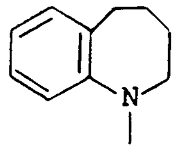

$R^2$: 3-$OCH_3$

$R^3$: 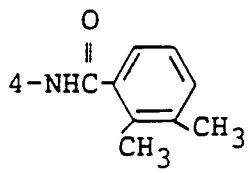

Crystalline form: White powder

Recrystallization solvent: Ethanol/diethyl ether

Melting Point: 185 - 186°C

Form: Free

Example 160

Structure

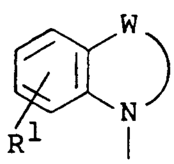 : 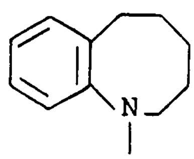

$R^2$: H

$R^3$: 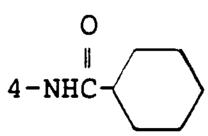

Crystalline form: White powder

Recrystallization solvent: Ethanol/diethyl ether

Melting Point: 213 - 214°C

Form: Free

Example 161

Structure

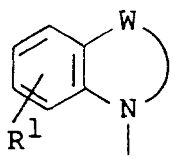 : 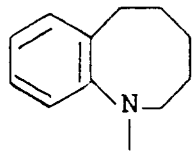 $R^2$: H

$R^3$: 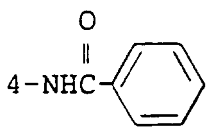

Crystalline form: White powder

Recrystallization solvent: Ethanol/diethyl ether

Melting Point: 240 - 241°C

Form: Free

Example 162

Structure

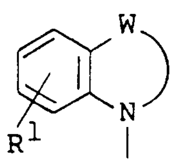 : 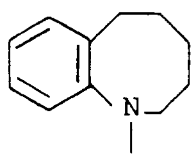 $R^2$: H

$R^3$: 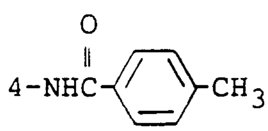

Crystalline form: White powder

Recrystallization solvent: Ethanol/diethyl ether

Melting Point: 225 - 226°C

Form: Free

Example 163

Structure

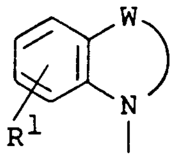 : 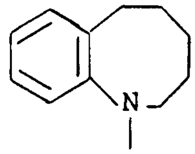

$R^2$: H

$R^3$: 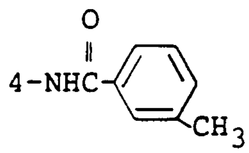

Crystalline form: White powder

Recrystallization solvent: Ethanol/diethyl ether

Melting Point: 209.5 - 210.5°C

Form: Free

Example 164

Structure

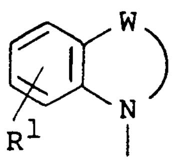 : 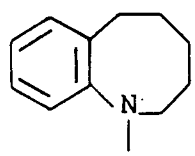

$R^2$: H

$R^3$: 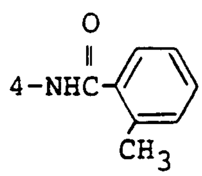

Crystalline form: White powder

Recrystallization solvent: Ethanol/diethyl ether

Melting Point: 198 - 199°C

Form: Free

Example 165

Structure

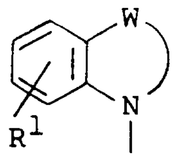 : 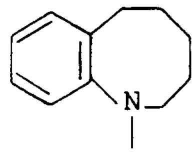

$R^2$: H

$R^3$: 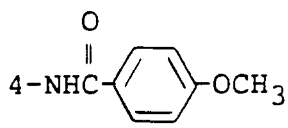

Crystalline form: White powder

Recrystallization solvent: Ethanol/diethyl ether

Melting Point: 214.5 - 215.5°C

Form: Free

Example 166

Structure

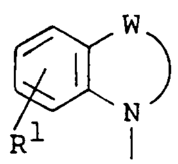 : 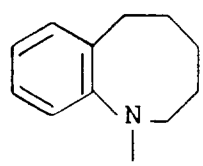

$R^2$: H

$R^3$: 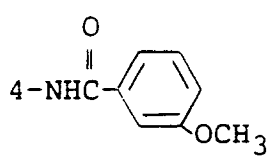

Crystalline form: White powder

Recrystallization solvent: Ethanol/diethyl ether

Melting Point: 196.5 - 197.5°C

Form: Free

Example 167

Structure

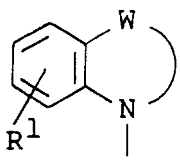 : 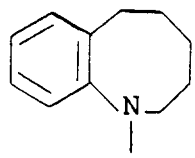 $R^2$: H

$R^3$: 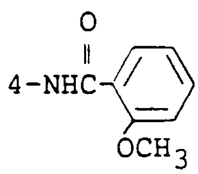

Crystalline form: White powder

Recrystallization solvent: Ethanol/diethyl ether

Melting Point: 194 - 195°C

Form: Free

Example 168

Structure

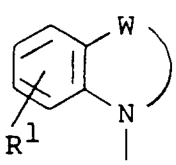 : 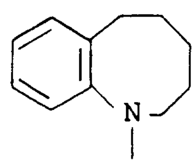 $R^2$: H

$R^3$: 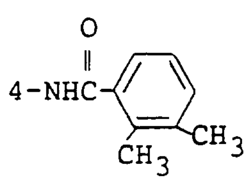

Crystalline form: White powder

Recrystallization solvent: Ethanol/diethyl ether

Melting Point: 191 - 192°C

Form: Free

Example 169

Structure

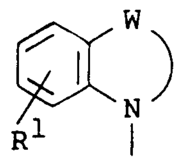 : 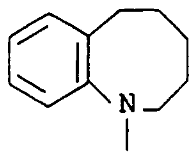 $R^2$: H

$R^3$: 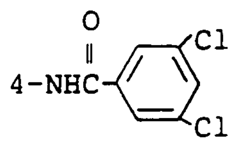

Crystalline form: White powder

Recrystallization solvent: Dichloromethane/ethanol

Melting Point: 227 - 228°C

Form: Free

Example 170

Structure

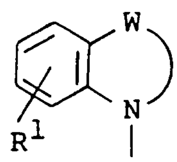 : 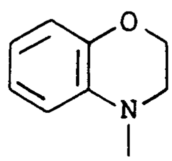 $R^2$: H

$R^3$: 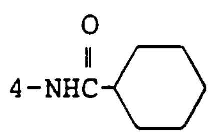

Crystalline form: White powder

Recrystallization solvent: Dichloromethane/diethyl ether

Melting Point: 182 - 183°C

Form: Free

Example 171

Structure

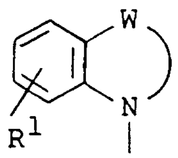 : 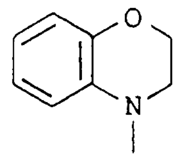

$R^2$: H

$R^3$: 4-NHC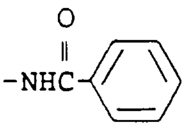

Crystalline form: White powder

Recrystallization solvent: Dichloromethane/diethyl ether

Melting Point: 222 - 223°C

Form: Free

---

Example 172

Structure

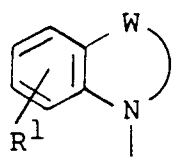 : 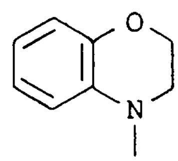

$R^2$: H

$R^3$: 4-NHC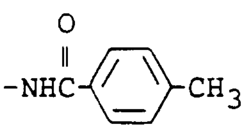

Crystalline form: White powder

Recrystallization solvent: Dichloromethane/diethyl ether

Melting Point: 204 - 205°C

Form: Free

Example 173

Structure

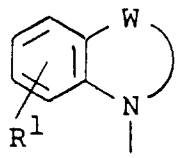 : 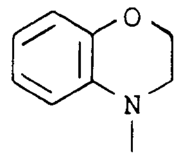

$R^2$: H

$R^3$: 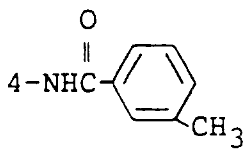

Crystalline form: White powder

Recrystallization solvent: Dichloromethane/diethyl ether

Melting Point: 194 - 195°C

Form: Free

Example 174

Structure

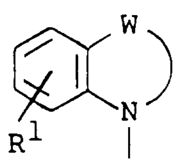 : 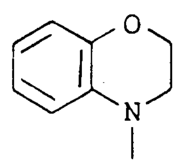

$R^2$: H

$R^3$: 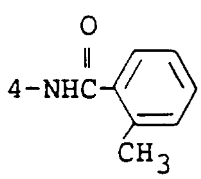

Crystalline form: White powder

Recrystallization solvent: Dichloromethane/diethyl ether

Melting Point: 213 - 214°C

Form: Free

Example 175

Structure

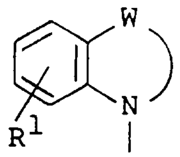 : 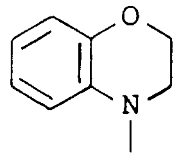

$R^2$: H

$R^3$: 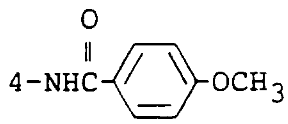

Crystalline form: White powder

Recrystallization solvent: Dichloromethane/diethyl ether

Melting Point: 201 - 202°C

Form: Free

Example 176

Structure

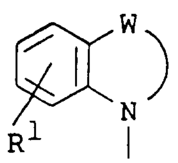 : 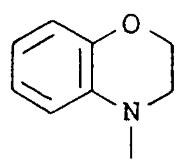

$R^2$: H

$R^3$: 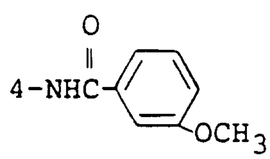

Crystalline form: Colorless needles

Recrystallization solvent: Dichloromethane/diethyl ether

Melting Point: 173 - 174°C

Form: Free

Example 177

Structure

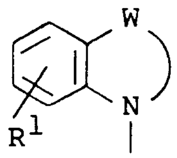 : 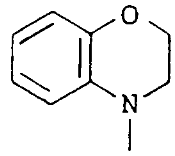

$R^2$: H

$R^3$: 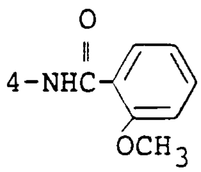

Crystalline form: White powder

Recrystallization solvent: Dichloromethane/diethyl ether

Melting Point: 150.5 - 151.5°C

Form: Free

Example 178

Structure

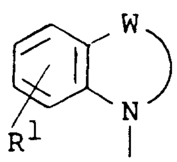 : 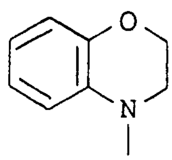

$R^2$: H

$R^3$: 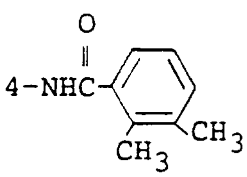

Crystalline form: White powder

Recrystallization solvent: Dichloromethane/diethyl ether

Melting Point: 207.5 - 208.5°C

Form: Free

Example 179

Structure

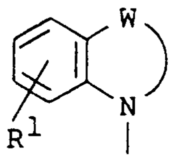 : 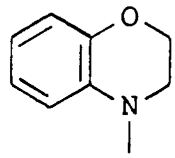 $R^2$: H

$R^3$: 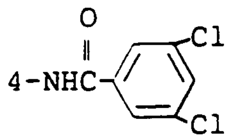

Crystalline form: White powder

Recrystallization solvent: Dichloromethane/diethyl ether

Melting Point: 256.5 - 257.5°C

Form: Free

Example 180

Structure

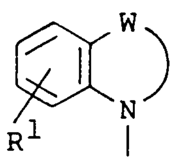 : 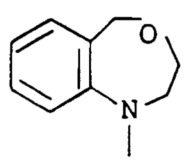 $R^2$: H

$R^3$: 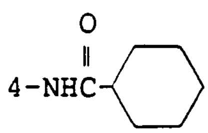

Crystalline form: White powder

Recrystallization solvent: Ethanol/diethyl ether

Melting Point: 199.5 - 200.5°C

Form: Free

Example 181

Structure

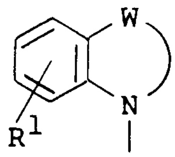 : 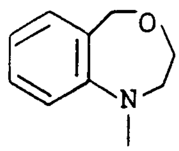

$R^2$: H

$R^3$: 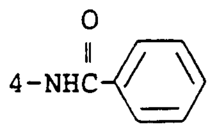

Crystalline form: White powder

Recrystallization solvent: Ethanol/diethyl ether

Melting Point: 211 - 212°C

Form: Free

Example 182

Structure

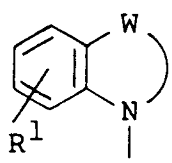 : 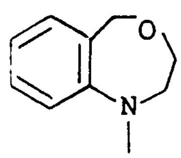

$R^2$: H

$R^3$: 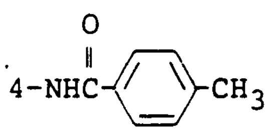

Crystalline form: White powder

Recrystallization solvent: Ethanol/diethyl ether

Melting Point: 189.5 - 190.5°C

Form: Free

Example 183

Structure

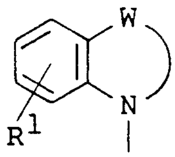 : 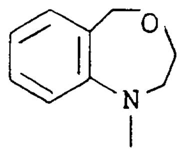

$R^2$: H

$R^3$: 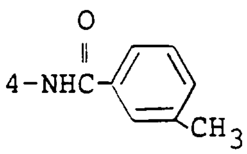

Crystalline form: White powder

Recrystallization solvent: Ethanol/diethyl ether

Melting Point: 176.5 - 177.5°C

Form: Free

Example 184

Structure

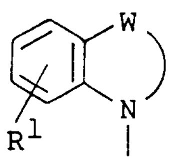 : 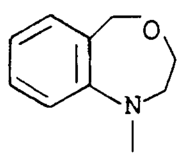

$R^2$: H

$R^3$: 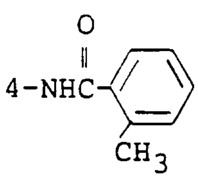

Crystalline form: Yellow powder

Recrystallization solvent: Ethanol/diethyl ether

Melting Point: 202 - 203°C

Form: Free

Example 185

Structure

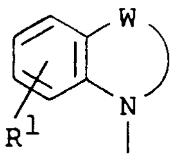 : 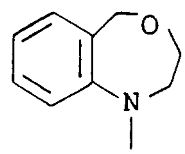 $R^2$: H

$R^3$: 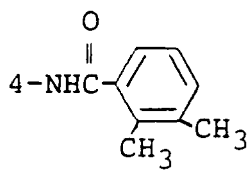

Crystalline form: White powder

Recrystallization solvent: Ethanol/diethyl ether

Melting Point: 219 - 220°C

Form: Free

Example 186

Structure

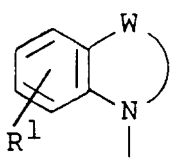 : 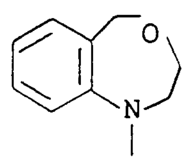 $R^2$: H

$R^3$: 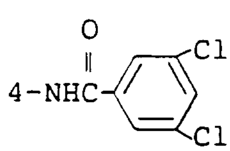

Crystalline form: White powder

Recrystallization solvent: Ethanol/diethyl ether

Melting Point: 272 - 273°C

Form: Free

Example 187

Structure

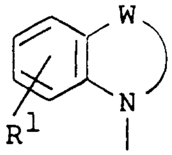 : 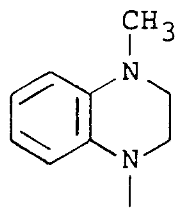

$R^2$: H

$R^3$: 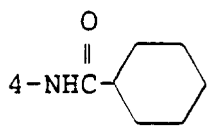

Crystalline form: Yellow powder

Recrystallization solvent: Ethanol/diethyl ether

Melting Point: 146 - 147°C

Form: Free

Example 188

Structure

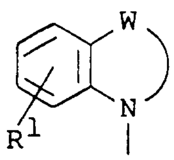 : 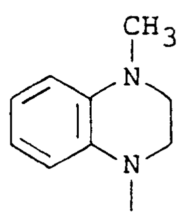

$R^2$: H

$R^3$: 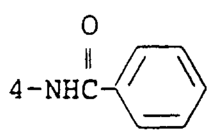

Crystalline form: Yellow powder

Recrystallization solvent: Ethanol/diethyl ether

Melting Point: 229.5 - 230.5°C

Form: Free

Example 189

Structure

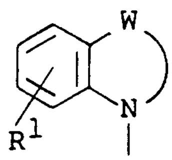 : 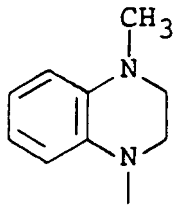

$R^2$: H

$R^3$: 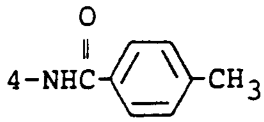

Crystalline form: Yellow powder

Recrystallization solvent: Ethanol/diethyl ether

Melting Point: 119.5 - 120.5°C

Form: Free

Example 190

Structure

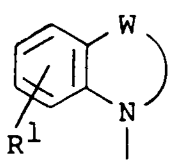 : 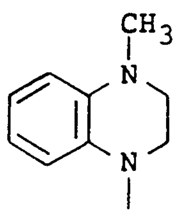

$R^2$: H

$R^3$: 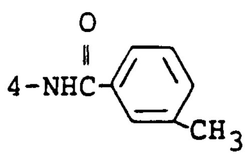

Crystalline form: Yellow powder

Recrystallization solvent: Ethanol/diethyl ether

Melting Point: 189 - 190°C

Form: Free

Example 191

Structure

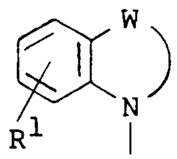 : 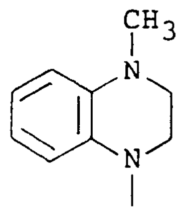

$R^2$: H

$R^3$: 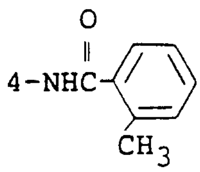

Crystalline form: Yellow powder

Recrystallization solvent: Ethanol/diethyl ether

Melting Point: 207 - 208°C

Form: Free

Example 192

Structure

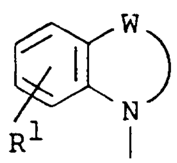 : 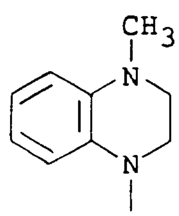

$R^2$: H

$R^3$: 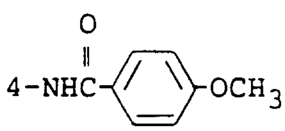

Crystalline form: Yellow powder

Recrystallization solvent: Ethanol/diethyl ether

Melting Point: 196.5 - 197.5°C

Form: Free

Example 193

Structure

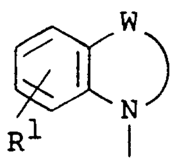 : 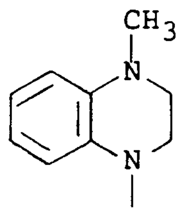

$R^2$: H

$R^3$: 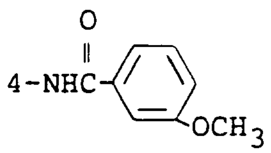

Crystalline form: Yellow powder

Recrystallization solvent: Ethanol/diethyl ether

Melting Point: 182 - 183°C

Form: Free

Example 194

Structure

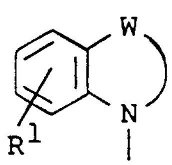 : 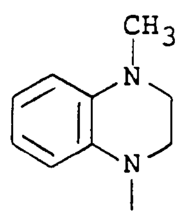

$R^2$: H

$R^3$: 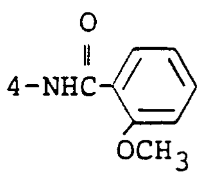

Crystalline form: Yellow powder

Recrystallization solvent: Ethanol/diethyl ether

Melting Point: 172 - 173°C

Form: Free

Example 195

Structure

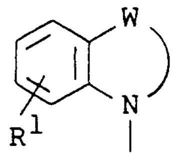 : 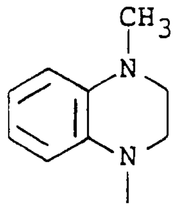 $R^2$: H

$R^3$: 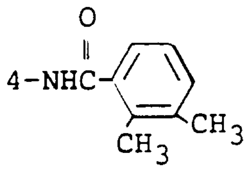

Crystalline form: Yellow powder

Recrystallization solvent: Ethanol/diethyl ether

Melting Point: 197.5 - 198.5°C

Form: Free

Example 196

Structure

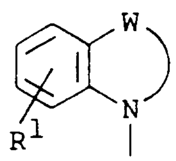 : 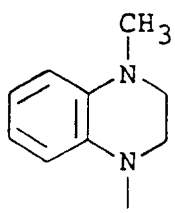 $R^2$: H

$R^3$: 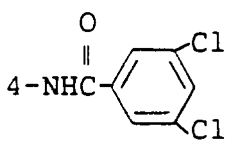

Crystalline form: Yellow powder

Recrystallization solvent: Ethanol/diethyl ether

Melting Point: 227 - 228°C

Form: Free

Example 197

Structure

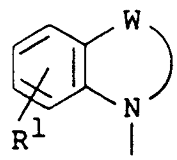 : 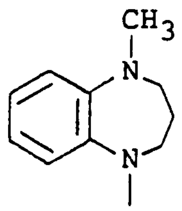 $R^2$: H

$R^3$: 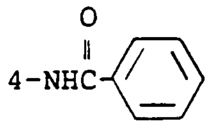

Crystalline form: White powder

Recrystallization solvent: Ethanol/diethyl ether

Melting Point: 216.5 - 217.5°C

Form: Free

Example 198

Structure

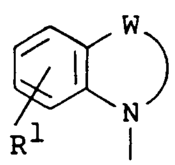 : 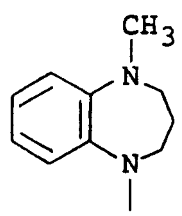 $R^2$: H

$R^3$: 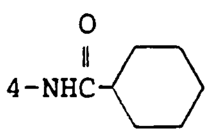

Crystalline form: White powder

Recrystallization solvent: Ethanol/diethyl ether

Melting Point: 207 - 208°C

Form: Free

Example 199

Structure

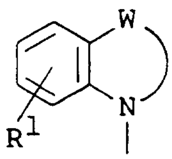 : 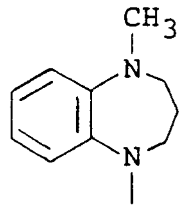

$R^2$: H

$R^3$: 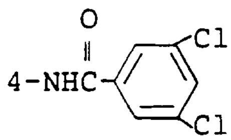

Crystalline form: White powder

Recrystallization solvent: Ethanol/diethyl ether

Melting Point: 236 - 237°C

Form: Free

Example 200

Structure

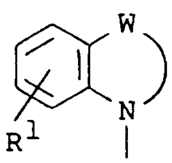 : 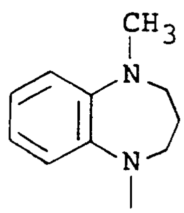

$R^2$: H

$R^3$: 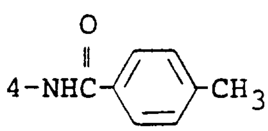

Crystalline form: White powder

Recrystallization solvent: Ethanol/diethyl ether

Melting Point: 199.5 - 200.5°C

Form: Free

Example 201

Structure

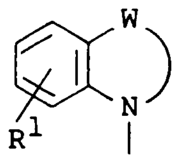 : 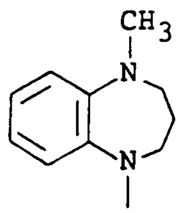

$R^2$: H

$R^3$: 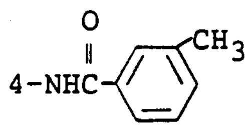

Crystalline form: White powder

Recrystallization solvent: Ethanol/diethyl ether

Melting Point: 171.5 - 172.5°C

Form: Free

Example 202

Structure

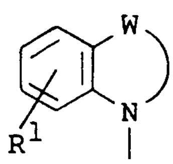 : 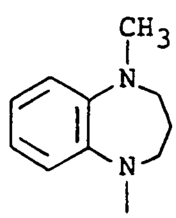

$R^2$: H

$R^3$: 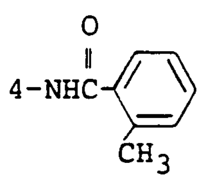

Crystalline form: White powder

Recrystallization solvent: Ethanol/diethyl ether

Melting Point: 222.5 - 223.5°C

Form: Free

Example 203

Structure

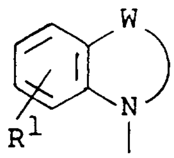 : 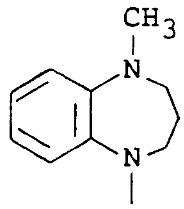

$R^2$: H

$R^3$: 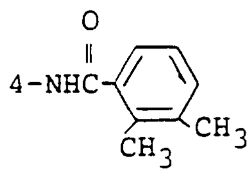

Crystalline form: White powder

Recrystallization solvent: Ethanol/diethyl ether

Melting Point: 209.5 - 210.5°C

Form: Free

Example 204

Structure

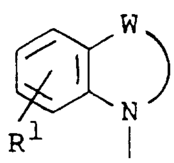 : 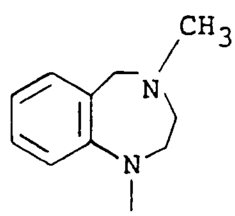

$R^2$: H

$R^3$: 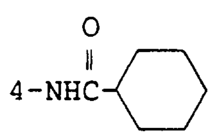

Crystalline form: White powder

Recrystallization solvent: Ethanol/water

NMR analysis: 14)

Form: Hydrochloride

Example 205

Structure

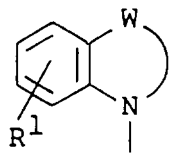 : 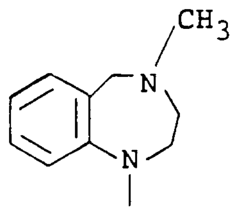 $R^2$: H

$R^3$: 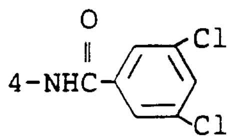

Crystalline form: White powder

Recrystallization solvent: Ethanol/water

NMR analysis: 15)

Form: Hydrochloride

Example 206

Structure

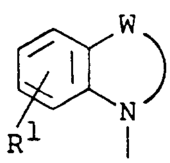 : 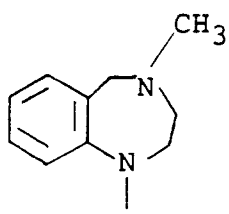 $R^2$: H

$R^3$: 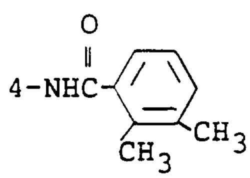

Crystalline form: White powder

Recrystallization solvent: Ethanol/water

NMR analysis: 16)

Form: Hydrochloride

Example 207

Structure

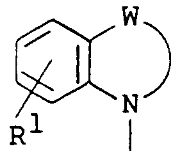 : 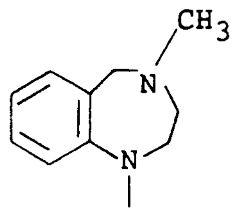 $R^2$: H

$R^3$: 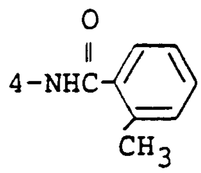

Crystalline form: White powder

Recrystallization solvent: Ethanol/water

NMR analysis: 17)

Form: Hydrochloride

Example 208

Structure

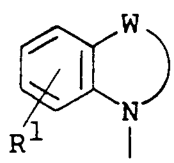 : 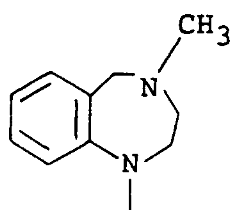 $R^2$: H

$R^3$: 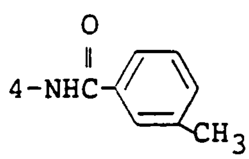

Crystalline form: White powder

Recrystallization solvent: Ethanol/water

NMR analysis: 18)

Form: Hydrochloride

Example 209

Structure

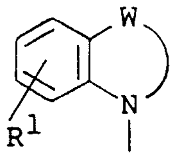 : 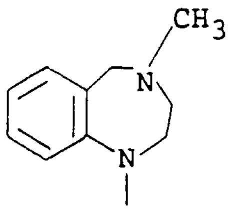

$R^2$: H

$R^3$: 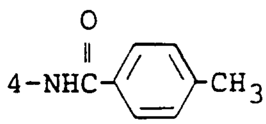

Crystalline form: Yellow powder

Recrystallization solvent: Ethanol/water

NMR analysis: 19)

Form: Hydrochloride

Example 210

Structure

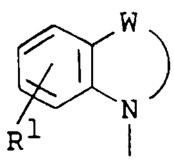 : 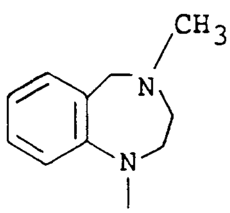

$R^2$: H

$R^3$: 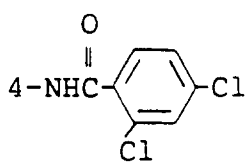

Crystalline form: White powder

Recrystallization solvent: Ethanol/water

NMR analysis: 20)

Form: Hydrochloride

Example 211

Structure

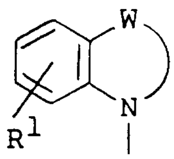 : 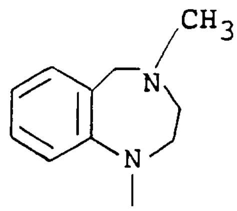 $R^2$: H

$R^3$: 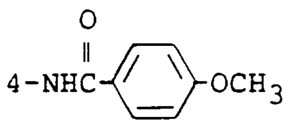

Crystalline form: White powder

Recrystallization solvent: Ethanol/diethyl ether

Melting Point: 159.5 - 160.5°C

Form: Free

Example 212

Structure

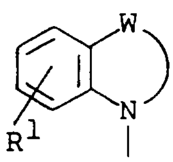 : 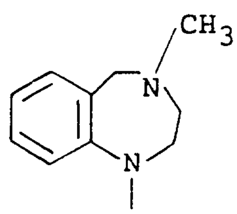 $R^2$: H

$R^3$: 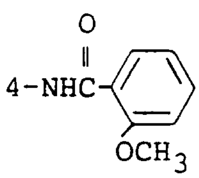

Crystalline form: White powder

Recrystallization solvent: Ethanol/diethyl ether

Melting Point: 189.5 - 190.5°C

Form: Free.

Example 213

Structure

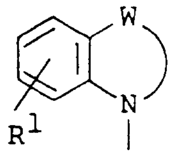 : 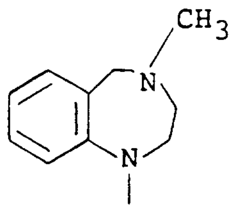 $R^2$: H

$R^3$: 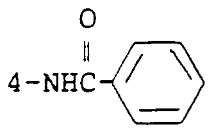

Crystalline form: White powder

Recrystallization solvent: Ethanol/diethyl ether

Melting Point: 170.5 - 171.5°C

Form: Free

Example 214

Structure

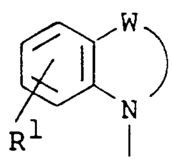 : 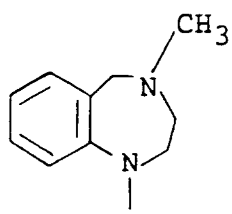 $R^2$: H

$R^3$: 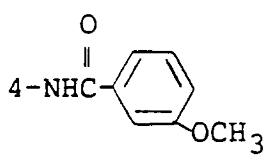

Crystalline form: White powder

Recrystallization solvent: Ethanol/diethyl ether

Melting Point: 165 - 166°C

Form: Free

Example 215

Structure

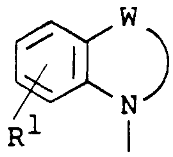 : 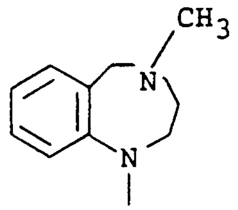

$R^2$: H

$R^3$: 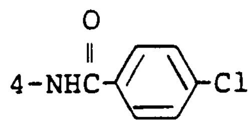

Crystalline form: White powder

Recrystallization solvent: Ethanol/diethyl ether

Melting Point: 173.5 - 174.5°C

Form: Free

Example 216

Structure

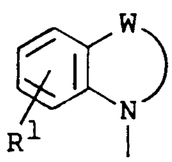 : 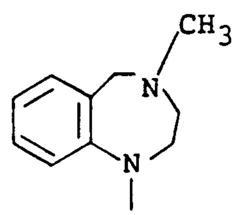

$R^2$: H

$R^3$: 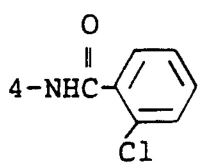

Crystalline form: White powder

Recrystallization solvent: Ethanol/diethyl ether

Melting Point: 182 - 183°C

Form: Free

Example 217

Structure

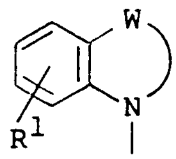 : 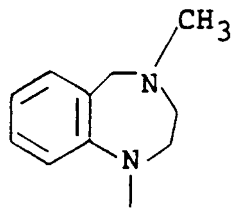

$R^2$: H

$R^3$: 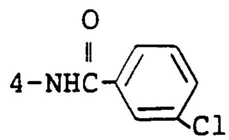

Crystalline form: White powder

Recrystallization solvent: Ethanol/diethyl ether

Melting Point: 225.5 - 226.5°C

Form: Free

Example 219

Structure

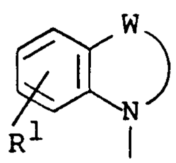 : 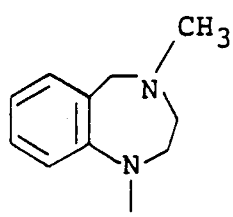

$R^2$: 3-$OCH_3$

$R^3$: 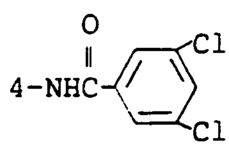

Crystalline form: White powder

Recrystallization solvent: Ethanol/water

NMR analysis: 21)

Form: Hydrochloride

Example 220

Structure

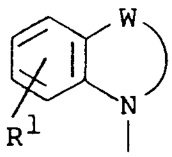 : 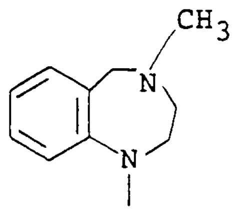 $R^2$: 3-$OCH_3$

$R^3$: 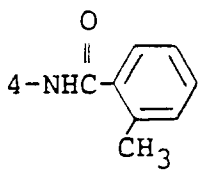

Crystalline form: White powderr

Recrystallization solvent: Ethanol/diethyl ether

Melting Point: 147.5 - 148.5°C

Form: Free

Example 221

Structure

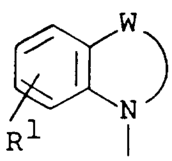 : 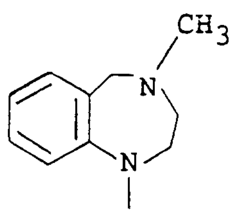 $R^2$: 3-$OCH_3$

$R^3$: 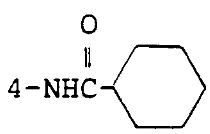

Crystalline form: White powder

Recrystallization solvent: Ethanol/diethyl ether

Melting Point: 136 - 137°C

Form: Free

Example 222

Structure

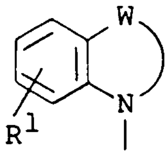 : 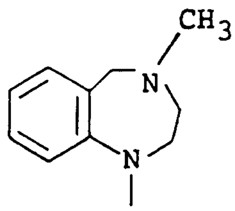

$R^2$: 3-$OCH_3$

$R^3$: 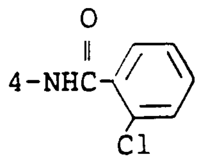

Crystalline form: White powder

Recrystallization solvent: Ethanol/diethyl ether

Melting Point: 191.5 - 192.5°C

Form: Free

Example 223

Structure

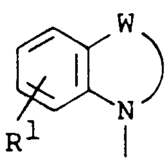 : 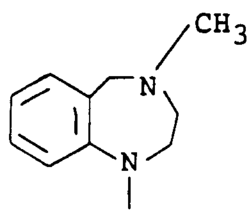

$R^2$: 3-$OCH_3$

$R^3$: 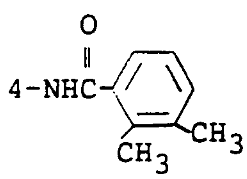

Crystalline form: White powder

Recrystallization solvent: Ethanol/diethyl ether

Melting Point: 145 - 146°C

Form: Free

Example 224

Structure

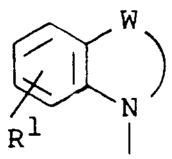 : 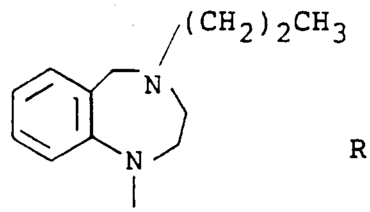 $R^2$: H

$R^3$: 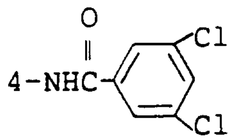

Crystalline form: White powder

Recrystallization solvent: Ethanol/water

NMR analysis: 22)

Form: Hydrochloride

Example 225

Structure

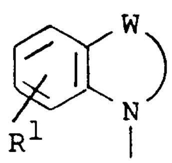 : 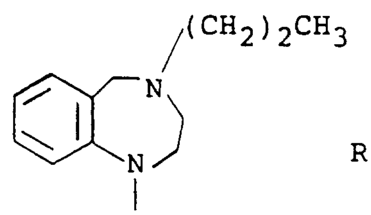 $R^2$: H

$R^3$: 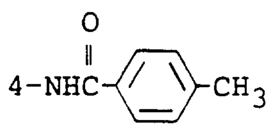

Crystalline form: White powder

Recrystallization solvent: Ethanol/water

NMR analysis: 23)

Form: Hydrochloride

Example 226

Structure

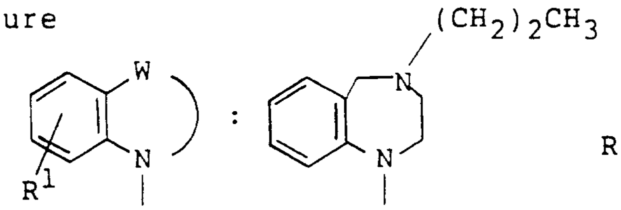

$R^2$: H

$R^3$: 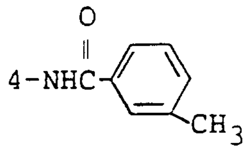

Crystalline form: White powder

Recrystallization solvent: Ethanol/water

NMR analysis: 24)

Form: Hydrochloride

Example 227

Structure

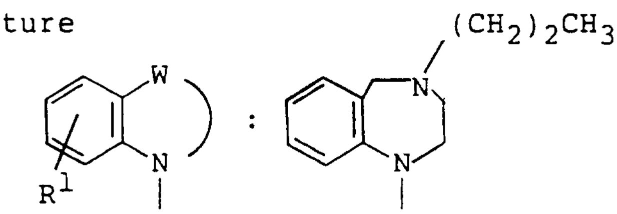

$R^2$: H

$R^3$: 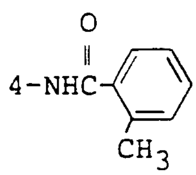

Crystalline form: White powder

Recrystallization solvent: Ethanol/water

NMR analysis: 25)

Form: Hydrochloride

Example 228

Structure

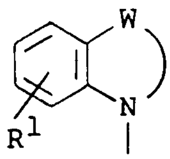

:

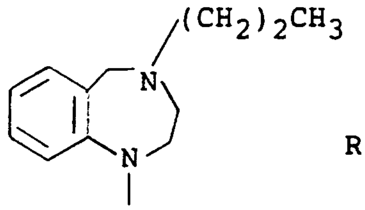

$R^2$: H

$R^3$: 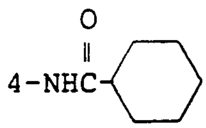

Crystalline form: White powder

Recrystallization solvent: Ethanol/water

NMR analysis: 26)

Form: Hydrochloride

Example 229

Structure

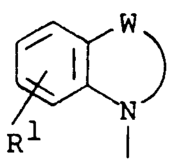

:

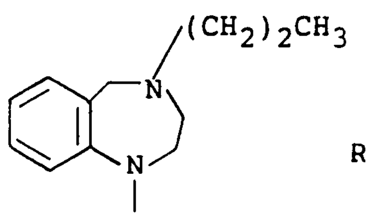

$R^2$: H

$R^3$: 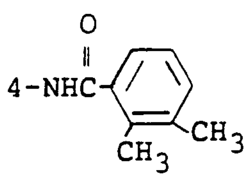

Crystalline form: White powder

Recrystallization solvent: Ethanol/water

NMR analysis: 27)

Form: Hydrochloride

Example 230

Structure

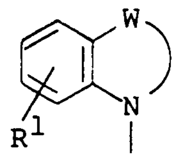 : 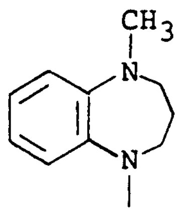

$R^2$: H

$R^3$: 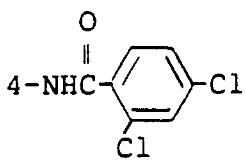

Crystalline form: White powder

Recrystallization solvent: Ethanol/diethyl ether

Melting Point: 206 - 207°C

Form: Free

Example 231

Structure

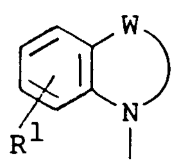 : 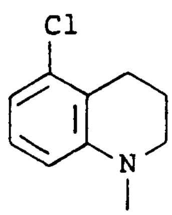

$R^2$: H

$R^3$: 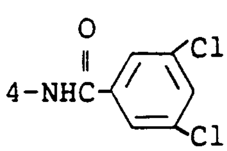

Crystalline form: White powder

Recrystallization solvent: Chloroform/methanol

Melting Point: 211 - 213°C

Form: Free

Example 232

Structure

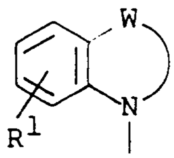

:

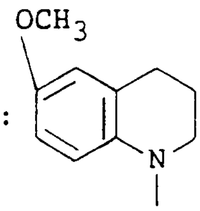

$R^2$: H

$R^3$: 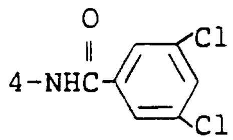

Crystalline form: White powder

Recrystallization solvent: Chloroform/methanol

Melting Point: 228.5 - 229.5°C

Form: Free

Example 233

Structure

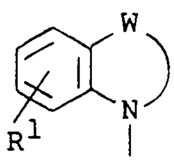

:

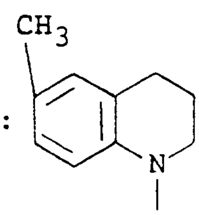

$R^2$: H

$R^3$: 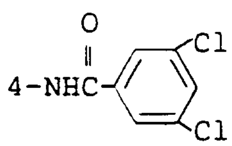

Crystalline form: White powder

Recrystallization solvent: Chloroform/methanol

Melting Point: 237 - 238°C

Form: Free

Example 234

Structure

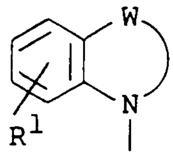 : 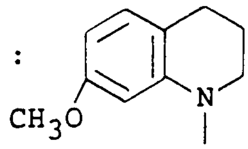 $R^2$: H

$R^3$: 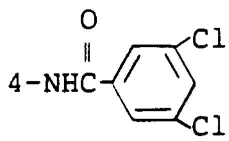

Crystalline form: White powder

Recrystallization solvent: Chloroform/methanol

Melting Point: 226 - 228°C

Form: Free

Example 235

Structure

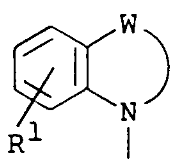 : 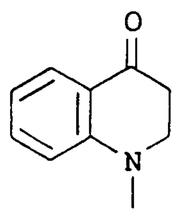 $R^2$: H

$R^3$: 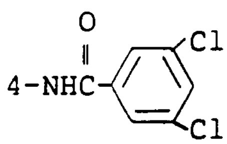

Crystalline form: White powder

Recrystallization solvent: Chloroform/methanol

Melting Point: 220 - 222°C

Form: Free

Example 236

Structure

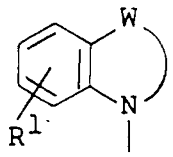
:
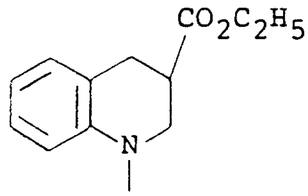

$R^2$: H

$R^3$: 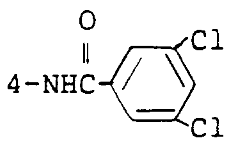

Crystalline form: Colorless amorphous

NMR analysis: 28)

Form: Free

Example 237

Structure

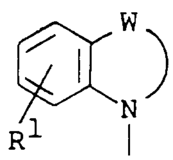
:
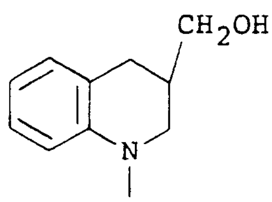

$R^2$: H

$R^3$: 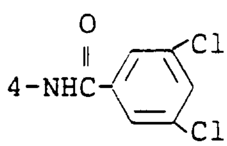

Crystalline form: White powder

Recrystallization solvent: Ethanol

Melting Point: 162 - 165°C

Form: Free

Example 238

Structure

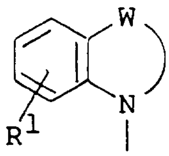 : 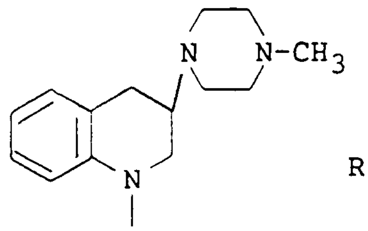 $R^2$: H

$R^3$: 4-NHC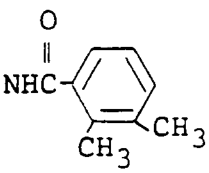

Crystalline form: Light brown amorphous

NMR analysis: 29)

Form: Free

Example 239

Structure

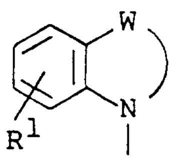 : 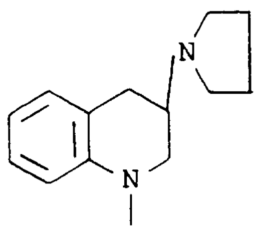 $R^2$: H

$R^3$: 4-NHC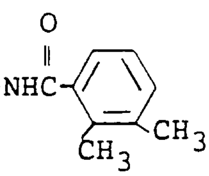

Crystalline form: Light brown amorphous

NMR analysis: 30)

Form: Free

Example 240

Structure

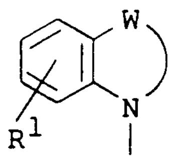 : 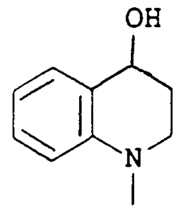

$R^2$: H

$R^3$: 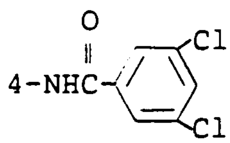

Crystalline form: White powder

Recrystallization solvent: Ethanol

Melting Point: 215 - 217°C

Form: Free

Example 241

Structure

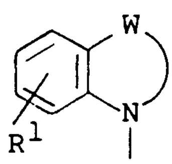 : 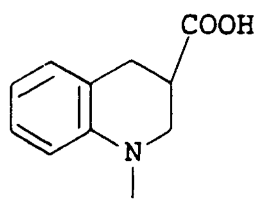

$R^2$: H

$R^3$: 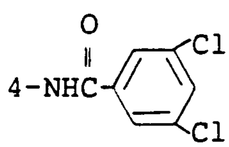

Crystalline form: White powder

Recrystallization solvent: Ethanol

Melting Point: 221 - 223°C

Form: Free

Example 242

Structure

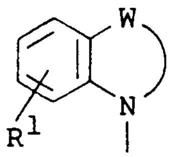
:
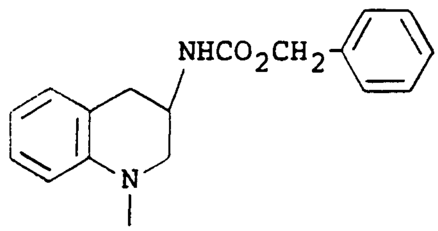
$R^2$: H

$R^3$: 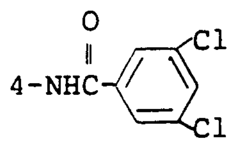

Crystalline form: Colorless amorphous

NMR analysis: 31)

Form: Free

Example 243

Structure

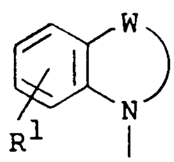
:
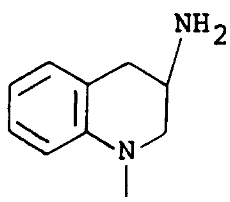
$R^2$: H

$R^3$: 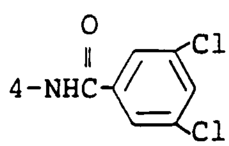

Crystalline form: White powder

Recrystallization solvent: Ethanol

Melting Point: 207 - 210°C

Form: Free

Example 244

Structure

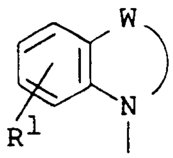 : 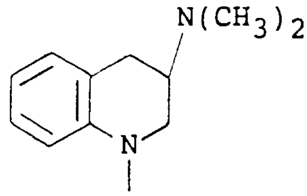

$R^2$: H

$R^3$: 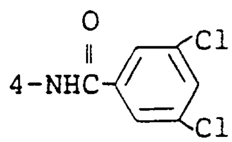

Crystalline form: Colorless amorphous

NMR analysis: 32)

Form: Free

Example 245

Structure

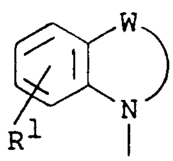 : 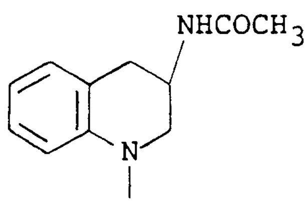

$R^2$: H

$R^3$: 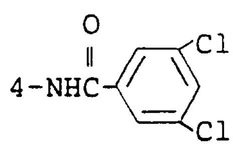

Crystalline form: Colorless amorphous

NMR analysis: 33)

Form: Free

Example 246

Structure

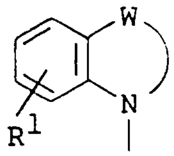 : 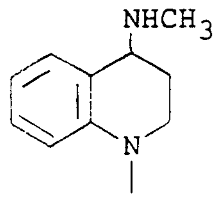

$R^2$: H

$R^3$: 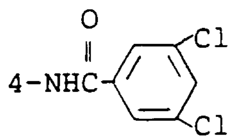

Crystalline form: Colorless amorphous

NMR analysis: 34)

Form: Free

---

Example 247

Structure

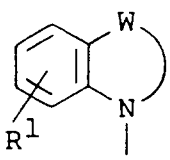 : 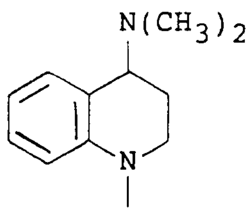

$R^2$: H

$R^3$: 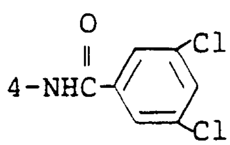

Crystalline form: Colorless amorphous

NMR analysis: 35)

Form: Free

Example 248

Structure

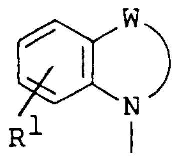 : 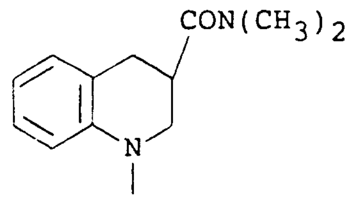

$R^2$: H

$R^3$: 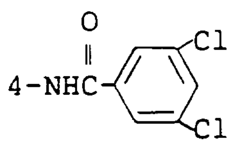

Crystalline form: Light yellow powder

Recrystallization solvent: Ethanol

Melting Point: 186 - 187°C

Form: Free

---

Example 249

Structure

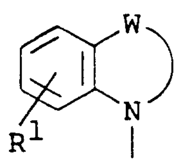 : 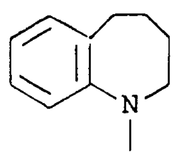

$R^2$: H

$R^3$: 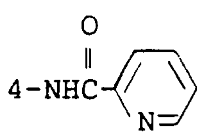

Crystalline form: Colorless needles

Recrystallization solvent: Ethanol

Melting Point: 190 - 191°C

Form: Free

Example 250

Structure

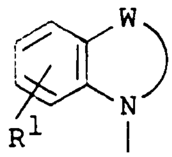 : 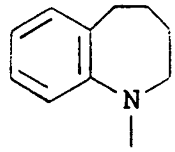

$R^2$: H

$R^3$: 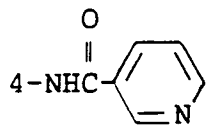

Crystalline form: Light yellow scales

Recrystallization solvent: Ethanol/water

Melting Point: 230 - 231°C

Form: Free

Example 251

Structure

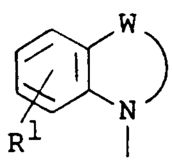 : 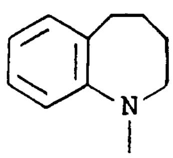

$R^2$: H

$R^3$: 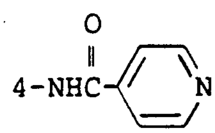

Crystalline form: Light yellow needles

Recrystallization solvent: Ethanol

Melting Point: 227 - 228°C

Form: Free

Example 252

Structure

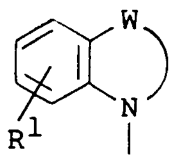 : 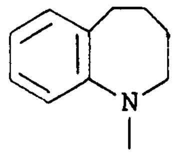

$R^2$: H

$R^3$: $4\text{-}NH\overset{\overset{O}{\|}}{C}CH_2CH_2COOH$

Crystalline form: Colorless needles

Recrystallization solvent: Ethyl acetate

Melting Point: 192°C

Form: Free

Example 253

Structure

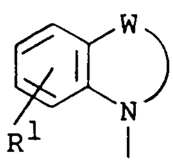 : 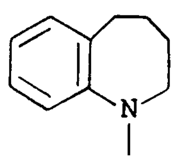

$R^2$: H

$R^3$: $4\text{-}NH\overset{\overset{O}{\|}}{C}CH_2CH_2CH_2COOH$

Crystalline form: White powder

Recrystallization solvent: Ethyl acetate

Melting Point: 186.5 - 189°C

Form: Free

Example 254

Structure

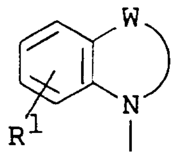 : 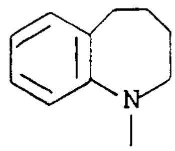 $R^2$: H

$R^3$: 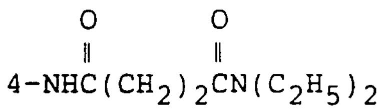

Crystalline form: Light yellow scales

Recrystallization solvent: n-Hexane/ethyl acetate

Melting Point: 165 - 167°C

Form: Free

Example 255

Structure

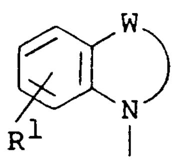 : 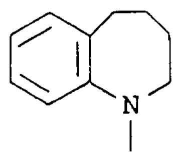 $R^2$: H

$R^3$: 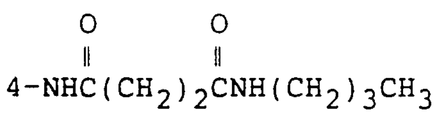

Crystalline form: White powder

Recrystallization solvent: Ethyl acetate

Melting Point: 169 - 170°C

Form: Free

Example 256

Structure

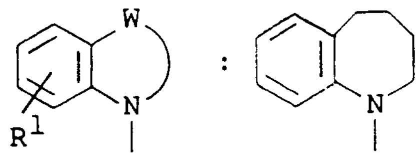

$R^2$: H

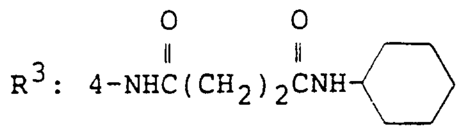

Crystalline form: Colorless scales

Recrystallization solvent: n-Hexane/ethyl acetate

Melting Point: 174 - 177°C

Form: Free

Example 257

Structure

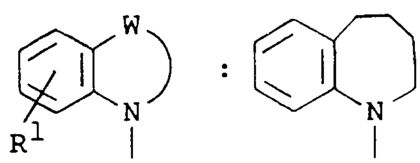

$R^2$: H

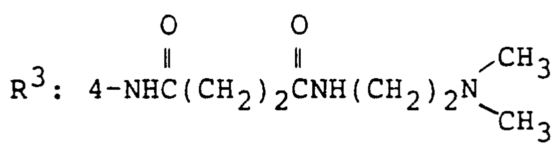

Crystalline form: White powder

Recrystallization solvent: Ethyl acetate

Melting Point: 114 - 118°C

Form: Free

Example 258

Structure

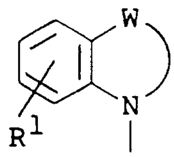 : 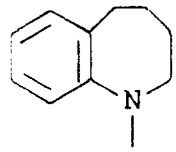

$R^2$: H

$R^3$: 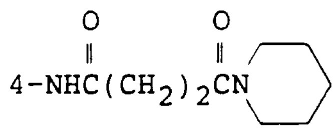

Crystalline form: White powder

Recrystallization solvent: Ethyl acetate

Melting Point: 170 - 172°C

Form: Free

Example 259

Structure

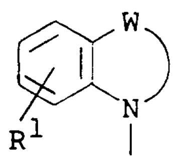 : 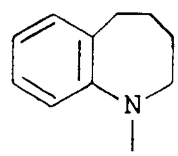

$R^2$: H

$R^3$: 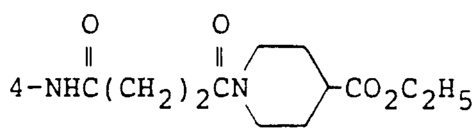

Crystalline form: White powder

Recrystallization solvent: n-Hexane/ethyl acetate

Melting Point: 179 - 181°C

Form: Free

Example 260

Structure

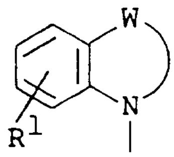 : 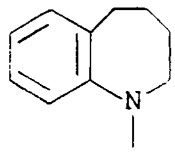

$R^2$: H

$R^3$: 4-$NH\overset{O}{\overset{\|}{C}}(CH_2)_2\overset{O}{\overset{\|}{C}}NH_2$

Crystalline form: White powder

Recrystallization solvent: Ethyl acetate

Melting Point: 118 - 121°C

Form: Free

Example 261

Structure

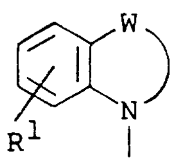 : 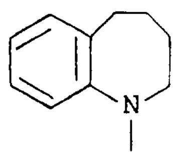

$R^2$: H

$R^3$: 4-$NH\overset{O}{\overset{\|}{C}}(CH_2)_3\overset{O}{\overset{\|}{C}}N(C_2H_5)_2$

Crystalline form: White powder

Recrystallization solvent: Ethyl acetate

Melting Point: 144 - 148°C

Form: Free

Example 262

Structure

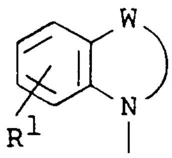 : 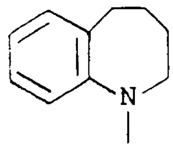

$R^2$: H

$R^3$: $4\text{-}NH\overset{\overset{O}{\|}}{C}(CH_2)_3\overset{\overset{O}{\|}}{C}NH(CH_2)_3CH_3$

Crystalline form: Colorless scales

Recrystallization solvent: Ethyl acetate

Melting Point: 156 - 157°C

Form: Free

Example 263

Structure

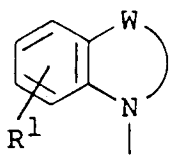 : 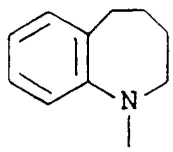

$R^2$: H

$R^3$: $4\text{-}NH\overset{\overset{O}{\|}}{C}(CH_2)_3\overset{\overset{O}{\|}}{C}NH$- 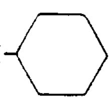

Crystalline form: White powder

Recrystallization solvent: Ethyl acetate

Melting Point: 204 - 206°C

Form: Free

Example 264

Structure

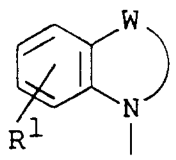 : 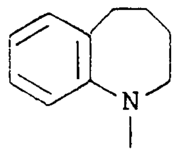

$R^2$: H

$R^3$: 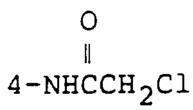

Crystalline form: Light yellow powder

Recrystallization solvent: n-Hexane/ethyl acetate

Melting Point: 165 - 167°C

Form: Free

---

Example 265

Structure

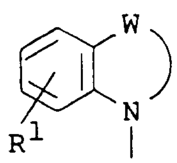 : 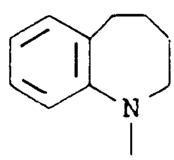

$R^2$: H

$R^3$: 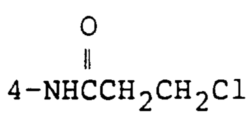

Crystalline form: Light yellow amorphous

NMR analysis: 36)

Form: Free

Example 266

Structure

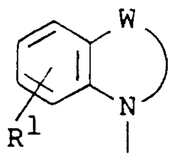 : 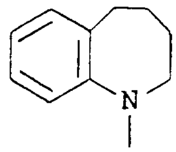

$R^2$: H

$R^3$: $4\text{-}NH\overset{O}{\overset{\|}{C}}(CH_2)_3Cl$

Crystalline form: White powder

Recrystallization solvent: n-Hexane/ethyl acetate

Melting Point: 122 - 124°C

Form: Free

Example 267

Structure

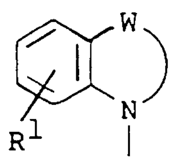 : 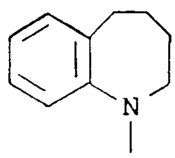

$R^2$: H

$R^3$: $4\text{-}NH\overset{O}{\overset{\|}{C}}CH_2CO_2C_2H_5$

Crystalline form: Light yellow powder

Recrystallization solvent: n-Hexane/ethyl acetate

Melting Point: 116 - 117°C

Form: Free

Example 268

Structure

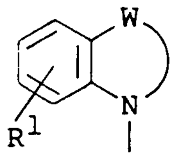 : 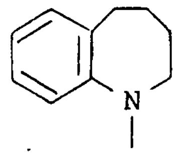

$R^2$: H

O
‖
$R^3$: 4-NHC$(CH_2)_2CO_2C_2H_5$

Crystalline form: White powder

Recrystallization solvent: n-Hexane/ethyl acetate

Melting Point: 121 - 123°C

Form: Free

Example 269

Structure

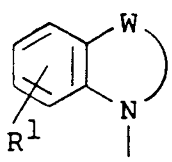 : 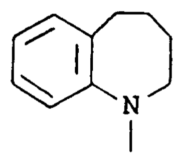

$R^2$: H

O Br
‖ |
$R^3$: 4-NHC-CH-$C_2H_5$

Crystalline form: Colorless needles

Recrystallization solvent: Ethyl acetate

Melting Point: 186 - 187°C

Form: Free

Example 270

Structure

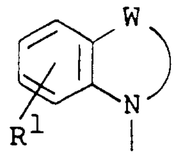

:

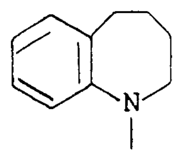

$R^2$: H

$R^3$: 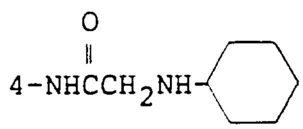

Crystalline form: White powder

Recrystallization solvent: n-Hexane/ethyl acetate

Melting Point: 139 - 142°C

Form: Free

Example 271

Structure

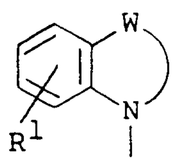

:

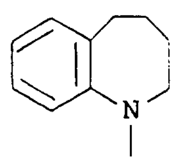

$R^2$: H

$R^3$: 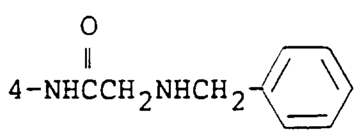

Crystalline form: Light yellow amorphous

NMR analysis: 37)

Form: Free

Example 272

Structure

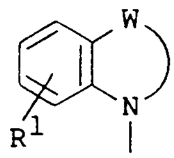 : 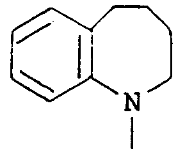

$R^2$: H

O
||
$R^3$: 4-NHCCH$_2$NHCH(CH$_3$)$_2$

Crystalline form: White powder

Recrystallization solvent: Ethyl acetate

Melting Point: 149.5 - 152.5°C

Form: Free

Example 273

Structure

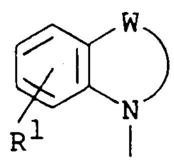 : 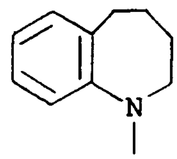

$R^2$: H

O
||
$R^3$: 4-NHCCH$_2$NHC(CH$_3$)$_3$

Crystalline form: Colorless needles

Recrystallization solvent: Ethanol

Melting Point: 150 - 152.5°C

Form: Free

Example 274

Structure

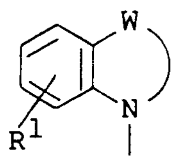 : 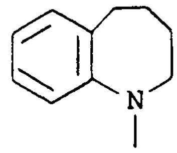

$R^2$: H

$R^3$: $4\text{-}NH\overset{\overset{O}{\|}}{C}CH_2NH(CH_2)_2OH$

Crystalline form: White powder

Recrystallization solvent: Ethyl acetate

Melting Point: 150°C

Form: Free

Example 275

Structure

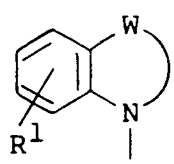 : 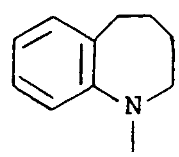

$R^2$: H

$R^3$: $4\text{-}NH\overset{\overset{O}{\|}}{C}CH_2N(C_2H_5)_2$

Crystalline form: Colorless needles

Recrystallization solvent: n-Hexane/ethyl acetate

Melting Point: 101 - 104°C

Form: Free

Example 276

Structure

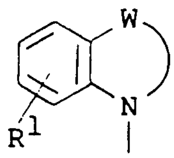 : 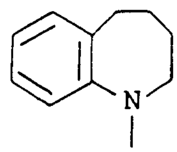

$R^2$: H

$R^3$: 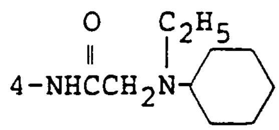

Crystalline form: White powder

Recrystallization solvent: n-Hexane/ethyl acetate

Melting Point: 120 - 122°C

Form: Free

---

Example 277

Structure

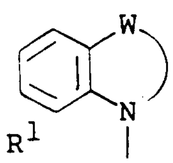 : 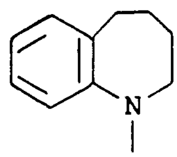

$R^2$: H

$R^3$: 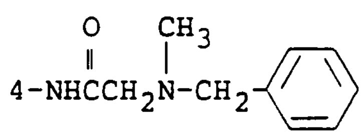

Crystalline form: Light yellow amorphous

NMR analysis: 38)

Form: Free

Example 278

Structure

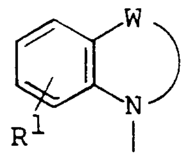 : 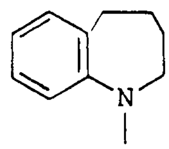 $R^2$: H

$R^3$: 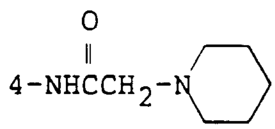

Crystalline form: Colorless needles

Recrystallization solvent: Ethanol

Melting Point: 183 - 186°C

Form: Free

---

Example 279

Structure

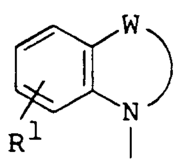 : 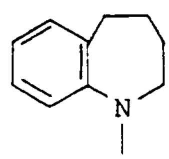 $R^2$: H

$R^3$: 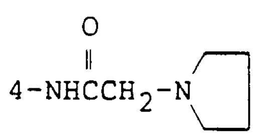

Crystalline form: Light brown powder

Recrystallization solvent: n-Hexane/ethyl acetate

Melting Point: 139 - 142°C

Form: Free

Example 280

Structure

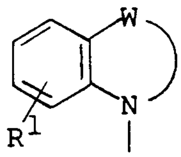 : 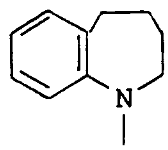 $R^2$: H

$R^3$: 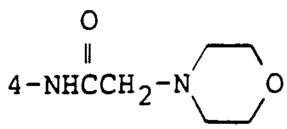

Crystalline form: Light yellow powder

Recrystallization solvent: Ethanol

Melting Point: 162 - 165°C

Form: Free

Example 281

Structure

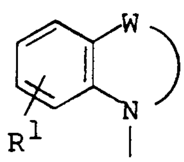 : 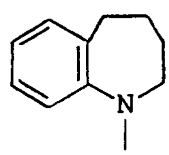 $R^2$: H

$R^3$: 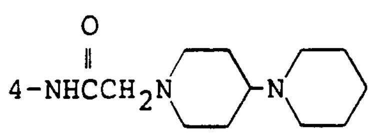

Crystalline form: Light yellow scales

Recrystallization solvent: Ethyl acetate

Melting Point: 224 - 227°C

Form: Free

Example 282

Structure

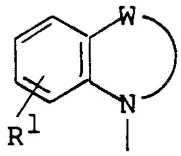 : 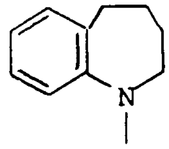

$R^2$: H

$R^3$: 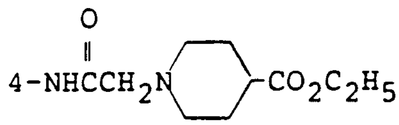

Crystalline form: Light yellow amorphous

NMR analysis: 39)

Form: Free

Example 283

Structure

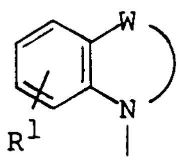 : 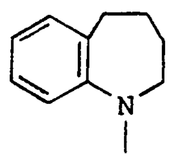

$R^2$: H

$R^3$: 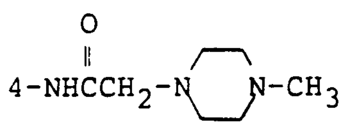

Crystalline form: Light yellow powder

Recrystallization solvent: Ethanol/water

Melting Point: 162 - 164°C

Form: Free

Example 284

Structure

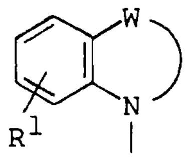 : 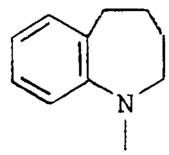 $R^2$: H

O
||
$R^3$: 4-NHCCH$_2$NH$_2$

Crystalline form: Light yellow powder

Recrystallization solvent: Ethanol

Melting Point: 238 - 241°C (decomposed)

Form: Hydrochloride

Example 285

Structure

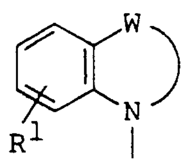 : 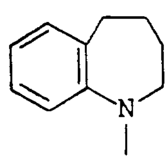 $R^2$: H

O O
|| ||
$R^3$: 4-NHCCH$_2$NHCCH$_3$

Crystalline form: Light yellow amorphous

NMR analysis: 40)

Form: Free

Example 286

Structure

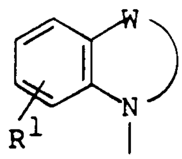 : 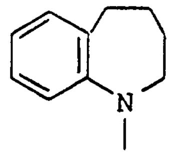

$R^2$: H

$R^3$: 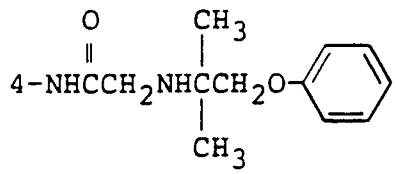

Crystalline form: Colorless amorphous

NMR analysis: 41)

Form: Free

Example 287

Structure

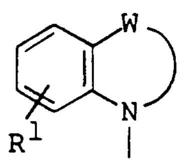 : 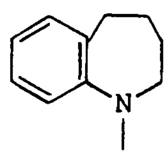

$R^2$: H

$R^3$: 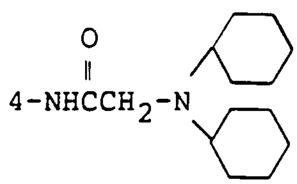

Crystalline form: Colorless needles

Recrystallization solvent: n-Hexane/ethyl acetate

Melting Point: 168 - 169°C

Form: Free

Example 288

Structure

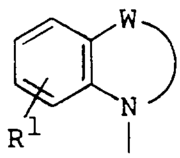 : 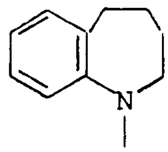

$R^2$: H

$R^3$: 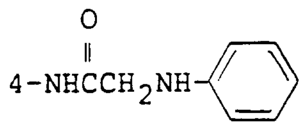

Crystalline form: Light brown powder

Recrystallization solvent: Ethanol

Melting Point: 189 - 191°C

Form: Free

Example 289

Structure

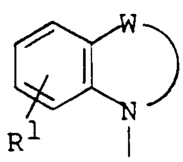 : 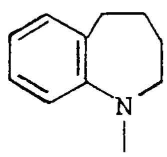

$R^2$: H

$R^3$: 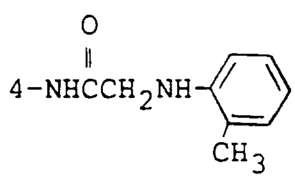

Crystalline form: White powder

Recrystallization solvent: n-Hexane/ethyl acetate

Melting Point: 200 - 202°C

Form: Free

Example 290

Structure

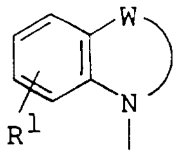 : 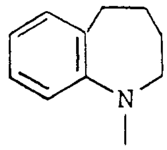

$R^2$: H

$R^3$: 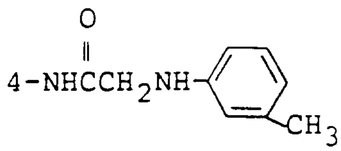

Crystalline form: Colorless scales

Recrystallization solvent: n-Hexane/ethyl acetate

Melting Point: 143 - 146°C

Form: Free

Example 291

Structure

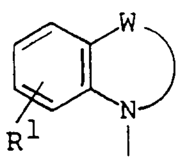 : 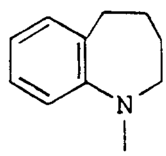

$R^2$: H

$R^3$: 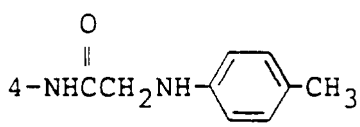

Crystalline form: White powder

Recrystallization solvent: n-Hexane/ethyl acetate

Melting Point: 117 - 117.5°C

Form: Free

Example 292

Structure

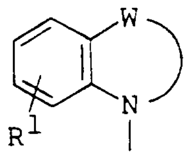 : 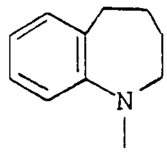

$R^2$: H

$R^3$: 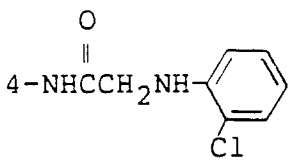

Crystalline form: Light brown powder

Recrystallization solvent: Diethyl ether/ethyl acetate

Melting Point: 225 - 226°C

Form: Free

Example 293

Structure

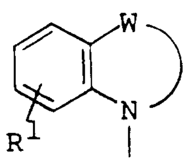 : 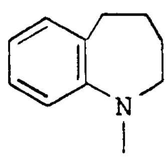

$R^2$: H

$R^3$: 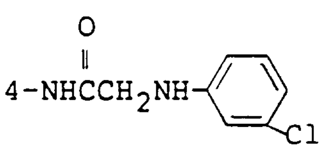

Crystalline form: White powder

Recrystallization solvent: n-Hexane/ethanol

Melting Point: 175 - 176.5°C

Form: Free

Example 294

Structure

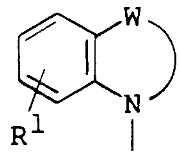 : 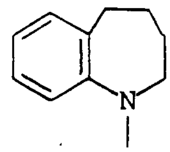

$R^2$: H

$R^3$: 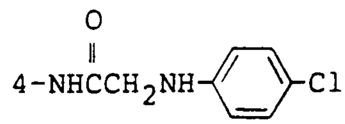

Crystalline form: White powder

Recrystallization solvent: Ethyl acetate

Melting Point: 234 - 236°C

Form: Free

Example 295

Structure

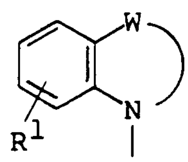 : 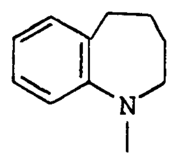

$R^2$: H

$R^3$: 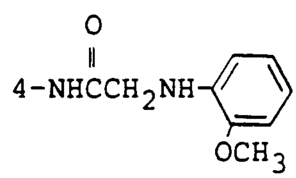

Crystalline form: Colorless scales

Recrystallization solvent: Ethyl acetate

Melting Point: 172 - 174°C

Form: Free

Example 296

Structure

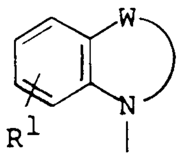

:

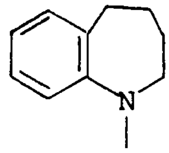

$R^2$: H

$R^3$: 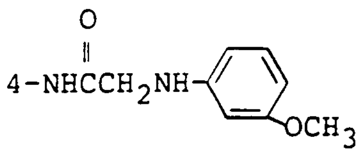

Crystalline form: White powder

Recrystallization solvent: Ethyl acetate

Melting Point: 154 - 155°C

Form: Free

Example 297

Structure

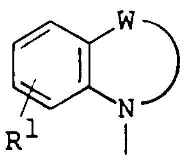

:

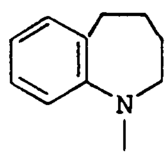

$R^2$: H

$R^3$: 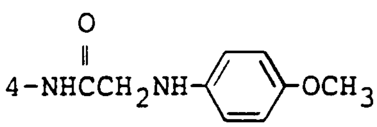

Crystalline form: Light yellow needles

Recrystallization solvent: n-Hexane/ethyl acetate

Melting Point: 181.5 - 183.5°C

Form: Free

Example 298

Structure

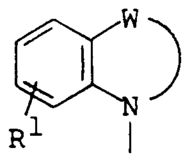 : 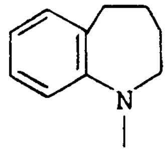

$R^2$: H

$R^3$: 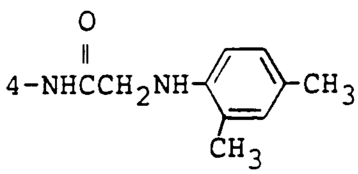

Crystalline form: White powder

Recrystallization solvent: n-Hexane/ethyl acetate

Melting Point: 173 - 175°C

Form: Free

Example 299

Structure

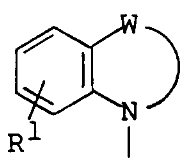 : 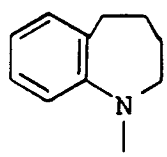

$R^2$: H

$R^3$: 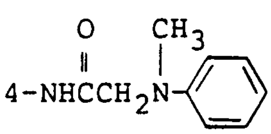

Crystalline form: White powder

Recrystallization solvent: n-Hexane/ethyl acetate

Melting Point: 137 - 138°C

Form: Free

Example 300

Structure

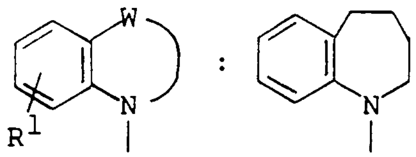

$R^2$: H

$R^3$: 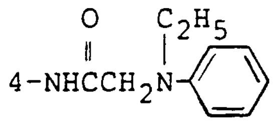

Crystalline form: Light yellow amorphous

NMR analysis: 42)

Form: Free

Example 301

Structure

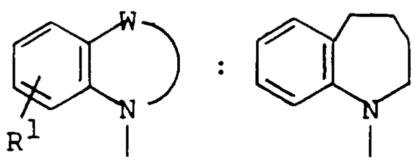

$R^2$: H

$R^3$: 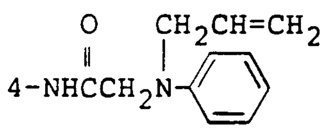

Crystalline form: Colorless needles

Recrystallization solvent: Diethyl ether/ethyl acetate

Melting Point: 129 - 130°C

Form: Free

Example 302

Structure

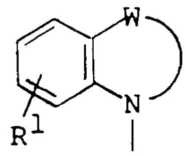 : 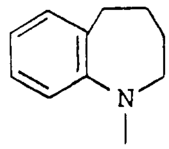

$R^2$: H

$R^3$: 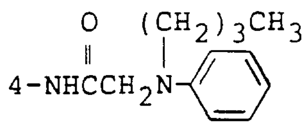

Crystalline form: Colorless needles

Recrystallization solvent: n-Hexane/ethyl acetate

Melting Point: 181 - 183°C

Form: Free

Example 303

Structure

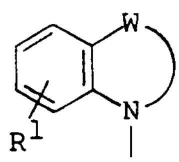 : 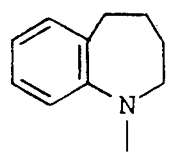

$R^2$: H

$R^3$: 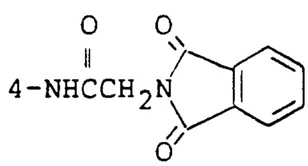

Crystalline form: White powder

Recrystallization solvent: Ethyl acetate

Melting Point: 248 - 249°C

Form: Free

Example 304

Structure

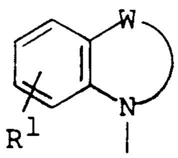 : 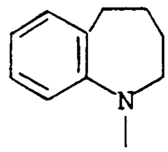

$R^2$: H

$R^3$: 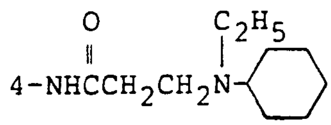

Crystalline form: Light yellow amorphous

NMR analysis: 43)

Form: Free

Example 305

Structure

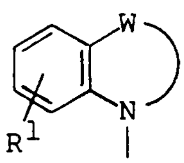 : 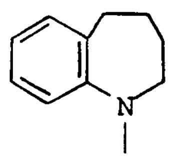

$R^2$: H

$R^3$: 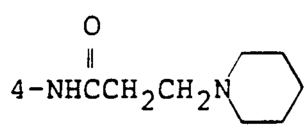

Crystalline form: Light yellow needles

Recrystallization solvent: Ethanol

Melting Point: 94 - 96°C

Form: Free

Example 306

Structure

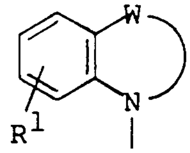 : 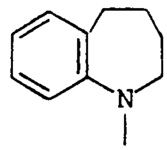

$R^2$: H

$R^3$: 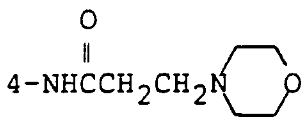

Crystalline form: Light brown powder

Recrystallization solvent: Ethyl acetate

Melting Point: 159 - 161°C

Form: Free

---

Example 307

Structure

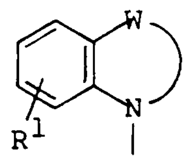 : 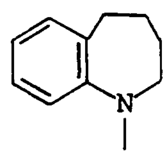

$R^2$: H

$R^3$: 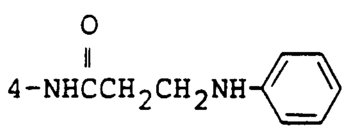

Crystalline form: White powder

Recrystallization solvent: Ethyl acetate

Melting Point: 180 - 183°C

Form: Free

Example 308

Structure

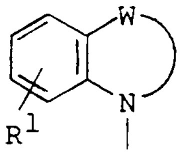 : 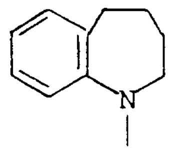

$R^2$: H

$R^3$: 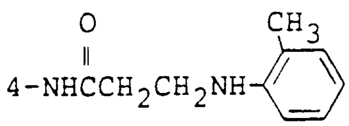

Crystalline form: Light brown powder

Recrystallization solvent: Ethanol

Melting Point: 177 - 180°C

Form: Free

Example 309

Structure

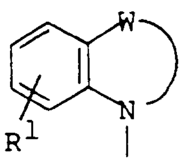 : 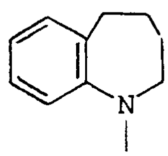

$R^2$: H

$R^3$: 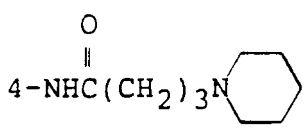

Crystalline form: White powder

Recrystallization solvent: Ethyl acetate

Melting Point: 91 - 93°C

Form: Free

Example 310

Structure

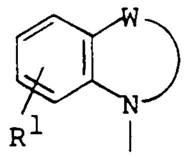 : 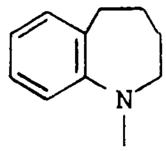

$R^2$: H

$R^3$: 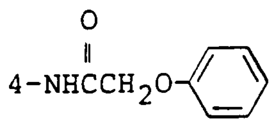

Crystalline form: Light brown scales

Recrystallization solvent: Ethanol

Melting Point: 155 - 156.5°C

Form: Free

Example 311

Structure

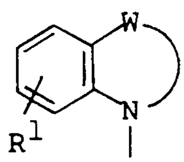 : 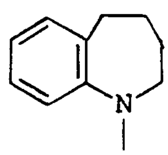

$R^2$: H

$R^3$: 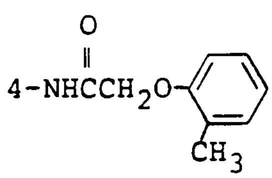

Crystalline form: Colorless scales

Recrystallization solvent: Ethyl acetate

Melting Point: 172.5 - 175°C

Form: Free

Example 312

Structure

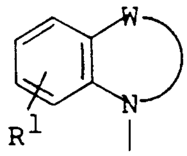 : 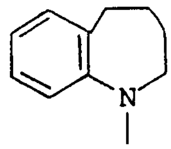

$R^2$: H

$R^3$: 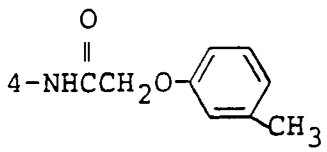

Crystalline form: White powder

Recrystallization solvent: Ethanol

Melting Point: 148 - 150.5°C

Form: Free

Example 313

Structure

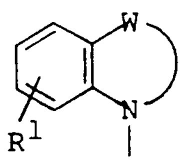 : 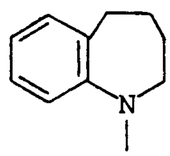

$R^2$: H

$R^3$: 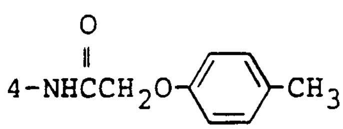

Crystalline form: White powder

Recrystallization solvent: Ethyl acetate

Melting Point: 172 - 173°C

Form: Free

Example 314

Structure

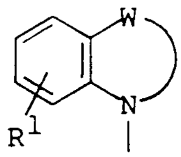 : 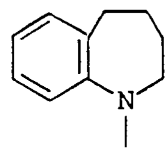  $R^2$: H

$R^3$: 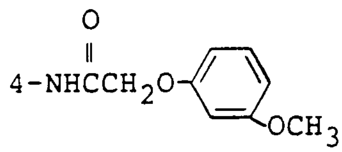

Crystalline form: Colorless scales

Recrystallization solvent: n-Hexane/ethyl acetate

Melting Point: 133 - 135°C

Form: Free

Example 315

Structure

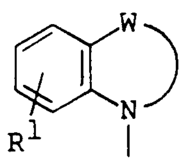 : 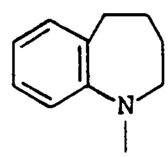  $R^2$: H

$R^3$: 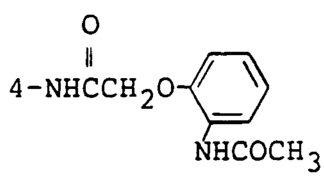

Crystalline form: White powder

Recrystallization solvent: Ethyl acetate

Melting Point: 217 - 219°C

Form: Free

Example 316

Structure

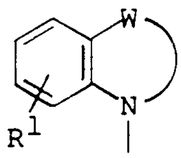 : 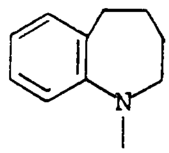

$R^2$: H

$R^3$: 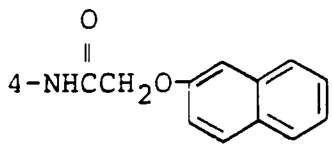

Crystalline form: Colorless needles

Recrystallization solvent: Ethyl acetate

Melting Point: 226 - 227.5°C

Form: Free

Example 317

Structure

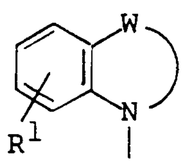 : 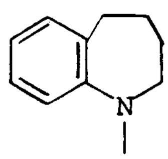

$R^2$: H

$R^3$: 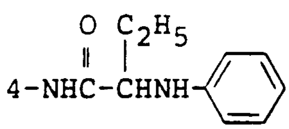

Crystalline form: Colorless amorphous

NMR analysis: 44)

Form: Free

Example 318

Structure

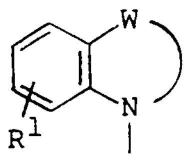 : 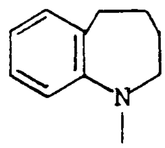

$R^2$: H

$R^3$: 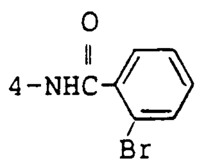

Crystalline form: White powder

Recrystallization solvent: Dichloromethane

Melting Point: 234 - 235°C

Form: Free

Example 319

Structure

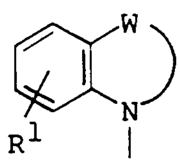 : 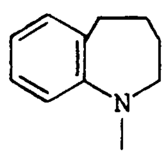

$R^2$: H

$R^3$: 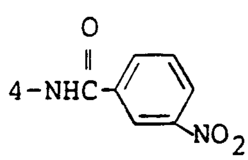

Crystalline form: Colorless prisms

Recrystallization solvent: Methanol

Melting Point: 218 - 218.5°C

Form: Free

Example 320

Structure

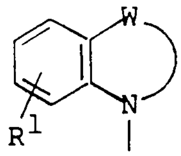 : 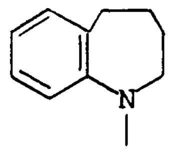

$R^2$: H

$R^3$: 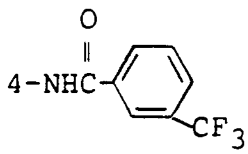

Crystalline form: Colorless prisms

Recrystallization solvent: Ethanol

Melting Point: 202.5 - 206°C

Form: Free

Example 321

Structure

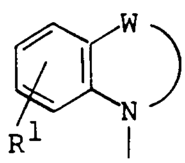 : 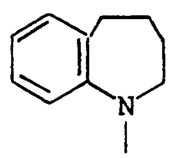

$R^2$: H

$R^3$: 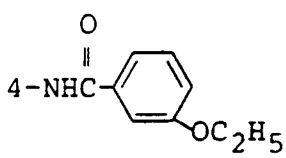

Crystalline form: White powder

Recrystallization solvent: Ethanol

Melting Point: 174 - 176°C

Form: Free

Example 322

Structure

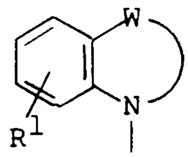 : 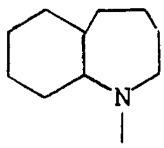

$R^2$: H

$R^3$: 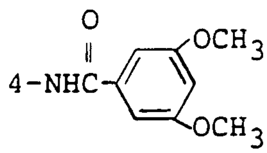

Crystalline form: Colorless prisms

Recrystallization solvent: Ethanol

Melting Point: 216 - 218°C

Form: Free

Example 323

Structure

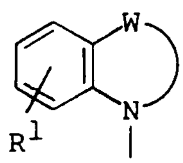 : 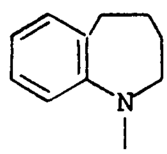

$R^2$: H

$R^3$: 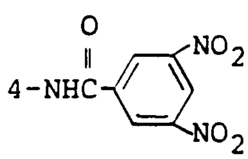

Crystalline form: White powder

Melting Point: >300°C

NMR analysis: 45)

Form: Free

Example 324

Structure

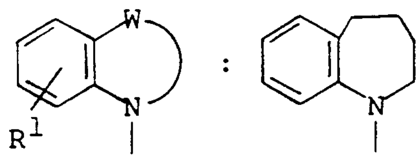

$R^2$: H

$R^3$: 4-NHC 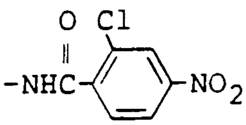

Crystalline form: Colorless prisms

Recrystallization solvent: Ethanol

Melting Point: 250.5 - 251°C

Form: Free

---

Example 325

Structure

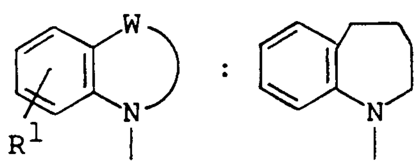

$R^2$: H

$R^3$: 4-NHC 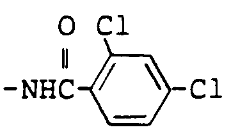

Crystalline form: Colorless prisms

Recrystallization solvent: Ethanol

Melting Point: 223 - 225°C

Form: Free

Example 326

Structure

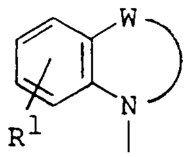 : 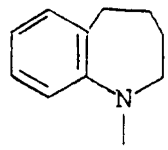 $R^2$: H

$R^3$: 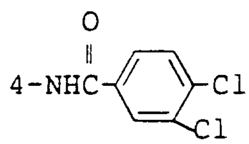

Crystalline form: Colorless prismsr

Recrystallization solvent: Methanol

Melting Point: 213 - 214°C

Form: Free

Example 327

Structure

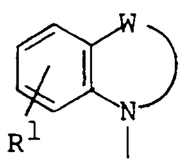 : 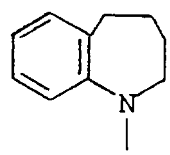 $R^2$: H

$R^3$: 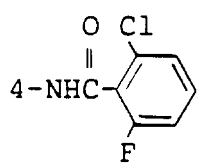

Crystalline form: Colorless prisms

Recrystallization solvent: Ethanol

Melting Point: 246 - 247°C

Form: Free

Example 328

Structure

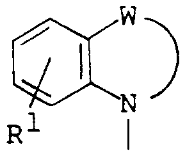
:
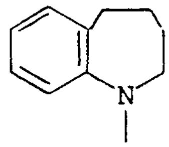

$R^2$: H

$R^3$:
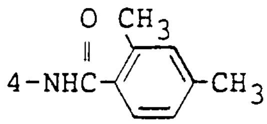

Crystalline form: Colorless prisms

Recrystallization solvent: Methanol

Melting Point: 248 - 251°C

Form: Free

Example 329

Structure

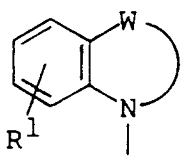
:
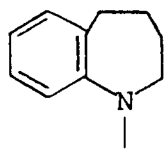

$R^2$: H

$R^3$:
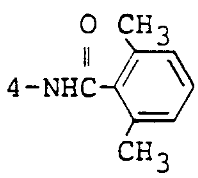

Crystalline form: Colorless prisms

Recrystallization solvent: Ethanol

Melting Point: 268.5 - 270.5°C

Form: Free

Example 330

Structure

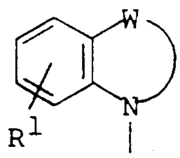 : 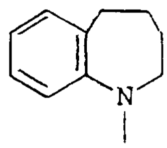

$R^2$: H

$R^3$: 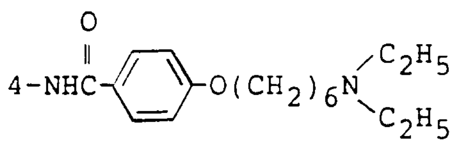

Crystalline form: White powder

Recrystallization solvent: Methanol/diethyl ether

Melting Point: 174 - 176°C

Form: Hydrochloride

Example 331

Structure

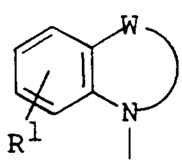 : 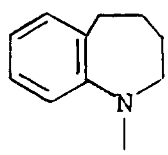

$R^2$: H

$R^3$: 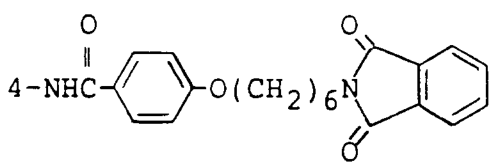

Crystalline form: White powder

Recrystallization solvent: Ethanol

Melting Point: 130 - 134°C

Form: Free

Example 332

Structure

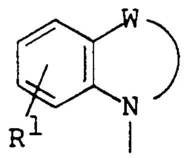 : 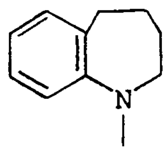

$R^2$: H

$R^3$: 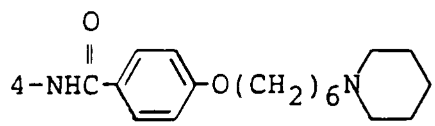

Crystalline form: White powder

Recrystallization solvent: Methanol/diethyl ether

Melting Point: 214 - 217°C

Form: Hydrochloride

Example 333

Structure

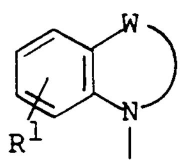 : 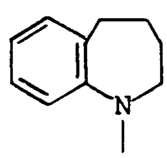

$R^2$: H

$R^3$: 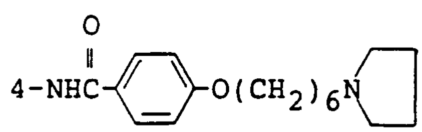

Crystalline form: White powder

Recrystallization solvent: Methanol/diethyl ether

Melting Point: 218 - 220°C

Form: Hydrochloride

Example 334

Structure

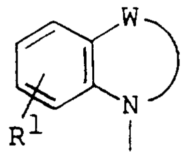 : 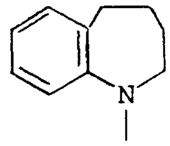

$R^2$: H

$R^3$: 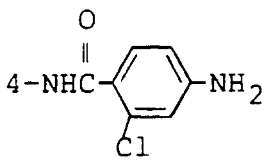

Crystalline form: White powder

Recrystallization solvent: Ethyl acetate

Melting Point: 222 - 225°C

Form: Free

Example 335

Structure

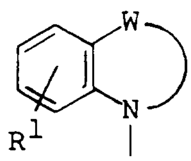 : 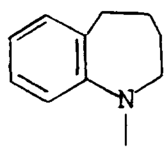

$R^2$: H

$R^3$: 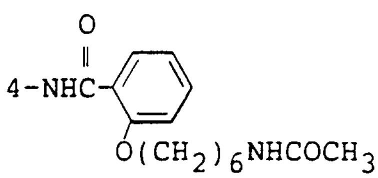

Crystalline form: Colorless needles

Recrystallization solvent: Methanol/diethyl ether

Melting Point: 171 - 172°C

Form: Free

Example 336

Structure

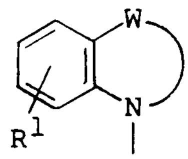 : 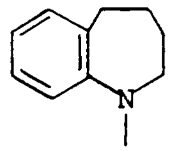 $R^2$: H

$R^3$: 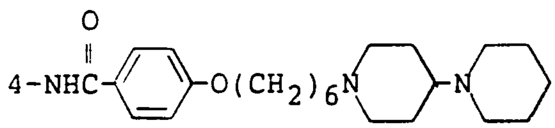

Crystalline form: White powder

Recrystallization solvent: Methanol/diethyl ether

Melting Point: 235.5 - 236°C

Form: Dihydrochloride

Example 337

Structure

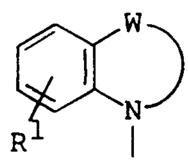 : 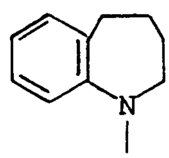 $R^2$: H

$R^3$: 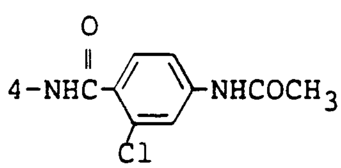

Crystalline form: White powder

Recrystallization solvent: Methanol/diethyl ether

Melting Point: 241 - 243°C

Form: Free

Example 338

Structure

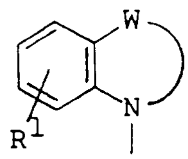 : 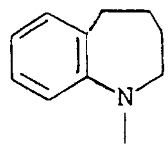 $R^2$: H

$R^3$: 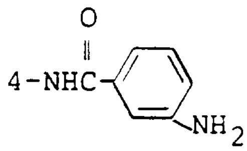

Crystalline form: White powder

Recrystallization solvent: Methanol/diethyl ether

Melting Point: 187 - 191°C

Form: Free

Example 339

Structure

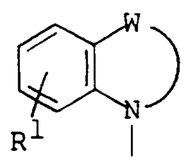 : 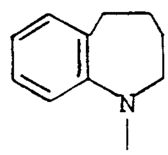 $R^2$: H

$R^3$: 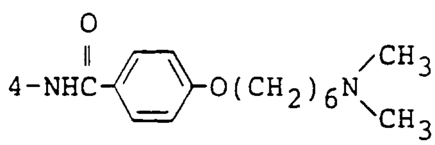

Crystalline form: White powder

Recrystallization solvent: Methanol/diethyl ether

Melting Point: 240 - 244°C

Form: Hydrochloride

Example 340

Structure

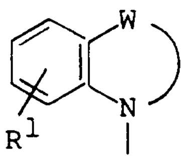 : 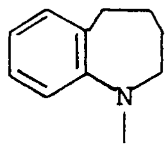

$R^2$: H

$R^3$: 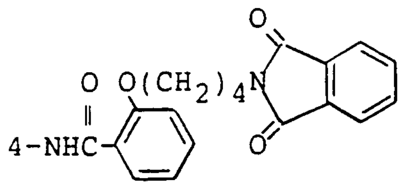

Crystalline form: Colorless prisms

Recrystallization solvent: Methanol/diethyl ether

Melting Point: 181 - 182°C

Form: Free

---

Example 341

Structure

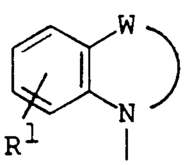 : 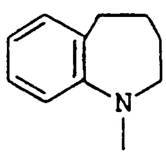

$R^2$: H

$R^3$: 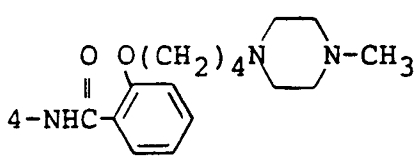

Crystalline form: Colorless prisms

Recrystallization solvent: Methanol/diethyl ether

Melting Point: 188 - 190°C

Form: Dihydrochloride

Example 342

Structure

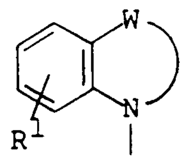 : 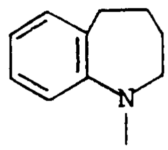

$R^2$: H

$R^3$: 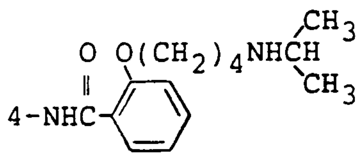

Crystalline form: White powder

Recrystallization solvent: Isopropyl alcohol

Melting Point: 218 - 218.5°C

Form: Hydrochloride

Example 343

Structure

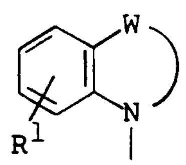 : 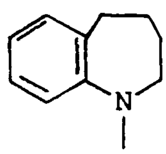

$R^2$: H

$R^3$: 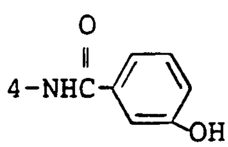

Crystalline form: White powder

Recrystallization solvent: Methanol/diethyl ether

Melting Point: 243 - 245.5°C

Form: Free

Example 344

Structure

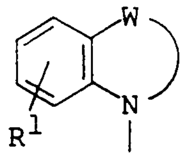 : 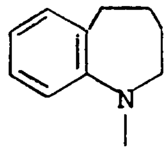 $R^2$: H

$R^3$: 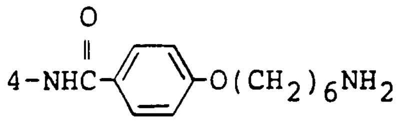

Crystalline form: White powder

Recrystallization solvent: Methanol/diethyl ether

Melting Point: 130 - 133°C

Form: Free

Example 345

Structure

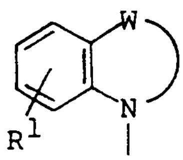 : 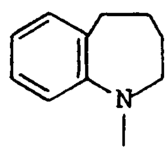 $R^2$: H

$R^3$: 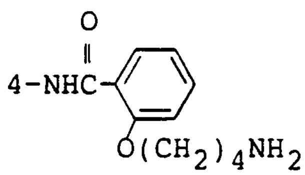

Crystalline form: White powder

Recrystallization solvent: Methanol/diethyl ether

Melting Point: 155 - 158°C

Form: Free

Example 346

Structure

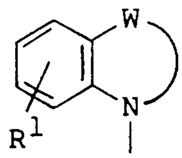
:
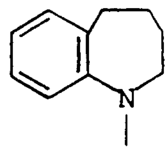

$R^2$: H

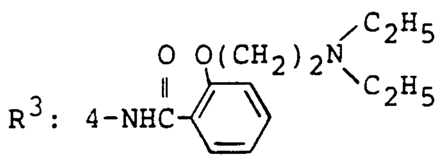

Crystalline form: White powder

Recrystallization solvent: Methanol/diethyl ether

Melting Point: 208 - 210°C

Form: Hydrochloride

Example 347

Structure

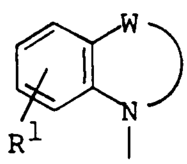
:
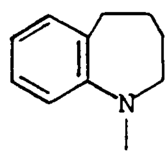

$R^2$: H

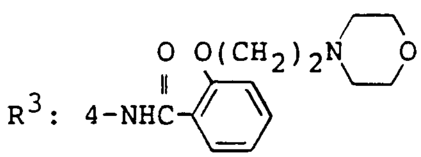

Crystalline form: Colorless prisms

Recrystallization solvent: Methanol/diethyl ether

Melting Point: 154 - 155°C

Form: Hydrochloride

Example 348

Structure

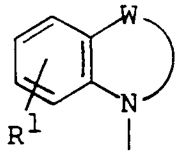 : 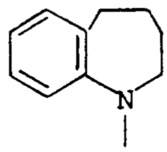 $R^2$: H

$R^3$: 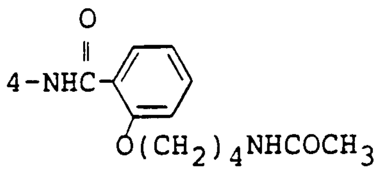

Crystalline form: White powder

Recrystallization solvent: Methanol/diethyl ether

Melting Point: 142 - 143°C

Form: Free

Example 349

Structure

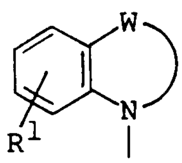 : 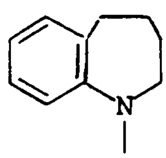 $R^2$: H

$R^3$: 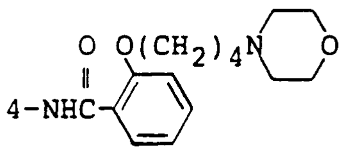

Crystalline form: White powder

Recrystallization solvent: Methanol/diethyl ether

Melting Point: 120 - 125°C

Form: Hydrochloride

Example 350

Structure

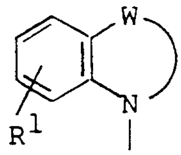 : 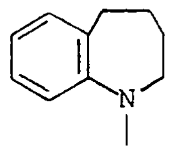

$R^2$: H

$R^3$: 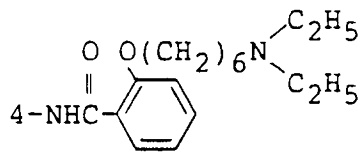

Crystalline form: White powder

Recrystallization solvent: Methanol/diethyl ether

Melting Point: 91 - 95°C

Form: Hydrochloride

Example 351

Structure

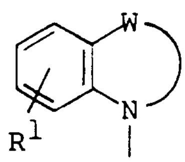 : 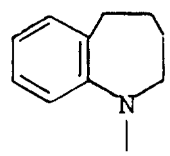

$R^2$: H

$R^3$: 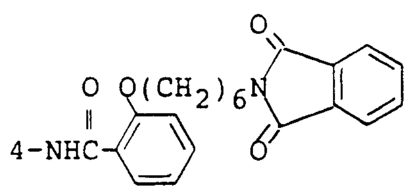

Crystalline form: White powder

Recrystallization solvent: Methanol/diethyl ether

Melting Point: 145 - 146.5°C

Form: Free

Example 352

Structure

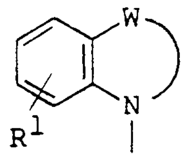 : 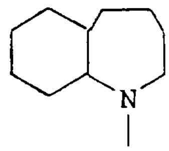 $R^2$: H

$R^3$: 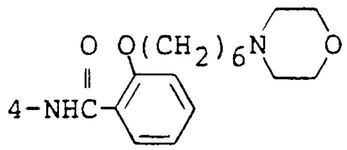

Crystalline form: White powder

Recrystallization solvent: Methanol/diethyl ether

Melting Point: 105 - 105.5°C

Form: Free

Example 353

Structure

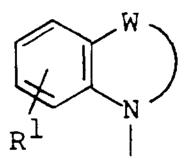 : 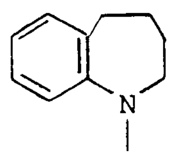 $R^2$: H

$R^3$: 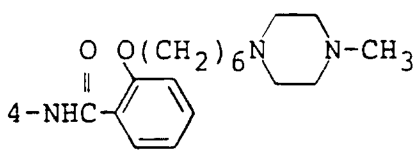

Crystalline form: White powder

Recrystallization solvent: Methanol/diethyl ether

Melting Point: 151 - 155°C

Form: Dihydrochloride

Example 354

Structure

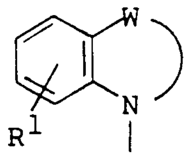 : 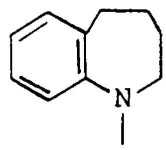

$R^2$: H

$R^3$: 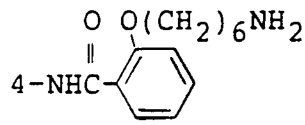

Crystalline form: White powder

Recrystallization solvent: Methanol/diethyl ether

Melting Point: 135.5 - 137.5°C

Form: Free

Example 355

Structure

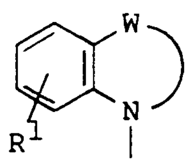 : 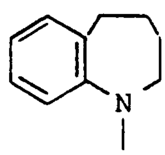

$R^2$: H

$R^3$: 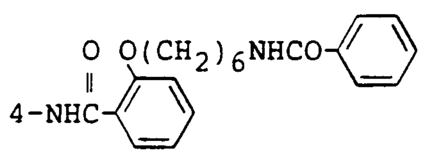

Crystalline form: White powder

Recrystallization solvent: Ethanol

Melting Point: 178 - 178.5°C

Form: Free

Example 356

Structure

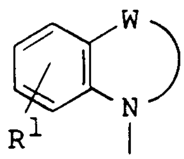 : 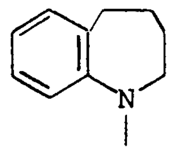

$R^2$: H

$R^3$: 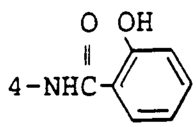

Crystalline form: White powder

Recrystallization solvent: Dichloromethane

Melting Point: 266.5 - 268°C

Form: Free

Example 357

Structure

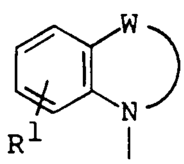 : 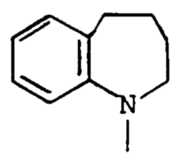

$R^2$: H

$R^3$: 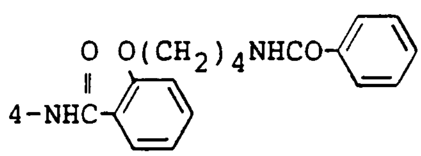

Crystalline form: White powder

Recrystallization solvent: Methanol/diethyl ether

Melting Point: 123 - 124°C

Form: Free

Example 358

Structure

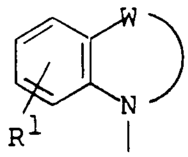 : 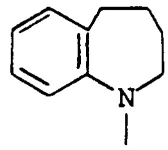 $R^2$: H

$R^3$: 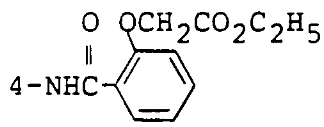

Crystalline form: White powder

Recrystallization solvent: Ethyl acetate

Melting Point: 212 - 213.5°C

Form: Free

Example 359

Structure

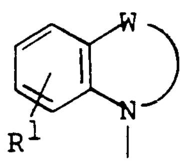 : 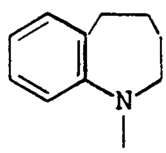 $R^2$: H

$R^3$: 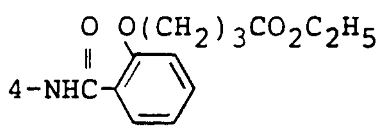

Crystalline form: Colorless scales

Recrystallization solvent: Ethyl acetate

Melting Point: 160.5 - 162°C

Form: Free

Example 360

Structure

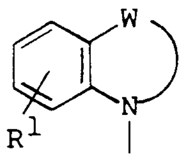 : 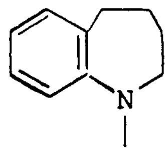

$R^2$: H

$R^3$: 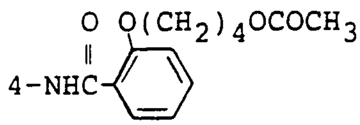

Crystalline form: Colorless needles

Recrystallization solvent: Ethanol

Melting Point: 103 - 105°C

Form: Free

Example 361

Structure

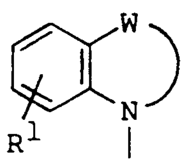 : 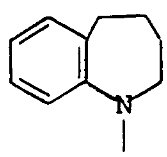

$R^2$: H

$R^3$: 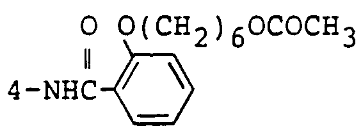

Crystalline form: White powder

Recrystallization solvent: Ethanol

Melting Point: 145 - 146°C

Form: Free

Example 362

Structure

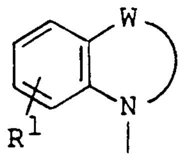 : 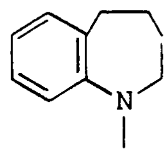

$R^2$: H

$R^3$: 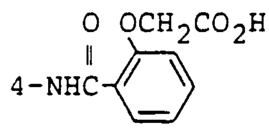

Crystalline form: White powder

Recrystallization solvent: Methanol/diethyl ether

Melting Point: 247 - 250°C

Form: Free

Example 363

Structure

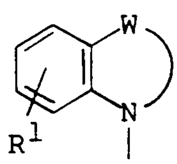 : 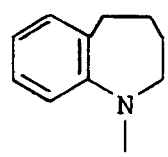

$R^2$: H

$R^3$: 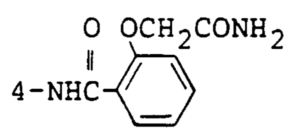

Crystalline form: White powder

Recrystallization solvent: Methanol/diethyl ether

Melting Point: 198 - 199°C

Form: Free

Example 364

Structure

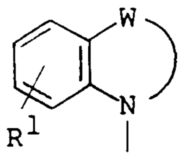 : 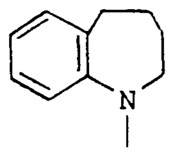

$R^2$: H

$R^3$: 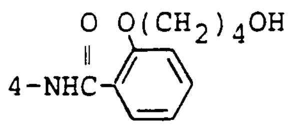

Crystalline form: White powder

Recrystallization solvent: Methanol/diethyl ether

Melting Point: 181.5 - 182.5°C

Form: Free

Example 365

Structure

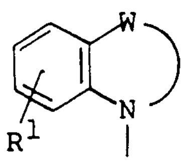 : 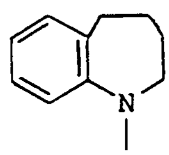

$R^2$: H

$R^3$: 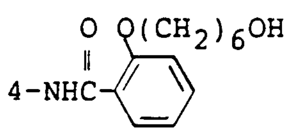

Crystalline form: White powder

Recrystallization solvent: Methanol/diethyl ether

Melting Point: 170 - 170.5°C

Form: Free

Example 366

Structure

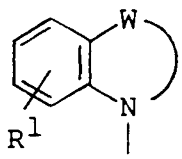 : 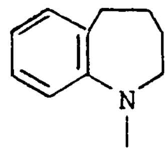

$R^2$: H

$R^3$: 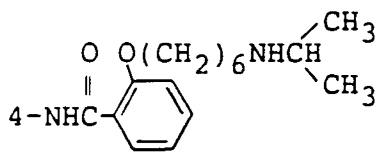

Crystalline form: White powder

Recrystallization solvent: Methanol/diethyl ether

Melting Point: 156 - 158°C

Form: Hydrochloride

Example 367

Structure

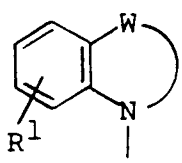 : 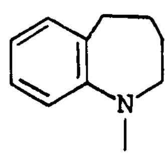

$R^2$: H

$R^3$: 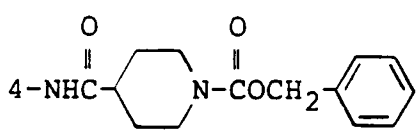

Crystalline form: White powder

Recrystallization solvent: Diethyl ether

Melting Point: 168.5 - 170.5°C

Form: Free

Example 368

Structure

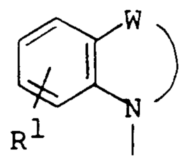

:

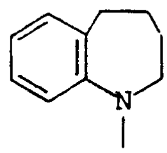

$R^2$: H

$R^3$: 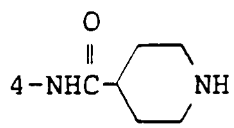

Crystalline form: White powder

Recrystallization solvent: Methanol/diethyl ether

Melting Point: 177 - 181.5°C

Form: Hydrochloride

Example 369

Structure

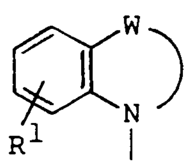

:

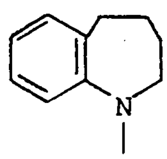

$R^2$: H

$R^3$: 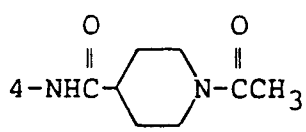

Crystalline form: White powder

Recrystallization solvent: Methanol/diethyl ether

Melting Point: 211 - 213°C

Form: Free

Example 370

Structure

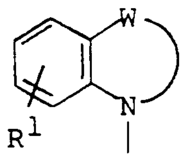 : 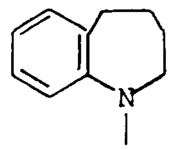

$R^2$: H

$R^3$: 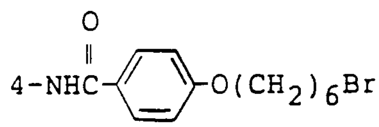

Crystalline form: White powder

NMR analysis: 46)

Form: Free

Example 371

Structure

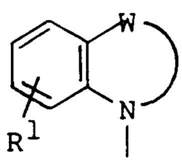 : 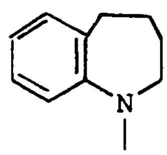

$R^2$: H

$R^3$: 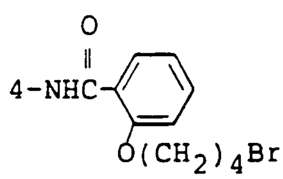

Crystalline form: White powder

Recrystallization solvent: Methanol/ethyl acetate

Melting Point: 166 - 167°C

Form: Free

Example 372

Structure

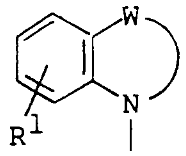 : 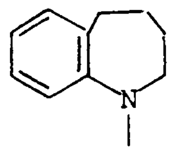

$R^2$: H

$R^3$: 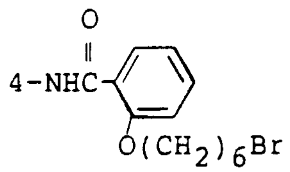

Crystalline form: White powder

Recrystallization solvent: Methanol/diethyl ether

Melting Point: 127 - 131°C

Form: Free

Example 373

Structure

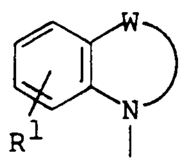 : 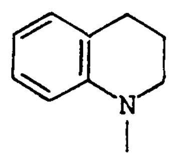

$R^2$: H

$R^3$: 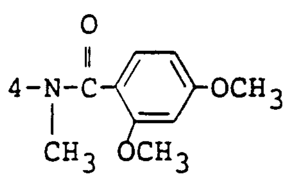

Crystalline form: White powder

Recrystallization solvent: Methanol

Melting Point: 170 - 171°C

Form: Free

Example 374

Structure

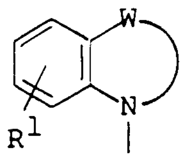 : 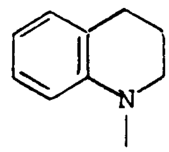 $R^2$: H

$R^3$: 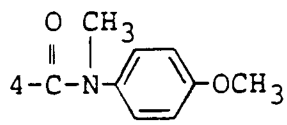

Crystalline form: White powder

Recrystallization solvent: Methanol

Melting Point: 125 - 126°C

Form: Free

Example 375

Structure

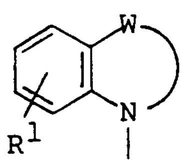 : 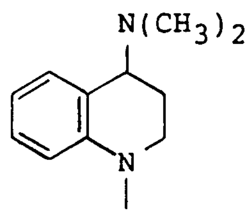 $R^2$: H

$R^3$: 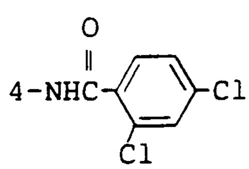

Crystalline form: Light yellow amorphous

NMR analysis: 47)

Form: Hydrochloride

Example 376

Structure

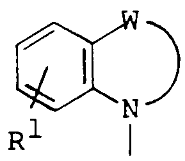 : 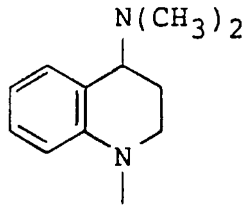

$R^2$: H

$R^3$: 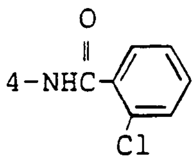

Crystalline form: Colorless amorphous

NMR analysis: 48)

Form: Hydrochloride

1) $^1$H-NMR ($CDCl_3$) δ : 1.92 (1H, t, J=6.2 Hz), 1.98 (1H, t, J=6.4 Hz), 2.8 (2H, t, J=6.4 Hz), 3.76 (2H, t, J=6.2 Hz), 6.75 (1H, d, J=7.6 Hz), 6.86 (2H, d, J=8.6 Hz), 6.8-7.1 (2H, m), 7.20 (1H, d, J=7 Hz), 7.30 (2H, d, J=8.6 Hz), 7.72 (2H, d, J=8.6 Hz), 7.84 (2H, d, J=8.6 Hz), 10.13 (1H, s)

2) $^1$H-NMR (DMSO-$d_6$) δ : 2.05 (2H, quint, J=6.4 Hz), 2.91 (2H, t, J=6.4 Hz), 3.86 (2H, t, J=6.4 Hz), 6.85 (1H, d, J=7.6 Hz), 6.9-7.2 (2H, m), 7.30 (1H, d, J=7.2 Hz), 7.44 (2H, d, J=8.5 Hz), 7.85 (2H, d, J=8.5 Hz), 8.1-8.2 (4H, m), 10.65 (1H, s), 13.2-13.4 (1H, br)

3) $^1$H-NMR ($CDCl_3$) δ : 1.9-2.1 (2H, m), 2.84 (2H, t, J=6.5 Hz), 3.82 (6H, s), 3.90 (2H, t, J=6.6 Hz), 6.5-7.2 (7H, m), 7.35 (2H, d, J=8.7 Hz), 7.55 (2H, d, J=8.7 Hz), 8.05 (1H, s)

4) $^1$H-NMR ($CDCl_3$) δ : 1.9-2.1 (2H, m), 2.37 (6H, s), 2.84 (2H, t, J=6.6 Hz), 3.90 (2H, t, J=6.6 Hz), 6.71 (1H, d, J=7.9 Hz), 6.8-7.2 (4H, m), 7.35 (2H, d, J=8.6 Hz), 7.44 (2H, s), 7.56 (2H, d, J=8.6 Hz), 8.00 (1H, s)

5) $^1$H-NMR ($CDCl_3$) δ : 1.9-2.2 (2H, m), 2.12 (3H, s), 2.84 (2H, t, J=6.6 Hz), 3.89 (2H, t, J=6.5 Hz), 6.71 (1H, d, J=7.8 Hz), 6.87 (1H, t, J=7 Hz), 6.99 (1H, t, J=7.3 Hz), 7.15 (1H, d, J=6.5 Hz), 7.28 (2H, d, J=8.6 Hz), 7.41 (2H, d, J=8.6 Hz), 8.03

(1H, s)

6) $^1$H-NMR ($CDCl_3$) δ : 0.8-1.3 (6H, m), 1.6-2.3 (9H, m), 2.83 (2H, t, J=6.6 Hz), 3.89 (2H, t, J=6.5 Hz), 6.72 (1H, d, J=7.9 Hz), 6.8-7.1 (2H, m), 7.15 (1H, d, J=7.4 Hz), 7.28 (2H, d, J=8.3 Hz), 7.44 (2H, d, J=8.4 Hz), 7.9-8.1 (1H, m)

7) $^1$H-NMR ($CDCl_3$) δ : 2.02 (2H, quint, J=6.5 Hz), 2.81 (2H, t, J=6.6 Hz), 3.69 (2H, s), 3.87 (2H, t, J=6.6 Hz), 6.66 (1H, d, J=8.2 Hz), 6.8-7.0 (2H, m), 7.13 (1H, d, J=7.3 Hz), 7.2-7.4 (9H, m), 7.59 (1H, s)

8) $^1$H-NMR ($CDCl_3$) δ : 1.7-2.1 (17H, m), 2.83 (2H, t, J=6.7 Hz), 3.90 (2H, t, J=6.6 Hz), 6.68 (1H, d, J=8.1 Hz), 6.8-7.1 (2H, m), 7.14 (1H, d, J=7 Hz), 7.32 (2H, d, J=8.7 Hz), 7.39 (1H, s), 7.46 (2H, d, J=8.7 Hz)

9) $^1$H-NMR ($CDCl_3$) δ : 1.99 (2H, quint, J=6.5 Hz), 2.82 (2H, t, J=6.6 Hz), 3.82 (2H, t, J=6.5 Hz), 6.8-7.1 (4H, m), 7.1-7.3 (2H, m), 7.4-7.6 (3H, m), 7.67 (1H, s), 7.8-8.0 (3H, m), 8.42 (1H, s)

10) $^1$H-NMR ($CDCl_3$) δ : 2.00 (2H, quint, J=6.5 Hz), 2.83 (2H, t, J=6.6 Hz), 3.85 (2H, t, J=6.6 Hz), 3.86 (3H, s), 6.8-7.1 (6H, m), 7.1-7.3 (2H, m), 7.64 (1H, s), 7.8-8.0 (3H, m), 8.22 (1H, s)

11) $^1$H-NMR ($CDCl_3$) δ : 1.98 (2H, quint, J=6.5 Hz), 2.82 (2H, t, J=6.5 Hz), 3.81 (2H, t, J=6.5 Hz),

3.84 (3H, s), 6.8-7.5 (10H, m), 7.68 (1H, s), 7.95 (1H, d, J=8.2 Hz), 8.52 (1H, s)

12) $^1$H-NMR ($CDCl_3$) δ : 1.7-1.9 (2H, m), 2.70 (2H, t, J=6.6 Hz), 3.70 (2H, t, J=6.4 Hz), 6.8-7.3 (6H, m), 7.4-7.7 (2H, m), 7.8-7.9 (5H, m), 8.04 (1H, d, J=8 Hz), 8.33 (1H, s), 8.90 (1H, s)

13) $^1$H-NMR ($CDCl_3$) δ : 1.7-2.1 (17H, m), 2.84 (2H, t, J=6.5 Hz), 3.89 (2H, t, J=6.4 Hz), 6.8-7.2 (6H, m), 7.42 (1H, s), 7.56 (1H, s), 7.81 (1H, d, J=8.1 Hz)

14) $^1$H-NMR (DMSO-$d_6$) δ : 1.0-1.5 (5H, m), 1.5-2.0 (5H, m), 2.2-3.8 (8H, m), 4.2-5.2 (3H, m), 6.77 (1H, d, J=7.2 Hz), 7.1-7.4 (4H, m), 7.47 (2H, d, J=8.6 Hz), 7.58 (1H, d, J=6.2 Hz), 10.06 (1H, s), 10.9-12.1 (1H, br)

15) $^1$H-NMR (DMSO-$d_6$) δ : 2.5-3.8 (6H, m), 4.2-5.2 (3H, m), 6.81 (1H, d, J=6.8 Hz), 7.1-7.3 (4H, m), 7.5-7.7 (3H, m), 7.8-8.0 (1H, m), 7.97 (2H, d, J=1.8 Hz), 10.66 (1H, s), 11.1-12.3 (1H, br)

16) $^1$H-NMR (DMSO-$d_6$) δ : 2.20 (3H, s), 2.27 (3H, s), 2.5-3.8 (6H, m), 4.3-5.3 (3H, m), 6.82 (1H, d, J=7.2 Hz), 7.1-7.4 (7H, m), 7.5-7.8 (3H, m), 10.43 (1H, s), 11.0-12.2 (1H, br)

17) $^1$H-NMR (DMCO-$d_6$) δ : 2.34 (3H, s), 2.5-3.7 (6H, m), 4.3-5.2 (3H, m), 6.82 (1H, d, J=6.8 Hz), 7.2-7.7 (11H, m), 10.41 (1H, s), 10.8-12.3 (1H, br)

18) $^1$H-NMR (DMSO-$d_6$) δ : 2.38 (3H, s), 2.5-3.8 (6H, m),

4.3-5.3 (3H, m), 6.81 (1H, d, J=7.0 Hz), 7.1-7.5 (6H, m), 7.5-7.8 (5H, m), 10.35 (1H, s), 10.9-12.2 (1H, br)

19) $^1$H-NMR (DMSO-$d_6$) δ : 2.37 (3H, s), 2.5-3.7 (6H, m), 4.3-5.2 (3H, m), 6.81 (1H, d, J=7.2 Hz), 7.2-7.4 (6H, m), 7.5-7.7 (3H, m), 7.84 (2H, d, J=8.0 Hz), 10.31 (1H, s), 10.9-12.2 (1H, br)

20) $^1$H-NMR (DMSO-$d_6$) δ : 2.5-3.8 (6H, m), 4.3-5.2 (3H, m), 6.82 (1H, d, J=7.4 Hz), 7.2-7.3 (4H, m), 7.5-7.8 (5H, m), 7.75 (1H, d, J=1.8 Hz), 10.70 (1H, s), 10.8-12.2 (1H, br)

21) $^1$H-NMR (DMSO-$d_6$) δ : 2.5-3.8 (9H, m), 4.3-4.7 (1H, m), 4.7-5.1 (2H, m), 6.8-7.1 (3H, m), 7.1-7.4 (2H, m), 7.5-7.7 (2H, m), 7.8-8.0 (3H, m), 9.79 (1H, s), 10.8-12.2 (1H, br)

22) $^1$H-NMR (DMSO-$d_6$) δ : 0.8-1.2 (3H, m), 1.7-2.2 (2H, m), 2.5-3.8 (5H, m), 4.3-5.2 (3H, m), 6.80 (1H, d, J=7.2 Hz), 7.1-7.3 (4H, m), 7.6-7.7 (3H, m), 7.85 (1H, s), 7.96 (2H, d, J=1.8 Hz), 10.62 (1H, s), 10.8-12.0 (1H, br)

23) $^1$H-NMR (DMSO-$d_6$) δ : 0.8-1.1 (3H, m), 1.7-2.1 (2H, m), 2.37 (3H, s), 2.7-3.8 (5H, m), 4.4-5.2 (3H, m), 6.81 (1H, d, J=7.6 Hz), 7.2-7.4 (6H, m), 7.6-7.7 (3H, m), 7.84 (2H, d, J=8.2 Hz), 10.29 (1H, s), 10.5-11.8 (1H, br)

24) $^1$H-NMR (DMSO-$d_6$) δ : 0.8-1.2 (3H, m), 1.7-2.1 (2H,

m), 2.38 (3H, s), 2.6-3.8 (5H, m), 4.3-5.2 (3H, m), 6.81 (1H, d, J=7.0 Hz), 7.2-7.5 (6H, m), 7.6-7.8 (5H, m), 10.33 (1H, s), 10.5-11.7 (1H, br)

25) $^1$H-NMR (DMSO-$d_6$) δ : 0.8-1.2 (3H, m), 1.7-2.1 (2H, m), 2.6-3.8 (5H, m), 3.8-5.2 (3H, m), 6.82 (1H, d, J=7.2 Hz), 7.1-7.5 (8H, m), 7.5-7.7 (3H, m), 10.42 (1H, s), 10.7-12.0 (1H, br)

26) $^1$H-NMR (DMSO-$d_6$) δ : 0.8-2.0 (15H, m), 2.2-2.5 (1H, m), 2.6-3.7 (5H, m), 4.3-5.2 (3H, m), 6.76 (1H, d, J=7.0 Hz), 7.1-7.4 (4H, m), 7.46 (2H, d, J=8.6 Hz), 7.61 (1H, d, J=6.4 Hz), 10.03 (1H, s), 10.5-11.8 (1H, br)

27) $^1$H-NMR (DMSO-$d_6$) δ : 0.8-1.1 (3H, m), 1.7-2.0 (2H, m), 2.20 (3H, s), 2.29 (3H, s), 2.6-3.7 (5H, m), 4.3-5.2 (3H, m), 6.82 (1H, d, J=7.0 Hz), 7.2-7.4 (7H, m), 7.5-7.7 (3H, m), 10.41 (1H, s), 10.6-12.0 (1H, br)

28) $^1$H-NMR ($CDCl_3$) δ : 1.21 (3H, t, J=7.1 Hz), 3.00-3.25 (3H, m), 4.00-4.30 (4H, m), 6.63 (1H, d, J=7.8 Hz), 6.86 (1H, t, J=7.3 Hz), 7.00 (1H, t, J=6.3 Hz), 7.10-7.31 (3H, m), 7.40-7.57 (3H, m), 7.77 (2H, d, J=1.9 Hz), 8.76 (1H, brs)

29) $^1$H-NMR ($CDCl_3$) δ : 2.29 (3H, s), 2.32 (3H, s), 2.34 (3H, s), 2.50-3.15 (11H, m), 3.79 (1H, dd, J=13.2 Hz, 7.3 Hz), 4.05 (1H, dd, J=13.2 Hz, 5.7 Hz), 6.62 (1H, d, J=7.7 Hz), 6.82-7.48 (8H, m), 7.53 (2H, d,

J=8.4 Hz), 8.05 (1H, brs)

30) $^1$H-NMR ($CDCl_3$) δ : 1.65-2.01 (4H, m), 2.31 (3H, s), 2.35 (3H, s), 2.55-3.02 (6H, m), 3.09 (1H, dd, J=15 Hz, 5 Hz), 3.70 (1H, dd, J=12.5 Hz, 8.0 Hz), 4.22 (1H, dd, J=12.5 Hz, 5 Hz), 6.67 (1H, d, J=7.8 Hz), 6.80-7.32 (7H, m), 7.37 (2H, d, J=8.6 Hz), 7.53 (1H, d, J=8.3 Hz), 7.66 (1H, brs)

31) $^1$H-NMR ($CDCl_3$) δ : 2.80 (1H, dd, J=16.1 Hz, 5.3 Hz), 3.16 (1H, dd, J=15.8 Hz, 5.3 Hz), 3.75-4.50 (3H, m), 4.87-5.10 (3H, m), 6.80-7.60 (14H, m), 7.74 (2H, d, J=1.9 Hz), 8.47 (1H, brs)

32) $^1$H-NMR ($CDCl_3$) δ : 2.35 (6H, s), 2.72-3.10 (3H, m), 3.65-3.78 (1H, m), 4.06-4.18 (1H, m), 6.60-7.62 (9H, m), 7.74 (2H, d, J=1.8 Hz), 8.52 (1H, brs)

33) $^1$H-NMR ($CDCl_3$) δ : 1.87 (3H, s), 2.68 (1H, dd, J=5.6 Hz, 16 Hz), 3.14 (1H, dd, J=5.6 Hz, 16 Hz), 3.70-3.95 (2H, m), 4.32-4.50 (1H, m), 6.29 (1H, d, J=7.6 Hz), 6.90-7.80 (11H, m), 9.16 (1H, brs)

34) $^1$H-NMR ($CDCl_3$) δ : 1.62 (1H, brs), 1.90-2.25 (2H, m), 2.55 (3H, s), 3.78 (1H, t, J=5.1 Hz), 3.95 (2H, t, J=6.7 Hz), 6.69 (1H, t, J=7.9 Hz), 6.90-7.13 (2H, m), 7.23-7.40 (3H, m), 7.42-7.56 (3H, m), 7.77 (2H, d, J=1.9 Hz), 8.53 (1H, brs)

35) $^1$H-NMR ($CDCl_3$) δ : 1.80-2.02 (1H, m), 2.20-2.35 (1H, m), 2.31 (6H, s), 3.52 (1H, t, J=5.4 Hz), 3.68-3.83 (1H, m), 3.95-4.15 (1H, m), 6.59 (1H, d,

J=7.8 Hz), 6.81-7.10 (2H, m), 7.16-7.50 (6H, m), 7.80 (2H, d, J=1.8 Hz), 9.13 (1H, brs)

36) $^1$H-NMR ($CDCl_3$) δ : 1.35-1.60 (1H, m), 1.65-2.20 (3H, m), 2.65-3.20 (5H, m), 3.81 (2H, d, J=6.5 Hz), 4.90-5.10 (1H, m), 6.60 (1H, d, J=8.0 Hz), 6.90 (1H, t, J=8.0 Hz), 7.00-7.50 (6H, m)

37) $^1$H-NMR ($CDCl_3$) δ : 1.30-2.25 (4H, m), 2.55-3.20 (3H, m), 3.35 (2H, s), 3.80 (2H, s), 4.90-5.10 (1H, m), 6.62 (1H, d, J=8.0 Hz), 6.85-7.45 (12H, m), 9.27 (1H, brs)

38) $^1$H-NMR ($CDCl_3$) δ : 1.35-2.25 (4H, m), 2.33 (3H, s), 2.60-3.20 (3H, m), 3.12 (2H, s), 3.61 (2H, s), 5.00 (1H, brs), 6.50-7.60 (13H, m), 9.14 (1H, brs)

39) $^1$H-NMR ($CDCl_3$) δ : 1.27 (3H, t, J=7.1 Hz), 1.25-2.50 (12H, m), 2.70-3.10 (4H, m), 3.05 (2H, s), 4.15 (2H, q, J=7.0 Hz), 4.90-5.10 (1H, m), 6.63 (1H, d, J=7.5 Hz), 6.91 (1H, t, J=7.5 Hz), 7.00-7.50 (6H, m), 9.14 (1H, brs)

40) $^1$H-NMR ($CDCl_3$) δ : 1.30-1.65 (1H, m), 1.80-2.25 (5H, m), 2.70-3.20 (3H, m), 4.01 (2H, d, J=5.0 Hz), 4.90-5.10 (1H, m), 6.61 (1H, d, J=7.7 Hz), 6.89 (1H, t, J=7.0 Hz), 7.00-7.45 (6H, m), 9.05 (1H, brs)

41) $^1$H-NMR ($CDCl_3$) δ : 1.18 (6H, s), 1.30-2.20 (4H, m), 2.60-3.20 (3H, m), 3.30 (2H, s), 3.73 (2H, s), 4.90-5.10 (1H, m), 6.61 (1H, d, J=7.3 Hz), 6.70-

7.45 (12H, m), 9.50 (1H, brs)

42) $^1$H-NMR ($CDCl_3$) δ : 1.19 (3H, t, J=7.0 Hz), 1.30-1.70 (1H, m), 1.75-2.20 (3H, m), 2.65-3.15 (3H, m), 3.46 (2H, q, J=7.0 Hz), 3.88 (2H, s), 4.90-5.10 (1H, m), 6.55-7.45 (13H, m), 8.36 (1H, brs)

43) $^1$H-NMR ($CDCl_3$) δ : 1.08 (3H, t, J=7.2 Hz), 1.05-2.25 (14H, m), 2.25-3.25 (10H, m), 4.90-5.10 (1H, m), 6.64 (1H, d, J=7.6 Hz), 6.90 (1H, t, J=7.2 Hz), 6.94-7.50 (6H, m), 11.50 (1H, brs)

44) $^1$H-NMR ($CDCl_3$) δ : 1.06 (3H, t, J=7.5 Hz), 1.30-2.20 (6H, m), 2.60-3.20 (3H, m), 3.65 (1H, m), 3.95 (1H, brs), 4.90-5.10 (1H, m), 6.50-6.75 (3H, m), 6.75-7.05 (2H, m), 7.05-7.55 (8H, m), 8.67 (1H, brs)

45) $^1$H-NMR (DMSO-$d_6$) δ : 1.28-1.57 (1H, m), 1.69-2.20 (3H, m), 2.59-3.15 (3H, m), 4.74-4.98 (1H, m), 6.62-6.80 (1H, m), 6.86-7.37 (5H, m), 7.50-7.70 (2H, m), 8.95-9.02 (1H, m), 9.03-9.15 (2H, m), 10.85 (1H, s)

46) $^1$H-NMR ($CDCl_3$) δ : 1.40-1.66 (5H, m), 1.72-2.20 (7H, m), 2.63-3.18 (3H, m), 3.42 (2H, t, J=6.7 Hz), 4.00 (2H, t, J=6.3 Hz), 4.91-5.13 (1H, m), 6.58-6.72 (1H, m), 6.82-7.00 (3H, m), 7.02-7.30 (4H, m), 7.36-7.51 (2H, m), 7.70-7.88 (2H, m), 7.91 (1H, s)

47) $^1$H-NMR (DMSO-$d_6$) δ : 2.05-2.95 (8H, m), 3.43-3.70 (1H, m), 4.08-4.30 (1H, m), 4.72-5.00 (1H, m),

6.70-8.08 (11H, m), 10.8 (1H, s), 11.1 (1H, brs)

48) $^1$H-NMR (DMSO-$d_6$) δ : 2.10-3.00 (8H, m), 3.47-3.70 (1H, m), 4.07-4.33 (1H, m), 4.75-4.98 (1H, m), 6.78-6.91 (1H, m), 7.05-7.22 (2H, m), 7.30-7.97 (9H, m), 10.75 (1H, s), 10.94 (1H, brs)

Example 377

To a solution of 1-[4-(4-formylbenzoylamino)benzoyl]-1,2,3,4-tetrahydroquinoline (0.3 g) in methanol (10 ml) is added gradually sodium borohydride (59 mg) under ice-cooling and the mixture is stirred at room temperature for 2 hours. Water is added to the mixture and the solvent is distilled off under reduced pressure. The resulting residue is extracted with dichloroinethane, washed with water, and dried over magnesium sulfate. The solvent is distilled off under reduced pressure and the resulting residue is purified by silica gel column chromatography (eluent; dichloromethane : methanol = 50 : 1), and recrystallized from methanol to give 1-[4-(4-hydroxymethylbenzoylamino)benzoyl]-1,2,3,4-tetrahydroquinoline (165 mg) as white powder, m.p. 224.5 - 225.5°C.

Using the suitable starting materials, the compound of the above Example 37 is obtained in the same manner as in Example 377.

Example 378

To a solution of 1-[4-(4-methoxycarbonylbenzoylamino)benzoyl]-1,2,3,4-tetrahydroquinoline (0.5 g) in methanol (20 ml) is added 5 % aqueous sodium hydroxide solution (10 ml) and the mixture is stirred at room temperature overnight. Methanol is distilled off under reduced pressure and the resulting residue is acidified with diluted aqueous hydrochloric acid solution. The precipitated crystal is collected by filtration to give 1-[4-(4-carboxybenzoylamino)benzoyl]-1,2,3,4-tetrahydroquinoline (0.4 g) as white powder, m.p. >300°C.

$^1$H-NMR (DMSO-$d_6$) δ : 2.05 (2H, quint, J=6.4 Hz), 2.91 (2H, t, J=6.4 Hz), 3.86 (2H, t, J=6.4 Hz), 6.85 (1H, d, J=7.6 Hz), 6.9-7.2 (2H, m), 7.30 (1H, d, J=7.2 Hz), 7.44 (2H, d, J=8.5 Hz), 7.85 (2H, d, J=8.5 Hz), 8.1-8.2 (4H, m), 10.65 (1H, s), 13.2-13.4 (1H, br)

Using the suitable starting materials, the compounds of the above Examples 39, 241, 252, 253 and 362 are obtained in the same manner as in Example 378.

Example 379

To a solution of 1-[4-(3-acetyloxybenzoylamino)benzoyl]-1,2,3,4-tetrahydroquinoline (1.5 g) in methanol (20 ml) is added 5 % aqueous sodium hydroxide solution (10 ml) and the mixture is stirred at room temperature overnight. Methanol is distilled off under reduced pressure and the resulting residue is acidified with diluted aqueous hydrochloric acid solution. The precipitated crystal is collected by filtration and recrystallized from methanol to give 1-[4-(3-hydroxybenzoylamino)benzoyl]-1,2,3,4-tetrahydroquinoline (1.22 g) as white powder, m.p. 217 - 218°C.

Using the suitable starting materials, the compounds of the above Examples 10, 343, 356, 364 and 365 are obtained in the same manner as in Example 379.

Example 380

To a solution of 1-[4-(3-hydroxybenzoylamino)benzoyl]-1,2,3,4-tetrahydroquinoline (0.4 g) in acetone (5 ml) are added potassium carbonate (0.22 g) and ethyl iodide (0.34 g), and the mixture is refluxed for 5 hours. Then, acetone is distilled off under reduced pressure and water is added to the residue. The precipitated crystal is collected by filtration, and recrystallised from methanol to give 1-[4-(3-ethoxybenzoylamino)benzoyl]-1,2,3,4-tetrahydroquinoline (0.36 g) as white powder, m.p. 170.5 - 171.5°C.

Using the suitable starting materials, the compounds of the above Examples 11, 12, 13, 14, 33, 35, 48, 50 - 55, 90 - 92, 97 - 100, 109 - 111, 120 - 122, 136 - 138, 165 - 167, 175 - 177, 192 - 194, 211, 212, 214, 321, 322, 330 - 333, 335, 336, 339 - 342, 344 - 355, 357 - 366 and 370 - 374 are obtained in the same manner as in Example 380.

Example 381

Ethanol (50 ml) is added to 10 % Pd-C (0.1 g) and thereto is added 1-[4-(3-nitrobenzoylamino)benzoyl]-1,2,3,4-tetrahydroquinoline (0.73 g). The mixture is subjected to catalytic reduction at ordinary temperature under atmospheric pressure of hydrogen. After completion of the reduction, 10 % Pd-C is removed by filtration and the filtrate is concentrated under reduced pressure. The residue is extracted with dichloromethane and the extract is dried over magnesium sulfate. The solvent is distilled off under reduced pressure and recrystallized from methanol to give 1-[4-(3-aminobenzoylamino)benzoyl]-1,2,3,4-tetrahydroquinoline (0.54 g) as white powder, m.p. 205.5 - 206.5°C.

Using the suitable starting materials, the compounds of the above Examples 24, 334 and 338 are obtained in the same manner as in Example 381.

Example 382

To a solution of 1-(4-aminobenzoyl)-1,2,3,4-tetrahydroquinoline (0.5 g) in dichloromethane (20 ml) is added triethylamine (0.3 g), and thereto is added benzoyl chloride (0.28 g) under ice-cooling. The mixture is stirred at room temperature for 1 hour. To the reaction mixture is added water and extracted with dichloromethane. The extract is dried over magnesium sulfate and the solvent is distilled off under reduced pressure. The resulting residue is purified by silica gel column chromatography (eluent; dichloromethane : methanol = 50 : 1) and recrystallized from methanol to give 1-[4-(benzoylamino)benzoyl]-1,2,3,4-tetrahydroquinoline (245 mg) as white powder, m.p. 202.5 - 203.5°C.

Using the suitable starting materials, the compounds of the above Examples 2 - 119, 131 - 373, 375 and 376 are obtained in the same manner as in Example 382.

Example 383

Thionyl chloride (10 ml) is added to 1-(4-carboxybenzoyl)-1,2,3,4-tetrahydroquinoline (0.5 g) and the mixture is refluxed for 1 hour. Thionyl chloride is distilled off under reduced pressure to give 4-[1-(1,2,3,4-tetrahydroquinolyl)carbonyl]benzoyl chloride. Separately, to a solution of m-anisidine (0.27 g) in dichloromethane (20 ml) is added triethylamine (0.34 g), and thereto is added gradually the above obtained 4-[1-(1,2,3,4-tetrahydroquinolyl)carbonyl]benzoyl chloride under ice-cooling and the mixture is stirred at room temperature for 1 hour. Water is added to the reaction mixture and the mixture is extracted with dichloromethane. The extract is dried over magnesium sulfate. The solvent is distilled off under reduced pressure and the resulting residue is purified by silica gel column chromatography (eluent; dichloromethane : methanol = 50 : 1), and recrystallized from methanol to give 1-[4-(3-methoxyanilinocarbonyl)benzoyl]-1,2,3,4-tetrahydroquinoline (203 mg) as colorless needles, m.p. 154 - 155°C.

Using the suitable starting materials, the compounds of the above Examples 120, 122 - 130 and 374 are obtained in the same manner as in Example 383.

Example 384

To 4-oxo-1-[4-(3,5-dichlorobenzoylamino)benzoyl]-1,2,3,4-tetrahydroquinoline (0.7 g) are added tetrahyrdofuran (10 ml) and methanol (10 ml). To the mixture is added sodium borohydride (0.1 g) in portions and the mixture is stirred at room temperature for 1 hour. Water is added to the reaction mixture and the mixture is extracted with dichloromethane. The solvent is concentrated and the resulting residue is purified by silica gel column chromatography

(eluent; dichloromethane → dichloromethane : methanol = 20 : 1),

and recrystallized from ethanol to give 4-hydroxy-1-[4-(3,5-dichlorobenzoylamino)benzoyl]-1,2,3,4-tetrahydroquinoline (0.4 g) as white powder, m.p. 215 - 217°C.

Example 385

To 3-ethoxycarbonyl-1-[4-(3,5-dichlorobenzoylamino)benzoyl]-1,2,3,4-tetrahydroquinoline (0.6 g) are added an aqueous solution of sodium hydroxide (0.1 g) in water (1 ml) and ethanol (5 ml). The mixture is stirred at room temperature for 15 minutes, and acidified with diluted hydrochloric acid, extracted with dichloromethane. The solvent is distilled off and the resulting residue is purified by silica gel column chromatography

(eluent; dichloromethane → dichloromethane : methanol = 50 : 1),

and recrystallized from ethanol to give 3-carboxy-1-[4-(3,5-dichlorobenzoylamino)benzoyl]-1,2,3,4-tetrahydroquinoline (0.4 g) as white powder, m.p. 221 - 223°C.

Example 386

To 3-carboxy-1-[4-(3,5-dichlorobenzoylamino)benzoyl]-1,2,3,4-tetrahydroquinoline (3.7 g) are added tetrahydrofuran (50 ml) and thionyl chloride (5 ml). The mixture is reacted at 60°C for 1 hour. The reaction mixture is concentrated and to the residue is added acetone (20 ml). To the mixture is added dropwise a solution of sodium azide (1.0 g) in water (5 ml) under ice-cooling. The reaction mixture is stirred at the same temperature for 30 minutes and extracted with dichloromethane, dried over magnesium sulfate. The solvent is concentrated and to the resulting residue are added anhydrous toluene (30 ml) and benzyl alcohol (1.7 g). The mixture is refluxed for 1 hour. The reaction mixture is concentrated and the resulting residue is purified by silica gel column chromatography

(eluent; dichloromethane → dichloromethane : methanol = 50 : 1)

to give 3-benzyloxycarbonylamino-1-[4-(3,5-dichlorobenzoylamino)benzoyl)-1,2,3,4-tetrahydroquinoline (3.7 g) as colorless amorphous.

$^1$H-NMR ($CDCl_3$) δ : 2.80 (1H, dd, J=16.1 Hz, 5.3 Hz), 3.16 (1H, dd, J=15.8 Hz, 5.3 Hz), 3.75-4.50 (3H, m), 4.87-5.10 (3H, m), 6.80-7.60 (14H, m), 7.74 (2H, d, J=1.9 Hz), 8.47 (1H, brs)

Example 387

To 3-benzyloxycarbonylamino-1-[4-(3,5-dichlorobenzoylamino)benzoyl]-1,2,3,4-tetrahydroquinoline (3.3 g) are added acetic acid (40 ml) and 10 % Pd-C (0.4 g) and the reaction mixture is subjected to catalytic reduction at ordinary temperature under atmospheric pressure of hydrogen. One hour thereafter, the catalyst is removed by filtration and the filtrate is concentrated. The resulting residue is purified by silica gel column chromatography (eluent; dichloromethane : methanol = 20 : 1), and recrystallized from ethanol to give 3-amino-1-[4-(3,5-dichlorobenzoylamino)benzoyl]-1,2,3,4-tetrahydroquinoline (1.6 g) as white powder, m.p. 207 - 210°C.

Example 388

To 3-amino-1-[4-(3,5-dichlorobenzoylamino)benzoyl]-1,2,3,4-tetrahydroquinoline (0.5 g) are added methanol (10 ml), 37 % formaline (0.8 ml) and sodium cyanoborohydride (0.16 g). To the mixture is added acetic acid (0.5 ml) under ice-cooling and the mixture is stirred at room temperature for 1 hour. Water is added to the reaction mixture and the mixture is basified with potassium carbonate and extracted with dichloromethane. The solvent is concentrated and the resulting residue is purified by silica gel column chromatography

(eluent; dichloromethane → dichloromethane : methanol = 20 : 1)

to give 3-dimethylamino-1-[4-(3,5-dichlorobenzoylamino)benzoyl]-1,2,3,4-tetrahydroquinoline (0.3 g) as colorless amorphous.

$^1$H-NMR ($CDCl_3$) δ : 2.35 (6H, s) 2.72-3.10 (3H, m), 3.65-3.78 (1H, m), 4.06-4.18 (1H, m), 6.60-7.62 (9H, m), 7.74 (2H, d, J=1.8 Hz), 8.52 (1H, brs)

Using the suitable starting materials, the compounds of the above Examples 246, 247, 375 and 376 are obtained in the same manner as in Example 388.

Example 389

To 3-amino-1-[4-(3,5-dichlorobenzoylamino)benzoyl]-1,2,3,4-tetrahydroquinoline (0.44 g) are added dichloromethane (5 ml) and acetic anhydride (0.12 g) and the mixture is stirred for 1 hour. The reaction mixture is concentrated and the resulting residue is purified by silica gel column chromatography

(eluent; dichloromethane → dichloromethane : methanol = 50 : 1)

to give 3-acetylamino-1-[4-(3,5-dichlorobenzoylamino)benzoyl]-1,2,3,4-tetrahydroquinoline (0.3 g) as colorless amorphous.

$^1$H-NMR ($CDCl_3$) δ : 1.87 (3H, s), 2.68 (1H, dd, J=5.6 Hz, 16 Hz), 3.14 (1H, dd, J=5.6 Hz, 16 Hz), 3.70-3.95 (2H, m), 4.32-4.50 (1H, m), 6.29 (1H, d, J=7.6 Hz), 6.90-7.80 (11H, m), 9.16 (1H, brs)

Using the suitable starting materials, the compound of the above Example 242 is obtained in the same manner as in Example 389.

Example 390

To 4-oxo-1-[4-(3,5-dichlorobenzoylamino)benzoyl]-1,2,3,4-tetrahydroquinoline (0.5 g) are added 40 % solution of methylamine in methanol (5 ml), molecular sieves 4A (1 g) and dimethylformamide (6 ml), and the mixture is refluxed for 4 hours. After cooling, the reaction mixture is filtered and to the filtrate is added sodium borohydride (80 mg), and the mixture is stirred at room temperature for 1 hour. The reaction mixture is concentrated and water is added to the resulting residue, and extracted with ethyl acetate. The solvent is concentrated and the resulting residue is purified by silica gel column chromatography (eluent; dichloromethane : methanol = 20 : 1) to give 4-methylamino-1-[4-(3,5-dichlorobenzoylamino)benzoyl]-1,2,3,4-tetrahydroquinoline (0.2 g) as colorless amorphous.

$^1$H-NMR ($CDCl_3$) δ : 1.62 (1H, brs), 1.90-2.25 (2H, m), 2.55 (3H, s), 3.78 (1H, t, J=5.1 Hz), 3.95 (2H, t, J=6.7 Hz), 6.99 (1H, d, J=7.9 Hz), 6.90-7.13 (2H, m)

Using the suitable starting materials, the compounds of the above Examples 238, 239, 244, 247, 375 and 376 are obtained in the same manner as in Example 390.

Example 391

To 3-carboxy-1-[4-(3,5-dichlorobenzoylamino)benzoyl]-1,2,3,4-tetrahydroquinoline (0.7 g) are added dimethylformamide (7 ml), diethyl cyanophosphate (0.3 ml) and dimethylamine hydrochloride (0.15 g). Further thereto is added triethylamine (0.8 ml) and the mixture is stirred at room temperature for 1 hour. Water is added to the reaction mixture and extracted with ethyl acetate. The solvent is concentrated and to the resulting aside is added diethyl ether. The precipitated crystal is collected by filtration to give 3-dimethylamido-1-[4-(3,5-dichlorobenzoylamino)benzoyl]-1,2,3,4-tetrahydroquinoline (0.5 g) as light yellow powder, m.p. 186 - 187°C.

Example 392

To a solution of 1-(4-aminobenzoyl)-2,3,4,5-tetrahydro-1H-benzazepine (3.0 g) in dichloromethane (50 ml) is added succinic anhydride (1.4 g) and the mixture is stirred at room temperature for 4.5 hours. The reaction mixture is evaporated under reduced pressure in order to remove the solvent therefrom, and the resulting crystal is recrystallized from ethyl acetate to give 1-[4-(3-carboxypropionylamino)benzoyl]-2,3,4,5-tetrahydro-1H-benzazepine (3.61 g) as colorless needles, m.p. 192°C.

Using the suitable starting materials, the compound of the above Example 253 is obtained in the same manner as in Example 392.

Example 393

1-[4-(3-Carboxypropionylamino)benzoyl]-2,3,4,5-tetrahydro-1H-benzazepine (0.5 g) is dissolved in dimethylformamide (1 ml) and thereto is added dropwise diethyl cyanophosphate (0.25 g) under ice-cooling. The mixture is stirred at room temperature for 30 minutes and then cooled again with ice. Thereto are added dropwise a solution of diethylamine (0.11 g) in dimethylformamide (1 ml) and triethylamine (0.34 g). The mixture is stirred at room temperature for 16 hours. The solvent is distilled off under reduced pressure and water is added to the resulting residue. The mixture is extracted with dichloromethane. The organic layer is washed successively with diluted hydrochloric acid, water, saturated sodium hydrogen carbonate solution, water and saturated saline solution, and dried over magnesium sulfate. The solvent is distilled off under reduced pressure and the resulting residue is purified by silica gel column chromatography (eluent; ethyl acetate), and recrystallized from n-hexane/ethyl acetate to give 1-[4-(3-diethylaminocarbonylpropionylamino)benzoyl]-2,3,4,5-tetrahydro-1H-benzazepine (0.42 g) as colorless scales, m.p. 165 - 167°C.

Using the suitable starting materials, the compounds of the above Examples 255 - 263 are obtained in the same manner as in Example 393.

Example 394

To a solution of 1-[4-(2-chloroacetylamino)benzoyl]-2,3,4,5-tetrahydro-1H-benzazepine (2.06 g) in dimethylformamide (5 ml) are added sodium iodide (0.90 g), potassium carbonate (1.1 g) and cyclohexylamine (0.89 g), and the mixture is stirred at room temperature for 2 hours. Dimethylformamide is distilled off under reduced pressure and water is added to the resulting residue. The mixture is extracted with dichloromethane. The organic layer is washed successively with

water and saturated saline solution, and dried over magnesium sulfate. The solvent is distilled off under reduced pressure and the resulting residue is purified by silica gel column chromatography (eluent; ethyl acetate), and recrystallized from n-hexane/ethyl acetate to give 1-[4-(2-cyclohexylaminoacetylamino)benzoyl]-2,3,4,5-tetrahydro-1H-benzazepine (2.03 g) as white powder, m.p. 139 - 142°C.

Using the suitable starting materials, the compounds of the above Examples 271 - 309 and 317 are obtained in the same manner as in Example 394.

Example 395

o-Cresol (0.36 g) is dissolved in dimethylsulfoxide (4 ml) containing sodium hydroxide powder (0.18 g) and thereto is added 1-[4-(2-chloroacetylamino)benzoyl]-2,3,4,5-tetrahydro-1H-benzazepine (1.03 g). The mixture is stirred at 90°C for 7.5 hours. The reaction mixture is poured into ice-water (300 ml) and the precipitated crystal is collected by filtration, washed with water, and purified by silica gel column chromatography (eluent; n-hexane : ethyl acetate = 2 : 1), and recrystallized from ethyl acetate to give 1-{4-[2-(2-methylphenoxy)acetylamino]benzoyl}-2,3,4,5-tetrahydro-1H-benzazepine (546 mg) as colorless scales, m.p. 172.5 - 175°C.

Using the suitable starting materials, the compounds of the above Examples 310 and 312 - 316 are obtained in the same manner as in Example 395.

Example 396

A mixture of 1-{4-[2-(6-bromohexyloxy)benzoylamino]benzoyl}-2,3,4,5-tetrahydro-1H-benzazepine (2.00 g), sodium acetate (0.36 g), sodium iodide (0.55 g) and acetic acid (20 ml) is refluxed for 1 day. The solvent is distilled off and the resulting residue is extracted with ethyl acetate. The organic layer is washed successively with 2N aqueous sodium hydroxide solution and saturated saline solution, and dried over magnesium sulfate. The solvent is concentrated and the resulting residue is purified by silica gel column chromatography (eluent; chloroform : methanol = 500 : 1), and recrystallized from ethanol to give 1-{4-[2-(6-acetyloxyhexyloxy)benzoylamino]benzoyl}-2,3,4,5-tetrahydro-1H-benzazepine (1.07 g) as white powder, m.p. 145 - 146°C.

Using the suitable starting materials, the compound of the above Example 360 is obtained in the same manner as in Example 396.

Example 397

A mixture of 1-{4-[2-(6-bromohexyloxy)benzoylamino]benzoyl}-2,3,4,5-tetrahydro-1H-benzazepine (0.70 g), diethylamine (0.16 ml), triethylamine (0.21 ml) and acetonitrile (20 ml) is refluxed overnight. The solvent is distilled off and the resulting residue is dissolved in chloroform, washed successively with water and saturated saline solution, and dried over magnesium sulfate. The solvent is distilled off and the resulting residue is purified by silica gel column chromatography

(eluent; chloroform : methanol = 200 : 1 $\rightarrow$ 50 : 1)

and converted into the hydrochloride thereof in methanol. The product is recrystallized from methanol/diethyl ether to give 1-{4-[2-(6-diethylaminohexyloxy)benzoylamino]benzoyl}-2,3,4,5-tetrahydro-1H-benzazepine hydrochloride (0.42 g) as white powder, m.p. 91 - 95°C.

Using the suitable starting materials, the compounds of the above Examples 330, 332, 333, 335, 336, 339, 341, 342, 344 - 349, 352 - 355, 357 and 366 are obtained in the same manner as in Example 397.

Example 398

A mixture of 1-{4-[2-(6-bromohexyloxy)benzoylamino]benzoyl}-2,3,4,5-tetrahydro-1H-benzazepine (4.00 g), potassium phthalimide (2.02 g) and dimethylformamide (100 ml) is stirred at 100°C for 5 hours. The reaction mixture is filtered and the filtrate is distilled off. The resulting residue is extracted with ethyl acetate and the organic layer is washed successively with water and saturated saline solution, and dried over magnesium sulfate. The solvent is distilled off and the resulting residue is purified by silica gel column chromatography (eluent; dichloromethane), and recrystallized from methanol/diethyl ether to give 1-{4-[2-(6-phthalimidohexyloxy)benzoylamino]benzoyl}-2,3,4,5-tetrahydro-1H-benzazepine (4.06 g) as white powder, m.p. 145 - 146.5°C.

Using the suitable starting materials, the compounds of the above Examples 331, 340, 364 and 365 are obtained in the same manner as in Example 398.

Example 399

A mixture of 1-{4-[2-[6-phthalimidohexyloxy)benzoylamino]benzoyl}-2,3,4,5-tetrahydro-1H-benzazepine (3.75 g), hydrazine hydrate (0.44 ml) and ethanol (30 ml) is refluxed for 3.5 hours. The precipitated crystal is collected by filtration, dried and purified by silica gel column chromatography (eluent; chloroform : methanol : aqueous ammonia = 100 : 10 : 1), and recrystallized from methanol/diethyl ether to give 1-{4-[2-(6-aminohexyloxy)benzoylamino]benzoyl}-2,3,4,5-tetrahydro-1H-benzazepine (2.52 g) as white powder, m.p. 135.5 - 137.5°C.

Using the suitable starting materials, the compounds of the above Examples 284, 344 and 345 are obtained in the same manner as in Example 399.

Example 400

A mixture of 1-{4-(2-(6-aminohexyloxy)benzoylamino]benzoyl}-2,3,4,5-tetrahydro-1H-benzazepine (0.70 g), acetic anhydride (20 ml) and two drops of conc. sulfuric acid is stirred at room temperature for 3 hours. To the reaction mixture is added aqueous 2N aqueous sodium hydroxide solution under ice-cooling and the mixture is extracted with chloroform. The organic layer is washed successively with water and saturated saline solution, and dried over magnesium sulfate. The solvent is distilled off and the resulting residue is purified by silica gel column chromatography (eluent; chloroform : methanol = 200 : 1), and recrystallized from methanol/diethyl ether to give 1-{4-[2-(6-acetylaminohexyloxy)benzoylamino]benzoyl}-2,3,4,5-tetrahydro-1H-benzazepine (0.60 g) as colorless needles, m.p. 171 - 172°C.

Example 401

A mixture of 1-{4-[2-(6-aminohexyloxy)benzoylamino]benzoyl}-2,3,4,5-tetrahydro-1H-benzazepine (0.70 g), benzoyl chloride (0.20 ml), triethylamine and dichloromethane (20 ml) is stirred at room temperature for 1 hour. The reaction mixture is washed successively with water and saturated saline solution, and dried over magnesium sulfate. The solvent is concentrated and the resulting residue is recrystallized from ethanol to give 1-{4-[2-(6-benzoylaminohexyloxy)benzoylamino]benzoyl}-2,3,4,5-tetrahydro-1H-benzazepine (0.71 g) as white powder, m.p. 178 - 178.5°C.

Using the suitable starting materials, the compounds of the above Examples 348 and 357 are obtained in the same manner as in Examples 400 and 401.

Example 402

A mixture of 1-[4-(2-ethoxycarbonylmethoxybenzoylamino)benzoyl]-2,3,4,5-tetrahydro-1H-benzazepine (1.00 g), aqueous ammonia (100 ml), ammonium chloride (0.3 g) and methanol (150 ml) is heated at 100°C for 4 hours in a sealed tube. The solvent is distilled off and the resulting residue is extracted with chloroform, washed successively with water and saturated saline solution, and dried over magnesium sulfate. The solvent is concentrated and the resulting residue is purified by silica gel column chromatography (eluent; chloroform : methanol = 50 : 1), and recrystallized from methanol/diethyl ether to give 1-[4-(2-carbamoylmethoxybenzoylamino)benzoyl]-2,3,4,5-tetrahydro-1H-benzazepine (0.43 g) as white powder, m.p. 198 - 199°C.

Example 403

A mixture of 1-[4-(2-chloro-4-aminobenzoylamino)benzoyl]-2,3,4,5-tetrahydro-1H-benzazepine (0.55 g), acetic anhydride (15 ml), acetic acid (5 ml) and a drop of sulfuric acid is stirred at room temperature for 1 hour. To the reaction mixture is added aqueous 2N aqueous sodium hydroxide solution and the mixture is extracted with chloroform. The extract is washed with saturated saline solution and dried over magnesium sulfate. The solvent is concentrated and the resulting residue is recrystallized from methanol/diethyl ether to give 1-[4-(2-chloro-4-acetylaminobenzoylamino)benzoyl]-2,3,4,5-tetrahydro-1H-benzazepine (0.28 g) as white powder, m.p. 214 - 243°C.

Using the suitable starting materials, the compound of the above Example 44 is obtained in the same manner as in Example 403.

Example 404

A mixture of 1-[4-(1-benzyloxycarbonyl-4-piperidinylcarbonylamino)benzoyl]-2,3,4,5-tetrahydro-1H-benzazepine (8.00 g), 10 % Pd-C (0.8 g) and ethanol (250 ml) is subjected to catalytic hydrogenation at 50°C under 4 atm. of hydrogen pressure for 6 hours. The catalyst is removed by filtration and the filtrate is evaporated under reduced pressure. The resulting residue is extracted with ethyl acetate and washed successively with water and saturated saline solution, and dried over magnesium sulfate. The solvent is distilled off and the resulting residue is purified by silica gel column chromatography (eluent; chloroform : methanol : ammonium hydroxide = 50 : 10 : 1) to give 1-{4-[4-(4-piperidinyl)benzoylamino)benzoyl]-2,3,4,5-tetrahydro-1H-benzazepine (4.80 g), and a part (0.5 g) thereof is converted into the hydrochloride thereof in methanol. The hydrochloride is recrystallized from methanol/diethyl ether to give 1-{4-[4-(4-piperidinyl)benzoylamino]benzoyl}-2,3,4,5-tetrahydro-1H-benzazepine hydrochloride (0.42 g) as white powder, m.p. 177 - 181.5°C.

## Example 405

Using the suitable starting materials, the following compound is obtained in the same manner as in the above Examples 1, 382 and 388.

1-[4-(4-Dimethylaminobenzoylamino)benzoyl]-1,2,3,4-tetrahydroquinoline, colorless amorphous

$^1$H-NMR (DMSO-$d_6$) δ : 1.90-2.00 (2H, m), 2.82 (2H, t, J=6.5 Hz), 2.98 (6H, s), 3.77 (2H, t, J=6.5 Hz), 6.70-7.30 (6H, m), 7.32 (2H, d, J=8.6 Hz), 7.73 (2H, d, J=8.6 Hz), 8.00-8.20 (1H, m), 8.39 (1H, d, J=2.2 Hz), 10.37 (1H, s)

Using the suitable starting materials, the following compounds are obtained in the same manner as in Example 1.

Table 2

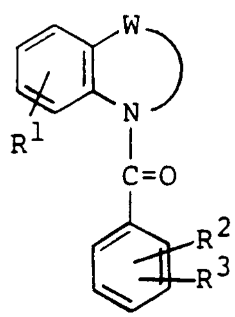

Example 406

Structure

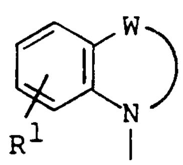

:

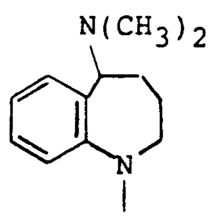

$R^2$: H

$R^3$: 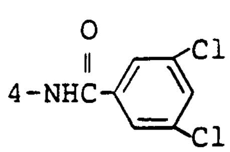

Crystalline form: White powder

Recrystallization solvent: Ethanol

Melting Point: 216 - 218°C

Form: Free

Example 407

Structure

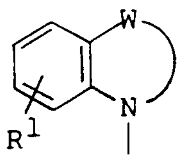 : 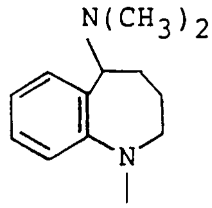

$R^2$: H

$R^3$: 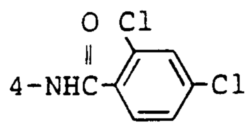

Crystalline form: White powder

Recrystallization solvent: Ethanol

Melting Point: 181 - 183°C

Form: Free

Example 408

Structure

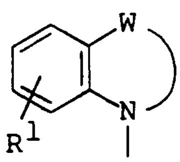 : 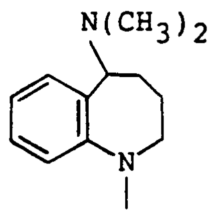

$R^2$: H

$R^3$: 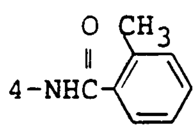

Crystalline form: White powder

Recrystallization solvent: Ethanol

Melting Point: 207 - 208°C

Form: Free

Example 409

Structure

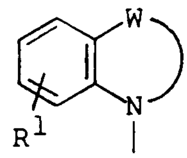 : 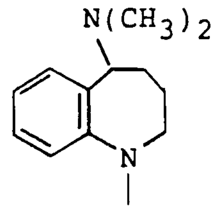

$R^2$: H

$R^3$: 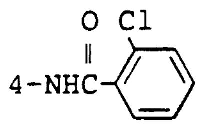

Crystalline form: White powder

Recrystallization solvent: Ethanol

Melting Point: 213 - 214°C

Form: Free

---

Example 410

Structure

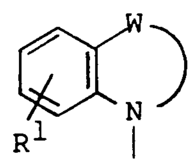 : 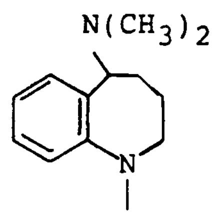

$R^2$: 3-$OCH_3$

$R^3$: 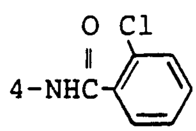

Crystalline form: White powder

Recrystallization solvent: Ethanol

Melting Point: 136 - 138°C

Form: Free

Example 411

Structure

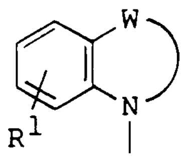

:

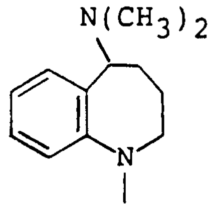

$R^2$: 3-$OCH_3$

$R^3$:

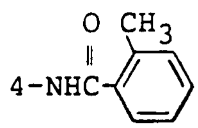

Crystalline form: White powder

Recrystallization solvent: Ethanol

Melting Point: 130 - 132°C

Form: Free

Example 412

Structure

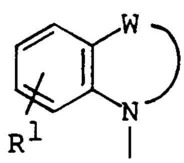

:

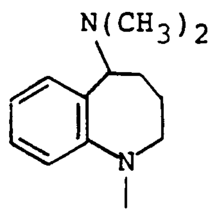

$R^2$: 3-$OCH_3$

$R^3$:

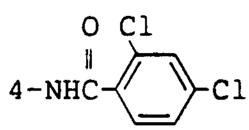

Crystalline form: White powder

Recrystallization solvent: Ethanol

Melting Point: 143 - 145°C

Form: Free

Example 413

Structure

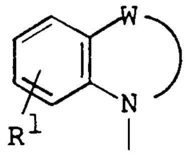 : 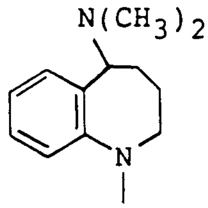

$R^2$: 3-$OCH_3$

$R^3$: 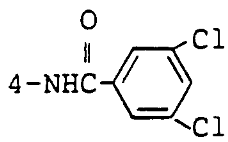

Crystalline form: White powder

Recrystallization solvent: Ethanol

Melting Point: 171 - 173°C

Form: Free

Example 414

Structure

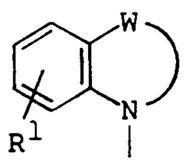 : 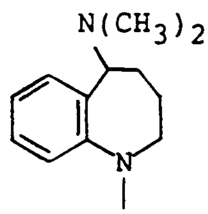

$R^2$: 3-$OCH_3$

$R^3$: 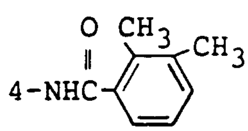

Crystalline form: White powder

Recrystallization solvent: Ethanol

Melting Point: 162 - 164°C

Form: Free

Example 415

Structure

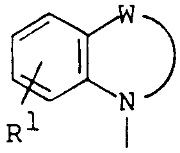 : 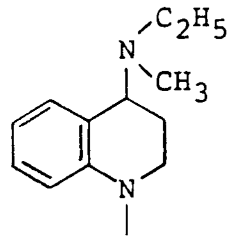

$R^2$: H

$R^3$: 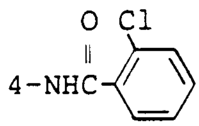

Crystalline form: Colorless amorphous

NMR analysis: 49)

Form: Free

Example 416

Structure

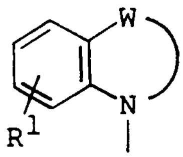 : 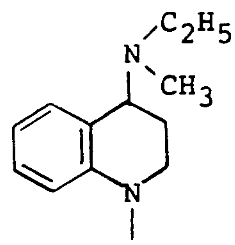

$R^2$: H

$R^3$: 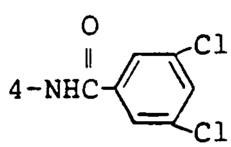

Crystalline form: Colorless amorphous

NMR analysis: 50)

Form: Free

Example 417

Structure

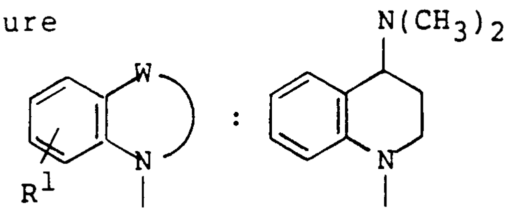

$R^2$: 3-$OCH_3$

$R^3$: 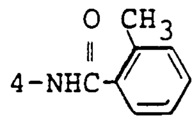

Crystalline form: Colorless amorphous

NMR analysis: 51)

Form: Free

Example 418

Structure

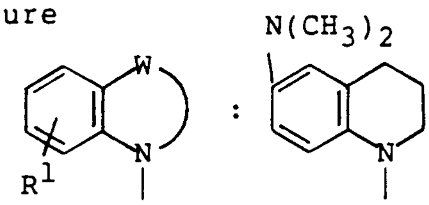

$R^2$: H

$R^3$: 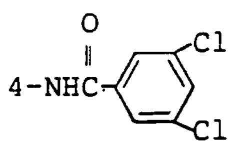

Crystalline form: Colorless needles

Recrystallization solvent: Ethyl acetate/n-hexane

Melting Point: 228.5 - 230°C

Form: Free

Example 419

Structure

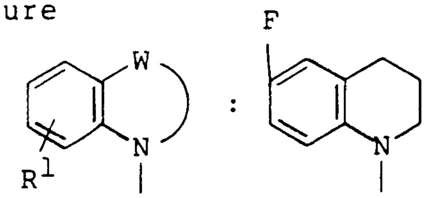

$R^2$: H

$R^3$: 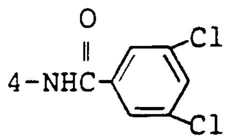

Crystalline form: White powder

Recrystallization solvent: Ethyl acetate/n-hexane

Melting Point: 205.5 - 206.5°C

Form: Free

Example 420

Structure

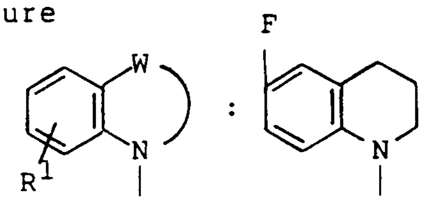

$R^2$: H

$R^3$: 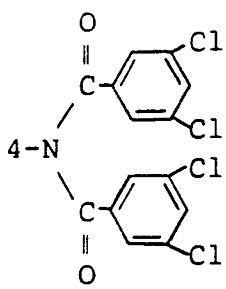

Crystalline form: White powder

Recrystallization solvent: Ethyl acetate/n-hexane

Melting Point: 210 - 212°C

Form: Free

Example 421

Structure

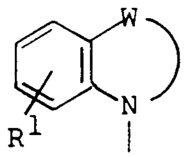

:

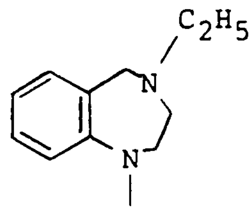

$R^2$: H

$R^3$: 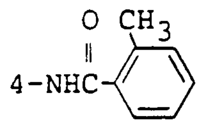

Crystalline form: White powder

Recrystallization solvent: Ethanol

Melting Point: 166 - 167°C

Form: Free

Example 422

Structure

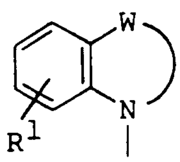

:

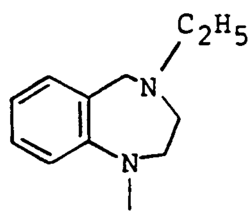

$R^2$: H

$R^3$: 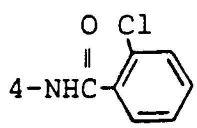

Crystalline form: White powder

Recrystallization solvent: Ethanol

Melting Point: 191.5 - 192.5°C

Form: Free

Example 423

Structure

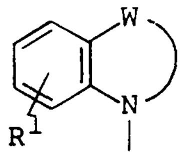 : 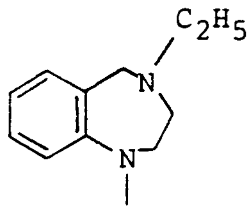 $R^2$: H

$R^3$: 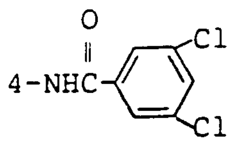

Crystalline form: White powder

Recrystallization solvent: Ethanol

Melting Point: 209 - 210°C

Form: Free

Example 424

Structure

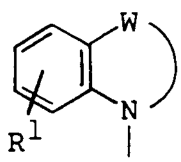 : 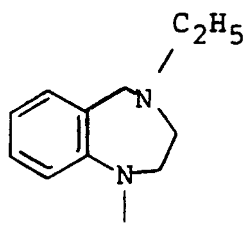 $R^2$: H

$R^3$: 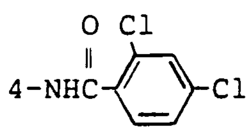

Crystalline form: Colorless amorphous

NMR analysis: 52)

Form: Free

Example 425

Structure

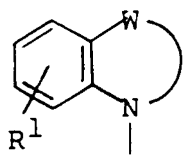

:

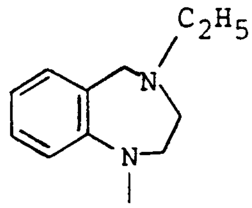

$R^2$: H

$R^3$: 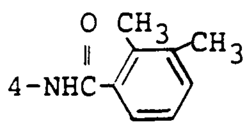

Crystalline form: White powder

Recrystallization solvent: Ethanol

Melting Point: 148 - 149°C

Form: Free

Example 426

Structure

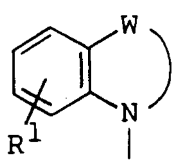

:

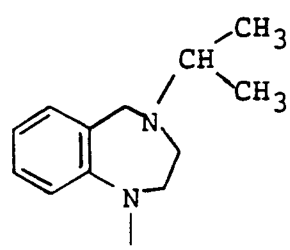

$R^2$: H

$R^3$: 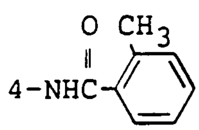

Crystalline form: White powder

Recrystallization solvent: Ethanol

Melting Point: 157 - 158°C

Form: Free

Example 427

Structure

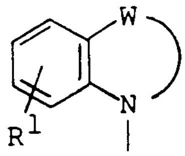 : 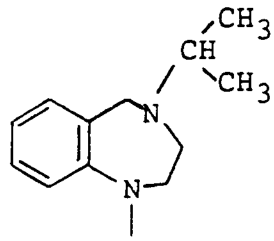

$R^2$: H

$R^3$: 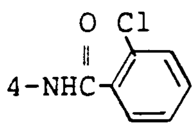

Crystalline form: White powder

Recrystallization solvent: Ethanol

Melting Point: 194.5 - 195.5°C

Form: Free

Example 1428

Structure

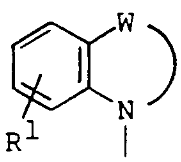 : 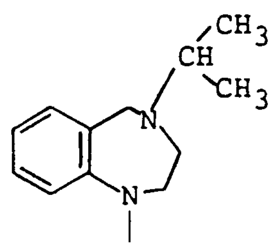

$R^2$: H

$R^3$: 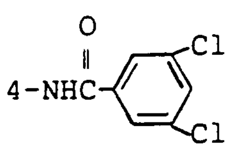

Crystalline form: White powder

Recrystallization solvent: Ethanol

Melting Point: 179.5 - 180.5°C

Form: Free

Example 429

Structure

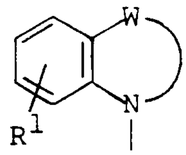 : 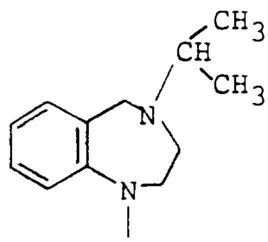

$R^2$: H

$R^3$: 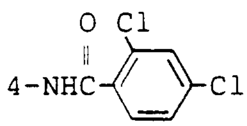

Crystalline form: White powder

Recrystallization solvent: Ethanol

Melting Point: 190 - 191°C

Form: Free

Example 430

Structure

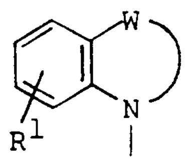 : 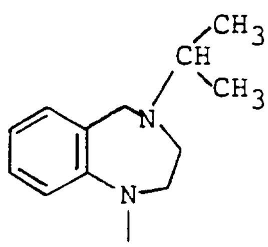

$R^2$: H

$R^3$: 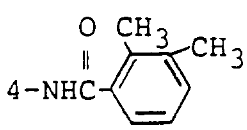

Crystalline form: White powder

Recrystallization solvent: Ethanol

Melting Point: 159 - 160°C

Form: Free

Example 431

Structure

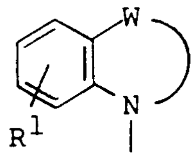 : 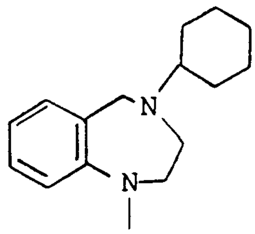

$R^2$: H

$R^3$: 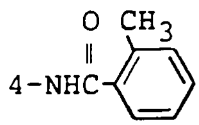

Crystalline form: Colorless amorphous

NMR analysis: 53)

Form: Hydrochloride

Example 432

Structure

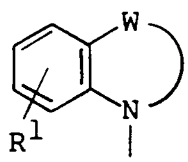 : 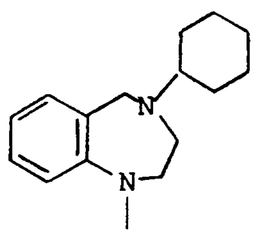

$R^2$: H

$R^3$: 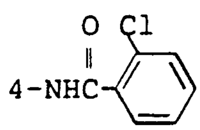

Crystalline form: White powder

Recrystallization solvent: Ethanol

Melting Point: 155 - 156°C

Form: Free

Example 433

Structure

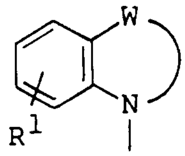 : 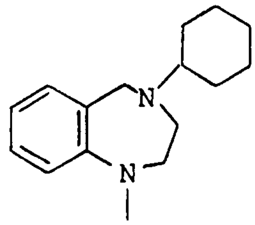 $R^2$: H

$R^3$: 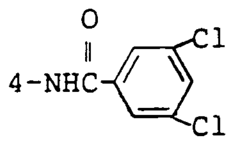

Crystalline form:Colorless amorphous

NMR analysis: 54)

Form: Free

Example 434

Structure

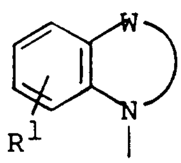 : 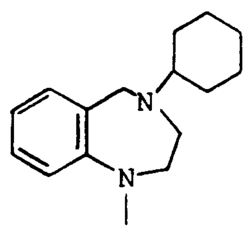 $R^2$: H

$R^3$: 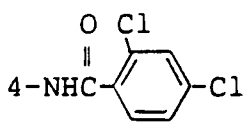

Crystalline form: Colorless amorphous

NMR analysis: 55)

Form: Free

Example 435

Structure

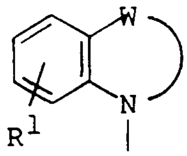 : 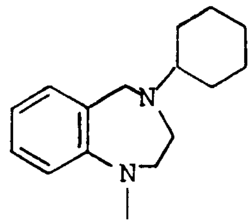

$R^2$: H

$R^3$: 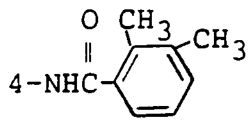

Crystalline form: White powder

Recrystallization solvent: Ethanol

Melting Point: 175 - 177°C

Form: Free

Example 436

Structure

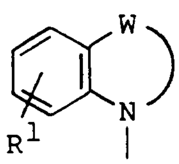 : 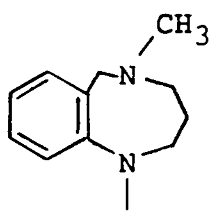

$R^2$: H

$R^3$: 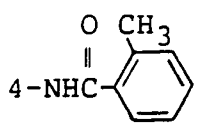

Crystalline form: Colorless amorphous

NMR analysis: 56)

Form: Free

Example 437

Structure

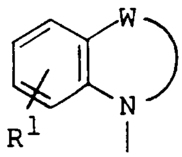 : 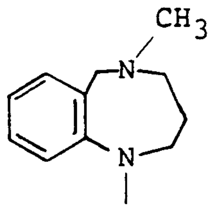 $R^2$: H

$R^3$: 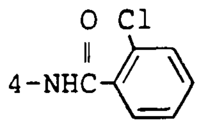

Crystalline form: Colorless amorphous

NMR analysis: 57)

Form: Free

Example 438

Structure

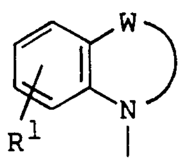 : 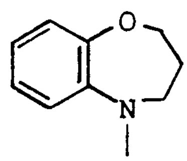 $R^2$: H

$R^3$: 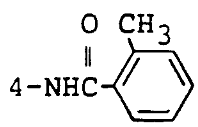

Crystalline form: White powder

Recrystallization solvent: Dichloromethane/diethyl ether

Melting Point: 219 - 220°C

Form: Free

Example 439

Structure

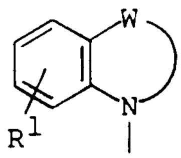 : 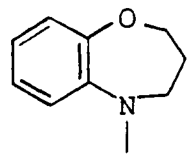

$R^2$: H

$R^3$: 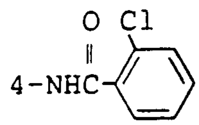

Crystalline form: White powder

Recrystallization solvent: Dichloromethane/diethyl ether

Melting Point: 215 - 218°C

Form: Free

Example 440

Structure

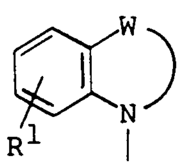 : 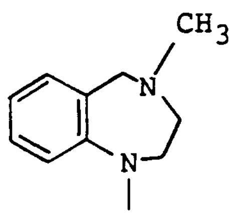

$R^2$: H

$R^3$: 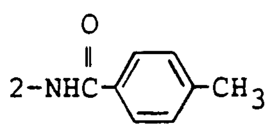

Crystalline form: White powder

Recrystallization solvent: Ethanol

Melting Point: 128.5 - 129.5°C

Form: Free

Example 441

Structure

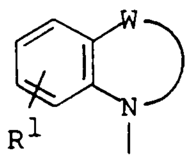 : 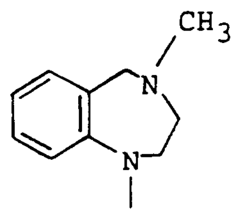

$R^2$: H

$R^3$: 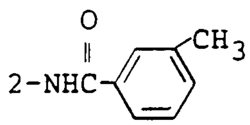

Crystalline form: Colorless amorphous

NMR analysis: 58)

Form: Free

Example 442

Structure

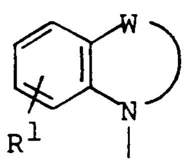 : 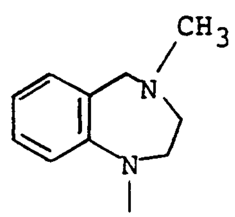

$R^2$: H

$R^3$: 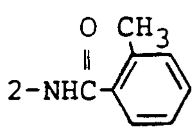

Crystalline form: White powder

Recrystallization solvent: Ethanol

Melting Point: 153 - 154°C

Form: Free

Example 443

Structure

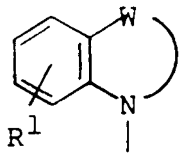 : 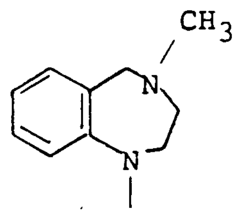

$R^2$: H

$R^3$: 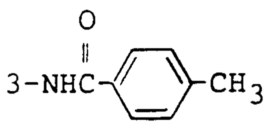

Crystalline form: White powder

Recrystallization solvent: Ethanol

Melting Point: 150 - 153°C

Form: Free

Example 444

Structure

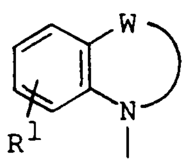 : 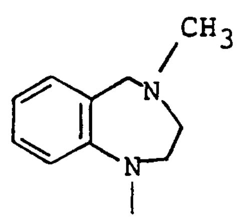

$R^2$: H

$R^3$: 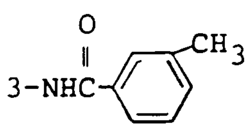

Crystalline form: White powder

Recrystallization solvent: Ethanol

Melting Point: 139 - 141°C

Form: Free

Example 445

Structure

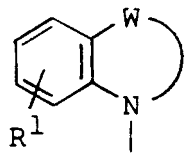 : 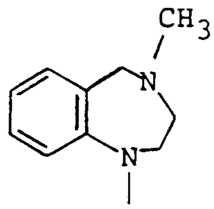

$R^2$: H

$R^3$: 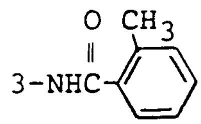

Crystalline form: Colorless amorphous

NMR analysis: 59)

Form: Free

Example 446

Structure

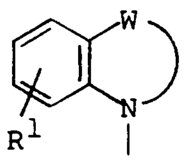 : 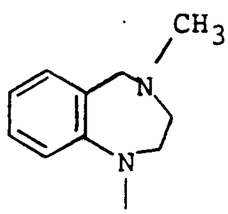

$R^2$: H

$R^3$: 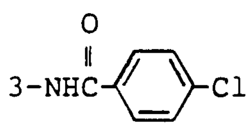

Crystalline form: Colorless amorphous

NMR analysis: 60)

Form: Free

Example 447

Structure

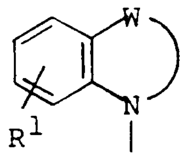 : 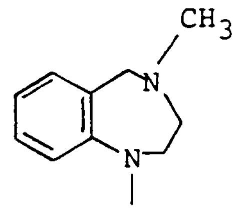

$R^2$: H

$R^3$: 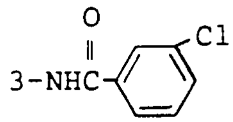

Crystalline form: Colorless amorphous

NMR analysis: 61)

Form: Free

Example 448

Structure

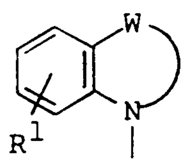 : 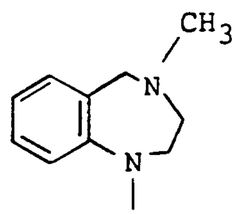

$R^2$: H

$R^3$: 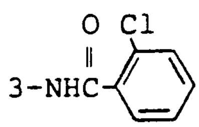

Crystalline form: Colorless amorphous

NMR analysis: 62)

Form: Free

Example 449

Structure

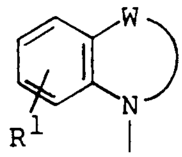 : 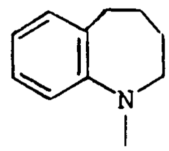

$R^2$: 3-$OCH_3$

$R^3$: 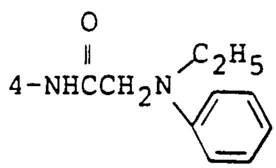

Crystalline form: Colorless amorphous

NMR analysis: 63)

Form: Free

Example 450

Structure

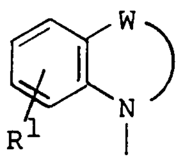 : 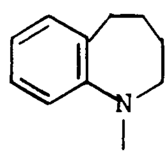

$R^2$: H

$R^3$: 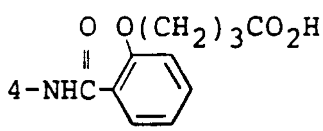

Crystalline form: White powder

Recrystallization solvent: Methanol/diethyl ether

Melting Point: 172.5 - 173.5°C

Form: Free

Example 451

Structure

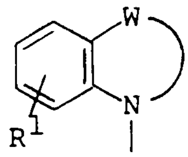 : 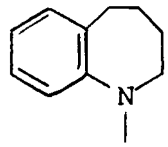

$R^2$: H

$R^3$: 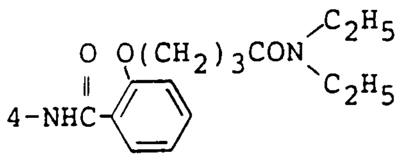

Crystalline form: Colorless prisms

Recrystallization solvent: Methanol/diethyl ether

Melting Point: 122.5 - 123°C

Form: Free

Example 452

Structure

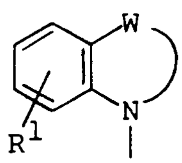 : 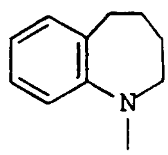

$R^2$: H

$R^3$: 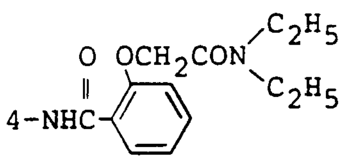

Crystalline form: White powder

Recrystallization solvent: Methanol/diethyl ether

Melting Point: 198 - 199.5°C

Form: Free

Example 453

Structure

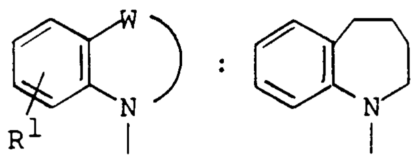

$R^2$: H

$R^3$: 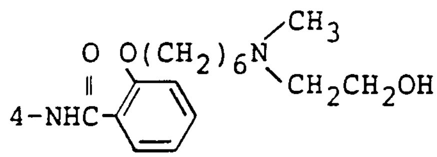

Crystalline form: White powder

Recrystallization solvent: Methanol/diethyl ether

Melting Point: 118 - 119.5°C

Form: Hydrochloride

Example 454

Structure

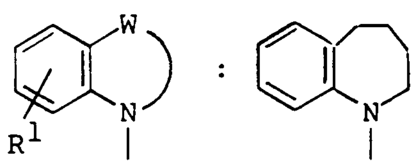

$R^2$: H

$R^3$: 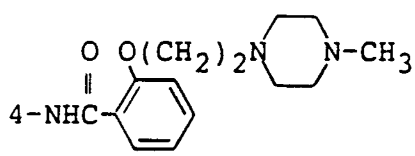

Crystalline form: White powder

Recrystallization solvent: Ethanol

Melting Point: 163 - 165°C

Form: Dihydrochloride

Example 455

Structure

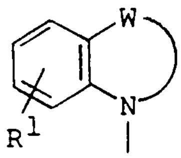 : 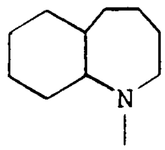

$R^2$: H

$R^3$: 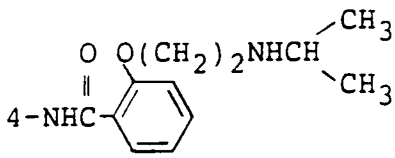

Crystalline form: White powder

Recrystallization solvent: Methanol/diethyl ether

Melting Point: 246 - 248°C

Form: Hydrochloride

Example 456

Structure

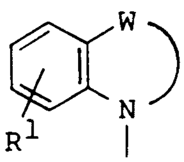 : 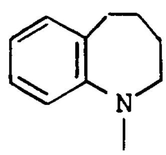

$R^2$: H

$R^3$: 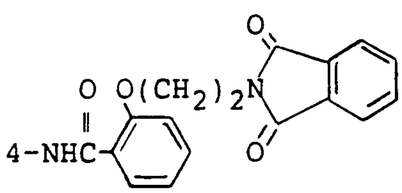

Crystalline form: White powder

Recrystallization solvent: Chloroform/ethanol

Melting Point: 204 - 205°C

Form: Free

Example 457

Structure

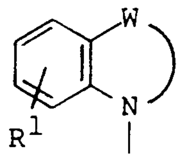 : 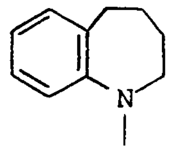

$R^2$: H

$R^3$: 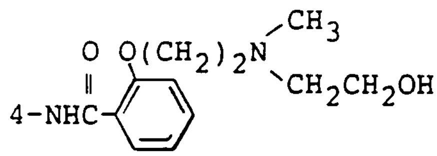

Crystalline form: Colorless prisms

Recrystallization solvent: Methanol/diethyl ether

Melting Point: 127 - 128°C

Form: Hydrochloride

Example 458

Structure

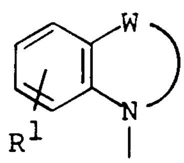 : 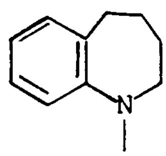

$R^2$: H

$R^3$: 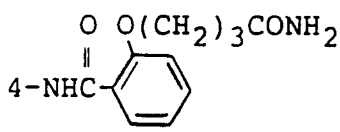

Crystalline form: White powder

Recrystallization solvent: Methanol/diethyl ether

Melting Point: 220 - 221°C

Form: Free

Example 459

Structure

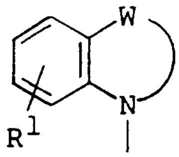 : 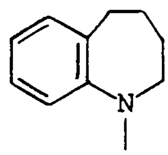

$R^2$: H

$R^3$: 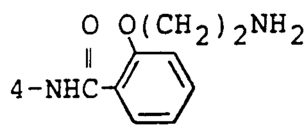

Crystalline form: Colorless needles

Recrystallization solvent: Ethanol

Melting Point: 190 - 192°C

Form: Free

Example 460

Structure

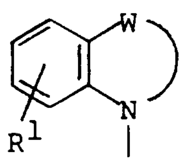 : 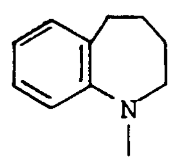

$R^2$: H

$R^3$: 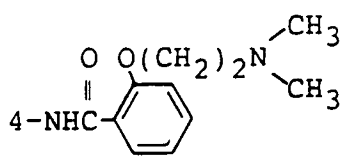

Crystalline form: White powder

Recrystallization solvent: Methanol/diethyl ether

Melting Point: 189 - 191°C

Form: Hydrochloride

Example 461

Structure

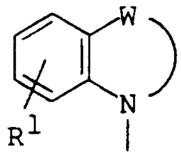 : 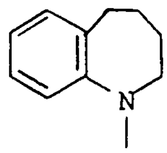

$R^2$: H

$R^3$: 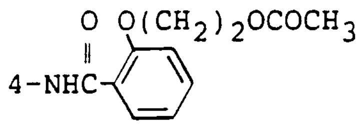

Crystalline form: Colorless needles

Recrystallization solvent: Ethanol

Melting Point: 173 - 174°C

Form: Free

Example 462

Structure

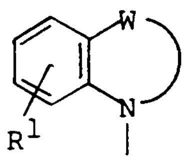 : 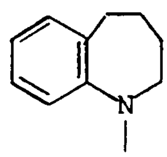

$R^2$: H

$R^3$: 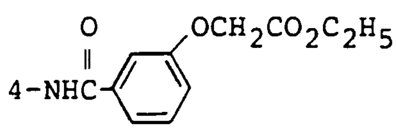

Crystalline form: White powder

Recrystallization solvent: Dichloromethane/ethanol

Melting Point: 129 - 130°C

Form: Free

Example 463

Structure

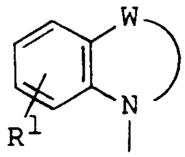 : 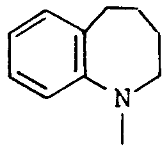

$R^2$: H

$R^3$: 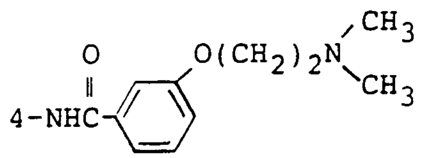

Crystalline form: White powder

Recrystallization solvent: Methanol/diethyl ether

Melting Point: 130 - 133°C

Form: Hydrochloride

Example 464

Structure

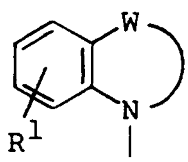 : 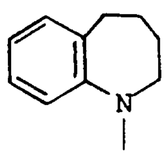

$R^2$: H

$R^3$: 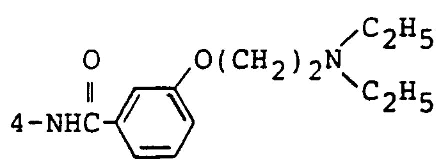

Crystalline form: Light yellow powder

Recrystallization solvent: Methanol/diethyl ether

Melting Point: 170.5 - 172°C

Form: Hydrochloride

Example 465

Structure

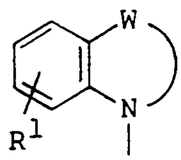 : 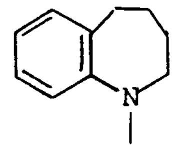

$R^2$: H

$R^3$: 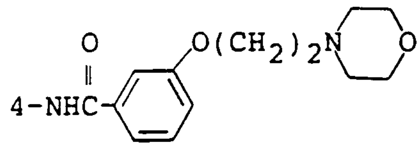

Crystalline form: White powder

Recrystallization solvent: Methanol/diethyl ether

Melting Point: 126 - 131°C

Form: Hydrochloride

Example 466

Structure

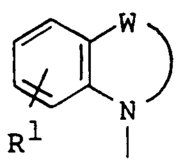 : 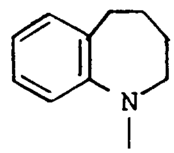

$R^2$: H

$R^3$: 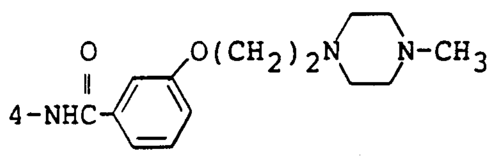

Crystalline form: White powder

Recrystallization solvent: Ethanol

Melting Point: 182 - 185°C

Form: Dihydrochloride

Example 467

Structure

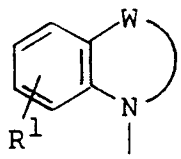 : 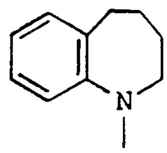

$R^2$: H

$R^3$: 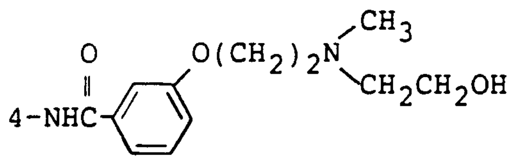

Crystalline form: White powder

Recrystallization solvent: Methanol/diethyl ether

Melting Point: 116 - 121°C

Form: Hydrochloride

Example 468

Structure

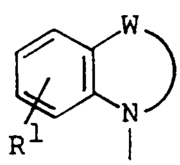 : 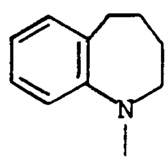

$R^2$: H

$R^3$: 4-$NHSO_2$-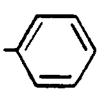

Crystalline form: Colorless prisms

Recrystallization solvent: Methanol/diethyl ether

Melting Point: 178 - 182.5°C

Form: Free

Example 469

Structure

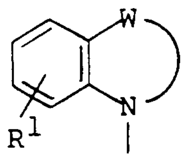 : 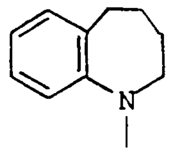

$R^2$: H

$R^3$: 4-$NHSO_2$- 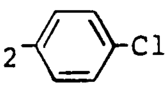

Crystalline form: Colorless particles

Recrystallization solvent: Methanol/diethyl ether

Melting Point: 185 - 187°C

Form: Free

Example 470

Structure

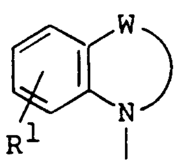 : 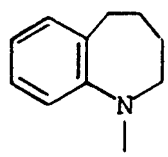

$R^2$: H

$R^3$: 4-$NHSO_2$- 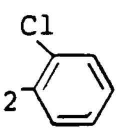

Crystalline form: White powder

Recrystallization solvent: Methanol/diethyl ether

Melting Point: 215 - 217°C

Form: Free

Example 471

Structure

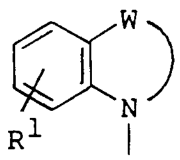 : 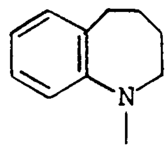

$R^2$: H

$R^3$: 4-NHSO$_2$- 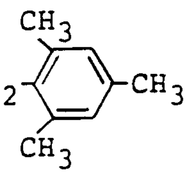

Crystalline form: White powder

Recrystallization solvent: Methanol/diethyl ether

Melting Point: 176 - 178°C

Form: Free

Example 472

Structure

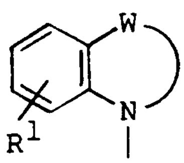 : 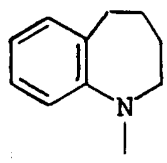

$R^2$: H

$R^3$: 4- 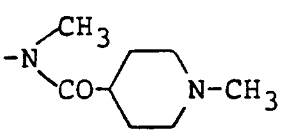

Crystalline form: Light yellow powder

Recrystallization solvent: Methanol/n-hexane

Melting Point: 194.5 - 197°C

Form: Free

Example 473

Structure

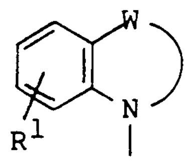 : 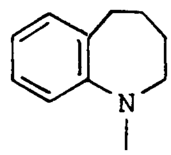

$R^2$: H

$R^3$: 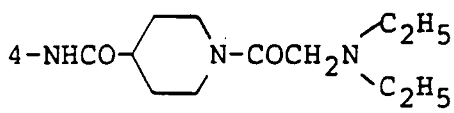

Crystalline form: White powder

Recrystallization solvent: Methanol/diethyl ether

Melting Point: 161.5 - 165.5°C

Form: Hydrochloride

Example 474

Structure

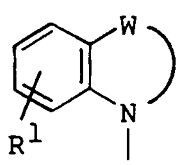 : 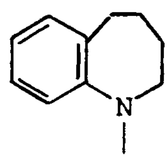

$R^2$: H

$R^3$: 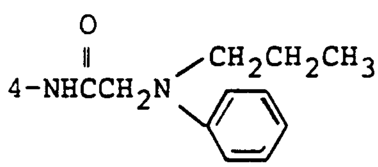

Crystalline form: White powder

Recrystallization solvent: Ethyl acetate

Melting Point: 152 - 153°C

Form: Free

Example 475

Structure

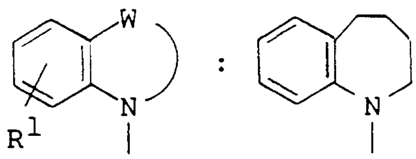

$R^2$: H

$R^3$: 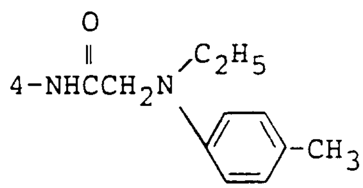

Crystalline form: Colorless needles

Recrystallization solvent: n-Hexane/ethyl acetate

Melting Point: 147 - 148°C

Form: Free

Example 476

Structure

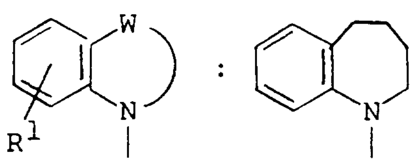

$R^2$: H

$R^3$: 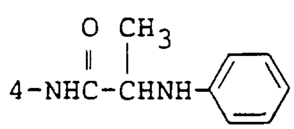

Crystalline form: Light yellow powder

Recrystallization solvent: Ethyl acetate

Melting Point: 215 - 217°C

Form: Free

Example 477

Structure

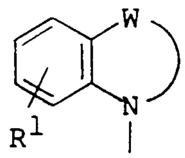 : 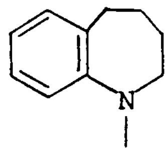 $R^2$: H

$R^3$: 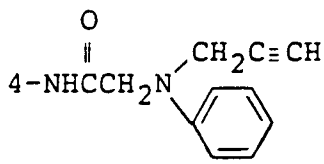

Crystalline form: Colorless amorphous

NMR analysis: 64)

Form: Free

Example 478

Structure

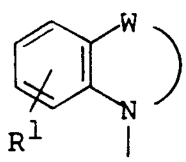 : 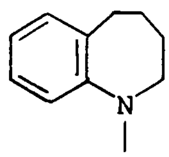 $R^2$: H

$R^3$: 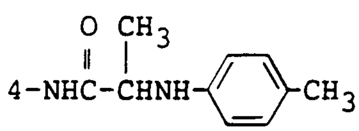

Crystalline form: White powder

Recrystallization solvent: Ethyl acetate

Melting Point: 180 - 181°C

Form: Free

Example 479

Structure

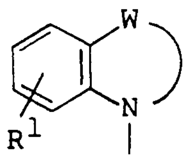 : 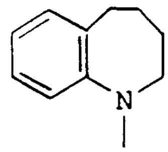

$R^2$: H

$R^3$: 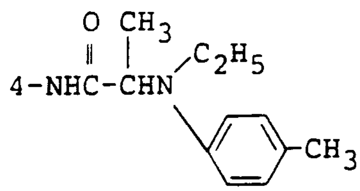

Crystalline form: Colorless amorphous

NMR analysis: 65)

Form: Free

Example 480

Structure

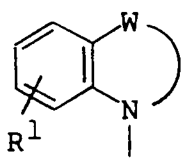 : 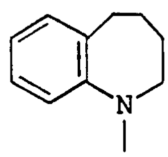

$R^2$: H

$R^3$: 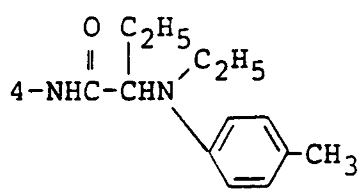

Crystalline form: Colorless amorphous

NMR analysis: 66)

Form: Free

Example 481

Structure

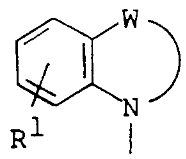 : 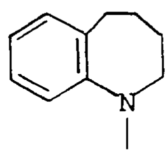

$R^2$: H

$R^3$: 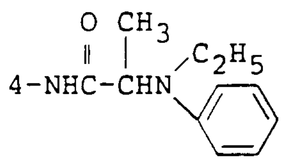

Crystalline form: Colorless amorphous

NMR analysis: 67)

Form: Free

Example 482

Structure

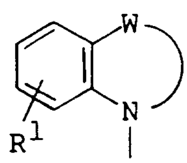 : 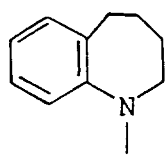

$R^2$: H

$R^3$: 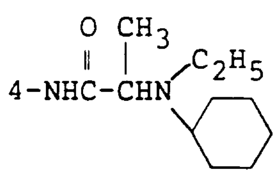

Crystalline form: Colorless scales

Recrystallization solvent: n-Hexane/ethyl acetate

Melting Point: 165 - 167°C

Form: Free

Example 483

Structure

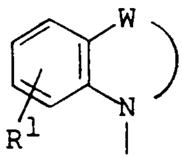 : 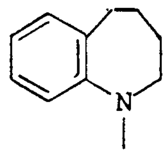 $R^2$: H

$R^3$: 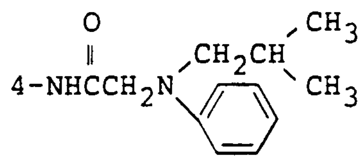

Crystalline form: Colorless amorphous

NMR analysis: 68)

Form: Free

Example 484

Structure

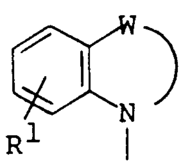 : 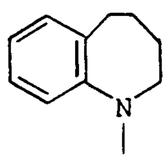 $R^2$: H

$R^3$: 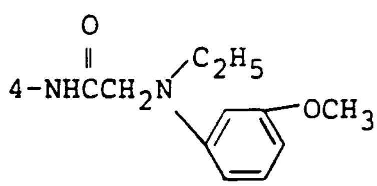

Crystalline form: Colorless amorphous

NMR analysis: 69)

Form: Free

Example 485

Structure

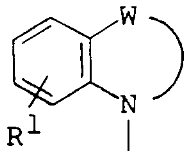 : 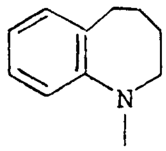

$R^2$: H

$R^3$: 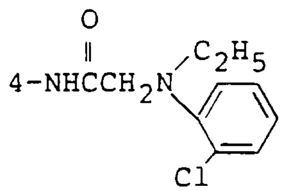

Crystalline form: Colorless amorphous

NMR analysis: 70)

Form: Free

Example 486

Structure

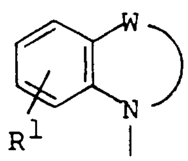 : 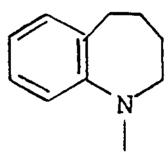

$R^2$: H

$R^3$: 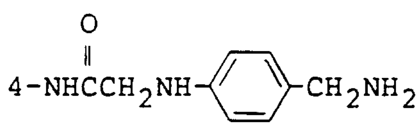

Crystalline form: Colorless amorphous

NMR analysis: 71)

Form: Free

Example 487

Structure

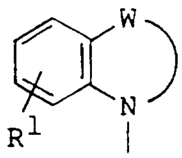 : 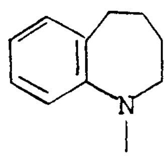

$R^2$: H

$R^3$: 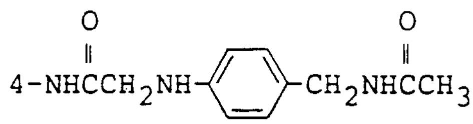

Crystalline form: Colorless amorphous

NMR analysis: 72)

Form: Free

Example 488

Structure

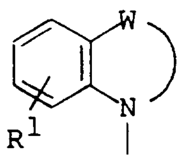 : 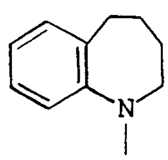

$R^2$: H

$R^3$: 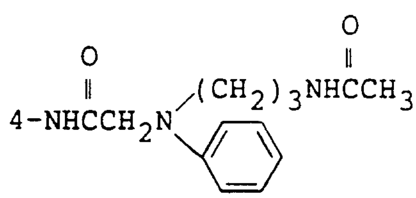

Crystalline form: Colorless amorphous

NMR analysis: 73)

Form: Free

Example 489

Structure

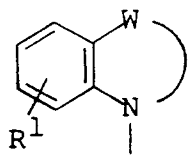 : 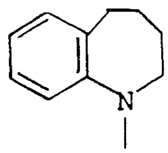

$R^2$: H

$R^3$: 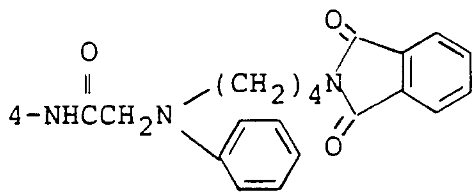

Crystalline form: Light yellow amorphous

NMR analysis: 74)

Form: Free

Example 490

Structure

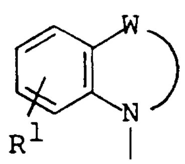 : 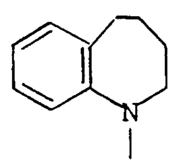

$R^2$: H

$R^3$: 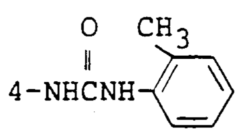

Crystalline form: White powder

Recrystallization solvent: Ethyl acetate

Melting Point: 182 - 182.5°C

Form: Free

Example 491

Structure

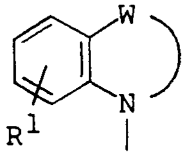 : 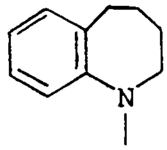

$R^2$: H

$R^3$: 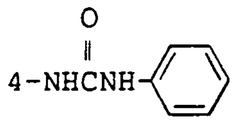

Crystalline form: White powder

Recrystallization solvent: Ethyl acetate

Melting Point: 244 - 245°C

Form: Free

Example 492

Structure

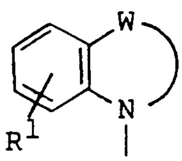 : 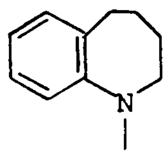

$R^2$: H

$R^3$: 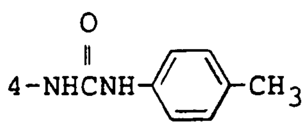

Crystalline form: White powder

Recrystallization solvent: Ethyl acetate

Melting Point: 220 - 221.5°C

Form: Free

Example 493

Structure

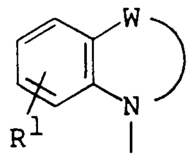 : 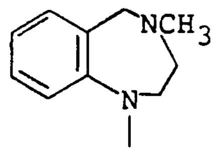 $R^2$: H

$R^3$: 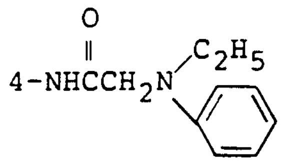

Crystalline form: Light yellow amorphous

NMR analysis: 75)

Form: Free

Example 494

Structure

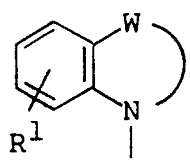 : 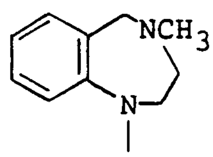 $R^2$: 3-$OCH_3$

$R^3$: 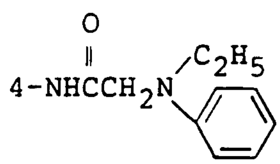

Crystalline form: Light yellow amorphous

NMR analysis: 76)

Form: Free

Example 495

Structure

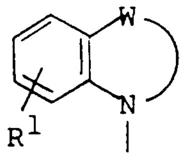 : 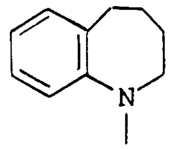

$R^2$: H

$R^3$: 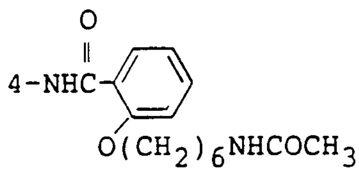

Crystalline form: Colorless needles

Recrystallization solvent: Methanol/diethyl ether

Melting Point: 171 - 172°C

Form: Free

---

Example 496

Structure

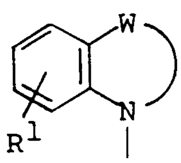 : 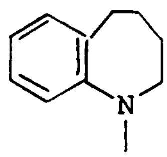

$R^2$: H

$R^3$: 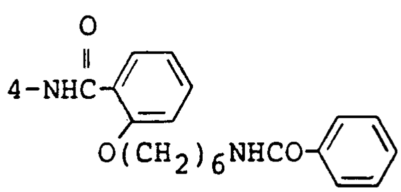

Crystalline form: White powder

Recrystallization solvent: Ethanol

Melting Point: 178 - 178.5°C

Form: Free

Example 497

Structure

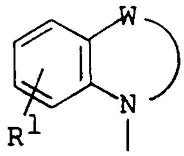 : 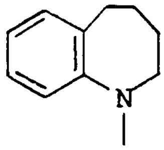

$R^2$: H

$R^3$: 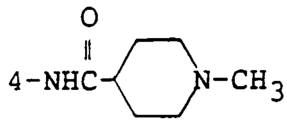

Example 498

Structure

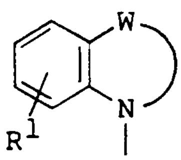 : 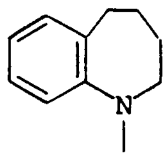

$R^2$: H

$R^3$: 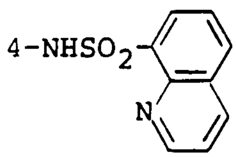

Example 499

Structure

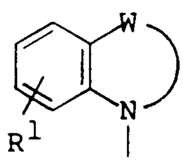 : 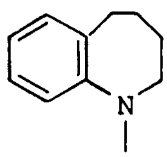

$R^2$: H

$R^3$: 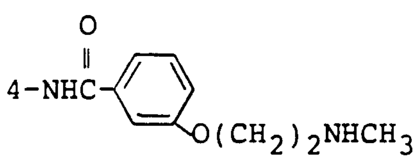

Example 500

Structure

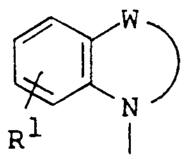 : 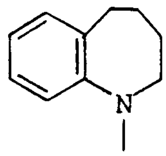

$R^2$: H

$R^3$: 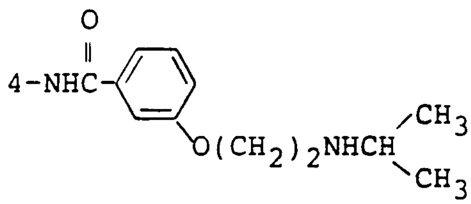

Example 501

Structure

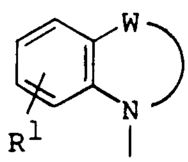 : 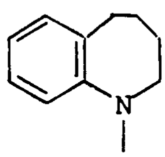

$R^2$: H

$R^3$: 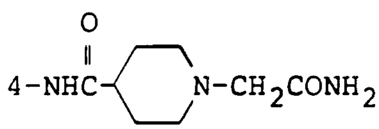

Example 502

Structure

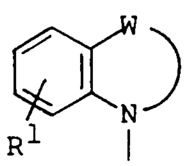 : 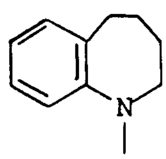

$R^2$: H

$R^3$: 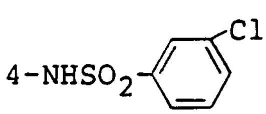

Example 503

Structure

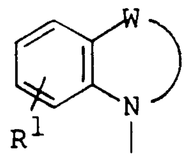 : 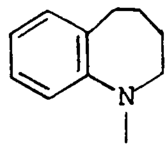

$R^2$: H

$R^3$: 4-$NHSO_2$- 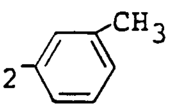

---

Example 504

Structure

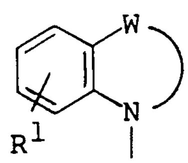 : 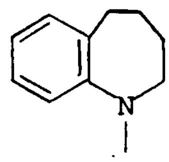

$R^2$: H

O
‖
$R^3$: 4-$NHCCH_2COOH$

Crystalline form: Light yellow scales

Recrystallization solvent: Ethanol/water

Melting Point: 129 - 131°C

Form: Free

Example 505

Structure

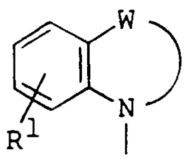 : 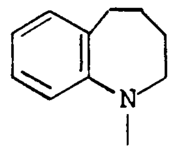 $R^2$: H

$R^3$: 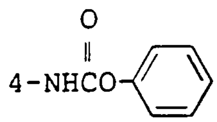

Crystalline form: White powder

Recrystallization solvent: Ethyl acetate

Melting Point: 199 - 201°C

Form: Free

Example 506

Structure

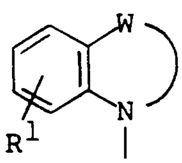 : 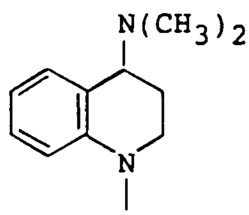 $R^2$: H

$R^3$: 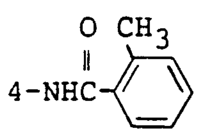

Crystalline form: Colorless amorphous

NMR analysis: 77)

Form: Free

Example 507

Structure

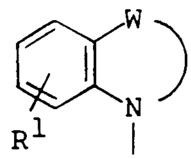 : 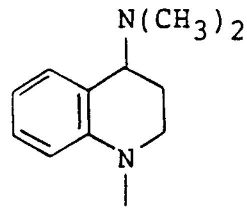 $R^2$: H

$R^3$: 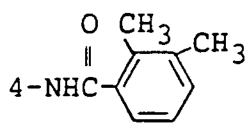

Crystalline form: White powder

Recrystallization solvent: n-Hexane/ethyl acetate

Melting Point: 187.5 - 189°C

Form: Free

Example 508

Structure

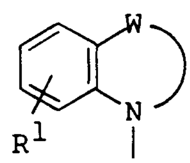 : 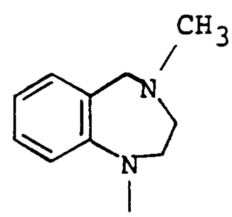 $R^2$: 2-Cl

$R^3$: 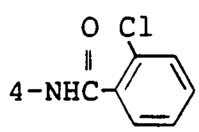

Crystalline form: White powder

Recrystallization solvent: Methanol/diethyl ether

Melting Point: 161 - 164°C

Form: Free

Example 509

Structure

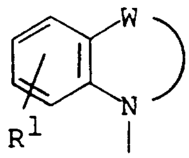 : 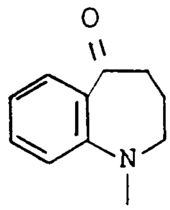

$R^2$: H

$R^3$: 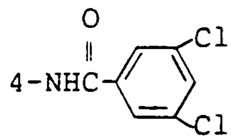

Crystalline form: Colorless prisms

Recrystallization solvent: Ethanol

Melting Point: 242 - 243°C

Form: Free

Example 510

Structure

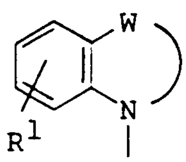 : 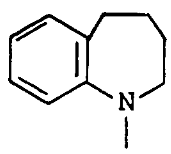

$R^2$: 3-$OCH_3$

$R^3$: 4-$NHCOCH_2Cl$

Crystalline form: White powder

Recrystallization solvent: Dichloroethane/diethyl ether

Melting Point: 186 - 188°C

Form: Free

Example 511

Structure

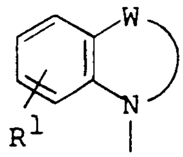 : 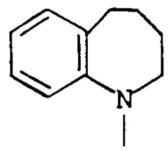

$R^2$: H

$R^3$: 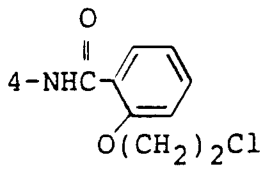

Crystalline form: Colorless amorphous

NMR analysis: 78)

Form: Free

Example 512

Structure

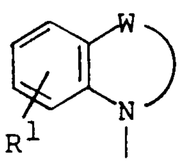 : 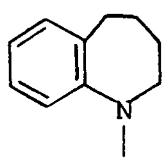

$R^2$: H

$R^3$: 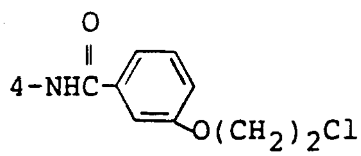

Crystalline form: Colorless amorphous

NMR analysis: 79)

Form: Free

49) $^1H$-NMR($CDCl_3$) δ ; 1.11 (3H, t, J=7.1 Hz), 1.90-2.25 (2H, m), 2.29 (3H, s), 2.55 (2H, q, J=7.1 Hz), 3.62-3.90 (2H, m), 4.00-4.20 (1H, m), 6.63 (1H, d, J=7.9 Hz), 6.85-7.10 (2H, m), 7.25-7.80 (9H, m), 8.25 (1H, brs)

50) $^1H$-NMR($CDCl_3$) δ ; 1.10 (3H, t, J=7.1 Hz), 1.90-2.20 (2H, m), 2.28 (3H, s), 3.60-3.90 (2H, m), 3.95-4.20 (1H, m), 6.62 (1H, d, J=7.9 Hz), 6.80-7.10 (2H, m), 7.20 (2H, d, J=8.6 Hz), 7.31-7.55 (4H, m), 7.80 (2H, d, J=1.9 Hz), 9.05 (1H, brs)

51) $^1H$-NMR($CDCl_3$) δ ; 1.80-2.05 (1H, m), 2.15-2.50 (1H, m), 2.34 (6H, s), 2.51 (3H, s), 3.48-3.62 (1H, m), 3.72 (3H, s), 3.70-3.85 (1H, m), 4.00-4.22 (1H, m), 6.64 (1H, d, J=7.8 Hz), 6.84-7.58 (9H, m), 8.16 (1H, brs), 8.40 (1H, d, J=8.7 Hz)

52) $^1H$-NMR($CDCl_3$) δ ; 1.16 (3H, t, J=7.1 Hz), 2.40-2.70 (2H, m), 2.90-3.30 (3H, m), 3.80-4.20 (2H, m), 4.80-5.00 (1H, m), 6.60-6.80 (1H, m), 7.00-7.70 (10H, m), 8.24 (1H, s)

53) $^1H$-NMR(DMSO-$d_6$) δ ; 1.0-2.5 (10H, m), 2.34 (3H, s), 3.30-3.80 (4H, m), 4.50-5.30 (3H, m), 6.70-7.00 (1H, m), 7.10-7.80 (11H, m), 10.43 (1H, s), 10.5-12.0 (1H, br)

54) $^1H$-NMR($CDCl_3$) δ ; 1.10-2.10 (10H, m), 2.40-2.70 (1H, m), 2.80-3.20 (3H, m), 3.92 (2H, s), 4.90-5.20 (1H, m), 6.50-6.70 (1H, m), 6.80-7.60 (8H, m), 7.75

(2H, s), 8.73 (1H, s)

55) $^1$H-NMR($CDCl_3$) δ ; 1.10-2.20 (10H, m), 2.40-2.70 (1H, m), 2.90-3.30 (3H, m), 3.93 (2H, s), 4.90-5.20 (1H, m), 6.62 (1H, d, J=7.6 Hz), 6.90-7.70 (10H, m), 8.29 (1H, s)

56) $^1$H-NMR($CDCl_3$) δ ; 1.50-2.10 (2H, m), 2.38 (6H, s), 2.30-2.70 (1H, m), 2.70-3.00 (2H, m), 3.45 (1H, d, J=13 Hz), 3.81 (1H, d, J=14 Hz), 4.70-5.00 (1H, m), 7.0-7.50 (12H, m), 8.23 (1H, s)

57) $^1$H-NMR($CDCl_3$) δ ; 1.50-2.10 (2H, m), 2.42 (3H, s), 2.40-2.70 (1H, m), 2.80-3.00 (2H, m), 3.52 (1H, d, J=13 Hz), 3.85 (1H, d, J=13 Hz), 4.70-5.00 (1H, m), 7.00-7.70 (12H, m), 8.54 (1H, s)

58) $^1$H-NMR($CDCl_3$) δ ; 2.43 (3H, s), 2.47 (3H, s), 3.00-3.30 (3H, m), 3.76 (1H, d, J=14 Hz), 4.06 (1H, d, J=14 Hz), 4.90-5.20 (1H, m), 6.50-6.80 (3H, m), 6.90-7.50 (6H, m), 7.70-8.00 (2H, m), 8.48 (1H, d, J=8 Hz), 10.58 (1H, s)

59) $^1$H-NMR($CDCl_3$) δ ; 2.41 (3H, s), 2.44 (3H, s), 2.90-3.20 (3H, m), 3.74 (1H, d, J=13 Hz), 4.07 (1H, d, J=14 Hz), 4.80-5.00 (1H, m), 6.67 (1H, d, J=7 Hz), 6.76 (1H, d, J=7 Hz), 7.00-7.50 (8H, m), 7.55 (1H, s), 7.70-7.90 (2H, m)

60) $^1$H-NMR($CDCl_3$) δ ; 2.41 (3H, s), 2.80-3.20 (3H, m), 3.73 (1H, d, J=13 Hz), 4.03 (1H, d, J=14 Hz), 6.66 (2H, d, J=7.6 Hz), 6.90-8.00 (10H, m), 8.57 (1H, s)

61) $^{1}H$-NMR($CDCl_3$) δ ; 2.40 (3H, s), 2.90-3.20 (3H, m), 3.73 (1H, d, J=13 Hz), 4.07 (1H, d, J=13 Hz), 4.70-5.00 (1H, m), 6.60-6.80 (2H, m), 6.90-8.00 (10H, m), 8.54 (1H, s)

62) $^{1}H$-NMR($CDCl_3$) δ ; 2.41 (3H, s), 2.90-3.20 (3H, m), 3.75 (1H, d, J=14 Hz), 4.08 (1H, d, J=14 Hz), 4.80-5.00 (1H, m), 6.67 (1H, d, J=7.6 Hz), 6.82 (1H, d, J=7.6 Hz), 6.90-7.90 (10H, m), 8.08 (1H, s)

63) $^{1}H$-NMR($CDCl_3$) δ ; 1.23 (3H, t, J=7 Hz), 1.40-1.70 (1H, m), 1.90-2.20 (3H, m), 2.70-3.30 (3H, m), 3.40-3.60 (5H, m), 3.91 (2H, s), 5.00-5.20 (1H, m), 6.60-7.40 (11H, m), 8.12 (1H, d, J=8 Hz), 8.99 (1H, s)

64) $^{1}H$-NMR($CDCl_3$) δ ; 1.35-1.70 (1H, m), 1.80-2.20 (3H, m), 2.25-2.35 (1H, m), 2.65-3.20 (3H, m), 4.01 (2H, s), 4.05-4.17 (2H, m), 4.90-5.10 (1H, m), 6.61 (1H, d, J=7.5 Hz), 6.75-7.50 (12H, m), 8.44 (1H, brs)

65) $^{1}H$-NMR($CDCl_3$) δ ; 1.13 (3H, t, J=7.0 Hz), 1.30-1.65 (4H, m), 1.80-2.20 (3H, m), 2.28 (3H, s), 2.65-3.40 (5H, m), 4.90-5.10 (1H, m), 6.63 (1H, d, J=7.8 Hz), 6.75-7.00 (3H, m), 7.00-7.45 (8H, m), 8.85 (1H, brs)

66) $^{1}H$-NMR($CDCl_3$) δ ; 0.88 (3H, t, J=7.4 Hz), 1.16 (3H, t, J=7.0 Hz), 1.35-2.20 (6H, m), 2.27 (3H, s), 2.60-3.20 (3H, m), 3.20-3.45 (2H, m), 3.85-4.10 (1H, m), 4.90-5.10 (1H, m), 6.63 (1H, d, J=7.4 Hz),

6.77 (2H, d, J=8.5 Hz), 6.92 (1H, t, J=8.0 Hz), 7.00-7.45 (8H, m), 8.85 (1H, brs)

67) $^1$H-NMR($CDCl_3$) δ ; 1.17 (3H, t, J=7.0 Hz), 1.35-1.65 (4H, m), 2.60-3.45 (5H, m), 4.20 (2H, q, J=7.0 Hz), 4.90-5.10 (1H, m), 6.63 (1H, d, J=7.6 Hz), 6.80-7.45 (12H, m), 8.66 (1H, brs)

68) $^1$H-NMR($CDCl_3$) δ ; 0.96 (6H, d, J=6.6 Hz), 1.35-1.65 (1H, m), 1.80-2.25 (4H, m), 2.65-3.15 (3H, m), 3.19 (2H, d, J=7.3 Hz), 3.99 (2H, s), 4.90-5.10 (1H, m), 6.60 (1H, d, J=7.8 Hz), 6.75-7.05 (4H, m), 7.05-7.40 (8H, m), 8.15 (1H, brs)

69) $^1$H-NMR($CDCl_3$) δ ; 1.19 (3H, t, J=7.0 Hz), 1.35-1.65 (1H, m), 1.80-2.25 (3H, m), 2.70-3.20 (3H, m), 3.44 (2H, q, J=7.0 Hz), 3.77 (3H, s), 3.87 (2H, s), 4.90-5.10 (1H, m), 6.25-6.50 (3H, m), 6.67 (1H, d, J=7.5 Hz), 6.85-7.45 (8H, m), 8.29 (1H, brs)

70) $^1$H-NMR($CDCl_3$) δ ; 1.05 (3H, t, J=7.1 Hz), 1.35-1.65 (1H, m), 1.85-2.25 (3H, m), 2.65-3.30 (5H, m), 3.74 (2H, s), 4.95-5.15 (1H, m), 6.63 (1H, d, J=7.5 Hz), 6.80-7.55 (11H, m), 9.51 (1H, brs)

71) $^1$H-NMR($CDCl_3$) δ ; 1.30-1.65 (1H, m), 1.80-2.30 (3H, m), 2.65-3.15 (3H, m), 3.75 (2H, s), 3.74 (2H, s), 4.95-5.10 (1H, m), 6.45-6.70 (3H, m), 6.88 (1H, t, J=6.8 Hz), 7.00-7.45 (8H, m), 8.74 (1H, brs)

72) $^1$H-NMR($CDCl_3$) δ ; 1.30-1.70 (1H, m), 1.75-2.25 (6H, m), 2.65-3.15 (3H, m), 3.78 (2H, d, J=5.4 Hz), 4.28

(2H, d, J=5.5 Hz), 4.53 (1H, brs), 4.90-5.10 (1H, m), 5.89 (1H, brs), 6.50-6.70 (3H, m), 6.89 (1H, t, J=7.5 Hz), 7.00-7.40 (8H, m), 8.61 (1H, brs)

73) $^1$H-NMR($CDCl_3$) δ ; 1.35-1.65 (1H, m), 1.70-2.20 (8H, m), 2.65-3.20 (3H, m), 3.25-3.55 (4H, m), 3.88 (2H, s), 4.90-5.10 (1H, m), 5.79 (1H, brs), 6.55-7.40 (13H, m), 8.37 (1H, brs)

74) $^1$H-NMR($CDCl_3$) δ ; 1.35-2.00 (8H, m), 2.65-3.20 (3H, m), 3.30-3.35 (2H, m), 3.60-3.85 (2H, m), 3.90 (2H, s), 4.95-5.15 (1H, m), 6.55-7.00 (5H, m), 7.00-7.40 (8H, m), 7.65-7.90 (4H, m), 8.22 (1H, brs)

75) $^1$H-NMR($CDCl_3$) δ ; 1.16 (3H, t, J=7.0 Hz), 2.39 (3H, s), 2.80-3.20 (3H, m), 3.44 (2H, q, J=7.0 Hz), 3.65-4.20 (4H, m), 4.80-5.05 (1H, m), 6.50-7.45 (13H, m), 8.50 (1H, brs)

76) $^1$H-NMR($CDCl_3$) δ ; 1.23 (3H, t, J=7.0 Hz), 2.41 (3H, s), 2.75-3.20 (3H, m), 3.40-3.60 (5H, m), 3.65-3.90 (1H, m), 3.92 (2H, s), 3.90-4.20 (1H, m), 4.85-5.10 (1H, m), 6.65-7.45 (11H, m), 8.13 (1H, d, J=8.4 Hz), 9.01 (1H, brs)

77) $^1$H-NMR($CDCl_3$) δ ; 1.80-1.95 (1H, m), 2.20-2.70 (10H, m), 3.50-3.60 (1H, m), 3.63-3.80 (1H, m), 4.00-4.15 (1H, m), 6.60 (1H, d, J=7.6 Hz), 6.92 (1H, t, J=7.6 Hz), 7.02 (1H, t, J=6.3 Hz), 7.20-7.65 (9H, m), 7.87 (1H, brs)

78) $^1$H-NMR($CDCl_3$) δ ; 1.40-1.62 (1H, m), 1.84-2.22 (3H,

m), 2.65-3.19 (3H, m), 3.97 (2H, t, J=4.9 Hz), 4.43 (2H, t, J=4.9 Hz), 4.95-5.18 (1H, m), 6.60-6.77 (1H, m), 6.85-7.02 (2H, m), 7.02-7.30 (5H, m), 7.40-7.68 (3H, m), 8.20-8.32 (1H, m), 9.62-9.81 (1H, m)

79) $^1$H-NMR($CDCl_3$) δ ; 1.38-1.65 (1H, m), 1.84-2.21 (3H, m), 2.64-3.15 (3H, m), 3.81 (2H, t, J=5.7 Hz), 4.25 (2H, t, J=5.7 Hz), 4.90-5.13 (1H, m), 6.58-6.71 (1H, m), 6.82-7.00 (1H, m), 7.00-7.52 (10H, m), 8.11 (1H, brs)

Example 513

To a solution of 1-(4-aminobenzoyl)-2,3,4,5-tetrahydro-1H-benzazepine (1.06 g) in dichloromethane (80 ml) is added o-methylphenyl isocyanate (0.66 g) under ice-cooling. The mixture is stirred at room temperature for 4 hours. After completion of the reaction, the solvent is concentrated under reduced pressure and the resulting residue is purified by silica gel column chromatography (eluent; n-hexane : ethyl acetate = 1 : 1), and recrystallized from ethyl acetate to give 1-[4-(2-methylanilinocarbonylamino)benzoyl]-2,3,4,5-tetrahydro-1H-benzazepine (0.97 g) as white powder, m.p. 182 - 182.5°C.

Using the suitable starting materials, the compounds of the above Examples 491 - 492 are obtained in the same manner as in Example 513.

Example 514

A mixture of 1-(4-aminobenzoyl)-2,3,4,5-tetrahydro-1H-benzazepine (0.50 g), phenylsulfonyl chloride (0.29 ml), triethylamine (0.32 ml) and dichloromethane (30 ml) is stirred at room temperature overnight. The reaction mixture is washed successively with water and saturated saline solution, and dried over magnesium sulfate. The solvent is distilled off and the resulting residue is purified by silica gel column chromatography (eluent; chloroform), and recrystallized from methanol/diethyl ether to give 1-(4-phenylsulfonylaminobenzoyl)-2,3,4,5-tetrahydro-1H-benzazepine (0.27 g) as colorless prisms, m.p. 178 - 182.5°C.

Using the suitable starting materials, the compounds of the above Examples 469 - 471, 498, 502 and 503 are obtained in the same manner as in Example 514.

Example 515

To a solution of 1-[4-(4-piperidinylcarbonylamino)benzoyl]-2,3,4,5-tetrahydro-1H-benzazepine (0.70 g) in dimethylformamide (20 ml) is added 60 % sodium hydride dispersion in mineral oil (82 mg) and the mixture is stirred at room temperature for 30 minutes. Thereto is added methyl iodide (0.14 ml) and the mixture is stirred ar room temperature overnight. The solvent is distilled off and the resulting residue is extracted with chloroform, and washed successively with water and saturated saline solution, and dried over magnesium sulfate. The solvent is distilled off and the resulting residue is purified by silica gel column chromatography (eluent; chloroform : methanol = 10 : 1), and recrystallized from methanol/n-hexane to give 1-{4-[N-(1-methyl-4-piperidinylcarbonyl)-N-methylamino]benzoyl}-2,3,4,5-tetrahydro-1H-benzazepine (0.03 g) as light yellow powder, m.p. 194.5 - 197°C.

Using the suitable starting materials, the compounds of the above Examples 497 and 501 are obtained in the same manner as in Example 515.

Example 516

6-Fluoro-1-(4-aminobenzoyl)-1,2,3,4-tetrahydroquinoline (0.15 g) is dissolved in dichloromethane (10 ml) and thereto is added triethylamine (0.31 ml). To the mixture is added dropwise a solution of 3,5-dichlorobenzoyl chloride (0.14 g) in dichloromethane (2.0 ml) under ice-cooling, and the mixture is stirred for 30 minutes under ice-cooling, and further, at room temperature for 1 hour. To the mixture are added triethylamine (0.31 ml) and 3,5-dichlorobenzoyl chloride (0.14 ml). The mixture is stirred at room temperature for 4 hours. The reaction mixture is washed with water, and dried over magnesium sulfate. The solvent is distilled off and the resulting residue is purified by silica gel column chromatography

(eluent; ethyl acetate : n-hexane = 1 : 5 → 1 : 4),

and recrystallized from ethyl acetate/n-hexane to give 6-fluoro-1-[4-(3,5-dichlorobenzoylamino)benzoyl]-1,2,3,4-tetrahydroquinoline (0.12 g) and 6-fluoro-1-{4-[bis-(3,5-dichlorobenzoyl)amino]benzoyl}-1,2,3,4-tetrahydroquinoline.

The former: White powder, m.p. 205.5 - 206.5°C
The latter: White powder, m.p. 210.5 - 212°C

Example 517

Using the suitable starting materials, the compounds of the above Examples 450 and 504 are obtained in the same manner as in Example 378.

Example 518

Using the suitable starting materials, the compounds of the above Examples 450 - 467, 495, 496, 499, 500, 511 and 512 are obtained in the same manner as in Example 380.

Example 519

Using the suitable starting materials, the compounds of the above Examples 449, 474 - 489, 493 and 494 are obtained in the same manner as in Example 394.

Example 520

Using the suitable starting materials, the compounds of the above Examples 453, 455, 457, 459, 460, 463 - 467, 495, 496 and 499 are obtained in the same manner as in Example 397.

Example 521

Using the suitable starting materials, the compound of the above Example 461 is obtained in the same manner as in Example 396.

Example 522

Using the suitable starting materials, the compound of the above Example 456 is obtained in the same manner as in Example 398.

Example 523

Using the suitable starting materials, the compound of the above Example 459 is obtained in the same manner as in Example 399.

Example 524

Using the suitable starting materials, the compounds of the above Examples 495 and 496 are obtained in the same manner as in Examples 400 and 401.

Example 525

Using the suitable starting materials, the compound of the above Example 458 is obtained in the same manner as in Example 402.

Using the suitable starting materials, the compounds of the following Table 3 are obtained in the same manner as in Examples 1 and 382.

Table 3

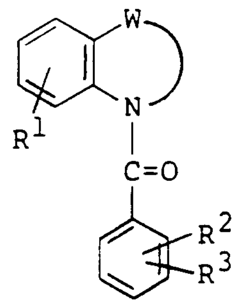

Example 527

Structure

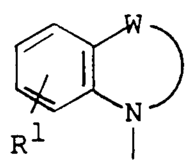

:

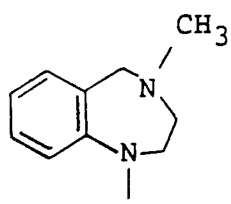

$R^2$: 2-Cl

$R^3$: 4-

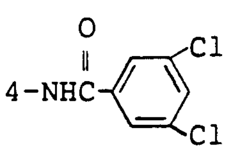

Crystalline form: White powder

Recrystallization solvent: Methanol/diethyl ether

Melting Point: 225 - 226°C

Form: Free

Example 528

Structure

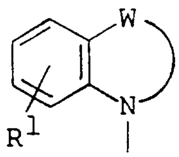 : 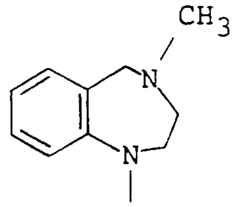

$R^2$: 2-Cl

$R^3$: 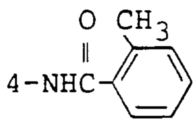

Crystalline form: White powder

Recrystallization solvent: Methanol/diethyl ether

Melting Point: 142.5 - 145°C

Form: Free

Example 529

Structure

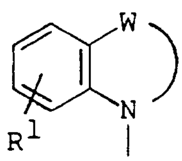 : 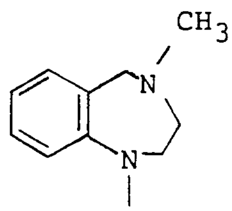

$R^2$: 2-Cl

$R^3$: 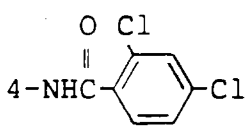

Crystalline form: White powder

Recrystallization solvent: Methanol/diethyl ether

Melting Point: 213 - 215°C

Form: Free

Example 530

Structure

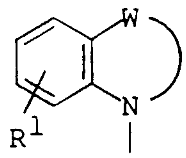 : 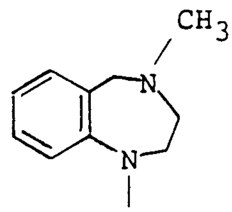

$R^2$: 3-$CH_3$

$R^3$: 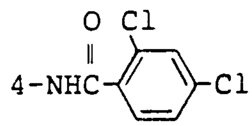

Crystalline form: White powder

Recrystallization solvent: Methanol/diethyl ether

Melting Point: 167 - 167.5°C

Form: Free

Example 531

Structure

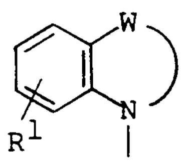 : 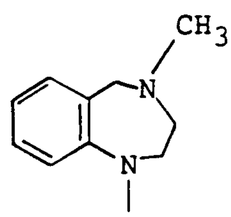

$R^2$: 3-$CH_3$

$R^3$: 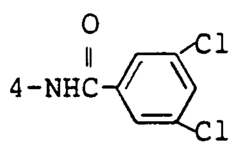

Crystalline form: Colorless scales

Recrystallization solvent: Methanol/diethyl ether

Melting Point: 217 - 221°C

Form: Free

Example 532

Structure

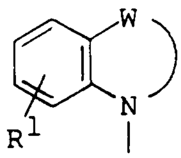 : 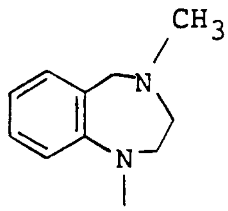

$R^2$: 3-$CH_3$

$R^3$: 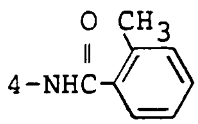

Crystalline form: White powder

Recrystallization solvent: Methanol/diethyl ether

Melting Point: 182 - 184°C

Form: Free

Example 533

Structure

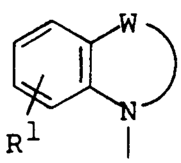 : 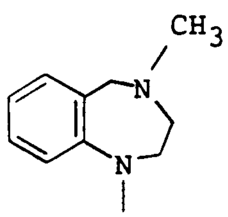

$R^2$: 3-$CH_3$

$R^3$: 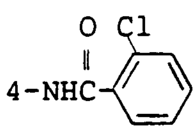

Crystalline form: White powder

Recrystallization solvent: Methanol/diethyl ether

Melting Point: 209 - 210°C

Form: Free

Example 534

Structure

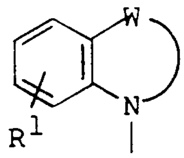 : 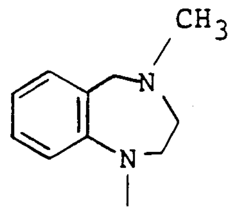

$R^2$: 3-$OCH_3$

$R^3$: 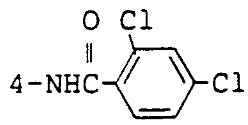

Crystalline form: White powder

Recrystallization solvent: Methanol/diethyl ether

Melting Point: 148 - 149°C

Form: Free

Example 535

Structure

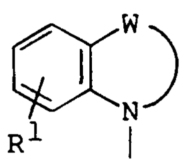 : 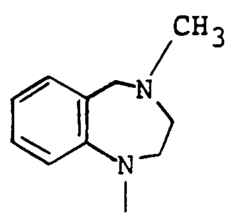

$R^2$: H

$R^3$: 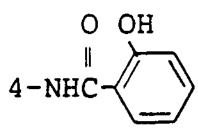

Crystalline form: White powder

Recrystallization solvent: Methanol/diethyl ether

Melting Point: 202 - 203°C

Form: Free

Example 536

Structure

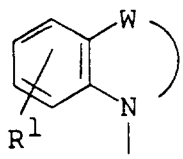 : 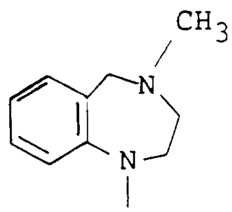

$R^2$: 3-$CH_3$

$R^3$: 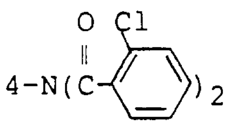

Crystalline form: White powder

Recrystallization solvent: Methanol/diethyl ether

Melting Point: 218 - 219°C

Form: Free

Example 537

Structure

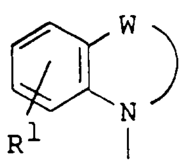 : 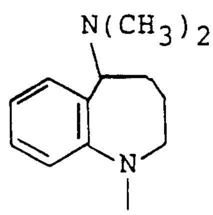

$R^2$: 2-Cl

$R^3$: 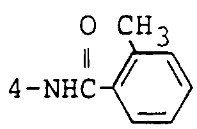

Crystalline form: White powder

Recrystallization solvent: Methanol/n-hexane

Melting Point: 159 - 160°C

Form: Free

Example 538

Structure

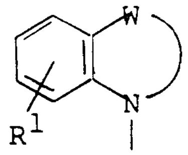 : 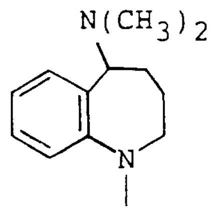 $R^2$: 2-Cl

$R^3$: 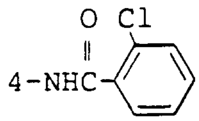

Crystalline form: White powder

Recrystallization solvent: Methanol/n-hexane

Melting Point: 201 - 202°C

Form: Free

---

Example 539

Structure

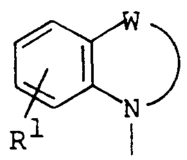 : 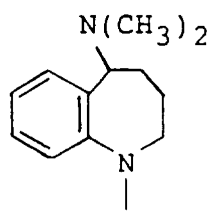 $R^2$: 2-Cl

$R^3$: 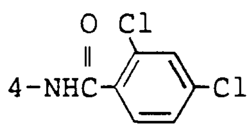

Crystalline form: White powder

Recrystallization solvent: Methanol/n-hexane

Melting Point: 205 - 207°C

Form: Free

Example 540

Structure

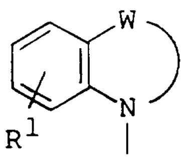

:

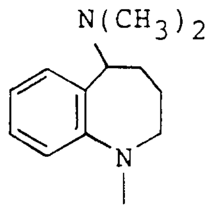

$R^2$: 3-OH

$R^3$: 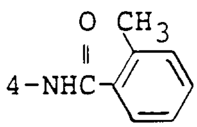

Crystalline form: White powder

Recrystallization solvent: Methanol/diethyl ether

Melting Point: 201.5 - 202.5°C

Form: Free

Example 541

Structure

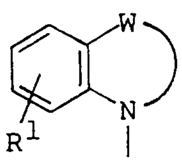

:

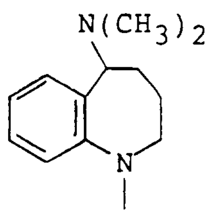

$R^2$: 2-$OCH_3$

$R^3$: 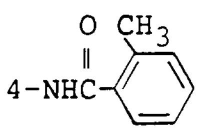

Crystalline form: White powder

Recrystallization solvent: Methanol/diethyl ether

Melting Point: 226 - 228°C

Form: Free

Example 542

Structure

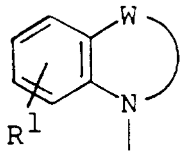 : 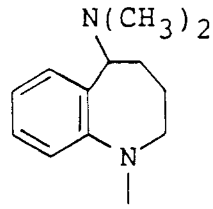

$R^2$: 2-$OCH_3$

$R^3$: 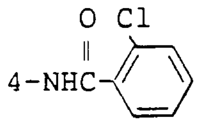

Crystalline form: White powder

Recrystallization solvent: Methanol/diethyl ether

Melting Point: 218 - 221°C

Form: Free

Example 543

Structure

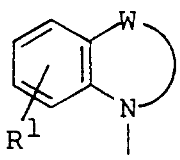 : 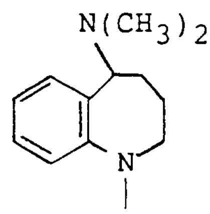

$R^2$: 2-$OCH_3$

$R^3$: 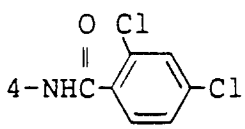

Crystalline form: White powder

Recrystallization solvent: Methanol/diethyl ether

Melting Point: 156 - 157°C

Form: Free

Example 544

Structure

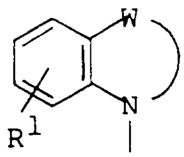 : 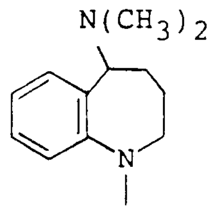 $R^2$: 2-$OCH_3$

$R^3$: 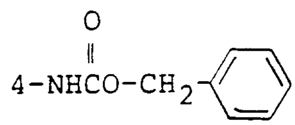

Crystalline form: White powder

NMR analysis: 80)

Form: Free

Example 545

Structure

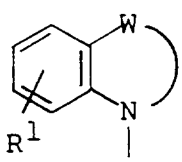 : 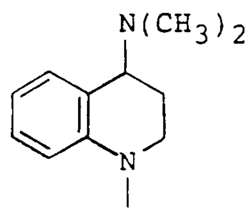 $R^2$: 3-$OCH_3$

$R^3$: 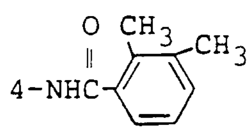

Crystalline form: Colorless amorphous

NMR analysis: 81)

Form: Free

Example 546

Structure

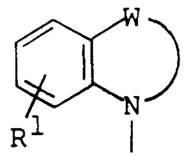 : 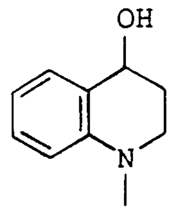

$R^2$: H

$R^3$: 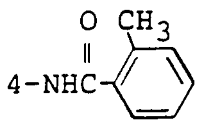

Crystalline form: Colorless amorphous

NMR analysis: 82)

Form: Free

Example 547

Structure

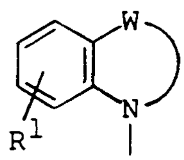 : 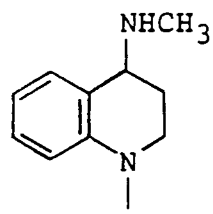

$R^2$: H

$R^3$: 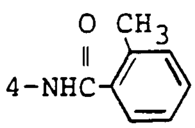

Crystalline form: Light yellow amorphous

NMR analysis: 83)

Form: Free

Example 548

Structure

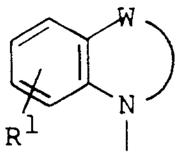

:

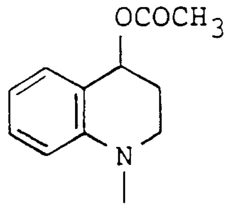

$R^2$: H

$R^3$: 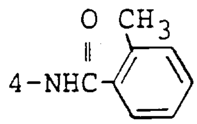

Crystalline form: Colorless amorphous

NMR analysis: 84)

Form: Free

Example 549

Structure

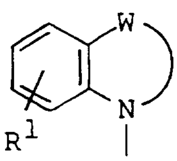

:

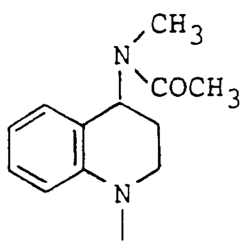

$R^2$: H

$R^3$: 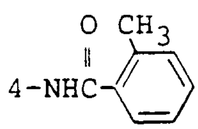

Crystalline form: Colorless amorphous

NMR analysis: 85)

Form: Free

Example 550

Structure

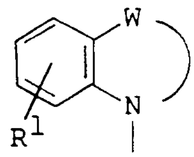

:

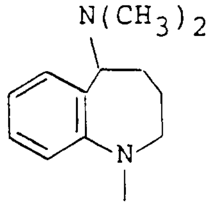

$R^2$: 3-$OCH_2CH_3$

$R^3$: 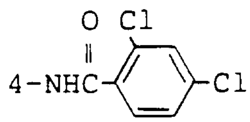

Crystalline form: White powder

Recrystallization solvent: n-Hexane/ethyl acetate

Melting Point: 135 - 136°C

Form: Free

Example 551

Structure

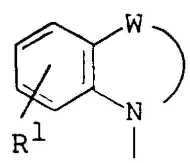

:

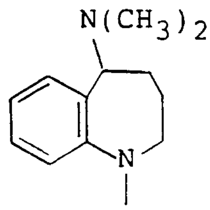

$R^2$: 3-$OCH_2CH_3$

$R^3$: 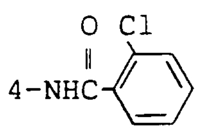

Crystalline form: Colorless prisms

Recrystallization solvent: n-Hexane/ethyl acetate

Melting Point: 122 - 123°C

Form: Free

Example 552

Structure

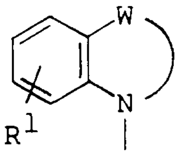
:
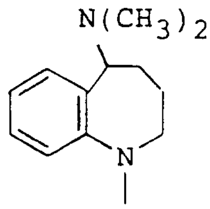

$R^2$: 3-$OCH_2CH_3$

$R^3$: 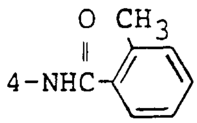

Crystalline form: Colorless prisms

Recrystallization solvent: n-Hexane/ethyl acetate

Melting Point: 118 - 119°C

Form: Free

Example 553

Structure

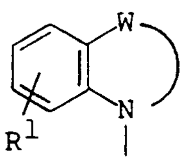
:
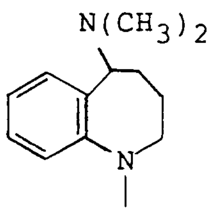

$R^2$: 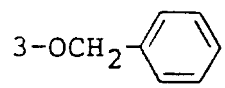

$R^3$: 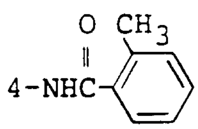

Crystalline form: Colorless prisms

Recrystallization solvent: n-Hexane/ethyl acetate

Melting Point: 145 - 147°C

Form: Free

Example 554

Structure

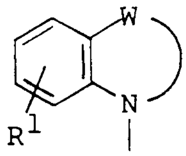 : 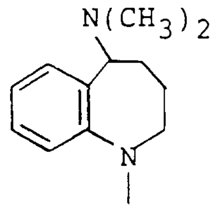

$R^2$: 3-OCH$_2$-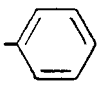

$R^3$: 4-NHC-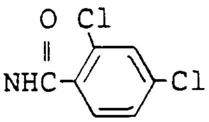

Crystalline form: Light yellow needles

Recrystallization solvent: n-Hexane/ethyl acetate

Melting Point: 169.5 - 170.5°C

Form: Free

Example 555

Structure

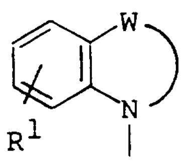 : 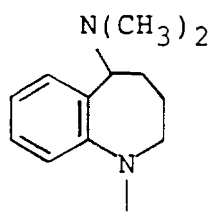

$R^2$: 3-OH

$R^3$: 4-NHC-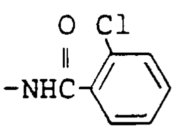

Crystalline form: Colorless prisms

Recrystallization solvent: n-Hexane/ethyl acetate

Melting Point: 194 - 195°C

Form: Free

Example 556

Structure

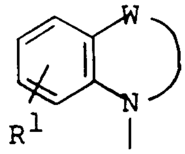 : 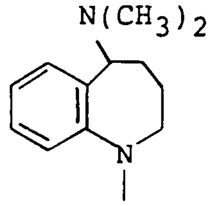

$R^2$: 3-OH

$R^3$: 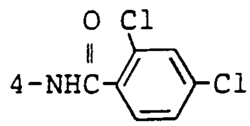

Crystalline form: Colorless needles

Recrystallization solvent: n-Hexane/ethyl acetate

Melting Point: 202 - 204°C

Form: Free

Example 557

Structure

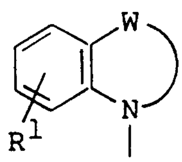 : 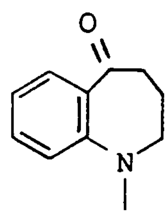

$R^2$: H

$R^3$: 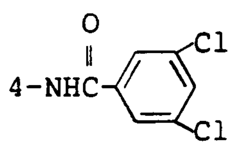

Crystalline form: Colorless prisms

Recrystallization solvent: Ethanol

Melting Point: 242 - 243°C

Form: Free

Example 558

Structure

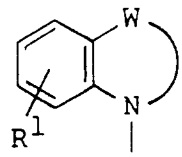 : 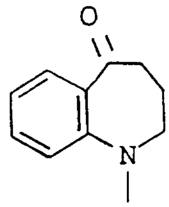

$R^2$: H

$R^3$: 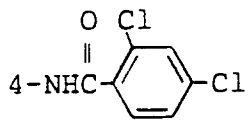

Crystalline form: Light yellow powder

NMR analysis: 86)

Form: Free

Example 559

Structure

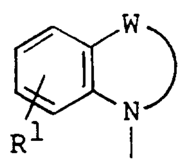 : 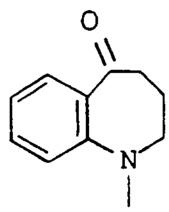

$R^2$: H

$R^3$: 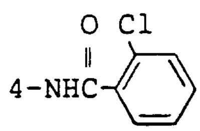

Crystalline form: Light yellow powder

NMR analysis: 87)

Form: Free

Example 560

Structure

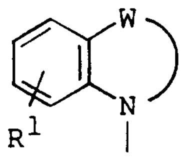 : 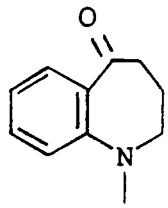

$R^2$: H

$R^3$: 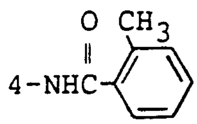

Crystalline form: Colorless needles

Recrystallization solvent: Ethanol

Melting Point: 237 - 238°C

Form: Free

Example 561

Structure

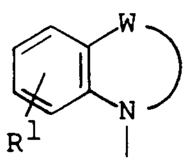 : 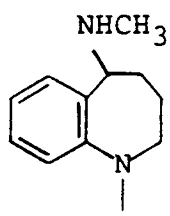

$R^2$: H

$R^3$: 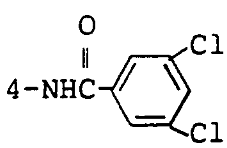

Crystalline form: Colorless prisms

Recrystallization solvent: Dioxane

Melting Point: 258 - 259°C

Form: Free

Example 562

Structure

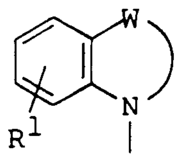 : 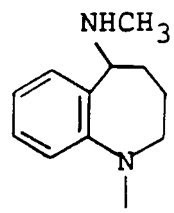

$R^2$: H

$R^3$: 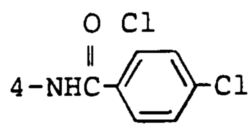

Crystalline form: Colorless prisms

Recrystallization solvent: Ethanol

Melting Point: 182.5 - 183.5°C

Form: Free

Example 563

Structure

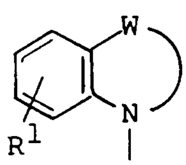 : 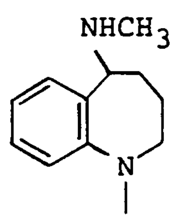

$R^2$: H

$R^3$: 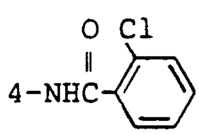

Crystalline form: Colorless prisms

Recrystallization solvent: Ethanol

Melting Point: 209 - 211°C

Form: Free

Example 564

Structure

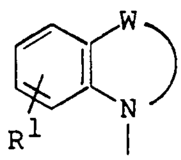 : 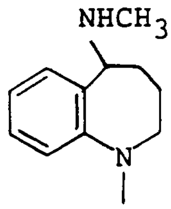 $R^2$: H

$R^3$: 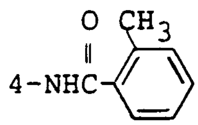

Crystalline form: Colorless prisms

Recrystallization solvent: Dioxane

Melting Point: 210 - 211°C

Form: Free

Example 565

Structure

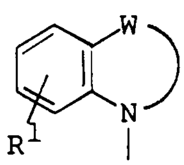 : 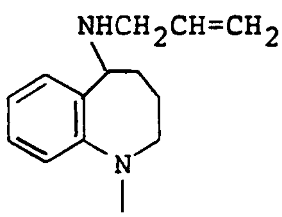 $R^2$: H

$R^3$: 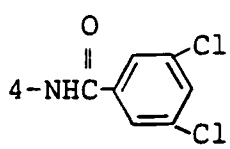

Crystalline form: Colorless needles

Recrystallization solvent: Ethyl acetate/n-hexane

Melting Point: 176 - 178°C

Form: Free

Example 566

Structure

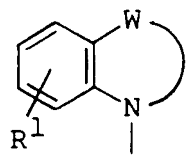

:

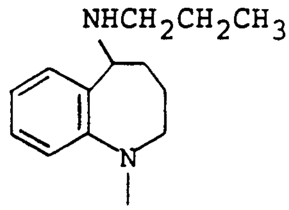

$R^2$: H

$R^3$: 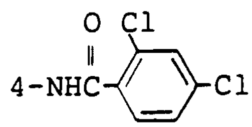

Crystalline form: Light yellow amorphous

NMR analysis: 88)

Form: Free

Example 567

Structure

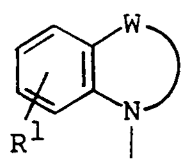

:

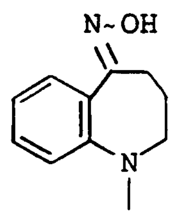

$R^2$: H

$R^3$: 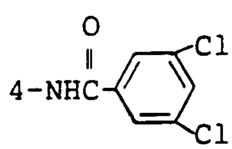

Crystalline form: White powder

Recrystallization solvent: Dioxane/water

Melting Point: 272 - 273°C

Form: Free

Example 568

Structure

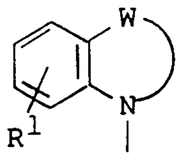 : 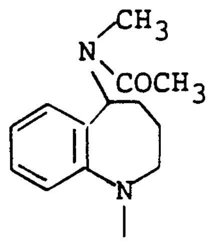

$R^2$: H

$R^3$: 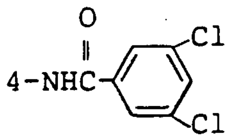

Crystalline form: Colorless prisms

Recrystallization solvent: Dioxane

Melting Point: 253 - 254°C

Form: Free

Example 569

Structure

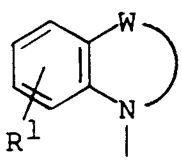 : 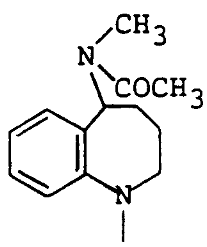

$R^2$: H

$R^3$: 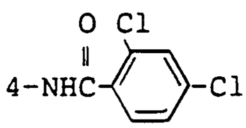

Crystalline form: Colorless prisms

Recrystallization solvent: Ethanol

Melting Point: 248.5 - 249.5°C

Form: Free

Example 570

Structure

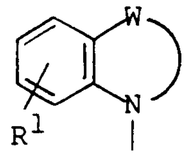 : 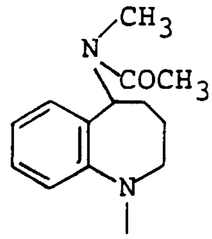

$R^2$: H

$R^3$: 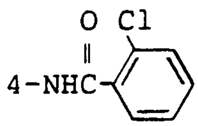

Crystalline form: Colorless needles

Recrystallization solvent: Ethanol

Melting Point: 266.5 - 267.5°C

Form: Free

Example 571

Structure

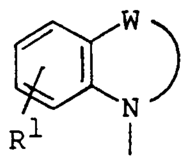 : 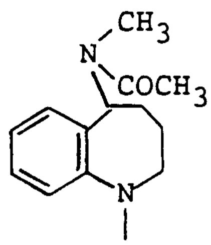

$R^2$: H

$R^3$: 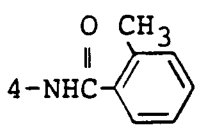

Crystalline form: Colorless needles

Recrystallization solvent: Ethanol

Melting Point: 252 - 253°C

Form: Free

Example 572

Structure

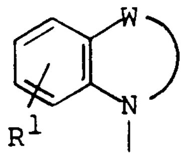 : 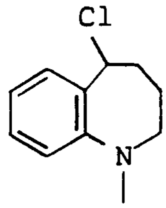 $R^2$: H

$R^3$: 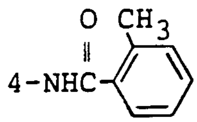

Crystalline form: Light yellow powder

NMR analysis: 89)

Form: Free

Example 573

Structure

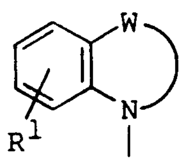 : 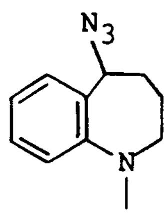 $R^2$: H

$R^3$: 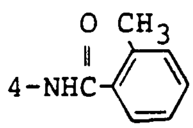

Crystalline form: Light brown powder

NMR analysis: 90)

Form: Free

Example 574

Structure

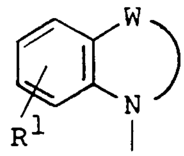 : 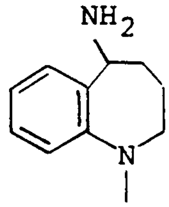

$R^2$: H

$R^3$: 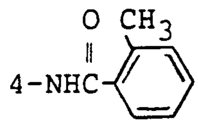

Crystalline form: White powder

Recrystallization solvent: Diethyl ether

Melting Point: 198.5 - 199.5°C

Form: Free

Example 575

Structure

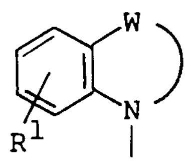 : 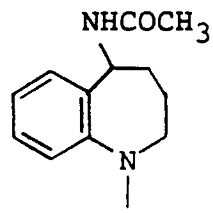

$R^2$: H

$R^3$: 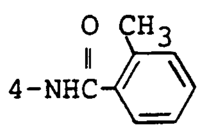

Crystalline form: Colorless needles

Recrystallization solvent: Ethanol

Melting Point: 297 - 299°C

Form: Free

Example 576

Structure

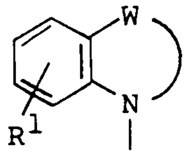 : 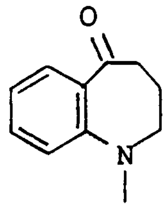

$R^2$: 2-Cl

$R^3$: 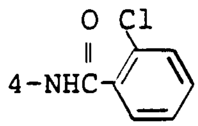

Crystalline form: Colorless amorphous

NMR analysis: 91)

Form: Free

Example 577

Structure

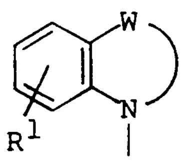 : 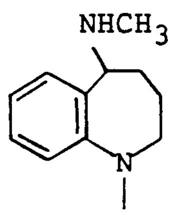

$R^2$: 2-Cl

$R^3$: 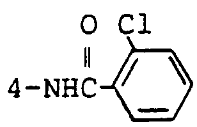

Crystalline form: White powder

Recrystallization solvent: Ethanol/petroleum ether

Melting Point: 202 - 203°C

Form: Free

Example 578

Structure

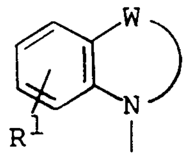 : 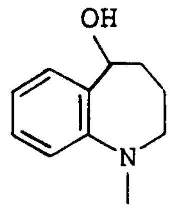

$R^2$: H

$R^3$: 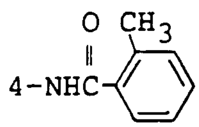

Crystalline form: Colorless amorphous

NMR analysis: 92)

Form: Free

Example 579

Structure

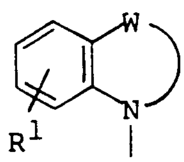 : 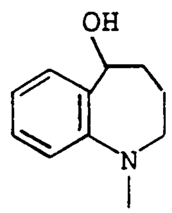

$R^2$: H

$R^3$: 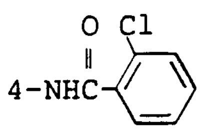

Crystalline form: White powder

Recrystallization solvent: Ethyl acetate/n-hexane

Melting Point: 232 - 233°C

Form: Free

Example 580

Structure

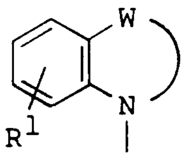 : 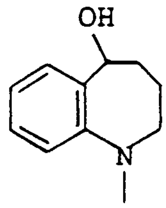

$R^2$: H

$R^3$: 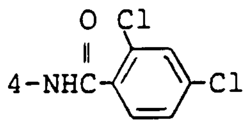

Crystalline form: Colorless amorphous

NMR analysis: 93)

Form: Free

Example 581

Structure

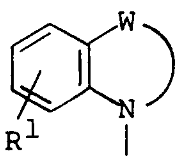 : 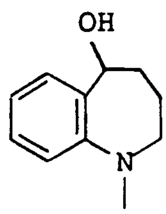

$R^2$: H

$R^3$: 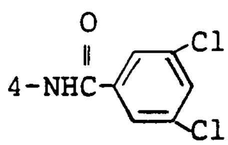

Crystalline form: White powder

Recrystallization solvent: Ethyl acetate/n-hexane

Melting Point: 256.5 - 257°C

Form: Free

Example 582

Structure

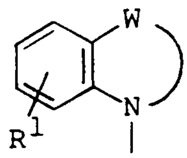

:

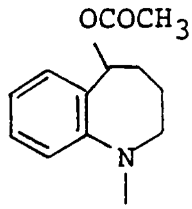

$R^2$: H

$R^3$: 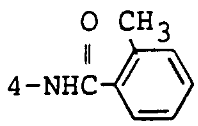

Crystalline form: White powder

Recrystallization solvent: Ethyl acetate/n-hexane

Melting Point: 193 - 194°C

Form: Free

Example 583

Structure

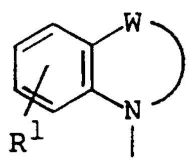

:

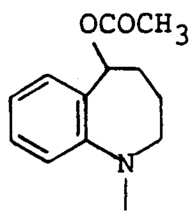

$R^2$: H

$R^3$: 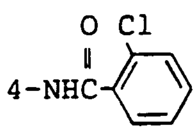

Crystalline form: White powder

Recrystallization solvent: Ethyl acetate/n-hexane

Melting Point: 227 - 230°C

Form: Free

Example 584

Structure

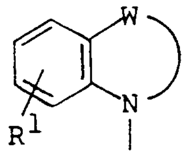

:

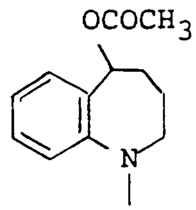

$R^2$: H

$R^3$: 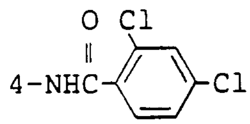

Crystalline form: White powder

Recrystallization solvent: Ethyl acetate/n-hexane

Melting Point: 199.5 - 202°C

Form: Free

Example 585

Structure

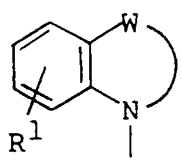

:

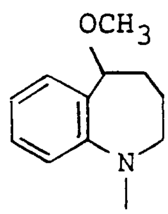

$R^2$: H

$R^3$: 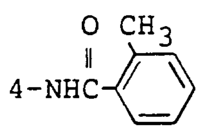

Crystalline form: White powder

Recrystallization solvent: Ethyl acetate/n-hexane

Melting Point: 219 - 220°C

Form: Free

Example 586

Structure

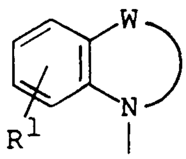
:
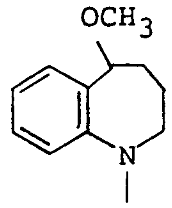

$R^2$: H

$R^3$:
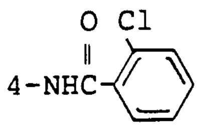

Crystalline form: White powder

Recrystallization solvent: Ethyl acetate/n-hexane

Melting Point: 190 - 191.5°C

Form: Free

Example 587

Structure

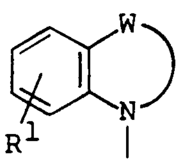
:
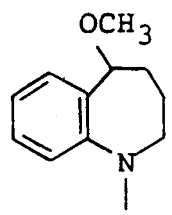

$R^2$: H

$R^3$:
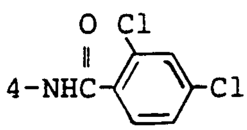

Crystalline form: White powder

Recrystallization solvent: Ethyl acetate/n-hexane

Melting Point: 184 - 185°C

Form: Free

Example 588

Structure

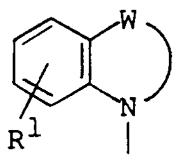 : 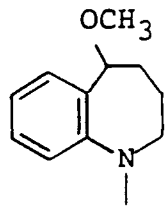

$R^2$: H

$R^3$: 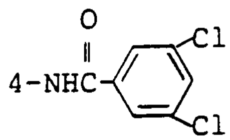

Crystalline form: White powder

Recrystallization solvent: Ethyl acetate/n-hexane

Melting Point: 223 - 224°C

Form: Free

Example 589

Structure

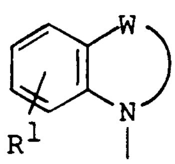 : 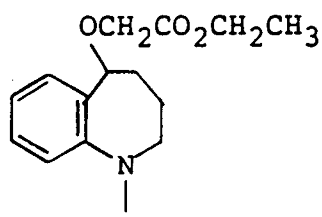

$R^2$: H

$R^3$: 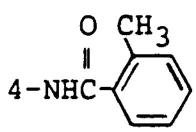

Crystalline form: White powder

Recrystallization solvent: Ethyl acetate/n-hexane

Melting Point: 178 - 181°C

Form: Free

Example 590

Structure

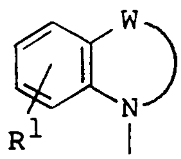

:

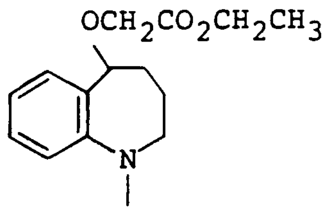

$R^2$: H

$R^3$: 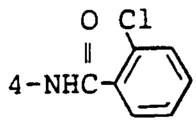

Crystalline form: Colorless needles

Recrystallization solvent: Ethyl acetate/n-hexane

Melting Point: 168 - 168.5°C

Form: Free

Example 591

Structure

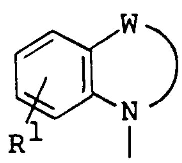

:

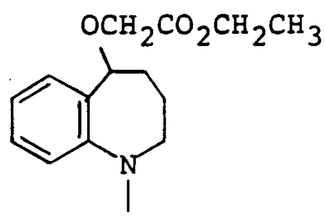

$R^2$: H

$R^3$: 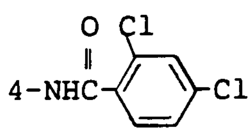

Crystalline form: Colorless amorphous

NMR analysis: 94)

Form: Free

Example 592

Structure 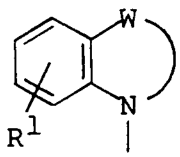 : 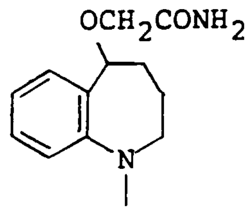 $R^2$: H

$R^3$: 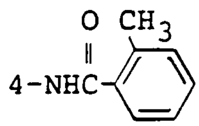

Crystalline form: Colorless amorphous

NMR analysis: 95)

Form: Free

Example 593

Structure 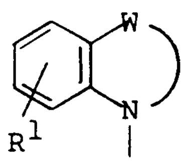 : 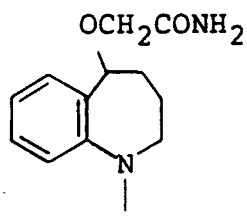 $R^2$: H

$R^3$: 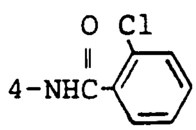

Crystalline form: White powder

Recrystallization solvent: Ethyl acetate/n-hexane

Melting Point: 196 - 197°C

Form: Free

Example 594

Structure

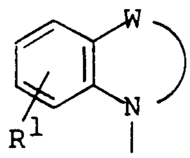 : 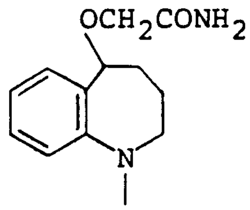

$R^2$: H

$R^3$: 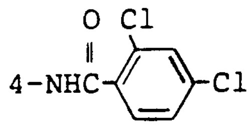

Crystalline form: Colorless amorphous

NMR analysis: 96)

Form: Free

Example 595

Structure

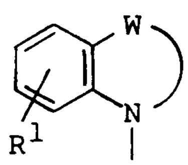 : 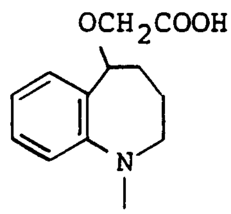

$R^2$: H

$R^3$: 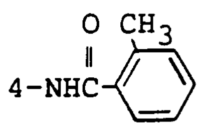

Crystalline form: White powder

Recrystallization solvent: Ethanol/water

Melting Point: 188 - 189°C

Form: Free

Example 596

Structure

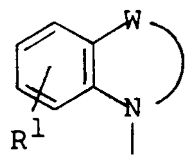 : 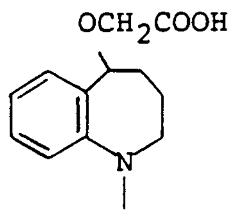

$R^2$: H

$R^3$: 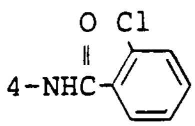

Crystalline form: Colorless amorphous

NMR analysis: 97)

Form: Free

Example 597

Structure

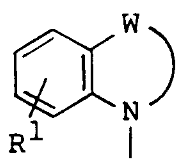 : 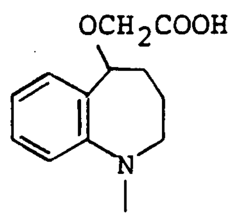

$R^2$: H

$R^3$: 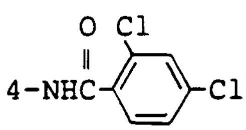

Crystalline form: Colorless amorphous

NMR analysis: 98)

Form: Free

Example 598

Structure

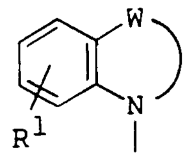 : 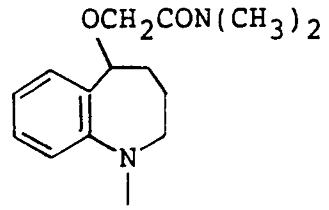 $R^2$: H

$R^3$: 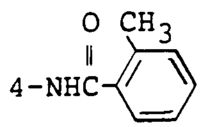

Crystalline form: Colorless prisms

Recrystallization solvent: Ethyl acetate/n-hexane

Melting Point: 203 - 204°C

Form: Free

Example 599

Structure

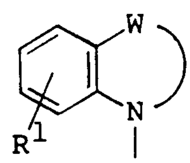 : 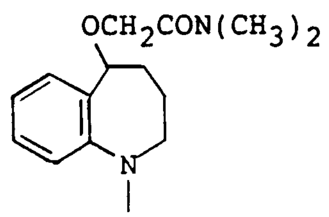 $R^2$: H

$R^3$: 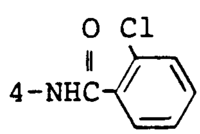

Crystalline form: White powder

Recrystallization solvent: Ethyl acetate/n-hexane

Melting Point: 196 - 197°C

Form: Free

Example 600

Structure

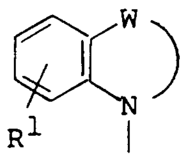 : 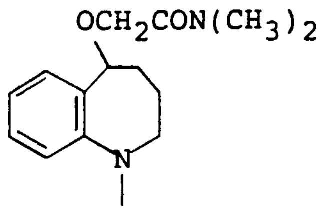 $R^2$: H

$R^3$: 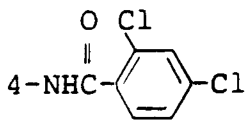

Crystalline form: Colorless amorphous

NMR analysis: 99)

Form: Free

Example 601

Structure

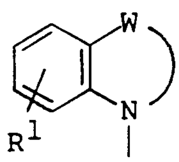 : 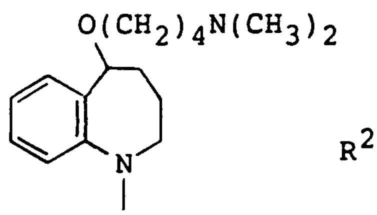 $R^2$: H

$R^3$: 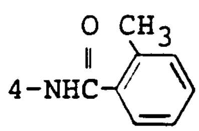

Crystalline form: Colorless amorphous

NMR analysis: 100)

Form: Free

Example 602

Structure

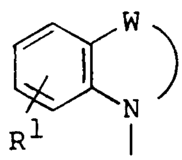 : 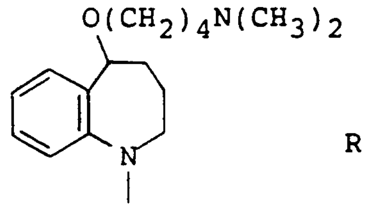

$R^2$: H

$R^3$: 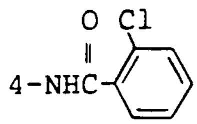

Crystalline form: Colorless amorphous

NMR analysis: 101)

Form: Free

Example 603

Structure

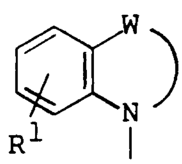 : 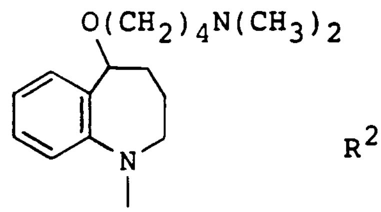

$R^2$: H

$R^3$: 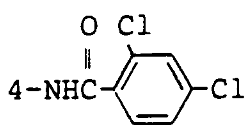

Crystalline form: Colorless amorphous

NMR analysis: 102)

Form: Free

Example 604

Structure

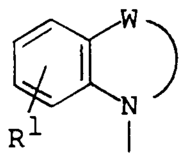 : 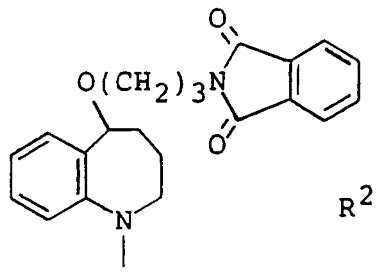 $R^2$: H

$R^3$: 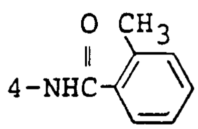

Crystalline form: Colorless amorphous

NMR analysis: 103)

Form: Free

Example 605

Structure

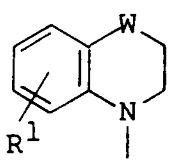 : 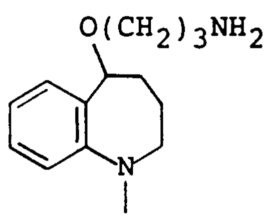 $R^2$: H

$R^3$: 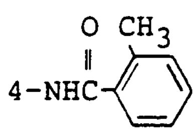

Crystalline form: Colorless amorphous

NMR analysis: 104)

Form: Free

Example 606

Structure

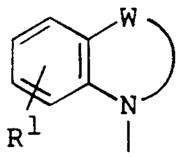

:

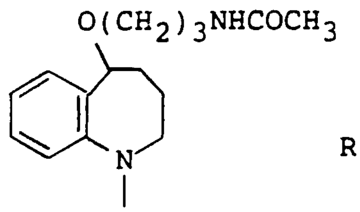

$R^2$: H

$R^3$: 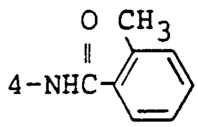

Crystalline form: Colorless amorphous

NMR analysis: 105)

Form: Free

Example 607

Structure

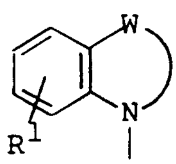

:

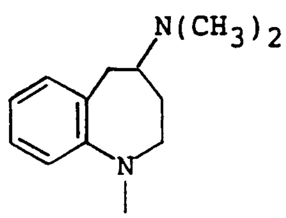

$R^2$: H

$R^3$: 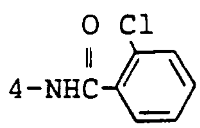

Crystalline form: Colorless needles

Recrystallization solvent: Ethanol

Melting Point: 169 - 171°C

Form: Free

Example 608

Structure

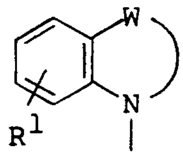 : 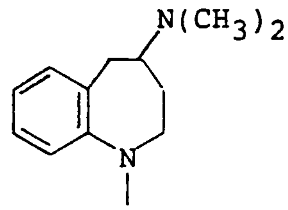

$R^2$: H

$R^3$: 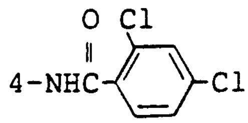

Crystalline form: Colorless needles

Recrystallization solvent: Ethanol/diethyl ether

Melting Point: 178 - 181°C

Form: Free

Example 609

Structure

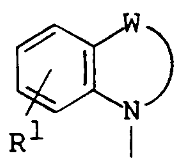 : 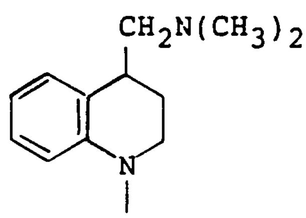

$R^2$: H

$R^3$: 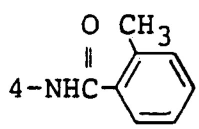

Crystalline form: White powder

Recrystallization solvent: Ethanol

Melting Point: 187 - 188°C

Form: Free

Example 610

Structure

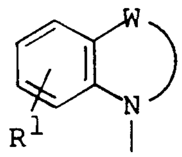 : 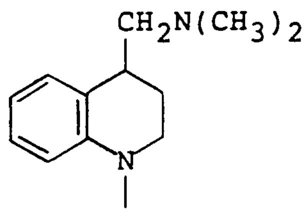

$R^2$: H

$R^3$: 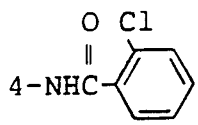

Crystalline form: White powder

Recrystallization solvent: Ethanol

Melting Point: 181 - 183°C

Form: Free

Example 611

Structure

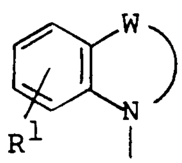 : 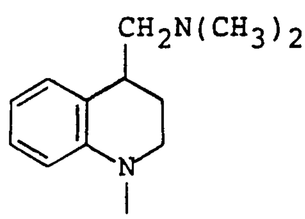

$R^2$: H

$R^3$: 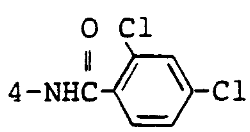

Crystalline form: White powder

Recrystallization solvent: Ethanol

Melting Point: 124 - 127°C

Form: Free

Example 612

Structure

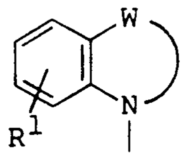 : 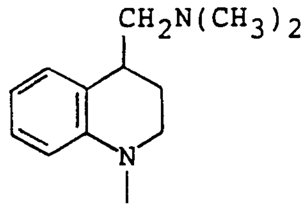

$R^2$: H

$R^3$: 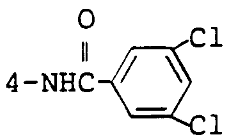

Crystalline form: White powder

Recrystallization solvent: Ethanol

Melting Point: 179 - 181°C

Form: Free

Example 613

Structure

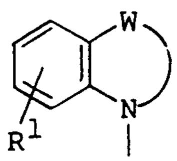 : 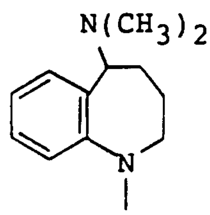

$R^2$: H

$R^3$: 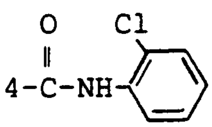

Crystalline form: White powder

Recrystallization solvent: Ethanol

Melting Point: 148 - 150°C

Form: Free

Example 614

Structure

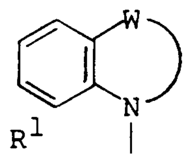 : 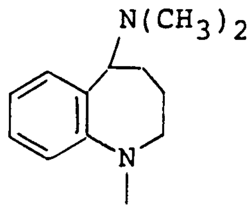

$R^2$: H

$R^3$: 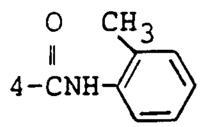

Crystalline form: Colorless amorphous

NMR analysis: 106)

Form: Free

Example 615

Structure

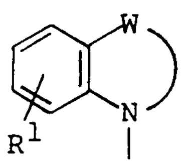 : 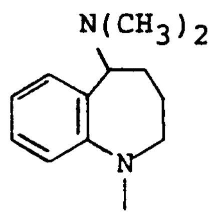

$R^2$: 2-Cl

$R^3$: 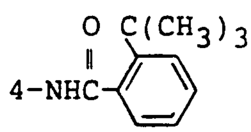

Crystalline form: White powder

Recrystallization solvent: Ethanol

Melting Point: 219 - 220°C

Form: Free

Example 616

Structure

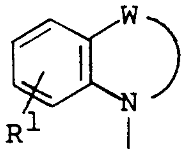 : 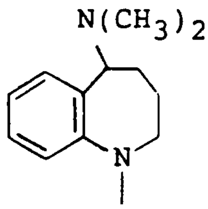

$R^2$: H

$R^3$: 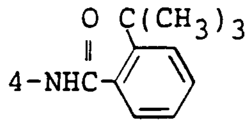

Crystalline form: White powder

Recrystallization solvent: Ethanol

Melting Point: 226 - 228°C

Form: Free

Example 617

Structure

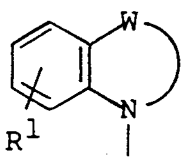 : 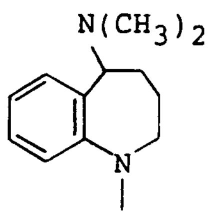

$R^2$: 3-$OCH_3$

$R^3$: 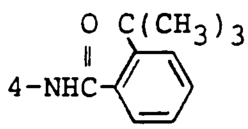

Crystalline form: Colorless amorphous

NMR analysis: 107)

Form: Free

Example 618

Structure

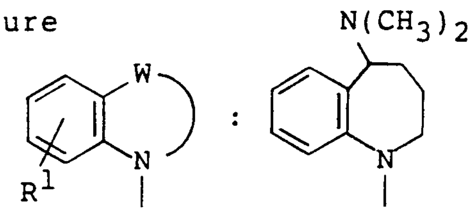

$R^2$: 3-$OCH_3$

$R^3$: 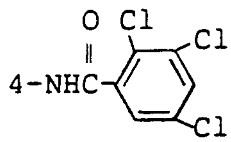

Crystalline form: Colorless amorphous

NMR analysis: 108)

Form: Free

Example 619

Structure

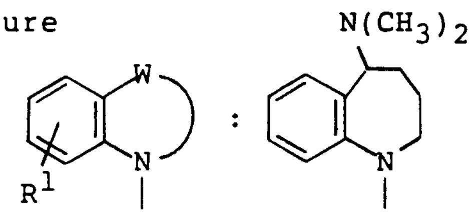

$R^2$: 3-$OCH_3$

$R^3$: 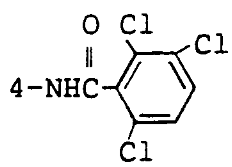

Crystalline form: Colorless amorphous

NMR analysis: 109)

Form: Free

Example 620

Structure

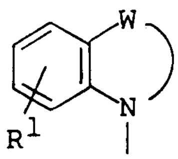 : 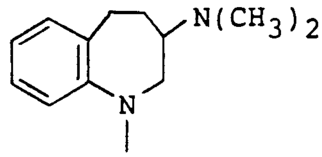 $R^2$: H

$R^3$: 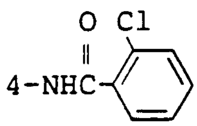

Crystalline form: Colorless amorphous

NMR analysis: 110)

Form: Free

Example 621

Structure

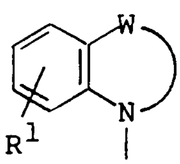 : 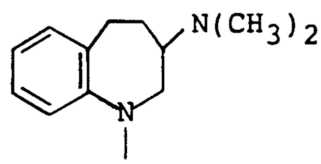 $R^2$: H

$R^3$: 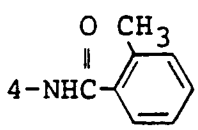

Crystalline form: Colorless amorphous

NMR analysis: 111)

Form: Free

Example 622

Structure

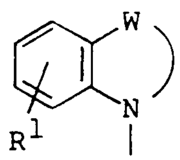 : 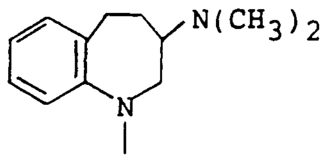 $R^2$: H

$R^3$: 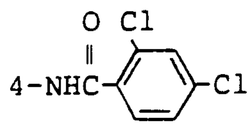

Crystalline form: Colorless amorphous

NMR analysis: 112)

Form: Free

Example 623

Structure

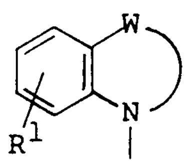 : 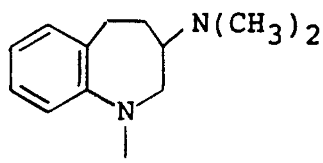 $R^2$: 3-$OCH_3$

$R^3$: 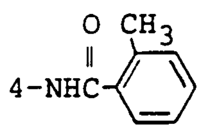

Crystalline form: Colorless amorphous

NMR analysis: 113)

Form: Free

Example 624

Structure

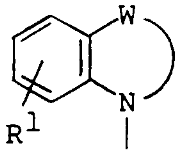 : 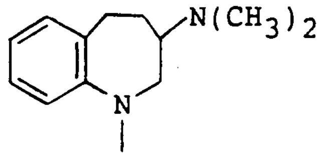 $R^2$: 3-$OCH_3$

$R^3$: 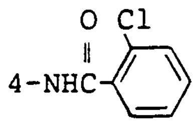

Crystalline form: Colorless amorphous

NMR analysis: 114)

Form: Free

Example 625

Structure

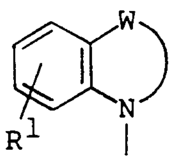 : 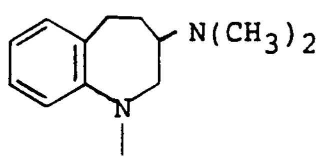 $R^2$: 3-$OCH_3$

$R^3$: 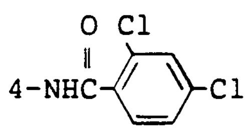

Crystalline form: Colorless amorphous

NMR analysis: 115)

Form: Free

Example 626

Structure

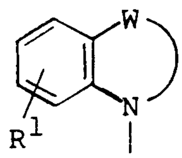 : 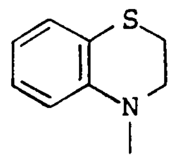

$R^2$: H

$R^3$: 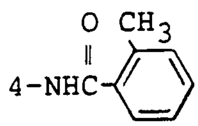

Crystalline form: White powder

Recrystallization solvent: Diethyl ether/dichloromethane

Melting Point: 183 - 184°C

Form: Free

Example 627

Structure

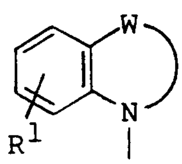 : 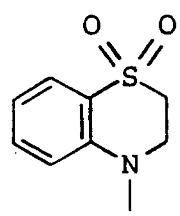

$R^2$: H

$R^3$: 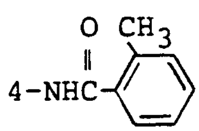

Crystalline form: White powder

Recrystallization solvent: Diethyl ether/dichloromethane

Melting Point: 219 - 220°C

Form: Free

Example 628

Structure

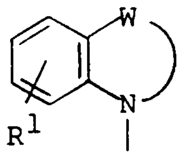 : 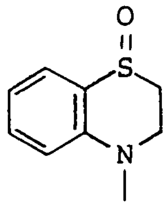

$R^2$: H

$R^3$: 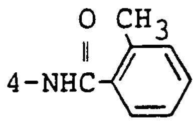

Crystalline form: White powder

Recrystallization solvent: Diethyl ether/dichloromethane

Melting Point: 240 - 241°C

Form: Free

Example 629

Structure

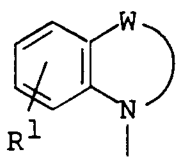 : 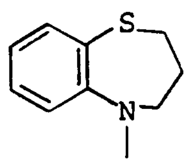

$R^2$: H

$R^3$: 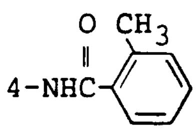

Crystalline form: White powder

Recrystallization solvent: Diethyl ether/dichloromethane

Melting Point: 205 - 206°C

Form: Free

Example 630

Structure

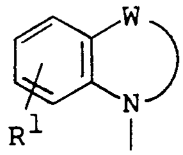 : 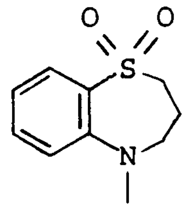 $R^2$: H

$R^3$: 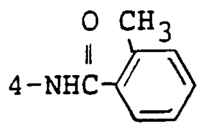

Crystalline form: White powder

Recrystallization solvent: Diethyl ether/dichloromethane

Melting Point: 238 - 239°C

Form: Free

Example 631

Structure

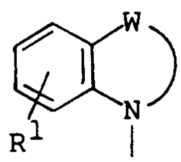 : 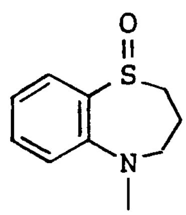 $R^2$: H

$R^3$: 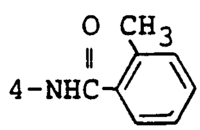

Crystalline form: White powder

Recrystallization solvent: Diethyl ether/dichloromethane

Melting Point: 233 - 234°C

Form: Free

Example 632

Structure

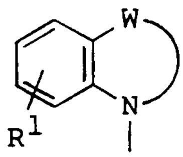 : 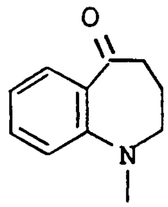

$R^2$: 2-Cl

$R^3$: 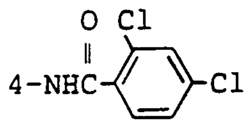

Crystalline form: Colorless amorphous

NMR analysis: 116)

Form: Free

Example 633

Structure

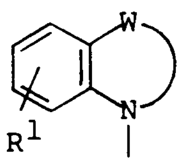 : 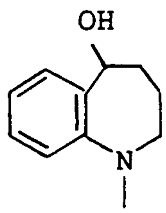

$R^2$: 2-Cl

$R^3$: 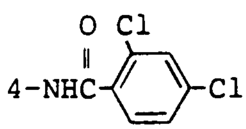

Crystalline form: White powder

Recrystallization solvent: Diethyl ether/dichloromethane

Melting Point: 259.5 - 260.5°C

Form: Free

80) $^1$H-NMR($CDCl_3$) δ ; 1.24-5.26 (18H, m), 6.39-7.59 (13H, m)

81) $^1$H-NMR($CDCl_3$) δ ; 1.70-2.10 (m, 2H), 2.15-2.60 (m, 12H), 3.56 (t, J=5.8 Hz, 1H), 3.65-3.95 (m, 4H) 4.05-4.25 (m, 1H), 6.64 (d, J=7.7 Hz, 1H), 6.85-7.50 (m, 9H), 8.11 (brs, 1H), 8.42 (d, J=8.8 Hz, 1H)

82) $^1$H-NMR($CDCl_3$) δ ; 2.00-2.90 (m, 3H), 2.49 (s, 3H), 3.70-3.90 (m, 1H), 4.00-4.20 (m, 1H), 4.80-5.00 (m, 1H), 6.89 (d, J=6.3 Hz, 1H), 6.95-7.65 (m, 11H), 7.70 (brs, 1H)

83) $^1$H-NMR($CDCl_3$) δ ; 1.95-2.90 (m, 2H), 2.48 (s, 3H), 2.55 (s, 3H), 3.77 (t, J=5.1 Hz, 1H), 3.92 (t, J=6.7 Hz, 2H), 6.72 (d, J=8.0 Hz, 1H), 6.90-7.15 (m, 2H), 7.15-7.70 (m, 9H), 7.81 (brs, 1H)

84) $^1$H-NMR($CDCl_3$) δ ; 2.11 (s, 3H), 2.20-2.40 (m, 2H), 2.50 (s, 3H), 3.80-4.10 (m, 1H), 4.12-4.25 (m, 1H), 6.03 (t, J=4.3 Hz, 1H), 6.80-7.65 (m, 12H), 7.80 (brs, 1H)

85) $^1$H-NMR($CDCl_3$) δ ; 1.80-2.40 (m, 5H), 2.45 (s, 3H), 2.81 (s, 3H), 3.55-3.82 (m, 1H), 4.15-4.40 (m, 1H), 5.90-6.10 (m, 1H), 6.80-7.80 (m, 12H), 8.67 (brs, 1H)

86) $^1$H-NMR($CDCl_3$) δ ; 1.95-2.35 (2H, m), 2.75-3.0 (2H, m), 3.0-5.4 (2H, m), 6.55-7.95 (11H, m), 8.09 (1H, s)

87) $^1$H-NMR(DMSO-$d_6$) δ ; 1.85-2.2 (2H, m), 2.7-2.95 (2H, m), 3.5-5.0 (2H, m), 6.8-7.8 (12H, m), 10.60 (1H, s)

88) $^1$H-NMR($CDCl_3$) δ ; 0.8-1.1 (3H, m), 1.2-2.35 (6H, m), 2.35-5.25 (6H, m), 6.63 (1H, d, J=7.7 Hz), 6.8-7.6 (9H, m), 7.67 (1H, d, J=8.2 Hz), 7.9-8.15 (1H, m)

89) $^1$H-NMR($CDCl_3$) δ ; 1.7-2.9 (7H, m), 4.5-6.5 (3H, m), 6.55-6.75 (1H, m), 6.85-7.6 (12H, m)

90) $^1$H-NMR($CDCl_3$) δ ; 1.65-3.1 (7H, m), 4.7-6.6 (3H, m), 6.6-6.8 (1H, m), 6.85-7.65 (12H, m)

91) $^1$H-NMR($CDCl_3$) δ ; 1.8-2.4 (2H, m), 2.86 (2H, t, J=6 Hz), 3.1-5.15 (2H, m), 6.85-7.5 (8H, m), 7.5-7.85 (3H, m), 8.19 (1H, s)

92) $^1$H-NMR($CDCl_3$) δ ; 1.46-2.28 (4H, m), 2.37 (3H, s), 2.58-2.90 (1H, m), 4.57-5.10 (2H, m), 6.59 (1H, d, J=7.6 Hz), 6.91-7.52 (11H, m), 7.62 (1H, d, J=7.6 Hz), 8.10-8.40 (1H, m)

93) $^1$H-NMR($CDCl_3$) δ ; 1.45-1.91 (2H, m), 1.91-2.65 (2H, m), 2.65-2.90 (1H, m), 4.63-5.22 (2H, m), 6.63 (1H, d, J=7.4 Hz), 7.34-8.03 (11H, m), 10.16-10.44 (1H, m)

94) $^1$H-NMR($CDCl_3$) δ ; 1.08-1.47 (3H, m), 1.50-1.97 (2H, m), 1.97-2.48 (2H, m), 2.65-3.02 (1H, m), 4.00-4.43 (4H, m), 4.52-5.15 (2H, m), 6.50-6.79 (1H, m), 6.90-7.70 (10H, m), 8.26-8.60 (1H, m)

95) $^1$H-NMR($CDCl_3$) δ ; 1.56-2.67 (4H, m), 2.46 (3H, s), 2.67-3.03 (1H, m), 3.82-4.32 (2H, m), 4.45-5.15 (2H, m), 5.43-5.83 (1H, m), 6.20-6.45 (1H, m), 6.50-6.86 (2H, m), 6.86-7.70 (10H, m), 7.76-8.10 (1H, m)

96) $^1$H-NMR($CDCl_3$) δ ; 1.52-1.90 (2H, m), 1.90-2.54 (2H, m), 2.67-3.05 (1H, m), 3.74-4.32 (2H, m), 4.38-5.17 (2H, m), 5.52-5.98 (1H, brs), 6.20-6.48 (1H, brs), 6.55-6.84 (1H, m), 6.89-7.55 (9H, m), 7.55-7.77 (1H, m), 8.15-8.86 (1H, brs)

97) $^1$H-NMR(DMSO-$d_6$) δ ; 1.26-2.49 (4H, m), 2.57-2.93 (1H, m), 4.07-4.43 (2H, m), 4.44-4.98 (2H, m), 6.62-6.87 (1H, m), 6.92-7.80 (11H, m), 10.57 (1H, s), 12.74 (1H, s)

98) $^1$H-NMR($CDCl_3$) δ ; 1.52-1.89 (2H, m), 1.89-2.56 (2H, m), 2.65-3.02 (1H, m), 3.90-4.40 (2H, m), 4.40-5.07 (2H, m), 6.58-6.78 (1H, m), 6.90-7.70 (10H, m), 8.57-8.81 (1H, brs)

99) $^1$H-NMR($CDCl_3$) δ ; 1.49-1.89 (2H, m), 1.89-2.60 (2H, m), 2.63-3.23 (7H, m), 4.04-4.49 (2H, m), 4.52-5.21 (2H, m), 6.52-6.80 (1H, m), 6.89-7.84 (10H, m), 8.08-8.52 (1H, m)

100) $^1$H-NMR($CDCl_3$) δ ; 1.41-1.86 (6H, m), 1.86-2.53 (4H, m), 2.25 (3H, s), 2.29 (3H, s), 2.43 (3H, s), 2.60-2.97 (1H, m), 3.36-3.77 (2H, m), 4.40-5.10 (2H, m), 6.54-6.72 (1H, m), 6.88-7.67 (11H, m), 8.27-8.58

(1H, m)

101) $^1$H-NMR($CDCl_3$) δ ; 1.44-1.85 (6H, m), 1.85-2.61 (4H, m), 2.32 (3H, s), 2.35 (3H, s), 2.61-3.00 (1H, m), 3.33-3.76 (2H, m), 4.40-5.20 (2H, m), 6.57-6.75 (1H, m), 6.90 - 7.70 (11H, m), 8.50-8.93 (1H, m)

102) $^1$H-NMR($CDCl_3$) δ ; 1.49-2.04 (6H, m), 2.10-3.02 (5H, m), 2.47 (6H, s), 3.40-3.88 (2H, m), 4.30-5.17 (2H, m), 6.59-6.78 (1H, m), 6.93-7.76 (10H, m), 8.75-9.40 (1H, m)

103) $^1$H-NMR($CDCl_3$) δ ; 1.47-2.47 (6H, m), 2.44 (3H, s), 2.62-3.03 (1H, m), 3.47-4.03 (4H, m), 4.48-5.17 (2H, m), 6.51-6.74 (1H, m), 6.87-7.62 (11H, m), 7.62-7.77 (2H, m), 7.77-8.03 (3H, m)

104) $^1$H-NMR($CDCl_3$) δ ; 1.42-2.32 (6H, m), 2.44 (3H, s), 2.57-2.97 (1H, m), 3.12-3.83 (4H, m), 4.39-5.13 (2H, m), 6.50-6.71 (1H, m), 6.90-7.73 (12H, m)

105) $^1$H-NMR($CDCl_3$) δ ; 1.50-2.63 (9H, m), 2.47 (3H, s), 2.66-3.07 (1H, m), 3.10-3.88 (4H, m), 4.40-5.17 (2H, m), 5.87-6.23 (1H, brs), 6.60-6.79 (1H, m), 6.94-7.60 (11H, m), 7.67 (1H, s)

106) $^1$H-NMR($CDCl_3$) δ ; 1.20-2.53 (13H, m), 2.63-2.82, 3.00-3.13, 3.50-3.67, 4.05-4.23 (total 3H, m), 6.55 - 8.00 (13H, m)

107) $^1$H-NMR($CDCl_3$) δ ; 1.41 (9H, s), 1.20-2.55 (10H, m), 3.42-4.20 (5.8H, m), 5.00-5.20 (0.2H, m), 6.60-7.67 (10H, m), 7.99 (1H, brs), 8.26 (1H, d, J=8.4 Hz)

108) $^1H$-NMR($CDCl_3$) δ ; 1.2-3.0 (10H, m), 3.0-5.2 (6H, m), 6.5-7.7 (8H, m), 8.22 (1H, d, J=8.4 Hz), 8.36 (1H, s)

109) $^1H$-NMR($CDCl_3$) δ ; 1.2-3.0 (10H, m), 3.0-5.2(6H, m), 6.3-7.7 (10H, m)

110) $^1H$-NMR($CDCl_3$) δ ; 1.5-1.7 (1H, m), 2.2-2.7 (2H, m), 2.40 (6H, s), 2.7-3.0 (3H, m), 5.1-5.3 (1H, m), 6.67 (1H, d, J=7.7 Hz), 6.9-7.5 (10H, m), 7.69 (1H, d, J=6 Hz), 8.06 (1H, s)

111) $^1H$-NMR($CDCl_3$) δ ; 1.4-1.7 (1H, m), 2.1-2.7 (2H, m), 2.40 (6H, s), 2.44 (3H, s), 2.7-3.0 (3H, m), 5.1-5.3 (1H, m), 6.68 (1H, d, J=7.8 Hz), 6.9-7.5 (11H, m), 7.66 (1H, s)

112) $^1H$-NMR($CDCl_3$) δ ; 1.5-1.8 (1H, m), 2.1-2.7 (2H, m), 2.38 (6H, s), 2.7-3.0 (3H, m), 5.1-5.3 (1H, m), 6.66 (1H, d, J=7.7 Hz), 6.9-7.0 (9H, m), 7.57 (1H, d, J=8.3 Hz), 8.42 (1H, s)

113) $^1H$-NMR($CDCl_3$) δ ; 1.5-1.8 (1H, m), 2.1-2.7 (2H, m), 2.41 (6H, s), 2.48 (3H, s), 2.7-3.0 (3H, m), 3.68 (3H, s), 5.2-5.4 (1H, m), 6.6-6.8 (2H, m), 6.9-7.5 (8H, m), 8.09 (1H, s), 8.26 (1H, d, J=8.1 Hz)

114) $^1H$-NMR($CDCl_3$) δ ; 1.5-1.7 (1H, m), 2.1-2.3 (1H, m), 2.41 (6H, s), 2.4-2.6 (1H, m), 2.8-3.0 (3H, m), 3.71 (3H, s), 5.2-5.4 (1H, m), 6.6-6.8 (2H, m), 6.9-7.5 (7H, m), 7.7-7.8 (1H, m), 8.27 (1H, d, J=8.4 Hz), 8.57 (1H, s)

115) $^1$H-NMR($CDCl_3$) δ ; 1.5-1.7 (1H, m), 2.1-2.7 (2H, m), 2.41 (6H, s), 2.7-3.0 (3H, m), 3.71 (3H, s), 5.2-5.4 (1H, m), 6.6-7.6 (8H, m), 7.70 (1H, d, J=8.3 Hz), 8.24 (1H, d, J=8.5 Hz), 8.59 (1H, s)

116) $^1$H-NMR($CDCl_3$) δ ; 1.8-2.3 (3H, m), 2.7-2.9 (2H, m), 3.5-3.7 (1H, m), 6.8-8.0 (10H, m), 8.7-9.1 (1H, br)

Example 634

To a mixture of 5-oxo-1-[4-(3,5-dichlorobenzoylamino)benzoyl]-2,3,4,5-tetrahydro-1H-benzazepine (4 g) and pyridine (50 ml) is added hydroxylamine hydrochloride (1.84 g) and the mixture is refluxed for 2.5 hours. The reaction solution is concentrated and water is added to the resulting residue. The precipitated crystal is collected by filtration, and recrystallized from dioxane/water to give 5-hydroxyimino-1-[4-(3,5-dichlorobenzoylamino)benzoyl]-2,3,4,5-tetrahydro-1H-benzazepine (2 g) as white powder, m.p. 272 - 273°C.

Example 635

5-Chloro-1-[4-(2-methylbenzoylamino)benzoyl]-2,3,4,5-tetrahydro-1H-benzazepine (0.8 g) is dissolved in dimethylformamide and thereto is added sodium azide (0.18 g) at room temperature. The mixture is stirred at room temperature overnight, and further reacted with heating at 50°C for 5 hours. Water is added to the reaction mixture and the precipitated crystal is collected by filtration to give 5-azido-1-[4-(2-methylbenzoylamino)benzoyl]-2,3,4,5-tetrahydro-1H-benzazepine (0.68 g) as light brown powder.

$^1$H-NMR($CDCl_3$) δ ; 1.65-3.1 (8H, m), 4.7-6.6 (3H, m), 6.6-6.8 (1H, m), 6.85-7.65 (12H, m)

Example 636

5-Azido-1-[4-(2-methylbenzoylamino)benzoyl]-2,3,4,5-tetrahydro-1H-benzazepine (0.63 g) is dissolved in ethanol and thereto is added 10 % Pd-C (0.1 g). The mixture is subjected to catalytic hydrogenation at room temperature under 1 atm. of hydrogen. Pd-C is removed by filtration and the filtrate is evaporated. The resulting residue is purified by silica gel column chromatography (eluent; dichloromethane : methanol = 50 : 1), and recrystallized from diethyl ether to give 5-amino-1-[4-(2-methylbenzoylamino)benzoyl]-2,3,4,5-tetrahydro-1H-benzazepine (0.34 g) as white powder, m.p. 198.5 - 199.5°C.

Example 637

To 5-hydroxy-1-[4-(2-methylbenzoylamino)benzoyl]-2,3,4,5-tetrahydro-1H-benzazepine (0.58 g) are added acetic anhydride (8.0 ml) and pyridine (2.0 ml). The mixture is stirred at room temperature for 1 hour. Water is added to the reaction mixture and the precipitated crystal is collected by filtration, and recrystallized from ethyl acetate/n-hexane to give 5-acetyloxy-1-[4-(2-methylbenzoylamino)benzoyl]-2,3,4,5-tetrahydro-1H-benzazepine (0.56 g) as white powder. m.p. 193 - 194°C.

Example 638

5-Ethoxycarbonylmethoxy-1-[4-(2-methylbenzoylamino)benzoyl]-2,3,4,5-tetrahydro-1H-benzazepine (1.00 g) is dissolved in methanol (35 ml) and thereto are added aqueous ammonia (20 ml) and ammonium chloride (0.50 g). The mixture is heated at 100°C for 3.5 hours in a sealed tube. After cooling, the reaction solution is concentrated under reduced pressure and acidified with hydrochloric acid, and extracted with dichloromethane. The extract is dried over magnesium sulfate and the solvent is distilled off. The resulting residue is purified by silica gel column chromatography

(eluent; dichloromethane : methanol = 15 : 1) to give 5-carbamoylmethoxy-1-[4-(2-methylbenzoylamino)benzoyl]-2,3,4,5-tetrahydro-1H-benzazepine (0.68 g) as colorless amorphous.

$^1$H-NMR($CDCl_3$) δ ; 1.56-2.67 (4H, m), 2.46 (3H, s), 2.67-3.03 (1H, m), 3.82-4.32 (2H, m), 4.45-5.15 (2H, m), 5.43-5.83 (1H, m), 6.20-6.45 (1H, m), 6.50-6.86 (2H, m), 6.86-7.70 (10H, m), 7.76-8.10 (1H, m)

Using the suitable starting materials, the compounds of the above Examples 593 and 594 are obtained in the same manner as in Example 638.

Example 639

5-Ethoxycarbonylmethoxy-1-[4-(2,4-dichlorobenzoylamino)benzoyl]-2,3,4,5-tetrahydro-1H-benzazepine (0.94 g) is dissolved in ethanol (100 ml) and thereto is added 5N aqueous sodium hydroxide solution (0.50 ml). The mixture is stirred at room temperature for 2 hours. The reaction solution is concentrated under reduced pressure and to the resulting residue is added diluted hydrochloric acid and then extracted with dichloromethane. The extract is dried over magnesium sulfate and the solvent is distilled off. The resulting residue is washed with n-hexane and collected by filtration to give 5-carboxymethoxy-1-[4-(2,4-dichlorobenzoylamino)benzoyl]-2,3,4,5-tetrahydro-1H-benzazepine (0.79 g) as colorless amorphous.

$^1$H-NMR($CDCl_3$) δ ; 1.52-1.89 (2H, m); 1.89-2.56 (2H, m), 2.65-3.02 (1H, m), 3.90-4.40 (2H, m), 4.40-5.07 (2H, m), 6.58-6.78 (1H, m), 6.90-7.70 (10H, m), 8.57-8.81 (1H, brs)

Using the suitable starting materials, the compounds of the above Examples 595 and 596 are obtained in the same manner as in Example 639.

Example 640

5-Carboxymethoxy-1-[4-(2-methylbenzoylamino)benzoyl]-2,3,4,5-tetrahydro-1H-benzazepine (0.55 g) is dissolved in dimethylformamide (20 ml) and thereto are added dimethylamine hydrochloride (0.20 g) and diethyl chlorophosphate (0.33 g). To the mixture is added triethylamine (1.0 ml) under ice-cooling, and the mixture is stirred under ice-cooling for 30 minutes, and at room temperature for more 2 hours. Water is added to the reaction solution and the precipitated crystal is collected by filtration and recrystallized from ethyl acetate/n-hexane to give 5-dimethylaminocarbonylmethoxy-1-[4-(2-methylbenzoylamino) benzoyl]-2,3,4,5-tetrahydro-1H-benzazepine (0.50 g) as colorless prisms, m.p. 203 - 204°C.

Using the suitable starting materials, the compounds of the above Examples 599 and 600 are obtained in the same manner as in Example 640.

Example 641

5-[3-(Phthalimid-1-yl)propoxy]-1-[4-(2-methylbenzoylamino)benzoyl]-2,3,4,5-tetrahydro-1H-benzazepine (1.26 g) is dissolved in ethanol (100 ml) and thereto is added hydrazine hydrate (1.0 ml). The mixture is refluxed with stirring for 1 hour. The reaction solution is evaporated under reduced pressure and to the resulting residue is added dichloromethane. The insoluble materials are removed by filtration. The filtrate is purified by silica gel column chromatography (eluent; dichloromethane : methanol : aqueous ammonia = 70 : 10 : 1) to give 5-(3-aminopropoxy)-1-[4-(2-methylbenzoylamino)benzoyl]-2,3,4,5-tetrahydro-1H-benzazepine (0.70 g) as colorless amorphous.

$^1$H-NMR($CDCl_3$) δ ; 1.42-2.32 (6H, m), 2.44 (3H, s), 2.57-2.97 (1H, m), 3.12-3.83 (4H, m), 4.39-5.13 (2H, m), 6.50-6.71 (1H, m), 6.90-7.73 (12H, m)

Example 642

A solution of 5-dimethylamino-1-[3-methoxy-4-(2-methylbenzoylamino)benzoyl]-2,3,4,5-tetrahydro-1H- benzazepine (0.50 g) in dichloromethane (30 ml) is added dropwise to a solution of 1M boron tribromide in dichloromethane (5.46 ml) at -45°C. After completion of the dropping, the mixture is stirred for 1 day while the temperature of the reaction mixture is gradually raised to room temperature. To the reaction solution is added water and the mixture is neutralized with sodium hydrogen carbonate, and extracted with dichloromethane. The extract is washed with saturated saline solution and dried over magnesium sulfate. The solvent is distilled off and the resulting residue is purified by silica gel column chromatography (eluent; chloroform : methanol = 500 : 1), and recrystallized from methanol/diethyl ether to give 5-dimethylamino-1-[3-hydroxy-4-(2-methylbenzoylamino)benzoyl]-2,3,4,5-tetrahydro-1H-benzazepine (0.33 g) as white powder, m.p. 201.5 - 202.5°C.

Using the suitable starting materials, the compounds of the above Examples 10, 32, 343, 356, 535, 555 and 556 are obtained in the same manner as in Example 642.

Example 643

To a solution of 4-[4-(2-methylbenzoylamino)benzoyl]-3,4-dihydro-2H-1,4-benzazepine (0.5 g) in dichloromethane (10 ml) is added m-chloroperbenzoic acid (0.58 g) under ice-cooling, and the mixture is stirred at room temperature for 6 hours. The above reaction solution is poured into an aqueous solution of sodium carbonate (0.6 g) in water (10 ml) and the mixture is extracted with dichloromethane. The extract is washed with water, and dried over magnesium sulfate. The solvent is distilled off under reduced pressure and the resulting residue is purified by silica gel column chromatography (eluent; dichloromethane : methanol = 100 : 1), and recrystallized from diethyl ether/dichloromethane to give 4-[4-(2-methylbenzoylamino)benzoyl]-3,4-dihydro-2H-1,4-benzothiazine-1,1-dioxide (0.49 g) as white powder, m.p. 219 - 220°C.

Using the suitable starting materials, the compound of the above Example 630 is obtained in the same manner as in Example 643.

Example 644

To a suspension of 4-[4-(2-methylbenzoylamino)benzoyl]-3,4-dihydro-2H-1,4-benzothiazine (0.5 g) in methanol (15 ml) is added an aqueous solution of sodium metaperiodate (0.28 g) in water (2.5 ml) and the mixture is stirred at room temperature for 72 hours. Water is added to the reaction solution and extracted with dichloromethane. The extract is dried over magnesium sulfate and the solvent is distilled off under reduced pressure. The resulting residue is purified by silica gel column chromatography (eluent; dichloromethane : methanol = 100 : 1), and recrystallized from dichloromethane/diethyl ether to give 4-[4-(2-methylbenzoylamino)benzoyl]-3,4-dihydro-2H-1,4-benzothiazin-1-oxide (0.34 g) as white powder, m.p. 240 - 241°C.

Using the suitable starting materials, the compound of the above Example 631 is obtained in the same manner as in Example 644.

Example 645

5-Hydroxy-1-[4-(2-methylbenzoylamino)benzoyl]-2,3,4,5-tetrahydro-1H-benzazepine (3.57 g) is dissolved in dichloromethane (30 ml) and pyridine (1.1 ml), and thereto is added dropwise methanesulfonyl chloride (0.9 ml) in small portions at 0°C. Then, the mixture is stirred at room temperature for 3 days. The solvent is distilled off and the resulting residue is poured into ice-water. The precipitated crystal is collected by filtration, washed with water, and dried to give 5-chloro-1-[4-(2-methylbenzoylamino)benzoyl]-2,3,4,5-tetrahydro-1H-benzazepine (3.10 g) as light yellow powder.

$^1$H-NMR($CDCl_3$) δ ; 1.7-2.9 (8H, m), 4.5-6.5 (3H, m), 6.55-6.75 (1H, m), 6.85-7.6 (12H, m)

Example 646

5-Hydroxy-1-[4-(2-methylbenzoylamino)benzoyl]-2,3,4,5-tetrahydro-1H-benzazepine (2.69 g) is dissolved in dimethylformamide (30 ml) and thereto are added 60 % sodium hydride dispersion in mineral oil (0.44 g) and ethyl bromoacetate (1.00 ml) under ice-cooling, and the mixture is stirred at room temperature for 4 hours. The reaction solution is poured into an aqueous ammonium chloride solution under ice-cooling, and extracted with ethyl acetate. The extract is dried over magnesium sulfate and the solvent is distilled off. The resulting residue is purified by silica gel column chromatography (eluent; ethyl acetate : n-hexane = 1 : 2), and recrystallized from ethyl acetate/n-hexane to give 5-ethoxycarbonylmethoxy-1-[4-(2-methylbenzoylamino)benzoyl-2,3,4,5-tetrahydro-1H-benzazepine (2.10 g) as white powder, m.p. 178 - 181°C.

Using the suitable starting materials, the compounds of the above Examples 585 - 588 and 590 - 606 are obtained in the same manner as in Example 646.

Example 647

Using the suitable starting materials, the compounds of the above Examples 546 and 578 - 581 are obtained in the same manner as in Example 384.

Example 648

Using the suitable starting materials, the compounds of the above Examples 537 - 545, 547, 549 - 556, 561 - 564, 566, 568 - 571, 577, 601 - 603 and 607 - 625 are obtained in the same manner as in Example 388.

Example 649

Using the suitable starting materials, the compounds of the above Examples 549, 568 - 571, 575 and 606 are obtained in the same manner as in Example 389.

Example 650

Using the suitable starting materials, the compounds of the above Examples 537 - 545, 547, 549 - 556, 561 - 566, 568 - 571, 575, 577, 607, 608 and 613 - 625 are obtained in the same manner as in Example 390.

Example 651

Using the suitable starting materials, the compounds of the above Examples 601 - 603, 605 and 606 are obtained in the same manner as in Example 397.

Example 652

Using the suitable starting materials, the compound of the above Example 604 is obtained in the same manner as in Example 398.

Example 653

Using the suitable starting materials, the following compound is obtained in the same manner as in Examples 1, 382, 388 and 390.

5-Methylamino-1-[2-chloro-4-(2-methylbenzoylamino)benzoyl]-2,3,4,5-tetrahydro-1H-benzazepine, white powder, m.p. 184.5 - 185.5°C (recrystallized from ethanol)

Using the suitable starting materials, the compounds of the following Table 4 are obtained in the same manner

as in Examples 1 and 382.

Table 4

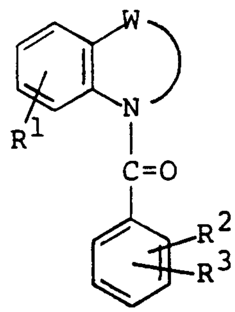

Example 654

Structure

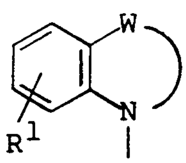 : 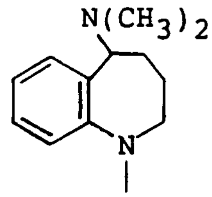

$R^2$: 2-OH

$R^3$: 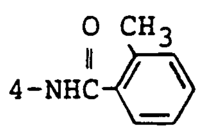

Crystalline form: White powder

Recrystallization solvent: Methanol/n-hexane

Melting Point: 193.5 - 196°C

Form: Free

Example 655

Structure

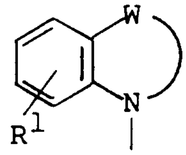 : 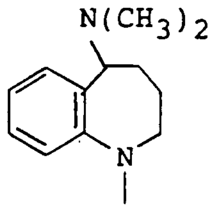

$R^2$: 2-OH

$R^3$: 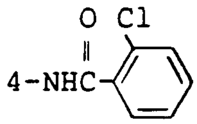

Crystalline form: White powder

Recrystallization solvent: Methanol/n-hexane

Melting Point: 195 - 198°C

Form: Free

Example 656

Structure

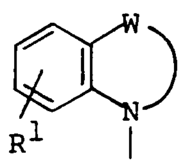 : 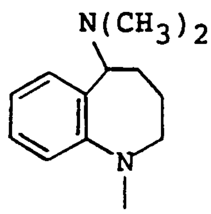

$R^2$: 2-$OC_2H_5$

$R^3$: 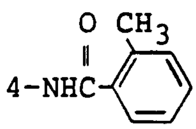

Crystalline form: White powder

Recrystallization solvent: Methanol

Melting Point: 230.5 - 231.5°C

Form: Free

Example 657

Structure

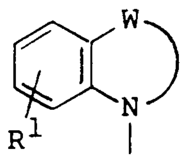 : 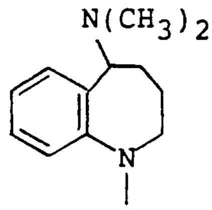

$R^2$: 2-$OC_2H_5$

$R^3$: 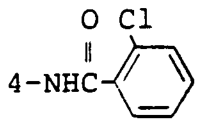

Crystalline form: White powder

Recrystallization solvent: Methanol/diethyl ether

Melting Point: 223 - 224.5°C

Form: Free

Example 658

Structure

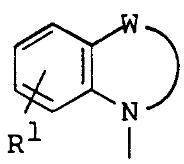 : 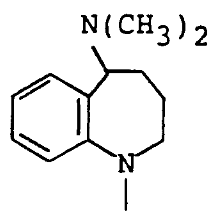

$R^2$: 2-$OC_2H_5$

$R^3$: 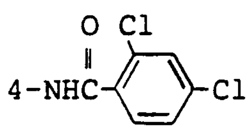

Crystalline form: White powder

Recrystallization solvent: Methanol/diethyl ether

Melting Point: 173 - 174°C

Form: Free

Example 659

Structure

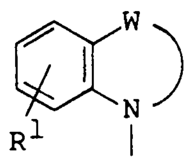 : 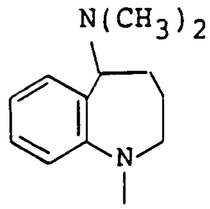

$R^2$: 3-$CH_3$

$R^3$: 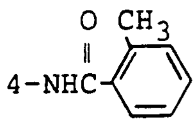

Crystalline form: White powder

Recrystallization solvent: Methanol/diethyl ether

Melting Point: 174 - 175°C

Form: Free

Example 660

Structure

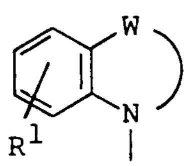 : 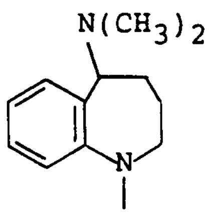

$R^2$: 3-$CH_3$

$R^3$: 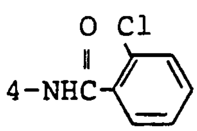

Crystalline form: White powder

Recrystallization solvent: Methanol/diethyl ether

Melting Point: 198 - 200°C

Form: Free

Example 661

Structure

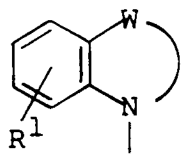 : 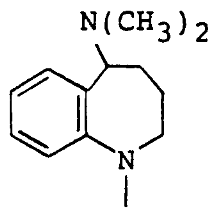

$R^2$: 3-$CH_3$

$R^3$: 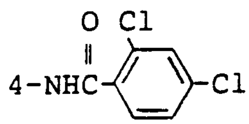

Crystalline form: White powder

Recrystallization solvent: Methanol/n-hexane

Melting Point: 149 - 150.5°C

Form: Free

Example 662

Structure

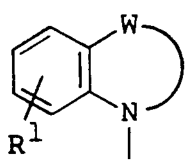 : 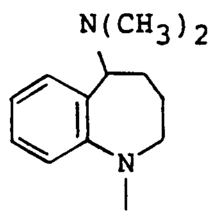

$R^2$: 2-$CH_3$

$R^3$: 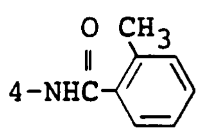

Crystalline form: White powder

Recrystallization solvent: Methanol/diethyl ether

Melting Point: 183 - 185°C

Form: Free

Example 663

Structure

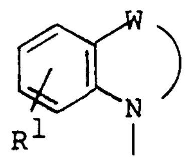 : 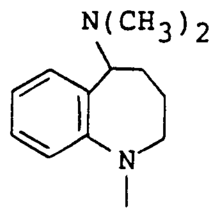

$R^2$: 2-$CH_3$

$R^3$: 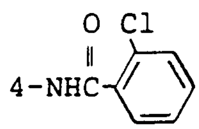

Crystalline form: White powder

Recrystallization solvent: Methanol/diethyl ether

Melting Point: 203 - 207°C

Form: Free

Example 664

Structure

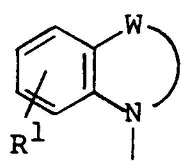 : 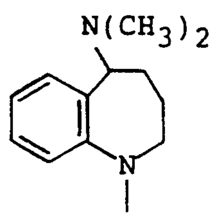

$R^2$: 2-$CH_3$

$R^3$: 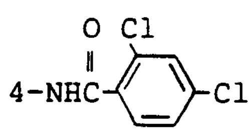

Crystalline form: White powder

Recrystallization solvent: Methanol/diethyl ether

Melting Point: 221 - 222°C

Form: Free

Example 665

Structure

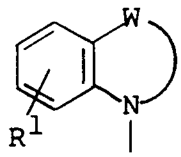 : 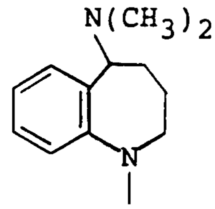

$R^2$: 2-F

$R^3$: 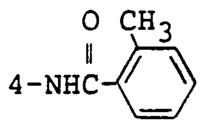

Crystalline form: White powder

Recrystallization solvent: Methanol/diethyl ether

Melting Point: 189 - 191°C

Form: Free

Example 666

Structure

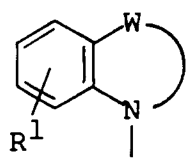 : 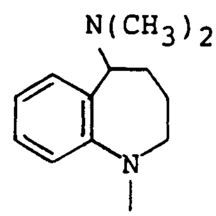

$R^2$: 2-F

$R^3$: 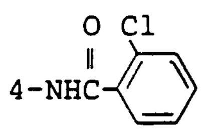

Crystalline form: White powder

Recrystallization solvent: Methanol/diethyl ether

Melting Point: 215.5 - 217°C

Form: Free

Example 667

Structure

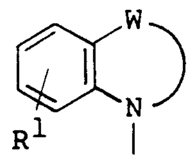 : 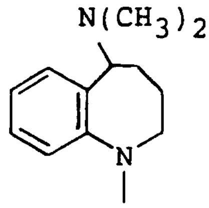

$R^2$: 2-F

$R^3$: 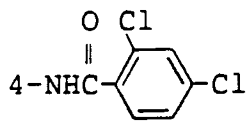

Crystalline form: White powder

Recrystallization solvent: Methanol/n-hexane

Melting Point: 192 - 194°C

Form: Free

Example 668

Structure

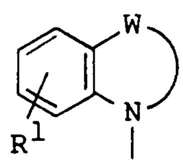 : 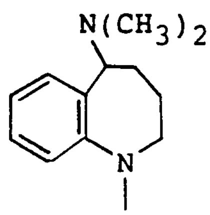

$R^2$: 3-F

$R^3$: 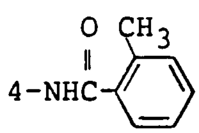

Crystalline form: White powder

Recrystallization solvent: Methanol/diethyl ether

Melting Point: 195 - 196°C

Form: Free

Example 669

Structure

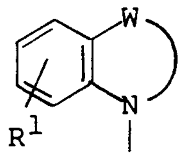 : 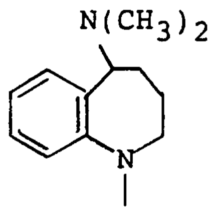

$R^2$: 3-F

$R^3$: 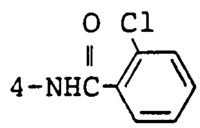

Crystalline form: White powder

Recrystallization solvent: Methanol/diethyl ether

Melting Point: 202 - 204.5°C

Form: Free

---

Example 670

Structure

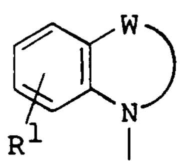 : 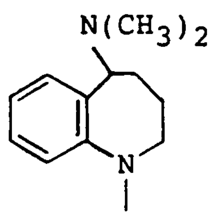

$R^2$: H

$R^3$: 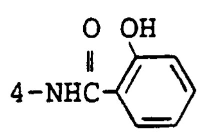

Crystalline form: White powder

Recrystallization solvent: Methanol/diethyl ether

Melting Point: 183 - 187°C

Form: Free

Example 671

Structure

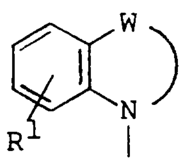 : 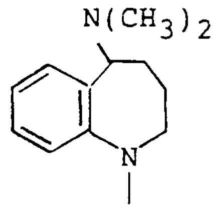

$R^2$: H

$R^3$: 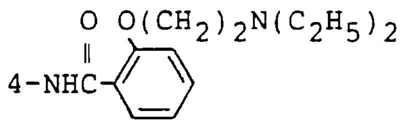

Crystalline form: White powder

Recrystallization solvent: Methanol/diethyl ether

Melting Point: 120 - 122°C

Form: Free

Example 672

Structure

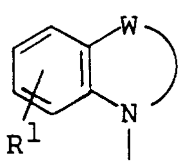 : 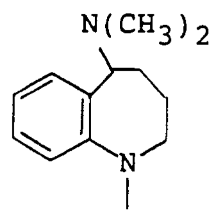

$R^2$: H

$R^3$: 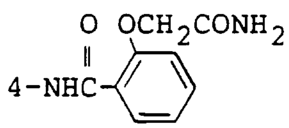

Crystalline form: White powder

Recrystallization solvent: Chloroform/diethyl ether

Melting Point: 208 - 210°C

Form: Free

Example 673

Structure

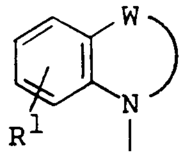 : 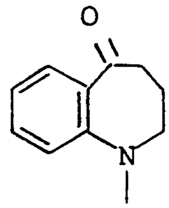

$R^2$: 2-$OCH_3$

$R^3$: 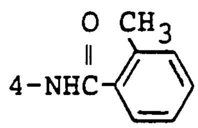

Crystalline form: White powder

Recrystallization solvent: Methanol/diethyl ether

Melting Point: 182 - 183°C

Form: Free

Example 674

Structure

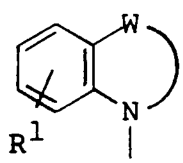 : 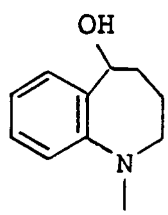

$R^2$: 2-$OCH_3$

$R^3$: 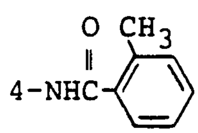

Crystalline form: White powder

Recrystallization solvent: Methanol/diethyl ether

Melting Point: 257 - 259°C

Form: Free

Example 675

Structure

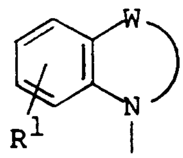 : 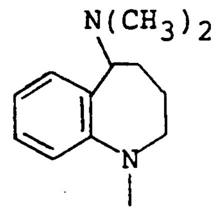

$R^2$: 2-$OC_2H_5$

$R^3$: 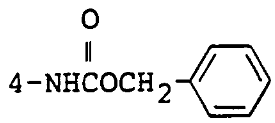

Crystalline form: White powder

Recrystallization solvent: Dichloromethane/diethyl ether

Melting Point: 134 - 135°C

Form: Free

Example 676

Structure

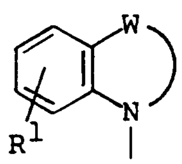 : 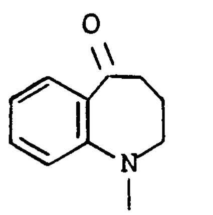

$R^2$: 2-$OCH_3$

$R^3$: 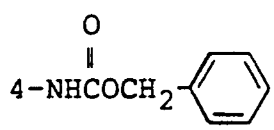

Crystalline form: White powder

Recrystallization solvent: Methanol/diethyl ether

Melting Point: 167 - 169°C

Form: Free

Example 677

Structure

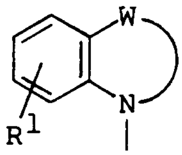 : 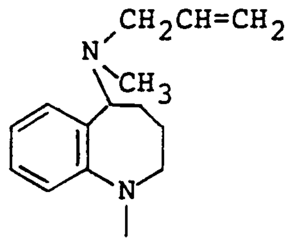

$R^2$: 2-Cl

$R^3$: 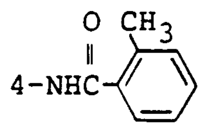

Crystalline form: Light brown prisms

Recrystallization solvent: Ethyl acetate/n-hexane

Melting Point: 170 - 172°C

Form: Free

Example 678

Structure

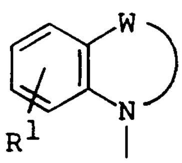 : 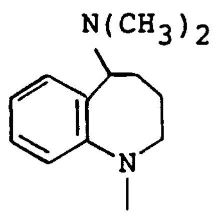

$R^2$: 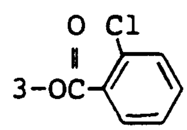

$R^3$: 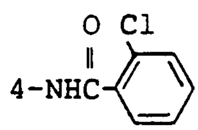

Crystalline form: White powder

Recrystallization solvent: Ethyl acetate/n-hexane

Melting Point: 181.5 - 182.5°C

Form: Free

Example 679

Structure 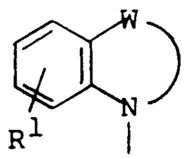 : 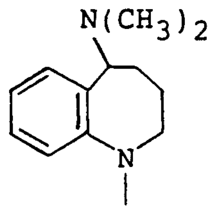

$R^2$: 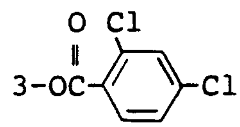

$R^3$: 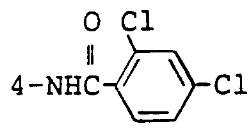

Crystalline form: White powder

Recrystallization solvent: Ethyl acetate/n-hexane

Melting Point: 176.5 - 177°C

Form: Free

---

Example 680

Structure 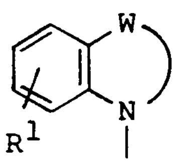 : 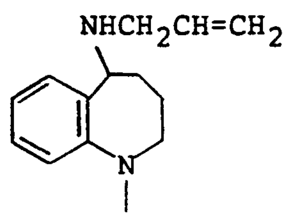

$R^2$: 2-Cl

$R^3$: 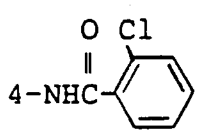

Crystalline form: Yellow amorphous

NMR analysis: 117)

Form: Free

Example 681

Structure

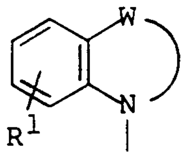 : 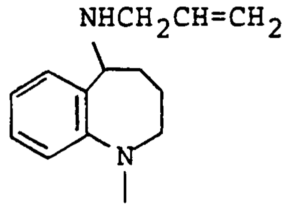

$R^2$: 2-Cl

$R^3$: 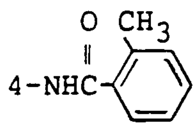

Crystalline form: Yellow amorphous

NMR analysis: 118)

Form: Free

Example 682

Structure

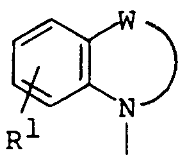 : 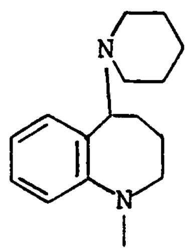

$R^2$: H

$R^3$: 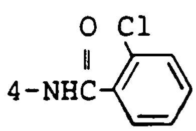

Crystalline form: White powder

Recrystallization solvent: Ethanol

Melting Point: 236 - 239°C

Form: Free

Example 683

Structure

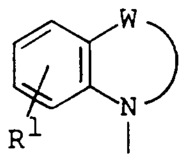 : 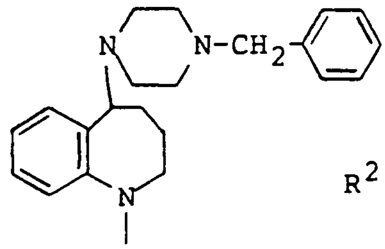

$R^2$: H

$R^3$: 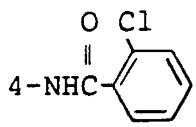

Crystalline form: White powder

Recrystallization solvent: Ethyl acetate/n-hexane

Melting Point: 153 - 154°C

Form: Free

Example 684

Structure

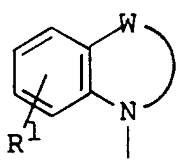 : 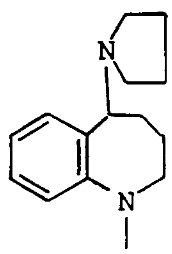

$R^2$: H

$R^3$: 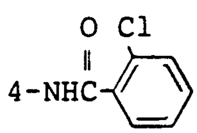

Crystalline form: White powder

Recrystallization solvent: Ethyl acetate

Melting Point: 128 - 130°C

Form: Free

Example 685

Structure

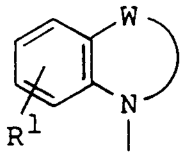 : 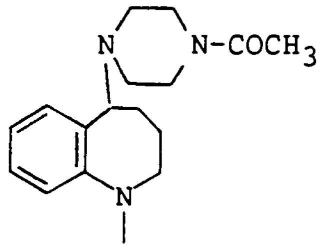

$R^2$: H

$R^3$: 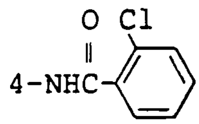

Crystalline form: White powder

Recrystallization solvent: Ethanol/diethyl ether

Melting Point: 231 - 234°C

Form: Free

Example 686

Structure

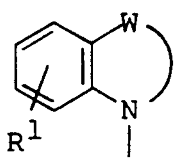 : 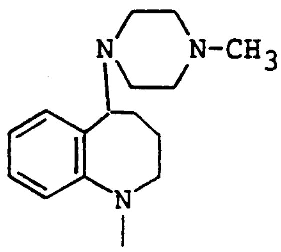

$R^2$: H

$R^3$: 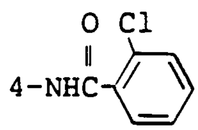

Crystalline form: White powder

Recrystallization solvent: Ethyl acetate

Melting Point: 246 - 248°C

Form: Free

Example 687

Structure

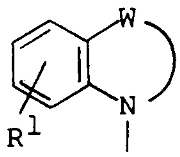 : 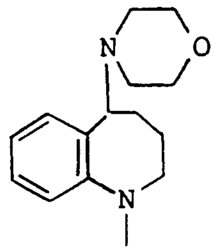

$R^2$: H

$R^3$: 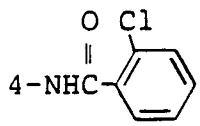

Crystalline form: White powder

Recrystallization solvent: Ethanol/water

Melting Point: 248 - 248.5°C

Form: Free

Example 688

Structure

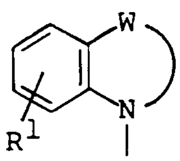 : 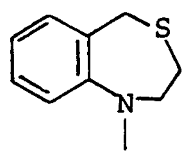

$R^2$: H

$R^3$: 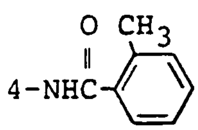

Crystalline form: White powder

Recrystallization solvent: Dichloromethane/diethyl ether

Melting Point: 204 - 205°C

Form: Free

Example 689

Structure

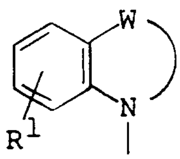 : 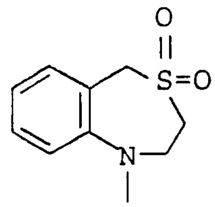 $R^2$: H

$R^3$: 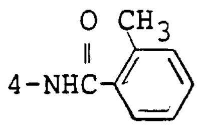

Crystalline form: White powder

Recrystallization solvent: Dichloromethane/diethyl ether

Melting Point: >300°C

NMR analysis: 119)

Form: Free

Example 690

Structure

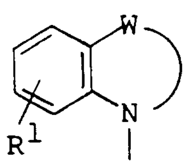 : 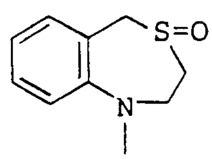 $R^2$: H

$R^3$: 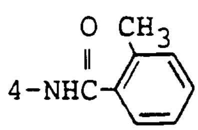

Crystalline form: White powder

Recrystallization solvent: Dichloromethane/diethyl ether

Melting Point: 292 - 294°C

Form: Free

Example 691

Structure

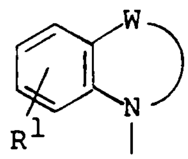 : 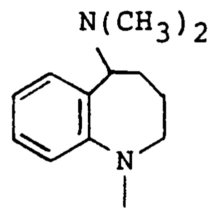

$R^2$: 2-$N(CH_3)_2$

$R^3$: 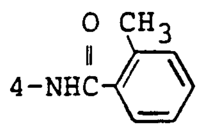

Crystalline form: Colorless amorphous

NMR analysis: 120)

Form: Free

Example 692

Structure

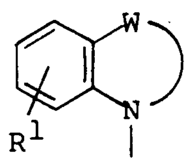 : 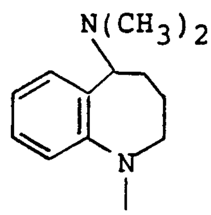

$R^2$: 2-$N(CH_3)_2$

$R^3$: 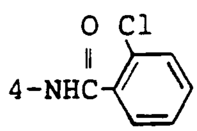

Crystalline form: Colorless amorphous

NMR analysis: 121)

Form: Free

Example 693

Structure

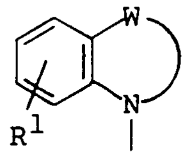 : 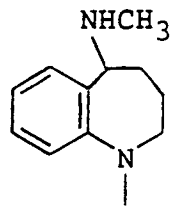

$R^2$: 2-Cl

$R^3$: 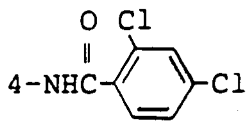

Crystalline form: Colorless amorphous

NMR analysis: 122)

Form: Free

Example 694

Structure

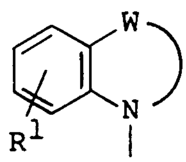 : 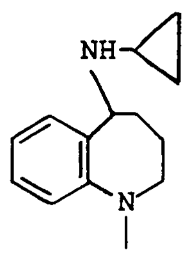

$R^2$: 2-Cl

$R^3$: 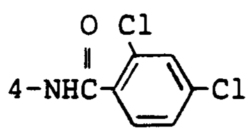

Crystalline form: Colorless amorphous

NMR analysis: 123)

Form: Free

Example 695

Structure

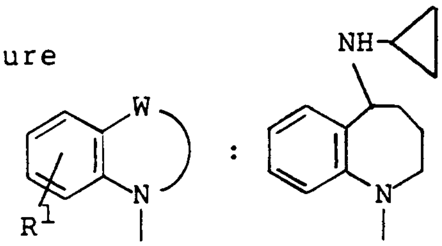

$R^2$: 2-Cl

$R^3$: 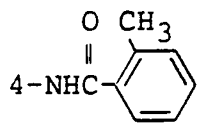

Crystalline form: White powder

Recrystallization solvent: Ethanol

Melting Point: 198.5 - 199°C

Form: Free

Example 696

Structure

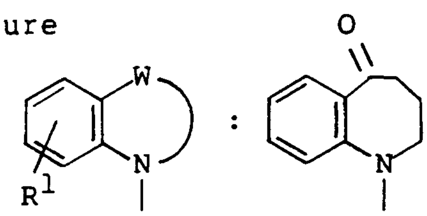

$R^2$: 2-Cl

$R^3$: 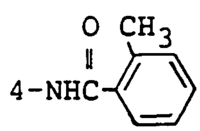

Crystalline form: White powder

Recrystallization solvent: Diethyl ether/dichloromethane

Melting Point: 168 - 170°C

Form: Free

Example 697

Structure 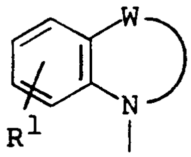 : 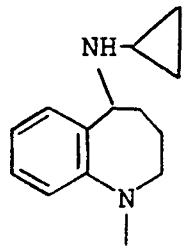 $R^2$: 2-Cl

$R^3$: 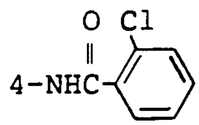

Crystalline form: White powder

Recrystallization solvent: Diethyl ether/dichloromethane

Melting Point: 175 - 176°C

Form: Free

Example 698

Structure 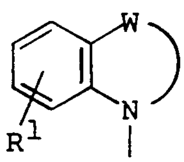 : 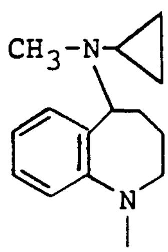 $R^2$: 2-Cl

$R^3$: 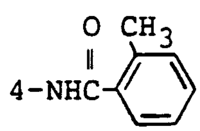

Crystalline form: White powder

Recrystallization solvent: Diethyl ether/dichloromethane

Melting Point: 177 - 178°C

Form: Free

Example 699

Structure

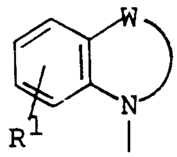 : 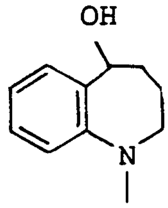

$R^2$: 2-Cl

$R^3$: 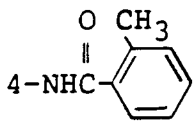

Crystalline form: White powder

Recrystallization solvent: Diethyl ether/dichloromethane

Melting Point: 222 - 223.5°C

Form: Free

Example 700

Structure

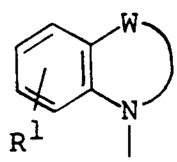 : 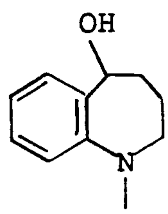

$R^2$: 2-Cl

$R^3$: 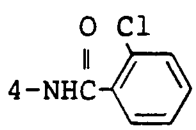

Crystalline form: White powder

Recrystallization solvent: Diethyl ether/dichloromethane

Melting Point: 243 - 244°C

Form: Free

Example 701

Structure

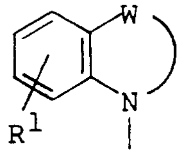 : 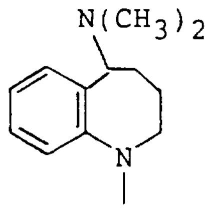

$R^2$: H

$R^3$: 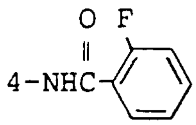

Crystalline form: White powder

Recrystallization solvent: Diethyl ether/dichloromethane

Melting Point: 180 - 181°C

Form: Free

Example 702

Structure

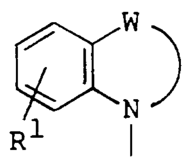 : 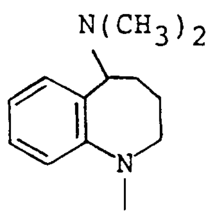

$R^2$: H

$R^3$: 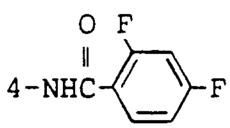

Crystalline form: Colorless amorphous

NMR analysis: 124)

Form: Free

Example 703

Structure

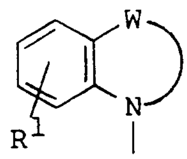 : 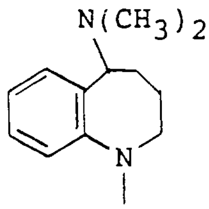

$R^2$: H

$R^3$: 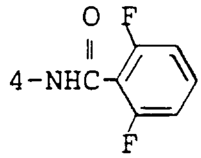

Crystalline form: White powder

Recrystallization solvent: Diethyl ether/dichloromethane

Melting Point: 231 - 233°C

Form: Free

Example 704

Structure

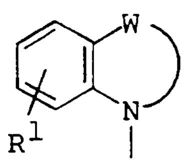 : 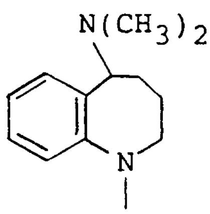

$R^2$: H

$R^3$: 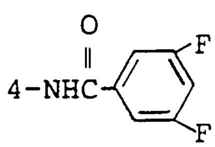

Crystalline form: White powder

Recrystallization solvent: Diethyl ether/dichloromethane

Melting Point: 196 - 198°C

Form: Free

Example 705

Structure

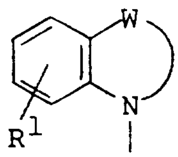 : 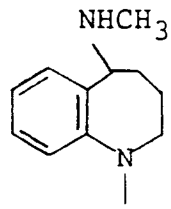 $R^2$: 2-Cl

$R^3$: 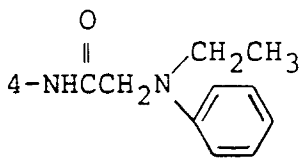

Crystalline form: Colorless amorphous

NMR analysis: 125)

Form: Free

Example 706

Structure

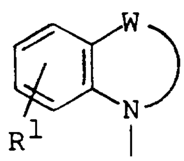 : 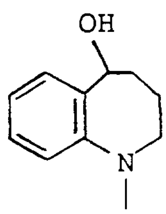 $R^2$: 2-Cl

$R^3$: 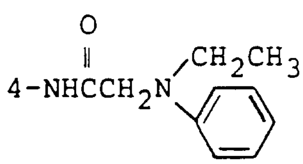

Crystalline form: Yellow amorphous

NMR analysis: 126)

Form: Free

Example 707

Structure

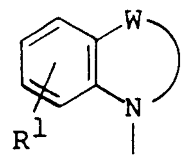 : 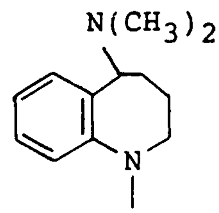

$R^2$: H

$R^3$: 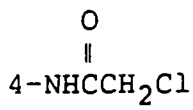

Crystalline form: Yellow powder

Recrystallization solvent: Diethyl ether/dichloromethane

Melting Point: 146 - 147°C

Form: Free

Example 708

Structure

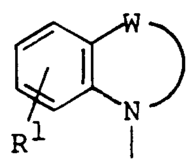 : 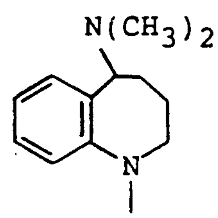

$R^2$: H

$R^3$: 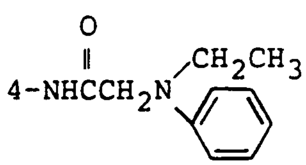

Crystalline form: Colorless amorphous

NMR analysis: 127)

Form: Free

Example 709

Structure

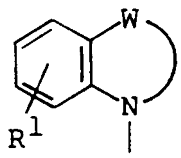 : 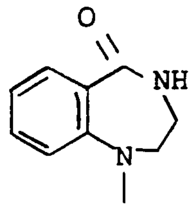

$R^2$: H

$R^3$: 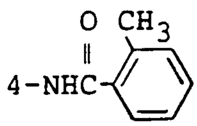

Crystalline form: White powder

Recrystallization solvent: Diethyl ether/dichloromethane

Melting Point: 220 - 221°C

Form: Free

Example 710

Structure

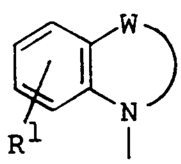 : 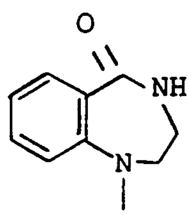

$R^2$: H

$R^3$: 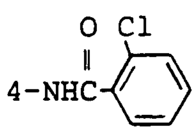

Crystalline form: White powder

Recrystallization solvent: Diethyl ether/dichloromethane

Melting Point: 170 - 172°C

Form: Free

Example 711

Structure

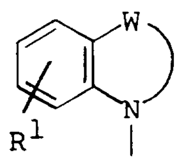 : 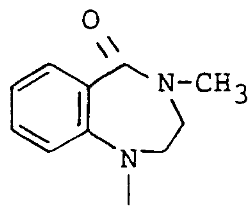 $R^2$: H

$R^3$: 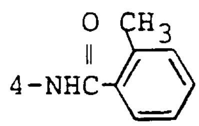

Crystalline form: Colorless amorphous

NMR analysis: 128)

Form: Free

Example 712

Structure

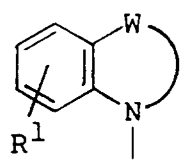 : 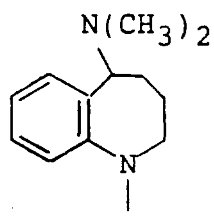 $R^2$: H

$R^3$: 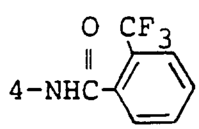

Crystalline form: White powder

Recrystallization solvent: Ethanol

Melting Point: 224 - 225°C

Form: Free

Example 713

Structure

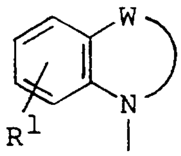
:
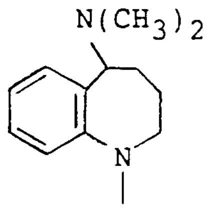

$R^2$: 3-$OCH_3$

$R^3$: 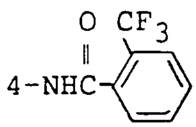

Crystalline form: White powder

Recrystallization solvent: Ethanol

Melting Point: 193 - 196°C

Form: Free

Example 714

Structure

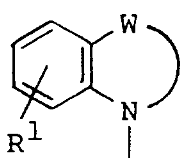
:
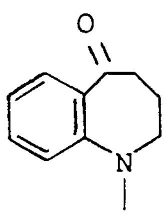

$R^2$: 2-Cl

$R^3$: 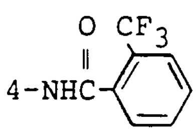

Crystalline form: White powder

Recrystallization solvent: Ethanol

Melting Point: 212 - 214°C

Form: Free

Example 715

Structure

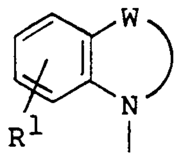 : 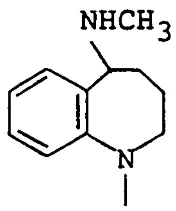

$R^2$: 2-Cl

$R^3$: 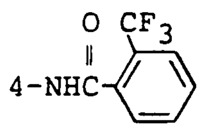

Crystalline form: White powder

Recrystallization solvent: Ethanol

Melting Point: 211 - 213°C

Form: Free

Example 716

Structure

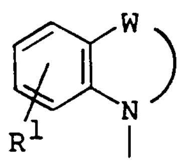 : 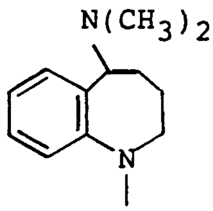

$R^2$: 2-Cl

$R^3$: 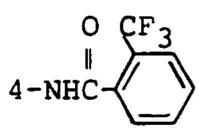

Crystalline form: White powder

Recrystallization solvent: Ethanol

Melting Point: 213 - 215°C

Form: Free

Example 717

Structure 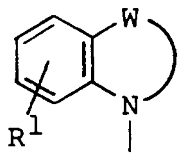 : 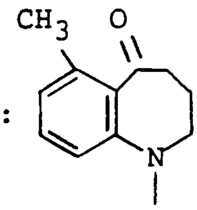 $R^2$: 2-Cl

$R^3$: 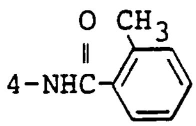

Crystalline form: White powder

Recrystallization solvent: Ethanol

Melting Point: 199 - 201°C

Form: Free

Example 718

Structure 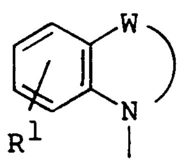 : 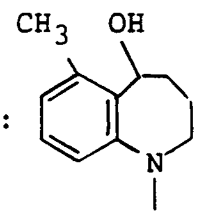 $R^2$: 2-Cl

$R^3$: 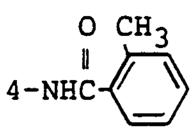

Crystalline form: White powder

Recrystallization solvent: Ethanol

Melting Point: 238 - 240°C

Form: Free

Example 719

Structure

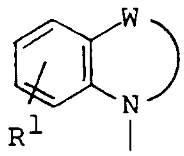 : 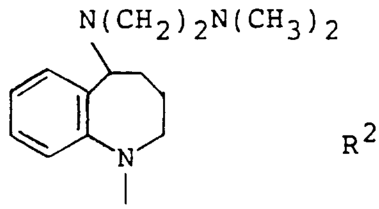

$R^2$: 2-Cl

$R^3$: 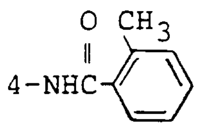

Crystalline form: White powder

Recrystallization solvent: Ethanol

Melting Point: 188 - 189°C

Form: Free

Example 720

Structure

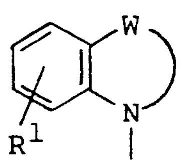 : 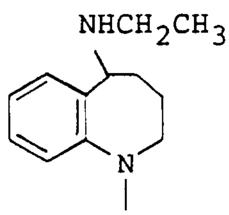

$R^2$: H

$R^3$: 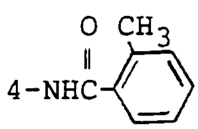

Crystalline form: Colorless prisms

Recrystallization solvent: Dioxane/water

Melting Point: 135.5 - 137°C

Form: Free

Example 721

Structure

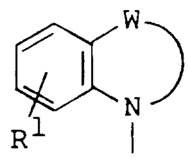
:
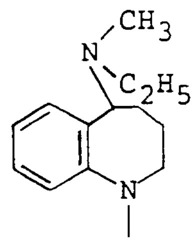

$R^2$: H

$R^3$: 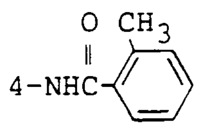

Crystalline form: White powder

Recrystallization solvent: Isopropyl alcohol/petroleum ether

Melting Point: 192 - 193°C

Form: Free

Example 722

Structure

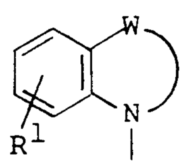
:
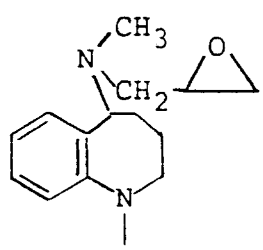

$R^2$: H

$R^3$: 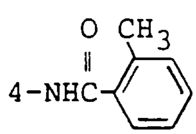

Crystalline form: Colorless needles

Recrystallization solvent: Ethyl acetate

Melting Point: 239 - 240°C

Form: Free

Example 723

Structure

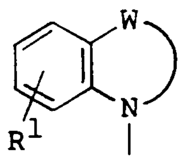
:
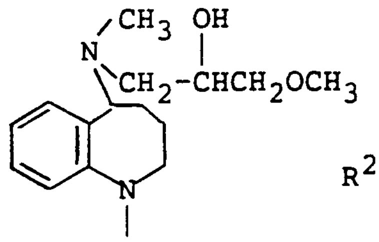
$R^2$: H

$R^3$: 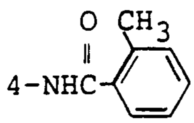

Crystalline form: Colorless amorphous

NMR analysis: 129)

Form: Free

Example 724

Structure

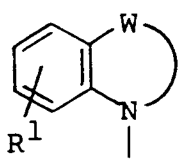
:
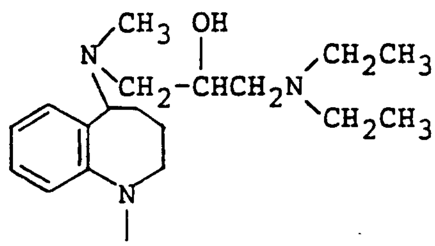
$R^2$: H

$R^3$: 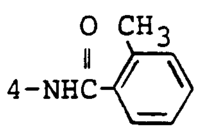

Crystalline form: Colorless amorphous

NMR analysis: 130)

Form: Free

Example 725

Structure

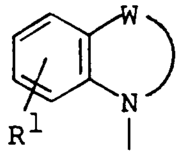

:

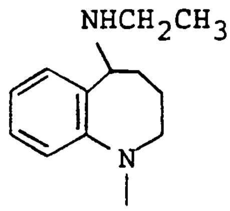

$R^2$: H

$R^3$: 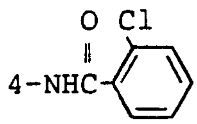

Crystalline form: Colorless needles

Recrystallization solvent: Ethanol/petroleum ether

Melting Point: 193 - 194°C

Form: Free

Example 726

Structure

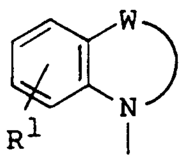

:

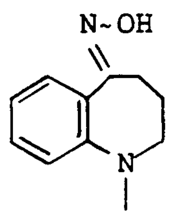

$R^2$: H

$R^3$: 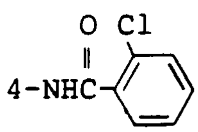

Crystalline form: Light yellow prisms

Recrystallization solvent: Ethanol

Melting Point: 245.5 - 247°C

Form: Free

Example 727

Structure

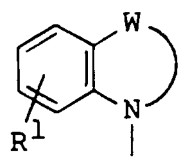 : 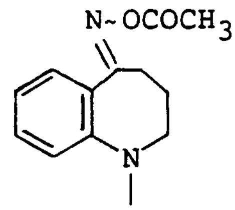

$R^2$: H

$R^3$: 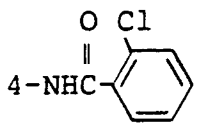

Crystalline form: Colorless prisms

Recrystallization solvent: Ethanol/petroleum ether

Melting Point: 142 - 144°C

Form: Free

Example 728

Structure

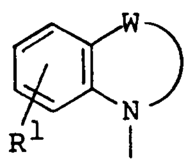 : 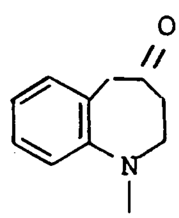

$R^2$: H

$R^3$: 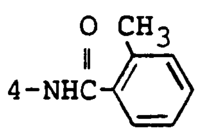

Crystalline form: Light yellow prisms

Recrystallization solvent: Ethanol

Melting Point: 214 - 217°C

Form: Free

Example 729

Structure

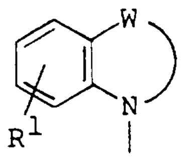 : 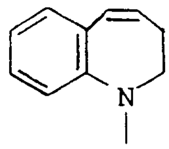

$R^2$: H

$R^3$: 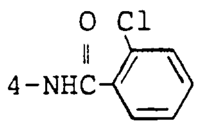

Crystalline form: Colorless needles

Recrystallization solvent: Ethanol

Melting Point: 205 - 207°C

Form: Free

Example 730

Structure

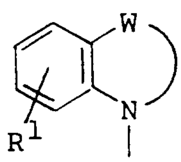 : 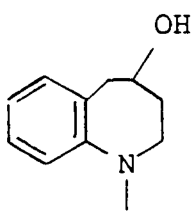

$R^2$: H

$R^3$: 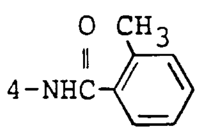

Crystalline form: Colorless needles

Recrystallization solvent: Ethanol/diethyl ether

Melting Point: 201 - 203°C

Form: Free

Example 731

Structure

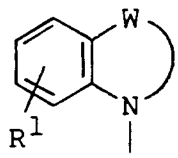 : 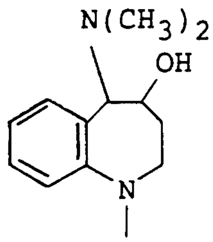

$R^2$: H

$R^3$: 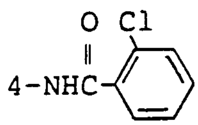

Crystalline form: Colorless needles

Recrystallization solvent: Ethanol/diethyl ether

Melting Point: 180 - 182°C

Form: Free

Example 732

Structure

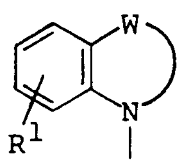 : 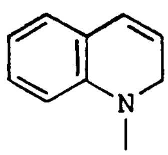

$R^2$: H

$R^3$: 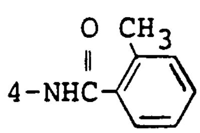

Crystalline form: Light yellow scales

Recrystallization solvent: Ethanol/diethyl ether

Melting Point: 178 - 180°C

Form: Free

Example 733

Structure

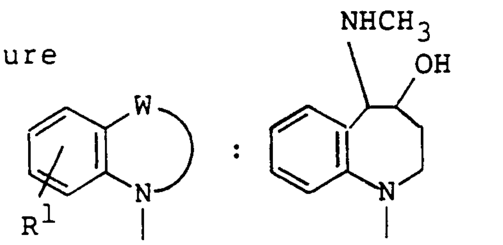

$R^2$: H

$R^3$: 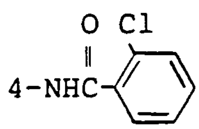

Crystalline form: Colorless needles

Recrystallization solvent: Methanol/diethyl ether

Melting Point: 208 - 213°C

Form: Free

Example 734

Structure

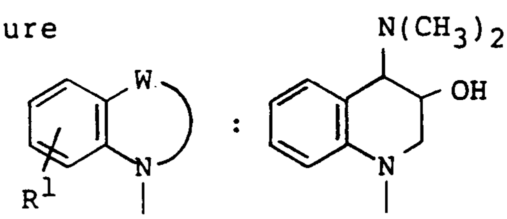

$R^2$: H

$R^3$: 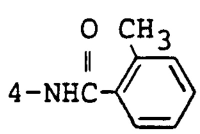

Crystalline form: White powder

Recrystallization solvent: Ethanol/diethyl ether

Melting Point: 175 - 177°C

Form: Free

Example 735

Structure

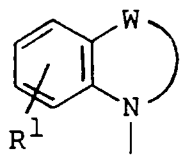
:
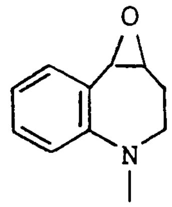

$R^2$: H

$R^3$: 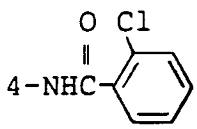

Crystalline form: White powder

NMR analysis: 131)

Form: Free

Example 736

Structure

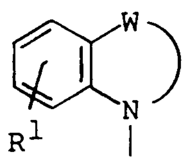
:
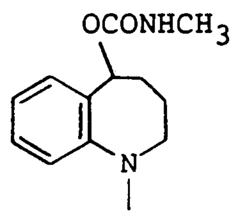

$R^2$: H

$R^3$: 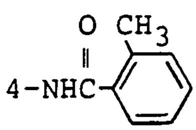

Crystalline form: White powder

Recrystallization solvent: Ethyl acetate/n-hexane

Melting Point: 277 - 279°C

Form: Free

Example 737

Structure 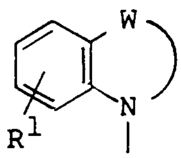 : 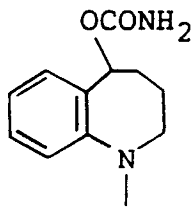 $R^2$: H

$R^3$: 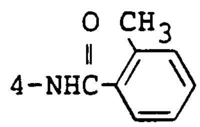

Crystalline form: Colorless amorphous

NMR analysis: 132)

Form: Free

Example 738

Structure 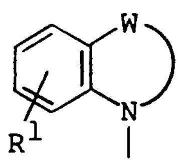 : 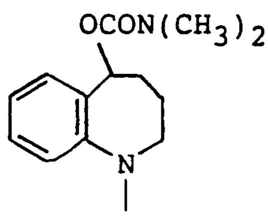 $R^2$: H

$R^3$: 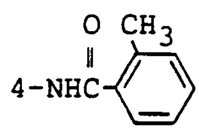

Crystalline form: Colorless amorphous

NMR analysis: 133)

Form: Free

Example 739

Structure

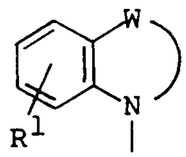 : 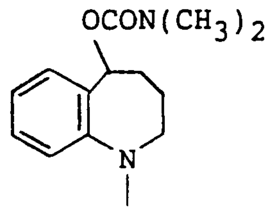

$R^2$: H

$R^3$: 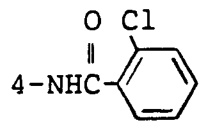

Crystalline form: Colorless amorphous

NMR analysis: 134)

Form: Free

Example 740

Structure

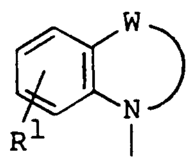 : 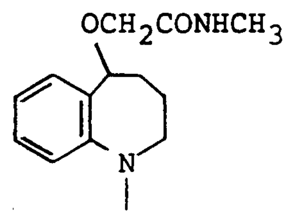

$R^2$: H

$R^3$: 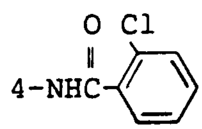

Crystalline form: Colorless amorphous

NMR analysis: 135)

Form: Free

Example 741

Structure

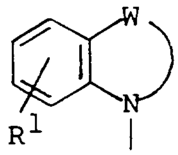

:

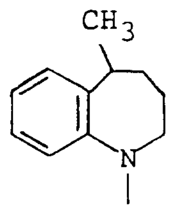

$R^2$: H

$R^3$: 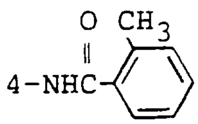

Crystalline form: White powder

Recrystallization solvent: Ethyl acetate/n-hexane

Melting Point: 213 - 214°C

Form: Free

Example 742

Structure

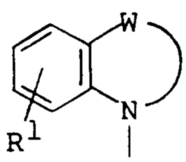

:

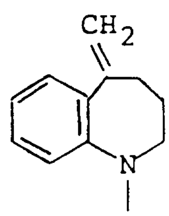

$R^2$: H

$R^3$: 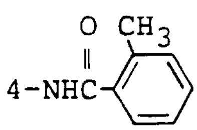

Crystalline form: White powder

Recrystallization solvent: Ethyl acetate/n-hexane

Melting Point: 216 - 217°C

Form: Free

Example 743

Structure

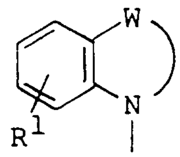 : 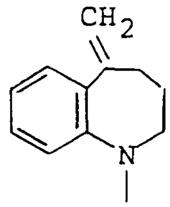

$R^2$: 2-Cl

$R^3$: 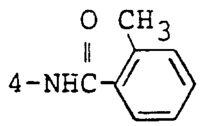

Crystalline form: White powder

Recrystallization solvent: Ethyl acetate/n-hexane

Melting Point: 165 - 167°C

Form: Free

Example 744

Structure

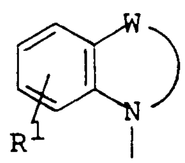 : 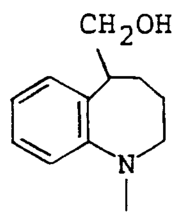

$R^2$: H

$R^3$: 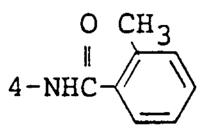

Crystalline form: White powder

Recrystallization solvent: Ethyl acetate/n-hexane

Melting Point: 202 - 206°C

Form: Free

Example 745

Structure

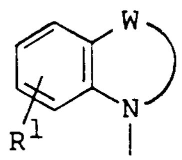 : 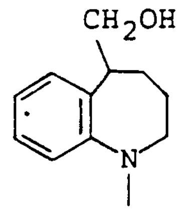

$R^2$: 2-Cl

$R^3$: 4-NHC 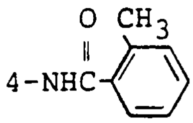

Crystalline form: White powder

Recrystallization solvent: Ethyl acetate/n-hexane

Melting Point: 220 - 221.5°C

Form: Free

Example 746

Structure

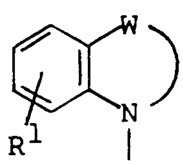 : 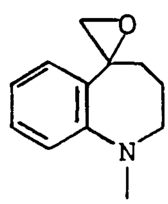

$R^2$: H

$R^3$: 4-NHC 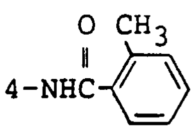

Crystalline form: Colorless needles

Recrystallization solvent: Ethyl acetate/n-hexane

Melting Point: 186 - 186.5°C

Form: Free

Example 747

Structure

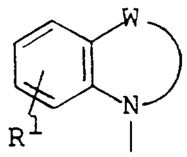 : 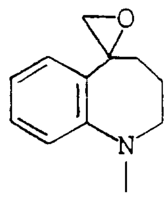

$R^2$: 2-Cl

$R^3$: 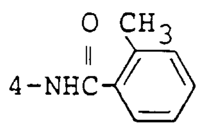

Crystalline form: Colorless amorphous

NMR analysis: 136)

Form: Free

Example 748

Structure

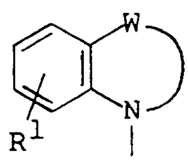 : 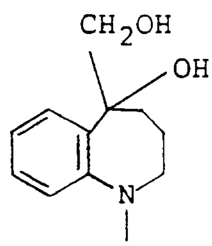

$R^2$: H

$R^3$: 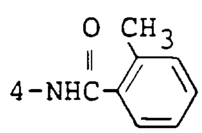

Crystalline form: White powder

Recrystallization solvent: Ethyl acetate/n-hexane

Melting Point: 136 - 140°C

Form: Free

Example 749

Structure

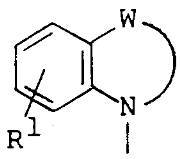 : 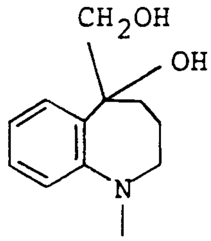

$R^2$: 2-Cl

$R^3$: 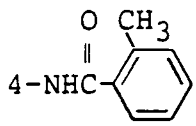

Crystalline form: White powder

Recrystallization solvent: Ethyl acetate/n-hexane

Melting Point: 151 - 153°C

Form: Free

Example 750

Structure

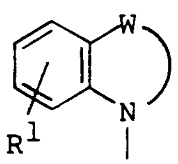 : 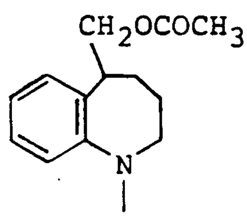

$R^2$: H

$R^3$: 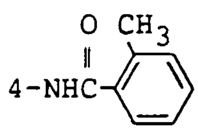

Crystalline form: Colorless needles

Recrystallization solvent: Ethyl acetate/n-hexane

Melting Point: 155 - 156°C

Form: Free

Example 751

Structure

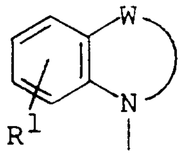

:

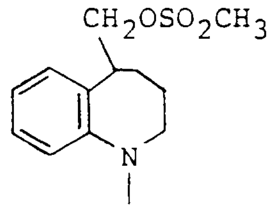

$R^2$: H

$R^3$: 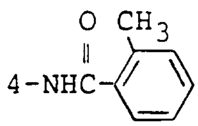

Crystalline form: White powder

Recrystallization solvent: Ethyl acetate/n-hexane

Melting Point: 189 - 190°C

Form: Free

Example 752

Structure

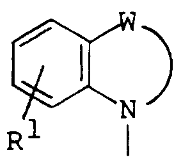

:

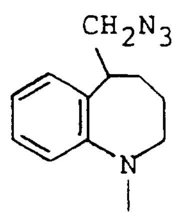

$R^2$: H

$R^3$: 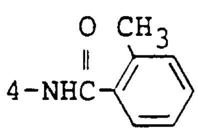

Crystalline form: White powder

Recrystallization solvent: Ethyl acetate/n-hexane

Melting Point: 188 - 190°C

Form: Free

Example 753

Structure

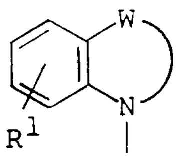 : 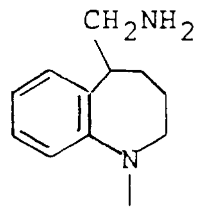

$R^2$: H

$R^3$: 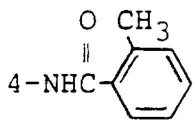

Crystalline form: Colorless needles

Recrystallization solvent: Ethanol

Melting Point: 233 - 235°C

Form: Free

Example 754

Structure

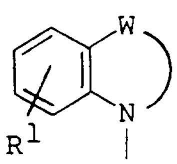 : 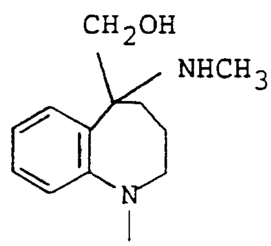

$R^2$: H

$R^3$: 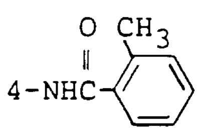

Crystalline form: Colorless amorphous

NMR analysis: 137)

Form: Free

Example 755

Structure

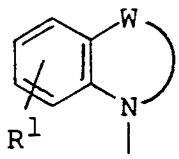 : 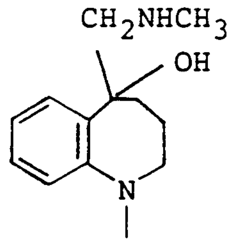

$R^2$: H

$R^3$: 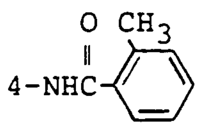

Crystalline form: White powder

Recrystallization solvent: Ethyl acetate/n-hexane

Melting Point: 176 - 179°C

Form: Free

Example 756

Structure

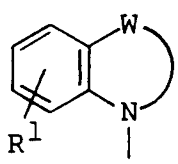 : 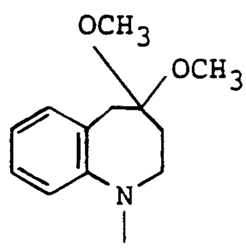

$R^2$: H

$R^3$: 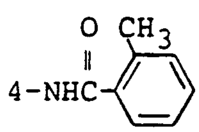

Crystalline form: Colorless needles

Recrystallization solvent: Methanol/diethyl ether

Melting Point: 183 - 185°C

Form: Free

117) $^{1}$H-NMR($CDCl_3$) δ ; 1.3-2.3 (4H, m), 3.1-3.4 (3H, m), 3.8-4.6 (2H, m), 5.0-5.3 (2H, m), 5.8-6.1 (1H, m), 6.8-8.5 (11H, m)

118) $^{1}$H-NMR($CDCl_3$) δ ; 1.6-2.2 (4H, m), 2.46, 2.53 (3H, each s), 3.1-3.5 (3H, m), 3.8-4.6 (2H, m), 5.0-5.3 (2H, m), 5.8-6.1 (1H, m), 6.8-8.0 (11H, m)

119) $^{1}$H-NMR(DMSO-$d_6$) δ ; 2.33 (3H, s), 3.36 (2H, m), 3.89 (1H, m), 4.41 (1H, m), 5.07 (1H, m), 5.40 (1H, d, J=14.8 Hz), 6.85 (1H, d, J=7.2 Hz), 7.15-7.65 (11H, m), 10.35 (1H, s)

120) $^{1}$H-NMR($CDCl_3$) δ ; 1.25-5.05 (22H, m), 6.65-7.65 (11H, m), 7.75-8.25 (1H, m)

121) $^{1}$H-NMR($CDCl_3$) δ ; 1.15-5.05 (19H, m), 6.75-7.85 (11H, m), 7.85-8.25 (1H, m)

122) $^{1}$H-NMR($CDCl_3$) δ ; 1.25-2.85 (8H, m), 2.95 - 4.95 (2H, m), 6.75-7.85 (10H, m), 9.25-9.75 (1H, m)

123) $^{1}$H-NMR($CDCl_3$) δ ; 0.20-0.70 (4H, m), 0.95-2.35 (6H, m), 2.65-5.00 (2H, m), 6.75-7.90 (10H, m), 8.65-9.25 (1H, m)

124) $^{1}$H-NMR($CDCl_3$) δ ; 1.20-3.15 (11H, m), 3.45-3.70 (1H, m), 4.05-5.20 (1H, m), 6.60-7.65 (10H, m), 8.15-8.45 (2H, m)

125) $^{1}$H-NMR($CDCl_3$) δ ; 1.19 (3H, t, J=7 Hz), 1.25-3.25 (8H, m), 3.46 (2H, q, J=7 Hz), 3.40-4.10 (3H, m), 4.45-5.10 (1H, m), 6.65-7.75 (12H, m), 8.30-8.60 (1H, m)

126) $^1H$-NMR($CDCl_3$) δ ; 1.10-1.30 (3H, m), 1.50-2.35 (4H, m), 2.65-3.05 (2H, m), 3.35-3.60 (2H, m), 3.80-4.05 (2H, m), 4.65-5.15 (2H, m), 6.55-7.85 (12H, m), 8.35-8.65 (1H, m)

127) $^1H$-NMR($CDCl_3$) δ ; 1.20 (3H, t, J=7 Hz), 1.10-3.15 (11H, m), 3.45-3.65 (3H, m), 3.88 (2H, s), 3.95-5.15 (1H, m), 6.55-7.65 (13H, m), 8.37 (1H, s)

128) $^1H$-NMR($CDCl_3$) δ ; 2.45 (3H, s), 3.40 (3H, s), 4.01 (2H, m), 4.38 (2H, m), 7.20-7.77 (13H, m)

129) $^1H$-NMR($CDCl_3$) δ ; 1.35-4.55 (22H, m), 6.3-7.8 (13H, m)

130) $^1H$-NMR($CDCl_3$) δ ; 1.10 (6H, t, J=7 Hz), 1.35-5.1 (23H, m), 6.55-7.8 (13H, m)

131) $^1H$-NMR($CDCl_3$) δ ; 1.94-3.21 (3H, m), 3.30-4.82 (3H, m), 6.57 (1H, d, J=7.5 Hz), 6.86-8.10 (11H, m), 8.72 (1H, brs)

132) $^1H$-NMR(DMSO-$d_6$) δ ; 1.57-1.85 (2H, m), 1.85-2.28 (2H, m), 2.33 (3H, s), 2.64-2.86 (1H, m), 4.53-5.07 (1H, m), 5.79-5.94 (1H, m), 6.47-7.68 (2H, br), 6.64-6.77 (1H, m), 6.96-7.62 (12H, m)

133) $^1H$-NMR($CDCl_3$) δ ; 1.61-1.97 (2H, m), 2.00-2.54 (2H, m), 2.47 (3H, s), 2.60-3.23 (7H, m), 4.76-5.22 (1H, m), 5.94-6.19 (1H, m), 6.61-6.74 (1H, m), 6.91-7.62 (12H, m)

134) $^1H$-NMR($CDCl_3$) δ ; 1.68-1.97 (2H, m), 2.03-2.53 (2H, m), 2.61-3.24 (7H, m), 4.76-5.22 (1H, m), 5.97-6.17

(1H, m), 6.59-6.74 (1H, m), 6.92-7.13 (1H, m), 7.13-7.58 (9H, m), 7.66-7.85 (1H, m), 7.85-8.00 (1H, m)

135) $^1$H-NMR($CDCl_3$) δ ; 1.57-1.93 (2H, m), 1.93-2.54 (2H, m), 2.54-2.72 (1H, m), 2.79-3.09 (3H, m), 3.90-4.32 (2H, m), 4.49-5.18 (2H, m), 6.31-6.93 (2H, m), 6.96-7.63 (10H, m), 7.63-7.89 (1H, m), 7.89-8.16 (1H, m)

136) $^1$H-NMR($CDCl_3$) δ ; 1.44-1.95 (2H, m), 1.95-2.28 (2H, m), 2.40-2.67 (3H, m), 2.73-3.38 (3H, m), 3.40-3.97 (1H, m), 4.50-5.20 (1H, m), 6.67-8.11 (11H, m)

137) $^1$H-NMR($CDCl_3$) δ ; 1.50-2.10 (3H, m), 2.10-2.28 (1H, m), 2.36 (3H, s), 2.48 (3H, s), 2.68-2.97 (1H, m), 3.26-3.47 (1H, m), 4.16 (1H, d, J=13.8 Hz), 4.25 (1H, d, J=13.8 Hz), 5.95 (1H, brs), 6.60-6.76 (1H, m), 6.97-7.52 (8H, m), 7.52-7.73 (2H, m), 7.73-7.97 (2H, m)

Example 757

A mixture of 5-dimethylamino-1-[4-(2-chlorobenzoylamino)benzoyl]-2,3,4,5-tetrahydro-1H-benzazepine (10 g), methyl iodide (1.7 ml) and chloroform (10 ml) is heated with stirring at 100°C for 3 hours in an autoclave. After completion of the reaction, the solvent is distilled off under reduced pressure and the resulting residue is dissolved in methanol. The mixture is treated with IRA-400 (trade mark; Organo Co., Ltd., $OH^-$ type). Methanol is distilled off and the resulting residue is suspended in t-butyl alcohol (90 ml), and thereto is added potassium t-butoxide (2.3 g). The mixture is refluxed for 5 hours. The solvent is distilled off under reduced pressure, and the resulting residue is dissolved in dichloromethane. The mixture is washed successively with water and saturated saline solution and dried over magnesium sulfate. The solvent is distilled off and to the resulting residue is added dichloromethane/diethyl ether. The precipitated crude crystal is recrystallized from ethanol to give 1-[4-(2-chlorobenzoylamino)benzoyl]-2,3-dihydro-1H-benzazepine (5.15 g) as colorless needles, m.p. 205 - 207°C.

Example 758

1-[4-(2-Chlorobenzoylamino)benzoyl]-2,3-dihydro-1H-benzazepine (4.7 g) is dissolved in dichloromethane (50 ml) and thereto is added 80 % m-chloroperbenzoic acid (3 g). The mixture is stirred at room temperature overnight. The dichloromethane layer is washed successively with saturated aqueous sodium hydrogen carbonate solution and saturated saline solution, and the solvent is distilled off under reduced pressure. The resulting residue is purified by silica

gel column chromatography (eluent; dichloromethane : methanol = 50 : 1) to give 4,5-epoxy-1-[4-(2-chlorobenzoylamino)benzoyl]-2,3,4,5-tetrahydro-1H-benzazepine (4.26 g) as white powder.

$^1$H-NMR($CDCl_3$) δ ; 1.94-3.21 (3H, m), 3.30-4.82 (3H, m), 6.57 (1H, d, J=7.5 Hz), 6.86-8.10 (11H, m), 8.72 (1H, brs)

Example 759

A mixture of 4,5-epoxy-1-[4-(2-chlorobenzoylamino)benzoyl]-2,3,4,5-tetrahydro-1H-benzazepine (0.5 g), dimethylamine hydrochloride (2.6 g), triethylamine (4.5 g) and methanol (15 ml) is refluxed for 19 hours. After completion of the reaction, the solvent is distilled off and the resulting residue is dissolved in dichloromethane. The mixture is washed successively with water and saturated saline solution. The solvent is distilled off and the resulting residue is purified by silica gel column chromatography (eluent; dichloromethane : methanol = 50 : 1), and recrystallized from ethanol/diethyl ether to give trans-5-dimethylamino-4-hydroxy-1-[4-(2-chlorobenzoylamino)benzoyl]-2,3,4,5-tetrahydro-1H-benzazepine (0.38 g) as colorless needles, m.p. 180 - 182°C.

Using the suitable starting materials, the compounds of the above Examples 733 and 734 are obtained in the same manner as in Example 759.

Example 760

Methyltriphenylphosphonium bromide (4.30 g) is suspended in tetrahydrofuran (100 ml) and thereto is added potassium t-butoxide (1.58 g) under ice-cooling. The mixture is stirred at -5°C for 1 hour and thereto is added 5-oxo-1-[4-(2-methylbenzoylamino)benzoyl]-2,3,4,5-tetrahydro-1H-benzazepine (1.60 g) and the mixture is stirred at room temperature for 1 hour. The reaction solution is poured into ice-water (200 ml) and extracted with ethyl acetate. The extract is washed with saturated saline solution and dried over magnesium sulfate. The solvent is distilled off and the resulting residue is purified by silica gel column chromatography (eluent; ethyl acetate : n-hexane = 1 : 2), and recrystallized from ethyl acetate/n-hexane to give 5-methylidene-1-[4-(2-methylbenzoylamino)benzoyl]-2,3,4,5-tetrahydro-1H-benzazepine (1.34 g) as white powder, m.p. 216 - 217°C.

Using the suitable starting materials, the compound of the above Example 743 is obtained in the same manner as in Example 760.

Example 761

5-Methylidene-1-[4-(2-methylbenzoylamino)benzoyl]-2,3,4,5-tetrahydro-1H-benzazepine (2.84 g) is suspended in tetrahydrofuran (50 ml) and thereto is added 1 M solution of boran-tetrahydrofuran complex in tetrahydrofuran (43 ml). The mixture is stirred at room temperature for 6 hours. After completion of the reaction, the reaction solution is cooled with ice, and thereto is added water (70 ml). After termination of the evolution of hydrogen gas, to the reaction solution are added 25 % aqueous sodium hydroxide solution (7.0 ml), and subsequently 31 % aqueous hydrogen peroxide solution (4.7 ml), and the mixture is heated with stirring at 50°C for 1 hour. After cooling, to the reaction solution is added saturated saline solution and the tetrahydrofuran layer is collected, washed with saturated saline solution and dried over magnesium sulfate. The solvent is distilled off and the resulting residue is recrystallized from ethyl acetate/n-hexane to give 5-hydroxymethyl-1-[4-(2-methylbenzoylamino)benzoyl]-2,3,4,5-tetrahydro-1H-benzazepine (1.96 g) as white powder, m.p. 202 - 206°C.

Using the suitable starting materials, the compound of the above Example 745 is obtained in the same manner as in Example 761.

Example 762

5-Methylidene-1-[2-chloro-4-(2-methylbenzoylamino)benzoyl]-2,3,4,5-tetrahydro-1H-benzazepine (0.81 g) is dissolved in dichloromethane (30 ml) and thereto is added m-chloroperbenzoic acid (0.57 g). The mixture is stirred at room temperature for 15 hours. After completion of the reaction, the reaction solution is washed successively with aqueous sodium hydrogensulfite solution, aqueous sodium hydrogen carbonate solution and saturated saline solution, and dried over magnesium sulfate. The solvent is distilled off and the resulting residue is purified with silica gel column chromatography (eluent; ethyl acetate : n-hexane = 2 : 3) to give 1-[2-chloro-4-(2-methylbenzoylamino)benzoyl]-2,3,4,5-tetrahydro-1H-benzazepine-5-spiro-3'-oxirane (0.70 g) as colorless amorphous.

$^1$H-NMR($CDCl_3$) δ ; 1.44-1.95 (2H, m), 1.95-2.28 (2H, m), 2.40-2.67 (3H, m), 2.73-3.38 (3H, m), 3.40-3.97 (1H, m), 4.50-5.20 (1H, m), 6.67-8.11 (11H, m)

Using the suitable starting materials, the compound of the above Example 746 is obtained in the same manner as in Example 762.

Example 763

To 5-methylidene-1-[4-(2-methylbenzoylamino)benzoyl]-2,3,4,5-tetrahydro-1H-benzazepine (0.60 g) are added successively t-butyl alcohol (6.0 ml), water (1.2 ml), pyridine (0.3 ml), osmium tetroxide (1.2 mg) and trimethylamine N-oxide dihydrate (0.22 g), and the mixture is refluxed with stirring for 2.5 hours. After cooling, to the reaction solution is added 20 % aqueous sodium hydrogensulfite solution (10 ml), and the mixture is stirred at room temperature for 1.5 hour. The reaction solution is extracted with a mixture of ethyl acetate/tetrahydrofuran (1:1). The extract is washed successively with diluted hydrochloric acid and saturated saline solution, and dried over magnesium sulfate. The solvent is distilled off and the resulting residue is recrystallized from ethyl acetate/n-hexane to give 5-hydroxymethyl-5-hydroxy-1-[4-(2-methylbenzoylamino)benzoyl]-2,3,4,5-tetrahydro-1H-benzazepine (0.55 g) as white powder, m.p. 136 - 140°C.

Using the suitable starting materials, the compound of the above Example 749 is obtained in the same manner as in Example 763.

Example 764

To 5-hydroxymethyl-1-[4-(2-methylbenzoylamino)benzoyl]-2,3,4,5-tetrahydro-1H-benzazepine (0.40 g) are added acetic anhydride (4.0 ml) and pyridine (0.5 ml), and the mixture is stirred at room temperature for 5 hours. After completion of the reaction, the reaction solution is poured into ice-water and extracted with ethyl acetate. The extract is washed successively with diluted hydrochloric acid and saturated saline solution, and dried over magnesium sulfate. The solvent is distilled off and the resulting residue is recrystallized from ethyl acetate/n-hexane to give 5-acetyloxymethyl-1-[4-(2-methylbenzoylamino)benzoyl]-2,3,4,5-tetrahydro-1H-benzazepine (0.43 g) as colorless needles, m.p. 155 - 156°C.

Example 765

5-Hydroxymethyl-1-[4-(2-methylbenzoylamino)benzoyl]-2,3,4,5-tetrahydro-1H-benzazepine (0.70 g) is dissolved in a mixture (30 ml) of dichloromethane/acetonitrile (1:1) and thereto are added methanesulfonyl chloride (0.8 ml) and pyridine (1.0 ml), and the mixture is refluxed with stirring for 2 hours. After cooling, the reaction solution is evaporated under reduced pressure and to too resulting residue is added water and then extracted with ethyl acetate. The extract is washed successively with diluted hydrochloric acid and saturated saline solution, and dried over magnesium sulfate. The solvent is distilled off and the resulting residue is recrystallized from ethyl acetate/n-hexane to give 5-methanesulfonyloxymethyl-1-[4-(2-methylbenzoylamino)benzoyl]-2,3,4,5-tetrahydro-1H-benzazepine (0.72 g) as white powder, m.p. 189 - 190°C.

Example 766

5-Methanesulfonyloxymethyl-1-[4-(2-methylbenzoylamino)benzoyl]-2,3,4,5-tetrahydro-1H-benzazepine (0.49 g) is dissolved in a mixture (25 ml) of acetonitrile/dimethylformamide (4:1) and thereto is added sodium aside (0.11 g). The mixture is refluxed with stirring for 3.5 hours. After cooling, the reaction solution is poured into ice-water (40 ml), extracted with ethyl acetate, washed with saturated saline solution, and dried over magnesium sulfate. The solvent is distilled off and the resulting residue is purified by silica gel column chromatography (eluent; ethyl acetate : n-hexane = 1 : 2), and recrystallized from ethyl acetate/n-hexane to give 5-azidomethyl-1-[4-(2-methylbenzoylamino)benzoly]-2,3,4,5-tetrahydro-1H-benzazepine (0.29 g) as white powder, m.p. 188 - 189°C.

Example 767

5-Azidomethyl-1-[4-(2-methylbenzoylamino)benzoyl]-2,3,4,5-tetrahydro-1H-benzazepine (0.27 g) is suspended in ethanol (50 ml) and the mixture is subjected to catalytic hydrogenation at room temperature under 3 kg/cm$^2$ for 6 hours by using 10 % Pd-C (27 mg). The catalyst is removed by filtration with celite and the filtrate is distilled off and the resulting residue is recrystallized from ethanol to give 5-aminomethyl-1-[4-(2-methylbenzoylamino)benzoyl]-2,3,4,5-tetrahydro-1H-benzazepine (0.12 g) as colorless needles, m.p. 233 - 235°C.

Example 768

To 1-[4-(2-methylbenzoylamino)benzoyl]-2,3,4,5-tetrahydro-1H-benzazepine-5-spiro-3'-oxirane (0.30 g) is added 30 % solution of methylamine in methanol (30 ml), and the mixture is refluxed for 14 hours. After compeltion of the reaction, the reaction solution is evaporated under reduced pressure and the resulting residue is purified by silica gel column chromatography

(eluent; ethyl acetate : n-hexane = 1 : 1 → dichloromethane : methanol : aqueous ammonia = 60 : 10 : 1)

to give 5-hydroxymethyl-5-methylamino-1-[4-(2-methylbenzoylamino]benzoyl]-2,3,4,5-tetrahydro-1H-benzazepine (A; 35.3 mg) and 5-methylaminomethyl-5-hydroxy-1-[4-(2-methylbenzoylamino)benzoyl]-2,3,4,5-tetrahydro-1H-benzazepine (B; 109 mg).

(A); Colorless amorphous

$^1$H-NMR($CDCl_3$) δ ; 1.50-2.10 (3H, m), 2.10-2.28 (1H, m), 2.36 (3H, s), 2.48 (3H, s), 2.68-2.97 (1H, m), 3.26-3.47 (1H, m), 4.16 (1H, d, J=13.8 Hz), 4,25 (1H, d, J=13.8 Hz), 5.95 (1H, brs), 6.60-6.76 (1H, m), 6.97-7.52 (8H, m), 7.52-7.73 (2H, m), 7.73-7.97 (2H, m)

(B); White powder (recrystallized from ethyl acetate/n-hexane)

m.p. 176 - 179°C

Example 769

5-Methylamino-1-[4-(2-methylbenzoylamino)benzoyl]-2,3,4,5-tetrahydro-1H-benzazepine (1 g) is dissolved in dimethylformamide (10 ml) and thereto are added potassium carbonate (0.5 g) and ethyl iodide (0.45 g). The mixture is stirred at room temperature overnight. After completion of the reaction, the reaction solution is poured into ice-water and the precipitated crystal is collected by filtration, and purified by silica gel column chromatography (eluent; dichloromethane : methanol = 90 : 1), and recrystallized from isopropyl alcohol/petroleum ether to give 5-(N-methyl-N-ethylamino)-1-[4-(2-methylbenzoylamino)benzoyl]-2,3,4,5-tetrahydro-1H-benzazepine (50 mg) as white powder, m. p. 192 - 193°C.

Using the suitable starting materials, the compounds of the above Examples 244, 246 - 248, 330, 339, 342, 346, 350, 366, 375, 376, 406 - 418, 453, 455, 457, 460, 464, 467, 506, 507, 537 - 545, 547, 549 - 556, 561 - 566, 568 - 571, 577, 601 - 603, 607 - 625, 654 - 672, 675, 677 - 681, 691 - 695, 697, 698, 701 - 705, 707, 708, 712, 713, 715, 716, 719, 720 and 722 - 725 are obtained in the same manner as in Example 769.

Example 770

To a suspension of 5-methylamino-1-[4-(2-methylbenzoylamino)benzoyl]-2,3,4,5-tetrahydro-1H-benzazepine (3 g) in methanol (30 ml) are added potassium carbonate (1.5 g) and epichlorohydrine (5.7 ml), and the mixture is refluxed for 3 hours. The solvent is distilled off and to the resulting residue is added water and extracted three times with dichloromethane. The extract is washed with saturated saline solution and dried over magnesium sulfate. The resulting residue is purified by silica gel column chromatography (eluent; dichloromethane : methanol = 80 : 1) to give 5-(N-methyl-N-oxiranylmethylamino)-1-[4-(2-methylbenzoylamino)benzoyl]-2,3,4,5-tetrahydro-1H-benzazepine (C; 1.92 g) and 5-[N-methyl-N-(2-hydroxy-3-methoxypropyl)amino]-1-[4-(2-methylbenzoylamino)benzoyl]-2,3,4,5-tetrahydro-1H-benzazepine (D; 0.38 g).

(C); Colorless needles (recrystallization from ethyl acetate)

m.p. 239 - 240°C

(D); Colorless amorphous

$^1$H-NMR($CDCl_3$) δ ; 1.35-4.55 (22H, m), 6.3-7.8 (13H, m)

Example 771

5-[N-Methyl-N-oxiranylmethylamino)-1-[4-(2-methylbenzoylamino)benzoyl]-2,3,4,5-tetrahydro-1H-benzazepine (0.5 g) is dissolved in methanol (10 ml) and thereto is added diethylamine (0.13 ml). The mixture is refluxed for 3 hours. After completion of the reaction, the solvent is distilled off and the resulting residue is purified by silica gel column chromatography

(eluent; dichloromethane : methanol = 30 : 1 → dichloromethane : methanol : aqueous ammonia = 9 : 1 : 0.1 )

to give 5-[N-methyl-N-(2-hydroxy-3-diethylaminopropyl)amino]-1-[4-(2-methylbenzoylamino)benzoyl]-2,3,4,5-tetrahydro-1H-benzazepine (0.38 g) as colorless amorphous.

$^1$H-NMR($CDCl_3$) δ ; 1.10 (6H, t, J=7 Hz), 1.35-5.1 (23H, m), 6.55-7.8 (13H, m)

Example 772

A solution of 5-hydroxyimino-1-[4-(2-chlorobenzoylamino)benzoyl]-2,3,4,5-tetrahydro-1H-benzazepine (1.06 g) in acetic anhydride (10 ml) and pyridine (10 ml) is stirred at room temperature overnight. After completion of the reaction, the reaction solution is concentrated. To the resulting residue is added water and the mixture is extracted with dichloromethane. The extract is washed with saturated saline solution and dried over magnesium sulfate. The solvent is distilled

off and the resulting residue is purified by silica gel column chromatography (eluent; dichloromethane : methanol = 80 : 1), and recrystallized from ethanol/petroleum ether to give 5-acetyloxyimino-1-[4-(2-chlorobenzoylamino)benzoyl]-2,3,4,5-tetrahydro-1H-benzazepine (0.75 g) as colorless prisms, m.p. 142 - 144°C.

Example 773

Using the suitable starting materials, the compounds of the above Examples 671 and 672 are obtained in the same manner as in Example 380.

Example 774

Using the suitable starting materials, the compounds of the above Examples 674, 699, 700, 706, 718 and 730 are obtained in the same manner as in Example 384.

Example 775

Using the suitable starting materials, the compounds of the above Examples 654 - 672, 675, 677 - 687, 691 - 695, 697, 698, 701 - 705, 707, 708, 712, 713, 715, 716 and 719 - 725 are obtained in the same manner as in Example 390.

Example 776

Using the suitable starting materials, the compounds of the above Examples 654 - 672, 675, 677 - 679, 691 - 693, 698, 701 - 705, 707, 708, 712, 713, 715, 716 and 719 - 725 are obtained in the same manner as in Example 388.

Example 777

Using the suitable starting materials, the compounds of the above Examples 705, 706 and 708 are obtained in the same manner as in Example 394.

Example 778

Using the suitable starting materials, the compound or the above Example 671 is obtained in the same manner as in Example 397.

Example 779

Using the suitable starting materials, the compound of the above Example 672 is obtained in the same manner as in Example 402.

Example 780

Using the suitable starting materials, the compound of the above Example 726 is obtained in the same manner as in Example 634.

Example 781

Using the suitable starting materials, the compound of the above Example 740 is obtained in the same manner as in Examples 638 and 640.

Example 782

Using the suitable starting materials, the compound of the above Example 689 is obtained in the same manner as in Example 643.

Example 783

Using the suitable starting materials, the compound of the above Example 690 is obtained in the same manner as in Example 644.

Example 784

Using the suitable starting materials, the following compound is obtained in the same manner as in Examples 1, 382, 388 and 390.

5-Dimethylamino-1-[4-(2-methylbenzoylamino)benzoyl]-2,3,4,5-tetrahydro-1H-benzazepine hydrochloride, colorless needles (recrystallized from ethanol/water), m.p. 233 - 237°C

Reference Example 13

Using the suitable starting materials, the following compounds are obtained in the same manner as in Reference Example 1.

5-(2-Chloroacetyloxy)-1-(4-nitrobenzoyl)-2,3,4,5-tetrahydro-1H-benzazepine, white powder, m.p. 156 - 159°C (recrystallized from ethyl acetate/n-hexane)

5-(2-Dimethylaminoacetyloxy)-1-(4-nitrobenzoyl)-2,3,4,5-tetrahydro-1H-benzazepine, white powder, m.p. 108 - 109°C (recrystallized from ethyl acetate/n-hexane)

5-Oxo-7-chloro-1-(4-nitrobenzoyl)-2,3,4,5-tetrahydro-1H-benzazepine, white powder, m.p. 157.5 - 159.5°C (recrystallized from diethyl ether/dichloromethane)

5-Oxo-8-chloro-1-(4-nitrobenzoyl)-2,3,4,5-tetrahydro-1H-benzazepine, white powder, m.p. 151.5 - 153.5°C (recrystallized from diethyl ether/dichloromethane)

Reference Example 14

Using the suitable starting materials, the following compounds are obtained in the same manner as in Reference Example 2.

5-(2-Dimethylaminoacetyloxy)-1-(4-aminobenzoyl)-2,3,4,5-tetrahydro-1H-benzazepine, colorless amorphous

$^1$H-NMR($CDCl_3$) δ ; 1.63-1.98 (2H, m), 1.98-2.25 (1H, m), 2.27 (3H, s), 2.43 (3H, s), 2.65-3.23 (2H, m), 3.38 (2H, s), 3.67 (2H, brs), 4.77-5.28 (1H, m), 6.04-6.31 (1H, m), 6.31-6.56 (2H, m), 6.58-6.86 (1H, m), 6.86-7.46 (5H, m)

5-Oxo-7-chloro-1-(4-aminobenzoyl)-2,3,4,5-tetrahydro-1H-benzazepine, white powder, m.p. 193 - 193.5°C (recrystallized from diethyl ether/dichloromethane)

5-Oxo-8-chloro-1-(4-aminobenzoyl)-2,3,4,5-tetrahydro-1H-benzazepine, white powder, m.p. 171 - 174°C (recrystallized from diethyl ether/dichloromethane)

Reference Example 15

Using the suitable starting materials, the following compounds are obtained in the same manner as in Reference Example 1.

5-Dimethylaminocarbonylmethoxy-1-(4-nitrobenzoyl)-2,3,4,5-tetrahydro-1H-benzazepine, white powder, m.p. 129 - 131°C (recrystallized from ethyl acetate/n-hexane)

6-Oxo-1-(4-nitrobenzoyl)-1,2,3,4,5,6-hexahydrobenzazocine, yellow needles

$^1$H-NMR($CDCl_3$) δ ; 1.65-2.3 (4H, m), 2.5-5.2 (4H, m), 6.7-6.9 (1H, m), 7.27-7.5 (4H, m), 7.90-8.15 (3H, m)

6-Chloro-5-oxo-1-(4-nitrobenzoyl)-2,3,4,5-tetrahydro-1H-benzazepine, white powder, m.p. 198 - 202°C (recrystallized from dichloromethane/diethyl ether)

Reference Example 16

Using the suitable starting materials, the following compounds are obtained in the same manner as in Reference Example 2.

5-Dimethylaminocarbonylmethoxy-1-(4-aminobenzoyl)-2,3,4,5-tetrahydro-1H-benzazepine, colorless amorphous

$^1$H-NMR($CDCl_3$) δ ; 1.52-2.10 (3H, m), 2.10-3.20 (2H, m), 2.97 (3H, s), 3.05 (3H, s), 4.03-4.48 (2H, m), 4.50-5.35 (2H, m), 6.26-6.57 (2H, m), 6.57-6.88 (1H, m), 6.88-7.76 (5H, m)

6-Oxo-1-(4-aminobenzoyl)-1,2,3,4,5,6-hexahydrobenzazocine, light yellow amorphous

$^1$H-NMR ($CDCl_3$) δ ; 1.7-2.2 (4H, m), 2.5-5.2 (6H, m), 6.42 (2H, d, J=8.7 Hz), 6.75-6.9 (1H, m), 7.05-7.4 (4H, m), 7.95-8.1 (1H, m)

6-Chloro-5-oxo-1-(4-aminobenzoyl)-2,3,4,5-tetrahydro-1H-benzazepine, white powder, m.p. 166 - 169°C (recrystallized from dichloromethane/diethyl ether)

9-Chloro-5-oxo-1-(4-aminobenzoyl)-2,3,4,5-tetrahydro-1H-benzazepine, yellow powder, m.p. 192.5 - 195°C (recrystallized from dichloromethane/diethyl ether)

Reference Example 17

5-Dimethylamino-1-(2-methyl-4-nitrobenzoyl)-2,3,4,5-tetrahydro-1H-benzazepine (86.0 g) is dissolved in ethanol (800 ml), and thereto is added platinum oxide (10 g). The mixture is subjected to hydrogenation at ordinary temperature under atmospheric pressure of hydrogen for 4 hours. The catalyst is removed by filtration, and the solvent is distilled off. The resulting residue is purified by silica gel column chromatography

(eluent; dichloromethane : methanol = 200 : 1 → 100 : 1),

and further purified by silica gel thin layer chromatography (developer; chloroform : methanol = 10 : 1), and recrystallized from methanol/diethyl ether to give 5-dimethylamino-1-(2-methyl-4-aminobenzoyl)-2,3,4,5-tetrahydro-1H-benzazepine (G) (Rf: 0.52, 27.4 g) and 5-dimethylamino-1-(2-methyl-4-amino-benzoyl)-2,3,4,5-tetrahydro-1H-benzazepine (H) (Rf: 0.48, 12.3 g).

(G) : White powder

M.p. 154 - 156°C

$[\alpha]_D^{22}$ = 0° (c=1.0, chloroform)

$^1$H-NMR ($CDCl_3$) δ ; 1.10-1.50 (1H, m), 1.50-2.00 (1H, m), 2.00-2.35 (11H, m), 2.90-5.18 (5H, m), 6.00-6.76 (3H, m), 6.81-7.64 (4H, m)

(H): White powder

M.p. 169.5 - 170°C

$[\alpha]_D^{22}$ = 0° (c=1.5, chloroform)

$^1$H-NMR ($CDCl_3$) δ ; 1.11 - 2.90 (13H, m), 2.91-5.23 (5H, m), 6.15-6.53 (1H, m), 6.57-7.62 (6H, m)

Using the suitable starting materials, the compounds of the following Table 5 are obtained in the same manner as in above Examples 1 and 382.

Table 5

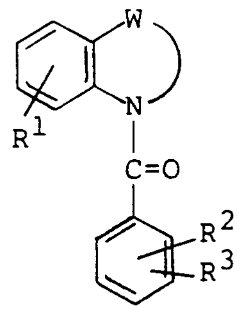

Example 785

Structure

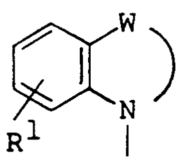 : 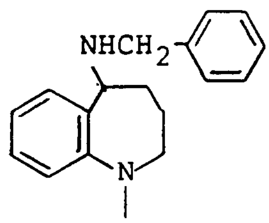

$R^2$: H

$R^3$: 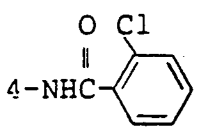

Crystalline form: Colorless needles

Recrystallization solvent: Ethanol

Melting Point: 174 - 175°C

Form: Free

Example 786

Structure

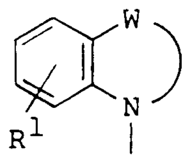 : 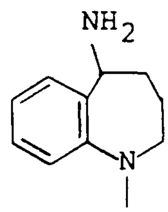

$R^2$: H

$R^3$: 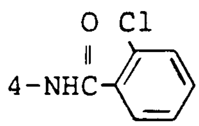

Crystalline form: Colorless prisms

Recrystallization solvent: Ethanol/diethyl ether

Melting Point: 176 - 178°C

Form: Free

Example 787

Structure

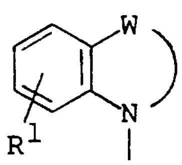 : 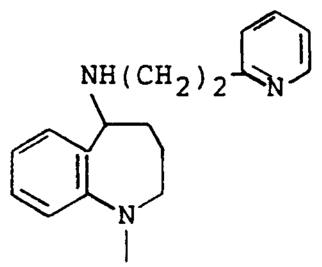

$R^2$: H

$R^3$: 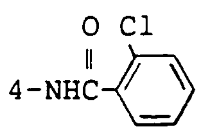

Crystalline form: Colorless prisms

Recrystallization solvent: Ethyl acetate/petroleum ether

Melting Point: 154.5 - 155°C

Form: Free

Example 788

Structure

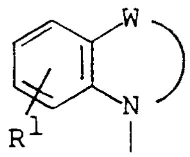 : 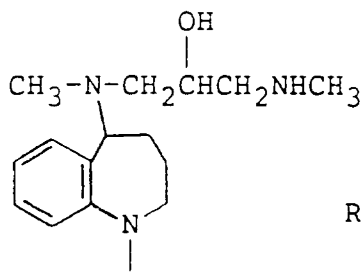

$R^2$: H

$R^3$: 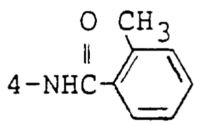

Crystalline form: Colorless amorphous

NMR analysis: 138)

Form: Free

Example 789

Structure

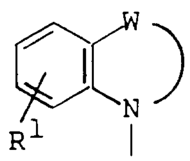 : 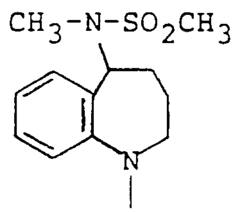

$R^2$: H

$R^3$: 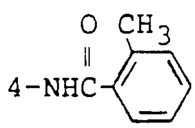

Crystalline form: Colorless scales

Recrystallization solvent: Ethanol

Melting Point: 197 - 198°C

Form: Free

Example 790

Structure 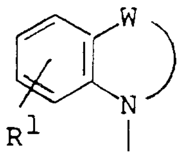 : 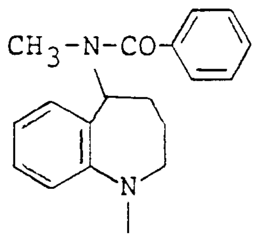 $R^2$: H

$R^3$: 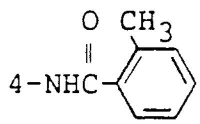

Crystalline form: Colorless needles

Recrystallization solvent: Ethanol

Melting Point: 248 - 249°C

Form: Free

Example 791

Structure 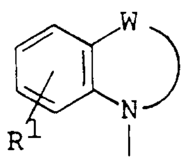 : 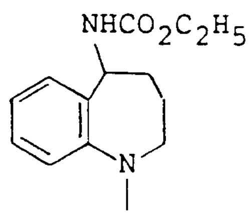 $R^2$: H

$R^3$: 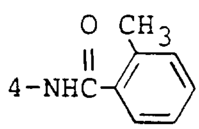

Crystalline form: Colorless needles

Recrystallization solvent: Ethanol/n-hexane

Melting Point: 162 - 163°C

Form: Free

Example 792

Structure

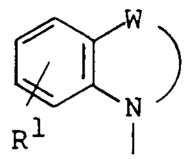 : 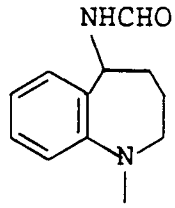 $R^2$: H

$R^3$: 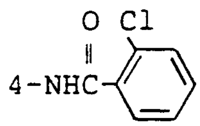

Crystalline form: Colorless prisms

Recrystallization solvent: Ethanol/petroleum ether

Melting Point: 235 - 236.5°C

Form: Free

Example 793

Structure

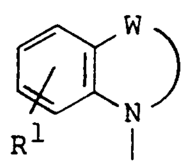 : 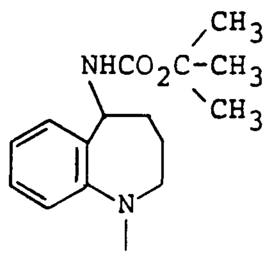 $R^2$: H

$R^3$: 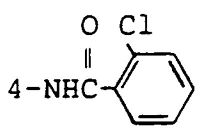

Crystalline form: Colorless amorphous

NMR analysis: 139)

Form: Free

Example 794

Structure

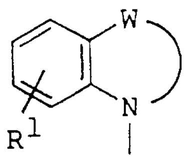 : 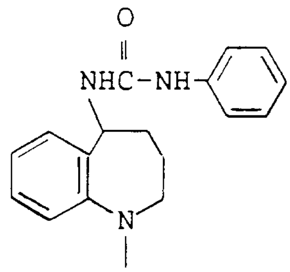

$R^2$: H

$R^3$: 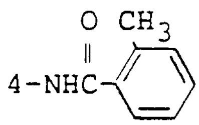

Crystalline form: Colorless prisms

Recrystallization solvent: Dioxane

Melting Point: 269 - 271°C

Form: Free

Example 795

Structure

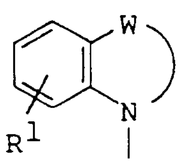 : 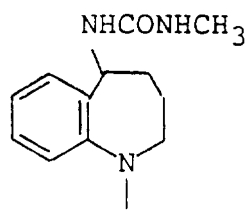

$R^2$: H

$R^3$: 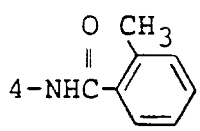

Crystalline form: Colorless prisms

Recrystallization solvent: Dimethylformamide

Melting Point: 286 - 287°C

Form: Free

Example 796

Structure 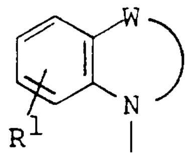 : 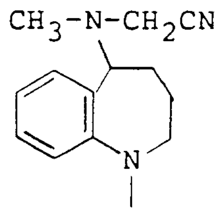 $R^2$: H

$R^3$: 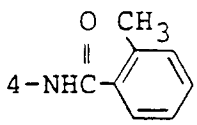

Crystalline form: Colorless needles

Recrystallization solvent: Acetonitrile

Melting Point: 227 - 228°C

Form: Free

Example 797

Structure 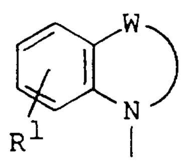 : 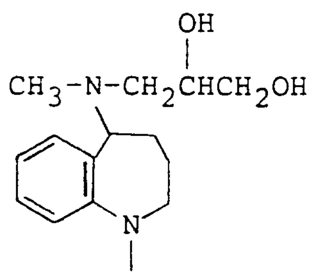 $R^2$: H

$R^3$: 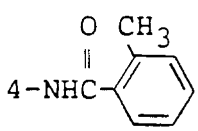

Crystalline form: Colorless amorphous

NMR analysis: 140)

Form: Free

Example 798

Structure

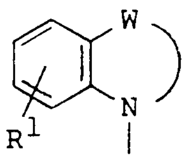

:

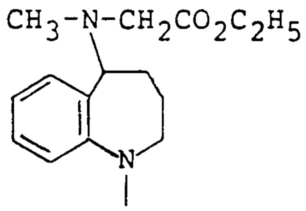

$R^2$: H

$R^3$:

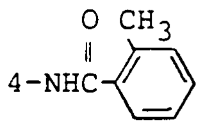

Crystalline form: Colorless prisms

Recrystallization solvent: Ethyl acetate/petroleum ether

Melting Point: 167 - 168°C

Form: Free

Example 799

Structure

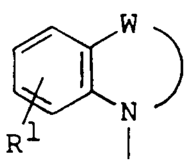

:

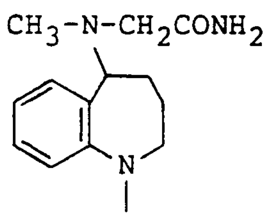

$R^2$: H

$R^3$:

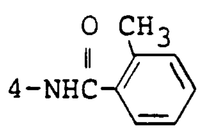

Crystalline form: Colorless amorphous

NMR analysis: 141)

Example 800

Structure

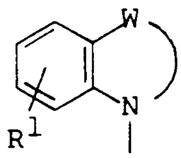 : 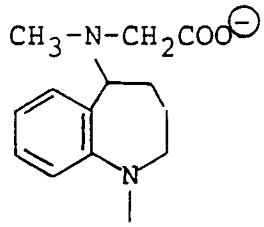

$R^2$: H

$R^3$: 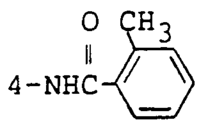

Crystalline form: Colorless needles

Recrystallization solvent: Diethyl ether

Melting Point: 164 - 171°C

Form: $K^{\oplus}$

Example 801

Structure

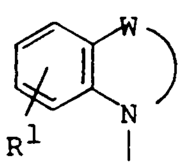 : 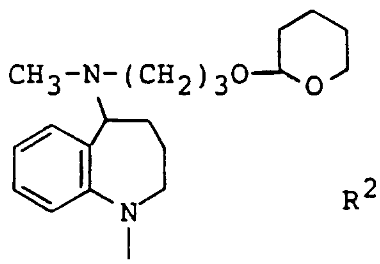

$R^2$: H

$R^3$: 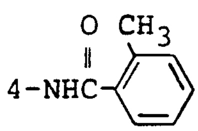

Crystalline form: Colorless amorphous

NMR analysis: 142)

Form: Free

Example 802

Structure

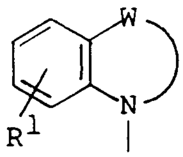 : 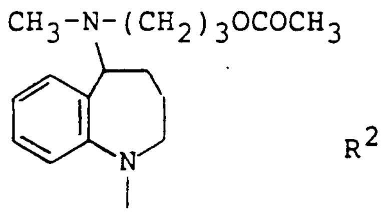 $R^2$: H

$R^3$: 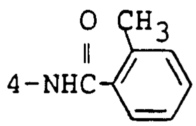

Crystalline form: Colorless amorphous

NMR analysis: 143)

Form: Free

Example 803

Structure

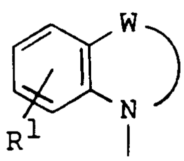 : 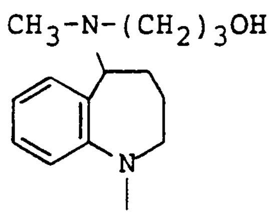 $R^2$: H

$R^3$: 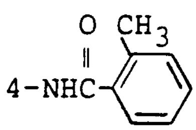

Crystalline form: Colorless amorphous

NMR analysis: 144)

Form: Free

Example 804

Structure

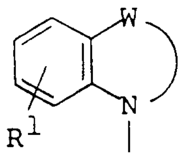 : 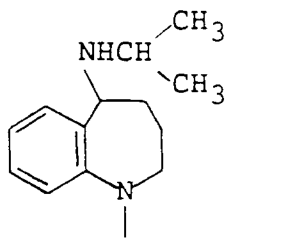

$R^2$: H

$R^3$: 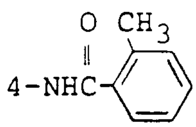

Crystalline form: Colorless amorphous

NMR analysis: 145)

Form: Free

---

Example 805

Structure

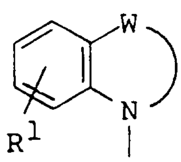 : 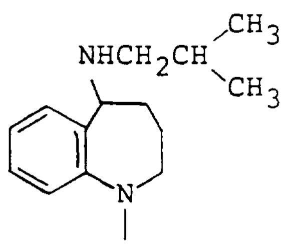

$R^2$: H

$R^3$: 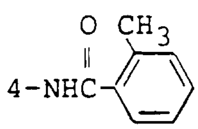

Crystalline form: Colorless needles

Recrystallization solvent: Ethanol

Melting Point: 207 - 208°C

Form: Free

Example 806

Structure

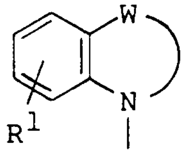 : 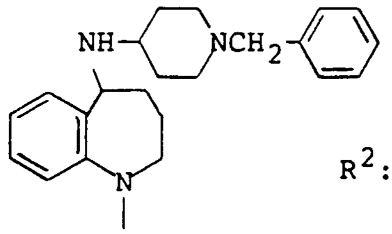

$R^2$: H

$R^3$: 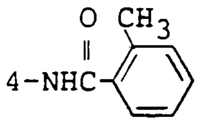

Crystalline form: White powder

Recrystallization solvent: Dichloromethane/diethyl ether

Melting Point: 187 - 189°C

Form: Free

Example 807

Structure

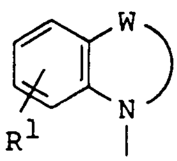 : 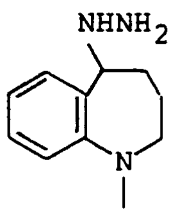

$R^2$: H

$R^3$: 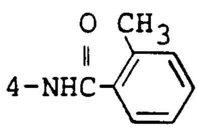

Crystalline form: Colorless prisms

Recrystallization solvent: Ethanol/petroleum ether

Melting Point: 217 - 218°C

Form: Free

Example 808

Structure

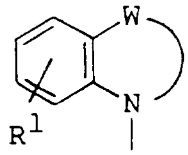 : 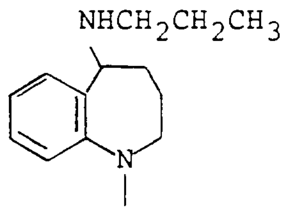

$R^2$: H

$R^3$: 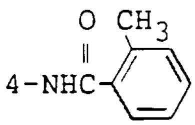

Crystalline form: Colorless needles

Recrystallization solvent: Ethyl acetate

Melting Point: 170 - 171°C

Form: Free

---

Example 809

Structure

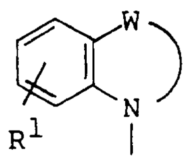 : 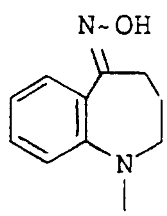

$R^2$: H

$R^3$: 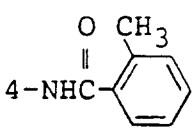

Crystalline form: Colorless prisms

Recrystallization solvent: Ethanol

Melting Point: 239.5 - 241°C

Form: Free

Example 810

Structure

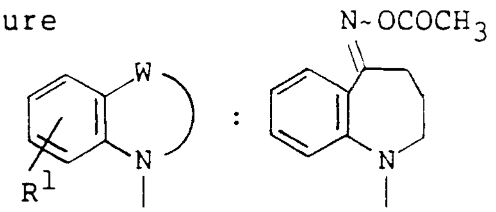

$R^2$: H

$R^3$: 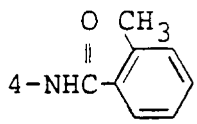

Crystalline form: Colorless needles

Recrystallization solvent: Ethanol

Melting Point: 190 - 191°C

Form: Free

Example 811

Structure

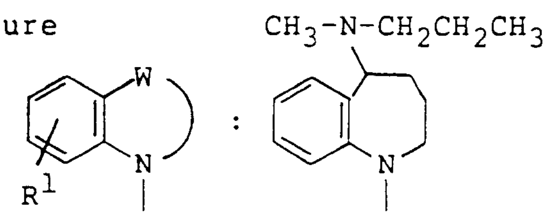

$R^2$: H

$R^3$: 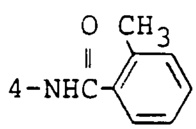

Crystalline form: Colorless prisms

Recrystallization solvent: Diethyl ether

Melting Point: 163 - 163.5°C

Form: Free

Example 812

Structure

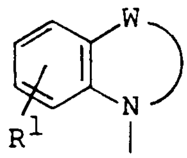

:

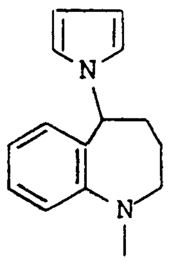

$R^2$: H

$R^3$: 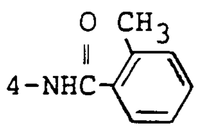

Crystalline form: Colorless prisms

Recrystallization solvent: Ethyl acetate/n-hexane

Melting Point: 208 - 210°C

Form: Free

Example 813

Structure

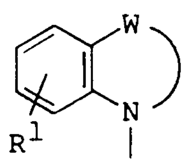

:

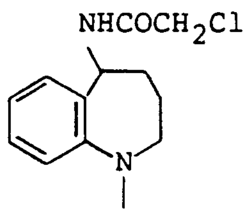

$R^2$: H

$R^3$: 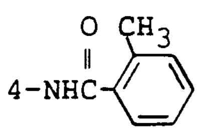

Crystalline form: White powder

NMR analysis: 146)

Form: Free

Example 814

Structure

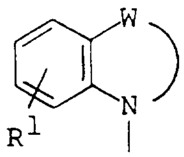 : 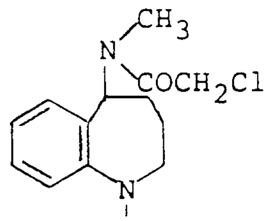 $R^2$: H

$R^3$: 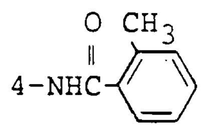

Crystalline form: Colorless amorphous

NMR analysis: 147)

Form: Free

Example 815

Structure

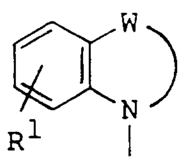 : 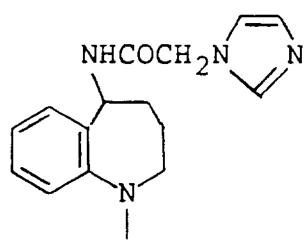 $R^2$: H

$R^3$: 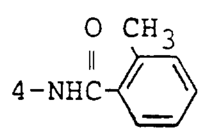

Crystalline form: Colorless needles

Recrystallization solvent: Ethanol/n-hexane

Melting Point: 250 - 252°C

Form: Free

Example 816

Structure

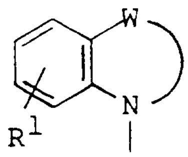 : 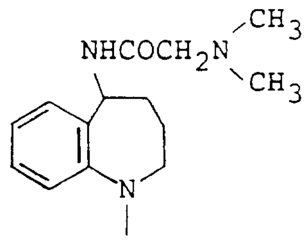

$R^2$: H

$R^3$: 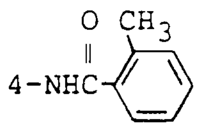

Crystalline form: Colorless prisms

Recrystallization solvent: Ethyl acetate

Melting Point: 214 - 216°C

Form: Free

Example 817

Structure

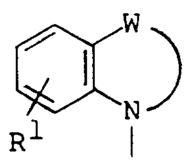 : 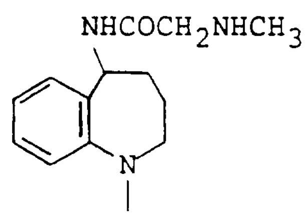

$R^2$: H

$R^3$: 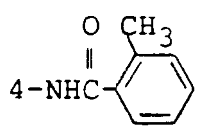

Crystalline form: Colorless prisms

Recrystallization solvent: Ethanol/n-hexane

Melting Point: 243 - 245°C

Form: Free

Example 818

Structure

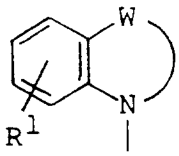 : 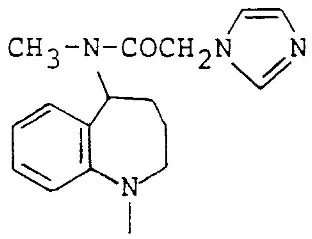

$R^2$: H

$R^3$: 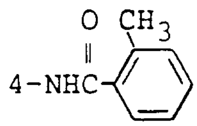

Crystalline form: Colorless prisms

Recrystallization solvent: Diethyl ether

Melting Point: 159 - 162°C

Form: Free

Example 819

Structure

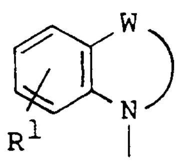 : 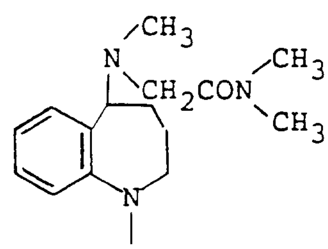

$R^2$: H

$R^3$: 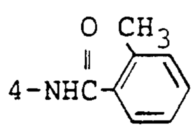

Crystalline form: Colorless amorphous

NMR analysis: 148)

Form: Free

Example 820

Structure

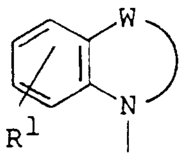 : 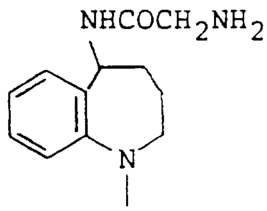

$R^2$: H

$R^3$: 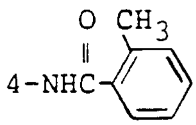

Crystalline form: Colorless prisms

Recrystallization solvent: Ethyl acetate

Melting Point: 287 - 289°C

Form: Free

Example 821

Structure

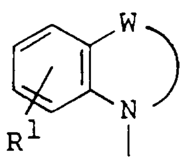 : 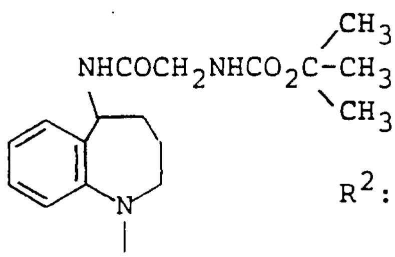

$R^2$: H

$R^3$: 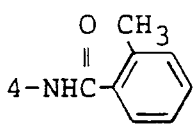

Crystalline form: Colorless prisms

Recrystallization solvent: Diethyl ether

Melting Point: 170 - 171°C

Form: Free

Example 822

Structure

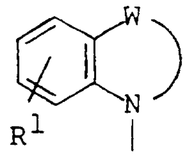 : 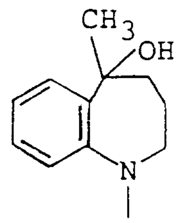

$R^2$: H

$R^3$: 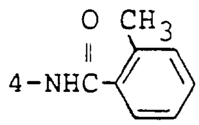

Crystalline form: White powder

Recrystallization solvent: Ethyl acetate/n-hexane

Melting Point: 204 - 205°C

Form: Free

Example 823

Structure

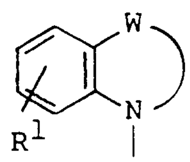 : 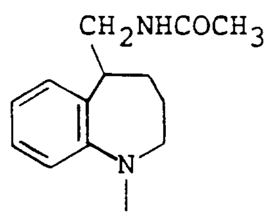

$R^2$: H

$R^3$: 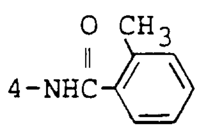

Crystalline form: White powder

Recrystallization solvent: Ethyl acetate/n-hexane

Melting Point: 273 - 273.5°C

Form: Free

Example 824

Structure

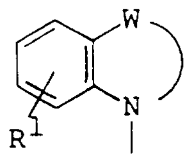 : 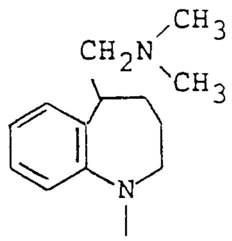

$R^2$: H

$R^3$: 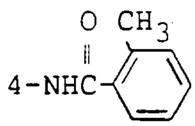

Crystalline form: Colorless amorphous

NMR analysis: 149)

Form: Free

Example 825

Structure

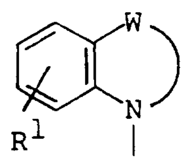 : 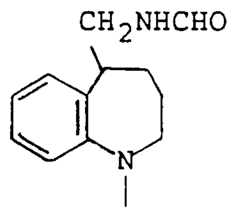

$R^2$: H

$R^3$: 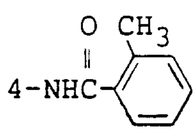

Crystalline form: White powder

Recrystallization solvent: Ethyl acatete/n-hexane

Melting Point: 240 - 241°C

Form: Free

Example 826

Structure

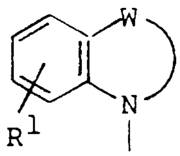 : 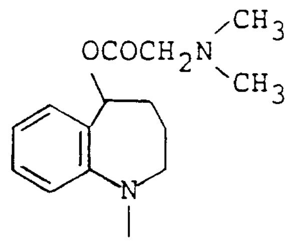

$R^2$: H

$R^3$: 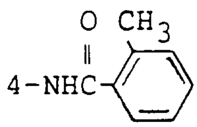

Crystalline form: White powder

Recrystallization solvent: Acetonitrile/ethanol

Melting Point: 231 - 232°C

Form: Free

---

Example 827

Structure

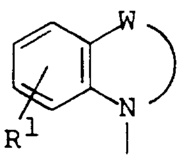 : 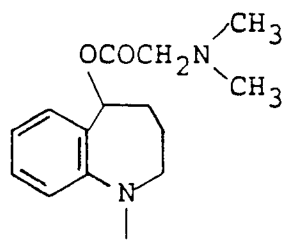

$R^2$: H

$R^3$: 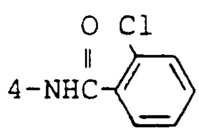

Crystalline form: White powder

Recrystallization solvent: Acetonitrile/ethanol

Melting Point: 222 - 224°C

Form: Free

Example 828

Structure

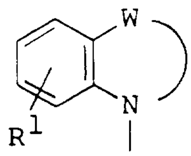

:

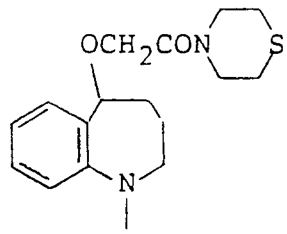

$R^2$: H

$R^3$: 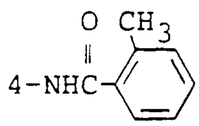

Crystalline form: White powder

Recrystallization solvent: Ethyl acetate/n-hexane

Melting Point: 235 - 237°C

Form: Free

Example 829

Structure

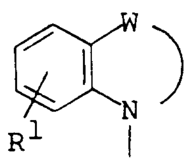

:

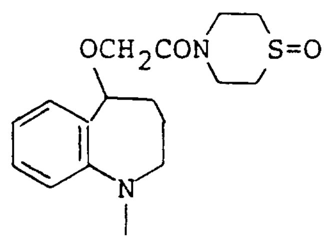

$R^2$: H

$R^3$: 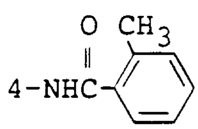

Crystalline form: Colorless amorphous

NMR analysis: 150)

Form: Free

Example 830

Structure

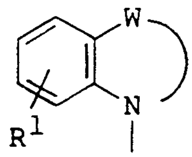 : 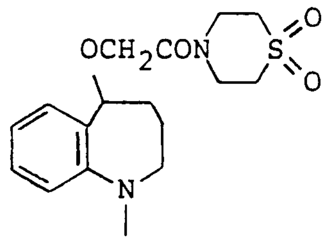

$R^2$: H

$R^3$: 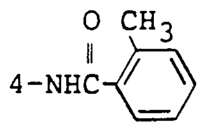

Crystalline form: Colorless amorphous

NMR analysis: 151)

Form: Free

Example 831

Structure

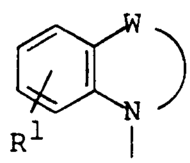 : 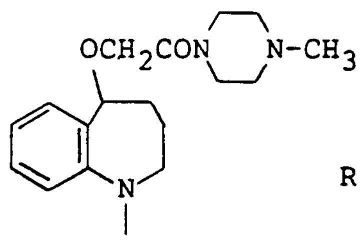

$R^2$: H

$R^3$: 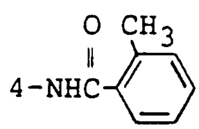

Crystalline form: Colorless amorphous

NMR analysis: 152)

Form: Free

Example 832

Structure

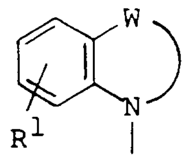 : 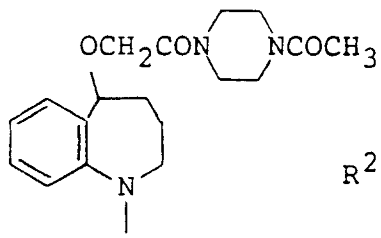 $R^2$: H

$R^3$: 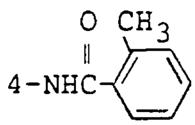

Crystalline form: Colorless amorphous

NMR analysis: 153)

Form: Free

Example 834

Structure

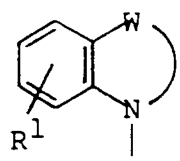 : 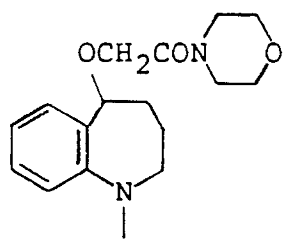 $R^2$: H

$R^3$: 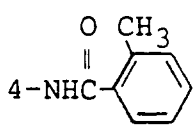

Crystalline form: White powder

Recrystallization solvent: Ethyl acetate/n-hexane

Melting Point: 247 - 248°C

Form: Free

Example 835

Structure

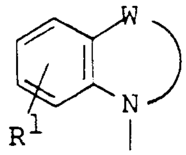

:

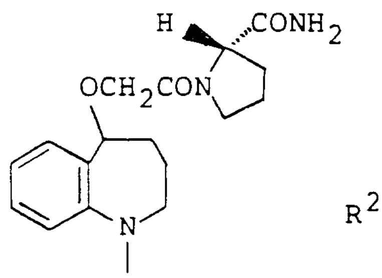

$R^2$: H

$R^3$: 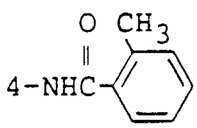

Crystalline form: Colorless amorphous

NMR analysis: 154)

Form: Free

Example 836

Structure

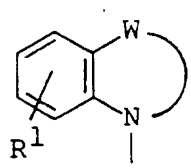

:

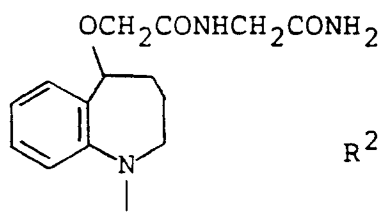

$R^2$: H

$R^3$: 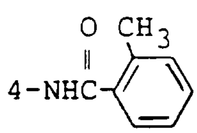

Crystalline form: Colorless amorphous

NMR analysis: 155)

Form: Free

Example 837

Structure

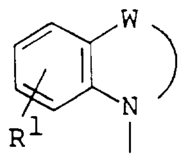 : 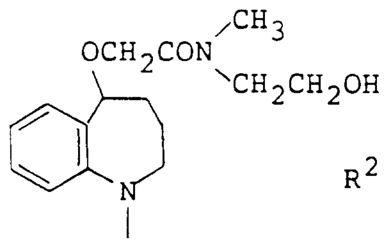

$R^2$: H

$R^3$: 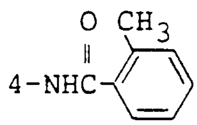

Crystalline form: Colorless amorphous

NMR analysis: 156)

Form: Free

Example 838

Structure

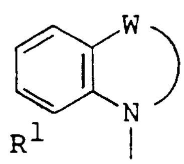 : 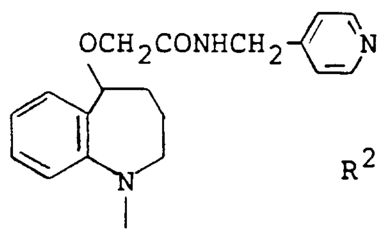

$R^2$: H

$R^3$: 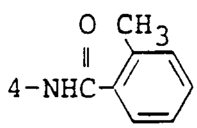

Crystalline form: Colorless amorphous

NMR analysis: 157)

Form: Free

Example 839

Structure

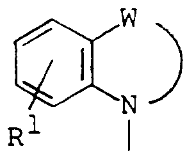 : 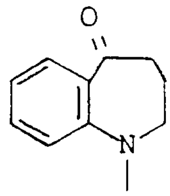

$R^2$: H

$R^3$: 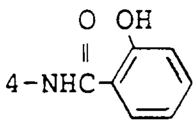

Crystalline form: White powder

Recrystallization solvent: Ethyl acetate/n-hexane

Melting Point: 234 - 235°C

Form: Free

Example 840

Structure

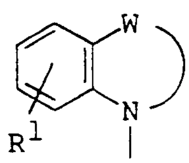 : 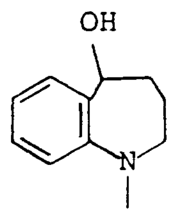

$R^2$: H

$R^3$: 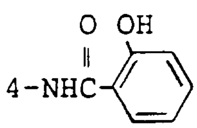

Crystalline form: White powder

Recrystallization solvent: Ethyl acetate/n-hexane

Melting Point: 234 - 235°C

Form: Free

Example 841

Structure

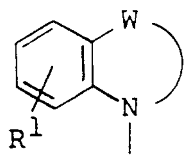 : 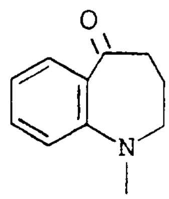

$R^2$: H

$R^3$: 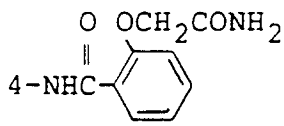

Crystalline form: White powder

Recrystallization solvent: Ethyl acetate/n-hexane

Melting Point: 226 - 228°C

Form: Free

Example 842

Structure

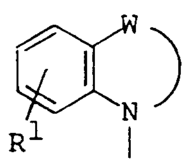 : 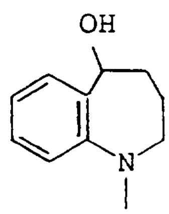

$R^2$: H

$R^3$: 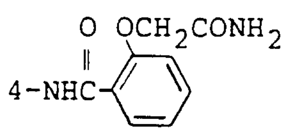

Crystalline form: White powder

Recrystallization solvent: Ethyl acetate/n-hexane

Melting Point: 230 - 231°C

Form: Free

Example 843

Structure

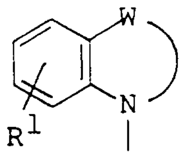 : 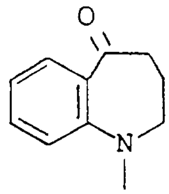 $R^2$: H

$R^3$: 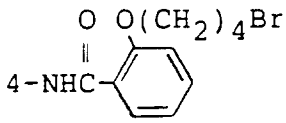

Crystalline form: White powder

Recrystallization solvent: Ethyl acetate/n-hexane

Melting Point: 186 - 188°C

Form: Free

---

Example 844

Structure

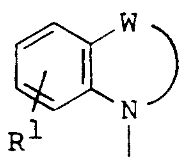 : 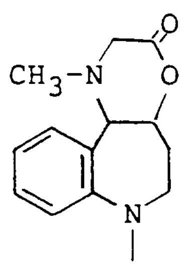 $R^2$: H

$R^3$: 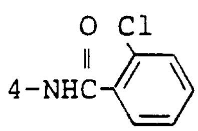

Crystalline form: Colorless prisms

Recrystallization solvent: Chloroform/methanol

Melting Point: 286 - 290°C

Form: Free

Example 845

Structure

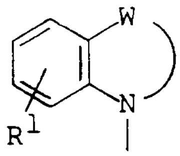 : 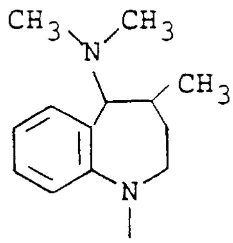 $R^2$: H

$R^3$: 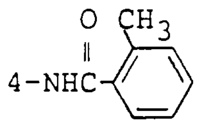

Crystalline form: Colorless needles

Recrystallization solvent: Ethanol

Melting Point: 186 - 188.5°C

Form: Free

Example 846

Structure

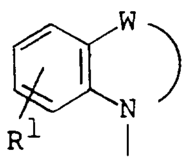 : 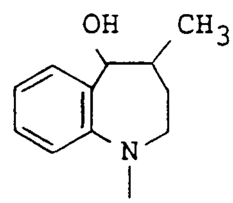 $R^2$: H

$R^3$: 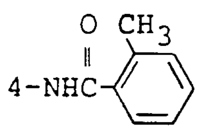

Crystalline form: Colorless prisms

Recrystallization solvent: Ethanol

Melting Point: 220 - 222°C

Form: Free

Example 847

Structure

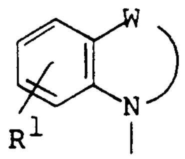
:
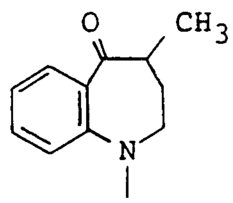

$R^2$: H

$R^3$: 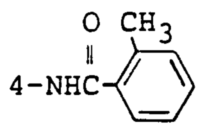

Crystalline form: White powder

NMR analysis: 158)

Form: Free

Example 848

Structure

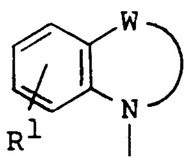
:
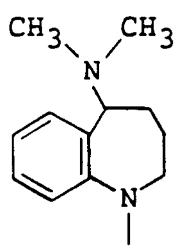

$R^2$: H

$R^3$: 4-$NHCOCH_2CONH_2$

Crystalline form: Colorless prisms

Recrystallization solvent: Dichloromethane/diethyl ether

Melting Point: 189 - 192°C

Form: Free

Example 849

Structure

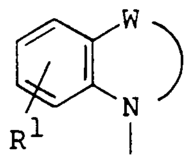 : 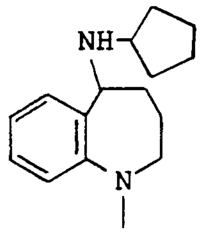

$R^2$: 2-Cl

$R^3$: 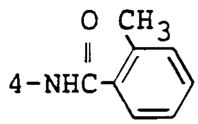

Crystalline form: Colorless amorphous

NMR analysis: 159)

Form: Free

Example 850

Structure

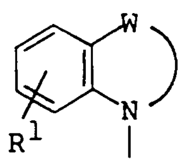 : 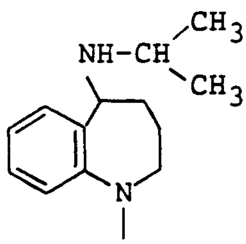

$R^2$: 2-Cl

$R^3$: 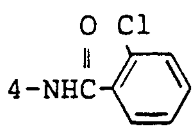

Crystalline form: White powder

Recrystallization solvent: Dichloromethane/diethyl ether

Melting Point: 207 - 209°C (decomposed)

Form: Free

Example 851

Structure

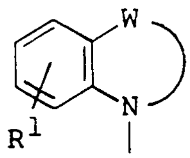 : 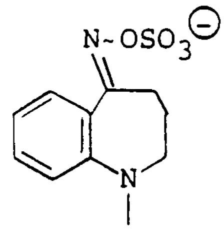

$R^2$: 2-Cl

$R^3$: 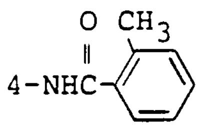

Crystalline form: White powder

NMR analysis: 160)

Form: $K^{+}$

Example 852

Structure

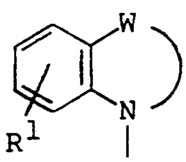 : 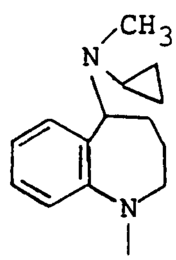

$R^2$: 2-Cl

$R^3$: 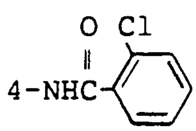

Crystalline form: White powder

Recrystallization solvent: Dichloromethane/diethyl ether

Melting Point: 193 - 194°C

Form: Free

Example 853

Structure 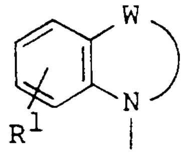 : 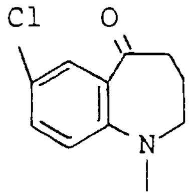 $R^2$: H

$R^3$: 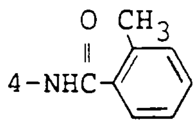

Crystalline form: White powder

Recrystallization solvent: Diethyl ether/dichloromethane

Melting Point: 185.5 - 186°C

Form: Free

Example 854

Structure 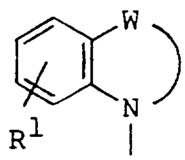 : 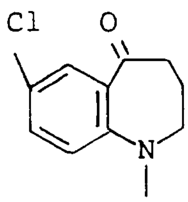 $R^2$: H

$R^3$: 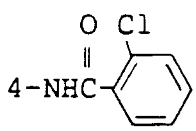

Crystalline form: White powder

Recrystallization solvent: Diethyl ether/dichloromethane

Melting Point: 223.5 - 226°C (decomposed)

Form: Free

Example 855

Structure

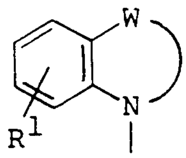
:
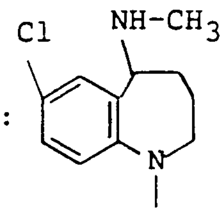

$R^2$: H

$R^3$:
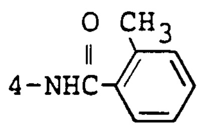

Crystalline form: Colorless amorphous

NMR analysis: 161)

Form: Free

Example 856

Structure

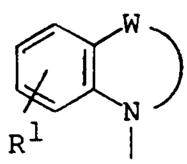
:
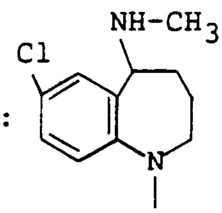

$R^2$: H

$R^3$:
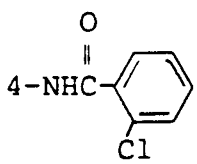

Crystalline form: White powder

Recrystallization solvent: Diethyl ether/dichloromethane

Melting Point: 225.5 - 227°C

Form: Free

Example 857

Structure

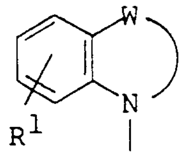 : 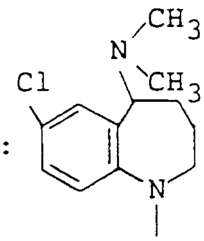

$R^2$: H

$R^3$: 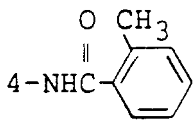

Crystalline form: White powder

Recrystallization solvent: Diethyl ether/dichloromethane

Melting Point: 212 - 214°C

Form: Free

Example 858

Structure

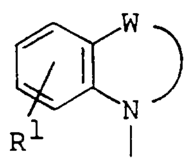 : 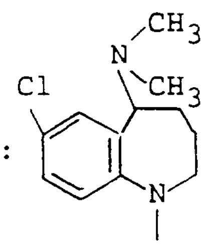

$R^2$: H

$R^3$: 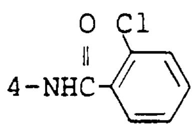

Crystalline form: White powder

Recrystallization solvent: Diethyl ether/dichloromethane

Melting Point: 230.5 - 233°C

Form: Free

Example 859

Structure

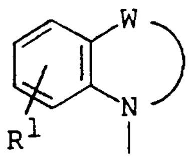
:
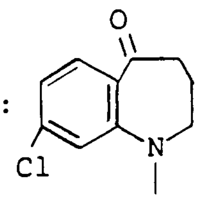

$R^2$: H

$R^3$: 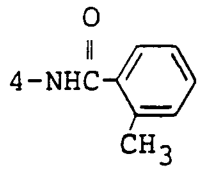

Crystalline form: White powder

Recrystallization solvent: Diethyl ether/dichloromethane

Melting Point: 212.5 - 215°C (decomposed)

Form: Free

Example 860

Structure

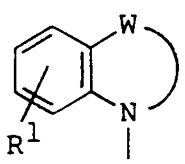
:
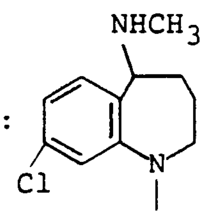

$R^2$: H

$R^3$: 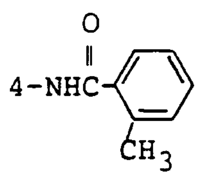

Crystalline form: White powder

Recrystallization solvent: Diethyl ether/dichloromethane

Melting Point: 192 - 194.5°C

Form: Free

Example 861

Structure

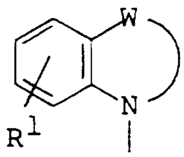

:

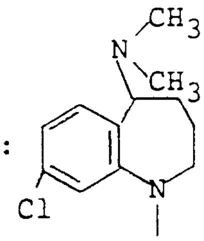

$R^2$: H

$R^3$:

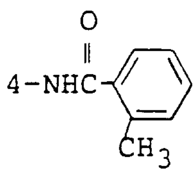

Crystalline form: White powder

Recrystallization solvent: Diethyl ether/dichloromethane

Melting Point: 175 - 177°C

Form: Free

Example 862

Structure

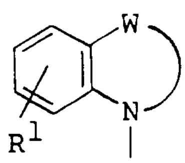

:

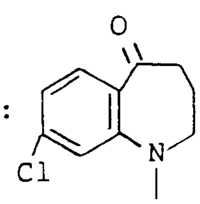

$R^2$: H

$R^3$:

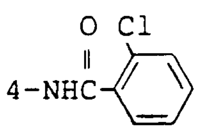

Crystalline form: White powder

Recrystallization solvent: Diethyl ether/dichloromethane

Melting Point: 208.5 - 209.5°C

Form: Free

Example 863

Structure

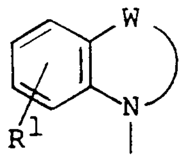 : 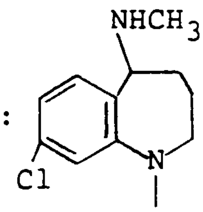

$R^2$: H

$R^3$: 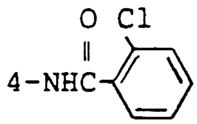

Crystalline form: White powder

Recrystallization solvent: Diethyl ether/dichloromethane

Melting Point: 191 - 193.5°C

Form: Free

Example 864

Structure

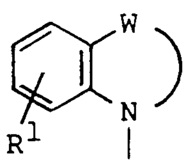 : 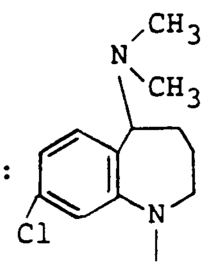

$R^2$: H

$R^3$: 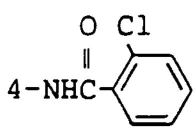

Crystalline form: White powder

Recrystallization solvent: Diethyl ether/dichloromethane

Melting Point: 204 - 205.5°C

Form: Free

Example 865

Structure

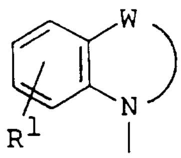 : 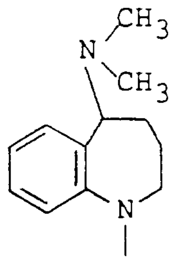 $R^2$: H

$R^3$: 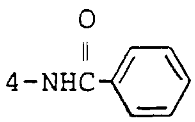

Crystalline form: Light yellow prisms

Recrystallization solvent: Ethanol

Melting Point: 221 - 223°C

Form: Free

Example 866

Structure

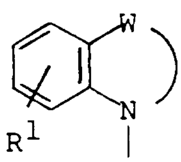 : 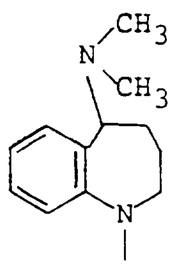 $R^2$: H

$R^3$: 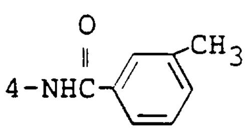

Crystalline form: Colorless prisms

Recrystallization solvent: Ethyl acetate

Melting Point: 171 - 173°C

Form: Free

Example 867

Structure

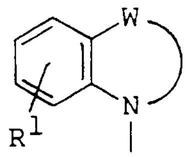 : 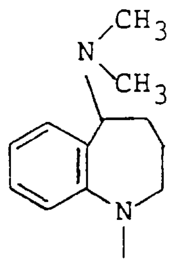

$R^2$: H

$R^3$: 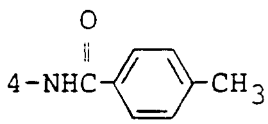

Crystalline form: Colorless prisms

Recrystallization solvent: Ethyl acetate

Melting Point: 185 - 187°C

Form: Free

Example 868

Structure

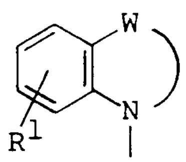 : 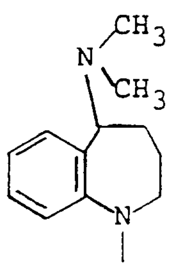

$R^2$: H

$R^3$: 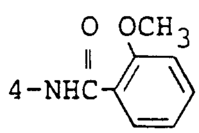

Crystalline form: Colorless prisms

Recrystallization solvent: Ethanol

Melting Point: 190 - 192°C

Form: Free

Example 869

Structure

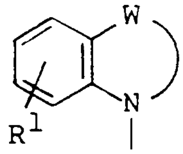 : 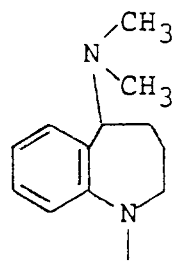

$R^2$: H

$R^3$: 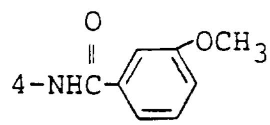

Crystalline form: Colorless prisms

Recrystallization solvent: Ethanol

Melting Point: 175- 177°C

Form: Free

Example 870

Structure

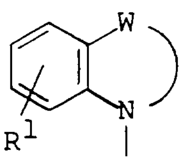 : 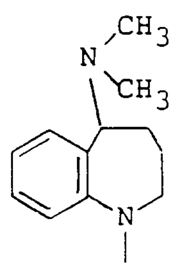

$R^2$: H

$R^3$: 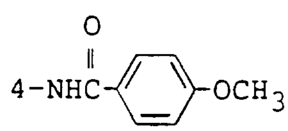

Crystalline form: Colorless powder

Recrystallization solvent: Ethyl acetate/n-hexane

Melting Point: 148 - 151°C

Form: Free

Example 871

Structure

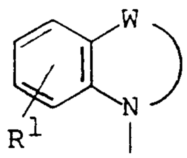 : 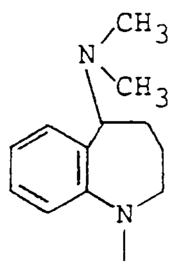

$R^2$: H

$R^3$: 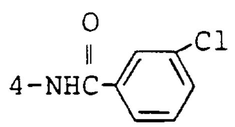

Crystalline form: Colorless prisms

Recrystallization solvent: Ethanol

Melting Point: 200 - 202°C

Form: Free

Example 872

Structure

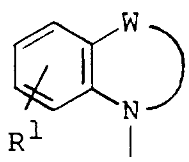 : 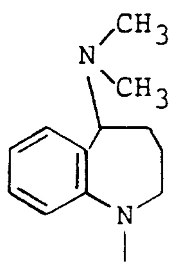

$R^2$: H

$R^3$: 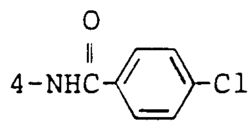

Crystalline form: Colorless prisms

Recrystallization solvent: Ethanol

Melting Point: 200 - 202°C

Form: Free

Example 873

Structure

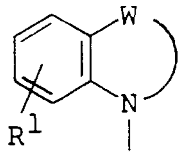 : 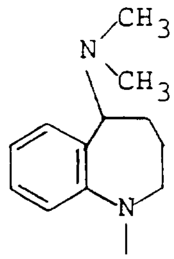

$R^2$: H

$R^3$: 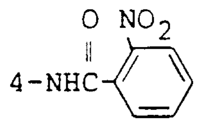

Crystalline form: Light yellow powder

Recrystallization solvent: Acetone

Melting Point: 235 - 238°C

Form: Free

Example 874

Structure

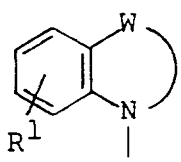 : 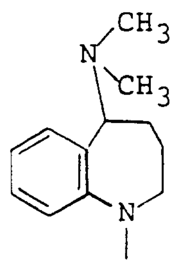

$R^2$: H

$R^3$: 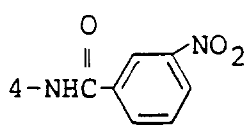

Crystalline form: Light yellow powder

Recrystallization solvent: Acetone

Melting Point: 198 - 201°C

Form: Free

Example 875

Structure

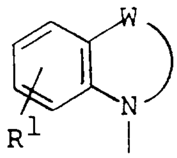 : 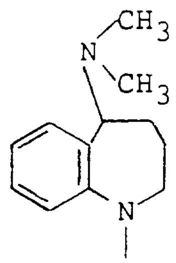

$R^2$: H

$R^3$: 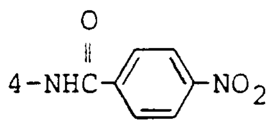

Crystalline form: Light yellow needles

Recrystallization solvent: Chloroform/ethyl acetate

Melting Point: 232 - 237°C

Form: Free

Example 876

Structure

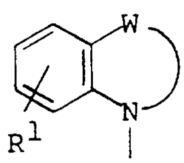 : 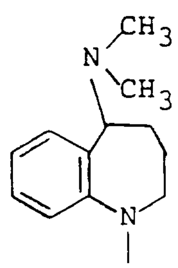

$R^2$: H

$R^3$: 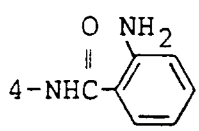

Crystalline form: Colorless prisms

Recrystallization solvent: Chloroform/ethyl acetate

Melting Point: 224 - 227°C

Form: Free

Example 877

Structure

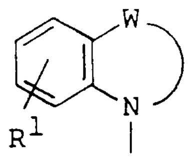 : 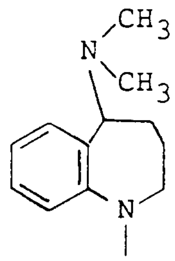

$R^2$: H

$R^3$: 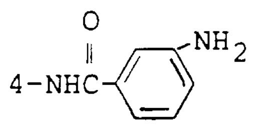

Crystalline form: Colorless prisms

Recrystallization solvent: Ethanol

Melting Point: 211 - 214°C

Form: Free

Example 878

Structure

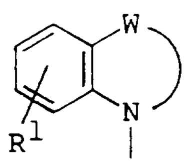 : 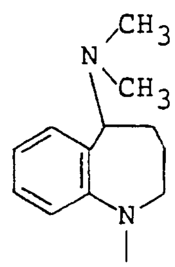

$R^2$: H

$R^3$: 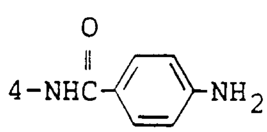

Crystalline form: Colorless powder

Recrystallization solvent: Dichloromethane/n-hexane

Melting Point: 238 - 243°C

Form: Free

Example 879

Structure

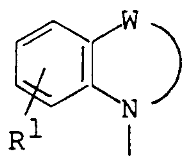 : 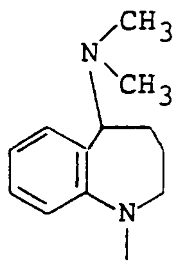 $R^2$: H

$R^3$: 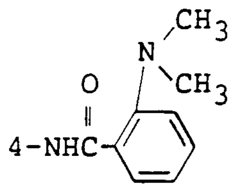

Crystalline form: Colorless amorphous

NMR analysis: 162)

Form: Free

---

Example 880

Structure

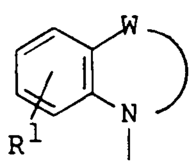 : 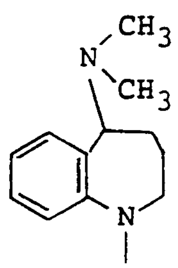 $R^2$: H

$R^3$: 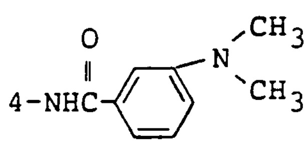

Crystalline form: Colorless amorphous

NMR analysis: 163)

Form: Free

Example 881

Structure

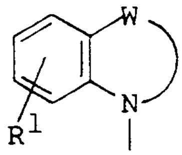

:

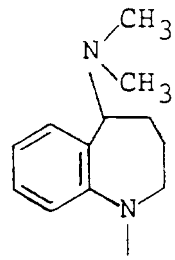

$R^2$: H

$R^3$: 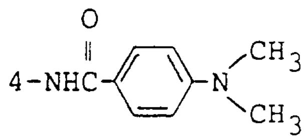

Crystalline form: Colorless prisms

Recrystallization solvent: Dichloromethane/diethyl ether

Melting Point: 198 - 202°C

Form: Free

Example 882

Structure

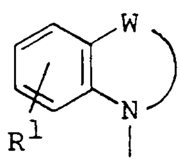

:

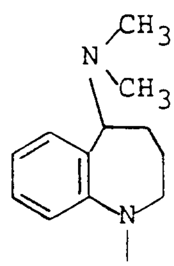

$R^2$: H

$R^3$: 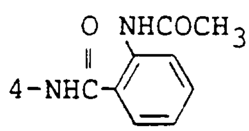

Crystalline form: Colorless prisms

Recrystallization solvent: Chloroform/ethyl acetate

Melting Point: 226 - 229°C

Form: Free

Example 883

Structure

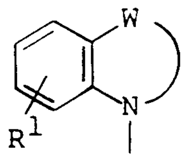 : 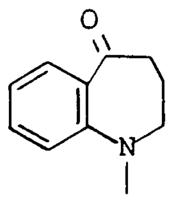

$R^2$: 2-$CH_3$

$R^3$: 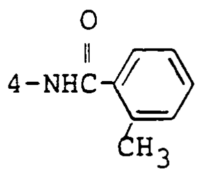

Crystalline form: White powder

Recrystallization solvent: Methanol/diethyl ether

Melting Point: 139 - 140°C

Form: Free

Example 884

Structure

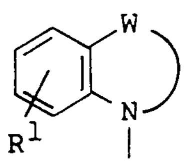 : 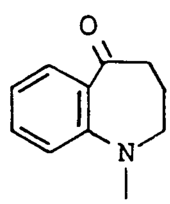

$R^2$: 2-$CH_3$

$R^3$: 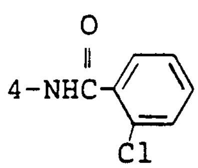

Crystalline form: White powder

Recrystallization solvent: Methanol/diethyl ether

Melting Point: 149 - 152°C

Form: Free

Example 885

Structure

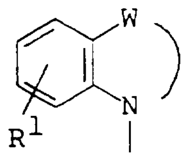 : 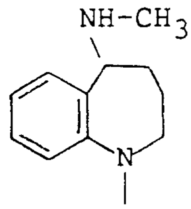

$R^2$: 2-$CH_3$

$R^3$: 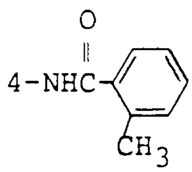

Crystalline form: White powder

Recrystallization solvent: Methanol/diethyl ether

Melting Point: 180.5 - 182°C

Form: Free

Example 886

Structure

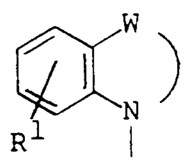 : 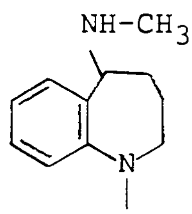

$R^2$: 2-$CH_3$

$R^3$: 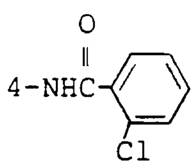

Crystalline form: White powder

Recrystallization solvent: Chloroform/diethyl ether

Melting Point: 211 - 214°C

Form: Free

Example 887

Structure

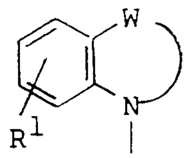 : 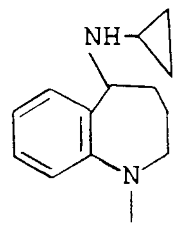  $R^2$: 2-$CH_3$

$R^3$: 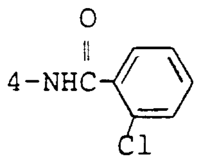

Crystalline form: White powder

Recrystallization solvent: Chloroform/diethyl ether

Melting Point: 171 - 174.5°C

Form: Free

Example 888

Structure

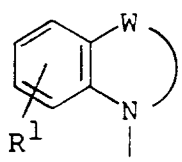 : 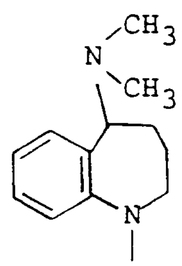  $R^2$: H

$R^3$: 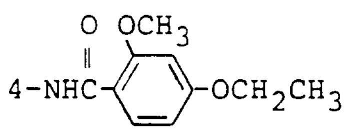

Crystalline form: White powder

Recrystallization solvent: Methanol/diethyl ether

Melting Point: 203 - 205°C

Form: Free

Example 889

Structure

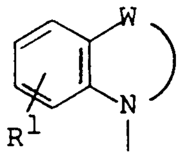 : 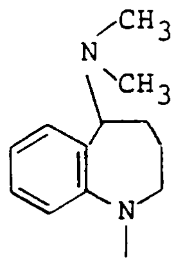

$R^2$: 3-$OCH_3$

$R^3$: 4-NHC 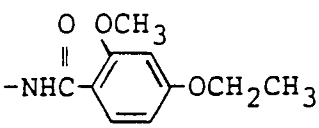

Crystalline form: White powder

Recrystallization solvent: Ethanol

Melting Point: 202 - 202.5

Form: Free

Example 890

Structure

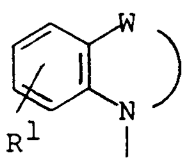 : 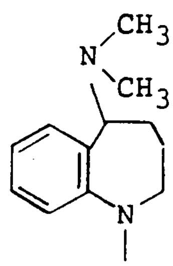

$R^2$: 3-$OCH_2CONH_2$

$R^3$: 4-NHC 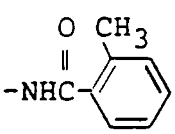

Crystalline form: White powder

Recrystallization solvent: Methanol/diethyl ether

Melting Point: 130 - 133°C

Form: Free

Example 891

Structure

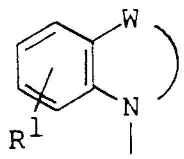 : 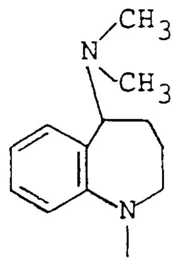

$R^2$: 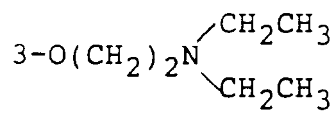

$R^3$: 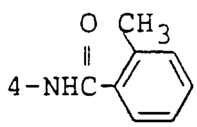

Crystalline form: White powder

Recrystallization solvent: Methanol/n-hexane

Melting Point: 104.5 - 106°C

Form: Free

Example 892

Structure

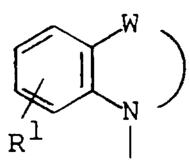 : 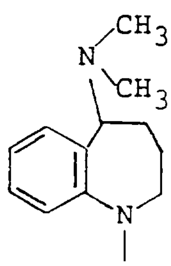

$R^2$: H

$R^3$: 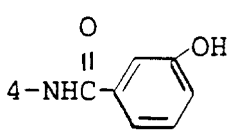

Crystalline form: White powder

Recrystallization solvent: Methanol/diethyl ether

Melting Point: 197 - 198°C

Form: Free

Example 893

Structure

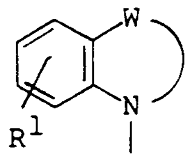 : 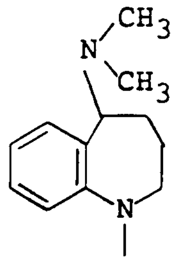

$R^2$: 2-$CH_3$

$R^3$: 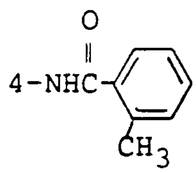

Crystalline form: White powder

Recrystallization solvent: Dichloromethane/ethyl acetate

Melting Point: 191 - 192°C

Form: Free

Example 894

Structure

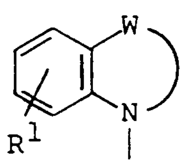 : 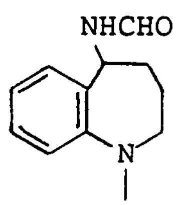

$R^2$: H

$R^3$: 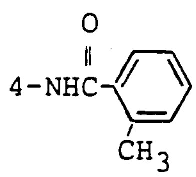

Crystalline form: Colorless columnar

Recrystallization solvent: Ethanol/petroleum ether

Melting Point: 211 - 213°C

Form: Free

Example 895

Structure

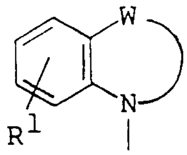

:

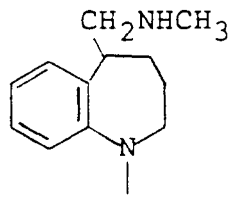

$R^2$: H

$R^3$:

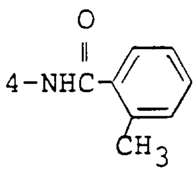

Crystalline form: Colorless amorphous

NMR analysis: 164)

Form: Free

Example 896

Structure

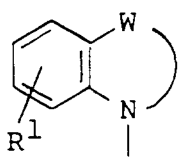

:

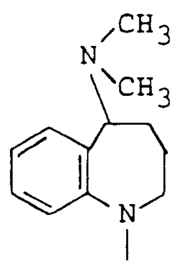

$R^2$: H

O

‖

$R^3$: 4-NHCCH$_2$CO$_2$C$_2$H$_5$

Crystalline form: Colorless amorphous

NMR analysis: 165)

Form: Free

Example 897

Structure

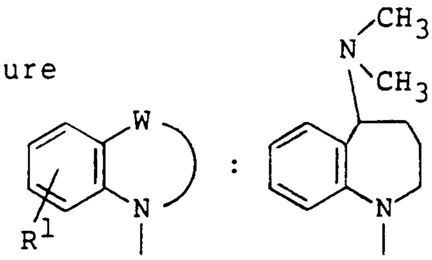

$R^2$: H

$R^3$: 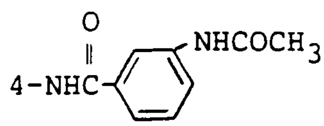

Crystalline form: Colorless amorphous

NMR analysis: 166)

Form: Free

Example 898

Structure

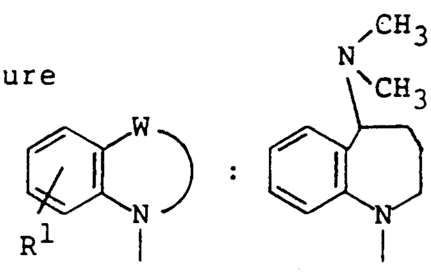

$R^2$: H

$R^3$: 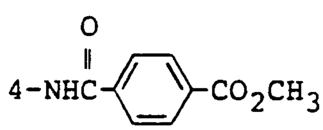

Crystalline form: Colorless prisms

Recrystallization solvent: Ethanol

Melting Point: 224 - 228°C

Form: Free

138) $^1$H-NMR ($CDCl_3$) δ ; 1.3-2.95 (19H, m), 3.05-3.3 (1H, m), 3.85-4.1 (2H, m), 4.3-4.6 (1H, m), 6.64 (1H, d, J=7.8 Hz), 6.9-7.8 (12H, m)

139) $^1$H-NMR ($CDCl_3$) δ ; 1.1-2.3 (13H, m), 2.65-3.2 (1H, m), 4.55-5.6 (3H, m), 6.55-6.7 (1H, m), 6.9-7.6 (12H, m)

140) $^1$H-NMR ($CDCl_3$) δ ; 1.3-4.15 (19H, m), 4.3-5.0 (1H, m), 6.65 (1H, d, J=7.7 Hz), 6.9-8.05 (12H, m)

141) $^1$H-NMR ($CDCl_3$) δ ; 1.4-3.0 (9H, m), 3.05-3.6 (3H, m), 3.9-4.1 (1H, m), 4.35-4.55 (1H, m), 4.9-5.65 (1H, m), 6.67 (1H, d, J=7.4 Hz), 6.85-7.6 (12H, m), 7.6-7.85 (2H, m)

142) $^1$H-NMR ($CDCl_3$) δ ; 1.3-2.85 (21H, m), 3.2-4.0 (4H, m), 4.3-4.4 (1H, m), 4.45-5.2 (2H, m), 6.61 (1H, d, J=7.6 Hz), 6.9-7.65 (12H, m)

143) $^1$H-NMR ($CDCl_3$) δ ; 1.3-3.45 (17H, m), 3.8-5.7 (5H, m), 6.5-7.65 (13H, m)

144) $^1$H-NMR ($CDCl_3$) δ ; 1.25-3.1 (14H, m), 3.3-4.0 (4H, m), 4.15-4.4 (1H, m), 4.45-5.2 (1H, m), 6.64 (1H, d, J=7.4 Hz), 6.9-7.7 (12H, m)

145) $^1$H-NMR ($CDCl_3$) δ ; 0.9-3.25 (16H, m), 3.9-5.9 (2H, m), 6.65 (1H, d, J=7.4 Hz), 6.85-7.5 (11H, m), 7.9-8.3 (1H, m)

146) $^1$H-NMR (DMSO-$d_6$) δ ; 1.3-2.15 (4H, m), 2.32 (3H, s), 2.8-3.05 (1H, m), 4.24 (2H, AB-q, J=12.8, 15.4 Hz), 4.35-4.55 (1H, m), 4.9-5.25 (1H, m), 6.68 (1H,

d, J=7.6 Hz), 6.9-7.45 (9H, m), 7.52 (2H, d, J=8.6 Hz), 8.9-9.05 (1H, m), 10.31 (1H, s)

147) $^1$H-NMR ($CDCl_3$) δ ; 1.5-2.35 (4H, m), 2.45 (3H, s), 2.6-2.85 (1H, m), 3.32 (3H, s), 4.19 (2H, AB-q, J=12.2 Hz, 15.6 Hz), 5.0-5.2 (1H, m), 5.82 (1H, d, J=10.3 Hz), 6.69 (1H, d, J=7.8 Hz), 6.75-7.95 (12H, m)

148) $^1$H-NMR ($CDCl_3$) δ ; 1.2-3.3 (17H, m), 3.45 (2H, AB-q, J=14.7, 22.9 Hz), 3.9-4.35 (2H, m), 6.60 (2H, d, J=7.7 Hz), 6.8-8.0 (11H, m), 8.39 (1H, s)

149) $^1$H-NMR ($CDCl_3$) δ ; 1.45-3.40 (8H, m), 2.23 (3H, s), 2.33 (3H, s), 2.46 (3H, s), 4.44-5.23 (1H, m), 6.54-6.78 (1H, m), 6.84-7.94 (12H, m)

150) $^1$H-NMR ($CDCl_3$) δ ; 1.50-1.92 (3H, m), 1.92-2.05 (1H, m), 2.47 (3H, s), 2.55-3.06 (5H, m), 3.43-5.76 (8H, m), 6.63-6.82 (1H, m), 6.97-8.08 (12H, m)

151) $^1$H-NMR ($CDCl_3$) δ ; 1.43-2.65 (4H, m), 2.48 (3H, s), 2.69-3.25 (5H, m), 3.90-5.40 (8H, m), 6.64-6.94 (1H, m), 6.94-7.77 (12H, m)

152) $^1$H-NMR ($CDCl_3$) δ ; 1.50-1.90 (3H, m), 1.90-2.20 (1H, m), 2.20-2.64 (4H, m), 2.32 (3H, s), 2.47 (3H, s), 2.64-3.27 (1H, m), 3.36-3.83 (4H, m), 3.93-4.52 (2H, m), 4.52-5.27 (2H, m), 6.57-6.82 (1H, m), 6.93-7.87 (12H, m)

153) $^1$H-NMR ($CDCl_3$) δ ; 1.52-1.93 (2H, m), 1.93-2.23 (4H, m), 2.23-2.57 (1H, m), 2.45 (3H, s), 2.72-3.02 (1H, m), 3.02-3.77 (8H, m), 3.93-4.50 (2H, m),

4.50-5.20 (2H, m), 6.60-6.80 (1H, m), 6.94-7.64 (11H, m), 8.16 (1H, brs)

154) $^1$H-NMR ($CDCl_3$) δ ; 1.48-2.60 (8H, m), 2.46 (3H, s), 2.65-3.01 (1H, m), 3.20-3.74 (2H, m), 3.80-5.14 (4H, m), 5.30-5.84 (1H, m), 6.51-8.14 (13H, m)

155) $^1$H-NMR ($CDCl_3$) δ ; 1.54-1.91 (2H, m), 1.91-2.20 (1H, m), 2.22-2.64 (1H, m), 2.44 (3H, s), 2.70-3.13 (1H, m), 3.60-4.40 (4H, m), 4.50-5.20 (2H, m), 6.07-8.00 (13H, m), 9.93 (1H, s)

156) $^1$H-NMR ($CDCl_3$) δ; 1.56-1.92 (2H, m), 1.92-2.19 (1H, m), 2.19-2.60 (1H, m), 2.46 (3H, s), 2.66-3.26 (4H, m), 3.33-3.95 (4H, m). 4.00-5.20 (4H, m), 6.58-6.82 (1H, m), 6.93-8.21 (12H, m)

157) $^1$H-NMR ($CDCl_3$) δ ; 1.57-2.17 (3H, m), 2.21-2.68 (1H, m), 2.47 (3H, s), 2.73-3.04 (1H, m), 3.91-4.42 (4H, m), 4.50-5.17 (2H, m), 6.61-6.99 (2H, m), 6.99-8.10 (14H, m), 8.21-8.71 (2H, m)

158) $^1$H-NMR ($CDCl_3$) δ ; 1.31 (3H, d, J=6.7 Hz), 1.53-1.90 (1H, m), 2.29-2.58 (1H, m), 2.47 (3H, s), 2.94-3.63 (2H, m), 4.57-5.05 (1H, m), 6.68-6.82 (1H, m), 7.10-7.59 (10H, m), 7.72 (1H, s), 7.78-7.96 (1H, m)

159) $^1$H-NMR ($CDCl_3$) δ ; 1.20-2.60 (17H, m), 2.65-5.10 (3H, m), 6.85-3.85 (12H, m)

160) $^1$H-NMR (DMSO-$d_6$) δ ; 1.40-1.75 (1H, m), 1.90-2.15 (1H, m), 2.33 (3H, s), 2.50-2.80 (2H, m), 3.10-3.50

(1H, m), 4.40-4.65 (1H, m), 6.85-7.60 (10H, m), 7.85 (1H, s), 10.44 (1H, s)

161) $^1$H-NMR ($CDCl_3$) δ ; 1.30-2.70 (11H, m), 3.00-5.20 (3H, m), 6.58 (1H, d, J=8 Hz), 6.90-7.05 (1H, m), 7.10-7.70 (10H, m)

162) $^1$H-NMR ($CDCl_3$) δ ; 1.25-2.90 (4H, m), 2.44 (6H, s), 2.79-3.57 (2H, m), 2.79 (6H, s), 4.10-5.25 (1H, m), 6.60-6.80 (1H, m), 6.94-7.60 (10H, m), 8.23 (1H, d, J=6.2 Hz), 12.41 (1H, m)

163) $^1$H-NMR ($CDCl_3$) δ ; 1.25-3.00 (4H, m), 2.42 (6H, s), 2.99 (6H, s), 3.40-3.65 (2H, m), 4.01-5.15 (1H, m), 6.58-7.59 (12H, m), 7.94 (1H, brs)

164) $^1$H-NMR (DMSO-$d_6$) δ ; 1.40-2.18 (4H, m), 2.34 (3H, s), 2.47 (3H, s), 2.54-3.50 (4H, m), 4.30-5.08 (1H, m), 6.56-6.82 (1H, m), 6.87-7.48 (10H, m), 7.48-7.75 (2H, m), 10.35 (1H, s)

165) $^1$H-NMR ($CDCl_3$) δ ; 1.08-5.20 [20H, m, 1.30 (3H, t, J=7.2 Hz), 3.41 (2H, s), 4.22 (2H, q, J=7.2 Hz)], 6.49-7.73 (8H, m), 9.25-9.58 (1H, m)

166) $^1$H-NMR ($CDCl_3$) δ ; 1.17-2.80 (4H, m), 2.05 (3H, s), 2.42 (6H, s), 3.02-3.53 (2H, m), 4.06-5.15 (1H, m), 6.55-7.80 (12H, m), 8.53-8.74 (2H, m)

Example 899

To a solution of 5-acetyloxyimino-1-[4-(2-chlorobenzoylamino)benzoyl]-2,3,4,5-tetrahydro-1H-benzazepine (0.48 g) in acetic acid (20 ml) is added platinum oxide (0.05 g) and the mixture is subjected to catalytic reduction under hydrogen atmosphere. After completion of the reaction, the catalyst is removed by filtration, and the filtrate is concentrated. The resulting residue is purified by silica gel column chromatography

(eluent; dichloromethane : methanol = 20 : 1 → 10 :1),

and recrystallized from ethanol/diethyl ether to give 5-amino-1-[4-(2-chlorobenzoylamino)benzoyl]-2,3,4,5-tetrahydro-1H-benzazepine (0.19 g) as colorless prisms, m.p. 176 - 178°C.

Example 900

To a solution of 5-methylamino-1-[4-(2-methylbenzoylamino)benzoyl]-2,3,4,5-tetrahydro-1H-benzazepine (0.6 g) in dichloromethane (10 ml) is added triethylamine (0.24 ml). Subsequently, thereto is added methanesulfonyl chloride (0.14 ml) under ice-cooling, and then, the mixture is warmed to room temperature and stirred overnight. Water is added to the reaction solution, extracted three times with dichloromethane. The extract is washed with saturated saline solution, and dried over magnesium sulfate. The solvent is distilled off and the resulting residue is purified by silica gel column chromatography (eluent; dichloromethane : methanol = 20 : 1), and recrystallized from ethanol to give 5-(N-methyl-N-methanesulfonylamino)-1-[4-(2-methylbenzoylamino)benzoyl]-2,3,4,5-tetrahydro-1H-benzazepine (0.48 g) as colorless scales, m p. 197 - 198°C.

Example 901

To a solution of 5-methylamino-1-[4-(2-methylbenzoylamino)benzoyl]-2,3,4,5-tetrahydro-1H-benzazepine (0.6 g) in dichloromethane is added triethylamine (0.24 ml). Subsequently, thereto is added benzoyl chloride (0.2 ml) under ice-cooling, and the temperature thereof is raised to room temperature, and the mixture is stirred overnight. Water is added to the reaction solution and extracted three times with dichloromethane. The extract is washed with saturated saline solution and dried over magnesium sulfate. The solvent is distilled off and the resulting residue is purified by silica gel column chromatography (eluent; dichloromethane : methanol = 20 : 1), and recrystallized from ethanol to give 5-(N-methyl-N-benzoylamino)-1-[4-(2-methylbenzoylamino)benzoyl]-2,3,4,5-tetrahydro-1H-benzazepine (0.64 g) as colorless needles, m.p. 248 - 249°C.

Example 902

A mixture of 5-amino-1-[4-(2-methylbenzoylamino)benzoyl]-2,3,4,5-tetrahydro-1H-benzazepine (0.6 g) and ethyl formate (10 ml) is refluxed for 4 hours. The reaction solution is concentrated and the resulting residue is recrystallized from ethanol/petroleum ether to give 5-formylamino-1-[4-(2-methylbenzoylamino)benzoyl]-2,3,4,5-tetrahydro-1H-benzazepine (0.38 g) as colorless columnar crystal, m.p. 211 - 213°C.

Using the suitable starting materials, the compounds of above Examples 825 and 894 are obtained in the same manner as in above Example 902.

Example 903

To a solution of 5-amino-1-[4-(2-chlorobenzoylamino)benzoyl]-2,3,4,5-tetrahydro-1H-benzazepine (0.6 g) in dichloromethane (10 ml) is added triethylamine (0.22 ml). Subsequently, thereto is added di-tert-butyl dicarbonate (0.34 g) at room temperature and the mixture is stirred for 2 hours. Then, thereto is added additional di-tert-butyl dicarbonate (0.1 g) and the mixture is stirred for 1 hour. The reaction mixture is concentrated and the resulting residue is purified by silica gel column chromatography (eluent; n-hexane : ethyl aceate = 1 : 1) to give 5-t-butoxycarbonylamino-1-[4-(2-chlorobenzoylamino)benzoyl]-2,3,4,5-tetrahydro-1H-benzazepine (0.66 g) as colorless amorphous.

$^1$H-NMR ($CDCl_3$) $\delta$ ; 1.1-2.3 (13H, m), 2.65-3.2 (1H, m), 4.55-5.6 (3H, m), 6.55-6.7 (1H, m), 6.9-7.6 (12H, m)

Using the suitable starting materials, the compound of above Example 791 is obtained in the same manner as in above Example 903.

Example 904

To a solution of 5-amino-1-[4-(2-methylbenzoylamino)benzoyl]-2,3,4,5-tetrahydro-1H-benzazepine (0.6 g) in dichloromethane (10 ml) is added phenyl isocyanate (0.2 g) under ice-cooling. The mixture is stirred at the same temperature for 30 minutes, and the temperature thereof is raised to room temperature and then the mixture is stirred overnight. The reaction solution is distilled off and the resulting residue is recrystallized from dioxane to give 5-anilinocarbonylamino-1-[4-(2-methylbenzoylamino)benzoyl]-2,3,4,5-tetrahydro-1H-benzazepine (0.65 g) as colorless prisms, m.p. 269 - 271°C.

Using the suitable starting materials, the compound of above Example 795 is obtained in the same manner as in above Example 904.

Example 905

To a solution of 5-methylamino-1-[4-(2-methylbenzoylamino)benzoyl]-2,3,4,5-tetrahydro-1H-benzazepine (0.6 g) in methanol (10 ml) is added glycolonitrile (50 %, 0.19 ml) and the mixture is stirred at room temperature for 20 minutes, and then refluxed for 30 minutes. Thereto is added additional glycolonitrile (0.5 ml) and the mixture is refluxed for 5.5

hours. The reaction solution is concentrated and to the resulting residue is added ethyl acetate. The precipitated crystal is collected by filtration, and recrystallized from acetonitrile to give 5-(N-methyl-N-cyanomethylamino)-1-[4-(2-methylbenzoylamino)benzoyl]-2,3,4,5-tetrahydro-1H-benzazepine (0.32 g) as colorless needles, m.p. 227 - 228°C.

Example 906

To 5-(N-methyl-N-oxiranylmethylamino)-1-[4-(2-methylbenzoylamino)benzoyl]-2,3,4,5-tetrahydro-1H-benzazepine (0.62 g) is added trifluoroacetic acid (1.22 ml) under ice-cooling and the mixture is stirred for 4 hours. The reaction solution is neutralized with aqueous sodium carbonate solution, and extracted three times with dichloromethane. The extract is washed with saturated saline solution and dried over magnesium sulfate. The solvent is distilled off and the resulting residue is dissolved in methanol (10 ml). Thereto is added 40 % aqueous sodium hydroxide solution (10 ml) and water (10 ml), and the mixture is stirred at room temperature overnight. Methanol is distilled off and the resulting residue is purified by silica gel column chromatography (eluent; dichloromethane : methanol = 30 : 1) to give 5-[N-methyl-N-(2,3-dihydroxypropyl)amino)-1-[4-(2-methylbenzoylamino)benzoyl]-2,3,4,5-tetrahydro-1H-benzazepine (0.23 g) as colorless amorphous.

$^1$H-NMR ($CDCl_3$) δ ; 1.3-4.15 (19H, m), 4.3-5.0 (1H, m), 6.65 (1H, d, J=7.7 Hz), 6.9-8.05 (12H, m)

Example 907

A mixture of 5-methylamino-1-[4-(2-methylbenzoylamino)benzoyl]-2,3,4,5-tetrahydro-1H-benzazepine (1.64 g), acetonitrile (20 ml), potassium carbonate (0.6 g) and ethyl bromoacetate (0.44 ml) is refluxed for 3 hours. The reaction solution is concentrated and water is added to the resulting residue, and the mixture is extracted three times with dichloromethane. The extract is washed with saturated saline solution, and dried over magnesium sulfate. The solvent is distilled off and the resulting residue is purified by silica gel column chromatography (eluent; dichloromethane : methanol = 30 : 1), and recrystallized from ethyl acetate/petroleum ether to give 5-(N-methyl-N-ethoxycarbonylmethylamino)-1-[4-(2-methylbenzoylamino)benzoyl]-2,3,4,5-tetrahydro-1H-benzazepine (0.82 g) as colorless prisms, m.p. 167 - 168°C.

Using the suitable starting materials, the compounds of above Examples 785, 787, 799, 800, 802 - 806, 808, 811, 819, 824, 826, 827, 845, 848, 849, 850, 852, 855 - 858, 860, 861, 863 - 882, 885 - 893 and 895 - 898 are obtained in the same manner as in above Example 907.

Example 908

5-(N-Methyl-N-ethoxycarbonylmethylamino)-1-[4-(2-methylbenzoylamino)benzoyl]-2,3,4,5-tetrahydro-1H-benzazepine (0.6 g) is dissolved in saturated solution of ammonia in methanol (20 ml), and the mixture is heated at 100°C for 8 hours in a sealed tube. The reaction solution is concentrated and the resulting residue is purified by silica gel column chromatography (eluent; dichloromethane : methanol = 30 : 1) to give 5-(N-methyl-N-carbamoylmethylamino)-1-[4-(2-methylbenzoylamino)benzoyl]-2,3,4,5-tetrahydro-1H-benzazepine (0.4 g) as colorless amorphous.

$^1$H-NMR ($CDCl_3$) δ ; 1.4-3.0 (9H, m), 3.05-3.6 (3H, m), 3.9-4.1 (1H, m), 4.35-4.55 (1H, m), 4.9-5.65 (1H, m), 6.67 (1H, d, J=7.4 Hz), 6.85-7.6 (12H, m), 7.6-7.85 (2H, m)

Example 909

To a solution of 5-(N-methyl-N-ethoxycarbonylmethylamino)-1-[4-(2-methylbenzoylamino)benzoyl]-2,3,4,5-tetrahydro-1H-benzazepine (0.6 g) in dioxane (10 ml) is added aqueous solution (1 ml) of sodium hydroxide (0.07 g) and the mixture is stirred at room temperature for 2 days. The reaction solution is concentrated and to the resulting residue is added water. The insoluble materials are removed by filtration. The filtrate is neutralized with 10 % hydrochloric acid and extracted three times with dichloromethane. The extract is washed with saturated saline solution and dried over magnesium sulfate. The solvent is distilled off and to the resulting residue is added a solution of potassium ethylhexanoate (0.2 g) in dichloromethane (20 ml). The solvent is distilled off, and diethyl ether is added to the resulting residue. The precipitated crystal is collected by filtration, and recrystallized from diethyl ether to give potassium 2-[N-methyl-N-{1-[4-(2-methylbenzoylamino)benzoyl]-2,3,4,5-tetrahydro-1H-benzazepin-5-yl}amino]acetate (0.6 g) as colorless needles, m.p. 164 - 171°C.

Example 910

To a solution of 5-methylamino-1-[4-(2-methylbenzoylamino)benzoyl]-2,3,4,5-tetrahydro-1H-benzazepine (1.5 g) in dimethyformamide (20 ml) are added potassium carbonate (0.6 g), potassium iodide (0.72 g) and 2-(3-bromopropyloxy)-3,4,5,6-tetrahydro-2H-pyrane (0.97 g) and the mixture is stirred at room temperature overnight. The reaction solution is concentrated and to the resulting residue is added water. The mixture is extracted three times with dichloromethane.

The extract is washed with saturated saline solution, and dried over magnesium sulfate. The solvent is distilled off and the resulting residue is purified by silica gel column chromatography

(eluent; dichloromethane → dichloromethane : methanol = 50 : 1)

to give 5-{N-methyl-N-[3-(3,4,5,6-tetrahydro-2H-pyran-2-yloxy)propyl]amino}-1-[4-(2-methylbenzoylamino)benzoyl]-2,3,4,5-tetrahydro-1H-benzazepine (1.3 g) as colorless amorphous.

$^1$H-NMR ($CDCl_3$) ; 1.3-2.85 (21H, m), 3.2-4.0 (4H, m), 4.3-4.4 (1H, m), 4.45-5.2 (2H, m), 6.61 (1H, d, J=7.6 Hz), 6.9-7.65 (12H, m)

Example 911

To 5-{N-methyl-N-[3-(3,4,5,6-tetrahydro-2H-pyran-2-yloxy)propyl]amino}-1-[4-(2-methylbenzoylamino)benzoyl]-2,3,4,5-tetrahydro-1H-benzazepine (0.4 g) is added a mixture of acetyl chloride (0.5 ml) and acetic acid (5 ml) at room temperature, and the mixture is stirred overnight. The reaction solution is concentrated and the resulting residue is purified by silica gel column chromatography (eluent; dichloromethane : methanol = 30 : 1), and further purified again by silica gel column chromatography (eluent; n-hexane : ethyl acetate = 1 : 2) to give 5-[N-methyl-N-(3-acetyloxypropyl)amino]-1-[4-(2-methylbenzoylamino)benzoyl]-2,3,4,5-tetrahydro-1H-benzazepine (0.06 g) as colorless amorphous.

$^1$H-NMR ($CDCl_3$) δ ; 1.3-3.45 (17H, m), 3.8-5.7 (5H, m), 6.5-7.65 (13H, m)

Example 912

To a solution of 5-{N-methyl-N-[3-(3,4,5,6-tetrahydro-2H-pyran-2-yloxy)propyl]amino}-1-[4-(2-methylbenzoylamino)benzoyl]-2,3,4,5-tetrahydro-1H-benzazepine (0.55 g) in ethanol (10 ml) is added pyridinium p-toluenesulfonate (0.03 g) and the mixture is heated at 60°C overnight. After the mixture is refluxed for more 2 hours, water and pyridinium p-toluenesulfonate (0.03 g) are added thereto. The mixture is refluxed for 4 hours. The reaction solution is concentrated and to the resulting residue is added dichloromethane. The mixture is basified with aqueous sodium hydrogen carbonate solution and extracted three times with dichloromethane. The extract is washed with saturated saline solution and dried over magnesium sulfate. The solvent is distilled off and the resulting residue is purified by silica gel column chromatography (eluent; dichloromethane : methanol = 30 : 1) to give 5-[N-methyl-N-(3-hydroxypropyl)amino]-1-[4-(2-methylbenzoylamino)benzoyl]- 2,3,4,5-tetrahydro-1H-benzazepine (0.26 g) as colorless amorphous.

$^1$H-NMR ($CDCl_3$) δ ; 1.25-3.1 (14H, m), 3.3-4.0 (4H, m), 4.15-4.4 (1H, m), 4.45-5.2 (1H, m), 6.64 (1H, d, J=7.4 Hz), 6.9-7.7 (12H, m)

Example 913

To a solution of 5-amino-1-[4-(2-methylbenzoylamino)benzoyl]-2,3,4,5-tetrahydro-1H-benzazepine (0.6 g) in acetic acid (10 ml) is added dropwise 2,5-dimethoxytetrahydrofuran (0.19 ml), and the mixture is refluxed for 1 hour. The reaction solution is concentrated and the resulting residue is purified by silica gel column chromatography (eluent; dichloromethane : methanol = 50 : 1), and recrystallized from ethyl acetate/n-hexane to give 5-(1-pyrrolyl)-1-[4-(2-methylbenzoylamino)benzoyl]-2,3,4,5-tetrahydro-1H-benzazepine (0.31 g) as colorless prisms, m.p. 208 - 210°C.

Example 914

To a solution of 5-amino-1-[4-(2-methylbenzoylamino)benzoyl]-2,3,4,5-tetrahydro-1H-benzazepine (2.5 g) in dichloromethane (30 ml) is added triethylamine (0.96 ml) and further thereto is added dropwise chloroacetyl chloride (0.55 ml) under ice-cooling. The mixture is stirred for 5 minutes. The reaction solution is concentrated and to the resulting residue is added water. The precipitated crystal is collected by filtration, washed with water, and dried to give 5-(2-chloroacetylamino)-1-[4-(2-methylbenzoylamino)benzoyl]-2,3,4,5-tetrahydro-1H-benzazepine (1.4 g) as white powder.

$^1$H-NMR (DMSO-$d_6$) δ ; 1.3-2.15 (4H, m), 2.32 (3H, s), 2.8-3.05 (1H, m), 4.24 (2H, AB-q, J=12.8, 15.4 Hz), 4.35-4.55 (1H, m), 4.9-5.25 (1H, m), 6.68 (1H, d, J=7.6 Hz), 6.9-7.45 (9H, m), 7.52 (2H, d, J=8.6 Hz), 8.9-9.05 (1H, m), 10.31 (1H, s)

Using the suitable starting materials, the compound of above Example 814 is obtained in the same manner as in above Example 914.

Example 915

A mixed solution of 5-(2-chloroacetylamino)-1-[4-(2-methylbenzoylamino)benzoyl]-2,3,4,5-tetrahydro-1H-benzazepine (0.6 g), imidazole (0.1 g) and potassium carbonate (0.19 g) in acetonitrile (30 ml) is refluxed for 8 hours. The

reaction solution is concentrated and the resulting residue is washed with water and separated by decantation. The remainder is purified by silica gel column chromatography

(eluent; dichloromethane : methanol = 20 : 1 → 15 : 1),

and recrystallized from ethanol/n-hexane to give 5-[2-(1-imidazolyl)acetylamino]-1-(4-(2-methylbenzoylamino)benzoyl]-2,3,4,5-tetrahydro-1H-benzazepine (0.15 g) as colorless needles, m.p. 250 - 252°C.

Using the suitable starting materials, the compound of above Example 818 is obtained in the same manner as in above Example 915.

Example 916

To a solution of 5-(2-chloroacetylamino)-1-[4-(2-methylbenzoylamino)benzoyl]-2,3,4,5-tetrahydro-1H-benzazepine (0.6 g) in dimethylformamide (20 ml) are added dimethylamine hydrochloride (0.21 g) and potassium carbonate (0.54 g), and the mixture is stirred at room temperature for 2 days. The reaction solution is concentrated and water is added to the resulting residue. The precipitated crystal is collected by filtration, and recrystallized from ethyl acetate to give 5-(2-dimethylaminoacetylamino)-1-[4-(2-methylbenzoylamino)benzoyl]-2,3,4,5-tetrahydro-1H-benzazepine (0.24 g) as colorless prisms, m.p. 214 - 216°C.

Using the suitable starting materials, the compounds of above Examples 816, 817, 820, 821, 826 and 827 are obtained in the same manner as above Example 916.

Example 917

A mixture of 5-methylamino-1-[4-(2-methylbenzoylamino)benzoyl]-2,3,4,5-tetrahydro-1H-benzazepine (0.6 g), N,N-dimethyl-2-chloroacetamide (0.19 g) and potassium carbonate (0.22 g) is refluxed for 24 hours. The reaction solution is concentrated and water is added to the resulting residue. The mixture is extracted three times with dichloromethane. The extract is washed with saturated saline solution, and dried over magnesium sulfate. The solvent is distilled off and the resulting residue is purified by silica gel column chromatography (eluent; dichloromethane : methanol = 30 : 1) to give 5-[N-methyl-N-(dimethylaminocarbonylmethyl)amino]-1-[4-(2-methylbenzoylamino)benzoyl]-2,3,4,5-tetrahydro-1H-benzazepine (0.05 g) as colorless amorphous.

$^1$H-NMR ($CDCl_3$) δ ; 1.2-3.3 (17H, m), 3.45 (2H, AB-q, J=14.7, 22.9 Hz), 3.9-4.35 (2H, m), 6.60 (1H, d, J=7.7 Hz), 6.8-8.0 (11H, m), 8.39 (1H, s)

Example 918

To a solution of t-butoxycarbonylglycine (0.84 g) in dimethylformamide (20 ml) are added diethyl cyanophosphate (0.73 ml) and 5-amino-1-[4-(2-methylbenzoylamino)benzoyl]-2,3,4,5-tetrahydro-1H-benzazepine (1.74 g), and further thereto is added triethylamine (1.8 ml) under ice-cooling. The mixture is stirred for 30 minutes, and then stirred at room temperature overnight. The reaction solution is concentrated and water is added to the resulting residue. The precipitated crystal is collected by filtration, washed with water, and recrystallized from ethyl acetate to give 5-(2-aminoacetylamino)-1-[4-(2-methylbenzoylamino)benzoyl]-2,3,4,5-tetrahydro-1H-benzazepine (E) (0.16 g). Separately, the filtrate is concentrated and purified by silica gel column chromatography (eluent; dichloromethane : methanol = 50 : 1), and recrystallized from diethyl ether to give 5-[2-(t-butoxycarbonylamino)acetylamino]-1-[4-(2-methylbenzoylamino)benzoyl]-2,3,4,5-tetrahydro-1H-benzazepine (F) (0.19 g).

(E): Colorless prisms, m.p. 287 - 289°C
(F): Colorless prisms, m.p. 170 - 171°C

Example 919

5-Oxo-1-[4-(2-methylbenzoylamino)benzoyl]-2,3,4,5-tetrahydro-1H-benzazepine (0.50 g) is suspended in tetrahydrofuran (20 ml), and thereto is added dropwise a 3.0 M solution of methyl magnesium bromide in diethyl ether (1.5 ml) at room temperature. The mixture is stirred at room temperature for 1 hour. The reaction solution is poured into ice-water (20 ml), and extracted with ethyl acetate. The extract is dried over magnesium sulfate, and the solvent is distilled off. The resulting residue is purified by silica gel column chromatography

(eluent; ethyl acetate : n-hexane = 2 : 3 → 1 : 1),

and recrystallized from ethyl acetate/n-hexane to give 5-methyl-5-hydroxy-1-[4-(2-methylbenzoylamino)benzoyl]-2,3,4,5-tetrahydro-1H-benzazepine (0.23 g) as white powder, m.p. 204 - 205°C.

Example 920

To a solution of 5-carboxymethoxy-1-[4-(2-methylbenzoylamino)benzoyl]-2,3,4,5-tetrahydro-1H-benzazepine (1.50 g) in dimethylformamide (60 ml) are added successively thiomorpholine (0.66 ml), diethyl cyanophosphate (0.89 g) and triethylamine (1.37 ml) with stirring under ice-cooling. The mixture is stirred for 30 minutes under ice-cooling, and at room temperature for 20 minutes. Water (60 ml) is added to the reaction solution, and extracted with dichloromethane. The extract is dried over magnesium sulfate, and the solvent is distilled off. The resulting residue is purified by silica gel column chromatography

(eluent; ethyl acetate : n-hexane = 5 : 2 → 3 : 1),

and recrystallized from ethyl acetate/n-hexane to give 5-(thiomorpholinocarbonylmethoxy)-1-[4-(2-methylbenzoylamino)benzoyl)-2,3,4,5-tetrahydro-1H-benzazepine (1.60 g) as white powder, m.p. 235 - 237°C.

Using the suitable starting materials, the compounds of above Examples 829 - 838 are obtained in the same manner as in above Example 920.

Example 921

To a solution of 5-(thiomorpholinocarbonylmethoxy)-1-[4-(2-methylbenzoylamino)benzoyl]-2,3,4,5-tetrahydro-1H-benzazepine (0.40 g) in dichloromethane (40 ml) is added 80 % m-chloroperbenzoic acid (175 mg) with stirring at -8°C, and the mixture is stirred at -8°C for 1 hour. To the reaction solution is added 20 % aqueous sodium hydrogensulfite solution (40 ml) and the mixture is stirred at room temperature for 30 minutes. The dichloromethane layer is collected, washed with saturated saline solution and dried over magnesium sulfate. The solvent is distilled off and the resulting residue is purified by silica gel column chromatography (eluent; dichloromethane : methanol = 20 : 1) to give 5-[(1-oxothiomorpholino)carbonylmethoxy]-1-[4-(2-methylbenzoylamino)benzoyl]-2,3,4,5-tetrahydro-1H-benzazepine (0.32 g) as colorless amorphous.

$^1$H-NMR ($CDCl_3$) δ ; 1.50-1.92 (3H, m), 1.92-2.05 (1H, m), 2.47 (3H, s), 2.55-3.06 (5H, m), 3.43-5.76 (8H, m), 6.63-6.82 (1H, m), 6.97-8.08 (12H, m)

Example 922

To a solution of 5-(thiomorpholinocarbonylmethoxy)-1-[4-(2-methylbenzoylamino)benzoyl]-2,3,4,5-tetrahydro-1H-benzazepine (0.40 g) in dichloromethane (40 ml) is added 80 % m-chloroperbenzoic acid (0.35 g), and the mixture is stirred at room temperature for 1 hour. The reaction solution is washed successively with an aqueous sodium hydrogensulfite solution and saturated saline solution, and dried over magnesium sulfate. The solvent is distilled off to give 5-[(1,1-dioxothiomorpholino)carbonylmethoxy]-1-[4-(2-methylbenzoylamino)benzoyl]-2,3,4,5-tetrahydro-1H-benzazepine (0.41 g) as colorless amorphous.

$^1$H-NMR ($CDCl_3$) δ ; 1.43-2.65 (4H, m), 2,48 (3H, s), 2.69-3.25 (5H, m), 3.90-5.40 (8H, m), 6.64-6.94 (1H, m), 6.94-7.77 (12H, m)

Example 923

To a solution of 5-oxo-1-[4-(2-hydroxybenzoylamino)benzoyl]-2,3,4,5-tetrahydro-1H-benzazepine (400 mg) in acetone (20 ml) are added potassium carbonate (210 mg), potassium iodide (250 mg) and 2-chloroacetamide (120 mg), and the mixture is refluxed for 2 hours. The insoluble materials are removed by filtration, and the filtrate is distilled off. Dichloromethane is added to the resulting residue, and the mixture is washed with saturated saline solution, and dried over magnesium sulfate. The solvent is distilled off and the resulting residue is recrystallized from ethyl acetate/n-hexane to give 5-oxo-1-[4-(2-carbamoylmethoxybenzoylamino)benzoyl]-2,3,4,5-tetrahydro-1H-benzazepine (436 mg) as white powder, m.p. 226 - 228°C.

Using the suitable starting materials, the compound of above Example 842 is obtained in the same manner as above Example 923.

Example 924

A mixture of 5-methylamino-4-hydroxy-1-[4-(2-chlorobenzoylamino)benzoyl]-2,3,4,5-tetrahydro-1H-benzazepine (0.13 g), ethyl α-bromoacetate (58 mg), diisopropylethylamine (49 mg) and acetonitrile (5 ml) is refluxed for 10 hours.

Acetonitrile is distilled off under reduced pressure, and the resulting residue is dissolved in dichloromethane, washed with water, dried over magnesium sulfate, and distilled off under reduced pressure. The resulting residue is purified by silica gel column chromatography (eluent; dichloromethane : methanol = 50 : 1), and recrystallized from chloroform/methanol to give 7-[4-(2-chlorobenzoylamino)benzoyl]-1-methyl-1,2,3,4a,5,6,7,11b- octahydro-3-oxo[1]benzazepino[4,5-b][1,4]oxazine (80 mg) as colorless prisms, m.p. 286 - 290°C.

Example 925

To a solution of 5-oxo-1-[2-chloro-4-(2-methylbenzoylamino)benzoyl]-2,3,4,5-tetrahydro-1H-benzazepine (1 g) in methanol (20 ml) and dichloromethane (20 ml) is added hydroxylamine-O-sulfonic acid (0.28 g) with stirring at room temperature, and the mixture is stirred at the same temperature for 1 hour. Subsequently, to the reaction solution is added with stirring an aqueous solution of patassium carbonate (0.34 g) in water (1 ml) at room temperature, and the mixture is stirred at the same temperature for 2 hours. The precipitated crystal is removed by filtration, and the filtrate is concentrated under reduced pressure. The resulting residue is purified by silica gel column chromatography to give potassium {1-[2-chloro-4-(2-methylbenzoylamino)benzoyl]-2,3,4,5-tetrahydro-1H-benzazepin-5-yl}imino-O-sulfonate (0.4 g) as white powder.

$^1$H-NMR (DMSO-$d_6$) δ ; 1.40-1.75 (1H, m), 1.90-2.15 (1H, m), 2.33 (3H, s), 2.50-2.80 (2H, m), 3.10-3.50 (1H, m), 4.40-4.65 (1H, m), 6.85-7.60 (10H, m), 7.85 (1H, s), 10.44 (1H, s)

Example 926

Using the suitable starting materials, the compounds of above Examples 841 - 843, 868 - 870, 888 and 889 are obtained in the same manner as in above Example 380.

Example 927

Using the suitable starting materials, the compounds of above Examples 876 - 878 are obtained in the same manner as in above Example 381.

Example 928

Using the suitable starting materials, the compounds of above Examples 840, 842 and 846 are obtained in the same manner as in above Example 384.

Example 929

Using the suitable starting materials, the compounds of above Examples 788 - 790, 796 - 804, 805, 808, 811, 814, 818, 819, 824, 826, 827, 837, 845, 848, 850, 852, 855, 856 - 858, 860, 861, 863 - 882, 885, 886, 888 - 893 and 895 - 898 are obtained in the same manner as in above Example 388.

Example 930

Using the suitable starting materials, the compound of above Example 848 is obtained in the same manner as in above Example 393.

Example 931

Using the suitable starting materials, the compounds of above Examples 841 and 842 are obtained in the same manner as in above Example 402.

Example 932

Using the suitable starting materials, the compounds of above Examples 882 and 897 are obtained in the same manner as in above Example 403.

Example 933

Using the suitable starting materials, the compound of above Example 809 is obtained in the same manner as in above Example 634.

Example 934

Using the suitable starting materials, the compounds of above Examples 828 - 838 are obtained in the same manner as in above Example 640.

Example 935

Using the suitable starting materials, the compound of above Example 810 is obtained in the same manner as in above Example 772.

Example 936

Using the suitable starting materials, the compound of above Example 788 is obtained in the same manner as in above Example 771.

Example 937

Using the suitable starting materials, the compounds of above Examples 785, 787, 788 - 790, 796 - 805, 806, 807, 808, 811, 814, 818, 819, 845, 848, 849, 850, 852, 855, 856 - 858, 860, 861, 863 - 882, 885, 886, 888 - 893 and 896 - 898 are obtained in the same manner as in above Example 390.

Example 938

To 5-methanesulfonyloxymethyl-1-[4-(2-methylbenzoylamino)benozyl]-2,3,4,5-tetrahydro-1H-benzazepine (0.50 g) is added a 30 % solution of methylamine in methanol (50 ml), and the mixture is heated at 100°C for 3 hours in a sealed tube. After cooling, the reaction solution is evaporated under reduced pressure, and the resulting residue is purified by silica gel column chromatography (eluent; dichloromethane : methanol : aqueous ammonia = 100 : 10 : 1) to give 5-methylaminomethyl-1-[4-(2-methylbenzoylamino)benzoyl]-2,3,4,5-tetrahydro-1H-benzazepine (0.07 g).

$^1$H-NMR (DMSO-$d_6$) δ ; 1.40-2.18 (4H, m), 2.34 (3H, s), 2.47 (3H, s), 2.54-3.50 (4H, m), 4.30-5.08 (1H, m), 6.56-6.82 (1H, m), 6.87-7.48 (10H, m), 7.48-7.75 (2H, m), 10.35 (1H, s)

Using the suitable starting materials, the compounds of above Examples 823 - 825 are obtained in the same manner as in above Example 938.

Using the above suitable starting materials, the compounds of the following Table 6 are obtained in the same manner as in Examples 1 and 382.

Table 6

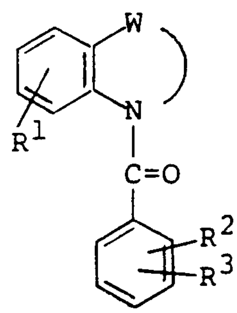

Example 939

Structure

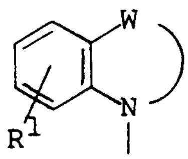 : 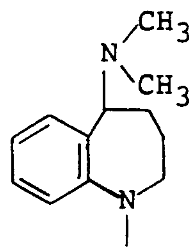

$R^2$: H

$R^3$: 4-NHCO- 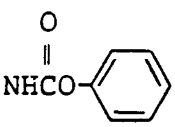

Crystalline form: White powder

Recrystallization solvent: Ethanol

Melting Point: 208 - 211°C

Form: Free

Example 940

Structure

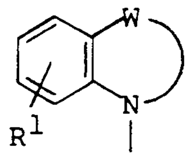 : 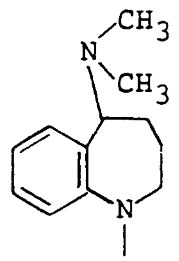

$R^2$: H

$R^3$: 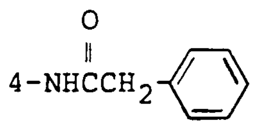

Crystalline form: White powder

Recrystallization solvent: Methanol/diethyl ether

Melting Point: 171.5 - 172.5°C

Form: Free

Example 941

Structure

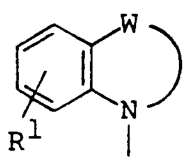 : 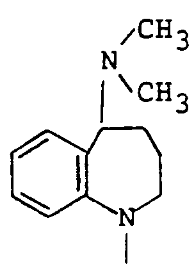

$R^2$: H

$R^3$: 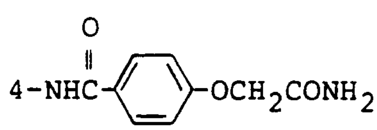

Crystalline form: White powder

Recrystallization solvent: Methanol/diethyl ether

Melting Point: 151 - 154°C

Form: Free

Example 942

Structure

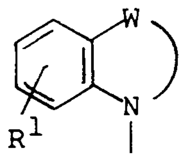 : 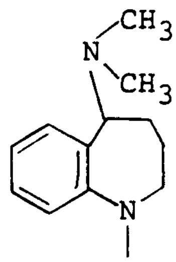 $R^2$: H

$R^3$: 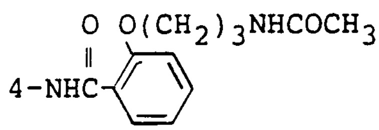

Crystalline form: Colorless amorphous

NMR analysis: 167)

Form: Free

Example 943

Structure

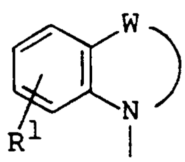 : 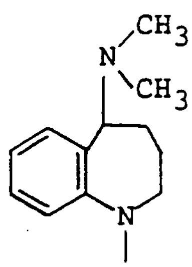 $R^2$: H

$R^3$: 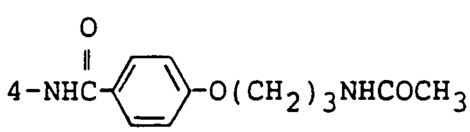

Crystalline form: White powder

Recrystallization solvent: Methanol/diethyl ether

Melting Point: 180 - 183°C

Form: Free

Example 944

Structure

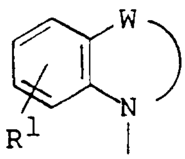 : 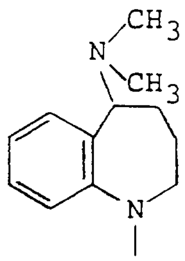

$R^2$: H

$R^3$: 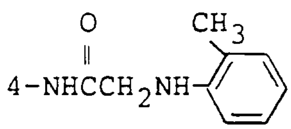

Crystalline form: White powder

Recrystallization solvent: Methanol/diethyl ether

Melting Point: 109 - 110°C

Form: Free

Example 945

Structure

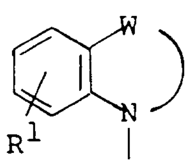 : 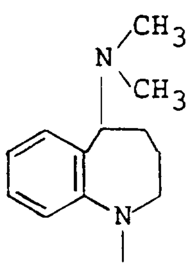

$R^2$: H

$R^3$: 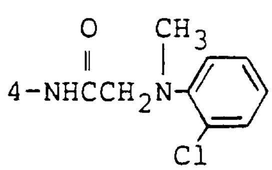

Crystalline form: Colorless oil

NMR analysis: 168)

Form: Free

Example 946

Structure

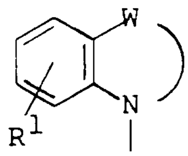 : 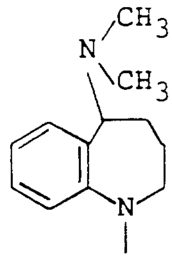

$R^2$: H

$R^3$: 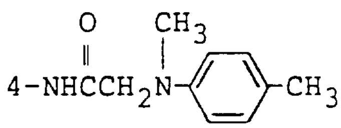

Crystalline form: Colorless oil

NMR analysis: 169)

Form: Free

Example 947

Structure

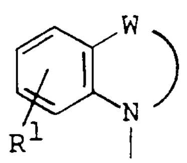 : 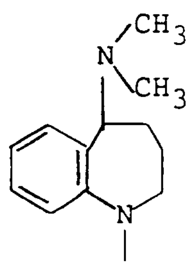

$R^2$: H

$R^3$: 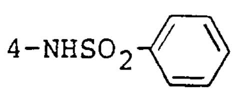

Crystalline form: White powder

Recrystallization solvent: Methanol/diethyl ether

Melting Point: 177 - 178.5°C

Form: Free

Example 948

Structure

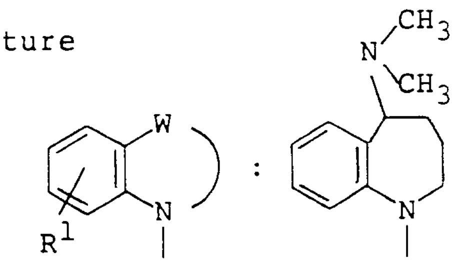

$R^2$: H

$R^3$: 4-$NHCOCH_2CH_2$- 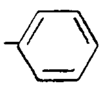

Crystalline form: Colorless amorphous

NMR analysis: 170)

Form: Free

Example 949

Structure

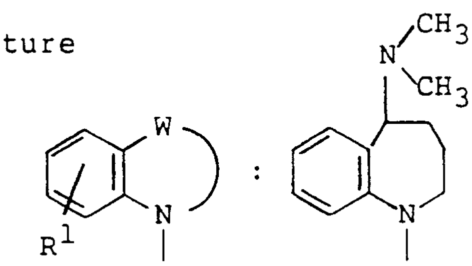

$R^2$: H

$R^3$: 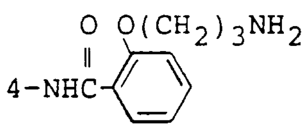

Crystalline form: White powder

Recrystallization solvent: Methanol/diethyl ether

Melting Point: 162 - 165°C

Form: Free

Example 950

Structure

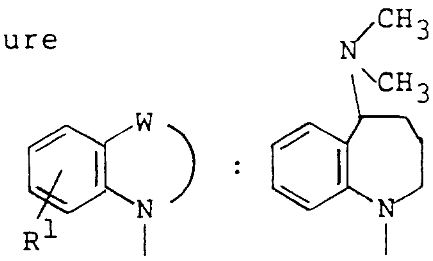

$R^2$: H

$R^3$: 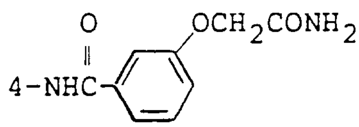

Crystalline form: White powder

Recrystallization solvent: Methanol/diethyl ether

Melting Point: 212 - 215°C

Form: Free

Example 951

Structure

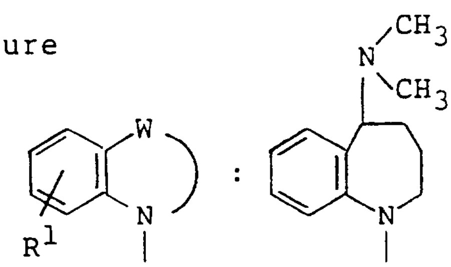

$R^2$: H

$R^3$: 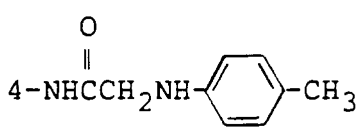

Crystalline form: Colorless oil

NMR analysis: 171)

Form: Free

Example 952

Structure

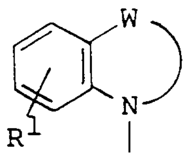 : 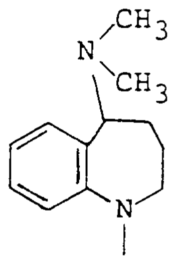

$R^2$: H

$R^3$: 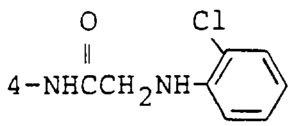

Crystalline form: White powder

Recrystallization solvent: Methanol/diethyl ether

Melting Point: 112 - 114°C

Form: Free

Example 953

Structure

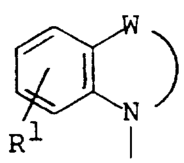 : 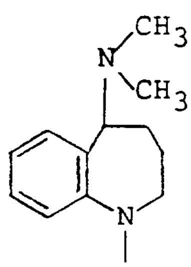

$R^2$: H

$R^3$: 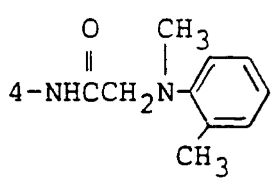

Crystalline form: Colorless oil

NMR analysis: 172)

Form: Free

Example 954

Structure

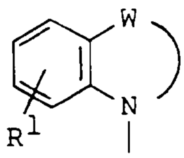 : 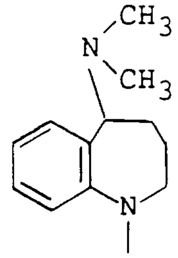

$R^2$: H

$R^3$: 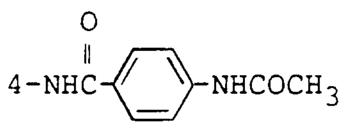

Crystalline form: Colorless amorphous

NMR analysis: 173)

Form: Free

Example 955

Structure

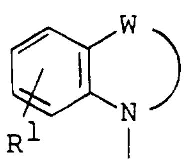 : 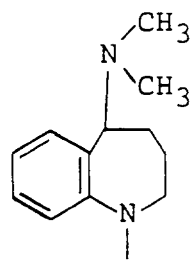

$R^2$: H

$R^3$: 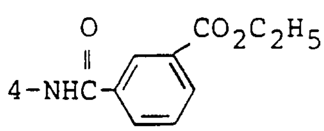

Crystalline form: Light yellow amorphous

NMR analysis: 174)

Form: Free

Example 956

Structure

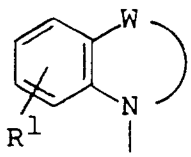 : 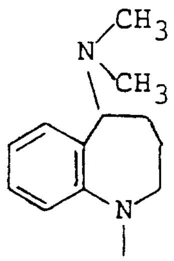

$R^2$: H

$R^3$: 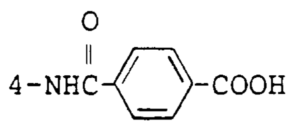

Crystalline form: White powder

Recrystallization solvent: Diethyl ether

Melting Point: 189 - 193°C

Form: Free

Example 957

Structure

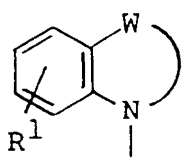 : 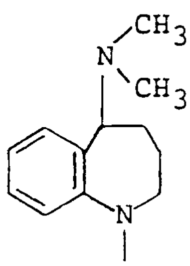

$R^2$: H

$R^3$: 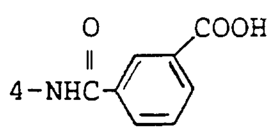

Crystalline form: Colorless amorphous

NMR analysis: 175)

Form: Free

Example 958

Structure

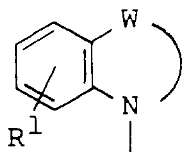 : 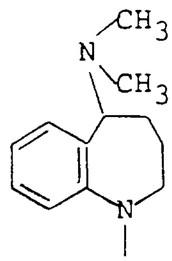

$R^2$: H

$R^3$: 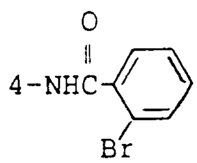

Crystalline form: Colorless prisms

Recrystallization solvent: Ethanol

Melting Point: 234 - 238°C

Form: Free

Example 959

Structure

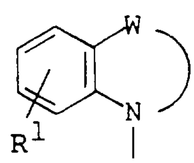 : 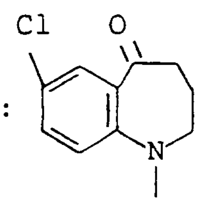

$R^2$: H

$R^3$: 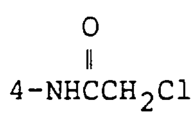

Crystalline form: White powder

Recrystallization solvent: Diethyl ether/dichloromethane

Melting Point: 183 - 184.5°C

Form: Free

Example 960

Structure

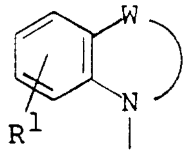

:

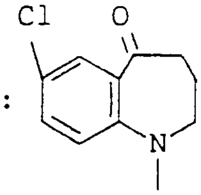

$R^2$: H

$R^3$: 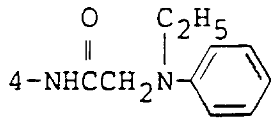

Crystalline form: Brown oil

NMR analysis: 176)

Form: Free

Example 961

Structure

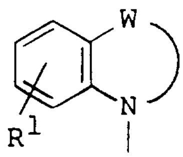

:

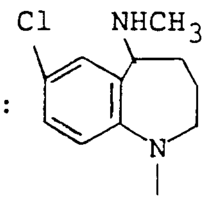

$R^2$: H

$R^3$: 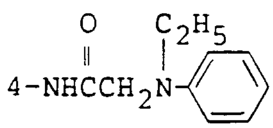

Crystalline form: Colorless amorphous

NMR analysis: 177)

Form: Free

Example 962

Structure

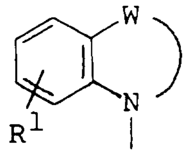

:

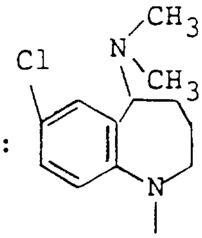

$R^2$: H

$R^3$: 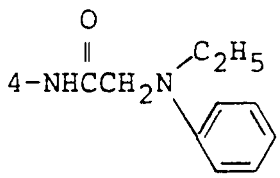

Crystalline form: Colorless amorphous

NMR analysis: 178)

Form: Free

---

Example 963

Structure

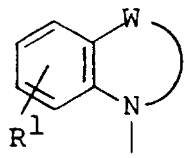

:

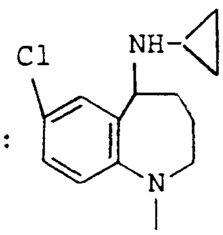

$R^2$: H

$R^3$: 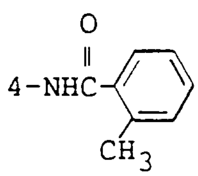

Crystalline form: White powder

Recrystallization solvent: Dichloromethane/diethyl ether

Melting Point: 202.5 - 204.5°C

Form: Free

Example 964

Structure

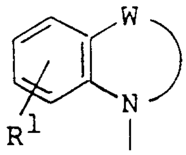 : 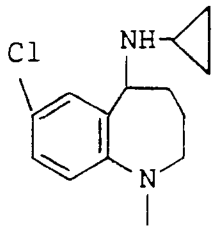

$R^2$: H

$R^3$: 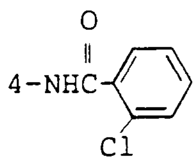

Crystalline form: White powder

Recrystallization solvent: Dichloromethane/diethyl ether

Melting Point: 199.5 - 201°C

Form: Free

Example 965

Structure

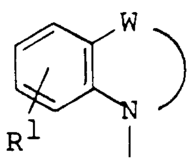 : 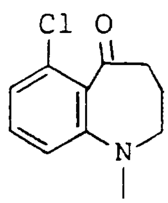

$R^2$: H

$R^3$: 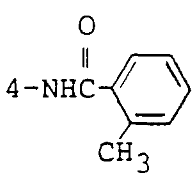

Crystalline form: White powder

Recrystallization solvent: Dichloromethane/diethyl ether

Melting Point: 196.5 - 197°C

Form: Free

Example 966

Structure

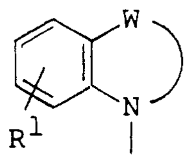 : 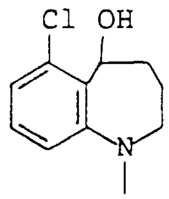

$R^2$: H

$R^3$: 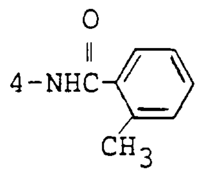

Crystalline form: White powder

Recrystallization solvent: Dichloromethane/diethyl ether

Melting Point: 204 - 205°C

Form: Free

Example 967

Structure

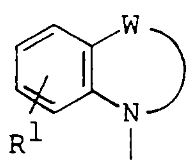 : 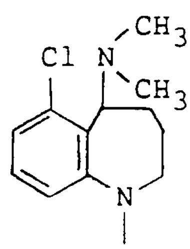

$R^2$: H

$R^3$: 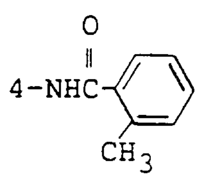

Crystalline form: White powder

Recrystallization solvent: Dichloromethane/diethyl ether

Melting Point: 175 - 177°C

Form: Free

Example 968

Structure

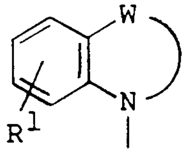 : 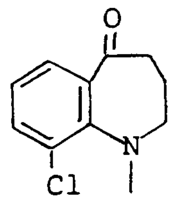

$R^2$: H

$R^3$: 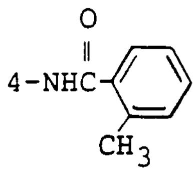

Crystalline form: Pink amorphous

NMR analysis: 179)

Form: Free

Example 969

Structure

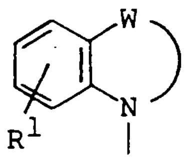 : 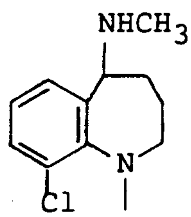

$R^2$: H

$R^3$: 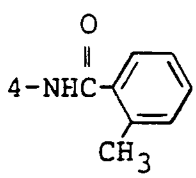

Crystalline form: White powder

Recrystallization solvent: Dichloromethane/diethyl ether

Melting Point: 186 - 189°C

Form: Free

Example 970

Structure

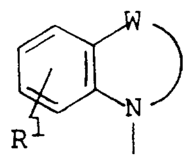 : 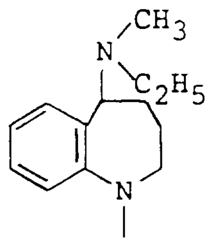

$R^2$: 2-Cl

$R^3$: 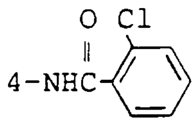

Crystalline form: Colorless prisms

Recrystallization solvent: Ethyl acetate/n-hexane

Melting Point: 211 - 212°C

Form: Free

Example 971

Structure

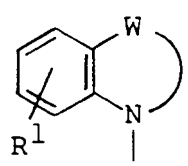 : 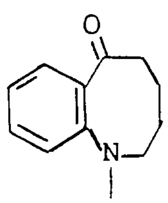

$R^2$: H

$R^3$: 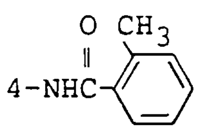

Crystalline form: Colorless amorphous

NMR analysis: 180)

Form: Free

Example 972

Structure

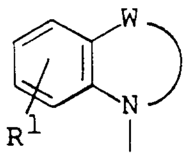 : 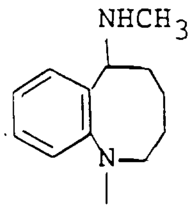

$R^2$: H

$R^3$: 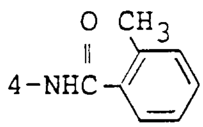

Crystalline form: Colorless needles

Recrystallization solvent: Ethanol

Melting Point: 206 - 207°C

Form: Free

Example 973

Structure

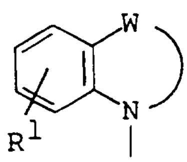 : 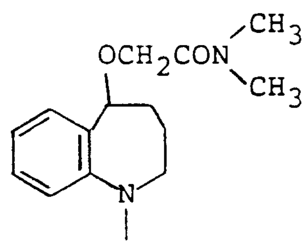

$R^2$: H

$R^3$: 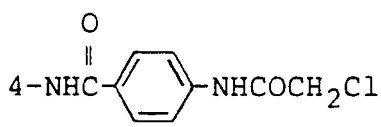

Crystalline form: Colorless amorphous

NMR analysis: 181)

Form: Free

Example 974

Structure

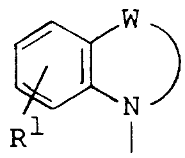 : 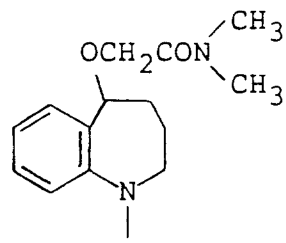 $R^2$: H

$R^3$: 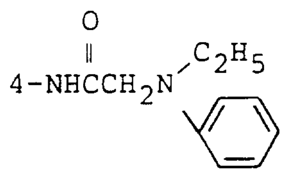

Crystalline form: White powder

Recrystallization solvent: Ethyl acetate/n-hexane

Melting Point: 152 - 154°C

Form: Free

---

Example 975

Structure

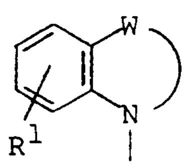 : 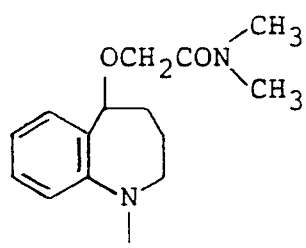 $R^2$: H

$R^3$: 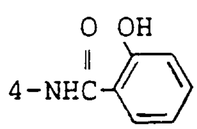

Crystalline form: Colorless amorphous

NMR analysis: 182)

Form: Free

Example 976

Structure

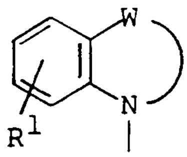

:

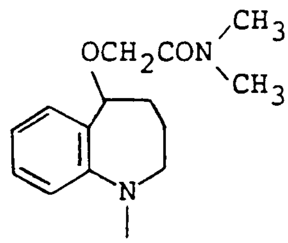

$R^2$: H

$R^3$: 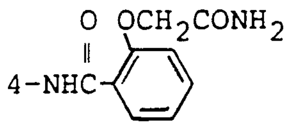

Crystalline form: White powder

Recrystallization solvent: Ethyl acetate/n-hexane

Melting Point: 204 - 206°C

Form: Free

Example 977

Structure

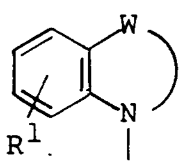

:

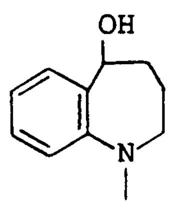

$R^2$: H

$R^3$: 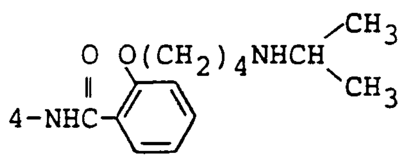

Crystalline form: Colorless needles

Recrystallization solvent: Ethyl acetate/n-hexane

Melting Point: 162 - 163°C

Form: Free

167) $^1$H-NMR ($CDCl_3$) δ ; 1.14-2.83 (13H, m), 2.43 (3H, s), 2.95-5.19 (4H, m), 4.12 (2H, t, J=6.2 Hz), 6.27-6.83 (2H, m), 6.83-7.36 (6H, m), 7.36-7.67 (4H, m), 7.93-8.11 (1H, m), 9.77 (1H, brs)

168) $^1$H-NMR ($CDCl_3$) δ ; 1.11-2.98 (11H, m), 2.80 (3H, s), 3.69 (2H, s), 2.98-5.24 (2H, m), 6.50-7.71 (12H, m), 9.37 (1H, brs)

169) $^1$H-NMR ($CDCl_3$) δ ; 1.10-2.80 (14H, m), 2.99 (3H, s), 3.39-5.20 (2H, m), 4.00 (2H, s), 6.49-7.67 (12H, m), 8.51 (1H, brs)

170) $^1$H-NMR ($CDCl_3$) δ ; 1.10-1.98 (3H, m), 1.98-2.82 (10H, m), 2.82-3.20 (2H, m), 3.34-5.15 (2H, m), 6.48-7.68 (15H, m), 7.86 (1H, brs)

171) $^1$H-NMR ($CDCl_3$) δ ; 1.10-2.84 (10H, m), 2.40 (3H, s), 2.90-5.20 (2H, m), 3.79 (2H, d, J=2.7 Hz), 4.33 (1H, br), 6.30-7.68 (12H, m), 8.67 (1H, brs)

172) $^1$H-NMR ($CDCl_3$) δ ; 1.10-2.85 (14H, m), 2.72 (3H, s), 2.98-5.20 (2H, m), 3.62 (2H, s), 6.50-7.75 (12H, m), 9.18 (1H, brs)

173) $^1$H-NMR (DMSO-$d_6$) δ ; 1.28-2.62 (4H, m), 2.07 (3H, s), 2.34 (6H, s), 3.04-3.57 (2H, m), 3.99-4.86 (1H, m), 6.62-7.88 (12H, m), 10.12-10.20 (2H, m)

174) $^1$H-NMR ($CDCl_3$) δ ; 1.39 (3H, t, J=7.1 Hz), 1.64-2.68 (4H, m), 2.42 (6H, s), 3.04-3.58 (2H, m), 3.98-5.01 (1H, m), 4.38 (2H, q, J=7.1 Hz), 6.57-8.57 (13H, m)

175) $^1$H-NMR (DMSO-$d_6$) δ ; 1.67-5.02 (7H, m), 3.35 (6H, s), 6.75-8.17 (12H, m), 8.46 (1H, s), 10.54 (1H, s)

176) $^1$H-NMR ($CDCl_3$) δ ; 1.21 (3H, t, J=7.1 Hz), 1.95-2.30 (2H, m), 2.88 (2H, t, J=6.2 Hz), 3.40-3.65 (2H, m), 3.70-4.50 (2H, m), 3.91 (2H, s), 6.66 (1H, d, J=8.5 Hz), 6.70-7.00 (3H, m), 7.10-7.50 (7H, m), 7.81 (1H, d, J=2.5 Hz), 8.44 (1H, s)

177) $^1$H-NMR ($CDCl_3$) δ ; 1.21 (3H, t, J=7 Hz), 1.30-5.20 (11H, m), 3.48 (2H, q, J=7 Hz), 3.90 (2H, s), 6.53 (1H, d, J=8.3 Hz), 6.65-7.00 (4H, m), 7.00-7.40 (6H, m), 7.51 (1H, d, J=2.5 Hz), 8.40 (1H, s)

178) $^1$H-NMR ($CDCl_3$) δ ; 1.21 (3H, t, J=7 Hz), 1.20-5.20 (15H, m), 3.90 (2H, s), 6.48 (1H, d, J=8.3 Hz), 6.50-7.70 (11H, m), 8.39 (1H, s)

179) $^1$H-NMR ($CDCl_3$) δ ; 1.60-2.20 (1H, m), 2.10-2.35 (1H, m), 2.45 (3H, s), 2.70-2.95 (2H, m), 3.25-3.45 (1H, m), 4.60-4.85 (1H, m), 7.10-7.80 (12H, m)

180) $^1$H-NMR ($CDCl_3$) δ ; 1.65-2.15 (4H, m), 2.46 (3H, s), 2.6-5.15 (4H, m), 6.75-6.95 (1H, m), 7.15-7.55 (10H, m), 7.61 (1H, s), 7.95-8.1 (1H, m)

181) $^1$H-NMR ($CDCl_3$) δ ; 1.60-2.15 (3H, m), 2.15-2.90 (2H, m), 2.90-3.22 (6H, m), 4.00-4.50 (2H, m), 4.13 (2H, s), 4.58-5.22 (2H, m), 6.53-6.80 (1H, m), 6.90-7.90 (7H, m), 8.48 (1H, s)

182) $^1$H-NMR ($CDCl_3$) δ ; 1.48-2.20 (3H, m), 2.20-2.85 (2H, m), 2.85-3.27 (6H, m), 4.05-4.47 (2H, m),

4.47-5.22 (2H, m), 6.50-6.76 (1H, m), 6.76-6.91 (1H, m), 6.91-7.69 (9H, m), 7.69-8.13 (1H, m), 9.28 (1H, s), 11.87 (1H, brs)

Example 978

5-Dimethylamino-1-(2-methyl-4-aminobenzoyl)-2,3,4,5-tetrahydro-1H-benzazepine (H) (1.00 g) is dissolved in dichloromethane (30 ml), and thereto is added triethylamine (0.48 ml) under ice-cooling, and further added dropwise 2-methylbenzoyl chloride (0.44 ml). The mixture is stirred at room temperature for 1 hour. The reaction solution is washed with water, and dried over magnesium sulfate. The solvent is distilled off, and the resulting residue is crystallized by adding thereto ethyl acetate. The precipitated crystal is recrystallized from dichloromethane/ethyl acetate to give 5-dimethylamino-1-[2-methyl-4-(2-methylbenzoylamino)benzoyl]-2,3,4,5-tetrahydro-1H-benzazepine (0.92 g) as white powder, m.p. 191 - 192°C.

HPLC retention time: 7.5 minutes

Column; Wakosil II $5C_{18}$ (trade mark; Wako Pure Chemical Co., Ltd.)
Solvent; acetonitrile : 50 mN aqueous $Na_2SO_4$ solution : acetic acid = 27 : 73 : 1
Rate; 1.0 ml/min.

$[\alpha]_D^{22}$ = 0° (c=1.0, chloroform)

$^1$H-NMR ($CDCl_3$) δ ; 1.15-3.25 (17H, m), 3.35-5.14 (2H, m), 6.62-8.05 (12H, m)

Charts of $^1$H-NMR ($CDCl_3$) of the starting compound (H) and the compound obtained in Exmaple 978 are shown in Fig. 1 and Fig. 2, respectively.

Example 979

Using 5-dimethylamino-1-(2-methyl-4-aminobenzoyl)-2,3,4,5-tetrahydro-1H-benzazepine (G) (1.00 g), 5-dimethylamino-1-[2-methyl-4-(2-methylbenzoylamino)benzoyl]-2,3,4,5-tetrahydro-1H-benzazepine (0.48 g) is obtained in the same manner as in Example 978 except that methanol/diethyl ether is used instead of ethyl acetate as recrystallization solvent, as white powder, m.p. 183 - 185°C.

HPLC retention time : 8.1 minutes
(the conditions of HPLC are same as those in Example 978)

$[\alpha]_D^{22}$ = 0° (c=1.3, chloroform)

$^1$H-NMR ($CDCl_3$) δ ; 1.10-3.20 (17H, m), 3.35-5.15 (2H, m), 6.50-6.80 (1H, m), 6.86-7.62 (10H, m), 7.65-8.09 (1H, m)

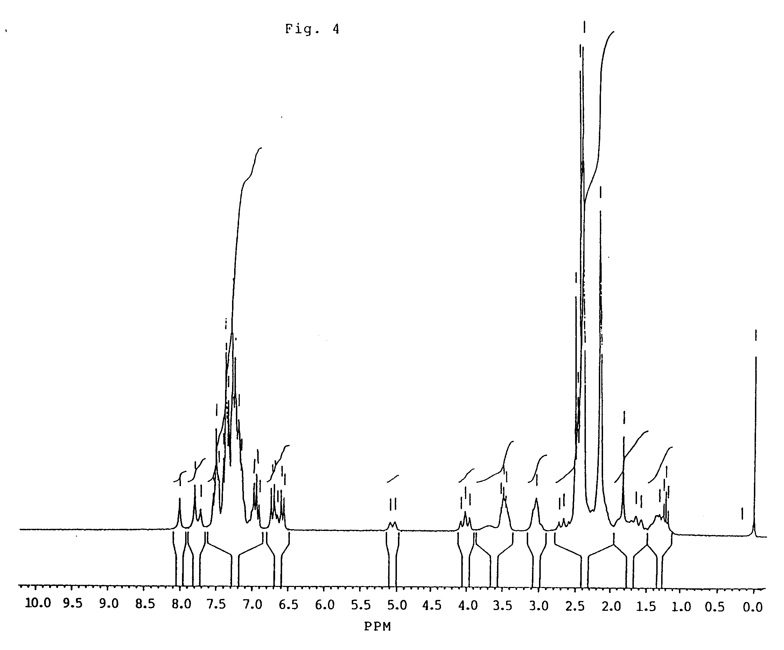

Charts of $^1$H-NMR ($CDCl_3$) of the starting compound (G) and the compound obtained in Exmaple 979 are shown in Fig. 3 and Fig. 4, respectively.

Reference Example 18

Using the suitable starting materials, the following compounds are obtained in the same manner as in Reference Example 1.

7-Methoxy-5-oxo-1-(4-nitrobenzoyl)-2,3,4,5-tetrahydro-1H-benzazepine, colorless needles, m.p. 178 - 178.5°C (recrystallized from ethyl acetate/n-hexane)

7-Methoxy-5-oxo-1-(2-chloro-4-nitrobenzoyl)-2,3,4,5-tetrahydro-1H-benzazepine, white powder, m.p. 150 - 151°C (recrystallized from ethyl acetate/n-hexane)

7-Methoxy-5-oxo-1-(3-methoxy-4-nitrobenzoyl)-2,3,4,5-tetrahydro-1H-benzazepine, white powder, m.p. 116 - 118°C (recrystallized from ethyl acetate/n-hexane)

7-Chloro-5-oxo-1-(3-methoxy-4-nitrobenzoyl)-2,3,4,5-tetrahydro-1H-benzazepine, yellow powder, m.p. 156 - 158°C (recrystallized from diethyl ether/dichloromethane)

Reference Example 19

Using the suitable starting materials, the following compounds are obtained in the same manner as in Reference Example 2.

7-Methoxy-5-oxo-1-(4-aminobenzoyl)-2,3,4,5-tetrahydro-1H-benzazepine, white powder, m.p. 172.5 - 173.5°C (recrystallized from ethyl acetate/n-hexane)

7-Methoxy-5-oxo-1-(2-chloro-4-aminobenzoyl)-2,3,4,5-tetrahydro-1H-benzazepine, white powder, m.p. 153 - 155°C (recrystallized from ethyl acetate/n-hexane)

7-Methoxy-5-oxo-1-(3-methoxy-4-aminobenzoyl)-2,3,4,5-tetrahydro-1H-benzazepine, colorless needles, m.p. 170 - 171°C (recrystallized from ethyl acetate/n-hexane)

7-Chloro-5-oxo-1-(3-methoxy-4-aminobenzoyl)-2,3,4,5-tetrahydro-1H-benzazepine, yellow oil

$^1$H-NMR ($CDCl_3$) δ ; 2.05-2.30 (2H, m), 2.85-3.00 (2H, m), 3.70 (3H, s), 3.85-4.30 (4H, m), 6.42 (1H, d, J=8.1 Hz), 6.64 (1H, dd, J=1.7 Hz, 8.1 Hz), 6.72 (1H, d, J=8.5 Hz), 6.80 (1H, d, J=1.8 Hz), 7.19 (1H, dd, J=2.6 Hz, 8.5 Hz), 7.81 (1H, d, J=2.5 Hz)

Using the suitable starting materials, the compounds of the following Table 7 are obtained in the same manner as in above Examples 1 and 382.

Table 7

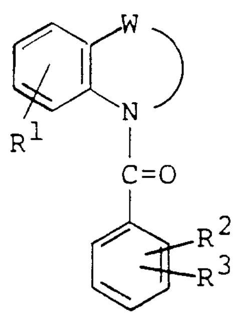

Example 980

Structure

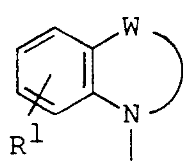 : 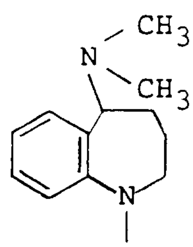

$R^2$: H

$R^3$: 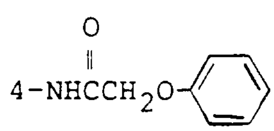

Crystalline form: Colorless amorphous

NMR analysis: 183)

Form: Free

Example 981

Structure

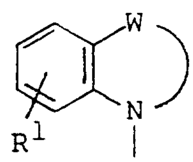 : 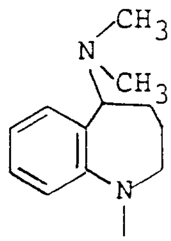

$R^2$: H

$R^3$: 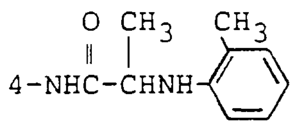

Crystalline form: Colorless amorphous

NMR analysis: 184)

Form: Free

Example 982

Structure

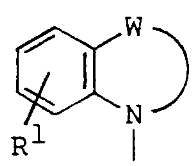 : 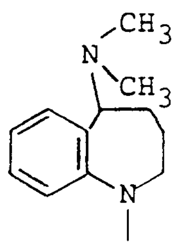

$R^2$: H

$R^3$: 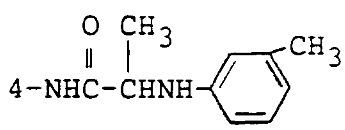

Crystalline form: Colorless amorphous

NMR analysis: 185)

Form: Free

Example 983

Structure

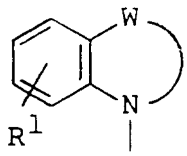 : 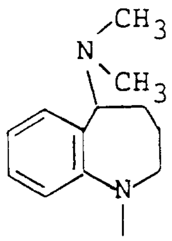

$R^2$: H

$R^3$: 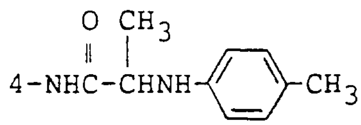

Crystalline form: Colorless amorphous

NMR analysis: 186)

Form: Free

Example 984

Structure

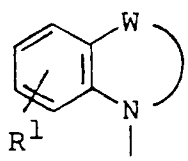 : 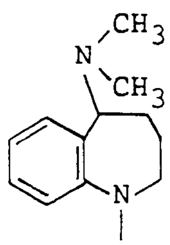

$R^2$: H

$R^3$: 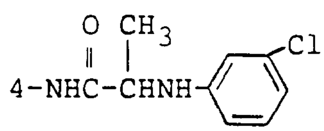

Crystalline form: Colorless amorphous

NMR analysis: 187)

Form: Free

Example 985

Structure

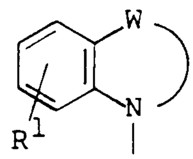
:
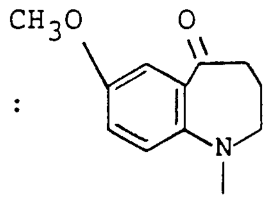

$R^2$: 2-Cl

$R^3$: 4-NHCO-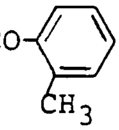

Crystalline form: Colorless amorphous

NMR analysis: 188)

Form: Free

Example 986

Structure

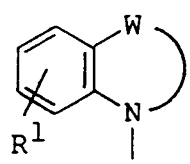
:
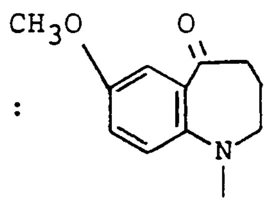

$R^2$: 2-Cl

$R^3$: 4-NHCO-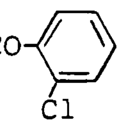

Crystalline form: Colorless amorphous

NMR analysis: 189)

Form: Free

Example 987

Structure 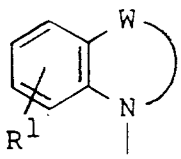 : 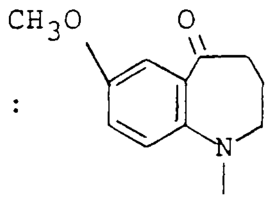 $R^2$: H

$R^3$: 4-NHCO- 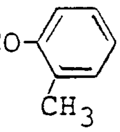

Crystalline form: White powder

Recrystallization solvent: Ethanol/water

Melting Point: 267 - 268°C

Form: Free

Example 988

Structure 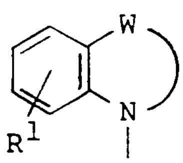 : 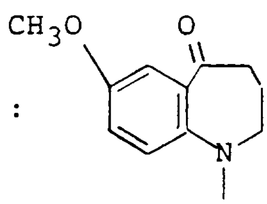 $R^2$: H

$R^3$: 4-NHCO- 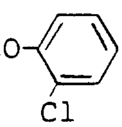

Crystalline form: White powder

Recrystallization solvent: Ethanol/water

Melting Point: 264 - 266°C

Form: Free

Example 989

Structure

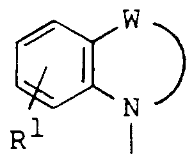 : 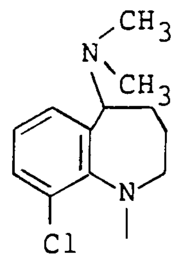

$R^2$: H

$R^3$: 4-NHCO- 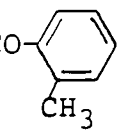

Crystalline form: White powder

Recrystallization solvent: Dichloromethane/diethyl ether

Melting Point: 218 - 220°C

Form: Free

Example 990

Structure

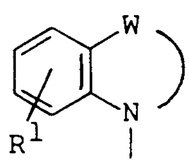 : 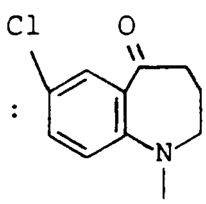

$R^2$: 3-$OCH_3$

$R^3$: 4-NHCO- 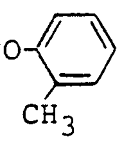

Crystalline form: Yellow oil

NMR analysis: 190)

Form: Free

Example 991

Structure

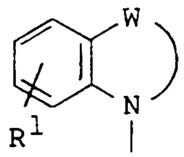 : 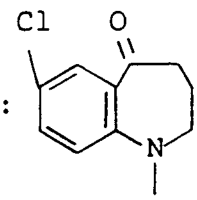

$R^2$: 3-$OCH_3$

$R^3$: 4-NHCO- 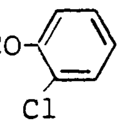

Crystalline form: Yellow oil

NMR analysis: 191)

Form: Free

Example 992

Structure

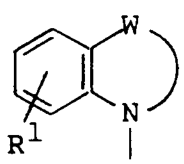 : 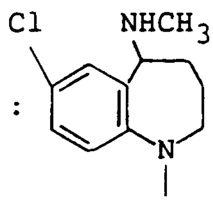

$R^2$: 3-$OCH_3$

$R^3$: 4-NHCO- 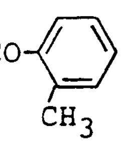

Crystalline form: Yellow powder

Recrystallization solvent: Dichloromethane/diethyl ether

Melting Point: 174 - 177°C

Form: Free

Example 993

Structure

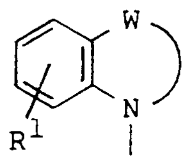 : 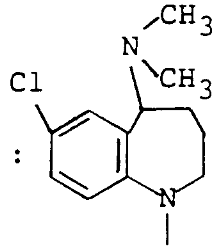

$R^2$: 3-$OCH_3$

$R^3$: 4-NHCO- 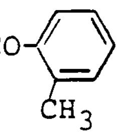

Crystalline form: Yellow amorphous

NMR analysis: 192)

Form: Free

Example 994

Structure

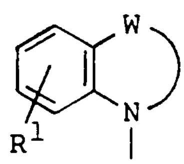 : 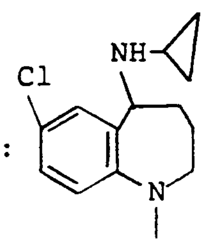

$R^2$: 3-$OCH_3$

$R^3$: 4-NHCO- 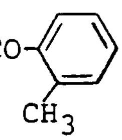

Crystalline form: Colorless amorphous

NMR analysis: 193)

Form: Free

Example 995

Structure

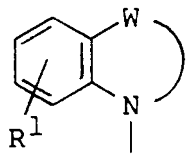
:
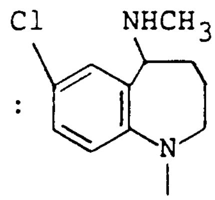

$R^2$: 3-$OCH_3$

$R^3$: 4-NHCO- 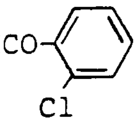

Crystalline form: White powder

Recrystallization solvent: Diethyl ether/dichloromethane

Melting Point: 163 - 165°C

Form: Free

Example 996

Structure

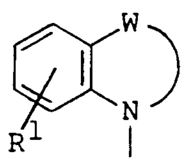
:
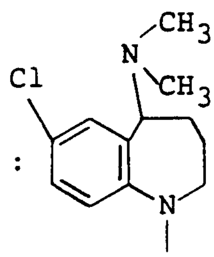

$R^2$: 3-$OCH_3$

$R^3$: 4-NHCO- 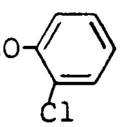

Crystalline form: Colorless amorphous

NMR analysis: 194)

Form: Free

Example 997

Structure

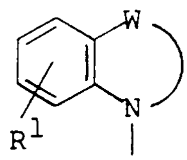 : 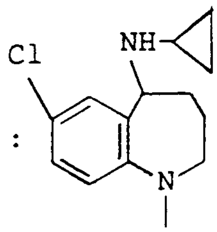

$R^2$: 3-$OCH_3$

$R^3$: 4-NHCO- 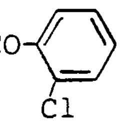

Crystalline form: Colorless amorphous

NMR analysis: 195)

Form: Free

183) $^1$H-NMR ($CDCl_3$) δ ; 1.10-2.83 (11H, m), 2.96-5.21 (2H, m), 4.55 (2H, s), 6.48-7.72 (13H, m), 8.30 (1H, brs)

184) $^1$H-NMR ($CDCl_3$) δ ; 1.10-2.85 (11H, m), 1.58 (3H, d, J=6.8 Hz), 2.23 (3H, s), 2.95-5.19 (4H, m), 6.38-7.70 (12H, m), 8.69 (1H, brs)

185) $^1$H-NMR ($CDCl_3$) δ ; 1.10-2.85 (14H, m), 2.26 (3H, s), 2.96-5.19 (4H, m), 6.36-7.68 (12H, m), 8.72 (1H, brs)

186) $^1$H-NMR ($CDCl_3$) δ ; 1.09-2.72 (11H, m), 1.53 (3H, d, J=6.9 Hz), 2.24 (3H, s), 2.93-5.21 (4H, m), 6.30-7.78 (12H, m), 8.76 (1H, brs)

187) $^1$H-NMR ($CDCl_3$) δ ; 1.10-2.82 (14H, m), 2.96-5.20 (4H, m), 6.38-7.70 (12H, m), 8.54 (1H, brs)

188) $^1$H-NMR ($CDCl_3$) δ ; 1.64-2.28 (2H, m), 2.41 (3H, s), 2.60-2.90 (2H, m), 2.90-3.70 (1H, m), 3.76 (3H, s), 4.10-5.10 (1H, m), 6.60-7.70 (10H, m), 8.51 (1H, s)

189) $^1$H-NMR ($CDCl_3$) δ ; 1.64-2.43 (2H, m), 2.67-2.97 (2H, m), 3.00-3.70 (1H, m), 3.77 (3H, s), 4.20-5.10 (1H, m), 6.60-7.75 (10H, m), 8.51 (1H, s)

190) $^1$H-NMR ($CDCl_3$) δ ; 2.00-2.35 (2H, m), 2.49 (3H, s), 2.89 (2H, t, J=6.2 Hz), 3.72 (3H, s), 3.40-4.80 (2H, m), 6.74 (2H, d, J=8.5 Hz), 6.80-7.00 (2H, m), 7.25-7.60 (5H, m), 7.80 (1H, d, J=2.6 Hz), 8.16 (1H, s), 8.37 (1H, d, J=8.6 Hz)

191) $^1$H-NMR ($CDCl_3$) δ ; 1.90-2.40 (2H, m), 2.90 (2H, t, J=6.2 Hz), 3.75 (3H, s), 3.40-4.80 (2H, m), 6.74 (1H, d, J=8.5 Hz), 6.80-7.00 (2H, m), 7.10-7.50 (4H, m), 7.73 (1H, dd, J=2.3 Hz, 6 Hz), 7.80 (1H, d, J=2.5 Hz), 8.38 (1H, d, J=8.8 Hz), 8.65 (1H, s)

192) $^1$H-NMR ($CDCl_3$) δ ; 1.10-2.10 (13H, m), 2.90-5.20 (6H, m), 6.56 (1H, d, J=8.4 Hz), 6.69 (1H, d, J=7 Hz), 6.85-7.70 (7H, m), 8.15 (1H, s), 8.31 (1H, d, J=8.4 Hz)

193) $^1$H-NMR ($CDCl_3$) δ ; 0.30-0.65 (4H, m), 1.20-2.50 (7H, m), 2.50 (3H, s), 3.10-5.20 (2H, m), 3.75 (3H, s), 6.60 (1H, d, J=8.3 Hz), 6.70-7.60 (8H, m), 8.14 (1H, s), 8.20-8.40 (1H, m)

194) $^1$H-NMR ($CDCl_3$) δ ; 0.80-2.50 (10H, m), 2.90-4.10 (6H, m), 6.50-7.80 (9H, m), 8.32 (1H, d, J=8 Hz), 8.62 (1H, s)

195) $^1$H-NMR ($CDCl_3$) δ ; 0.30-0.65 (4H, m), 0.70-2.40 (6H, m), 2.60-5.20 (6H, m), 6.50-7.80 (9H, m), 8.30 (1H, d, J=8 Hz), 8.62 (1H, s)

Reference Example 20

Using the suitable starting materials, the following compounds are obtained in the same manner as in Reference Example 1.

7-Methyl-5-oxo-1-(4-nitrobenzoyl)-2,3,4,5-tetrahydro-1H-benzazepine, white needles

$^1$H-NMR ($CDCl_3$) δ ; 2.20 (2H, brs), 2.32 (3H, s), 2.88 (2H, t, J=6.3 Hz), 3.40-4.79 (2H, m), 6.57 (1H, d, J=8.0 Hz), 7.04 (1H, d, J=7.7 Hz), 7.36 (2H, d, J=8.6 Hz), 7.62 (1H, d, J=1.7 Hz), 8.04 (2H, d, J=8.7 Hz)

7-Dimethylamino-5-oxo-1-(4-nitrobenzoyl)-2,3,4,5-tetrahydro-1H-benzazepine, red brown prisms (recrystallized from dichloromethane/diethyl ether)

$^1$H-NMR ($CDCl_3$) δ ; 1.75-2.47 (2H, m), 2.60-3.62, 4.51-4.92 (total 4H, m), 2.93 (6H, s), 6.46 (1H, dd, J=2.2 Hz, 7.0 Hz), 6.52 (1H, d, J=7.0 Hz), 7.33 (2H, d, J=7.0 Hz), 8.00 (2H, d, J=7.0 Hz)

7-Bromo-5-oxo-1-(4-nitrobenzoyl)-2,3,4,5-tetrahydro-1H-benzazepine, white powder (recrystallized from dichloromethane/diethyl ether), m.p. 177 - 182°C

7-Chloro-5-oxo-1-(2-methyl-4-nitrobenzoyl)-2,3,4,5-tetrahydro-1H-benzazepine, white powder (recrystallized from dichloromethane/diethyl ether)

$^1$H-NMR ($CDCl_3$) δ ;1.78-2.37 (2H, m), 2.48 (3H, s), 2.88 (2H, t, J=6.1 Hz), 3.30-5.12 (2H, m), 6.47-6.82 (1H, m),

6.82-7.09 (1H, m), 7.09-7.27 (1H, m), 7.48-8.35 (3H, m)

6-Oxo-1-(2-chloro-4-nitrobenzoyl)-1,2,3,4,5,6-hexahydrobenzazocine, yellow amorphous

$^1$H-NMR ($CDCl_3$) δ ; 1.7-2.1 (4H, m), 2.85-4.7 (4H, m), 7.12 (1H, d, J=8.4 Hz), 7.17-7.51 (4H, m), 7.89 (1H, dd, J=7.8 Hz, 2.1 Hz), 8.11 (1H, d, J=2.2 Hz)

8-Chloro-6-oxo-1-(2-chloro-4-nitrobenzoyl)-1,2,3,4,5,6-hexahydrobenzazocine, yellow amorphous

$^1$H-NMR ($CDCl_3$) δ ; 1.7-2.15 (4H, m), 2.85-4.8 (4H, m), 7.14 (1H, d, J=8.5 Hz), 7.16 (1H, d, J=8.4 Hz), 7.34 (1H, dd, J=8.3 Hz, 2.5 Hz), 7.85 (1H, d, J=2.5 Hz), 7.94 (1H, dd, J=8.4 Hz, 2.2 Hz), 8.13 (1H, d, J=2.1 Hz)

8-Methyl-6-oxo-1-(2-chloro-4-nitrobenzoyl)-1,2,3,4,5,6-hexahydrobenzazocine, yellow amorphous

$^1$H-NMR ($CDCl_3$) δ ; 1.65-2.2 (4H, m), 2.33 (3H, s), 2.7-5.0 (4H, m), 7.0-7.25 (3H, m), 7.67 (1H, d, J=2.0 Hz), 7.89 (1H, dd, J=8.4 Hz, 2.2 Hz), 8.10 (1H, d, J=2.1 Hz)

8-Methoxy-6-oxo-1-(2-chloro-4-nitrobenzoyl)-1,2,3,4,5,6-hexahydrobenzazocine, light yellow amorphous

$^1$H-NMR ($CDCl_3$) δ ; 1.6-2.05 (4H, m), 2.8-5.2 (4H, m), 3.78 (3H, s), 6.88 (1H, dd, J=8.6 Hz, 3.1 Hz), 7.11 (1H, d, J=8.4 Hz), 7.12 (1H, d, J=8.6 Hz), 7.38 (1H, d, J=3.0 Hz), 7.90 (1H, dd, J=8.4 Hz, 2.2 Hz), 8.11 (1H, d, J=2.2 Hz)

7-Chloro-5-oxo-1-(2-chloro-4-nitrobenzoyl)-2,3,4,5-tetrahydro-1H-benzazepine, yellow powder (recrystallized from diethyl ether/dichloromethane), m.p. 125 - 126.5°C

Reference Example 21

Using the suitable starting materials, the following compounds are obtained in the same manner as in Reference Example 2.

7-Methyl-5-oxo-1-(4-aminobenzoyl)-2,3,4,5-tetrahydro-1H-benzazepine, yellow powder

$^1$H-NMR ($CDCl_3$) δ ; 2.13 (2H, brs), 2.32 (3H, s), 2.86 (2H, t, J=6.2 Hz), 2.89-5.29 (2H, m), 3.86 (2H, brs), 6.41 (2H, m), 6.65 (1H, d, J=8.1 Hz), 7.06 (3H, m), 7.65 (1H, d, J=1.7 Hz)

7-Dimethylamino-5-oxo-1-(4-aminobenzoyl)-2,3,4,5-tetrahydro-1H-benzazepine, yellow needles (recrystallized from dichloromethane/diethyl ether)

$^1$H-NMR ($CDCl_3$) δ ; 1.78-2.49 (2H, m), 2.64-3.78, 4.07-5.02 (total 4H, m), 2.93 (6H, m), 3.96 (2H, m), 6.38 (2H, d, J=8.7 Hz), 6.55 (1H, dd, J=2.7, 8.7 Hz), 6.62 (1H, d, J=8.7 Hz), 6.96-7.18 (3H, m)

7-Bromo-5-oxo-1-(4-aminobenzoyl)-2,3,4,5-tetrahydro-1H-benzazepine, white powder (recrystallized from methanol/diethyl ether)

$^1$H-NMR ($CDCl_3$) δ ; 1.98-2.37 (2H, m), 2.88 (2H, t, J=6.3 Hz), 3.52-4.55 (4H, m), 6.28-6.57 (2H, m), 6.57-6.76 (1H, m), 6.92-7.20 (2H, m), 7.28-7.42 (1H, m), 7.90-8.09 (1H, m)

7-Chloro-5-oxo-1-(2-methyl-4-aminobenzoyl)-2,3,4,5-tetrahydro-1H-benzazepine, white powder (recrystallized from dichloromethane/diethyl ether), m.p. 190 - 191°C

6-Oxo-1-(2-chloro-4-aminobenzoyl)-1,2,3,4,5,6-hexahydrobenzazocine, light yellow amorphous

$^1$H-NMR ($CDCl_3$) δ ; 1.3-2.25 (4H, m), 2.8-4.4 (6H, m), 6.1-6.9 (3H, m), 6.95-7.75 (3H, m), 7.8-8.3 (1H, m)

8-Chloro-6-oxo-1-(2-chloro-4-aminobenzoyl)-1,2,3,4,5,6-hexahydrobenzazocine, light yellow amorphous

$^1$H-NMR ($CDCl_3$) δ ; 1.59-2.2 (4H, m), 2.6-4.4 (6H, m), 6.1-6.9 (3H, m), 6.95-7.5 (2H, m), 7.8-8.05 (1H, m)

7-Chloro-5-oxo-1-(2-chloro-4-aminobenzoyl)-2,3,4,5-tetrahydro-1H-benzazepine, yellow powder (recrystallized from diethyl ether/dichloromethane), m.p. 188 - 191.5°C

Using the suitable starting materials, the compounds of the following Table 8 are obtained in the same manner

as in above Examples 1 and 382.

Table 8

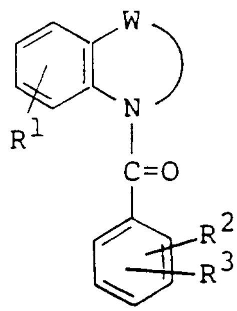

Example 998

Structure

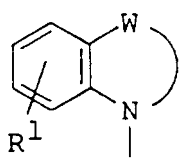 : 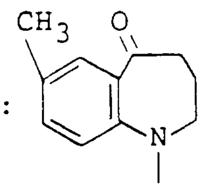

$R^2$: H

$R^3$: 4-NHCO- 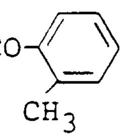

Crystalline form: White powder

NMR analysis: 196)

Form: Free

Example 999

Structure

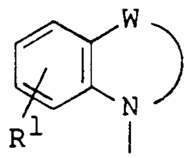 : 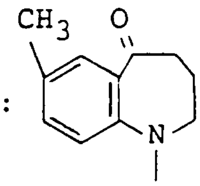

$R^2$: H

$R^3$: 4-NHCO- 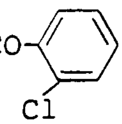

Crystalline form: White powder

NMR analysis: 197)

Form: Free

Example 1000

Structure

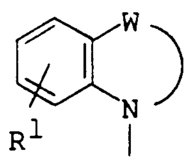 : 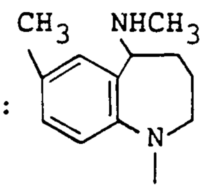

$R^2$: H

$R^3$: 4-NHCO- 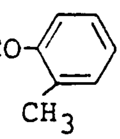

Crystalline form: White powder

Recrystallization solvent: Dichloromethane/diethyl ether

Melting Point: 200 - 205°C

Form: Free

Example 1001

Structure

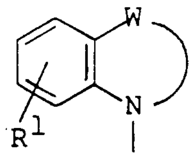 : 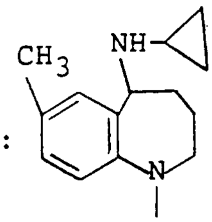

$R^2$: H

$R^3$: 4-NHCO- 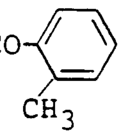

Crystalline form: Colorless amorphous

NMR analysis: 198)

Form: Free

Example 1002

Structure

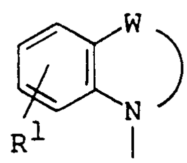 : 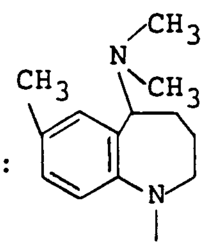

$R^2$: H

$R^3$: 4-NHCO- 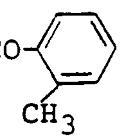

Crystalline form: White powder

Recrystallization solvent: Dichloromethane/diethyl ether

Melting Point: 234 - 238°C

Form: Free

Example 1003

Structure

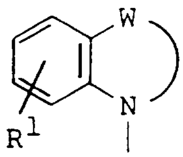 : 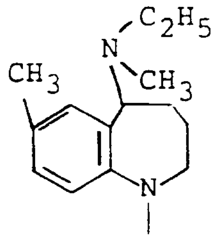 $R^2$: H

$R^3$: 4-NHCO- 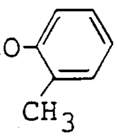

Crystalline form: White powder

Recrystallization solvent: Ethyl acetate/n-hexane

Melting Point: 174 - 178°C

Form: Free

Example 1004

Structure

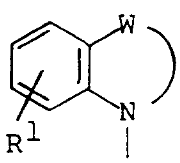 : 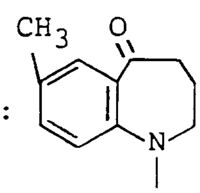 $R^2$: 2-Cl

$R^3$: 4-NHCO- 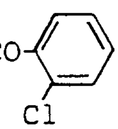

Crystalline form: Light yellow amorphous

NMR analysis: 199)

Form: Free

Example 1005

Structure

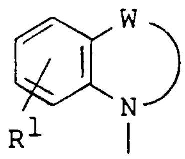
:
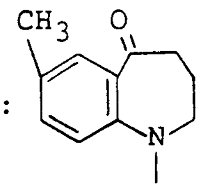

$R^2$: 2-Cl

$R^3$: 4-NHCO-
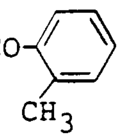

Crystalline form: Light yellow amorphous

NMR analysis: 200)

Form: Free

Example 1006

Structure

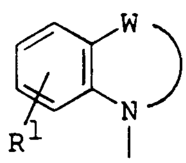
:
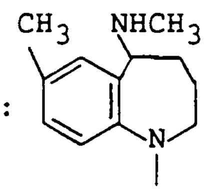

$R^2$: 2-Cl

$R^3$: 4-NHCO-
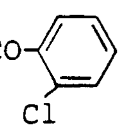

Crystalline form: Light yellow amorphous

NMR analysis: 201)

Form: Free

Example 1007

Structure

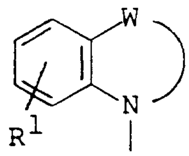 : 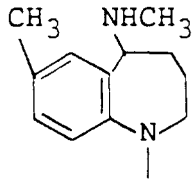

$R^2$: 2-Cl

$R^3$: 4-NHCO- 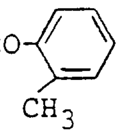

Crystalline form: Light yellow amorphous

NMR analysis: 202)

Form: Free

Example 1008

Structure

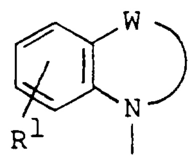 : 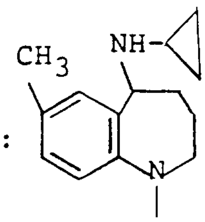

$R^2$: 2-Cl

$R^3$: 4-NHCO- 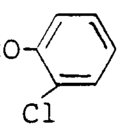

Crystalline form: Light yellow amorphous

MNR analysis: 203)

Form: Free

Example 1009

Structure

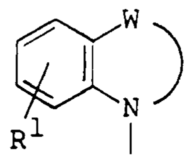

:

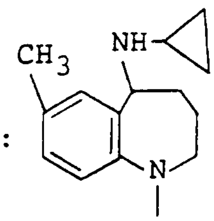

$R^2$: 2-Cl

$R^3$: 4-NHCO-

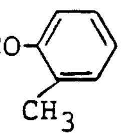

Crystalline form: Light yellow amorphous

NMR analysis: 204)

Form: Free

Example 1010

Structure

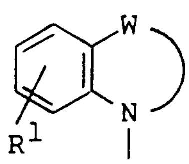

:

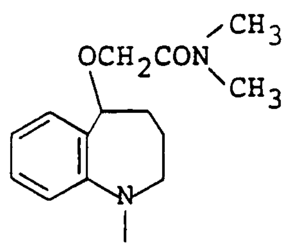

$R^2$: H

$R^3$: 4-NHCO-

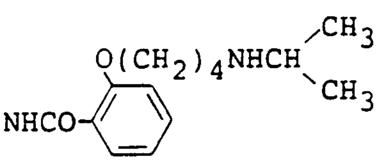

Crystalline form: Colorless amorphous

NMR analysis: 205)

Form: Free

Example 1011

Structure

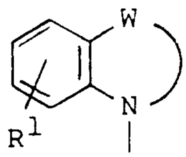
:
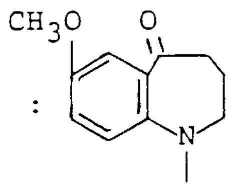

$R^2$: 3-$OCH_3$

$R^3$: 4-NHCO- 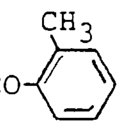

Crystalline form: White powder

Recrystallization solvent: Ethyl acetate/n-hexane

Melting Point: 153 - 155°C

Form: Free

Example 1012

Structure

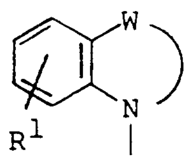
:
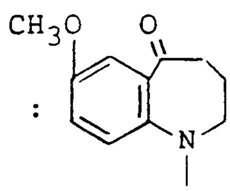

$R^2$: 3-$OCH_3$

$R^3$: 4-NHCO- 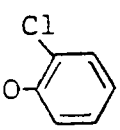

Crystalline form: White powder

Recrystallization solvent: Ethyl acetate/n-hexane

Melting Point: 142 - 143°C

Form: Free

Example 1013

Structure

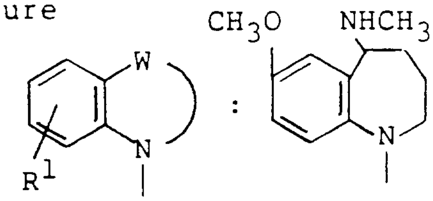

$R^2$: H

$R^3$: 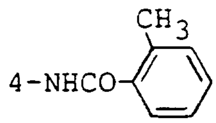

Crystalline form: White powder

Recrystallization solvent: Ethyl acetate/n-hexane

Melting Point: 176 - 178°C

Form: Free

Example 1014

Structure

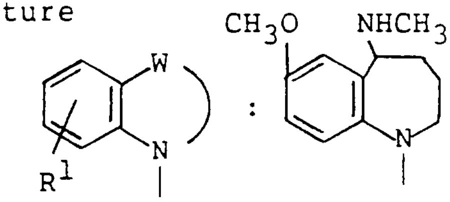

$R^2$: H

$R^3$: 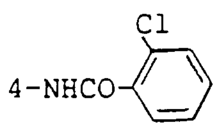

Crystalline form: White powder

Recrystallization solvent: Ethyl acetate/n-hexane

Melting Point: 186 - 188°C

Form: Free

Example 1015

Structure

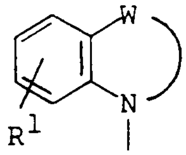 : 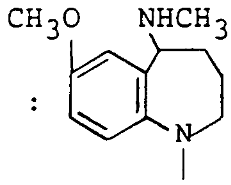

$R^2$: 2-Cl

$R^3$: 4-NHCO- 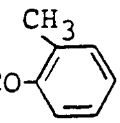

Crystalline form: Colorless amorphous

NMR analysis: 206)

Form: Free

---

Example 1016

Structure

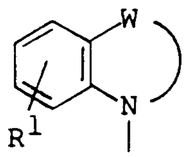 : 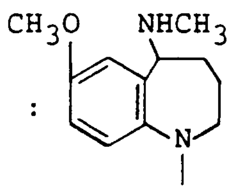

$R^2$: 2-Cl

$R^3$: 4-NHCO- 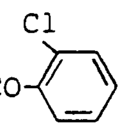

Crystalline form: Colorless amorphous

NMR analysis: 207)

Form: Free

Example 1017

Structure

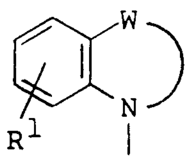 : 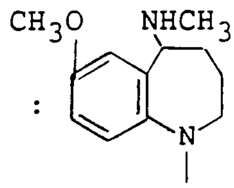

$R^2$: 3-$OCH_3$

$R^3$: 4-NHCO- 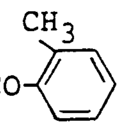

Crystalline form: White powder

Recrystallization solvent: Ethyl acetate/n-hexane

Melting Point: 191 - 191.5°C

Form: Free

Example 1018

Structure

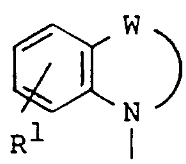 : 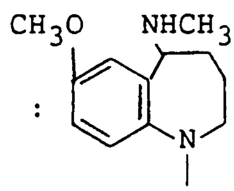

$R^2$: 3-$OCH_3$

$R^3$: 4-NHCO- 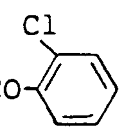

Crystalline form: White powder

Recrystallization solvent: Ethyl acetate/n-hexane

Melting Point: 210 - 212°C

Form: Free

Example 1019

Structure

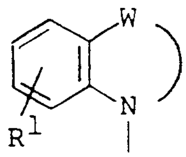 : 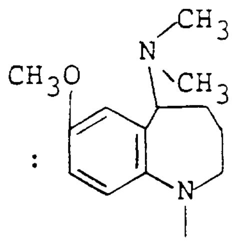

$R^2$: H

$R^3$: 4-NHCO- 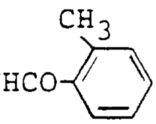

Crystalline form: White powder

Recrystallization solvent: Ethyl acetate/n-hexane

Melting Point: 196 - 198°C

Form: Free

Example 1020

Structure

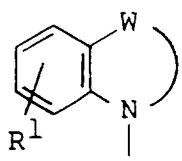 : 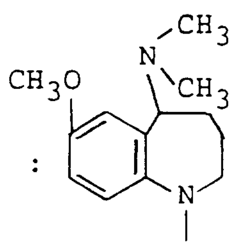

$R^2$: H

$R^3$: 4-NHCO- 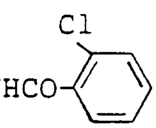

Crystalline form: Colorless amorphous

NMR analysis: 208)

Form: Free

Example 1021

Structure

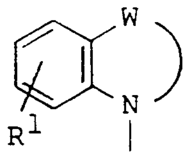 : 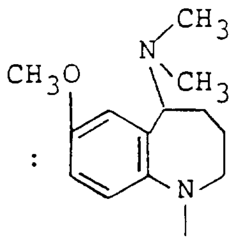

$R^2$: 2-Cl

$R^3$: 4-NHCO- 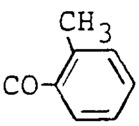

Crystalline form: Colorless amorphous

NMR analysis: 209)

Form: Free

Example 1022

Structure

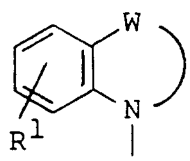 : 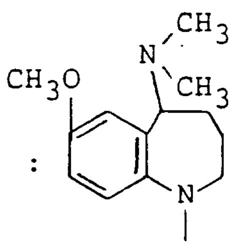

$R^2$: 2-Cl

$R^3$: 4-NHCO- 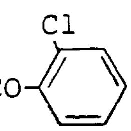

Crystalline form: Colorless amorphous

NMR analysis: 210)

Form: Free

Example 1023

Structure

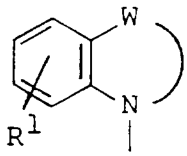 : 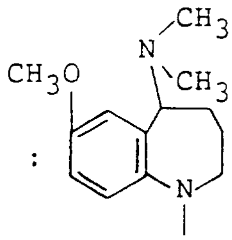

$R^2$: 3-$OCH_3$

$R^3$: 4-NHCO- 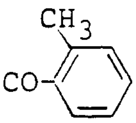

Crystalline form: Colorless amorphous

NMR analysis: 211)

Form: Free

---

Example 1024

Structure

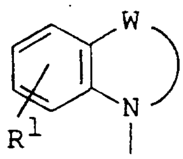 : 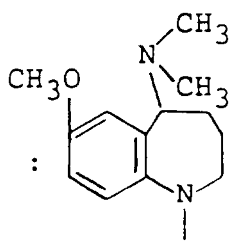

$R^2$: 3-$OCH_3$

$R^3$: 4-NHCO- 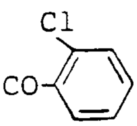

Crystalline form: Colorless amorphous

NMR analysis: 212)

Form: Free

Example 1025

Structure

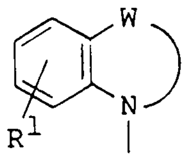 : 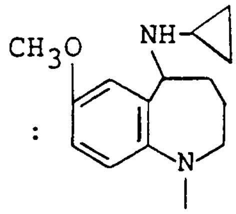

$R^2$: 2-Cl

$R^3$: 4-NHCO- 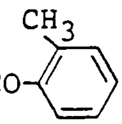

Crystalline form: Colorless amorphous

NMR analysis: 213)

Form: Free

Example 1026

Structure

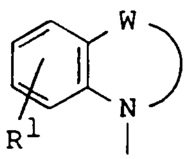 : 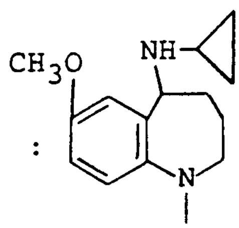

$R^2$: 2-Cl

$R^3$: 4-NHCO- 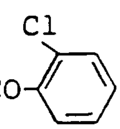

Crystalline form: Colorless amorphous

NMR analysis: 214)

Form: Free

Example 1027

Structure

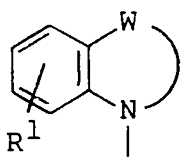 : 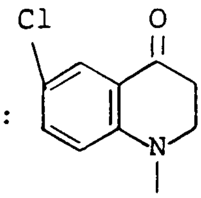

$R^2$: 2-Cl

$R^3$: 4-NHCO- 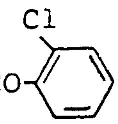

Crystalline form: Colorless prisms

Recrystallization solvent: Ethanol

Melting Point: 207 - 208°C

Form: Free

Example 1028

Structure

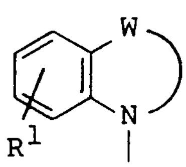 : 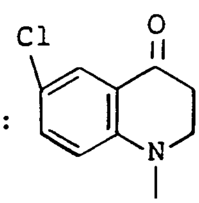

$R^2$: 2-Cl

$R^3$: 4-NHCO- 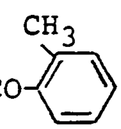

Crystalline form: White powder

Recrystallization solvent: Ethanol

Melting Point: 201 - 202°C

Form: Free

Example 1029

Structure

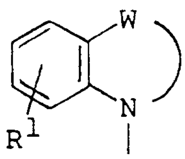 : 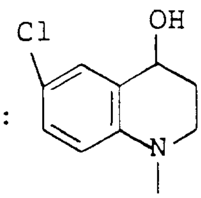

$R^2$: 2-Cl

$R^3$: 4-NHCO- 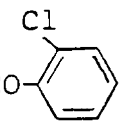

Crystalline form: White powder

Recrystallization solvent: Ethanol

Melting Point: 193 - 194°C

Form: Free

Example 1030

Structure

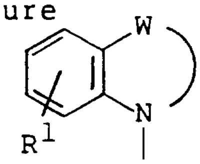 : 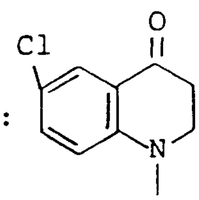

$R^2$: H

$R^3$: 4-NHCO- 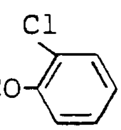

Crystalline form: White powder

Recrystallization solvent: Ethanol

Melting Point: 205 - 208°C

Form: Free

Example 1031

Structure

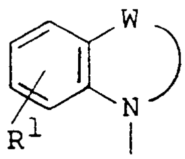

:

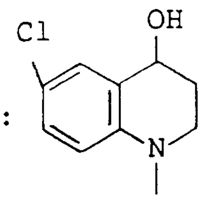

$R^2$: H

$R^3$: 4-NHCO-

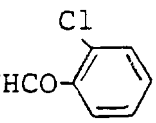

Crystalline form: White powder

Recrystallization solvent: Ethanol

Melting Point: 214 - 216°C

Form: Free

Example 1032

Structure

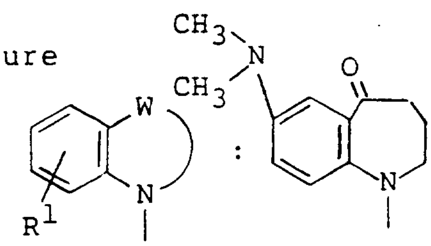

:

$R^2$: H

$R^3$: 4-NHCO-

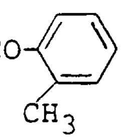

Crystalline form: Yellow needles

Recrystallization solvent: Ethanol

Melting Point: 223 - 226°C

Form: Free

Example 1033

Structure

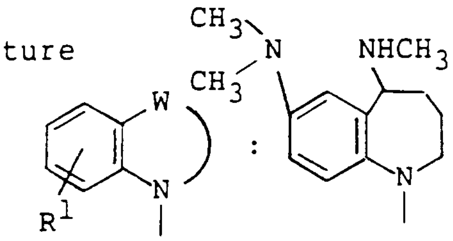

$R^2$: H

$R^3$: 4-NHCO-

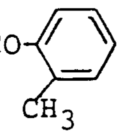

Crystalline form: Colorless needles

Recrystallization solvent: Ethanol/diethyl ether

Melting Point: 203 - 206°C

Form: Free

Example 1034

Structure

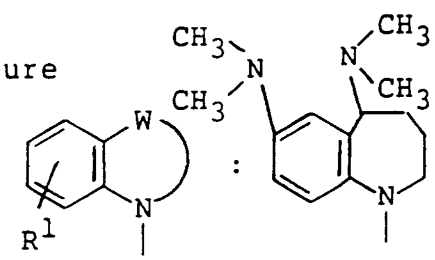

$R^2$: H

$R^3$: 4-NHCO-

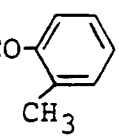

Crystalline form: Colorless needles

Recrystallization solvent: Ethanol/diethyl ether/n-hexane

Melting Point: 168 - 171°C

Form: Free

Example 1035

Structure

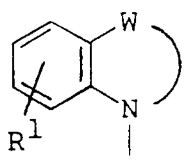 : 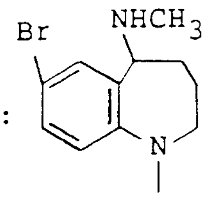 $R^2$: H

$R^3$: 4-NHCO- 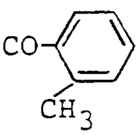

Crystalline form: White powder

Recrystallization solvent: Methanol/diethyl ether

Melting Point: 206 - 208°C

Form: Free

Example 1036

Structure

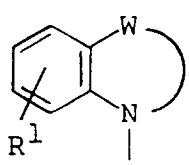 : 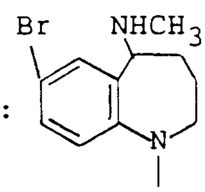 $R^2$: H

$R^3$: 4-NHCO- 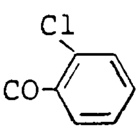

Crystalline form: White powder

Recrystallization solvent: Methanol/diethyl ether

Melting Point: 229 - 232°C

Form: Free

Example 1037

Structure

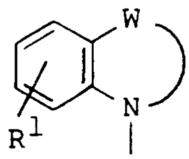 : 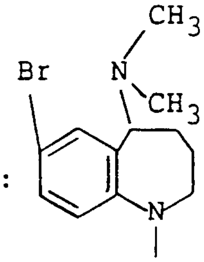

$R^2$: H

$R^3$: 4-NHCO- 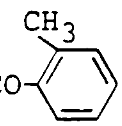

Crystalline form: White powder

Recrystallization solvent: Methanol/diethyl ether

Melting Point: 220 - 222°C

Form: Free

Example 1038

Structure

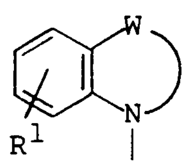 : 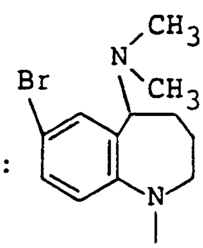

$R^2$: H

$R^3$: 4-NHCO- 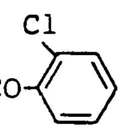

Crystalline form: White powder

Recrystallization solvent: Methanol/diethyl ether

Melting Point: 232 - 233.5°C

Form: Free

Example 1039

Structure

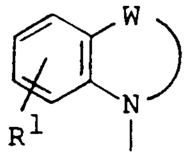 : 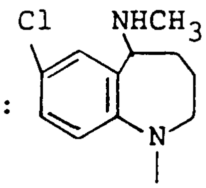

$R^2$: 2-$CH_3$

$R^3$: 4-NHCO- 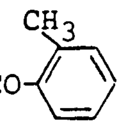

Crystalline form: Colorless amorphous

NMR analysis: 215)

Form: Free

Example 1040

Structure

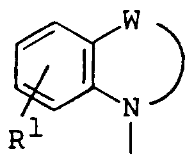 : 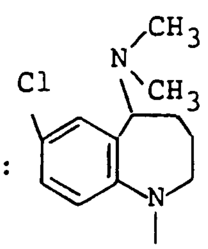

$R^2$: 2-$CH_3$

$R^3$: 4-NHCO- 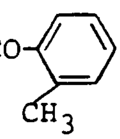

Crystalline form: Colorless amorphous

NMR analysis: 216)

Form: Free

Example 1041

Structure

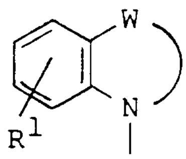 : 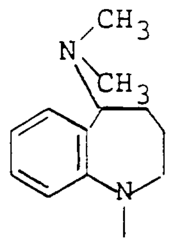

$R^2$: H

$R^3$: 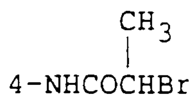

Crystalline form: White powder

Recrystallization solvent: Methanol/diethyl ether

Melting Point: 147 - 151°C

Form: Free

Example 1042

Structure

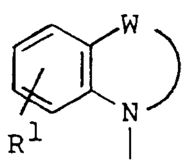 : 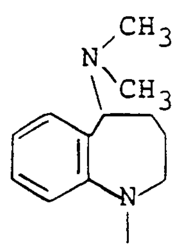

$R^2$: H

$R^3$: 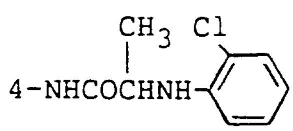

Crystalline form: White powder

Recrystallization solvent: Methanol/n-hexane

Melting Point: 127 - 129°C

Form: Free

Example 1043

Structure

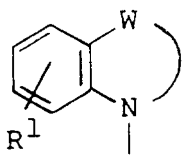 : 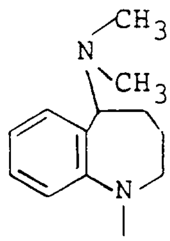

$R^2$: H

$R^3$: 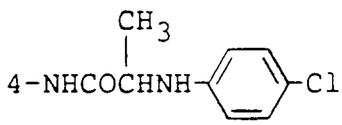

Crystalline form: White powder

Recrystallization solvent: Methanol/n-hexane

Melting Point: 109 - 112°C

Form: Free

Example 1044

Structure

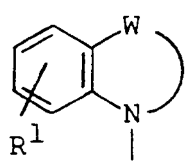 : 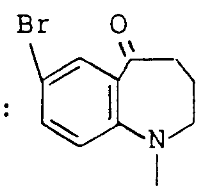

$R^2$: H

$R^3$: 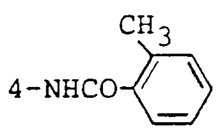

Crystalline form: White powder

Recrystallization solvent: Methanol/diethyl ether

Melting Point: 198 - 200°C

Form: Free

Example 1045

Structure

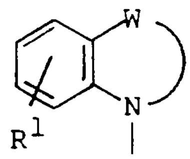 : 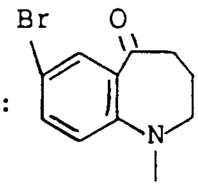 $R^2$: H

$R^3$: 4-NHCO-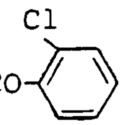

Crystalline form: White powder

Recrystallization solvent: Methanol/diethyl ether

Melting Point: 210 - 211°C

Form: Free

Example 1046

Structure

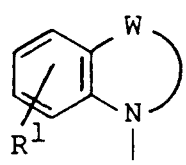 : 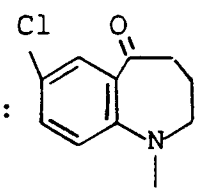 $R^2$: 2-$CH_3$

$R^3$: 4-NHCO-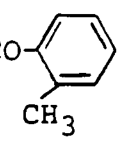

Crystalline form: Colorless amorphous

NMR analysis: 217)

Form: Free

Example 1047

Structure

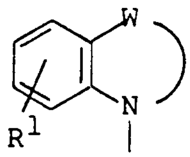 : 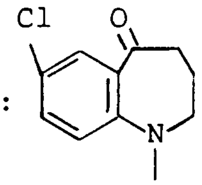

$R^2$: 2-$CH_3$

$R^3$: 4-NHCO- 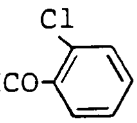

Crystalline form: Colorless amorphous

NMR analysis: 218)

Form: Free

Example 1048

Structure

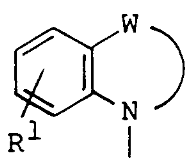 : 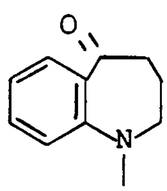

$R^2$: H

$R^3$: 4-NHCO- 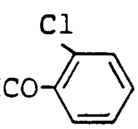

Crystalline form: Colorless amorphous

NMR analysis: 219)

Form: Free

Example 1049

Structure

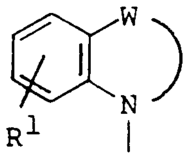 : 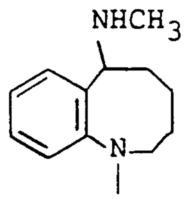

$R^2$: H

$R^3$: 4-NHCO- 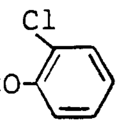

Crystalline form: Colorless needles

Recrystallization solvent: Ethanol

Melting Point: 243 - 243.5°C

Form: Free

Example 1050

Structure

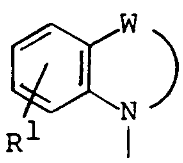 : 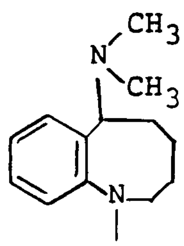

$R^2$: H

$R^3$: 4-NHCO- 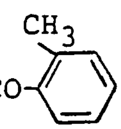

Crystalline form: Colorless prisms

Recrystallization solvent: Ethanol/petroleum ether

Melting Point: 207 - 209°C

Form: Free

Example 1051

Structure

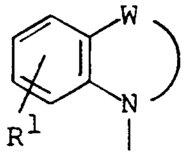 : 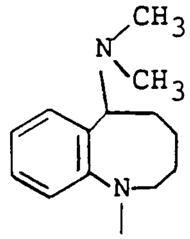

$R^2$: H

$R^3$: 4-NHCO- 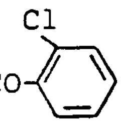

Crystalline form: Colorless needles

Recrystallization solvent: Ethanol/petroleum ether

Melting Point: 239 - 241°C

Form: Free

---

Example 1052

Structure

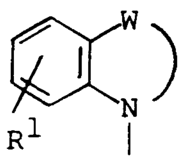 : 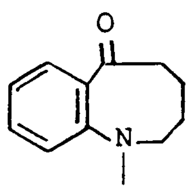

$R^2$: 2-Cl

$R^3$: 4-NHCO- 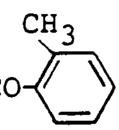

Crystalline form: Colorless amorphous

NMR analysis: 220)

Form: Free

Example 1053

Structure

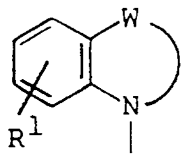 : 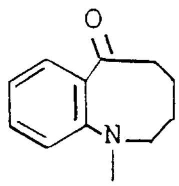

$R^2$: 2-Cl

$R^3$: 4-NHCO- 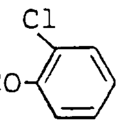

Crystalline form: Colorless amorphous

NMR analysis: 221)

Form: Free

Example 1054

Structure

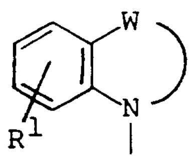 : 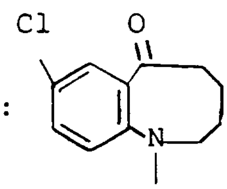

$R^2$: 2-Cl

$R^3$: 4-NHCO- 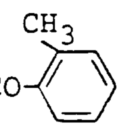

Crystalline form: Light yellow amorphous

NMR analysis: 222)

Form: Free

Example 1055

Structure

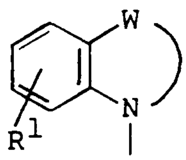 : 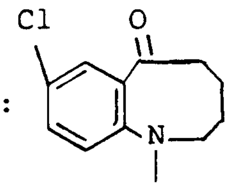

$R^2$: 2-Cl

$R^3$: 4-NHCO- 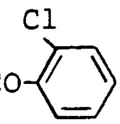

Crystalline form: Light yellow amorphous

NMR analysis: 223)

Form: Free

Example 1056

Structure

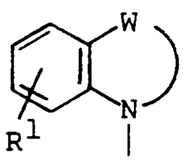 : 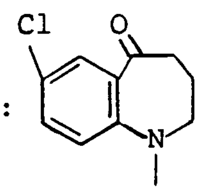

$R^2$: 2-Cl

$R^3$: 4-NHCO- 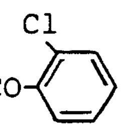

Crystalline form: White powder

Recrystallization solvent: Diethyl ether/dichloromethane

Melting Point: 169.5 - 173°C

Form: Free

Example 1057

Structure

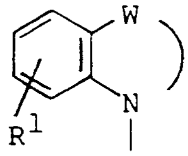 : 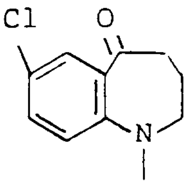

$R^2$: 2-Cl

$R^3$: 4-NHCO- 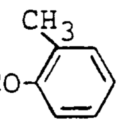

Crystalline form: Colorless amorphous

NMR analysis: 224)

Form: Free

Example 1058

Structure

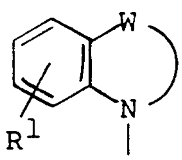 : 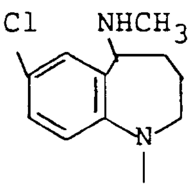

$R^2$: 2-Cl

$R^3$: 4-NHCO- 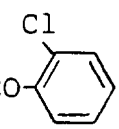

Crystalline form: Colorless amorphous

NMR analysis: 225)

Example 1059

Structure

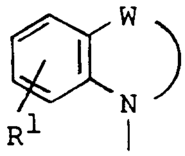 : 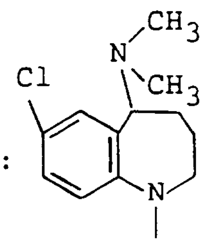

$R^2$: 2-Cl

$R^3$: 4-NHCO- 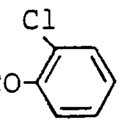

Crystalline form: Colorless amorphous

NMR analysis: 226)

Form: Free

Example 1060

Structure

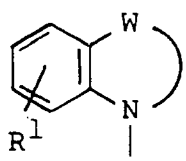 : 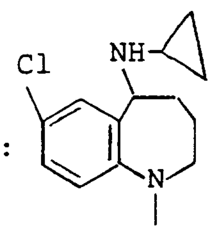

$R^2$: 2-Cl

$R^3$: 4-NHCO- 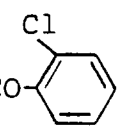

Crystalline form: Colorless amorphous

NMR analysis: 227)

Form: Free

Example 1061

Structure

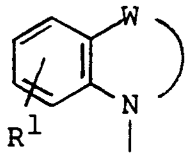 : 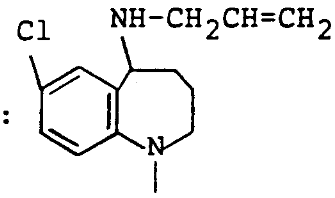

$R^2$: 2-Cl

$R^3$: 4-NHCO- 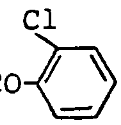

Crystalline form: Colorless amorphous

NMR analysis: 228)

Form: Free

Example 1062

Structure

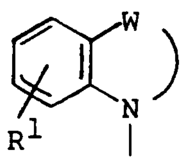 : 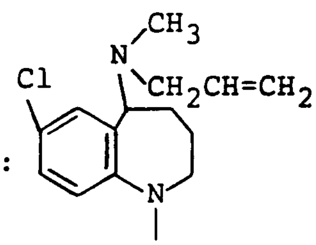

$R^2$: 2-Cl

$R^3$: 4-NHCO- 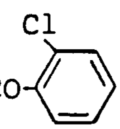

Crystalline form: Colorless amorphous

NMR analysis: 229)

Form: Free

Example 1063

Structure

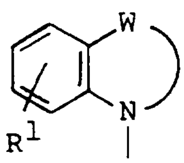 : 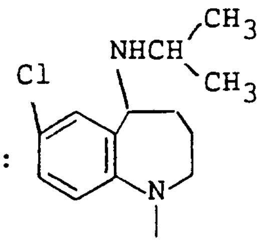

$R^2$: 2-Cl

$R^3$: 4-NHCO- 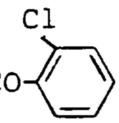

Crystalline form: Colorless amorphous

NMR analysis: 230)

Form: Free

Example 1064

Structure

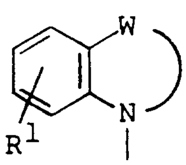 : 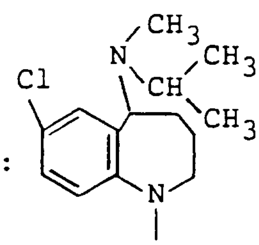

$R^2$: 2-Cl

$R^3$: 4-NHCO- 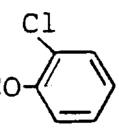

Crystalline form: Colorless amorphous

NMR analysis: 231)

Form: Free

Example 1065

Structure

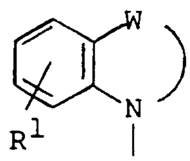 : 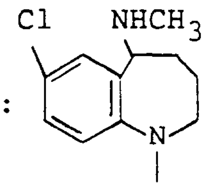

$R^2$: 2-Cl

$R^3$: 4-NHCO-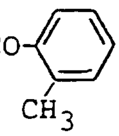

Crystalline form: Colorless amorphous

NMR analysis: 232)

Form: Free

Example 1066

Structure

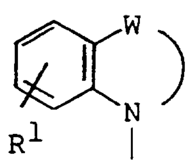 : 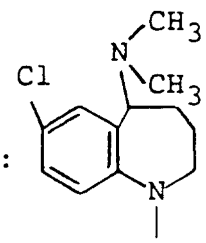

$R^2$: 2-Cl

$R^3$: 4-NHCO-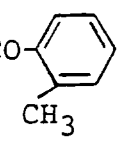

Crystalline form: Colorless amorphous

NMR analysis: 233)

Form: Free

Example 1067

Structure

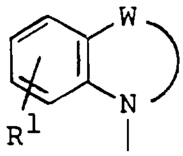

:

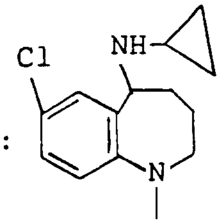

$R^2$: 2-Cl

$R^3$: 4-NHCO-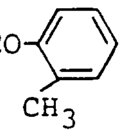

Crystalline form: Colorless amorphous

NMR analysis: 234)

Form: Free

Example 1068

Structure

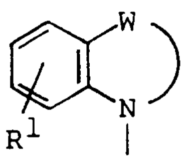

:

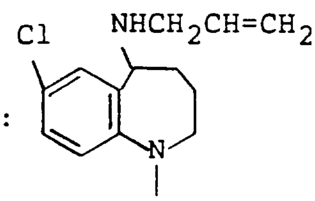

$R^2$: 2-Cl

$R^3$: 4-NHCO-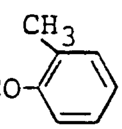

Crystalline form: Colorless amorphous

NMR analysis: 235)

Form: Free

Example 1069

Structure 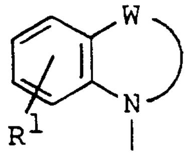 : 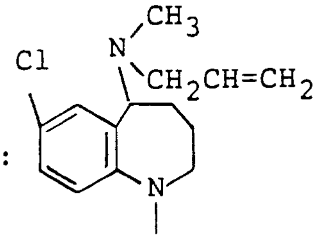 $R^2$: 2-Cl

$R^3$: 4-NHCO-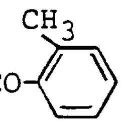

Crystalline form: Colorless amorphous

NMR analysis: 236)

Form: Free

Example 1070

Structure 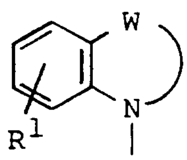 : 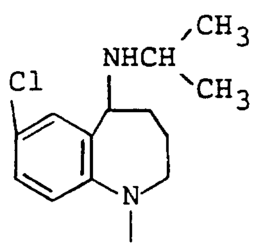 $R^2$: 2-Cl

$R^3$: 4-NHCO-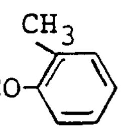

Crystalline form: Colorless amorphous

NMR analysis: 237)

Form: Free

196) $^1$H-NMR ($CDCl_3$) δ ; 2.14 (2H, brs), 2.33 (3H, s), 2.46 (3H, s), 2.85 (2H, t, J=6.1 Hz), 4.83 (2H, brs), 6.64 (1H, d, J=8.1 Hz), 7.07 (1H, d, J=8.0 Hz), 7.21-7.48 (8H, m), 7.65 (1H, m), 7.74 (1H, brs)

197) $^1$H-NMR ($CDCl_3$) δ ; 2.12 (2H, brs), 2.33 (3H, s), 2.85 (2H, t, J=6.2 Hz), 2.88-5.28 (2H, m), 6.63 (1H, d, J=8.1 Hz), 7.06 (1H, dd, J=1.7 Hz, 8.1 Hz), 7.19-7.69 (9H, m), 8.26 (1H, brs)

198) $^1$H-NMR ($CDCl_3$) δ ; 0.49 (4H, m), 1.25-5.13 (9H, m), 2.33 (3H, s), 2.45 (3H, s), 6.53 (1H, m), 6.79 (1H, m), 7.07-7.42 (9H, m), 7.73 (1H, m)

199) $^1$H-NMR ($CDCl_3$) δ ; 2.04 (2H, brs), 2.29 (3H, s), 2.82 (2H, t, J=5.9 Hz), 2.85-5.29 (2H, m), 6.82-7.69 (10H, m), 8.31 (1H, brs)

200) $^1$H-NMR ($CDCl_3$) δ ; 2.05 (2H, brs), 2.29 (3H, s), 2.44 (3H, s), 2.79 (2H, t, J=5.5 Hz), 2.82-5.28 (2H, m), 6.82-8.12 (11H, m)

201) $^1$H-NMR ($CDCl_3$) δ ; 1.40-4.85 (11H, m), 2.51 (3H, s), 6.78-7.63 (10H, m), 8.64 (1H, brs)

202) $^1$H-NMR ($CDCl_3$) δ ; 1.40-4.85 (11H, m), 2.45 (3H, s), 2.50 (3H, s), 6.78-7.55 (10H, m), 8.10 (1H, brs)

203) $^1$H-NMR ($CDCl_3$) δ ; 0.49 (4H, m), 1.25-4.85 (9H, m), 2.28 (3H, s), 6.77-7.62 (10H, m), 8.64 (1H, brs)

204) $^1$H-NMR ($CDCl_3$) δ ; 0.48 (4H, m), 1.26-4.85 (9H, m), 2.29 (3H, s), 2.44 (3H, s), 6.78-7.58 (10H, m), 8.18 (1H, brs)

205) $^1$H-NMR ($CDCl_3$) δ ; 1.14 (6H, d, J=6.3 Hz), 1.52-2.20 (7H, m), 2.20-2.60 (1H, m), 2.64-3.66 (10H, m), 4.00-4.50 (4H, m), 4.50-5.23 (2H, m), 6.57-7.90 (11H, m), 8.10-8.30 (1H, m), 9.97 (1H, s)

206) $^1$H-NMR ($CDCl_3$) δ ; 1.24-2.08 (4H, m), 2.08-2.26 (3H, m), 2.26-3.16 (4H, m), 3.47-4.03 (4H, m), 4.18-4.92 (1H, m), 6.40-7.94 (10H, m), 8.45-9.03 (1H, m)

207) $^1$H-NMR ($CDCl_3$) δ ; 1.26-2.10 (4H, m), 2.10-2.28 (3H, m), 2.28-3.20 (1H, m), 3.43-4.06 (4H, m), 4.20-4.93 (1H, m), 6.40-8.00 (10H, m), 8.78-9.30 (1H, m)

208) $^1$H-NMR ($CDCl_3$) δ ; 1.10-1.98 (4H, m), 1.98-3.10 (7H, m), 3.30-3.90 (4H, m), 3.90-5.10 (1H, m), 6.45-8.25 (12H, m)

209) $^1$H-NMR ($CDCl_3$) δ ; 1.06-1.94 (4H, m), 1.94-3.19 (10H, m), 3.19-3.90 (4H, m), 3.90-5.10 (1H, m), 6.44-8.60 (11H, m)

210) $^1$H-NMR ($CDCl_3$) δ ; 1.06-1.97 (4H, m), 1.97-3.20 (7H, m), 3.20-3.92 (4H, m), 3.92-5.10 (1H, m), 6.44-8.55 (11H, m)

211) $^1$H-NMR ($CDCl_3$) δ ; 1.07-1.98 (4H, m), 1.98-3.10 (10H, m), 3.37-5.20 (8H, m), 6.44-6.86 (3H, m), 6.97-7.60 (6H, m), 8.13 (1H, s), 8.19-8.38 (1H, m)

212) $^1$H-NMR ($CDCl_3$) δ ; 1.08-1.99 (4H, m), 1.99-3.13 (7H, m), 3.33-5.14 (8H, m), 6.40-6.90 (3H, m),

6.95-7.56 (5H, m), 7.63-7.87 (1H, m), 8.17-8.37 (1H, m), 8.60 (1H, s)

213) $^1$H-NMR ($CDCl_3$) δ ; 0.30-0.64 (4H, m), 0.70-3.42 (9H, m), 3.42-5.10 (5H, m), 6.40-8.70 (11H, m)

214) $^1$H-NMR ($CDCl_3$) δ ; 0.30-0.76 (4H, m), 0.80-3.43 (6H, m), 3.50-5.00 (5H, m), 6.40-9.04 (11H, m)

215) $^1$H-NMR ($CDCl_3$) δ ; 1.25-3.25 (14H, m), 3.55-5.06 (2H, m), 6.43-7.00 (2H, m), 7.00-7.71 (8H, m), 7.91-8.45 (1H, m)

216) $^1$H-NMR ($CDCl_3$) δ ; 1.11-3.20 (17H, m), 3.28-5.12 (2H, m), 6.41-7.01 (2H, m), 7.02-7.63 (8H, m), 7.76-8.21 (1H, m)

217) $^1$H-NMR ($CDCl_3$) δ ; 1.92-2.29 (2H, m), 2.36 (3H, s), 2.45 (3H, s), 2.84 (2H, t, J=6.3 Hz), 3.32-4.64 (2H, m), 6.40-8.10 (11H, m)

218) $^1$H-NMR ($CDCl_3$) δ ; 1.92-2.25 (2H, m), 2.34 (3H, s), 2.83 (2H, t, J=6.3 Hz), 3.21-4.52 (2H, m), 6.39-7.97 (10H, m), 8.43 (1H, brs)

219) $^1$H-NMR ($CDCl_3$) δ ; 1.7-2.15 (4H, m), 2.5-5.2 (4H, m), 6.75-6.9 (1H, m), 7.27-7.6 (9H, m), 7.65-7.85 (1H, m), 7.9-8.15 (2H, m)

220) $^1$H-NMR ($CDCl_3$) δ ; 1.65-2.1 (4H, m), 2.44 (3H, s), 2.8-4.5 (4H, m), 6.75-8.0 (12H, m)

221) $^1$H-NMR ($CDCl_3$) δ ; 1.65-2.3 (4H, m), 2.7-4.8 (4H, m), 6.75-8.4 (12H, m)

222) $^1$H-NMR ($CDCl_3$) δ ; 1.45-2.15 (4H, m), 2.45-2.55

(3H, m), 2.85-4.6 (4H, m), 6.8-8.25 (11H, m)

223) $^1$H-NMR ($CDCl_3$) δ ; 1.5-2.2 (4H, m), 2.8-4.7 (4H, m), 6.8-8.4 (11H, m)

224) $^1$H-NMR ($CDCl_3$) δ ; 1.75-2.25 (2H, m), 2.30-2.70 (3H, m), 2.70-2.95 (2H, m), 3.20-5.10 (2H, m), 6.70-8.40 (11H, m)

225) $^1$H-NMR ($CDCl_3$) δ ; 1.20-2.60 (8H, m), 2.60-5.10 (3H, m), 6.80-7.90 (10H, m), 8.20-8.60 (1H, m)

226) $^1$H-NMR ($CDCl_3$) δ ; 1.20-2.60 (10H, m), 2.60-5.10 (3H, m), 6.80-8.15 (11H, m)

227) $^1$H-NMR ($CDCl_3$) δ ; 0.30-0.70 (4H, m), 1.20-2.45 (6H, m), 2.60-5.10 (3H, m), 6.80-7.95 (10H, m), 8.15-8.50 (1H, m)

228) $^1$H-NMR ($CDCl_3$) δ ; 1.20-2.40 (5H, m), 2.60-5.35 (7H, m), 5.80-6.15 (1H, m), 6.75-7.95 (10H, m), 8.20-8.70 (1H, m)

229) $^1$H-NMR ($CDCl_3$) δ ; 1.20-2.55 (7H, m), 2.60-5.35 (7H, m), 5.85-6.05 (1H, m), 6.70-7.10 (2H, m), 7.10-7.90 (8H, m), 8.15-8.60 (1H, m)

230) $^1$H-NMR ($CDCl_3$) δ ; 1.00-1.20 (6H, m), 1.00-2.40 (5H, m), 2.60-5.10 (4H, m), 6.80-8.00 (10H, m), 8.15-8.65 (1H, m)

231) $^1$H-NMR ($CDCl_3$) δ ; 0.80-2.50 (13H, m), 2.60-5.10 (4H, m), 6.70-8.85 (10H, m), 8.25-8.60 (1H, m)

232) $^1$H-NMR ($CDCl_3$) δ ; 1.30-2.60 (11H, m), 2.60-5.10 (3H, m), 6.80-8.15 (11H, m)

233) $^1$H-NMR ($CDCl_3$) δ ; 1.10-2.50 (13H, m), 2.50-5.10 (3H, m), 6.75-8.40 (11H, m)

234) $^1$H-NMR ($CDCl_3$) δ ; 0.30-0.65 (4H, m), 1.20-2.30 (6H, m), 2.35-2.55 (3H, m), 2.60-5.10 (3H, m), 6.75-8.35 (11H, m)

235) $^1$H-NMR ($CDCl_3$) δ ; 1.20-2.60 (8H, m), 2.60-5.40 (7H, m), 5.80-6.15 (1H, m), 6.80-8.20 (11H, m)

236) $^1$H-NMR ($CDCl_3$) δ ; 1.25-2.60 (10H, m), 2.60-5.40 (7H, m), 5.75-6.10 (1H, m), 6.75-7.10 (2H, m), 7.10-8.40 (9H, m)

237) $^1$H-NMR ($CDCl_3$) δ ; 0.95-1.20 (6H, m), 0.95-2.25 (5H, m), 2.40-2.60 (3H, m), 2.60-5.10 (4H, m), 6.75-7.05 (2H, m), 7.10-8.30 (9H, m)

Reference Example 22

Using the suitable starting materials, the following compounds are obtained in the same manner as in Reference Example 1.

8-Chloro-6-oxo-1-(4-nitrobenzoyl)-1,2,3,4,5,6-hexahydrobenzazocine, yellow prisms

$^1$H-NMR (DMSO-$d_6$) δ ; 1.3-2.2 (4H, m), 2.6-5.0 (4H, m), 7.05-8.5 (7H, m)

5-Oxo-7-methyl-1-(2-chloro-4-nitrobenzoyl)-2,3,4,5-tetrahydro-1H-benzazepine, light yellow amorphous

$^1$H-NMR ($CDCl_3$) δ ; 1.71-2.32 (2H, m), 2.29 (3H, s), 2.86 (2H, t, J=6.3 Hz), 3.10-5.30 (2H, m), 6.84-8.38 (6H, m)

5-Oxo-7-methyl-1-(3-methoxy-4-nitrobenzoyl)-2,3,4,5-tetrahydro-1H-benzazepine, light yellow amorphous

$^1$H-NMR ($CDCl_3$) δ ; 2.17 (2H, brs), 2.34 (3H, s), 2.84 (2H, t, J=6.0 Hz), 3.10-5.29 (2H, m), 3.77 (3H, s), 6.67 (1H, d, J=7.9 Hz), 6.85 (2H, m), 7.10 (1H, d, J=8.0 Hz), 7.57-7.65 (2H, m)

5-Oxo-7-dimethylamino-1-(2-chloro-4-nitrobenzoyl)-2,3,4,5-tetrahydro-1H-benzazepine, yellow powder

$^1$H-NMR ($CDCl_3$) δ ; 1.66-2.38 (2H, m), 2.65-2.88 (2H, m), 2.92 (6H, s), 3.08-3.64, 4.58-5.01 (total 2H, m), 6.49 (1H, dd, J=3.1, 8.7 Hz), 6.82 (1H, d, J=8.7 Hz), 6.90 (1H, d, J=3.1 Hz), 7.02-7.37 (1H, m), 7.94 (1H, dd, J=1.9, 8.4 Hz), 8.08 (1H, d, J=1.9 Hz)

Reference Example 23

Using the suitable starting materials, the following compounds are obtained in the same manner as in Reference Example 2.

8-Chloro-6-oxo-1-(4-aminobenzoyl)-1,2,3,4,5,6-hexahydrobenzazocine, light yellow amorphous

$^1$H-NMR ($CDCl_3$) δ ; 1.7-2.2 (4H, m), 2.3-4.8 (6H, m), 6.4-6.6 (2H, m), 6.74 (1H, d, J=8.5 Hz), 7.1-7.4 (3H, m), 7.99 (1H, d, J=2.6 Hz)

8-Methyl-6-oxo-(2-chloro-4-aminobenzoyl)-1,2,3,4,5,6-hexahydrobenzazocine, colorless amorphous

$^1$H-NMR ($CDCl_3$) δ ; 1.4-2.1 (4H, m), 2.15-2.6 (3H, m), 2.7-4.4 (6H, m), 6.15-6.35 (1H, m), 6.51 (1H, s), 6.6-6.85 (1H, m), 6.9-7.25 (2H, m), 7.72 (1H, s)

8-Methoxy-6-oxo-(2-chloro-4-aminobenzoyl)-1,2,3,4,5,6-hexahydrobenzazocine, light yellow amorphous

$^1$H-NMR ($CDCl_3$) δ ; 1.4-2.2 (4H, m), 2.7-5.0 (9H, m), 6.25 (1H, dd, J=8.3 Hz, 2.2 Hz), 6.51 (1H, d, J=2.2 Hz),

6.66 (1H, d, J=8.3 Hz), 6.88 (1H, dd, J=8.6 Hz, 3.0 Hz), 7.23 (1H, d, J=8.6 Hz), 7.43 (1H, d, J=3.0 Hz)

5-Oxo-7-chloro-1-(2-methoxy-4-aminobenzoyl)-2,3,4,5-tetrahydro-1H-benzazepine, colorless particles (recrystallized from methanol/diethyl ether), m.p. 206 - 208°C

5-Oxo-7-methyl-1-(2-chloro-4-aminobenzoyl)-2,3,4,5-tetrahydro-1H-benzazepine, light yellow amorphous

$^1$H-NMR ($CDCl_3$) δ ; 2.09 (2H, brs), 2.29 (3H, s), 3.10-5.00 (2H, m), 3.78 (2H, brs), 6.34-7.54 (6H, m)

5-Oxo-7-methyl-1-(3-methoxy-4-aminobenzoyl)-2,3,4,5-tetrahydro-1H-benzazepine, light yellow amorphous

$^1$H-NMR ($CDCl_3$) δ ; 2.12 (2H, brs), 2.32 (3H, s), 2.85 (2H, t, J=5.9 Hz), 3.30-5.00 (2H, m), 3.65 (3H, s), 3.98 (2H, brs), 6.40 (1H, d, J=8.1 Hz), 6.64-6.76 (3H, m), 7.06 (1H, dd, J=1.6, 8.1 Hz), 7.63 (1H, d, J=2.0 Hz)

5-Oxo-7-dimethylamino-1-(2-chloro-4-aminobenzoyl)-2,3,4,5-tetrahydro-1H-benzazepine, yellow amorphous

$^1$H-NMR ($CDCl_3$) δ ; 1.60-2.32 (2H, m), 2.67-5.13 (4H, m), 2.92 (6H, s), 3.75 (2H, s), 6.31 (1H, dd, J=2.1, 8.3 Hz), 6.46 (1H, d, J=2.1 Hz), 6.48 (1H, dd J=3.1, 8.7 Hz), 6.66-6.89 (2H, m), 6.95 (1H, d, J=3.1 Hz)

Using the stuitable starting materials, the compounds of the following Table 9 are obtained in the same manner as in above Examples 1 and 382.

Table 9

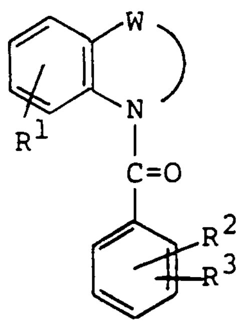

Example 1071

Structure

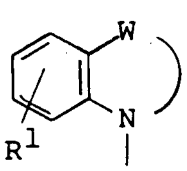 : 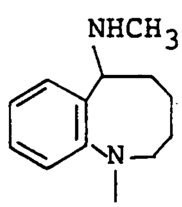

$R^2$: 2-Cl

$R^3$: 4-NHCO- 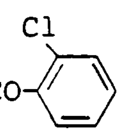

Crystalline form: Colorless prisms

Recrystallization solvent: Ethanol

Melting Point: 227 - 230°C

Form: Free

Example 1072

Structure 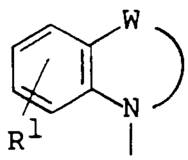 : 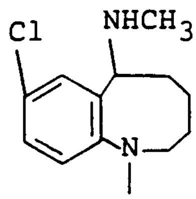 $R^2$: 2-Cl

$R^3$: 4-NHCO- 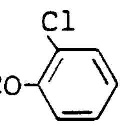

Crystalline form: Colorless needles

Recrystallization solvent: Ethanol/petroleum ether

Melting Point: 216 - 218°C

Form: Free

---

Example 1073

Structure 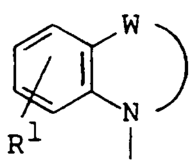 : 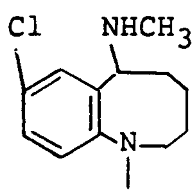 $R^2$: 2-Cl

$R^3$: 4-NHCO- 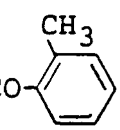

Crystalline form: Colorless prisms

Recrystallization solvent: Ethyl acetate/n-hexane

Melting Point: 227 - 228°C

Form: Free

Example 1074

Structure

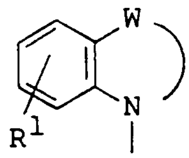 : 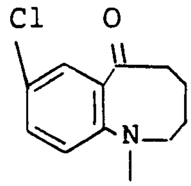

$R^2$: H

$R^3$: 4-NHCO- 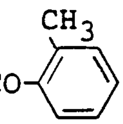

Crystalline form: White powder

NMR analysis: 238)

Form: Free

Example 1075

Structure

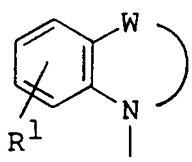 : 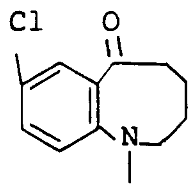

$R^2$: H

$R^3$: 4-NHCO- 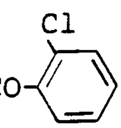

Crystalline form: White powder

NMR analysis: 239)

Form: Free

Example 1076

Structure

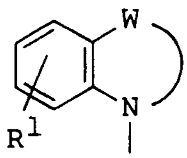 : 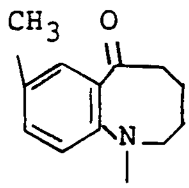

$R^2$: 2-Cl

$R^3$: 4-NHCO-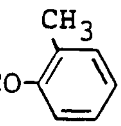

Crystalline form: Light yellow amorphous

NMR analysis: 240)

Form: Free

Example 1077

Structure

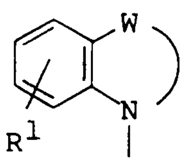 : 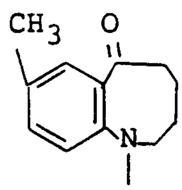

$R^2$: 2-Cl

$R^3$: 4-NHCO-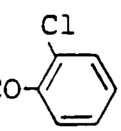

Crystalline form: Light yellow amorphous

NMR analysis: 241)

Form: Free

Example 1078

Structure

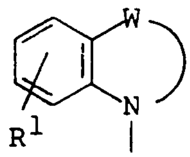

:

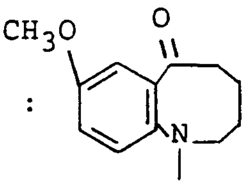

$R^2$: 2-Cl

$R^3$: 4-NHCO- 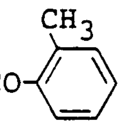

Crystalline form: Colorless amorphous

NMR analysis: 242)

Form: Free

Example 1079

Structure

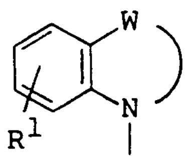

:

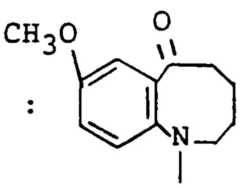

$R^2$: 2-Cl

$R^3$: 4-NHCO- 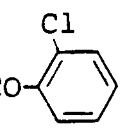

Crystalline form: Colorless amorphous

NMR analysis: 243)

Form: Free

Example 1080

Structure

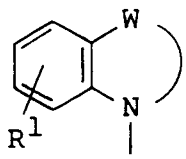 : 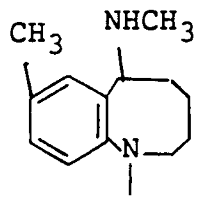  $R^2$: 2-Cl

$R^3$: 4-NHCO- 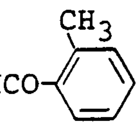

Crystalline form: Light yellow powder

Recrystallization solvent: Ethyl acetate/diethyl ether

Melting Point: 179 - 181°C

Form: Free

Example 1081

Structure

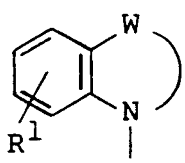 : 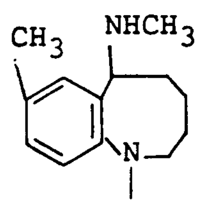  $R^2$: 2-Cl

$R^3$: 4-NHCO- 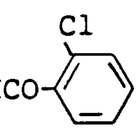

Crystalline form: White powder

Recrystallization solvent: Ethyl acetate/diethyl ether

Melting Point: 213 - 216°C

Form: Free

Example 1082

Structure

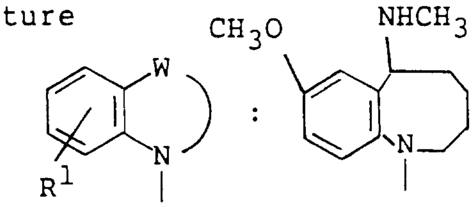

$R^2$: 2-Cl

$R^3$: 4-NHCO- 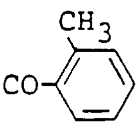

Crystalline form: Light yellow powder

Recrystallization solvent: Ethyl acetate/diethyl ether

Melting Point: 185 - 187°C

Form: Free

Example 1083

Structure

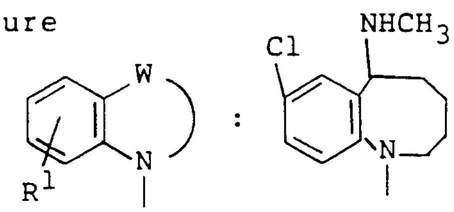

$R^2$: H

$R^3$: 4-NHCO- 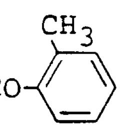

Crystalline form: Colorless prisms

Recrystallization solvent: Ethanol

Melting Point: 249 - 251°C

Form: Free

Example 1084

Structure

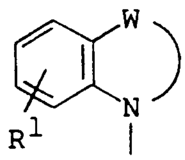

:

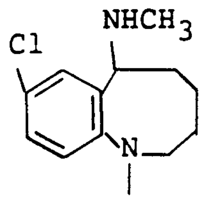

$R^2$: H

$R^3$: 4-NHCO-

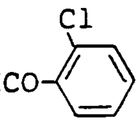

Crystalline form: Colorless needles

Recrystallization solvent: Ethanol

Melting Point: 239 - 241°C

Form: Free

Example 1085

Structure

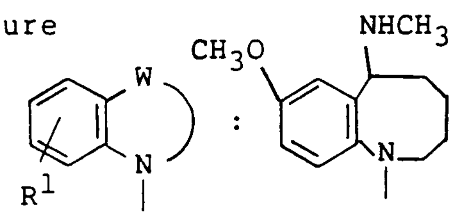

$R^2$: 2-Cl

$R^3$: 4-NHCO-

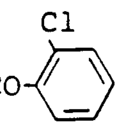

Crystalline form: White powder

Recrystallization solvent: Ethyl acetate/diethyl ether

Melting Point: 208 - 210°C

Form: Free

Example 1086

Structure

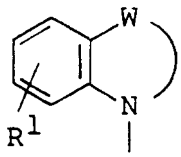

:

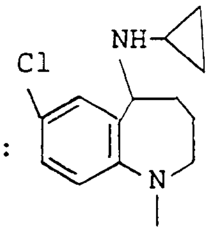

$R^2$: 2-$CH_3$

$R^3$: 4-NHCO-

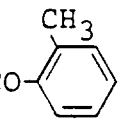

Crystalline form: White powder

Recrystallization solvent: Methanol/n-hexane

Melting Point: 178 - 180.5°C

Form: Free

Example 1087

Structure

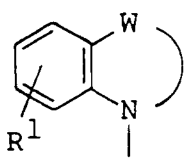

:

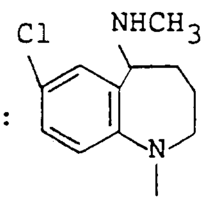

$R^2$: 2-$CH_3$

$R^3$: 4-NHCO-

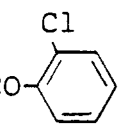

Crystalline form: Colorless amorphous

NMR analysis: 244)

Form: Free

Example 1088

Structure

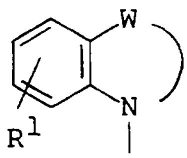 : 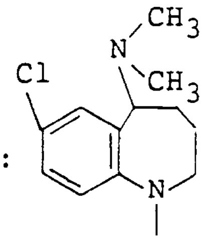

$R^2$: 2-$CH_3$

$R^3$: 4-NHCO- 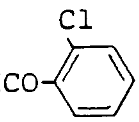

Crystalline form: Colorless amorphous

NMR analysis: 245)

Form: Free

---

Example 1089

Structure

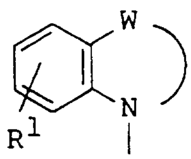 : 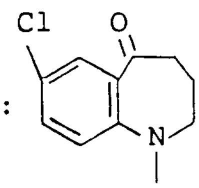

$R^2$: 2-$OCH_3$

$R^3$: 4-NHCOO-$CH_2$- 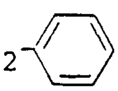

Crystalline form: White powder

Recrystallization solvent: Methanol/diethyl ether

NMR analysis: 246)

Form: Free

Example 1090

Structure

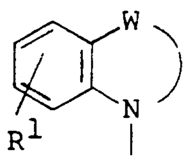

:

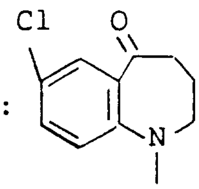

$R^2$: 2-$OCH_3$

$R^3$: 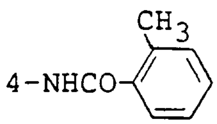

Crystalline form: Colorless amorphous

NMR analysis: 247)

Form: Free

Example 1091

Structure

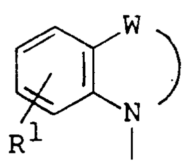

:

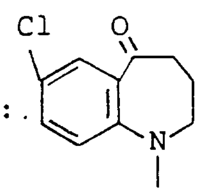

$R^2$: 2-$OCH_3$

$R^3$: 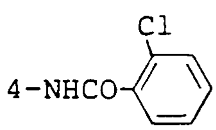

Crystalline form: Colorless amorphous

NMR analysis: 248)

Form: Free

Example 1092

Structure

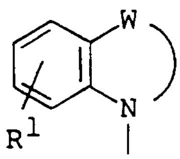 : 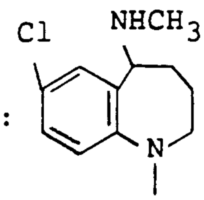

$R^2$: 2-$OCH_3$

$R^3$: 4-NHCO- 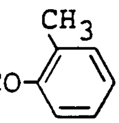

Crystalline form: Colorless amorphous

NMR analysis: 249)

Form: Free

Example 1093

Structure

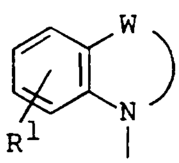 : 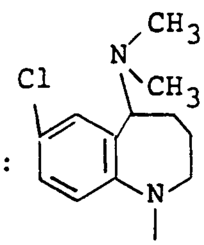

$R^2$: 2-$OCH_3$

$R^3$: 4-NHCO- 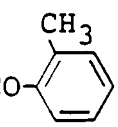

Crystalline form: White powder

Recrystallization solvent: Methanol/diethyl ether

Melting Point: 205 - 206°C

Form: Free

Example 1094

Structure

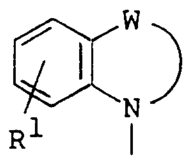 : 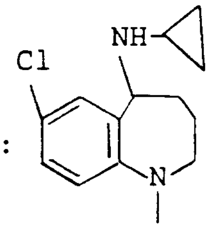

$R^2$: 2-$OCH_3$

$R^3$: 4-NHCO- 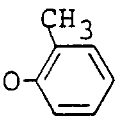

Crystalline form: Colorless amorphous

NMR analysis: 250)

Form: Free

Example 1095

Structure

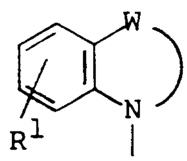 : 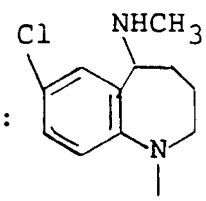

$R^2$: 2-$OCH_3$

$R^3$: 4-NHCO- 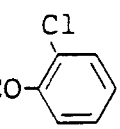

Crystalline form: White powder

Recrystallization solvent: Methanol/n-hexane

Melting Point: 172.5 - 174°C

Form: Free

Example 1096

Structure

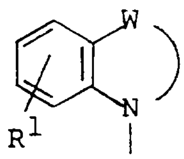 : 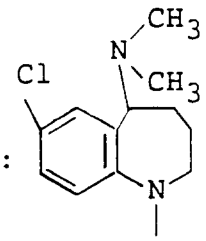

$R^2$: 2-$OCH_3$

$R^3$: 4-NHCO- 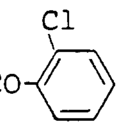

Crystalline form: White powder

Recrystallization solvent: Methanol/diethyl ether

Melting Point: 215 - 216.5°C

Form: Free

Example 1097

Structure

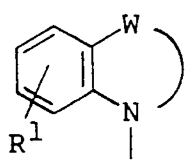 : 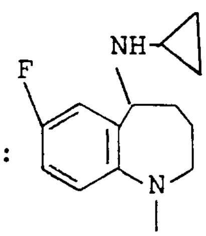

$R^2$: H

$R^3$: 4-NHCO- 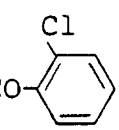

Crystalline form: White powder

Recrystallization solvent: Methanol/diethyl ether

Melting Point: 133 - 136°C

Form: Free

Example 1098

Structure

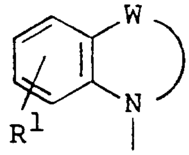 : 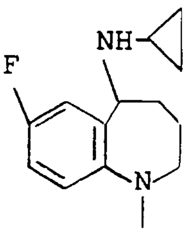

$R^2$: 2-Cl

$R^3$: 4-NHCO- 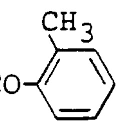

Crystalline form: Colorless amorphous

NMR analysis: 251)

Form: Free

Example 1099

Structure

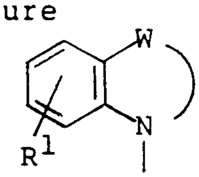 : 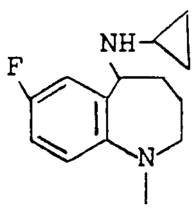

$R^2$: 2-Cl

$R^3$: 4-NHCO- 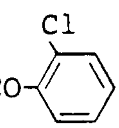

Crystalline form: White powder

Recrystallization solvent: Methanol/n-hexane

Melting Point: 179 - 180°C

Form: Free

Example 1100

Structure

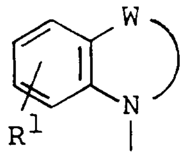 : 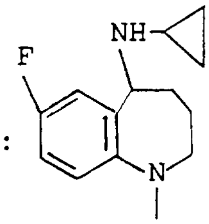

$R^2$: 3-$OCH_3$

$R^3$: 4-NHCO- 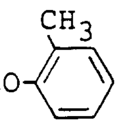

Crystalline form: White powder

Recrystallization solvent: Methanol/n-hexane

Melting Point: 167.5 - 169.5°C

Form: Free

---

Example 1101

Structure

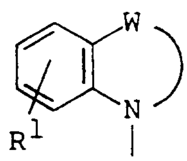 : 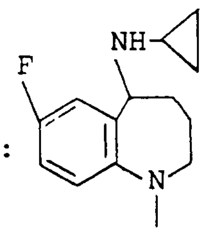

$R^2$: 3-$OCH_3$

$R^3$: 4-NHCO- 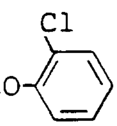

Crystalline form: White powder

Recrystallization solvent: Methanol/diethyl ether

Melting Point: 176 - 178°C

Form: Free

Example 1102

Structure

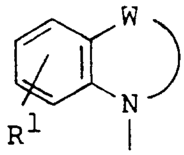 : 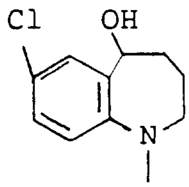 $R^2$: 2-$OCH_3$

$R^3$: 4-NHCO- 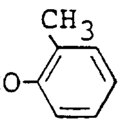

Crystalline form: Colorless amorphous

NMR analysis: 252)

Form: Free

Example 1103

Structure

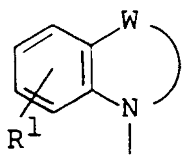 : 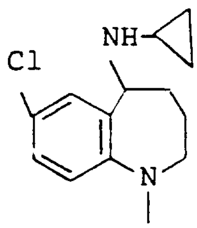 $R^2$: 2-$OCH_3$

$R^3$: 4-NHCO- 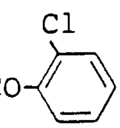

Crystalline form: White powder

Recrystallization solvent: Methanol/diethyl ether

Melting Point: 185 - 188°C

Form: Free

Example 1104

Structure

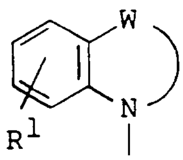 : 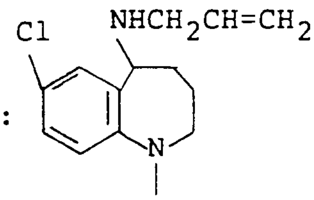

$R^2$: H

$R^3$: 4-NHCO- 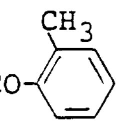

Crystalline form: White powder

Recrystallization solvent: Methanol/diethyl ether

Melting Point: 180 - 181.5°C

Form: Free

Example 1105

Structure

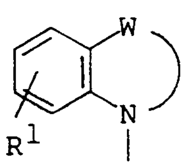 : 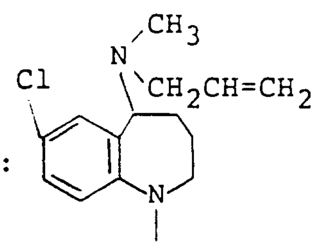

$R^2$: H

$R^3$: 4-NHCO- 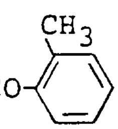

Crystalline form: White powder

Recrystallization solvent: Methanol/diethyl ether

Melting Point: 181 - 184°C

Form: Free

Example 1106

Structure

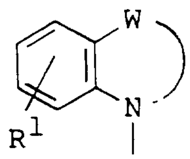 : 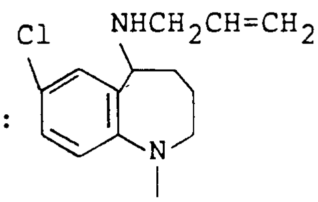

$R^2$: H

$R^3$: 4-NHCO- 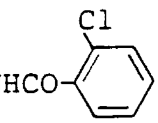

Crystalline form: White powder

Recrystallization solvent: Methanol/diethyl ether

Melting Point: 186.5 - 187°C

Form: Free

Example 1107

Structure

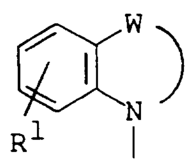 : 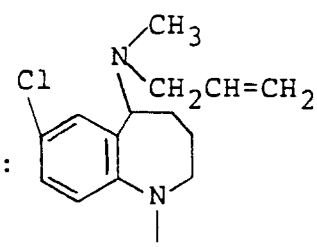

$R^2$: H

$R^3$: 4-NHCO- 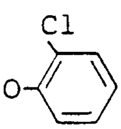

Crystalline form: White powder

Recrystallization solvent: Methanol/diethyl ether

Melting Point: 183 - 184°C

Form: Free

Example 1108

Structure

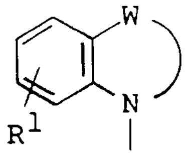 : 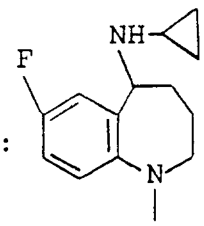

$R^2$: H

$R^3$: 4-NHCO- 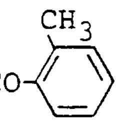

Crystalline form: White powder

Recrystallization solvent: Methanol/diethyl ether

Melting Point: 151 - 153°C

Form: Free

Example 1109

Structure

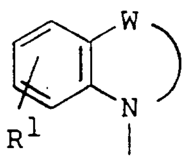 : 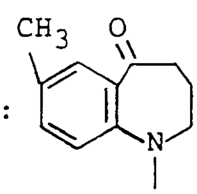

$R^2$: 3-$OCH_3$

$R^3$: 4-NHCO- 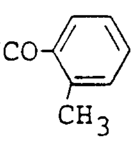

Crystalline form: Colorless amorphous

NMR analysis: 253)

Form: Free

Example 1110

Structure

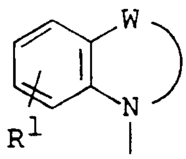 : 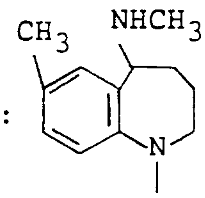 $R^2$: H

$R^3$: 4-NHCO- 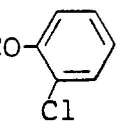

Crystalline form: Colorless amorphous

NMR analysis: 254)

Form: Free

Example 1111

Structure

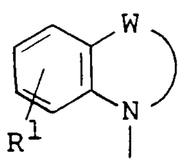 : 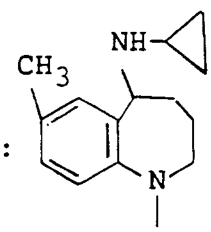 $R^2$: H

$R^3$: 4-NHCO- 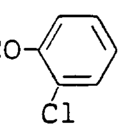

Crystalline form: White needles

Recrystallization solvent: Ethanol/n-hexane

Melting Point: 191 - 195°C

Form: Free

Example 1112

Structure

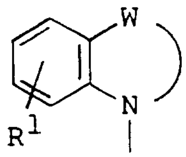 : 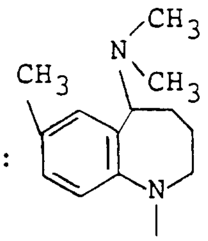

$R^2$: H

$R^3$: 4-NHCO- 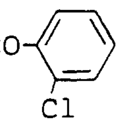

Crystalline form: White powder

Recrystallization solvent: Diethyl ether/n-hexane

Melting Point: 227 - 230°C

Form: Free

Example 1113

Structure

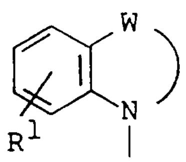 : 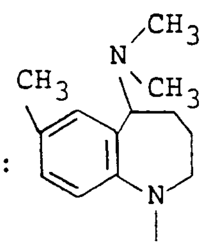

$R^2$: 2-Cl

$R^3$: 4-NHCO- 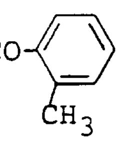

Crystalline form: Colorless amorphous

NMR analysis: 289)

Form: Free

Example 1114

Structure

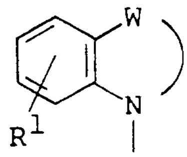 : 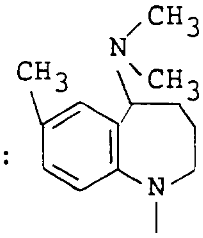

$R^2$: 2-Cl

$R^3$: 4-NHCO- 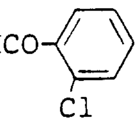

Crystalline form: Light yellow amorphous

NMR analysis: 255)

Example 1115

Structure

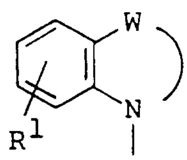 : 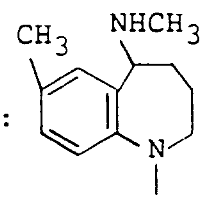

$R^2$: 3-$OCH_3$

$R^3$: 4-NHCO- 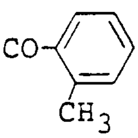

Crystalline form: White powder

Recrystallization solvent: Diethyl ether/n-hexane

Melting Point: 172 - 174°C

Form: Free

Example 1116

Structure

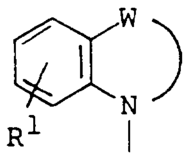 : 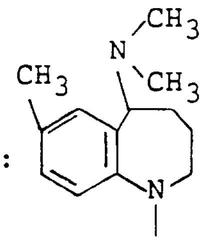

$R^2$: 3-$OCH_3$

$R^3$: 4-NHCO- 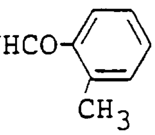

Crystalline form: Colorless amorphous

NMR analysis: 305)

Form: Free

Example 1117

Structure

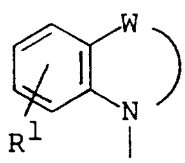 : 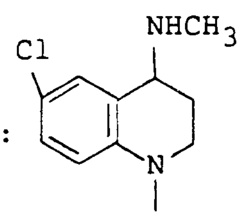

$R^2$: 2-$CH_3$

$R^3$: 4-NHCO- 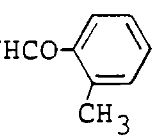

Crystalline form: Colorless amorphous

NMR analysis: 290)

Form: Free

Example 1118

Structure

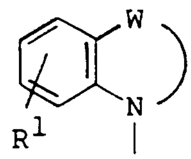 : 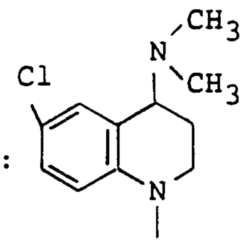

$R^2$: 2-$CH_3$

$R^3$: 4-NHCO- 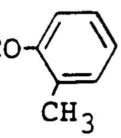

Crystalline form: Colorless amorphous

NMR analysis: 291)

Form: Free

Example 1119

Structure

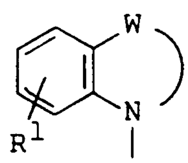 : 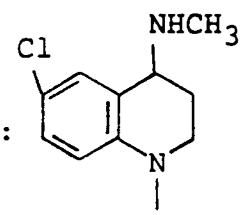

$R^2$: H

$R^3$: 4-NHCO- 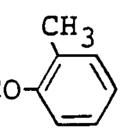

Crystalline form: Colorless amorphous

NMR analysis: 264)

Form: Free

Example 1120

Structure 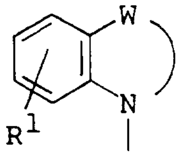 : 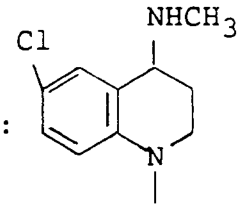 $R^2$: H

$R^3$: 4-NHCO- 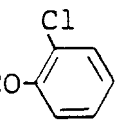

Crystalline form: Colorless amorphous

NMR analysis: 265)

Form: Free

Example 1121

Structure 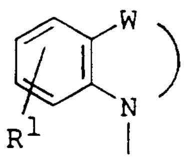 : 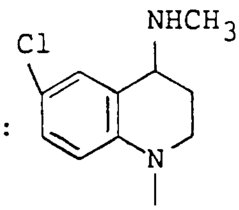 $R^2$: 2-Cl

$R^3$: 4-NHCO- 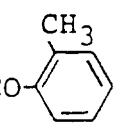

Crystalline form: Colorless amorphous

NMR analysis: 266)

Form: Free

Example 1122

Structure

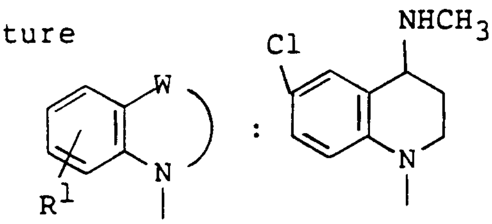

$R^2$: 2-Cl

$R^3$: 4-NHCO- 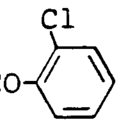

Crystalline form: Colorless amorphous

NMR analysis: 267)

Form: Free

Example 1123

Structure

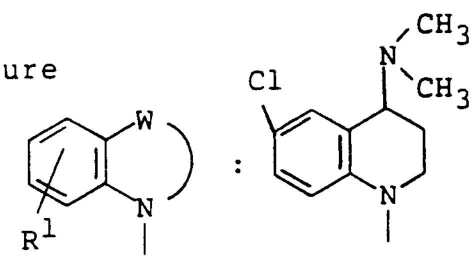

$R^2$: H

$R^3$: 4-NHCO- 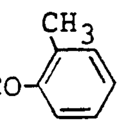

Crystalline form: Colorless amorphous

NMR analysis: 268)

Form: Free

Example 1124

Structure

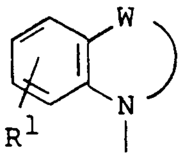 : 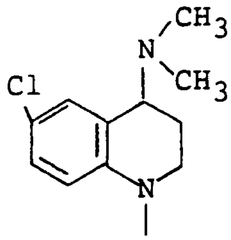

$R^2$: H

$R^3$: 4-NHCO- 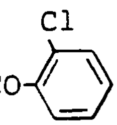

Crystalline form: Colorless amorphous

NMR analysis: 269)

Form: Free

Example 1125

Structure

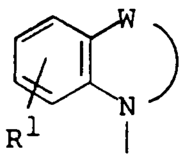 : 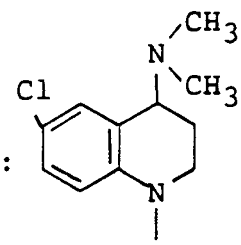

$R^2$: 2-Cl

$R^3$: 4-NHCO- 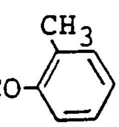

Crystalline form: Colorless amorphous

NMR analysis: 270)

Form: Free

Example 1126

Structure

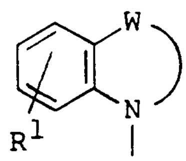 : 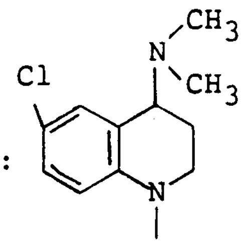

$R^2$: 2-Cl

$R^3$: 4-NHCO- 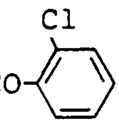

Crystalline form: Colorless amorphous

NMR analysis: 271)

Form: Free

Example 1127

Structure

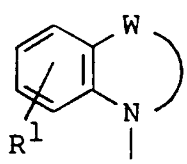 : 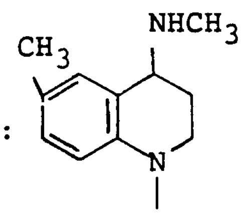

$R^2$: H

$R^3$: 4-NHCO- 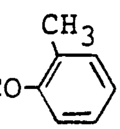

Crystalline form: Colorless amorphous

NMR analysis: 272)

Form: Free

Example 1128

Structure

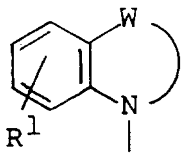

:

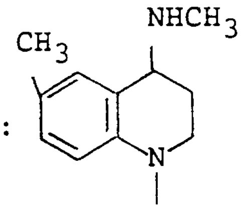

$R^2$: H

$R^3$: 4-NHCO- 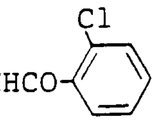

Crystalline form: Colorless amorphous

NMR analysis: 273)

Form: Free

Example 1129

Structure

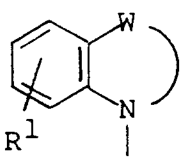

:

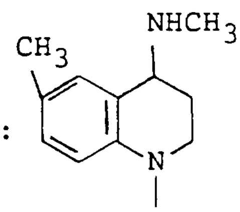

$R^2$: 2-Cl

$R^3$: 4-NHCO- 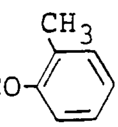

Crystalline form: Colorless amorphous

NMR analysis: 274)

Form: Free

Example 1130

Structure

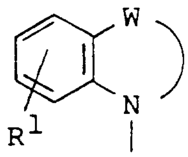 : 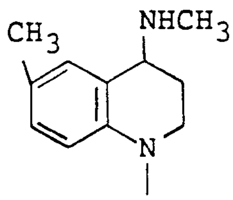

$R^2$: 2-Cl

$R^3$: 4-NHCO- 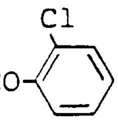

Crystalline form: Colorless amorphous

NMR analysis: 275)

Form: Free

Example 1131

Structure

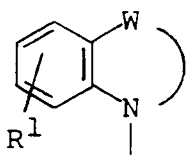 : 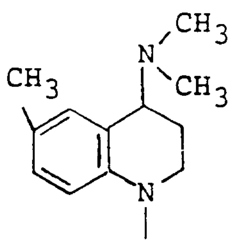

$R^2$: H

$R^3$: 4-NHCO- 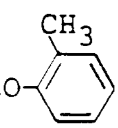

Crystalline form: Colorless amorphous

NMR analysis: 276)

Form: Free

Example 1132

Structure

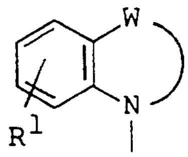 : 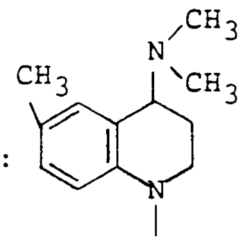

$R^2$: H

$R^3$: 4-NHCO- 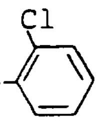

Crystalline form: Colorless amorphous

NMR analysis: 277)

Form: Free

Example 1133

Structure

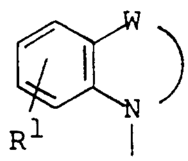 : 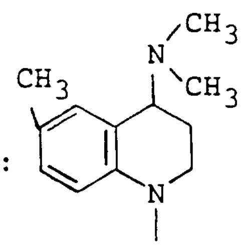

$R^2$: 2-Cl

$R^3$: 4-NHCO- 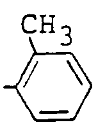

Crystalline form: Colorless amorphous

NMR analysis: 278)

Form: Free

Example 1134

Structure

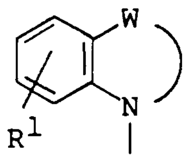 : 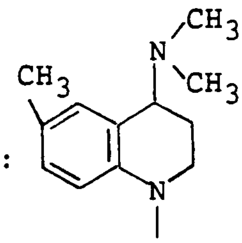 $R^2$: 2-Cl

$R^3$: 4-NHCO- 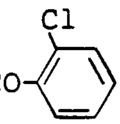

Crystalline form: Colorless amorphous

NMR analysis: 279)

Form: Free

Example 1135

Structure

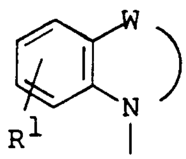 : 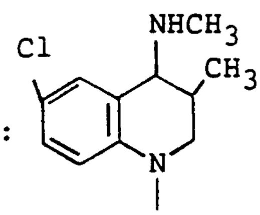 $R^2$: H

$R^3$: 4-NHCO- 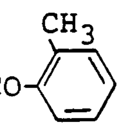

NMR analysis: 280)

Form: Free

Example 1136

Structure 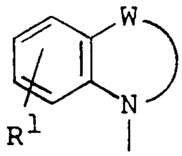 : 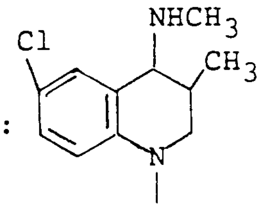 $R^2$: H

$R^3$: 4-NHCO-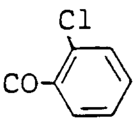

NMR analysis: 281)

Form: Free

Example 1137

Structure 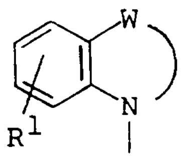 : 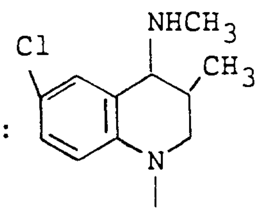 $R^2$: 2-Cl

$R^3$: 4-NHCO-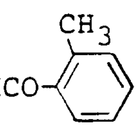

NMR analysis: 282)

Form: Free

Example 1138

Structure

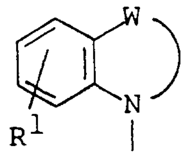 : 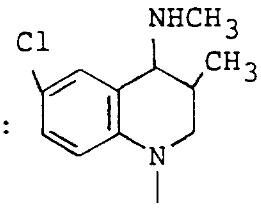

$R^2$: 2-Cl

$R^3$: 4-NHCO- 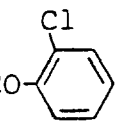

NMR analysis: 283)

Form: Free

---

Example 1139

Structure

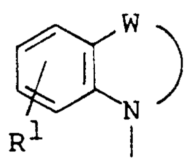 : 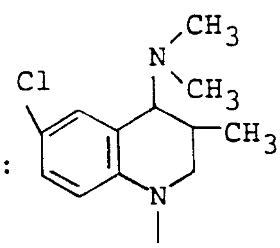

$R^2$: 2-Cl

$R^3$: 4-NHCO- 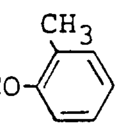

NMR analysis: 306)

Form: Free

Example 1140

Structure

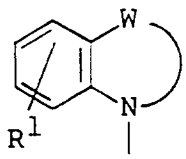 : 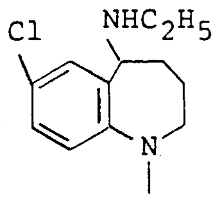

$R^2$: 2-Cl

$R^3$: 4-NHCO- 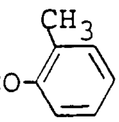

Crystalline form: Colorless amorphous

NMR analysis: 284)

Form: Free

Example 1141

Structure

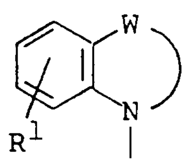 : 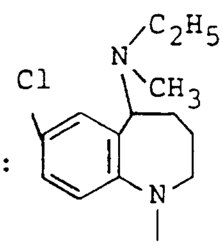

$R^2$: 2-Cl

$R^3$: 4-NHCO- 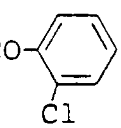

Crystalline form: Colorless amorphous

NMR analysis: 285)

Form: Free

Example 1142

Structure

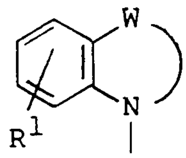 : 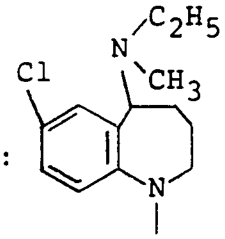

$R^2$: 2-Cl

$R^3$: 4-NHCO- 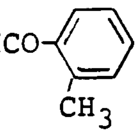

Crystalline form: Colorless amorphous

NMR analysis: 286)

Form: Free

---

Example 1143

Structure

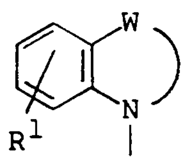 : 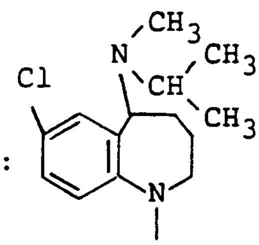

$R^2$: 2-Cl

$R^3$: 4-NHCO- 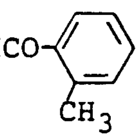

Crystalline form: Colorless amorphous

NMR analysis: 287)

Form: Free

Example 1144

Structure

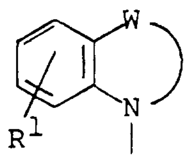 : 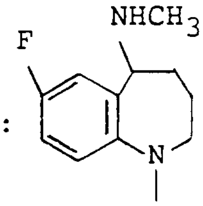

$R^2$: H

$R^3$: 4-NHCO- 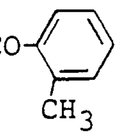

Crystalline form: White powder

Recrystallization solvent: Dichloromethane/diethyl ether

Melting Point: 203 - 207°C

Form: Free

Example 1145

Structure

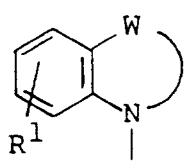 : 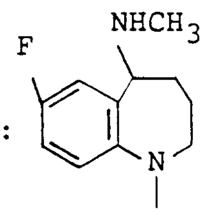

$R^2$: H

$R^3$: 4-NHCO- 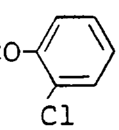

Crystalline form: White powder

Recrystallization solvent: Dichloromethane/diethyl ether

Melting Point: 199 - 203°C

Form: Free

Example 1146

Structure

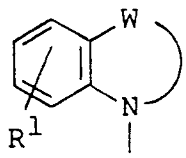 : 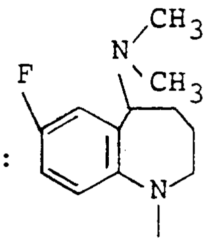

$R^2$: H

$R^3$: 4-NHCO- 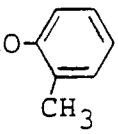

Crystalline form: White powder

Recrystallization solvent: Dichloromethane/diethyl ether

Melting Point: 210 - 212°C

Form: Free

Example 1147

Structure

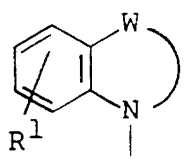 : 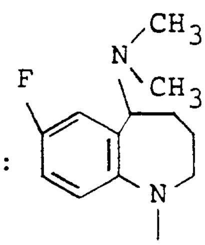

$R^2$: H

$R^3$: 4-NHCO- 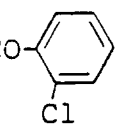

Crystalline form: White powder

Recrystallization solvent: Dichloromethane/diethyl ether

Melting Point: 211 - 214°C

Form: Free

Example 1148

Structure

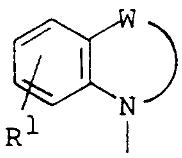 : 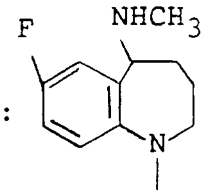

$R^2$: 2-Cl

$R^3$: 4-NHCO-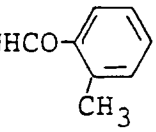

Crystalline form: White powder

Recrystallization solvent: Dichloromethane/diethyl ether

Melting Point: 186 - 189°C

Form: Free

Example 1149

Structure

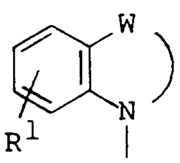 : 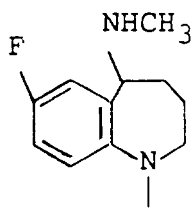

$R^2$: 2-Cl

$R^3$: 4-NHCO-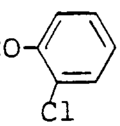

Crystalline form: Colorless amorphous

NMR analysis: 288)

Form: Free

Example 1150

Structure

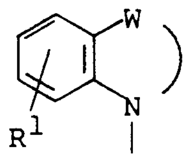 : 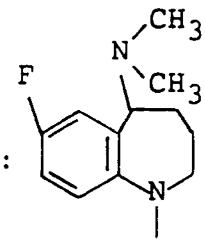 $R^2$: 2-Cl

$R^3$: 4-NHCO- 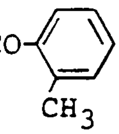

Crystalline form: Colorless amorphous

NMR analysis: 292)

Form: Free

---

Example 1151

Structure

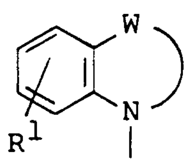 : 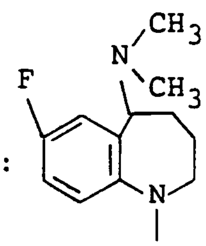 $R^2$: 2-Cl

$R^3$: 4-NHCO- 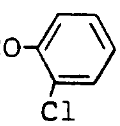

Crystalline form: Colorless amorphous

NMR analysis: 293)

Form: Free

Example 1152

Structure 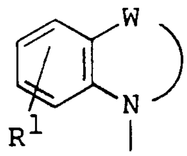 : 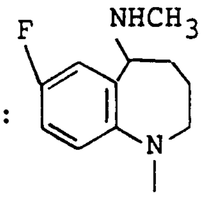 $R^2$: 3-$OCH_3$

$R^3$: 4-NHCO- 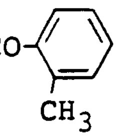

Crystalline form: White powder

Recrystallization solvent: Dichloromethane/diethyl ether

Melting Point: 144 - 145°C

Form: Free

Example 1153

Structure 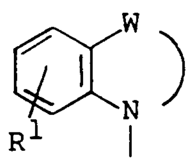 : 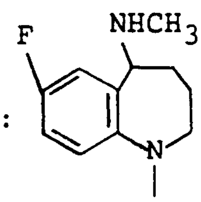 $R^2$: 3-$OCH_3$

$R^3$: 4-NHCO- 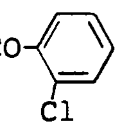

Crystalline form: White powder

Recrystallization solvent: Dichloromethane/diethyl ether

Melting Point: 149 - 150°C

Form: Free

Example 1154

Structure

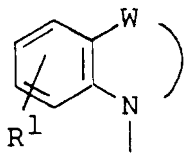 : 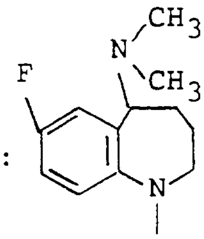

$R^2$: 3-$OCH_3$

$R^3$: 4-NHCO- 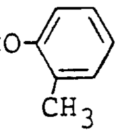

Crystalline form: Colorless amorphous

NMR analysis: 294)

Form: Free

Example 1155

Structure

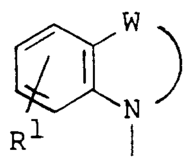 : 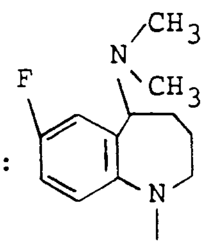

$R^2$: 3-$OCH_3$

$R^3$: 4-NHCO- 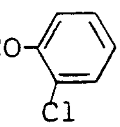

Crystalline form: Colorless amorphous

NMR analysis: 295)

Form: Free

Example 1156

Structure

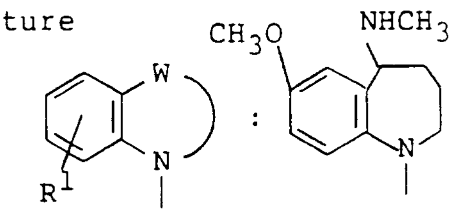

$R^2$: 2-$CH_3$

$R^3$: 4-NHCO-

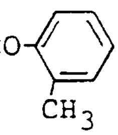

Crystalline form: Colorless amorphous

NMR analysis: 301)

Form: Free

Example 1157

Structure

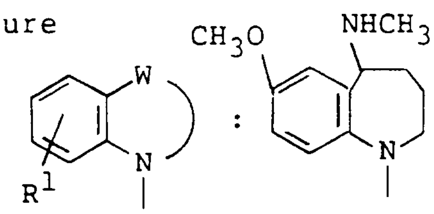

$R^2$: 2-$CH_3$

$R^3$: 4-NHCO-

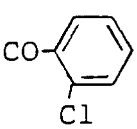

Crystalline form: Colorless amorphous

NMR analysis: 302)

Form: Free

Example 1158

Structure

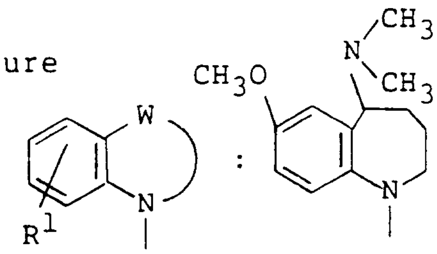

:

$R^2$: 2-$CH_3$

$R^3$: 4-NHCO-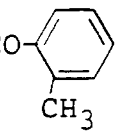

Crystalline form: Colorless amorphous

NMR analysis: 303)

Form: Free

Example 1159

Structure

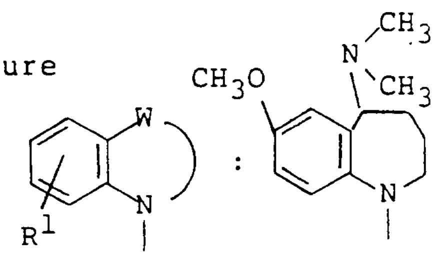

:

$R^2$: 2-$CH_3$

$R^3$: 4-NHCO-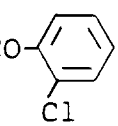

Crystalline form: Colorless amorphous

NMR analysis: 304)

Form: Free

Example 1160

Structure

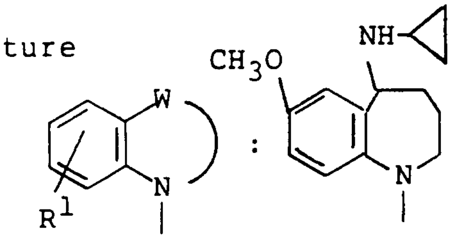

$R^2$: H

$R^3$: 4-NHCO- 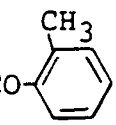

Crystalline form: White powder

Recrystallization solvent: Ethyl acetate/diisopropyl ether

Melting Point: 191 - 193°C

Form: Free

Example 1161

Structure

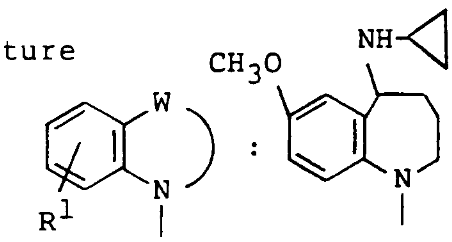

$R^2$: H

$R^3$: 4-NHCO- 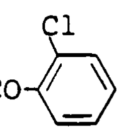

Crystalline form: White powder

Recrystallization solvent: Ethyl acetate/diisopropyl ether

Melting Point: 221 - 223°C

Form: Free

Example 1162

Structure 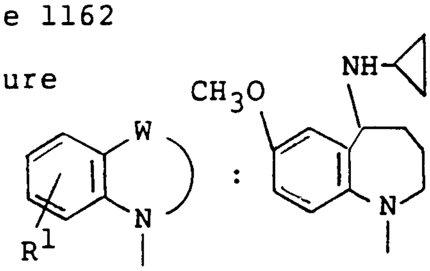 $R^2$: 3-$OCH_3$

$R^3$: 4-NHCO- 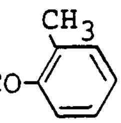

Crystalline form: White powder

Recrystallization solvent: Ethyl acetate/n-hexane

Melting Point: 159 - 161°C

Form: Free

Example 1163

Structure 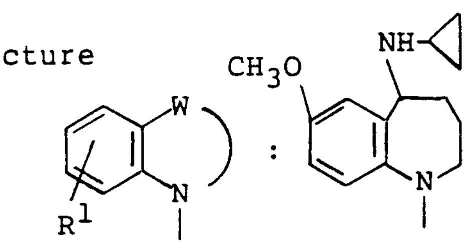 $R^2$: 3-$OCH_3$

$R^3$: 4-NHCO- 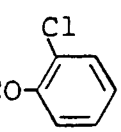

Crystalline form: White powder

Recrystallization solvent: Ethyl acetate/n-hexane

Melting Point: 174 - 175°C

Form: Free

Example 1164

Structure

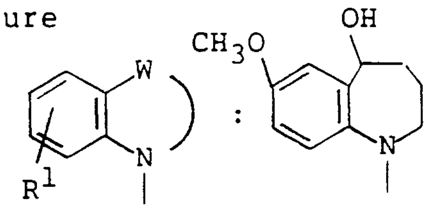

$R^2$: 2-Cl

$R^3$: 4-NHCO-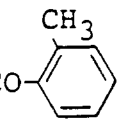

Crystalline form: Colorless amorphous

NMR analysis: 256)

Form: Free

Example 1165

Structure

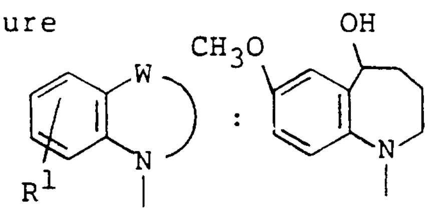

$R^2$: 2-Cl

$R^3$: 4-NHCO-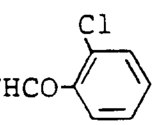

Crystalline form: Colorless amorphous

NMR analysis: 257)

Form: Free

Example 1166

Structure

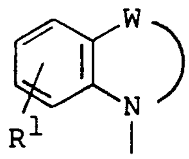 : 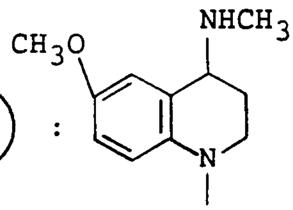

$R^2$: H

$R^3$: 4-NHCO- 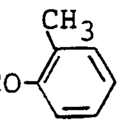

Crystalline form: Colorless amorphous

NMR analysis: 258)

Form: Free

Example 1167

Structure

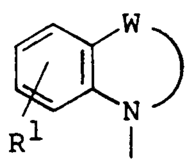 : 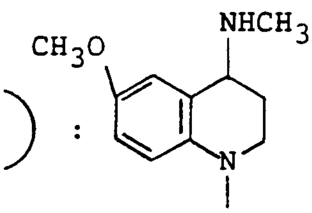

$R^2$: 2-Cl

$R^3$: 4-NHCO- 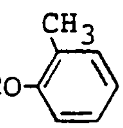

Crystalline form: Colorless amorphous

NMR analysis: 259)

Form: Free

Example 1168

Structure

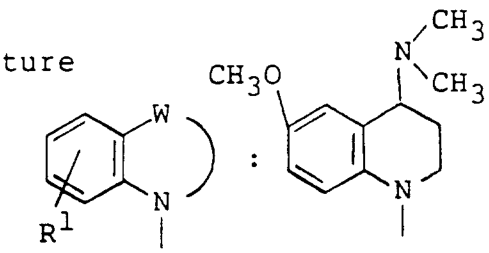

$R^2$: H

$R^3$: 4-NHCO-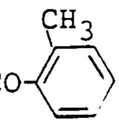

Crystalline form: Colorless amorphous

NMR analysis: 260)

Form: Free

Example 1169

Structure

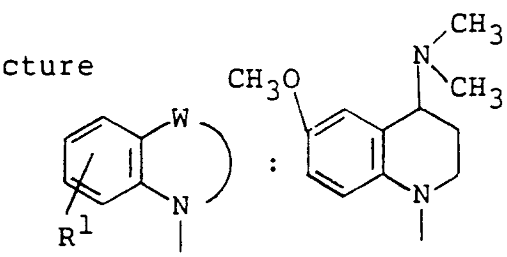

$R^2$: 2-Cl

$R^3$: 4-NHCO-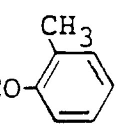

Crystalline form: Colorless amorphous

NMR analysis: 261)

Form: Free

Example 1170

Structure

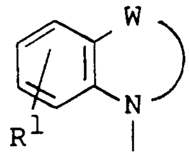 : 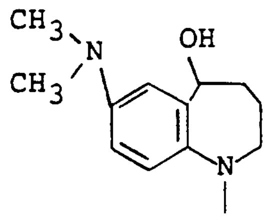 $R^2$: H

$R^3$: 4-NHCO-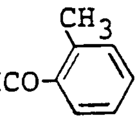

Crystalline form: Colorless amorphous

NMR analysis: 296)

Form: Free

Example 1171

Structure

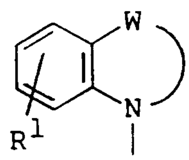 : 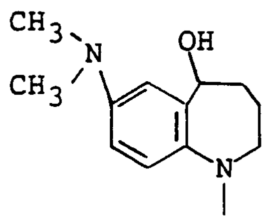 $R^2$: 2-Cl

$R^3$: 4-NHCO-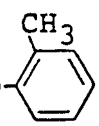

Crystalline form: White powder

Recrystallization solvent: Ethanol/diethyl ether/n-hexane

Melting Point: 159 - 162°C

Form: Free

Example 1172

Structure

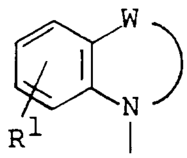 : 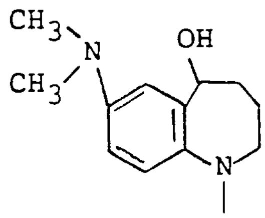

$R^2$: 2-Cl

$R^3$: 4-NHCO- 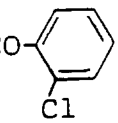

Crystalline form: Colorless needles

Recrystallization solvent: Ethanol/diethyl ether/n-hexane

Melting Point: 221 - 224°C

Form: Free

Example 1173

Structure

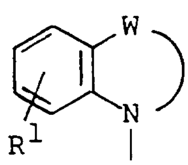 : 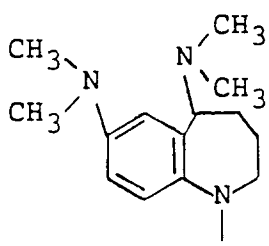

$R^2$: 2-Cl

$R^3$: 4-NHCO- 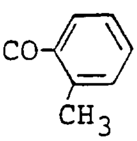

Crystalline form: Colorless prisms

Recrystallization solvent: Ethanol/diethyl ether

Melting Point: 199 - 202°C

Form: Free

Example 1174

Structure

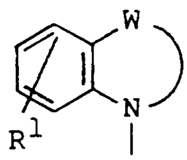 : 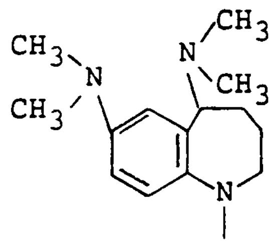

$R^2$: 2-Cl

$R^3$: 4-NHCO- 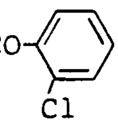

Crystalline form: Colorless prisms

Recrystallization solvent: Ethanol/diethyl ether

Melting Point: 215 - 218°C

Form: Free

Example 1175

Structure

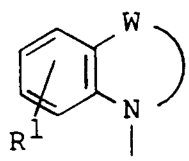 : 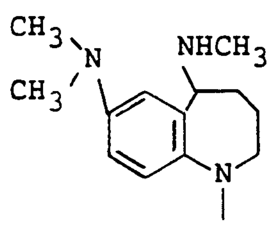

$R^2$: 2-Cl

$R^3$: 4-NHCO- 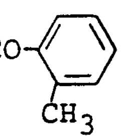

Crystalline form: Colorless needles

Recrystallization solvent: Ethanol/diethyl ether/n-hexane

Melting Point: 167 - 170°C

Form: Free

Example 1176

Structure

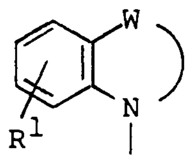 : 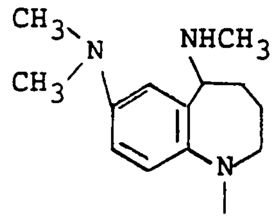

$R^2$: 2-Cl

$R^3$: 4-NHCO- 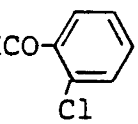

Crystalline form: White powder

Recrystallization solvent: Ethanol/diethyl ether/n-hexane

Melting Point: 191 - 193°C

Form: Free

Example 1177

Structure

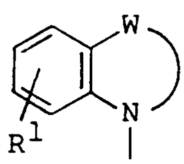 : 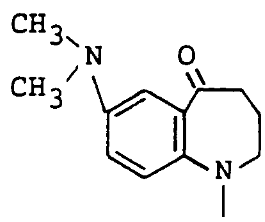

$R^2$: 2-Cl

$R^3$: 4-NHCO- 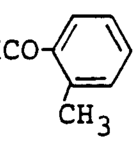

Crystalline form: Light yellow amorphous

NMR analysis: 262)

Form: Free

Example 1178

Structure

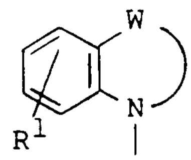 : 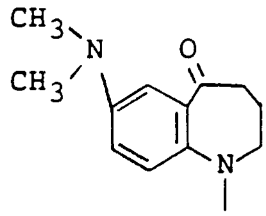

$R^2$: 2-Cl

$R^3$: 4-NHCO- 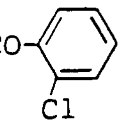

Crystalline form: Light yellow amorphous

NMR analysis: 263)

Form: Free

Example 1179

Structure

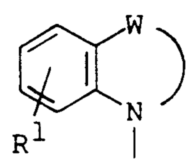 : 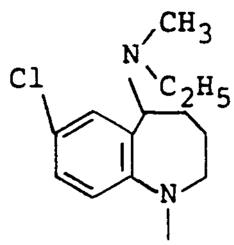

$R^2$: 3-$OCH_3$

$R^3$: 4-NHCO- 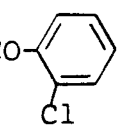

Crystalline form: Colorless amorphous

NMR analysis: 297)

Form: Free

Example 1180

Structure

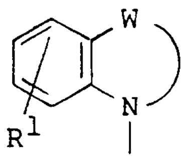 : 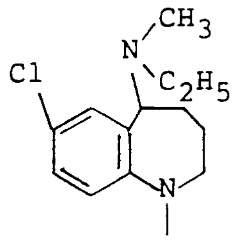

$R^2$: 3-$OCH_3$

$R^3$: 4-NHCO- 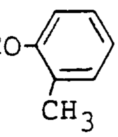

Crystalline form: Colorless amorphous

NMR analysis: 298)

Form: Free

Example 1181

Structure

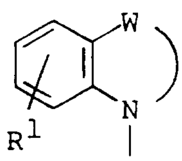 : 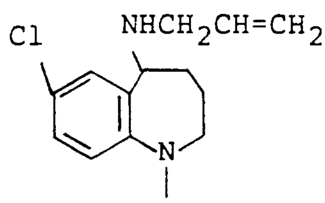

$R^2$: 3-$OCH_3$

$R^3$: 4-NHCO- 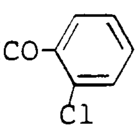

Crystalline form: Colorless amorphous

NMR analysis: 299)

Form: Free

Example 1182

Structure

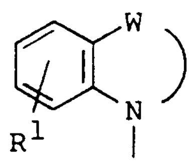 : 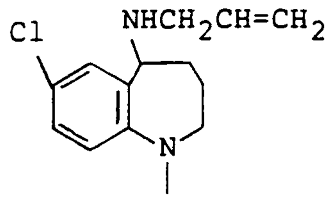

$R^2$: 3-$OCH_3$

$R^3$: 4-NHCO- 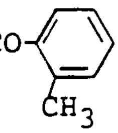

Crystalline form: Colorless amorphous

NMR analysis: 300)

Form: Free

Example 1183

Structure

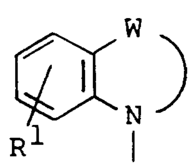 : 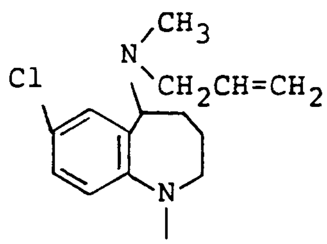

$R^2$: 3-$OCH_3$

$R^3$: 4-NHCO- 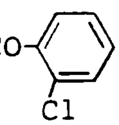

Crystalline form: Colorless amorphous

NMR analysis: 307)

Form: Free

Example 1184

Structure

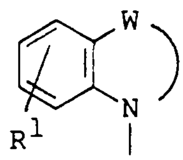 : 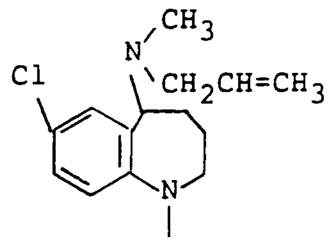

$R^2$: 3-$OCH_3$

$R^3$: 4-NHCO- 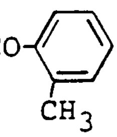

Crystalline form: Colorless amorphous

NMR analysis: 308)

Form: Free

Example 1185

Structure

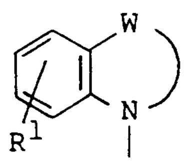 : 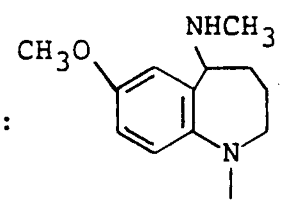

$R^2$: 2-$OCH_3$

$R^3$: 4-NHCO- 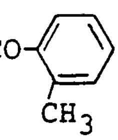

Crystalline form: Colorless amorphous

NMR analysis: 309)

Form: Free

Example 1186

Structure

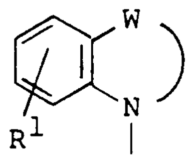 : 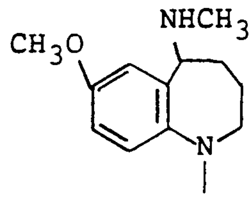

$R^2$: 2-$OCH_3$

$R^3$: 4-NHCO- 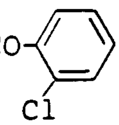

Crystalline form: Colorless amorphous

NMR analysis: 310)

Form: Free

Example 1187

Structure

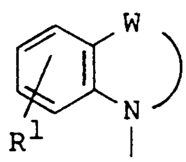 : 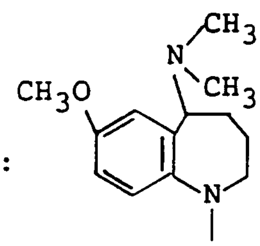

$R^2$: 2-$OCH_3$

$R^3$: 4-NHCO- 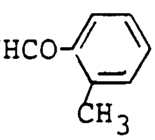

Crystalline form: Colorless amorphous

NMR analysis: 311)

Form: Free

Example 1188

Structure

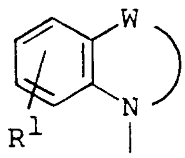 : 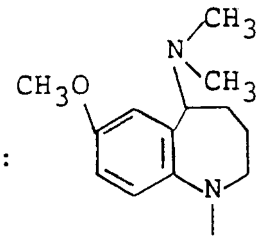

$R^2$: 2-$OCH_3$

$R^3$: 4-NHCO- 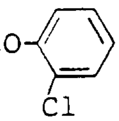

Crystalline form: Colorless amorphous

NMR analysis: 312)

Form: Free

238) $^1$H-NMR (DMSO-$d_6$) δ ; 1.4-2.1 (4H, m), 2.34 (3H, s), 2.8-5.4 (4H, m), 7.09 (1H, d, J=8.4 Hz), 7.15-7.7 (9H, m), 7.76 (1H, d, J=2.6 Hz), 10.41 (1H, s)

239) $^1$H-NMR (DMSO-$d_6$) δ ; 1.5-2.2 (4H, m), 2.8-5.2 (4H, m), 7.09 (1H, d, J=8.4 Hz), 7.2-7.7 (9H, m), 7.76 (1H, d, J=2.6 Hz), 10.63 (1H, s)

240) $^1$H-NMR ($CDCl_3$) δ ; 1.65-2.2 (4H, m), 2.25-2.65 (6H, m), 2.75-4.6 (4H, m), 6.8-8.15 (11H, m)

241) $^1$H-NMR ($CDCl_3$) δ ; 1.4-2.25 (4H, m), 2.25-2.55 (3H, m), 2.7-4.8 (4H, m), 6.8-8.3 (11H, m)

242) $^1$H-NMR ($CDCl_3$) δ ; 1.4-2.1 (4H, m), 2.35-2.6 (3H, m), 2.8-5.2 (7H, m), 6.8-8.05 (11H, m)

243) $^1$H-NMR ($CDCl_3$) δ ; 1.4-2.15 (4H, m), 2.4-5.2 (7H, m), 6.8-7.85 (10H, m), 7.9-8.3 (1H, m)

244) $^1$H-NMR ($CDCl_3$) δ ; 1.20-2.38 (11H, m), 2.98-5.10 (3H, m), 6.45-7.04 (2H, m), 7.05-7.86 (8H, m), 8.00-8.50 (1H, m)

245) $^1$H-NMR ($CDCl_3$) δ ; 1.05-2.78 (14H, m), 2.78-5.18 (2H, m), 6.36-7.03 (2H, m), 7.06-7.90 (8H, m), 7.98-8.39 (1H, m)

246) $^1$H-NMR ($CDCl_3$) δ ; 1.75-2.54 (2H, m), 2.60-4.03 (4H, m), 3.37 (3H, brs), 5.17 (2H, s), 6.60-6.83 (3H, m), 6.90-7.07 (1H, m), 7.07-7.20 (1H, m), 7.22-7.50 (6H, m), 7.73-7.84 (1H, m)

247) $^1$H-NMR ($CDCl_3$) δ ; 1.60-2.40 (2H, m), 2.45 (3H, s), 2.65-3.06 (2H, m), 3.06-5.28 (2H, m), 3.35 (3H,

brs), 6.59-7.60 (9H, m), 7.67-7.88 (1H, m), 8.12 (1H, brs)

248) $^1$H-NMR ($CDCl_3$) δ ; 1.60-2.52 (2H, m), 2.64-5.32 (4H, m), 3.37 (3H, brs), 6.60-7.98 (10H, m), 8.50 (1H, brs)

249) $^1$H-NMR ($CDCl_3$) δ ; 1.18-3.15 (12H, m), 3.40-4.38 (5H, m), 6.58-7.75 (10H, m), 8.30-8.71 (1H, m)

250) $^1$H-NMR ($CDCl_3$) δ ; 0.26-0.71 (4H, m), 1.15-3.29 (10H, m), 3.40-4.95 (5H, m), 6.60-7.85 (10H, m), 8.18-8.68 (1H, m)

251) $^1$H-NMR ($CDCl_3$) δ ; 0.25-0.72 (4H, m), 1.16-2.35 (6H, m), 2.35-3.30 (4H, m), 3.43-4.98 (2H, m), 6.57-7.94 (10H, m), 8.22-8.89 (1H, m)

252) $^1$H-NMR ($CDCl_3$) δ ; 0.69-2.90 (9H, m), 2.90-5.10 (5H, m), 6.40-7.85 (10H, m), 8.25-8.54 (1H, m)

253) $^1$H-NMR ($CDCl_3$) δ ; 2.17 (2H, brs), 2.34 (3H, s), 2.49 (3H, s), 2.87 (2H, t, J=6.0 Hz), 3.10-5.00 (2H, m), 3.70 (3H, s), 6.67 (1H, d, J=8.0 Hz), 6.85-6.88 (2H, m), 7.09 (1H, dd, J=1.5, 8.0 Hz), 7.21-7.50 (4H, m), 7.64 (1H, d, J=1.9 Hz), 8.11 (1H, m), 8.33 (1H, d, J=8.8 Hz)

254) $^1$H-NMR ($CDCl_3$) δ ; 1.42-5.06 (13H, m), 6.51 (1H, d, J=7.8 Hz), 6.76 (1H, m), 7.01-7.63 (10H, m), 8.53 (1H, m)

255) $^1$H-NMR ($CDCl_3$) δ ; 1.26-4.93 (16H, m), 6.69-7.73 (10H, m), 8.62-8.84 (1H, m)

256) $^1$H-NMR ($CDCl_3$) δ ; 1.45-1.90 (2H, m), 1.90-2.33 (2H, m), 2.33-3.25 (4H, m), 3.60-3.93 (3H, m), 4.45-5.15 (2H, m), 6.40-8.25 (11H, m)

257) $^1$H-NMR ($CDCl_3$) δ ; 1.49-1.97 (2H, m), 1.97-3.10 (3H, m), 3.58-3.98 (3H, m), 4.60-5.26 (2H, m), 6.44-8.36 (11H, m)

258) $^1$H-NMR ($CDCl_3$) δ ; 1.82-2.13 (1H, m), 2.13-2.43 (1H, m), 2.50 (3H, s), 2.57 (3H, s), 3.69-4.06 (3H, m), 3.78 (3H, s), 6.45-6.80 (2H, m), 6.85-7.00 (1H, m), 7.18-7.80 (9H, m)

259) $^1$H-NMR ($CDCl_3$) δ ; 1.72-2.05 (1H, m), 2.11-2.40 (1H, m), 2.51 (3H, s), 2.57 (3H, s), 3.40-4.20 (3H, m), 3.77 (3H, s), 6.35-6.64 (1H, m), 6.79-6.96 (1H, m), 7.15-8.13 (9H, m)

260) $^1$H-NMR ($CDCl_3$) δ ; 1.71-2.05 (1H, m), 2.07-2.32 (1H, m), 2.33 (6H, s), 2.47 (3H, s), 3.50-3.80 (2H, m), 3.76 (3H, s), 3.95-4.17 (1H, m), 6.40-6.70 (2H, m), 6.90-7.03 (1H, m), 7.14-7.77 (8H, m), 7.90-8.14 (1H, m)

261) $^1$H-NMR ($CDCl_3$) δ ; 1.76-2.70 (2H, m), 2.30 (6H, s), 2.47 (3H, s), 3.23-4.40 (3H, m), 3.73 (3H, s), 6.30-6.65 (2H, m), 6.65-8.76 (9H, m)

262) $^1$H-NMR ($CDCl_3$) δ ; 1.68-2.35 (2H, m), 2.36-5.11 [13H, m, 2.45 (3H, s), 2.92 (6H, s)], 6.56 (1H, dd, J=3.1, 8.7 Hz), 6.78-7.06 (2H, m), 6.82 (1H, d, J=8.7 Hz), 7.11-7.68 (6H, m), 7.97 (1H, brs)

263) $^1H$-NMR ($CDCl_3$) δ ; 1.69-2.30 (2H, m), 2.59-5.10 [10H, m, 2.92 (6H, s)], 6.56 (1H, dd, J=3.1, 8.8 Hz), 6.72-7.90 (9H, m), 8.42 (1H, brs)

264) $^1H$-NMR ($CDCl_3$) δ ; 1.49 (1H, brs), 1.82-2.01 (1H, m), 2.03-2.26 (1H, m), 2.46 (3H, s), 2.54 (3H, s), 3.67-3.76 (1H, m), 3.86 (2H, t, J=6.8 Hz), 6.67 (1H, d, J=8.6 Hz), 6.93 (1H, dd, J=8.6, 2.5 Hz), 7.13-7.43 (9H, m), 8.15 (1H, brs)

265) $^1H$-NMR ($CDCl_3$) δ ; 1.58 (1H, brs), 1.86-2.03 (1H, m), 2.08-2.30 (1H, m), 2.56 (3H, s), 3.69-3.78 (1H, m), 3.91 (2H, t, J=6.5 Hz), 6.69 (1H, d, J=8.7 Hz), 6.94 (1H, dd, J=8.6, 2.5 Hz), 7.33-7.47 (6H, m), 7.54-7.63 (2H, m), 7.67-7.77 (1H, m), 8.16 (1H, brs)

266) $^1H$-NMR ($CDCl_3$) δ ; 1.50 (1H, brs), 1.76-2.23 (2H, m), 2.42 (3H, s), 2.47 (3H, s), 3.55-3.94 (3H, m), 6.28-7.78 (10H, m), 8.91 (1H, brs)

267) $^1H$-NMR ($CDCl_3$) δ ; 1.46 (1H, brs), 1.82-2.28 (2H, m), 2.50 (3H, s), 3.52-4.08 (3H, m), 6.34-7.75 (10H, m), 8.61 (1H, brs)

268) $^1H$-NMR ($CDCl_3$) δ ; 1.80-2.31 (2H, m), 2.32 (3H, s), 2.48 (3H, s), 3.51-3.82 (2H, m), 3.95-4.15 (1H, m), 6.59 (1H, d, J=8.6 Hz), 6.90 (1H, dd, J=8.6, 2.5 Hz), 7.16-7.61 (9H, m), 7.88 (1H, brs)

269) $^1H$-NMR ($CDCl_3$) δ ; 1.86-2.04 (1H, m), 2.13-2.31 (1H, m), 2.33 (3H, s), 3.53-3.62 (1H, m), 3.76 (1H,

dt, J=12.8, 6.4 Hz), 6.60 (1H, d, J=8.7 Hz), 6.91 (1H, dd, J=8.7, 2.5 Hz), 7.33-7.52 (6H, m), 7.54-7.66 (2H, m), 7.73-7.82 (1H, m), 8.07 (1H, brs)

270) $^1$H-NMR ($CDCl_3$) δ ; 1.65-2.27 (2H, m), 2.28 (6H, s), 2.48 (3H, s), 3.37-4.07 (3H, m), 6.33-7.91 (10H, m), 8.20 (1H, brs)

271) $^1$H-NMR ($CDCl_3$) δ ; 1.71-2.26 (2H, m), 2.28 (6H, s), 3.36-4.10 (3H, m), 6.35-7.95 (10H, m), 8.59 (1H, brs)

272) $^1$H-NMR ($CDCl_3$) δ ; 2.02-2.23 (2H, m), 2.28 (3H, s), 2.47 (3H, s), 2.56 (3H, s), 3.73-4.07 (3H, m), 4.68 (1H, brs), 6.61 (1H, d, J=8.1 Hz), 6.72-6.83 (1H, m), 7.17-7.63 (11H, m), 8.03 (1H, brs)

273) $^1$H-NMR ($CDCl_3$) δ ; 1.61 (1H, brs), 1.87-2.25 (2H, m), 2.29 (3H, s), 2.56 (3H, s), 3.67-3.78 (1H, m), 3.91 (2H, t, J=6.9 Hz), 6.52-6.79 (2H, m), 7.09-7.15 (1H, m), 7.30-7.90 (8H, m), 8.23 (1H, brs)

274) $^1$H-NMR ($CDCl_3$) δ ; 1.58 (1H, brs), 1.82-2.23 (2H, m), 2.27 (3H, s), 2.48 (3H, s), 2.50 (3H, s), 3.47-4.05 (3H, m), 6.23-6.83 (2H, m), 7.00-7.50 (7H, m), 7.53-7.74 (1H, m), 8.28 (1H, brs)

275) $^1$H-NMR ($CDCl_3$) δ ; 1.60 (1H, brs), 1.82-2.35 (5H, m), 2.49 (3H, s), 3.41-4.08 (3H, m), 6.30-6.80 (1H, m), 6.98-7.68 (8H, m), 7.31-7.82 (1H, m), 8.77 (1H, brs)

276) $^1$H-NMR ($CDCl_3$) δ ; 1.76-2.03 (2H, m), 2.27 (3H, s),

2.32 (6H, s), 2.47 (3H, s), 3.48-3.58 (1H, m), 3.66 (1H, dt, J=12.7, 6.1 Hz), 3.97-4.14 (1H, m), 6.48 (1H, d, J=8.2 Hz), 6.65-6.77 (1H, m), 7.14-7.59 (9H, m), 7.96 (1H, brs)

277) $^1$H-NMR ($CDCl_3$) δ ; 1.75-2.04 (2H, m), 2.27 (3H, s), 2.33 (6H, s), 3.48-3.58 (1H, m), 3.67 (1H, dt, J=12.7, 6.1 Hz), 3.98-4.16 (1H, m), 6.48 (1H, d, J=8.2 Hz), 6.72 (1H, dd, J=8.2, 1.9 Hz), 7.16 (1H, d, J=1.9 Hz), 7.27-7.91 (8H, m), 8.31 (1H, brs)

278) $^1$H-NMR ($CDCl_3$) δ ; 1.72-2.05 (2H, m), 2.28 (9H, s), 2.47 (3H, s), 3.16-4.34 (3H, m), 6.38-7.79 (10H, m), 8.37 (1H, brs)

279) $^1$H-NMR ($CDCl_3$) δ ; 1.65-2.07 (2H, m), 2.28 (9H, s), 3.26-4.38 (3H, m), 6.34-8.06 (10H, m), 8.53 (1H, brs)

280) Two stereoisomers: Both colorless amorphous

Isomer A:

$[\alpha]_D^{22}$ = 0° (chloroform, c=1.0)

$^1$H-NMR ($CDCl_3$) δ ; 1.04 (3H, d, J=6.9 Hz), 1.59 (1H, brs), 2.25-2.45 (1H, m), 2.49 (3H, s), 2.52 (3H, s), 3.53-3.69 (2H, m), 3.91 (1H, abq, J=7.2, 12.9 Hz), 6.60 (1H, d, J=8.6 Hz), 6.93 (1H, dd, J=8.6, 2.5 Hz), 7.18-7.60 (9H, m), 7.76 (1H, brs)

Isomer B:

$[\alpha]_D^{22}$ = 0° (chloroform, c=1.0)

$^1H$-NMR ($CDCl_3$) δ ; 1.06 (3H, d, J=6.9 Hz), 1.60 (1H, brs), 2.21-2.43 (1H, m), 2.47 (3H, s), 2.52 (3H, s), 3.51-3.66 (2H, m), 3.93 (1H, abq, J=7.5, 12.9 Hz), 6.60-6.68 (1H, m), 6.95 (1H, dt, J=7.5, 1.8 Hz), 7.03 (1H, dt, J=7.4, 1.4 Hz), 7.17-7.55 (8H, m), 7.81 (1H, brs)

281) Two stereoisomers: Both colorless amorphous

Isomer A:

$[\alpha]_D^{22}$ = 0° (chloroform, c=1.0)

$^1H$-NMR ($CDCl_3$) δ ; 1.04 (3H, d, J=6.9 Hz), 1.55 (1H, brs), 2.23-2.46 (1H, m), 2.53 (3H, s), 3.53-3.67 (2H, m), 3.91 (1H, abq, J=7.1, 12.9 Hz), 6.61 (1H, d, J=8.6 Hz), 6.93 (1H, dd, J=8.6, 2.5 Hz), 7.28-7.52 (6H, m), 7.54-7.65 (2H, m), 7.70-7.79 (1H, m), 8.16 (1H, brs)

Isomer B:

$[\alpha]_D^{22}$ = 0° (chloroform, c=1.0)

$^1H$-NMR ($CDCl_3$) δ ; 1.06 (3H, d, J=6.9 Hz), 1.61 (1H, brs), 2.21-2.42 (1H, m), 2.51 (3H, s), 3.48-3.67 (2H, m), 3.90 (1H, abq, J=7.4, 12.9 Hz), 6.59-6.67 (1H, m), 6.94 (1H, dt, J=7.5, 1.9 Hz), 7.03 (1H, dt, J=7.4, 1.4 Hz), 7.23-7.75 (8H, m), 8.41 (1H, brs)

282) Two stereoisomers: Both colorless amorphous

Isomer A:

$[\alpha]_D^{22}$ = 0° (chloroform, c=1.0)

$^1$H-NMR ($CDCl_3$) δ ; 0.99 (3H, d, J=6.5 Hz), 1.37 (1H, brs), 2.16-2.40 (1H, m), 2.46 (3H, s), 2.48 (3H, s), 3.38-3.96 (3H, m), 6.30-7.28 (10H, m), 8.26 (1H, brs)

Isomer B:

$[\alpha]_D^{22}$ = 0° (chloroform, c=1.0)

$^1$H-NMR ($CDCl_3$) δ ; 1.03 (3H, d,J=6.7 Hz), 1.44 (1H, brs), 2.17-2.40 (1H, m), 2.45 (3H, s), 2.47 (3H, s), 3.40-3.98 (3H, m), 6.47-7.73 (10H, m), 8.23 (1H, brs)

283) Two stereoisomers: Both colorless amorphous

Isomer A:

$[\alpha]_D^{22}$ = 0° (chloroform, c=1.0)

$^1$H-NMR ($CDCl_3$) δ ; 1.00 (3H, d, J=6.6 Hz), 1.40 (1H, brs), 2.18-2.42 (1H, m), 2.47 (3H, s), 3.36-4.02 (3H, m), 6.32-7.78 (10H, m), 8.55 (1H, brs)

Isomer B:

$[\alpha]_D^{22}$ = 0° (chloroform, c=1.0)

$^1$H-NMR ($CDCl_3$) δ ; 1.03 (3H, d, J=6.5 Hz), 1.39 (1H, brs), 2.14-2.39 (1H, m), 2.45 (3H, s). 3.34-3.98 (3H, m), 6.53-7.98 (10H, m), 8.78 (1H, brs)

284) $^1$H-NMR ($CDCl_3$) δ ; 1.05-1.25 (3H, m), 1.25-2.80 (10H, m), 3.00-5.10 (3H, m), 6.75-8.40 (11H, m)

285) $^1H$-NMR ($CDCl_3$) δ ; 1.00-2.80 (12H, m), 3.00-5.10 (3H, m), 6.70-7.80 (10H, m), 8.30-8.80 (1H, m)

286) $^1H$-NMR ($CDCl_3$) δ ; 0.95-2.80 (15H, m), 2.80-5.15 (3H, m), 6.70-7.05 (2H, m), 7.10-7.80 (10H, m), 7.95-8.45 (1H, m)

287) $^1H$-NMR ($CDCl_3$) δ ; 0.80-2.60 (16H, m), 2.60-5.05 (4H, m), 6.70-7.70 (10H, m), 7.85-8.40 (1H, m)

288) $^1H$-NMR ($CDCl_3$) δ ; 1.30-2.60 (8H, m), 2.60-5.10 (3H, m), 6.60-7.95 (10H, m), 8.25-8.70 (1H, m)

289) $^1H$-NMR ($CDCl_3$) δ ; 1.27-4.91 (19H, m), 6.68-7.73 (10H, m), 8.40-8.71 (1H, m)

290) $^1H$-NMR ($CDCl_3$) δ ; 1.81-2.54 (6H, m), 2.15 (3H, s), 2.41 (3H, s), 2.46 (3H, s), 3.61-3.71 (3H, m), 6.91-7.43 (10H, m), 8.60 (1H, s)

291) $^1H$-NMR ($CDCl_3$) δ ; 1.86-2.50 (3H, m), 2.28 (9H, s), 2.49 (3H, s), 6.60-7.47 (10H, m), 7.75 (1H, m)

292) $^1H$-NMR ($CDCl_3$) δ ; 1.15-2.55 (13H, m), 2.55-5.10 (3H, m), 6.60-8.40 (11H, m)

293) $^1H$-NMR ($CDCl_3$) δ ; 1.15-2.45 (10H, m), 2.55-5.10 (3H, m), 6.60-7.80 (10H, m), 8.30-8.70 (1H, m)

294) $^1H$-NMR ($CDCl_3$) δ ; 1.10-2.60 (4H, m), 2.41 (6H, s), 2.49 (3H, s), 3.76 (3H, s), 2.60-5.20 (3H, m), 6.50-6.80 (3H, m), 6.90-7.60 (6H, m), 8.13 (1H, s), 8.30 (1H, d, J=8.5 Hz)

295) $^1H$-NMR ($CDCl_3$) δ ; 1.15-2.50 (4H, m), 2.41 (6H, s), 2.60-5.20 (3H, m), 3.77 (3H, s), 6.50-7.50 (8H, m),

7.65-7.80 (1H, m), 8.31 (1H, d, J=8.4 Hz), 8.61 (1H, s)

296) $^1$H-NMR ($CDCl_3$) δ ; 1.55-3.13 (12H, m), 2.44 (3H, s), 4.60-5.14 (2H, m), 6.28 (1H, dd, J=2.5, 8.5 Hz), 6.48 (1H, d, J=8.5 Hz), 6.99 (1H, d, J=2.5 Hz), 7.07-7.58 (8H, m), 7.80 (1H, brs)

297) $^1$H-NMR ($CDCl_3$) δ ; 1.01-2.88, 3.22-4.41, 4.90-5.28 [total 18H, 1.17 (3H, t, J=7.2 Hz), 2.40 (3H, s), 3.77 (3H, s)], 6.55 (1H, d, J=8.1 Hz), 6.60-7.98 (8H, m), 8.23-8.75 (2H, m)

298) $^1$H-NMR ($CDCl_3$) δ ; 1.00-3.04, 3.24-4.45. 4.91-5.27 [total 21H, m, 1.17 (3H, t, J=7.0 Hz), 2.39 (3H, s), 2.50 (3H, s), 3.75 (3H, s)], 8.56 (1H, d, J=8.3 Hz), 6.69 (1H, d, J=8.3 Hz), 6.82-7.75 (7H, m), 8.05-8.49 (2H, m)

299) $^1$H-NMR ($CDCl_3$) δ ; 1.21-4.62, 4.90-5.43 (total 15H, m), 5.70-6.11 (1H, m), 6.35-7.90 (9H, m), 8.07-8.92 (2H, m)

300) $^1$H-NMR ($CDCl_3$) δ ; 1.20-4.68, 5.01-5.3 [total 18H, m, 2.50 (3H, s)], 5.72-6.14 (1H, m), 6.49-7.69 (9H, m), 8.01-8.58 (2H, m)

301) $^1$H-NMR ($CDCl_3$) δ ; 1.25-2.80 (14H, m), 3.00-5.10 (6H, m), 6.40-8.00 (11H, m)

302) $^1$H-NMR ($CDCl_3$) δ ; 1.30-2.90 (11H, m), 3.00-5.10 (6H, m), 6.40-7.80 (10H, m), 8.00-8.35 (1H, m)

303) $^1$H-NMR ($CDCl_3$) δ ; 1.10-2.80 (16H, m), 2.85-5.15

(6H, m), 6.40-7.80 (11H, m)

304) $^1$H-NMR ($CDCl_3$) δ ; 1.10-2.80 (13H, m), 2.90-5.10 (6H, m), 6.40-7.85 (10H, m), 7.90-8.20 (1H, m)

305) $^1$H-NMR ($CDCl_3$) δ ; 1.27-5.28 (19H, m), 3.75 (3H, s), 6.51 (1H, d, J=7.9 Hz), 6.69-6.81 (2H, m), 7.05-7.49 (6H, m), 8.14 (1H, m), 8.27 (1H, d, J=8.4 Hz)

306) Two stereoisomers: Both colorless amorphous

Isomer A:

$[\alpha]_D^{22}$ = 0° (chloroform, c=1.0)

$^1$H-NMR ($CDCl_3$) δ ; 0.78-1.02 (3H, m), 2.23-2.52 (1H, m), 2.39 (6H, s), 2.48 (3H, s), 3.17-4.30 (3H, m), 6.85-7.84 (10H, m), 8.17 (1H, brs)

Isomer B:

$[\alpha]_D^{22}$ = 0° (chloroform, c=1.0)

$^1$H-NMR ($CDCl_3$) δ ; 0.73-1.00 (3H, m), 2.17-2.52 (1H, m), 2.39 (6H, s), 2.49 (3H, s), 3.15-4.33 (3H, m), 6.36-7.55 (8H, m), 7.58-7.83 (2H, m), 8.19 (1H, brs)

307) $^1$H-NMR ($CDCl_3$) δ ; 1.25-4.44, 4.98-5.41 [total 17H, m, 2.40 (3H, s), 3.76 (3H, s)], 5.72-6.13 (1H, m), 6.56 (1H, d, J=8.4 Hz), 6.69 (1H, d, J=7.9 Hz), 6.77-7.93 (7H, m), 8.32 (1H, d, J=8.3 Hz), 8.49-8.95 (1H, m)

308) $^1$H-NMR ($CDCl_3$) δ ; 1.23-5.42 (20H, m), 5.78-6.09

(1H, m), 6.56 (1H, d, J=8.3 Hz), 6.61-7.82 (8H, m), 8.14 (1H, s), 8.30 (1H, d, J=8 Hz)

309) $^1$H-NMR ($CDCl_3$) δ ; 1.20-2.70 (11H, m), 2.80-4.90 (9H, m), 6.40-7.70 (10H, m), 8.30-8.70 (1H, m)

310) $^1$H-NMR ($CDCl_3$) δ ; 1.20-2.80 (8H, m), 2.85-5.05 (9H, m), 6.40-7.80 (10H, m), 8.10-8.50 (1H, m)

311) $^1$H-NMR ($CDCl_3$) δ ; 1.20-2.75 (13H, m), 2.80-5.10 (9H, m), 6.40-8.00 (11H, m)

312) $^1$H-NMR ($CDCl_3$) δ ; 1.20-2.80 (10H, m), 2.90-5.10 (9H, m), 6.40-7.80 (10H, m), 8.00-8.40 (1H, m)

Example 1189

By using di-p-toluoyl-L-tartaric acid monohydride or di-p-toluoyl-D-tartaric acid monohydride, the compound obtained in above Example 408 is optically resovled to give the following compounds.

(+)-5-Dimethylamino-1-[4-(2-methylbenzoylamino)benzoyl]-2,3,4,5-tetrahydro-1H-benzazepine hydrochloride
White amorphous

$[\alpha]_D^{25}$ = +234° (methanol, c=0.2)

Purity; more than 99 % ee, determined by HPLC using an optical acitive column

HPLC conditions;

| | |
|---|---|
| Mobile phase; | n-hexane : ethanol : diethylamine = 950 : 50 : 1 |
| Flow rate; | 1.0 ml/min. |
| Column; | CHIRALCEL OD, 25 cm x 0.46 cm (manufactured by Daicel Chemical Ind. Ltd.) |

Concentration of sample; 0.1 % in methanol

Retention time; 34 minutes

$^1$H-NMR (DMSO-$d_6$) δ ; 0.85-1.20, 1.56-4.06, 4.94-5.21 (total 13H, m), 2.36 (3H, s), 6.79 (1H, d, J=7.6 Hz), 7.12-7.60 (8H, m), 7.62 (2H, d, J=8.4 Hz), 8.00 (1H, d, J=7.6 Hz), 10.43 (1H, s), 11.80 (1H, brs)

(-)-5-Dimethylamino-1-[4-(2-methylbenzoylamino)benzoyl]-2,3,4,5-tetrahydro-1H-benzazepine hydrochloride
White amorphous

$[\alpha]_D^{25}$ = -23.1° (methanol, c=0.2)

Purity; more than 99 % ee, determined by HPLC using an optical active column, and the conditions are the same as above except that the retention time is 40 minutes.

$^1$H-NMR (DMSO-$d_6$) δ ; 0.83-1.19, 1.55-4.06, 4.94-5.20 (total 13H, m), 2.36 (3H, s), 6.80 (1H, d, J=7.8 Hz), 7.12-7.60 (8H, m), 7.63 (2H, d, J=8.5 Hz), 8.00 (1H, d, J=7.8 Hz), 10.44 (1H, s), 11.74 (1H, brs)

Pharmacological Test

Experiment 1 : $V_1$ receptor binding assay

Using rat liver plasma membrane preparations prepared according to Ichihara's method [cf: Akira Ichihara, J. Bio. Chem., 258, 9283 (1983)], the plasma membrane (50000dpm, $2x10^{-10}$ M) of [$^3$H]-Arg-vasopressin and a test compound (60 μg, $10^{-8}$-$10^{-4}$ M) are incubated at 37°C for 10 minutes in 100 mM Tris-HCl buffer (pH: 8.0, 250 μl) containing 5 mM

$MgCl_2$, 1 mM EDTA and 0.1 % BSA. After incubation, the mixture is filtered three times using the glass filter (GF/F) so as to separate the membrane preparation combined with vasopressin and then washed with the buffer (5 ml). This glass filter is taken out and mixed with liquid scintillation cocktail. The amount of [$^3$H]-vasopressin combined with the membrane is measured by liquid scintillation counter and the rate of the inhibitory effect of the test compound is estimated according to the following equation.

$$\text{Rate of the inhibitory effect (\%)} = 100 - \frac{C_1 - B_1}{C_0 - B_1} \times 100$$

$C^1$: The amount of [$^3$H]-vasopressin combined with the membrane in the presence of the test compound (in prescribed amount).

$C^0$: The amount of [$^3$H]-vasopressin combined with the membrane in the absence of the test compound.

$B^1$: The amount of [$^3$H]-vasopressin combined with the membrane in the presence of the excess amount of vasopressin ($10^{-6}$ M).

The results are expressed as $IC_{50}$ values, which is the concentration of the test compound required to achieve the inhibitory effect in the rate of 50 %.

The results are shown in the following Table 5.

Test compound

1. 1-(4-Benzoylaminobenzoyl)-1,2,3,4-tetrahydroquinoline
2. 1-[4-(3-Chlorobenzoylamino)benzoyl]-1,2,3,4-tetrahydroquinoline
3. 1-[4-(3-Methoxybenzoylamino)benzoyl]-1,2,3,4-tetrahydroquinoline
4. 1-[4-(3-Cyanobenzoylamino)benzoyl]-1,2,3,4-tetrahydroquinoline
5. 1-[4-(3-Aminobenzoylamino)benzoyl]-1,2,3,4-tetrahydroquinoline
6. 1-[4-(2,3-Dimethylbenzoylamino)benzoyl]-1,2,3,4-tetrahydroquinoline
7. 1-[4-(2-Methylbenzoylamino)benzoyl]-1,2,3,4-tetrahydroquinoline
8. 1-[4-(2-Trifluoromethylbenzoylamino)benzoyl]-1,2,3,4-tetrahydroquinoline
9. 1-[4-(2-Nitrobenzoylamino)benzoyl]-1,2,3,4-tetrahydroquinoline
10. 1-[4-(3,5-Dichlorobenzoylamino)benzoyl]-1,2,3,4-tetrahydroquinoline
11. 1-[4-(3,3-Dimethylbutyrylamino)benzoyl]-1,2,3,4-tetrahydroquinoline
12. 1-[4-(2-Cyclohexylacetylamino)benzoyl]-1,2,3,4-tetrahydroquinoline
13. 1-[4-(2-Phenylacetylamino)benzoyl]-1,2,3,4-tetrahydroquinoline
14. 1-(4-Cyclohexylcarbonylaminobenzoyl)-1,2,3,4-tetrahydroquinoline
15. 1-(4-Cycloheptylcarbonylaminobenzoyl)-1,2,3,4-tetrahydroquinoline
16. 1-(4-Cyclooctylcarbonylaminobenzoyl)-1,2,3,4-tetrahydroquinoline
17. 1-(4-Tricyclo[3.3.1.1]decanylcarbonylaminobenzoyl)-1,2,3,4-tetrahydroquinoline
18. 1-[4-(α-Naphthylcarbonylamino)benzoyl]-1,2,3,4-tetrahydroquinoline
20. 1-[2-(β-Naphthylcarbonylamino)benzoyl]-1,2,3,4-tetrahydroquinoline
21. 1-[4-(4-Methoxyanilinocarbonyl)benzoyl]-1,2,3,4-tetrahydroquinoline
22. 1-[4-(2-Methylanilinocarbonyl)benzoyl]-1,2,3,4-tetrahydroquinoline
23. 1-[4-(3-Chloroanilinocarbonyl)benzoyl]-1,2,3,4-tetrahydroquinoline
24. 1-[4-(3,5-Dichloroanilinocarbonyl)benzoyl]-1,2,3,4-tetrahydroquinoline
25. 1-(4-Cyclohexylaminocarbonylbenzoyl)-1,2,3,4-tetrahydroquinoline
26. 1-(4-Cyclohexylcarbonylaminobenzoyl)-2,3,4,5-tetrahydro-1H-benzazepine
27. 1-(4-Benzoylaminobenzoyl)-2,3,4,5-tetrahydro-1H-benzazepine
28. 1-[4-(2-Methylbenzoylamino)benzoyl]-2,3,4,5-tetrahydro-1H-benzazepine
29. 1-[4-(3-Methoxybenzoylamino)benzoyl]-2,3,4,5-tetrahydro-1H-benzazepine
30. 1-[4-(3-Chlorobenzoylamino)benzoyl]-2,3,4,5-tetrahydro-1H-benzazepine
31. 1-[4-(3-Cyanobenzoylamino)benzoyl]-2,3,4,5-tetrahydro-1H-benzazepine
32. 1-[4-(3,5-Dichlorobenzoylamino)benzoyl]-2,3,4,5-tetrahydro-1H-benzazepine
33. 1-[4-(2,3-Dimethylbenzoylamino)benzoyl]-2,3,4,5-tetrahydro-1H-benzazepine
34. 1-(4-Cyclohexylcarbonylaminobenzoyl)-1,2,3,4,5,6-hexahydrobenzazocine
35. 1-(4-Benzoylaminobenzoyl)-1,2,3,4,5,6-hexahydrobenzazocine
36. 1-[4-(2-Methylbenzoylamino)benzoyl]-1,2,3,4,5,6-hexahydrobenzazocine
37. 1-[4-(3-Methoxybenzoylamino)benzoyl]-1,2,3,4,5,6-hexahydrobenzazocine
38. 1-[4-(2,3-Dimethylbenzoylamino)benzoyl]-1,2,3,4,5,6-hexahydrobenzazocine
39. 1-[4-(3,5-Dichlorobenzoylamino)benzoyl]-1,2,3,4,5,6-hexahydrobenzazocine
40. 1-(4-Cyclohexylcarbonylaminobenzoyl)-1,2,3,5-tntrahydro-4,1-benzoxazepine

41. 1-[4-(3-Methylbenzoylamino)benzoyl]-1,2,3,5-tetrahydro-4,1-benzoxazepine
42. 1-[4-(2,3-Dimethylbenzoylamino)benzoyl]-1,2,3,5-tetrahydro-4,1-benzoxazepine
43. 1-[4-(3,5-Dichlorobenzoylamino)benzoyl]-1,2,3,5-tetrahydro-4,1-benzoxazepine
44. 3-Methyl-1-(4-cyclohexylcarbonylaminobenzoyl)-1,2,3,4-tetrahydroquinoline
45. 3-Methyl-1-(4-benzoylaminobenzoyl)-1,2,3,4-tetrahydroquinoline
46. 3-Methyl-1-[4-(2-methylbenzoylamino)benzoyl]-1,2,3,4-tetrahydroquinoline
47. 3-Methyl-1-[4-(3-methoxybenzoylamino)benzoyl]-1,2,3,4-tetrahydroquinoline
48. 3-Methyl-1-[4-(2,3-dimethylbenzoylamino)benzoyl]-1,2,3,4-tetrahydroquinoline
49. 3-Methyl-1-[4-(3,5-dichlorobenzoylamino)benzoyl]-1,2,3,4-tetrahydroquinoline
50. 4-Methyl-1-(4-cyclohexylcarbonylaminobenzoyl)-1,2,3,4-tetrahydroquinoxaline
51. 4-Methyl-1-[4-(2-methylbenzoylamino)benzoyl]-1,2,3,4-tetrahydroquinoxaline
52. 4-Methyl-1-[4-(2,3-dimethylbenzoylamino)benzoyl]-1,2,3,4-tetrahydroquinoxaline
53. 4-Methyl-1-[4-(3,5-dichlorobenzoylamino)benzoyl]-1,2,3,4-tetrahydroquinoxaline
54. 2-Methyl-1-[4-(3,5-dichlorobenzoylamino)benzoyl]-1,2,3,4-tetrahydroquinoline
55. 4-Methyl-1-[4-(3,5-dichlorobenzoylamino)benzoyl]-1,2,3,4-tetrahydroquinoline
56. 1-[4-(2-Bromobenzoylamino)benzoyl]-2,3,4,5-tetrahydro-1H-benzazepine
57. 1-[4-(3-Nitrobenzoylamino)benzoyl]-2,3,4,5-tetrahydro-1H-benzazepine
58. 1-[4-(3-Trifluoromethylbenzoylamino)benzoyl]-2,3,4,5-tetrahydro-1H-benzazepine
59. 1-[4-(3-Ethoxybenzoylamino)benzoyl]-2,3,4,5-tetrahydro-1H-benzazepine
60. 1-[4-(3,5-Dimethoxybenzoylamino)benzoyl]-2,3,4,5-tetrahydro-1H-benzazepine
61. 1-[4-(2-Chloro-4-nitrobenzoylamino)benzoyl]-2,3,4,5-tetrahydro-1H-benzazepine
62. 1-[4-(2,4-Dichlorobenzoylamino)benzoyl]-2,3,4,5-tetrahydro-1H-benzazepine
63. 1-[4-(2-Chloro-6-fluorobenzoylamino)benzoyl]-2,3,4,5-tetrahydro-1H-benzazepine
64. 1-[4-(2,6-Dimethylbenzoylamino)benzoyl]-2,3,4,5-tetrahydro-1H-benzazepine
65. 1-[4-(2-Chloro-4-aminobenzoylamino)benzoyl]-2,3,4,5-tetrahydro-1H-benzazepine
66. 1-[4-(2-Chloro-4-acetylaminobenzoylamino)benzoyl]-2,3,4,5-tetrahydro-1H-benzazepine
67. 1-[4-(3-Aminobenzoylamino)benzoyl]-2,3,4,5-tetrahydro-1H-benzazepine
68. 1-{4-[2-(4-Isopropylaminobutoxy)benzoylamino]benzoyl}-2,3,4,5-tetrahydro-1H-benzazepine hydrochloride
69. 1-[4-(3-Hydroxybenzoylamino)benzoyl]-2,3,4,5-tetrahydro-1H-benzazepine
70. 1-{4-[2-(4-Aminobutoxy)benzoylamino]benzoyl}-2,3,4,5-tetrahydro-1H-benzazepine
71. 1-{4-[2-(2-Diethylaminoethoxy)benzoylamino]benzoyl}-2,3,4,5-tetrahydro-1H-benzazepine hydrochloride
72. 1-{4-[2-(4-Acetylaminobutoxy)benzoylamino]benzoyl}-2,3,4,5-tetrahydro-1H-benzazepine
73. 1-{4-[2-(6-Phthalimidohexyloxy)benzoylamino]benzoyl}-2,3,4,5-tetrahydro-1H-benzazepine
74. 1-{4-[2-(6-Morpholinohexyloxy)benzoylamino]benzoyl}-2,3,4,5-tetrahydro-1H-benzazepine
75. 1-{4-[2-(6-[4-Methyl-1-piperazinyl]hexyloxy)benzoylamino]benzoyl}-2,3,4,5-tetrahydro-1H-benzazepinedihydrochloride
76. 1-(3-Methoxy-4-cyclohexylcarbonylaminobenzoyl)-2,3,4,5-tetrahydro-1H-benzazepine
77. 1-(3-methoxy-4-benzoylamino)benzoyl]-2,3,4,5-tetrahydro-1H-benzazepine
78. 1-[3-Methyl-4-(2-methylbenzoylamino)benzoy]-2,3,4,5-tetrahydro-1H-benzazepine
79. 4-Methyl-1-(4-cyclohexylcarbonylaminobenzoyl)-2,3,4,5-tetrahydro-1H-1,4-benzodiazepine hydrochloride
80. 4-Methyl-1-[4-(3,5-dichlorobenzoylamino)benzoyl]-2,3,4,5-tetrahydro-1H-1,4-benzodiazepine hydrochloride
81. 4-Methyl-1-[4-(2,3-dimethylbenzoylamino)benzoyl]-2,3,4,5-tetrahydro-1H-1,4-benzodiazepine hydrochloride
82. 4-Methyl-1-[4-(2-methylbenzoylamino)benzoyl]-2,3,4,5-tetrahydro-1H-1,4-benzodiazepine hydrochloride
83. 4-Methyl-1-[4-(3-methoxybenzoylamino)benzoyl]-2,3,4,5-tetrahydro-1H-1,4-benzodiazepine
84. 4-Methyl-1-[4-(3-chlorobenzoylamino)benzoyl]-2,3,4,5-tetrahydro-1H-1,4-benzodiazepine
85. 4-Methyl-1-[4-(2,3,5-trichlorobenzoylamino)benzoyl]-2,3,4,5-tetrahydro-1H-1,4-benzodiazepine
86. 4-propyl-1-[4-(2,3-dimethylbenzoylamino)benzoyl]-2,3,4,5-tetrahydro-1H-1,4-benzodiazepine hydrochloride
87. 5-Methyl-1-(4-benzoylaminobenzoyl)-2,3,4,5-tetrahydro-1H-1,5-benzodiazepine
88. 5-Methyl-1-(4-cyclohexylcarbonylaminobenzoyl) -2,3,4,5-tetrahydro-1H-1,5-benzodiazepine
89. 5-Methyl-1-[4-(3,5-dichlorobenzoylamino)benzoyl]-2,3,4,5-tetrahydro-1H-1,5-benzodiazepine
90. 5-Methyl-1-[4-(2-methylbenzoylamino)benzoyl] -2,3,4,5-tetrahydro-1H-1,5-benzodiazepine
91. 5-Methyl-1-[4-(2,3-dimethylbenzoylamino)benzoyl]-2,3,4,5-tetrahydro-1H-1,5-benzodiazepine
92. 4-Methyl-1-[3-methoxy-4-(3,5-dichlorobenzoylamino)benzoyl]-2,3,4,5-tetrahydro-1H-1,4-benzodiazepine
93. 3-(1-Pyrrolidinyl)-1-[4-(2,3-dimethylbenzoylamino)benzoyl]-1,2,3,4-tetrahydroquinoline
94. 6-Methyl-1-[4-(3,5-dichlorobenzoylamino)benzoyl]-1,2,3,4-tetrahydroquinoline
95. 6-Methoxy-1-[4-(3,5-dichlorobenzoylamino)benzoyl]-1,2,3,4-tetrahydroquinoline
96. 3-Hydroxymethyl-1-[4-(3,5-dichlorobenzoylamino)benzoyl]-1,2,3,4-tetrahydroquinoline
97. 4-Methylamino-1-[4-(3,5-dichlorobenzoylamino)benzoyl]-1,2,3,4-tetrahydroquinoline
98. 3-Amino-1-[4-(3,5-dichlorobenzoylamino)benzoyl]-1,2,3,4-tetrahydroquinoline

99. 3-Acetylamino-1-[4-(3,5-dichlorobenzoylamino)benzoyl]-1,2,3,4-tetrahydroquinoline
100. 4-Dimethylamino-1-[4-(3,5-dichlorobenzoylamino)benzoyl]-1,2,3,4-tetrahydroquinoline
101. 1-[4-(2-t-Butylaminoacetylamino)benzoyl]-2,3,4,5-tetrahydroquinoline-1H-benzazepine
103. 1-{4-[2-(1-Piperidinyl)acetylamino]benzoyl}-2,3,4,5-tetrahydro-1H-benzazepine
104. 1-[4-(2-Phenoxyacetylamino)benzoyl]-2,3,4,5-tetrahydro-1H-benzazepine
105. 1-[4-(2-Phthalimidoacetylamino)benzoyl]-2,3,4,5-tetranydro-1H-benzazepine
106. 1-{4-[2-(1,1-Dimethyl-2-phenoxyethyl)aminoacetylamino]benzoyl}-2,3,4,5-tetrahydro-1H-benzazepine
107. 1-{4-[2-(3-Methylphenoxy)acetylamino]benzoyl}-2,3,4,5-tetrahydro-1H-benzazepine
108. 1-{4-[2-(3-Methoxyanilino)acetylamino]benzoyl}-2,3,4,5-tetrahydro-1H-benzazepine
109. 1-{4-[2-(β-Naphthyloxy)acetyamino]benzoyl}-2,3,4,5-tetrahydro-1H-benzazepine
110. 1-{4-[2-(4-Methylanilino)acetylamino]benzoyl}-2,3,4,5-tetrahydro-1H-benzazepine
111. 1-{4-[2-(3-Methoxyphenoxy)acetylamino]benzoyl}-2,3,4,5-tetrahydro-1H-benzazepine
112. 1-[4-(4-Pyridylcarbonylaminobenzoyl]-2,3,4,5-tetrahydro-1H-benzazepine
113. 1-{4-[2-(2,4-Dimethylanilino)acetylamino]benzoyl}-2,3,4,5-tetrahydro-1H-benzazepine
114. 1-{4-[2-(N-Ethylanilino)acetylamino]benzoyl}-2,3,4,5-tetrahydro-1H-benzazepine
115. 1-{4-[2-(N-Allylanilino)acetylamino]benzoyl}-2,3,4,5-tetrahydro-1H-benzazepine
116. 1-{4-[2-(2-Chloroanilino)acetylamino]benzoyl}-2,3,4,5-tetrahydro-1H-benzazepine
117. 1-{4-[2-(4-Acetyloxybutoxy)benzoylamino]benzoyl}-2,3,4,5-tetrahydro-1H-benzazepine
118. 1-[4-(2-Carboxymethoxybenzoylamino)benzoyl]-2,3,4,5-tetrahydro-1H-benzazepine
119. 1-[4-(2-Carbamoylmethoxybenzoylamino)benzoyl]-2,3,4,5-tetrahydro-1H-benzazepine
120. 1-{4-[2-(4-Hydroxybutoxy)benzoylamino]benzoyl}-2,3,4,5-tetrahydro-1H-benzazepine
121. 1-[4-(2-Ethoxycarbonylmethoxybenzoylamino)benzoyl]-2,3,4,5-tetrahydro-1H-benzazepine
122. 6-Fluoro-1-[4-(3,5-dichlorobenzoylamino)benzoyl]-1,2,3,4-tetrahydroquinoline
123. 6-Fluoro-1-{4-[di-(3,5-dichlorobenzoyl)amino]benzoyl}-1,2,3,4-tetrahydroquinoline
124. 1-[4-(2-Diethylaminocarbonylmethoxybenzoylamino)benzoyl]-2,3,4,5-tetrahydro-1H-benzazepine
125. 1-{4-[2-(2-[(N-(2-hydroxyethyl)-N-methylamino]ethoxy)benzoylamino]benzoyl}-2,3,4,5-tetrahydro-1H-benzoazepine hydrochloride
126. 1-[4-(2-Methylanilinocarbonylamino)benzoyl]-2,3,4,5-tetrahydro-1H-benzazepine
127. 1-[4-(2-Chlorophenylsulfonylamino)benzoyl]-2,3,4,5-tetrahydro-1H-benzazepine
128. 1-{4-[2-(4-Aminomethylanilino)acetylamino]benzoyl}-2,3,4,5-tetrahydro-1H-benzazepine
129. 1-{4-[2-(N-Phenyl-N-(3-acetylaminopropyl)amino)acetylamino]benzoyl}-2,3,4,5-tetrahydro-1H-benzazepine
130. 1-{4-[2-(N-Phenyl-N-propargylamino)acetylamino]benzoyl}-2,3,4,5-tetrahydro-1H-benzazepine
131. 4-(N-Methyl-N-ethylamino)-1-[4-(3,5-dichlorobenzoylamino)benzoyl]-1,2,3,4-tetrahydroquinoline
132. 5-Dimethylamino-1-[4-(2,4-dichlorobenzoylamino)benzoyl]-2,3,4,5-tetrahydro-1H-benzazepine
133. 4-Dimethylamino-1-[3-methoxy-4-(2-methylbenzoylamino)benzoyl]-1,2,3,4-tetrahydroquinoline
134. 5-Dimethylamino-1-[3-methoxy-4-(2-chlorobenzoylamino)benzoyl]-2,3,4,5-tetrahydro-1H-benzazepine
135. 1-[4-(2,3-Dimethylbenzoylamino)benzoyl]-4-ethyl-2,3,4,5-tetrahydro-1H-1,4-benzodiazepine
136. 1-[4-(3,5-Dichlorobenzoylamino)benzoyl]-4-isopropyl-2,3,4,5-tetrahydro-1H-1,4-benzodiazepine
137. 1-[4-(2-Methylbenzoylamino)benzoyl]-5-methyl1,2,3,4,5,6-hexahydro-1,5-benzodiazocine
138. 1-[4-(2-Methylbenzoylamino)benzoyl]-1,2,3,4-tetrahydro-5,1-benzoxazepine
139. 5-Oxo-1-[4-(3,5-dichlorobenzoylamino)benzoyl]-2,3,4,5-tetrahydro-1H-benzazepine
140. 4-Methyl-1-[2-chloro-4-(3,5-dichlorobenzoylamino)benzoyl]-2,3,4,5-tetrahydro-1H-1,4-benzodiazepine
141. 5-Methylamino-1-[4-(3,5-dichlorobenzoylamino)benzoyl]-2,3,4,5-tetrahydro-1H-benzazepine
142. 5-(N-Acetyl-N-methylamino)-1-[4-(3,5-dichlorobenzoylamino)benzoyl]-2,3,4,5-tetrahydro-1H-benzazepine
143. 5-Hydroxy-1-[4-(2-methylbenzoylamino)benzoyl]-2,3,4,5-tetrahydro-1H-benzazepine
144. 4-Dimethylamino-1-[3-methoxy-4-(2,3-dimethylbenzoylamino)benzoyl]-1,2,3,4-tetrahydroquinoline
145. 4-Dimethylaminomethyl-1-[4-(2-methylbenzoylamino)benzoyl]-1,2,3,4-tetrahydroquinoline
146. 4-Dimethylaminomethyl-1-[4-(3,5-dichlorobenzoylamino)benzoyl]-1,2,3,4-tetrahydroquinoline
147. 5-Methoxy-1-[4-(2-methylbenzoylamino)benzoyl]-2,3,4,5-tetrahydro-1H-benzazepine
148. 4-Methyl-1-[3-methyl-4-(2,4-dichlorobenzoylamino)benzoyl]-2,3,4,5-tetrahydro-1H-1,4-benzodiazepine
149. 5-Methoxy-1-[4-(2,4-dichlorobenzoylamino)benzoyl]-2,3,4,5-tetrahydro-1H-benzazepine
150. 4-Dimethylamino-1-[4-(2-chlorobenzoylamino)benzoyl]-2,3,4,5-tetrahydro-1H-benzazepine
151. 4-Acetyloxy-1-[4-(2-methylbenzoylamino)benzoyl]-1,2,3,4-tetrahydroquinoline
152. 5-Hydroxyimino-1-[4-(3,5-dichlorobenzoylamino)benzoyl]-2,3,4,5-tetrahydro-1H-benzazepine
153. 5-Acetyloxy-1-[4-(2-chlorobenzoylamino)benzoyl]-2,3,4,5-tetrahydro-1H-benzazepine
154. 5-Ethoxycarbonylmethoxy-1-[4-(2,4-dichlorobenzoylamino)benzoyl]-2,3,4,5-tetrahydro-1H-benzazepine
155. 4-Allylamino-1-[4-(3,5-dichlorobenzoylamino)benzoyl]-2,3,4,5-tetrahydro-1H-benzazepine
156. 5-Dimethylamino-1-[3-methoxy-4-(2,3,5-trichlorobenzoylamino)benzoyl]-2,3,4,5-tetrahydro-1H-benzazepine
157. 4-[4-(2-Methylbenzoylamino)benzoyl]-3,4-dihydro-2H-1,4-benzothiazine

158. 5-Dimethylamino-1-[2-chloro-4-(2-methylbenzoylamino)benzoyl]-2,3,4,5-tetrahydro-1H-benzazepine
159. 5-Dimethylamino-1-[4-(2-methylanilinocarbonyl)benzoyl]-2,3,4,5-tetrahydro-1H-benzazepine
160. 5-Ethoxycarbonylmethoxy-1-[4-(2-methylbenzoylamino)benzoyl]-2,3,4,5-tetrahydro-1H-benzazepine
161. 5-(4-dimethylaminobutoxy)-1-[4-(2-methylbenzoylamino)benzoyl]-2,3,4,5-tetrahydro-1H-benzazepine
162. 5-Carboxymethoxy-1-[4-(2-chlorobenzoylamino)benzoyl]-2,3,4,5-tetrahydro-1H-benzazepine
163. 5-Dimethylaminocarbonylmethoxy-1-[4-(2-methylbenzoylamino)benzoyl]-2,3,4,5-tetrahydro-1H-benzazepine
164. 5-Carbamoylmethoxy-1-[4-(2-chlorobenzoylamino)benzoyl]-2,3,4,5-tetrahydro-1H-benzazepine
165. 5-Dimethylamino-1-[3-ethoxy-4-(2-methylbenzoylamino)benzoyl]-2,3,4,5-tetrahydro-1H-benzazepine
166. 5-[4-(2-Methylbenzoylamino)benzoyl]-2,3,4,5-tetrahydro-1,5-benzothiazepine
167. 5-Amino-1-[4-(2-methylbenzoylamino)benzoyl]-2,3,4,5-tetrahydro-1H-benzazepine
168. 5-Dimethylamino-1-[3-hydroxy-4-[2-methylbenzoylamino)benzoyl]-2,3,4,5-tetrahydro-1H-benzazepine
169. 5-n-Propylamino-1-[4-(2,4-dichlorobenzoylamino)benzoyl]-2,3,4,5-tetrahydro-1H-benzazepine
170. 5-Dimethylamino-1-[3-benzyloxy-4-(2-methylbenzoylamino)benzoyl]-2,3,4,5-tetrahydro-1H-benzazepine
171. 5-[4-(2-Methylbenzoylamino)benzoyl]-2,3,4,5-tetrahydro-1,5-benzothiazepin-1-oxide
172. 5-[3-(Phthalimid-1-yl)-propoxy]-1-[4-(2-methylbenzoylamino)benzoyl]-2,3,4,5-tetrahydro-1H-benzazepine
173. 5-(3-Aminopropoxy)-1-[4-(2-methylbenzoylamino)benzoyl]-2,3,4,5-tetrahydro-1H-benzazepine
174. 5-(3-Acetylaminopropoxy)-1-[4-(2-methylbenzoylamino)benzoyl]-2,3,4,5-tetrahydro-1H-benzazepine
175. 5-Dimethylamino-1-[2-chloro-4-(2-t-butylbenzoylamino)benzoyl]-2,3,4,5-tetrahydro-1H-benzazepine
176. 5-Methylamino-1-[2-chloro-4-(2-chlorobenzoylamino)benzoyl]-2,3,4,5-tetrahydro-1H-benzazepine
177. 5-Dimethylamino-1-[2-methoxy-4-(2-chlorobenzoylamino)benzoyl]-2,3,4,5-tetrahydro-1H-benzazepine
178. 5-Hydroxy-1-[4-(3,5-dichlorobenzoylamino)benzoyl]-2,3,4,5-tetrahydro-1H-benzazepine
179. 5-Dimethylamino-1-[4-(2-methylbenzoylamino)benzoyl]-2,3,4,5-tetrahydro-1H-benzazepine
180. 5-Dimethylamino-1-[4-(2-chlorobenzoylamino)benzoyl]-2,3,4,5-tetrahydro-1H-benzazepine
181. 5-Methylamino-1-[4-(2-chlorobenzoylamino)benzoyl]-2,3,4,5-tetrahydro-1H-benzazepine
182. 5-Methylamino-1-[2-chloro-4-(2-methylbenzoylamino)benzoyl]-2,3,4,5-tetrahydro-1H-benzazepine
183. 5-Dimethylamino-1-[2-methoxy-4-(2-methylbenzoylamino)benzoyl]-2,3,4,5-tetrahydro-1H-benzazepine
184. 5-Dimethylamino-1-[2-chloro-4-(2-chlorobenzoylamino)benzoyl]-2,3,4,5-tetrahydro-1H-benzazepine
185. 5-Methylamino-1-[2-chloro-4-(2-methylbenzoylamino)benzoyl-2,3,4,5-tetrahydro-1H-benzazepine
186. 5-Cyclopropylamino-1-[2-chloro-4-(2,4-dichlorobenzoylamino)benzoyl]-2,3,4,5-tetrahydro-1H-benzazepine
187. 5-Dimethylaminocarbonyloxy-1-[4-(2-methylbenzoylamino)benzoyl]-2,3,4,5-tetrahydro-1H-benzazepine
188. 5-Dimethylamino-1-[4-(2-trifluoromethylbenzoylamino)benzoyl]-2,3,4,5-tetrahydro-1H-benzazepine
189. 5-Dimethylamino-1-[3-(2-chlorobenzoyloxy)-4-(2-chlorobenzoylamino)benzoyl]-2,3,4,5-tetrahydro-1H-benzazepine
190. 5-(N-Methyl-N-Allylamino)-1-[2-chloro-4-(2-methylbenzoylamino)benzoyl]-2,3,4,5-tetrahydro-1H-benzazepine
191. 5-Carbamoyloxy-1-[4-(2-methylbenzoylamino)benzoyl]-2,3,4,5-tetrahydro-1H-benzazepine
192. 1-[4-(2-Methylbenzoylamino)benzoyl]-1,2,3,5-tetrahydro-4,1-benzothiazepine
193. 4-Oxo-1-[4-(2-methylbenzoylamino)benzoyl]-2,3,4,5-tetrahydro-1H-benzazepine
194. 1-[4-(2-Methylbenzoylamino)benzoyl]-1,2,3,5-tetrahydro-4,1-benzothiazepine-1,1-dioxide
195. 5-Methylaminocarbonylmethoxy-1-[4-(2-chlorobenzoylamino)benzoyl]-2,3,4,5-tetrahydro-1H-benzazepine
196. 5-Methylaminocarbonyloxy-1-[4-(2-methylbenzoylamino)benzoyl]-2,3,4,5-tetrahyro-1H-benzazepine
197. 5-Dimethylamino-1-[2-dimethylamino-4-(2-methylbenzoylamino)benzoyl]-2,3,4,5-tetrahydro-1H-benzazepine
198. 5-Methylamino-1-[2-chloro-4-(2-trifluoromethylbenzoylamino)benzoyl]-2,3,4,5-tetrahydro-1H- benzazepine
199. 5-Cycloropropylamino-1-[2-chloro-4-(2-methylbenzoylamino)benzoyl]-2,3,4,5-tetrahydro-1H-benzazepine
200. 5-Cyclopropylamino-1-[2-chloro-4-(2-chlorobenzoylamino)benzoyl]-2,3,4,5-tetrahydro-1H-benzazepine
201. 5-Allylamino-1-[2-chloro-4-(2-methylbenzoylamino)benzoyl]-2,3,4,5-tetrahydro-1H-benzazepine
202. 5-(1-Piperidinyl)-1-[4-(2-chlorobenzoylamino)benzoyl]-2,3,4,5-tetrahydro-1H-benzazepine
203. 5-(4-Benzyl-1-piperazinyl)-1-[4-(2-chlorobenzoylamino)benzoyl]-2,3,4,5-tetrahydro-1H-benzazepine
204. 5-(1-Pyrrolidinyl)-1-[4-(2-chlorobenzoylamino)benzoyl]-2,3,4,5-tetrahydro-1H-benzazepine
205. 5-(4-Acetyl-1-piperazinyl)-1-[4-(2-chlorobenzoylamino)benzoyl]-2,3,4,5-tetrahydro-1H-benzazepine
206. 5-(4-Methyl-1-piperazinyl)-1-[4-(2-chlorobenzoylamino)benzoyl]-2,3,4,5-tetrahydro-1H-benzazepine
207. 1-[4-(2-Chlorobenzoylamino)benzoyl]-2,3-dihydro-1H-benzazepine
208. 5-Methyl-1-[4-(2-methylbenzoylamino)benzoyl]-2,3,4,5-tetrahydro-1H-benzazepine
209. 5-Methylidene-1-[4-(2-methylbenzoylamino)benzoyl]-2,3,4,5-tetrahydro-1H-benzazepine
210. 5-Hydroxy-1-[2-chloro-4-(2-methylbenzoylamino)benzoyl]-2,3,4,5-tetrahydro-1H-benzazepine
211. 5-(1-Morpholino)-1-[4-(2-chlorobenzoylamino)benzoyl]-2,3,4,5-tetrahydro-1H-benzazepine
212. 5-Dimethylamino-1-[4-(2-fluorobenzoylamino)benzoyl]-2,3,4,5-tetrahydro-1H-benzazepine
213. 5-Dimethylamino-1-[4-(2,4-difluorobenzoylamino)benzoyl]-2,3,4,5-tetrahydro-1H-benzazepine
214. 4-Hydroxy-1-[4-(2-methylbenzoylamino)benzoyl]-2,3,4,5-tetrahydro-1H-benzazepine
215. 5-Hydroxymethyl-1-[4-(2-methylbenzoylamino)benzoyl]-2,3,4,5-tetrahydro-1H-benzazepine

216. 5-Dimethylamino-4-hydroxy-1-[4-(2-chlorobenzoylamino)benzoyl]-2,3,4,5-tetrahydro-1H-benzazepine
217. 1-[4-(2-Methylbenzoylamino)benzoyl]-1,2-dihydroquinoline
218. 5-Dimethylamino-1-[2-methyl-4-(2-methylbenzoylamino)benzoyl]-2,3,4,5-tetrahydro-1H-benzazepine (the compound of Example 979)
219. 5-Dimethylamino-1-[2-methyl-4-(2-chlorobenzoylamino)benzoyl]-2,3,4,5-tetrahydro-1H-benzazepine
220. 5-Dimethylamino-1-[2-methyl-4-(2,4-dichlorobenzoylamino)benzoyl]-2,3,4,5-tetrahydro-1H-benzazepine
221. 5-Methylamino-1-{2-chloro-4-[2-(N-ethylanilino)acetylamino]benzoyl}-2,3,4,5-tetrahydro-1H-benzazepine
222. 5-Hydroxy-1-{2-chloro-4-[2-(N-ethylanilino)acetylamino]benzoyl}-2,3,4,5-tetrahydro-1H-benzazepine
223. 5-Dimethylamino-1-[2-fluoro-4-(2-chlorobenzoylamino)benzoyl]-2,3,4,5-tetrahydro-1H-benzazepine
224. 5-Methylamino-4-hydroxy-1-[2-chloro-4-(2-chlorobenzoylamino)benzoyl]-2,3,4,5-tetrahydro-1H-benzazepine · hydrochloride
225. 5-Hydroxymethyl-5-hydroxy-1-[2-chloro-4-(2-methylbenzoylamino)benzoyl]-2,3,4,5-tetrahydro-1H-benzazepine
226. 5-Dimethylamino-1-[2-fluoro-4-(2-methylbenzoylamino)benzoyl]-2,3,4,5-tetrahydro-1H-benzazepine
227. 5-Dimethylamino-1-[3-methyl-4-(2,4-dichlorobenzoylamino)benzoyl}-2,3,4,5-tetrahydro-1H-benzazepine
228. 5-(N-Methyl-N-ethylamino)-1-[4-(2-methylbenzoylamino)benzoyl}-2,3,4,5-tetrahydro-1H-benzazepine
229. 5-Ethylamino-1-[4-(2-methylbenzoylamino)benzoyl]-2,3,4,5-tetrahydro-1H-benzazepine
230. 5-Dimethylamino-1-[4-(3,5-difluorobenzoylamino)benzoyl]-2,3,4,5-tetrahydro-1H-benzazepine
231. 5-Acetyloxymethyl-1-[4-(2-methylbenzoylamino)benzoyl]-2,3,4,5-tetrahydro-1H-benzazepine
232. 5-Dimethylamino-1-[3-fluoro-4-(2-methylbenzoylamino)benzoyl]-2,3,4,5-tetrahydro-1H-benzazepine
233. 4,4-Dimethoxy-1-[4-(2-methylbenzoylamino)benzoyl]-2,3,4,5-tetrahydro-1H-benzazepine
234. 5-Acetyloxyimino-1-[4-(2-chlorobenzoylamino)benzoyl]-2,3,4,5-tetrahydro-1H-benzazepine
235. 5-Methylsulfonyloxymethyl-1-[4-(2-methylbenzoylamino)benzoyl]-2,3,4,5-tetrahydro-1H-benzazepine
236. 5,5-Epoxy-1-[4-(2-methylbenzoylamino)benzoyl]-2,3,4,5-tetrahydro-1H-benzazepine
237. 5-Hydroxymethyl-5-hydroxy-1-[4-(2-methylbenzoylamino)benzoyl]-2,3,4,5-tetrahydro-1H-benzazepine
238. 5-Hydroxy-1-[2-methoxy-4-(2-methylbenzoylamino)benzoyl]-2,3,4,5-tetrahydro-1H-benzazepine
239. 5-Dimethylamino-1-[4-(2-carbamoylmethoxybenzoylamino)benzoyl]-2,3,4,5-tetrahydro-1H-benzazepine
240. 5-Hydroxy-6-methyl-1-[2-chloro-4-(2-methylbenzoylamino)benzoyl]-2,3,4,5-tetrahydro-1H-benzazepine
241. 5-(2-Dimethylaminoethyl)amino-1-[2-chloro-4-(2-methylbenzoylamino)benzoyl)-2,3,4,5-tetrahydro-1H-benzazepine
242. 5-Hydroxymethyl-5-methylamino-1-[4-(2-methylbenzoylamino)benzoyl]-2,3,4,5-tetrahydro-1H-benzazepine
243. 5-Methylaminomethyl-5-hydroxy-1-[4-(2-methylbenzoylamino)benzoyl]-2,3,4,5-tetrahydro-1H-benzazepine
244. 5-Aminomethyl-1-[4-(2-methylbenzoylamino)benzoyl]-2,3,4,5-tetrahydro-1H-benzazepine
245. 5-[N-Methyl-N-(3-methoxy-2-hydroxypropyl)amino]-1-[4-(2-methylbenzoylamino)benzoyl]-2,3,4,5-tetrahydro-1H-benzazepine
246. 5-[N-Methyl-N-(3-diethylamino-2-hydroxypropyl)amino]-1-[4-(2-methylbenzoylamino)benzoyl]-2,3,4,5-tetrahydro-1H-benzazepine
247. 5-Dimethylamino-1-[3-methoxy-4-(2-methylbenzoylamino)benzoyl]-2,3,4,5-tetrahydro-1H-benzazepine
248. 5-Dimethylamino-1-[3-methoxy-4-(2,4-dichlorobenzoylamino)benzoyl]-2,3,4,5-tetrahydro-1H-benzazepine
249. 5-Dimethylamino-1-[4-(2-methylbenzoylamino)benzoyl]-2,3,4,5-tetrahydro-1H-benzazepine · hydrochloride
250. 5-Azidomethyl-1-[4-(2-methylbenzoylamino)benzoyl]-2,3,4,5-tetrahydro-1H-benzazepine
251. 7-[4-(2-Chlorobenzoylamino)benzoyl]-1-methyl-1,2,3,4a,5,6,7,11b-octahydro-3-oxo[1]benzazepino[4,5-b][1,4]oxazine
252. 5-Benzylamino-1-[4-(2-chlorobenzoylamino)benzoyl]-2,3,4,5-tetrahydro-1H-benzazepine
253. 5-Amino-1-[4-(2-chlorobenzoylamino)benzoyl]-2,3,4,5-tetrahydro-1H-benzazepine
254. 5-Dimethylamino-4-methyl-1-[4-(2-methylbenzoylamino)benzoyl]-2,3,4,5-tetrahydro-1H-benzazepine
255. 5-Acetylaminomethyl-1-[4-(2-methylbenzoylamino)benzoyl]-2,3,4,5-tetrahydro-1H-benzazepine
256. 5-Hydroxy-4-methyl-1-[4-(2-methylbenzoylamino)benzoyl]-2,3,4,5-tetrahydro-1H-benzazepine
257. 5-[2-(2-Pyridyl)ethylamino]-1-[4-(2-chlorobenzoylamino)benzoyl]-2,3,4,5-tetrahydro-1H-benzazepine
258. 5-(N-Methyl-N-methanesulfonylamino)-1-[4-(2-methylbenzoylamino)benzoyl]-2,3,4,5-tetrahydro-1H- benzazepine
259. 5-(N-Methyl-N-benozylamino)-1-[4-(2-methylbenzoylamino)benzoyl]-2,3,4,5-tetrahydro-1H-benzazepine
260. 5-Ethoxycarbonylamino-1-[4-(2-methylbenzoylamino)benzoyl]-2,3,4,5-tetrahydro-1H-benzazepine
261. 5-Methyl-5-hydroxy-1-[4-(2-methylbenzoylamino)benzoyl]-2,3,4,5-tetrahydro-1H-benzazepine
262. 5-(N-Methyl-N-ethoxycarbonylmethylamino)-1-[4-(2-methylbenzoylamino)benzoyl]-2,3,4,5-tetrahydro-1H-benzazepine
263. 5-Cyclopentylamino-1-[2-chloro-4-(2-methylbenzoylamino)benzoyl]-2,3,4,5-tetrahydro-1H-benzazepine
264. 5-[N-Methyl-N-(2,3-dihydroxypropyl)amino]-1-[4-(2-methylbenzoylamino)benzoyl]-2,3,4,5-tetrahydro-1H-benzazepine

265. 5-(N-Methyl-N-cyanomethylamino)-1-[4-(2-methylbenzoylamino)benzoyl]-2,3,4,5-tetrahydro-1H-benzazepine
266. 5-(N-Methyl-N-carbamoylmethylamino)-1-[4-(2-methylbenzoylamino)benzoyl]-2,3,4,5-tetrahydro-1H-benzazepine
267. 5-{N-Methyl-N-[3-(3,4,5,6-tetrahydro-2H-pyran-2-yloxy)propyl]amino}-1-[4-(2-methylbenzoylamino)benzoyl]-2,3,4,5-tetrahydro-1H-benzazepine
268. 5-Dimethylaminomethyl-1-[4-(2-methylbenzoylamino)benzoyl]-2,3,4,5-tetrahydro-1H-benzazepine
269. 5-Formylaminomethyl-1-[4-(2-methylbenzoylamino)benzoyl]-2,3,4,5-tetrahydro-1H-benzazepine
270. 5-[N-Methyl-N-(3-acetyloxypropyl)amino]-1-[4-(2-methylbenzoylamino)benzoyl]-2,3,4,5-tetrahydro-1H-benzazepine
271. 5-[N-Methyl-N-(3-hydroxypropyl)amino]-1-[4-(2-methylbenzoylamino)benzoyl]-2,3,4,5-tetrahydro-1H-benzazepine
272. Potassium {1-[2-chloro-4-(2-methylbenzoylamino)benzoyl]-2,3,4,5-tetrahydro-1H-benzazepin-5-yl}imino-o-sulfonate
273. 5-Dimethylamino-1-(4-benzoylaminobenzoyl)-2,3,4,5-tetrahydro-1H-benzazepine
274. 5-(1-Benzyl-4-piperidinyl)amino-1-[4-(2-methylbenzoylamino)benzoyl]-2,3,4,5-tetrahydro-1H-benzazepine
275. 5-(2-Dimethylaminoacetyloxy)-1-[4-(2-methylbenzoylamino)benzoyl]-2,3,4,5-tetrahydro-1H-benzazepine
276. 5-Dimethylamino-1-[4-(3-methoxybenzoylamino)benzoyl]-2,3,4,5-tetrahydro-1H-benzazepine
277. 5-[(4-Methyl-1-piperazinyl)carbonylmethoxy]-1-[4-(2-methylbenzoylamino)benzoyl]-2,3,4,5-tetrahydro-1H-benzazepine
278. 5-Morpholinocarbonylmethoxy-1-[4-(2-methylbenzoylamino)benzoyl]-2,3,4,5-tetrahydro-1H-benzazepine
279. 5-Thiomorpholinocarbonylmethoxy-1-[4-(2-methylbenzoylamino)benzoyl]-2,3,4,5-tetrahydro-1H- benzazepine
280. 5-Anilinocarbonylamino-1-[4-(2-methylbenzoylamino)benzoyl]-2,3,4,5-tetrahydro-1H-benzazepine
281. 5-(1-Oxothiomorpholino)carbonylmethoxy-1-[4-(2-methylbenzoylamino)benzoyl]-2,3,4,5-tetrahydro-1H-benzazepine
282. 5-Hydrazino-1-[4-(2-methylbenzoylamino)benzoyl]-2,3,4,5-tetrahydro-1H-benzazepine
283. 5-Methylaminocarbonylamino-1-[4-(2-methylbenzoylamino)benzoyl]-2,3,4,5-tetrahydro-1H-benzazepine
284. 5-[(2-α-Carbamoyl-1-pyrrolidinyl)carbonylmethoxy]-1-[4-(2-methylbenzoylamino)benzoyl]-2,3,4,5-tetrahydro-1H-benzazepine
285. 5-(Carbamoylmethylaminocarbonylmethoxy)-1-[4-(2-methylbenzoylamino)benzoyl]-2,3,4,5-tetrahydro-1H-benzazepine
286. 5-(1,1-Dioxothiomorpholino)carbonylmethoxy-1-[4-(2-methylbenzoylamino)benzoyl]-2,3,4,5-tetrahydro-1H-benzazepine
287. 7-Chloro-5-methylamino-1-[4-(2-methylbenzoylamino)benzoyl]-2,3,4,5-tetrahydro-1H-benzazepine
288. 5-[(4-Acetyl-1-piperazinyl)carbonylmethoxy]-1-[4-(2-methylbenzoylamino)benzoyl]-2,3,4,5-tetrahydro-1H-benzazepine
289. 5-Dimethylamino-1-[4-(3-nitrobenzoylamino)benzoyl]-2,3,4,5-tetrahydro-1H-benzazepine
290. 5-[(4-Pyridyl)methylaminocarbonylmethoxy]-1-[4-(2-methylbenzoylamino)benzoyl]-2,3,4,5-tetrahydro-1H-benzazepine
291. 5-[2-(Methylamino)acetylamino]-1-[4-(2-methylbenzoylamino)benzoyl]-2,3,4,5-tetrahydro-1H-benzazepine
292. 5-Dimethylamino-1-[4-(3-aminobenzoylamino)benzoyl]-2,3,4,5-tetrahydro-1H-benzazepine
293. 5-{[N-Methyl-N-(2-hydroxyethyl)amino]carbonylmethoxy}-1-[4-(2-methylbenzoylamino)benzoyl]-2,3,4,5-tetrahydro-1H-benzazepine
294. 5-Dimethylamino-1-[3-(2-diethylaminoethoxy)-4-(2-methylbenzoylamino)benzoyl]-2,3,4,5-tetrahydro-1H-benzazepine
295. 5-[N-Methyl-N-(dimethylaminocarbonylmethyl)amino]-1-[4-(2-methylbenzoylamino)benzoyl]-2,3,4,5-tetrahydro-1H-benzazepine
296. Potassium 2-[N-methyl-N-{1-[4-(2-methylbenzoylamino)benzoyl]-2,3,4,5-tetrahydro-1H-benzazepin-5-yl}amine]acetate
297. 5-{N-Methyl-N-[2-(1-imidazolyl)acetyl]amino}-1-[4-(2-methylbenzoylamino)benzoyl]-2,3,4,5-tetrahydro-1H-benzazepine
298. 5-Dimethylamino-1-[4-(2-dimethylaminobenzoylamino)benzoyl]-2,3,4,5-tetrahydro-1H-benzazepine
299. 5-[(2-Aminoacetyl)amino]-1-[4-(2-methylbenzoylamino)benzoyl]-2,3,4,5-tetrahydro-1H-benzazepine
300. 5-Dimethylamino-1-[4-(3-acetylaminobenzoylamino)benzoyl]-2,3,4,5-tetrahydro-1H-benzazepine
301. 5-(2-t-Butoxycarbonylaminoacetylamino)-1-[4-(2-methylbenzoylamino)benzoyl]-2,3,4,5-tetrahydro-1H-benzazepine
302. 5-Methylamino-7-chloro-1-[4-(2-chlorobenzoylamino)benzoyl]-2,3,4,5-tetrahydro-1H-benzazepine
303. 5-Dimethylamino-7-chloro-1-[4-(2-methylbenzoylamino)benzoyl]-2,3,4,5-tetrahydro-1H-benzazepine
304. 5-Dimethylamino-7-chloro-1-[4-(2-chlorobenzoylamino)benzoyl]-2,3,4,5-tetrahydro-1H-benzazepine
305. 5-Dimethylamino-1-[4-(phenylacetylamino)benzoyl]-2,3,4,5-tetrahydro-1H-benzazepine

306. 5-Dimethylamino-1-[4-(3-phenylpropionylamino)benzoyl]-2,3,4,5-tetrahydro-1H-benzazepine
307. 5-Methylamino-7-chloro-1-{4-[(N-ethylanilino)acetylamino]benzoyl}-2,3,4,5-tetrahydro-1H-benzazepine
308. 5-Dimethylamino-7-chloro-1-{4-[(N-ethylanilino)acetylamino]benzoyl}-2,3,4,5-tetrahydro-1H-benzazepine
309. 5-Dimethylamino-1-[4-(2-bromobenzoylamino)benzoyl]-2,3,4,5-tetrahydro-1H-benzazepine
310. 5-Cyclopropylamino-7-chloro-1-[4-(2-methylbenzoylamino)benzoyl]-2,3,4,5-tetrahydro-1H-benzazepine
311. 5-Cyclopropylamino-7-chloro-1-[4-(2-chlorobenzoylamino)benzoyl]-2,3,4,5-tetrahydro-1H-benzazepine
312. 5-hydroxy-1-{4-[2-(4-isopropylaminobutoxy)benzoylamino]benzoyl}-2,3,4,5-tetrahydro-1H-benzazepine
313. 5-Dimethylaminocarbonylmethoxy-1-{4-[(N-ethylanilino)acetylamino]benzoyl}-2,3,4,5-tetrahydro-1H-benzazepine
314. 5-(N-Methyl-N-ethylamino)-1-[2-chloro-4-(2-chlorobenzoylamino)benzoyl]-2,3,4,5-tetrahydro-1H-benzazepine
315. 5-Dimethylamino-1-{4-[(2-chloroanilino)acetylamino]benzoyl}-2,3,4,5-tetrahydro-1H-benzazepine
316. 5-Dimethylamino-1-{4-[(2-methylanilino)acetylamino]benzoyl}-2,3,4,5-tetrahydro-1H-benzazepine
317. 5-Dimethylamino-1-{4-[(N-methyl-2-methylanilino)acetylamino]benzoyl}-2,3,4,5-tetrahydro-1H-benzazepine
318. 5-Methylamino-9-chloro-1-[4-(2-methylbenzoylamino)benzoyl]-2,3,4,5-tetrahydro-1H-benzazepine
319. 5-Dimethylamino-1-[4-(phenoxyacetylamino)benzoyl]-2,3,4,5-tetrahydro-1H-benzazepine
320. 6-Methylamino-1-[4-(2-methylbenzoylamino)benzoyl]-1,2,3,4,5,6-hexahydrobenzazocine
321. 5-Methylamino-7-chloro-1-[3-methoxy-4-(2-chlorobenzoylamino)benzoyl]-2,3,4,5-tetrahydro-1H-benzazepine
322. 5-Cyclopropylamino-7-chloro-1-[3-methoxy-4-(2-chlorobenzoylamino)benzoyl]-2,3,4,5-tetrahydro-1H-benzazepine
323. 5-Methylamino-7-chloro-1-[3-methoxy-4-(2-methylbenzoylamino)benzoyl]-2,3,4,5-tetrahydro-1H-benzazepine

Table 10

| Test Comp. No. | $IC_{50}$ (μM) | Test Comp. No. | $IC_{50}$ (μM) |
|---|---|---|---|
| 26 | 0.071 | 78 | 0.10 |
| 27 | 0.095 | 88 | 0.34 |
| 28 | 0.056 | 90 | 0.38 |
| 29 | 0.15 | 114 | 0.011 |
| 30 | 0.15 | 115 | 0.012 |
| 32 | 0.30 | 116 | 0.04 |
| 33 | 0.092 | 117 | 0.22 |
| 36 | 0.41 | 119 | 0.049 |
| 46 | 0.40 | 120 | 0.29 |
| 56 | 0.025 | 121 | 0.45 |
| 57 | 0.46 | 124 | 0.15 |
| 58 | 0.40 | 125 | 0.091 |
| 59 | 0.31 | 130 | 0.023 |
| 60 | 0.18 | 143 | 0.15 |
| 62 | 0.098 | 147 | 0.28 |
| 63 | 0.14 | 161 | 0.14 |
| 64 | 0.069 | 163 | 0.22 |
| 67 | 0.34 | 164 | 0.15 |
| 68 | 0.013 | 172 | 0.26 |
| 69 | 0.066 | 173 | 0.15 |
| 70 | 0.041 | 174 | 0.14 |
| 71 | 0.18 | 187 | 0.45 |
| 72 | 0.12 | 188 | 0.47 |
| 74 | 0.10 | 192 | 0.054 |
| 75 | 0.069 | 193 | 0.17 |
| 76 | 0.042 | 195 | 0.17 |
| 77 | 0.085 | 196 | 0.40 |

| Test Comp. No. | $IC_{50}$ (μM) | Test Comp. No. | $IC_{50}$ (μM) |
|---|---|---|---|
| 207 | 0.16 | 284 | 0.29 |
| 208 | 0.11 | 285 | 0.18 |
| 209 | 0.074 | 286 | 0.40 |
| 214 | 0.27 | 287 | 0.064 |
| 215 | 0.13 | 288 | 0.26 |
| 222 | 0.096 | 290 | 0.21 |
| 231 | 0.16 | 293 | 0.19 |
| 235 | 0.088 | 298 | 0.29 |
| 236 | 0.16 | 302 | 0.071 |
| 238 | 0.39 | 303 | 0.19 |
| 244 | 0.23 | 304 | 0.21 |
| 250 | 0.19 | 307 | 0.024 |
| 252 | 0.36 | 308 | 0.11 |
| 255 | 0.046 | 309 | 0.43 |
| 256 | 0.049 | 310 | 0.065 |
| 266 | 0.29 | 311 | 0.078 |
| 269 | 0.48 | 312 | 0.056 |
| 274 | 0.11 | 313 | 0.032 |
| 275 | 0.18 | 315 | 0.38 |
| 277 | 0.23 | 316 | 0.47 |
| 278 | 0.30 | 321 | 0.059 |
| 279 | 0.15 | 322 | 0.044 |
| 280 | 0.47 | 323 | 0.064 |
| 281 | 0.18 | | |

Pharmacological Test

Experiment 2 : $V_2$ receptor binding assay

Using rat kidney plasma membrane preparations prepared according to O. Hechter's method [cf: J. Bio. Chem., 253, 3211 (1978)], the plasma membrane (100000dpm, $4x10^{-10}$ M) of [$^3$H]-Arg-vasopressin and a test compound (0.6 mg, $10^{-10}$ - $10^{-5}$ M) are incubated at 4°C for 3 hours in 100 mM Tris-HCl buffer (pH: 8.0, 250 μl) containing 5 mM $MgCl_2$, 1 mM EDTA and 0.1 % BSA. After incubation, the mixture is filtered using the glass filter (GF/F) so as to separate the membrane preparation combined with vasopressin and then washed twice with the buffer (5 ml). This glass filter is taken out and mixed with liquid scintillation cocktail. The amount of [$^3$H]-vasopressin combined with the membrane is measured

by liquid scintillation counter and the rate of the inhibitory effect of the test compound is estimated according to the following equation.

$$\text{Rate of the inhibitory effect (\%)} = 100 - \frac{C_1 - B_1}{C_1 - B_1} \times 100$$

$C^1$: The amount of [$^3$H]-vasopressin combined with the membrane in the presence of the test compound (in prescribed amount).

$C^0$: The amount of [$^3$H)-vasopressin combined with the membrane in the absence of the test compound.

$B^1$: The amount of [$^3$H]-vasopressin combined with the membrane in the presence of the excess amount of vasopressin ($10^{-6}$ M).

The results are expressed as $IC_{50}$ values, which is the concentration of the test compound required to achieve the inhibitory effect in the rate of 50 %.

The results are shown in the following Table 6.

Table 11

| Test Comp. No. | $IC_{50}$ (μM) | Test Comp. No. | $IC_{50}$ (μM) |
|---|---|---|---|
| 1 | 0.98 | 28 | 0.018 |
| 2 | 0.20 | 29 | 0.069 |
| 3 | 0.40 | 30 | 0.029 |
| 4 | 0.58 | 31 | 0.098 |
| 5 | 1.2 | 32 | 0.016 |
| 6 | 0.076 | 33 | 0.007 |
| 7 | 0.20 | 34 | 0.049 |
| 8 | 0.32 | 35 | 0.20 |
| 9 | 0.53 | 36 | 0.028 |
| 10 | 0.082 | 37 | 0.16 |
| 11 | 1.05 | 38 | 0.029 |
| 12 | 1.97 | 39 | 0.071 |
| 13 | 1.02 | 40 | 0.33 |
| 14 | 0.23 | 41 | 0.20 |
| 15 | 0.13 | 42 | 0.063 |
| 16 | 0.17 | 43 | 0.17 |
| 17 | 0.23 | 44 | 0.050 |
| 18 | 1.0 | 45 | 0.19 |
| 19 | 1.7 | 46 | 0.018 |
| 20 | 1.4 | 47 | 0.20 |
| 21 | 1 | 48 | 0.021 |
| 22 | 0.33 | 49 | 0.063 |
| 23 | 1.07 | 50 | 1.3 |
| 24 | 1.09 | 51 | 0.40 |
| 25 | 1.67 | 52 | 0.32 |
| 26 | 0.025 | 53 | 1.6 |
| 27 | 0.070 | 54 | 0.11 |

| Test Comp. No. | $IC_{50}$ (μM) | Test Comp. No. | $IC_{50}$ (μM) |
|---|---|---|---|
| 55 | 0.091 | 86 | 0.58 |
| 56 | 0.037 | 87 | 0.046 |
| 57 | 0.16 | 88 | 0.021 |
| 58 | 0.14 | 89 | 0.035 |
| 59 | 0.24 | 90 | 0.014 |
| 60 | 0.15 | 91 | 0.005 |
| 61 | 0.090 | 92 | 0.41 |
| 62 | 0.023 | 93 | 0.52 |
| 63 | 0.046 | 94 | 0.095 |
| 64 | 0.007 | 95 | 0.089 |
| 65 | 0.081 | 96 | 0.039 |
| 66 | 0.45 | 97 | 0.024 |
| 67 | 0.050 | 98 | 0.45 |
| 68 | 0.19 | 99 | 1.6 |
| 69 | 0.12 | 100 | 0.011 |
| 70 | 0.012 | 101 | 0.60 |
| 71 | 0.085 | 102 | 0.29 |
| 72 | 0.16 | 103 | 0.54 |
| 74 | 0.51 | 104 | 0.37 |
| 75 | 0.30 | 105 | 0.72 |
| 76 | 0.017 | 106 | 0.44 |
| 77 | 0.090 | 107 | 0.032 |
| 78 | 0.084 | 108 | 0.12 |
| 79 | 0.53 | 109 | 0.49 |
| 80 | 0.070 | 110 | 0.044 |
| 81 | 0.15 | 111 | 0.087 |
| 82 | 0.17 | 112 | 0.29 |
| 83 | 0.73 | 113 | 0.28 |
| 84 | 0.11 | 114 | 0.006 |
| 85 | 0.068 | 115 | 0.006 |

| Test Comp. No. | $IC_{50}$ (μM) | Test Comp. No. | $IC_{50}$ (μM) |
|---|---|---|---|
| 116 | 0.039 | 146 | 0.056 |
| 117 | 0.24 | 147 | 0.009 |
| 118 | 0.55 | 148 | 0.34 |
| 119 | 0.059 | 149 | 0.004 |
| 120 | 0.28 | 150 | 0.14 |
| 121 | 0.18 | 151 | 0.18 |
| 122 | 0.10 | 152 | 0.039 |
| 123 | 0.10 | 153 | 0.063 |
| 124 | 0.13 | 154 | 0.063 |
| 125 | 0.28 | 155 | 0.028 |
| 126 | 0.062 | 156 | 0.15 |
| 127 | 0.99 | 157 | 0.38 |
| 128 | 0.23 | 158 | 0.018 |
| 129 | 0.29 | 159 | 0.020 |
| 130 | 0.007 | 160 | 0.020 |
| 131 | 0.027 | 161 | 0.009 |
| 132 | 0.013 | 162 | 0.059 |
| 133 | 0.022 | 163 | 0.009 |
| 134 | 0.048 | 164 | 0.010 |
| 135 | 0.081 | 165 | 0.098 |
| 136 | 0.18 | 166 | 0.070 |
| 137 | 0.41 | 167 | 0.032 |
| 138 | 0.11 | 168 | 0.083 |
| 139 | 0.10 | 169 | 0.071 |
| 140 | 0.024 | 170 | 0.25 |
| 141 | 0.010 | 171 | 0.87 |
| 142 | 0.008 | 172 | 0.023 |
| 143 | 0.008 | 173 | 0.008 |
| 144 | 0.02 | 174 | 0.007 |
| 145 | 0.06 | 175 | 0.038 |

| Test Comp. No. | $IC_{50}$ (μM) | Test Comp. No. | $IC_{50}$ (μM) |
|---|---|---|---|
| 176 | 0.004 | 206 | 0.088 |
| 177 | 0.15 | 207 | 0.045 |
| 178 | 0.012 | 208 | 0.007 |
| 179 | 0.040 | 209 | 0.004 |
| 180 | 0.034 | 210 | 0.004 |
| 181 | 0.038 | 211 | 0.12 |
| 182 | 0.005 | 212 | 0.035 |
| 183 | 0.26 | 213 | 0.033 |
| 184 | 0.023 | 214 | 0.058 |
| 185 | 0.005 | 215 | 0.006 |
| 186 | 0.030 | 216 | 0.91 |
| 187 | 0.029 | 217 | 0.37 |
| 188 | 0.039 | 218 | 0.022 |
| 189 | 0.087 | 219 | 0.023 |
| 190 | 0.082 | 220 | 0.026 |
| 191 | 0.009 | 221 | 0.024 |
| 192 | 0.011 | 222 | 0.010 |
| 193 | 0.036 | 223 | 0.022 |
| 194 | 0.21 | 224 | 0.38 |
| 195 | 0.010 | 225 | 0.030 |
| 196 | 0.013 | 226 | 0.019 |
| 197 | 0.99 | 227 | 0.029 |
| 198 | 0.040 | 228 | 0.029 |
| 199 | 0.019 | 229 | 0.029 |
| 200 | 0.024 | 230 | 0.020 |
| 201 | 0.023 | 231 | 0.007 |
| 202 | 0.14 | 232 | 0.020 |
| 203 | 0.070 | 233 | 0.15 |
| 204 | 0.11 | 234 | 0.14 |
| 205 | 0.074 | 235 | 0.006 |

| Test Comp. No. | $IC_{50}$ (μM) | Test Comp. No. | $IC_{50}$ (μM) |
|---|---|---|---|
| 236 | 0.006 | 267 | 0.12 |
| 237 | 0.041 | 268 | 0.018 |
| 238 | 0.020 | 269 | 0.003 |
| 239 | 0.17 | 270 | 0.046 |
| 240 | 0.022 | 271 | 0.030 |
| 241 | 0.006 | 272 | 0.40 |
| 242 | 0.17 | 273 | 0.027 |
| 243 | 0.40 | 274 | 0.024 |
| 244 | 0.018 | 275 | 0.018 |
| 245 | 0.059 | 276 | 0.032 |
| 246 | 0.027 | 277 | 0.016 |
| 247 | 0.048 | 278 | 0.013 |
| 248 | 0.060 | 279 | 0.008 |
| 250 | 0.12 | 280 | 0.045 |
| 251 | 0.094 | 281 | 0.011 |
| 252 | 0.063 | 282 | 0.38 |
| 253 | 0.052 | 283 | 0.096 |
| 254 | 0.016 | 284 | 0.019 |
| 255 | 0.005 | 285 | 0.008 |
| 256 | 0.004 | 286 | 0.019 |
| 257 | 0.045 | 287 | 0.007 |
| 258 | 0.20 | 288 | 0.015 |
| 259 | 0.25 | 289 | 0.071 |
| 260 | 0.13 | 290 | 0.021 |
| 261 | 0.011 | 291 | 0.13 |
| 262 | 0.029 | 292 | 0.18 |
| 263 | 0.053 | 293 | 0.065 |
| 264 | 0.030 | 294 | 0.33 |
| 265 | 0.025 | 295 | 0.026 |
| 266 | 0.013 | 296 | 0.25 |

| Test Comp. No. | $IC_{50}$ (μM) | Test Comp. No. | $IC_{50}$ (μM) |
|---|---|---|---|
| 297 | 0.051 | 311 | 0.013 |
| 298 | 0.10 | 312 | 0.29 |
| 299 | 0.22 | 313 | 0.012 |
| 300 | 0.48 | 314 | 0.096 |
| 301 | 0.14 | 315 | 0.025 |
| 302 | 0.011 | 316 | 0.060 |
| 303 | 0.025 | 317 | 0.072 |
| 304 | 0.024 | 318 | 0.060 |
| 305 | 0.038 | 319 | 0.058 |
| 306 | 0.077 | 320 | 0.039 |
| 307 | 0.010 | 321 | 0.012 |
| 308 | 0.023 | 322 | 0.025 |
| 309 | 0.015 | 323 | 0.014 |
| 310 | 0.008 | | |

Experiment 3: Anti-antidiuretic activity (effect on endogenous ADH)

A test compound or solvent (dimethylformamide) is administered into a caudal vein of untreated, unrestrained SD rats (male, weight: 300 - 350 g) and the amount of urine, which is spontaneously excreted for a period of 2 hours thereafter, is collected and measured by using a metabolic gauge. During this measurement, the rats are allowed to take water and feed freely.

The amount of urine of control rats (solvent-treated group) is regarded as 100 %, and the results are expressed as $ED_3$ value, which is the dose of the test compound to be required to excrete the urine by three times than that of the control rats. The results are shown in the following Table 7.

Table 12

| Test compound No. | $ED_3$ (mg/kg) |
|---|---|
| 6 | 10 |
| 33 | 1.9 |
| 178 | 4.2 |
| 249 | 0.4 *) |

*): Physicological saline solution was used as a solvent instead of dimethylformamide.

## Claims

### Claims for the following Contracting States : CH, DE, DK, FR, GB, IT, LI, NL, SE

1. A benzoheterocyclic compound of the following formula

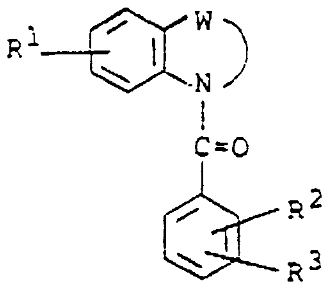

(1)

wherein $R^1$ is hydrogen atom, a halogen atom, a $C_1$-$C_6$ alkyl, an amino having optionally a $C_1$-$C_6$ alkyl substituent, or a $C_1$-$C_6$ alkoxy,

$R^2$ is hydrogen atom, a halogen atom, a $C_1$-$C_6$ alkoxy, a phenyl($C_1$-$C_6$)alkoxy, hydroxy, a $C_1$-$C_6$ alkyl, an amino having optionally a $C_1$-$C_6$ alkyl substituent, a carbamoyl-substituted $C_1$-$C_6$ alkoxy, an amino-substituted $C_1$-$C_6$ alkoxy having optionally a $C_1$-$C_6$ alkyl substituent, or a benzoyloxy which has optionally a halogen substituent on the phenyl ring,

$R^3$ is a group of the formula:

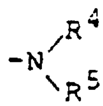

or a group of the formula:

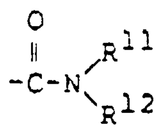

,

$R^4$ is hydrogen atom, a benzoyl which has optionally a halogen substituent on the phenyl ring, or a $C_1$-$C_6$ alkyl,

$R^5$ is (a) a group of the fromula:

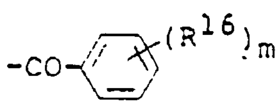

[wherein $R^{16}$ is (i) a halogen atom; (ii) a $C_1$-$C_6$ alkyl which has optionally a substituent selected from a halogen atom and hydroxy; (iii) hydroxy; (iv) a $C_1$-$C_6$ alkoxy; (v) a $C_1$-$C_6$ alkanoyloxy; (vi) a $C_1$-$C_6$ alkylthio; (vii) a $C_1$-$C_6$ alkanoyl; (viii) carboxy; (ix) a $C_1$-$C_6$ alkoxycarbonyl; (x) cyano; (xi) nitro; (xii) an amino which has optionally a substituent selected from a $C_1$-$C_6$ alkyl and a $C_1$-$C_6$ alkanoyl; (xiii) phenyl; (xiv) a $C_3$-$C_8$ cycloalkyl; (xv) a $C_2$-$C_6$ alkanoyloxy-substituted $C_1$-$C_6$ alkoxy; (xvi) a carboxy-substituted $C_1$-$C_6$ alkoxy; (xvii) a halogen-substituted $C_1$-$C_6$ alkoxy; (xviii) a carbamoyl-substituted $C_1$-$C_6$ alkoxy; (xix) a hydroxy-substituted $C_1$-$C_6$ alkoxy; (xx) a $C_1$-$C_6$ alkoxycarbonyl-substituted $C_1$-$C_6$ alkoxy; (xxi) a phthalimido-substituted $C_1$-$C_6$ alkoxy; (xxii) an aminocarbonyl-$C_1$-$C_6$ alkoxy having a

$C_1$-$C_6$ alkyl substituent; or (xxiii) a group of the formula;

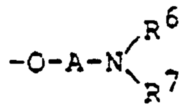

(A is alkylene with up to 6 carbon atoms, and

$R^6$ and $R^7$ are the same or different and are each hydrogen atom, a $C_1$-$C_6$ alkyl having optionally a hydroxy substituent, a $C_1$-$C_6$ alkanoyl, or benzoyl, or

$R^6$ and $R^7$ may bind together with nitrogen atom to which they bond to form a 5- or 6-membered saturated heterocyclic group with or without being intervened with nitrogen or oxygen atom wherein the heterocyclic group has optionally a substituent selected from piperidinyl and a $C_1$-$C_6$ alkyl);

and m is an integer of 0 to 3],

(b) a phenyl-$C_1$-$C_6$ alkoxycarbonyl,
(c) a $C_1$-$C_6$ alkanoyl,
(d) a phenyl-$C_2$-$C_6$ alkanoyl,
(e) a $C_3$-$C_8$ cycloalkyl-$C_2$-$C_6$ alkanoyl,
(f) a $C_3$-$C_8$ cycloalkylcarbonyl,
(g) tricyclo[3.3.1.1]decanylcarbonyl,
(h) naphthylcarbonyl,
(i) pyridylcarbonyl
(j) furoyl,
(k) thenoyl,
(l) a phenoxy-$C_2$-$C_6$ alkanoyl which phenyl ring has optionally 1 to 3 substituents selected from a $C_1$-$C_6$ alkyl, a $C_1$-$C_6$ alkoxy and an amino having optionally a $C_1$-$C_6$ alkanoyl substituent,
(m) a phthalimido-substituted $C_2$-$C_6$ alkanoyl,
(o) a $C_1$-$C_6$ alkoxycarbonyl-$C_2$-$C_6$ alkanoyl,
(p) a carboxy-$C_2$-$C_6$ alkanoyl,
(q) a naphthyloxy-$C_2$-$C_6$ alkanoyl,
(r) a halogen-substituted $C_1$-$C_6$ alkanoyl,
(s) a group of the formula:

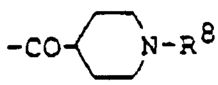

(wherein $R^8$ is (i) hydrogen atom, (ii) a $C_1$-$C_6$ alkyl, (iii) a phenyl-$C_1$-$C_6$ alkoxycarbonyl, (iv) a carbamoyl-$C_1$-$C_6$ alkyl, (v) an amino-$C_2$-$C_6$ alkanoyl having optionally a $C_1$-$C_6$ alkyl substituent, or (vi) a $C_1$-$C_6$ alkanoyl),
(t) an anilinocarbonyl which has optionally a $C_1$-$C_6$ alkyl substituent on the phenyl ring,
(u) phenoxycarbonyl,
(v) a phenylsulfonyl which has optionally a substituent selected from a halogen atom and a $C_1$-$C_6$ alkyl on the phenyl ring,
(w) quinolylsulfonyl, or
(x) a group of the formula:

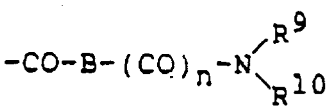

wherein B is a $C_1$-$C_6$ alkylene, n is an integer of 0 or 1, and $R^9$ and $R^{10}$ are the same or different and are each (i) hydrogen atom, (ii) alkyl with up to 6 carbon atoms having optionally a hydroxy substituent, (iii) a $C_3$-$C_8$ cycloalkyl, (iv) a phenyl-$C_1$-$C_6$ alkyl, (v) a $C_1$-$C_6$ alkanoyl, (vi) alkenyl having up to 6 carbon atoms, (vii) a phenoxy-$C_1$-$C_6$ alkyl, (viii) a phenyl which has optionally 1 to 3 substituents selected from an amino-$C_1$-$C_6$ alkyl having optionally a $C_1$-

$C_6$ alkanoyl substituent, a $C_1$-$C_6$ alkyl, a $C_1$-$C_6$ alkoxy and a halogen atom, (ix) a phthalimido-substituted $C_1$-$C_6$ alkyl, (x) an amino-$C_1$-$C_6$ alkyl having optionally a $C_1$-$C_6$ alkanoyl substituent, (xi) alkynyl having up to 6 carbon atoms, or (xii) an amino-$C_1$-$C_6$ alkyl having optionally a $C_1$-$C_6$ alkyl substituent, or $R^9$ and $R^{10}$ may bind together with nitrogen atom to which they bond to form a 5- or 6-membered saturated heterocyclic group with or without being intervened with nitrogen or oxygen atom wherein the heterocyclic group has optionally a substituent selected from a $C_1$-$C_6$ alkyl, a $C_1$-$C_6$ alkoxycarbonyl and piperidinyl),

$R^{11}$ is hydrogen atom or a $C_1$-$C_6$ alkyl,

$R^{12}$ is a $C_3$-$C_8$ cycloalkyl, or a phenyl which has optionally 1 to 3 substituents selected from a $C_1$-$C_6$ alkoxy, a $C_1$-$C_6$ alkyl and a halogen atom,

W is a group of the formula: $-(CH_2)_p-$ (p is an integer of 3 to 5), or a group of the formula: $-CH=CH(CH_2)_q-$(q is an integer of 1 to 3), or wherein the heterocyclic group of the formula:

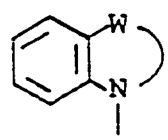

is 2,3,4,5-tetrahydro-1H-1,4-benzodiazepine

and further said $-(CH_2)_p-$ group having optionally 1 to 3 substituents selected from (i) a $C_1$-$C_6$ alkyl having optionally a hydroxy substituent, (ii) a $C_1$-$C_6$ alkoxycarbonyl, (iii) carboxy, (iv) hydroxy, (v) oxo, (vi) a $C_1$-$C_6$ alkanoyloxy having optionally a halogen substituent, (vii) an amino-$C_1$-$C_6$ alkyl having optionally a substituent selected from a $C_1$-$C_6$ alkyl and a $C_1$-$C_6$ alkanoyl, (viii) a $C_2$-$C_6$ alkanoyloxy-substituted $C_1$-$C_6$ alkyl, (ix) a $C_1$-$C_6$ alkylsulfonyloxy-$C_1$-$C_6$ alkyl, (x) an azido-$C_1$-$C_6$ alkyl, (xi) a group of the formula: $-O-CH_2-$ (which is bound to the group $-(CH_2)_p-$ or $-CH=CH-(CH_2)_q-$ so as to be in the spiro form), (xii) an aminocarbonyloxy having optionally a $C_1$-$C_6$ alkyl substituent, (xiii) a $C_1$-$C_6$ alkoxy, (xiv) a $C_1$-$C_6$ alkoxycarbonyl-substituted $C_1$-$C_6$ alkoxy, (xv) a carboxy-substituted $C_1$-$C_6$ alkoxy, (xvi) an aminocarbonyl-$C_1$-$C_6$ alkoxy having optionally a $C_1$-$C_6$ alkyl substituent, (xvii) an amino-$C_1$-$C_6$ alkoxy having optionally a substituent selected from a $C_1$-$C_6$ alkyl and a $C_1$-$C_6$ alkanoyl, (xviii) a phthalimido-substituted $C_1$-$C_6$ alkoxy, (xix) hydroxyimino, (xx) a $C_1$-$C_6$ alkanoyloxyimino, (xxi) a $C_1$-$C_6$ alkylidene, (xxii) a halogen atom, (xxiii) azido, (xxvi) hydrazino, (xxvii) pyrrolyl, (xxviii) an amino-$C_1$-$C_6$ alkanoyloxy having optionally a $C_1$-$C_6$ alkyl substituent, (xxix) a group of the formula:

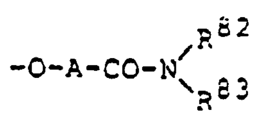

(A is as defined above,

and $R^{82}$ and $R^{83}$ are the same or different and are each hydrogen atom, a $C_1$-$C_6$ alkyl, a carbamoyl-substituted $C_1$-$C_6$ alkyl, a hydroxy-substituted $C_1$-$C_6$ alkyl, or a pyridyl-$C_1$-$C_6$ alkyl,

or $R^{82}$ and $R^{83}$ may bind together with nitrogen atom to which they bond to form a 5- or 6-membered saturated heterocyclic group with or without being intervened with nitrogen, oxygen or sulfur atom wherein the heterocyclic group has optionally a substituent selected from oxo, a $C_1$-$C_6$ alkyl, a $C_1$-$C_6$ alkanoyl, and carbamoyl), and

(xxx) a group of the formula:

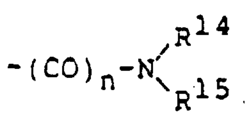

(wherein n is as defined above, and $R^{14}$ and $R^{15}$ are the same or different and are each (i) hydrogen atom, (ii) a $C_1$-$C_6$ alkyl, (iii) alkenyl having up to 6 carbon atoms, (iv) a $C_1$-$C_6$ alkanoyl, (v) a $C_3$-$C_8$ cycloalkyl, (vi) an oxiranyl-substituted $C_1$-$C_6$ alkyl, (vii) a $C_1$-$C_6$ alkyl having 1 to 2 substituents selected from a $C_1$-$C_6$ alkoxy, hydroxy and an amino having optionally a $C_1$-$C_6$ alkyl substituent, (viii) a phenyl-$C_1$-$C_6$ alkyl, (ix) a pyridyl-$C_1$-$C_6$ alkyl, (x) a $C_1$-$C_6$ alkylsulfonyl, (xi) benzoyl, (xii) a $C_1$-$C_6$ alkoxycarbonyl, (xiii) anilinocarbonyl, (xiv) an aminocarbonyl having optionally a $C_1$-$C_6$ alkyl substituent, (xv) a cyano-substituted $C_1$-$C_6$ alkyl, (xvi) a $C_1$-$C_6$ alkoxycarbonyl-substituted $C_1$-$C_6$ alkyl, (xvii) a carbamoyl-substituted $C_1$-$C_6$ alkyl, (xviii) a carboxy-substituted $C_1$-$C_6$ alkyl, (xix) a tetrahydropyranyloxy-substituted $C_1$-$C_6$ alkyl, (xx) a $C_2$-$C_6$ alkanoyloxy-substituted $C_1$-$C_6$ alkyl, (xxi) a piperidinyl having optionally a phe-

nyl-$C_1$-$C_6$ alkyl substituent on the piperidinyl ring, (xxii) a halogen-substituted $C_2$-$C_6$ alkanoyl, (xxiii) an imidazolyl-substituted $C_2$-$C_6$ alkanoyl, (xxiv) an amino-$C_2$-$C_6$ alkanoyl having optionally a substituent selected from a $C_1$-$C_6$ alkyl and a $C_1$-$C_6$ alkoxycarbonyl, (xxv) an aminocarbonyl-$C_1$-$C_6$ alkyl having optionally a $C_1$-$C_6$ alkyl substituent, or (xxvi) a phenyl-$C_1$-$C_6$ alkoxycarbonyl, or $R^{14}$ and $R^{15}$ may bind together with nitrogen atom to which they bond to form a 5- or 6-membered saturated heterocyclic group with or without being intervened with nitrogen or oxygen, wherein the heterocyclic group may optionally have a substituent selected from a $C_1$-$C_6$ alkyl, a phenyl-$C_1$-$C_6$ alkyl or a $C_1$-$C_6$ alkanoyl), and further said $-CH=CH-(CH_2)_q-$ group having optionally 1 to 3 substituents selected from

(i) A $C_1$-$C_6$-alkyl having optionally a hydroxy substituent and
(ii) an amino-$C_1$-$C_6$-alkyl having optionally a substituent selected from $C_1$-$C_6$ alkyl and a $C_1$-$C_6$ alkanoyl,

and a salt thereof.

2. The compound according to claim 1, wherein $R^1$ in the formula (1) is hydrogen atom, or a salt thereof.

3. The compound according to claim 1, wherein $R^1$ in the formula (1) is a halogen atom, and a salt thereof.

4. The compound according to claim 1, wherein $R^1$ in the formula (1) is a $C_1$-$C_6$ alkyl, an amino having optionally a $C_1$-$C_6$ alkyl substituent, or a $C_1$-$C_6$ alkoxy, and a salt thereof.

5. The compound according to claim 2, wherein $R^2$ is hydrogen atom, and a salt thereof.

6. The compound according to claim 2, wherein $R^2$ is a halogen atom, a $C_1$-$C_6$ alkoxy, or a $C_1$-$C_6$ alkyl, and a salt thereof.

7. The compound according to claim 2, wherein $R^2$ is a phenyl-$C_1$-$C_6$ alkoxy; hydroxy; an amino having optionally a $C_1$-$C_6$ alkyl substituent; a carbamoyl-substituted $C_1$-$C_6$ alkoxy; an amino-substituted $C_1$-$C_6$ alkoxy having optionally a $C_1$-$C_6$ alkyl substituent; or a benzoyloxy having optionally a halogen substituent on the phenyl ring thereof, and a salt thereof.

8. The compound according to claim 3, wherein $R^2$ is hydrogen atom, and a salt thereof.

9. The compound according to claim 3, wherein $R^2$ is a halogen atom, a $C_1$-$C_6$ alkoxy, or a $C_1$-$C_6$ alkyl, and a salt thereof.

10. The compound according to claim 3, wherein $R^2$ is a phenyl-$C_1$-$C_6$ alkoxy; hydroxy; an amino having optionally a $C_1$-$C_6$ alkyl substituent; a carbamoyl-substituted $C_1$-$C_6$ alkoxy; an amino-substituted $C_1$-$C_6$ alkoxy having optionally a $C_1$-$C_6$ alkyl substituent; or a benzoyloxy having optionally a halogen substituent on the phenyl ring thereof, and a salt thereof.

11. The compound according to claim 4, wherein $R^2$ is hydrogen atom, and a salt thereof.

12. The compound according to claim 4, wherein $R^2$ is a halogen atom, a $C_1$-$C_6$ alkoxy, or a $C_1$-$C_6$ alkyl, and a salt thereof.

13. The compound according to claim 4, wherein $R^2$ is a phenyl-$C_1$-$C_6$ alkoxy; hydroxy; an amino having optionally a $C_1$-$C_6$ alkyl substituent; a carbamoyl-substituted $C_1$-$C_6$ alkoxy; an amino-substituted $C_1$-$C_6$ alkoxy having optionally a $C_1$-$C_6$ alkyl substituent; or a benzoyloxy having optionally a halogen substituent on the phenyl ring thereof, and a salt thereof.

14. The compound according to claim 5, wherein $R^3$ is a group of the formula: $-NR^4R^5$ ($R^4$ and $R^5$ are as defined in claim 1), and a salt thereof.

15. The compound according to claim 5, wherein $R^3$ is a group of the formula: $-CO-NR^{11}R^{12}$ ($R^{11}$ and $R^{12}$ are as defined in claim 1), and a salt thereof.

16. The compound according to claim 6, wherein $R^3$ is a group of the formula: $-NR^4R^5$ ($R^4$ and $R^5$ are as defined in claim 1), and a salt thereof.

17. The compound according to claim 6, wherein $R^3$ is a group of the formula: $-CO-NR^{11}R^{12}$ ($R^{11}$ and $R^{12}$ are as defined in claim 1), and a salt thereof.

**18.** The compound according to claim 8, wherein $R^3$ is a group of the formula: $-NR^4R^5$ ($R^4$ and $R^5$ are as defined in claim 1), and a salt thereof.

**19.** The compound according to claim 8, wherein $R^3$ is a group of the formula: $-CO-NR^{11}R^{12}$ ($R^{11}$ and $R^{12}$ are as defined in claim 1), and a salt thereof.

**20.** The compound according to claim 9, wherein $R^3$ is a group of the formula: $-NR^4R^5$ ($R^4$ and $R^5$ are as defined in claim 1), and a salt thereof.

**21.** The compound according to claim 9, wherein $R^3$ is a group of the formula: $-CO-NR^{11}R^{12}$ ($R^{11}$ and $R^{12}$ are as defined in claim 1), and a salt thereof.

**22.** The compound according to claim 14, wherein $R^4$ is hydrogen atom, and $R^5$ is a group of the formula:

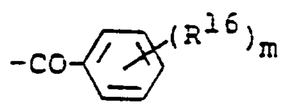

(wherein $R^{16}$ and m are as defined in claim 1), and a salt thereof.

**23.** The compound according to claim 14, wherein $R^4$ is hydrogen atom and $R^5$ is a phenyl-$C_1$-$C_6$ alkoxycarbonyl, a $C_1$-$C_6$ alkanoyl a phenyl-$C_2$-$C_6$ alkanoyl, a $C_3$-$C_8$ cycloalkyl-$C_2$-$C_6$ alkanoyl, a $C_3$-$C_8$ cycloalkylcarbonyl, tricyclo[3.3.1.1]decanylcarbonyl, naphthylcarbonyl, pyridylcarbonyl, furoyl, thenoyl, a phenoxy-$C_2$-$C_6$ alkanoyl which phenyl ring has optionally 1 to 3 substituents selected from a $C_1$-$C_6$ alkyl, a $C_1$-$C_6$ alkoxy and an amino having optionally a $C_1$-$C_6$ alkanoyl substituent, a phthalimido-substituted $C_2$-$C_6$ alkanoyl, a $C_1$-$C_6$ alkoxycarbonyl-$C_2$-$C_6$ alkanoyl, a carboxy-$C_2$-$C_6$ alkanoyl, a naphthyloxy-$C_2$-$C_6$ alkanoyl, a halogen-substituted $C_1$-$C_6$ alkanoyl, a group of the formula:

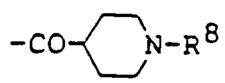

(wherein $R^8$ is hydrogen atom, a $C_1$-$C_6$ alkyl, a phenyl-$C_1$-$C_6$ alkoxycarbonyl, a carbamoyl-$C_1$-$C_6$ alkyl, an amino-$C_1$-$C_6$ alkanoyl having optionally a $C_1$-$C_6$ alkyl substituent, or a $C_1$-$C_6$ alkanoyl), an anilinocarbonyl which has optionally a $C_1$-$C_6$ alkyl substituent on the phenyl ring, phenoxycarbonyl, a phenylsulfonyl which has optionally a substituent selected from a halogen atom and a $C_1$-$C_6$ alkyl on the phenyl ring, quinolylsulfonyl, or a group of the formula:

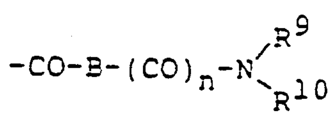

(wherein B is a $C_1$-$C_6$ alkylene, n is an integer of 0 or 1, and $R^9$ and $R^{10}$ are the same or different and are each hydrogen atom, a $C_1$-$C_6$ alkyl having optionally a hydroxy substituent, a $C_3$-$C_8$ cycloalkyl, a phenyl-$C_1$-$C_6$ alkyl, a $C_1$-$C_6$ alkanoyl, a $C_2$-$C_6$ alkenyl, a phenoxy-$C_1$-$C_6$ alkyl, a phenyl which has optionally 1 to 3 substituents selected from an amino-$C_1$-$C_6$ alkyl having optionally a $C_1$-$C_6$ alkanoyl substituent, a $C_1$-$C_6$ alkyl, a $C_1$-$C_6$ alkoxy and a halogen atom, a phthalimido-substituted $C_1$-$C_6$ alkyl, an amino-$C_1$-$C_6$ alkyl having optionally a $C_1$-$C_6$ alkanoyl substituent, a $C_2$-$C_6$ alkynyl, or an amino-$C_1$-$C_6$ alkyl having optionally a $C_1$-$C_6$ alkyl substituent, or $R^9$ and $R^{10}$ may bind together with nitrogen atom to which they bond to form a 5- or 6-membered saturated heterocyclic group with or without being intervened with nitrogen or oxygen atom wherein the heterocylic group has optionally a substituent selected from a $C_1$-$C_6$ alkyl, a $C_1$-$C_6$ alkoxycarbonyl and piperidinyl), and a salt thereof.

**24.** The compound according to claim 14, wherein $R^4$ is a $C_1$-$C_6$ alkyl, and a salt thereof.

**25.** The compound according to claim 16, wherein $R^4$ is hydrogen atom, and $R^5$ is a group of the formula:

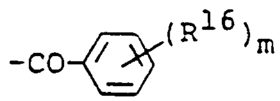

(wherein $R^{16}$ and m are as defined in claim 1), and a salt thereof.

**26.** The compound according to claim 16, wherein $R^4$ is hydrogen atom and $R^5$ is a phenyl-$C_1$-$C_6$ alkoxycarbonyl, a $C_1$-$C_6$ alkanoyl, a phenyl-$C_1$-$C_6$ alkanoyl, a $C_3$-$C_8$ cycloalkyl-$C_2$-$C_6$ alkanoyl, a $C_3$-$C_8$ cycloalkylcarbonyl, tricyclo[3.3.1.1]decanylcarbonyl, naphthylcarbonyl, pyridylcarbonyl, furoyl, thenoyl, a phenoxy-$C_2$-$C_6$ alkanoyl which phenyl ring has optionally 1 to 3 substituents selected from a $C_1$-$C_6$ alkyl, a $C_1$-$C_6$ alkoxy and an amino having optionally a $C_1$-$C_6$ alkanoyl substituent, a phthalimido-substituted $C_2$-$C_6$ alkanoyl, a $C_1$-$C_6$ alkoxycarbonyl-$C_2$-$C_6$ alkanoyl, a carboxy-$C_2$-$C_6$ alkanoyl, a naphthyloxy-$C_2$-$C_6$ alkanoyl, a halogen-substituted $C_1$-$C_6$ alkanoyl, a group of the formula:

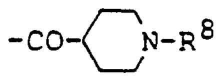

(wherein $R^8$ is hydrogen atom, a $C_1$-$C_6$ alkyl, a phenyl-$C_1$-$C_6$ alkoxycarbonyl, a carbamoyl-$C_1$-$C_6$ alkyl, an amino-$C_1$-$C_6$ alkanoyl having optionally a $C_1$-$C_6$ alkyl substituent, or a $C_1$-$C_6$ alkanoyl), an anilinocarbonyl which has optionally a $C_1$-$C_6$ alkyl substituent on the phenyl ring, phenoxycarbonyl, a phenylsulfonyl which has optionally a substituent selected from a halogen atom and a $C_1$-$C_6$ alkyl on the phenyl ring, quinolylsulfonyl, or a group of the formula:

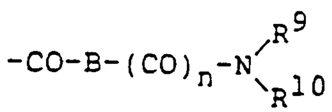

(wherein B is a $C_2$-$C_6$ alkylene, n is an integer of 0 or 1, and $R^9$ and $R^{10}$ are the same or different and are each hydrogen atom, a $C_1$-$C_6$ alkyl having optionally a hydroxy substituent, a $C_3$-$C_8$ cycloalkyl, a phenyl-$C_1$-$C_6$ alkyl, a $C_1$-$C_6$ alkanoyl, a $C_2$-$C_6$ alkenyl, a phenoxy-$C_1$-$C_6$ alkyl, a phenyl which has optionally 1 to 3 substituents selected from an amino-$C_1$-$C_6$ alkyl having optionally a $C_1$-$C_6$ alkanoyl substituent, a $C_1$-$C_6$ alkyl, a $C_1$-$C_6$ alkoxy and a halogen atom, a phthalimido-substituted $C_1$-$C_6$ alkyl, an amino-$C_1$-$C_6$ alkyl having optionally a $C_1$-$C_6$ alkanoyl substituent, a $C_2$-$C_6$ alkynyl, or an amino-$C_1$-$C_6$ alkyl having optionally a $C_1$-$C_6$ alkyl substituent, or $R^9$ and $R^{10}$ may bind together with nitrogen atom to which they bond to form a 5- or 6-membered saturated heterocyclic group with or without being intervened with nitrogen or oxygen atom wherein the heterocylic group has optionally a substituent selected from a $C_1$-$C_6$ alkyl, a $C_1$-$C_6$ alkoxycarbonyl and piperidinyl), and a salt thereof.

**27.** The compound according to claim 16, whrein $R^4$ is a $C_1$-$C_6$ alkyl, and a salt thereof.

**28.** The compound according to claim 7, wherein $R^4$ is hydrogen atom, and $R^5$ is a group of the formula:

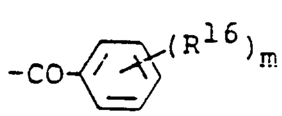

(wherein $R^{16}$ and m are as defined in claim 1), and a salt thereof.

**29.** The compound according to claim 7 10 to 13, 18 and 20, wherein $R^4$ is hydrogen atom and $R^5$ is as defined in claim 23

**30.** The compound according to claim 7, whrein $R^4$ is a $C_1$-$C_6$ alkyl, and a salt thereof.

31. The compound according to claim 18, wherein $R^4$ is hydrogen atom, and $R^5$ is a group of the formula:

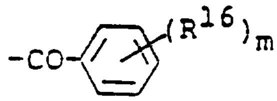

(wherein $R^{16}$ and m are as defined in claim 1), and a salt thereof.

32. The compound according to claim 18, whrein $R^4$ is a $C_1$-$C_6$ alkyl, and a salt thereof.

33. The compound according to claim 20, wherein $R^4$ is hydrogen atom, and $R^5$ is a group of the formula:

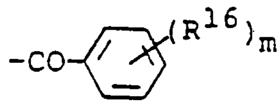

(wherein $R^{16}$ and m are as defined in claim 1), and a salt thereof.

34. The compound according to claim 20, whrein $R^4$ is a $C_1$-$C_6$ alkyl, and a salt thereof.

35. The compound according to claim 10, wherein $R^4$ is hydrogen atom, and $R^5$ is a group of the formula:

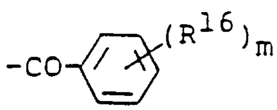

(wherein $R^{16}$ and m are as defined in claim 1), and a salt thereof.

36. The compound according to claim 10, whrein $R^4$ is a $C_1$-$C_6$ alkyl, and a salt thereof.

37. The compound according to claim 11, wherein $R^4$ is hydrogen atom, and $R^5$ is a group of the formula:

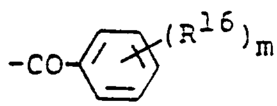

(wherein $R^{16}$ and m are as defined in claim 1), and a salt thereof.

38. The compound according to claim 11, wherein $R^4$ is a $C_1$-$C_6$ alkyl, and a salt thereof.

39. The compound according to claim 12, wherein $R^4$ is hydrogen atom, and $R^5$ is a group of the formula:

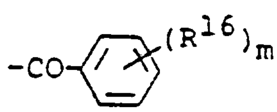

(wherein $R^{16}$ and m are as defined in claim 1), and a salt thereof.

40. The compound according to claim 12, wherein $R^4$ is a $C_1$-$C_6$ alkyl, and a salt thereof.

41. The compound according to claim 13, wherein $R^4$ is hydrogen atom, and $R^5$ is a group of the formula:

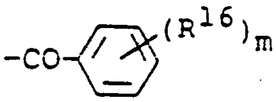

(wherein $R^{16}$ and m are as defined in claim 1), and a salt thereof.

42. The compound according to claim 13, wherein $R^4$ is a $C_1$-$C_6$ alkyl, and a salt thereof.

43. The compound according to claim 22, wherein $R^{16}$ is a halogen atom, or a $C_1$-$C_6$ alkyl having optionally a substituent selected from a halogen atom and hydroxy, and a salt thereof.

44. The compound according to claim 25, wherein $R^{16}$ is a halogen atom, or a $C_1$-$C_6$ alkyl having optionally a substituent selected from a halogen atom and hydroxy, and a salt thereof.

45. The compound according to claim 31, wherein $R^{16}$ is a halogen atom, or a $C_1$-$C_6$ alkyl having optionally a substituent selected from a halogen atom and hydroxy, and a salt thereof.

46. The compound according to claim 34, wherein $R^{16}$ is a halogen atom, or a $C_1$-$C_6$ alkyl having optionally a substituent selected from a halogen atom and hydroxy, and a salt thereof.

47. The compound according to claim 1, wherein W is a group of the formula: $-(CH_2)_p-$ wherein p is an integer of 3 to 5, and further said $-(CH_2)_p-$group has optionally 1 to 3 substituents as defined in claim 1.

48. The compound according to claim 1, wherein W is a group of the formula: $-CH=CH-(CH_2)_q-$ wherein q is an integer of 1 to 3, and further said $-CH=CH-(CH_2)_q-$ group has optionally 1 to 3 substituents selected from a $C_1$-$C_6$ alkyl having optionally a hydroxy substituent, and an amino-$C_1$-$C_6$ alkyl having optionally a substituent selected from a $C_1$-$C_6$ alkyl and a $C_1$-$C_6$ alkanoyl, and a salt thereof.

49. The compound according to claim 47, wherein W is a group of the formula: $-(CH_2)_p-$ (p is an integer of 3 to 5) and said $-(CH_2)_p-$ group has optionally 1 to 3 substituents as defined in claim 1.

50. The compound according to claim 49, wherein p in the group: $-(CH_2)_p-$ is 3 and the group has no substituent, and a salt thereof.

51. The compound according to claim 49, wherein p in the group: $-(CH_2)_p-$ is 3 and the group has a substituent of a group of the formula:

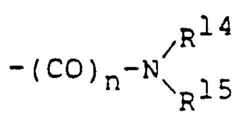

(wherein $R^{14}$, $R^{15}$, and n is as defined above), and a salt thereof.

52. The compound according to claim 49, wherein p in the group: $-(CH_2)_p-$ is 4 and the group has no substituent, and a salt thereof.

**53.** The compound according to claim 49, wherein p in the group: $-(CH_2)_p-$ is 4 and the group has a substituent of a group of the formula:

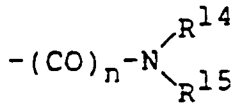

(wherein $R^{14}$, $R^{15}$, and n is as defined above), and a salt thereof.

**54.** The compound according to claim 49, wherein p in the group: $-(CH_2)_p-$ is 5, and a salt thereof.

**55.** The compound according to claim 48, wherein q in the group: $-CH=CH-(CH_2)_q-$ is 1, and a salt thereof.

**56.** The compound according to claim 48, wherein q in the group: $-CH=CH-(CH_2)_q-$ is 2, and a salt thereof.

**57.** The compound according to claim 48, wherein q in the group: $-CH=CH=(CH_2)_q-$ is 3, and a salt thereof.

**58.** The compound according to claim 51 or 53, wherein n in the substituent:

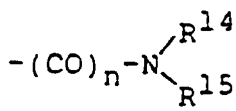

is 0, and $R^{14}$ and $R^{15}$ are the same or different and are each hydrogen atom, a $C_1$-$C_6$ alkyl, or a $C_3$-$C_8$ cycloalkyl, and a salt thereof.

**59.** The compound according to claim 56 wherein the heterocyclic group of the formula:

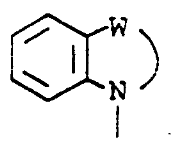

is 2,3-dihydro-1H-benzazepine, and a salt thereof.

**60.** 1-[4-(2-Methylbenzoylamino)benzoyl]-4-methyl-2,3,4,5-tetrahydro-1H-1,4-benzodiazepine.

**61.** 5-Dimethylamino-1-[4-(2-methylbenzoylamino)benzoyl]-2,3,4,5-tetrahydro-1H-benzazepine.

**62.** 5-Dimethylamino-1-[2-chloro-4-(2-methylbenzoylamino)benzoyl]-2,3,4,5-tetrahydro-1H-benzazepine.

**63.** 5-Methylamino-1-[2-chloro-4-(2-methylbenzoylamino)benzoyl]-2,3,4,5-tetrahydro-1H-benzazepine.

**64.** 5-Cyclopropylamino-1-[2-chloro-4-(2-methylbenzoylamino)benzoyl]-2,3,4,5-tetrahydro-1H-benzazepine.

**65.** 5-Cyclopropylamino-1-[2-chloro-4-(2-chlorobenzoylamino)benzoyl]-2,3,4,5-tetrahydro-1H-benzazepine.

**66.** 5-Dimethylamino-1-[2-methyl-4-(2-methylbenzoylamino)benzoyl]-2,3,4,5-tetrahydro-1H-benzazepine.

**67.** 4-Dimethylamino-1-[3-methoxy-4-(2-methylbenzoylamino)benzoyl]-1,2,3,4-tetrahydroquinoline.

**68.** 7-Chloro-5-methylamino-1-[4-(2-methylbenzoylamino)benzoyl]-2,3,4,5-tetrahydro-1H-benzazepine.

**69.** 7-Chloro-5-methylamino-1-[4-(2-chlorobenzoylamino)benzoyl]-2,3,4,5-tetrahydro-1H-benzazepine.

**70.** A vasopressin antagonistic composition which comprises as an active ingredient a compound of the formula (1) as set forth in claim 1, or a pharmaceutically acceptable salt thereof in admixture with a pharmaceutically acceptable carrier or diluent.

**71.** A process for preparing a compound of the formula (1) as set forth in claim 1, which comprises
reacting a compound of the formula (2):

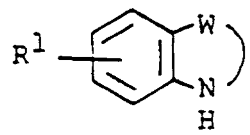

(2)

wherein $R^1$ and W are the same as defined in claim 1, with a compound of the formula (3):

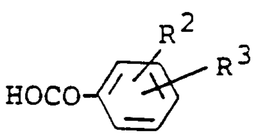

(3)

wherein $R^2$ and $R^3$ are the same as defined in claim 1.

**72.** A process for preparing a compound of the formula

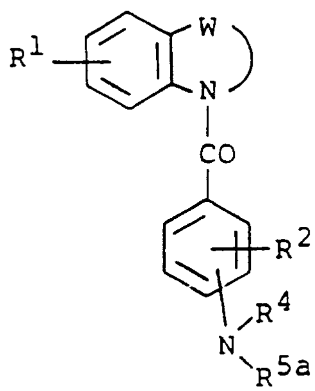

(1b)

wherein $R^1$, $R^2$, $R^4$ and W are as defined in claim 1, wherein $R^{5a}$ is the same as $R^5$ as defined in claim 1 except excluding an anilinocarbonyl having optionally a $C_1$-$C_6$ alkyl substituent on the phenyl ring, a phenylsulfonyl having optionally a substituent selected from a halogen atom and a $C_1$-$C_6$ alkyl on the phenyl ring and quinolylsulfonyl

which comprises reacting a compound of the formula (2b):

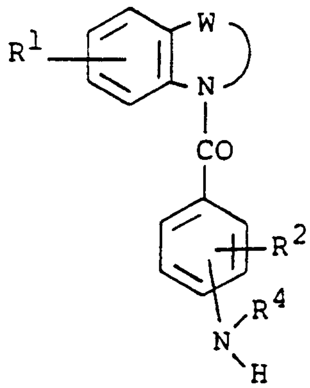

(2b)

wherein $R^1$, $R^2$, $R^4$ and W are as defined above, with a compound of the formula (4):

$$R^{5a}OH \qquad (4).$$

**73.** A process for preparing a compound of the formula (1c)

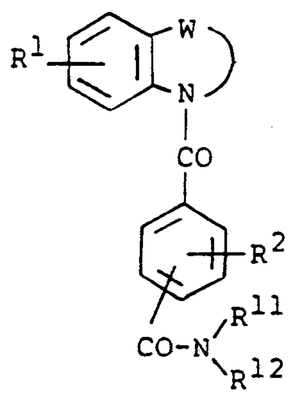

(1c)

wherein $R^1$, $R^2$, $R^{11}$, $R^{12}$ and W are as defined in claim 1,
which comprises reacting a compound of the formula (5):

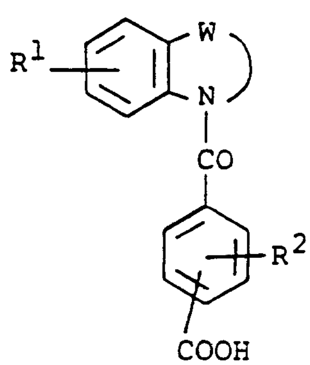

(5)

wherein $R^1$, $R^2$, and W are as defined above, with a compound of the formula (6):

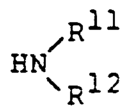

(6)

wherein $R^{11}$ and $R^{12}$ are as defined above.

**74.** A process for preparing a compound of the formula (1d)

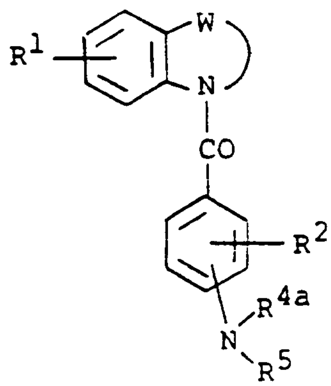

(1d)

wherein $R^1$, $R^2$, $R^5$ and W are as defined in claim 1, and $R^{4a}$ is a $C_1$-$C_6$ alkyl which comprises reacting a compound of the formula (7)

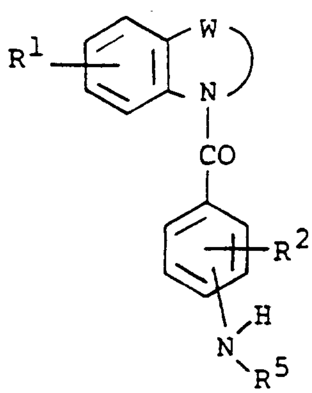

(7)

wherein $R^1$, $R^2$, $R^5$ and W are as defined above, with a compound of the formula (8) or (9):

$$R^{4a}X \qquad (8) \text{ or}$$

$$R^{17}COR^{18} \qquad (9)$$

wherein $R^{4a}$ is a $C_1$-$C_6$ alkyl, X is a halogen atom, and $R^{17}$ and $R^{18}$ are each hydrogen atom or a $C_1$-$C_6$ alkyl.

**75.** A process for preparing a compound of the formula (1e)

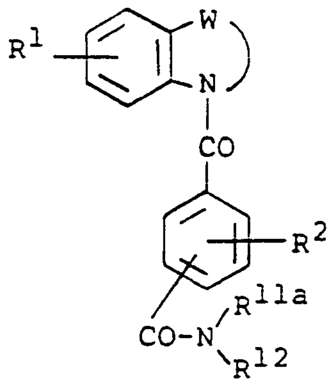

(1e)

wherein $R^1$, $R^2$, $R^{12}$ and W are as defined in claim 1, and $R^{11a}$ is $C_1$-$C_6$ alkyl which comprises reacting a compound of the formula (10):

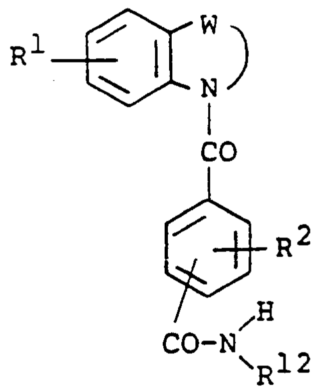

(10)

wherein $R^1$, $R^2$, $R^{12}$, and W are as defined above, with a compound of the formula (11) or (9):

$R^{11a}X$ (11) or

$R^{17}COR^{18}$ (9)

wherein $R^{11a}$ is a $C_1$-$C_6$ alkyl, and X, $R^{17}$ and $R^{18}$ are as defined above.

**76.** A process for preparing a compound of the formula (1f)

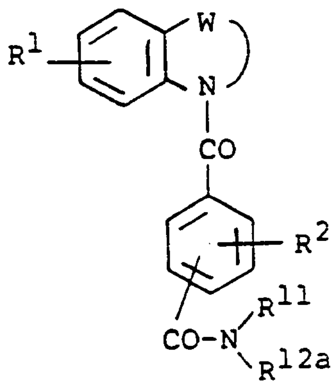

(1f)

wherein $R^1$, $R^2$, $R^{11}$, and W are as defined in claim 1, and $R^{12a}$ is $C_3$-$C_8$ cycloalkyl which comprises reacting a compound of the formula (12):

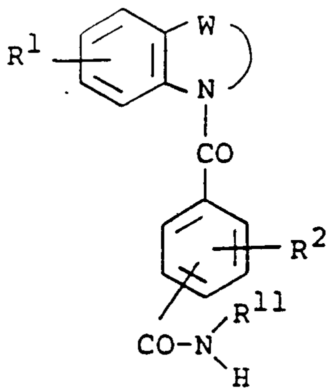

(12)

wherein $R^1$, $R^2$, $R^{11}$, and W are as defined above, with a compound of the formula (13):

$$R^{12a}X \quad (13)$$

wherein $R^{12a}$ is a $C_3$-$C_8$ cycloalkyl and X is halogen.

**77.** A process for preparing a compound of the formula (1cc)

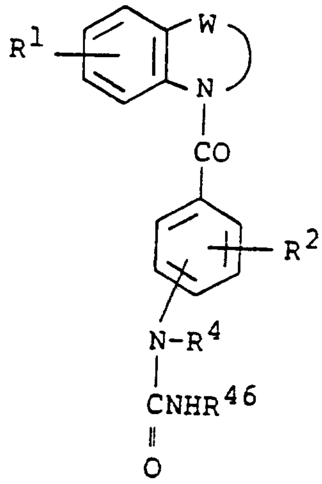

(1cc)

wherein $R^1$, $R^2$, $R^4$, and W are as defined in claim 1, and $R^{46}$ is a phenyl having optionally a $C_1$-$C_6$ alkyl substituent which comprises reacting a compound of the formula (2b):

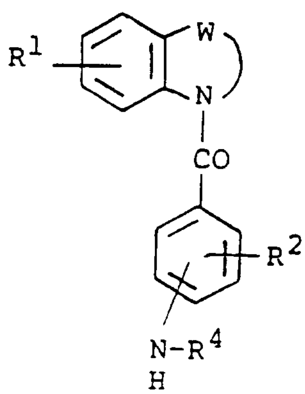

(2b)

wherein $R^1$, $R^2$, $R^4$, and W are as defined above, with a compound of the formula (38):

$$R^{46}N=C=O \quad (38)$$

wherein $R^{46}$ is as defined above.

**78.** A process for preparing a compound of the formula (1dd)

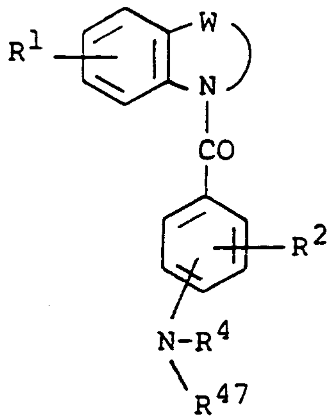

(1dd)

wherein $R^1$, $R^2$, $R^4$, and W are as defined in claim 1, and $R^{47}$ is a phenylsulfonyl which has optionally a substituent selected from a halogen atom and a $C_1$-$C_6$ alkyl on the phenyl ring, or quinolylsulfonyl, and X is halogen

which comprises reacting a compound of the formula (2b):

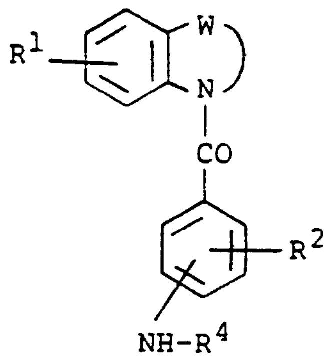

(2b)

wherein $R^1$, $R^2$, $R^4$, and W are as defined above, with a compound of the formula (39):

$$R^{47}X \quad (39)$$

wherein $R^{47}$ is as defined above.

**79.** A process for preparing a compound of the formula (1hh)

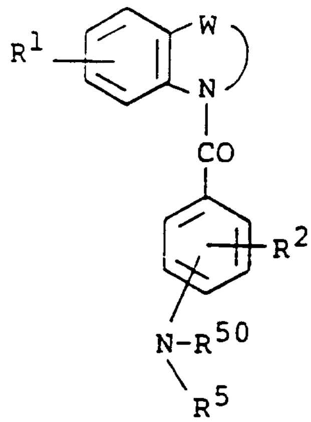

(1hh)

wherein $R^1$, $R^2$, $R^5$, and W are as defined in claim 1, and $R^{50}$ is a benzoyl having optionally a halogen substituent on the phenyl ring,

which comprises reacting a compound of the formula (7):

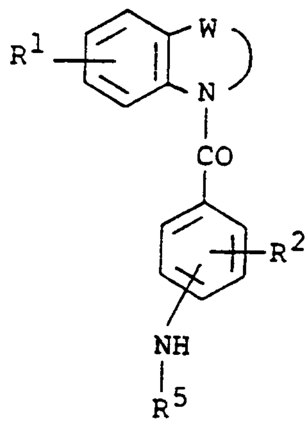

(7)

wherein $R^1$, $R^2$, $R^5$, and W are as defined above, with a compound of the formula (42):

$R^{50}OH$ (42)

wherein $R^{50}$ is as defined above.

**80.** 5-Hydroxysulfonyloxyimino-1-[2-chloro-4-(2-methylbenzoylamino)benzoyl]-2,3,4,5-tetrahydro-1H-benzazepine.

**81.** 7-Fluoro-5-hydroy-1-[2-chloro-4-(2-methylbenzoylamino)benzoyl]-2,3,4,5-tetrahydro-1H-benzazepine.

**82.** 7-[4-(2-Chlorobenzoylamino)benzoyl]-1-methyl-1,2,3,4a,5,6,7,11b-octahydro-3-oxo[1]benzazepino[4,5-b][1,4]oxazine.

**83.** Compounds of the formulae:

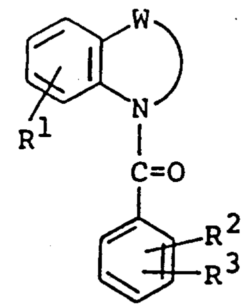

in which

a)

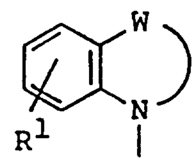

represents

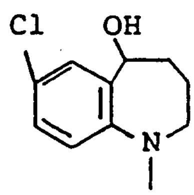,

$R^2$ represents 2-$OCH_3$ and
$R^3$ represents

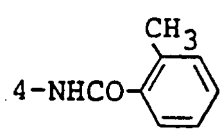;

and

b)

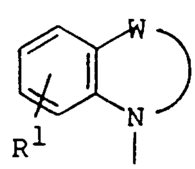

represents

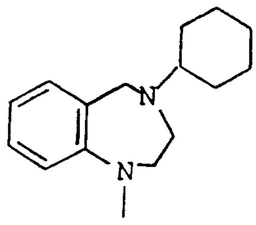

$R^2$ represents H and
$R^3$ represents

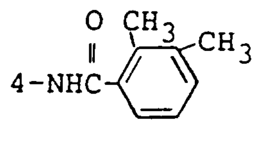

.

**84.** A benzoheterocyclic compound of the formula:

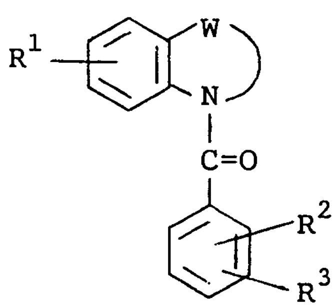

wherein $R^1$, $R^2$ and $R^3$ are as defined in claim 1, and the heterocyclic group of the formula:

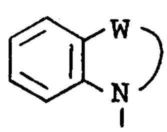

is a member selected from the group consisting of 3,4-dihydro-2H-1,4-benzoxazinyl, 1,2,3,5-tetrahydro-4,1-benzoxazepinyl, 1,2,3,4-tetrahydroquinoxalinyl, 1,2,3,4,5,6-hexahydro-1,5-benzodiazocinyl, 5-methyl-1,2,3,4,5,6-hexahydro-1,5-benzodiazocinyl, 4-methyl-1,2,3,4-tetrahydroquinoxalinyl, 1,2,3,4-tetrahydro-5,1-benzoxazepinyl, 3,4-dihydro-2H-1,4-benzothiazinyl, 2,3,4,5-tetrahydro-1,5-benzothiazepinyl, 1,2,3,5-tetrahydro-4,1-benzothiazepinyl, 4-ethyl-1,2,3,4-tetrahydroquinoxalinyl, 4-propyl-1,2,3,4-tetrahydroquinoxalinyl, 4-butyl-1,2,3,4-tetrahydroquinoxalinyl, 4-pentyl-1,2,3,4-tetrahydroquinoxalinyl, 4-hexyl-1,2,3,4-tetrahydroquinoxalinyl, 2,3,4,5-tetrahydro-1H-1,4-benzodiazepinyl, 4-methyl-2,3,4,5-tetrahydro-1H-1,4-benzodiazepinyl, 4-ethyl-2,3,4,5-tetrahydro-1H-1,4-benzodiazepinyl,4-propyl-2,3,4,5-tetrahydro-1H-1,4-benzodiazepinyl, 4-butyl-2,3,4,5-tetrahydro-1H-1,4-benzodiazepinyl, 4-pentyl-2,3,4,5-tetrahydro-1H-1,4-benzodiazepinyl, 4-hexyl-2,3,4,5-tetrahydro-1H-1,4-benzodiazepinyl, 2,3,4,5-tetrahydro-1H-1,5-benzodiazepinyl, 5-methyl-2,3,4,5-tetrahydro-1H-1,5-benzodiazepinyl, 5-ethyl-2,3,4,5-tetrahydro-1H-1,5-benzodiazepinyl,5-propyl-2,3,4,5-tetrahydro-1H-1,5-benzodiazepinyl, 5-butyl-2,3,4,5-tetrahydro-1H-1,5-benzodiazepinyl, 5-pentyl-2,3,4,5-tetrahydro-1H-1,5-benzodiazepinyl, 5-hexyl-2,3,4,5-tetrahydro-1H-1,5-benzodiazepinyl, 3,4-dihydro-1-oxo-2H-1,4-benzothiazepinyl, 3,4-dihydro-1,1-dioxo-2H-1,4-benzothiazepinyl, 1-oxo-2,3,4,5-tetrahydro-1,5-benzothiazepinyl, 1,1-dioxo-2,3,4,5-tetrahydro-1,5-benzothiazepinyl, 4-oxo-1,2,3,5-tetrahydro-4,1-benzothiazepinyl, and 4,4-dioxo-1,2,3,5-tetrahydro-4,1-benzothiazepinyl, and a salt thereof.

**Claims for the following Contracting State : ES**

1. A process for preparing a benzoheterocyclic compound of the following formula

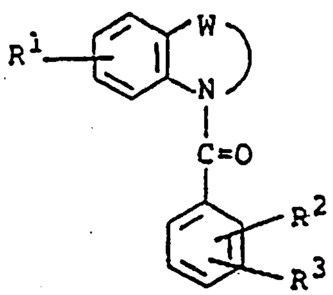

(1)

wherein $R^1$ is hydrogen atom, a halogen atom, a $C_1$-$C_6$ alkyl, an amino having optionally a $C_1$-$C_6$ alkyl substituent, or a $C_1$-$C_6$ alkoxy,

$R^2$ is hydrogen atom, a halogen atom, a $C_1$-$C_6$ alkoxy, a phenyl($C_1$-$C_6$)alkoxy, hydroxy, a $C_1$-$C_6$ alkyi, an amino having optionally a $C_1$-$C_6$ alkyl substituent, a carbamoyl-substituted $C_1$-$C_6$ alkoxy, an amino-substituted $C_1$-$C_6$ alkoxy having optionally a $C_1$-$C_6$ alkyl substituent, or a benzoyloxy which has optionally a halogen substituent on the phenyl ring,

$R^3$ is a group of the formula:

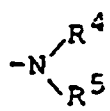

or a group of the formula:

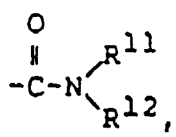

$R^4$ is hydrogen atom, a benzoyl which has optionally a halogen substituent on the phenyl ring, or a $C_1$-$C_6$ alkyl,

$R^5$ is (a) a group of the fromula:

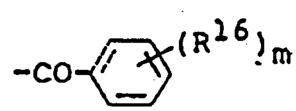

[wherein $R^{16}$ is (i) a halogen atom; (ii) a $C_1$-$C_6$ alkyl which has optionally a substituent selected from a halogen atom and hydroxy; (iii) hydroxy; (iv) a $C_1$-$C_6$ alkoxy; (v) a $C_1$-$C_6$ alkanoyloxy; (vi) a $C_1$-$C_6$ alkylthio; (vii) a $C_1$-$C_6$ alkanoyl; (viii) carboxy; (ix) a $C_1$-$C_6$ alkoxycarbonyl; (x) cyano; (xi) nitro; (xii) an amino which has optionally a substituent selected from a $C_1$-$C_6$ alkyl and a $C_1$-$C_6$ alkanoyl: (xiii) phenyl; (xiv) a $C_3$-$C_8$ cycloalkyl; (xv) a $C_2$-$C_6$ alkanoyloxy-substituted $C_1$-$C_6$ alkoxy; (xvi) a carboxy-substituted $C_1$-$C_6$ alkoxy; (xvii) a halogen-substituted $C_1$-$C_6$ alkoxy; (xviii) a carbamoyl-substituted $C_1$-$C_6$ alkoxy; (xix) a hydroxy-substituted $C_1$-$C_6$ alkoxy; (xx) a $C_1$-$C_6$ alkoxycarbonyl-substituted $C_1$-$C_6$ alkoxy; (xxi) a phthalimido-substituted $C_1$-$C_6$ alkoxy; (xxii) an aminocarbonyl-$C_1$-$C_6$ alkoxy having a

$C_1$-$C_6$ alkyl substituent; or (xxiii) a group of the formula:

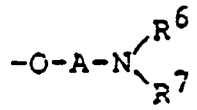

(A is alkylene with up to 6 carbon atoms, and

$R^6$ and $R^7$ are the same or different and are each hydrogen atom, a $C_1$-$C_6$ alkyl having optionally a hydroxy substituent, a $C_1$-$C_6$ alkanoyl, or benzoyl, or

$R^6$ and $R^7$ may bind together with nitrogen atom to which they bond to form a 5- or 6-membered saturated heterocyclic group with or without being intervened with nitrogen or oxygen atom wherein the heterocyclic group has optionally a substituent selected from piperidinyl and a $C_1$-$C_6$ alkyl);
and m is an integer of 0 to 3],

(b) a phenyl-$C_1$-$C_6$ alkoxycarbonyl,
(c) a $C_1$-$C_6$ alkanoyl,
(d) a phenyl-$C_2$-$C_6$ alkanoyl,
(e) a $C_3$-$C_8$ cycloalkyl-$C_2$-$C_6$ alkanoyl,
(f) a $C_3$-$c_8$ cycloalkylcarbonyl,
(g) tricyclo[3.3.1.1]decanylcarbonyl,
(h) naphthylcarbonyl,
(i) pyridylcarbonyl
(j) furoyl,
(k) thenoyl,
(l) a phenoxy-$C_2$-$C_6$ alkanoyl which phenyl ring has optionally 1 to 3 substituents selected from a $C_1$-$C_6$ alkyl, a $C_1$-$C_6$ alkoxy and an amino having optionally a $C_1$-$C_6$ alkanoyl substituent,
(m) a phthalimido-substituted $C_2$-$C_6$ alkanoyl,
(o) a $C_1$-$C_6$ alkoxycarbonyl-$C_2$-$C_6$ alkanoyl,
(p) a carboxy-$C_2$-$C_6$ alkanoyl,
(q) a naphthyloxy-$C_2$-$C_6$ alkanoyl,
(r) a halogen-substituted $C_1$-$C_6$ alkanoyl,
(s) a group of the formula:

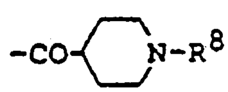

(wherein $R^8$ is (i) hydrogen atom, (ii) a $C_1$-$C_6$ alkyl, (iii) a phenyl-$C_1$-$C_6$ alkoxycarbonyl, (iv) a carbamoyl-$C_1$-$C_6$ alkyl, (v) an amino-$C_2$-$C_6$ alkanoyl having optionally a $C_1$-$C_6$ alkyl substituent, or (vi) a $C_1$-$C_6$ alkanoyl),
(t) an anilinocarbonyl which has optionally a $C_1$-$C_6$ alkyl substituent on the phenyl ring,
(u) phenoxycarbonyl,
(v) a phenylsulfonyl which has optionally a substituent selected from a halogen atom and a $C_1$-$C_6$ alkyl on the phenyl ring,
(w) quinolylsulfonyl, or
(x) a group of the formula:

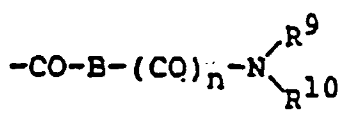

wherein B is a $C_1$-$C_6$ alkylene, n is an integer of 0 or 1, and $R^9$ and $R^{10}$ are the same or different and are each (i) hydrogen atom, (ii) alkyl with up to 6 carbon atoms having optionally a hydroxy substituent, (iii) a $C_3$-$C_8$ cycloalkyl, (iv) a phenyl-$C_1$-$C_6$ alkyl, (v) a $C_1$-$C_6$ alkanoyl, (vi) alkenyl having up to 6 carbon atoms, (vii) a phenoxy-$C_1$-$C_6$ alkyl, (viii) a phenyl which has optionally 1 to 3 substituents selected from an amino-$C_1$-$C_6$ alkyl having optionally a $C_1$-$C_6$ alkanoyl substituent, a $C_1$-$C_6$ alkyl, a $C_1$-$C_6$ alkoxy and a halogen atom, (ix) a phthalimido-substituted $C_1$-$C_6$

alkyl, (x) an amino-$C_1$-$C_6$ alkyl having optionally a $C_1$-$C_6$ alkanoyl substituent, (xi) alkynyl having up to 6 carbon atoms, or (xii) an amino-$C_1$-$C_6$ alkyl having optionally a $C_1$-$C_6$ alkyl substituent, or $R^9$ and $R^{10}$ may bind together with nitrogen atom to which they bond to form a 5- or 6-membered saturated heterocyclic group with or without being intervened with nitrogen or oxygen atom wherein the heterocyclic group has optionally a substituent selected from a $C_1$-$C_6$ alkyl, a $C_1$-$C_6$ alkoxycarbonyl and piperidinyl),

$R^{11}$ is hydrogen atom or a $C_1$-$C_6$ alkyl,

$R^{12}$ is a $C_3$-$C_8$ cycloalkyl, or a phenyl which had optionally 1 to 3 substituents selected from a $C_1$-$C_6$ alkoxy, a $C_1$-$C_6$ alkyl and a halogen atom,

W is a group of the formula: $-(CH_2)_p-$ (p is an integer of 3 to 5), or a group of the formula: $-CH=CH-(CH_2)_q-$ (q is an integer of 1 to 3), or wherein the heterocyclic group of the formula:

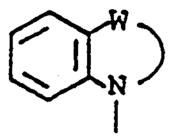

is 2,3,4,5-tetrahydro-1H-1,4-benzodiazepine,

and further said $-(CH_2)_p-$ group having optionally 1 to 3 substituents selected from (i) a $C_1$-$C_6$ alkyl having optionally a hydroxy substituent, (ii) a $C_1$-$C_6$ alkoxycarbonyl, (iii) carboxy, (iv) hydroxy, (v) oxo, (vi) a $C_1$-$C_6$ alkanoyloxy having optionally a halogen substituent, (vii) an amino-$C_1$-$C_6$ alkyl having optionally a substituent selected from a $C_1$-$C_6$ alkyl and a $C_1$-$C_6$ alkanoyl, (viii) a $C_2$-$C_6$ alkanoyloxy-substituted $C_1$-$C_6$ alkyl, (ix) a $C_1$-$C_6$ alkylsulfonyloxy-$C_1$-$C_6$ alkyl, (x) an azido-$C_1$-$C_6$ alkyl, (xi) a group of the formula: $-O-CH_2-$ (which is bound to the group $-(CH_2)_p-$ or $-CH=CH-(CH_2)_q-$ so as to be in the spiro form), (xii) an aminocarbonyloxy having optionally a $C_1$-$C_6$ alkyl substituent, (xiii) a $C_1$-$C_6$ alkoxy, (xiv) a $C_1$-$C_6$ alkoxycarbonyl-substituted $C_1$-$C_6$ alkoxy, (xv) a carboxy-substituted $C_1$-$c_6$ alkoxy, (xvi) an aminocarbonyl-$C_1$-$C_6$ alkoxy having optionally a $C_1$-$C_6$ alkyl substituent, (xvii) an amino-$C_1$-$C_6$ alkoxy having optionally a substituent selected from a $C_1$-$C_6$ alkyl and a $C_1$-$C_6$ alkanoyl, (xviii) a phthalimido-substituted $C_1$-$C_6$ alkoxy, (xix) hydroxyimino, (xx) a $C_1$-$C_6$ alkanoyloxyimino, (xxi) a $C_1$-$C_6$ alkylidene, (xxii) a halogen atom, (xxiii) azido, (xxvi) hydrazino, (xxvii) pyrrolyl, (xxviii) an amino-$C_1$-$C_6$ alkanoyloxy having optionally a $C_1$-$C_6$ alkyl substituent, (xxix) a group of the formula:

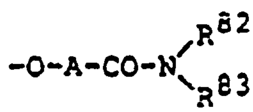

(A is as defined above,

and $R^{82}$ and $R^{83}$ are the same or different and are each hydrogen atom, a $C_1$-$C_6$ alkyl, a carbamoyl-substituted $C_1$-$C_6$ alkyl, a hydroxy-substituted $C_1$-$C_6$ alkyl, or a pyridyl-$C_1$-$C_6$ alkyl,

or $R^{82}$ and $R^{83}$ may bind together with nitrogen atom to which they bond to form a 5- or 6-membered saturated heterocyclic group with or without being intervened with nitrogen, oxygen or sulfur atom wherein the heterocyclic group has optionally a substituent selected from oxo, a $C_1$-$C_6$ alkyl, a $C_1$-$C_6$ alkanoyl, and carbamoyl), and

(xxx) a group of the formula:

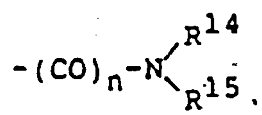

(wherein n is as defined above, and $R^{14}$ and $R^{15}$ are the same or different and are each (i) hydrogen atom, (ii) a $C_1$-$C_6$ alkyl, (iii) alkenyl having up to 6 carbon atoms, (iv) a $C_1$-$C_6$ alkanoyl, (v) a $C_3$-$C_8$ cycloalkyl, (vi) an oxiranyl-substituted $C_1$-$C_6$ alkyl, (vii) a $C_1$-$C_6$ alkyl having 1 to 2 substituents selected from a $C_1$-$C_6$ alkoxy, hydroxy and an amino having optionally a $C_1$-$C_6$ alkyl substituent, (viii) a phenyl-$C_1$-$C_6$ alkyl, (ix) a pyridyl-$C_1$-$C_6$ alkyl, (x) a $C_1$-$C_6$ alkylsulfonyl, (xi) benzoyl, (xii) a $C_1$-$C_6$ alkoxycarbonyl, (xiii) anilinocarbonyl, (xiv) an aminocarbonyl having optionally a $C_1$-$6_6$ alkyl substituent, (xv) a cyano-substituted $C_1$-$C_6$ alkyl, (xvi) a $C_1$-$C_6$ alkoxycarbonyl-substituted $C_1$-$C_6$ alkyl, (xvii) a carbamoyl-substituted $C_1$-$C_6$ alkyl, (xviii) a carboxy-substituted $C_1$-$C_6$ alkyl, (xix) a tetrahydropyranyloxy-substituted $C_1$-$C_6$ alkyl, (xx) a $C_2$-$C_6$ alkanoyloxy-substituted $C_1$-$C_6$ alkyl, (xxi) a piperidinyl having optionally a phe-

nyl-$C_1$-$C_6$ alkyl substituent on the piperidinyl ring, (xxii) a halogen-substituted $C_2$-$C_6$ alkanoyl, (xxiii) an imidazolyl-substituted $C_2$-$C_6$ alkanoyl, (xxiv) an amino-$C_2$-$C_6$ alkanoyl having optionally a substituent selected from a $C_1$-$C_6$ alkyl and a $C_1$-$C_6$ alkoxycarbonyl, (xxv) an aminocarbonyl-$C_1$-$C_6$ alkyl having optionally a $C_1$-$C_6$ alkyl substituent, or (xxvi) a phenyl-$C_1$-$C_6$ alkoxycarbonyl, or $R^{14}$ and $p^{15}$ may bind together with nitrogen atom to which they bond to form a 5- or 6-membered saturated heterocyclic group with or without being intervened with nitrogen or oxygen, wherein the heterocyclic group may optionally have a substituent selected from a $C_1$-$C_6$ alkyl, a phenyl-$C_1$-$C_6$ alkyl or a $C_1$-$C_6$ alkanoyl), and further said -CH=CH-$(CH_2)_q$- group having optionally 1 to 3 substituents selected from

(i) A $C_1$-$C_6$-alkyl having optionally a hydroxy substituent and
(ii) an amino-$C_1$-$C_6$-alkyl having optionally a substituent selected from $C_1$-$C_6$ alkyl and a $C_1$-$C_6$ alkanoyl,

and a salt thereof,
which comprises reacting a compound of the formula (2):

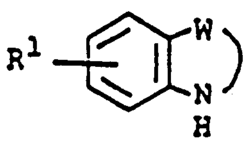

(2)

wherein $R^1$ and W are the same as defined above, with a compound of the formula (3):

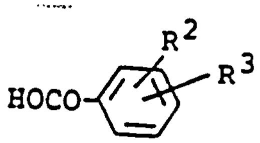

(3)

wherein $R^2$ and $R^3$ are the same as defined above.

**2.** A process for preparing a compound of the formula

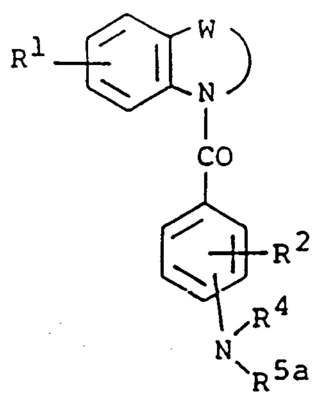

(1b)

wherein $R^1$, $R^2$, $R^4$ and W are as defined in claim 1, wherein $R^{5a}$ is the same as $R^5$ as defined in claim 1 except excluding an anilinocarbonyl having optionally a $C_1$-$C_6$ alkyl substituent on the phenyl ring, a phenylsulfonyl having optionally a substituent selected from a halogen atom and a $C_1$-$C_6$ alkyl on the phenyl ring and quinolylsulfonyl

which comprises reacting a compound of the formula (2b):

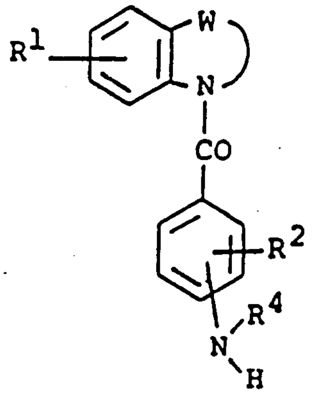

(2b)

wherein $R^1$, $R^2$, $R^4$ and W are as defined above, with a compound Of the formula (4):

$$R^{5a}OH \quad (4).$$

3. A process for preparing a compound of the formula (1c)

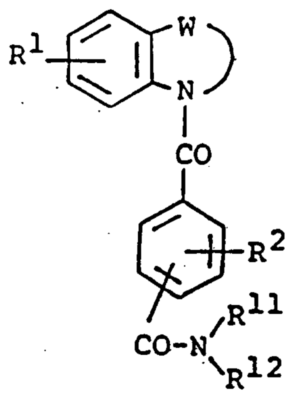

(1c)

wherein $R^1$, $R^2$, $R^{11}$, $R^{12}$ and W are as defined in claim 1,
which comprises reacting a compound of the formula (5):

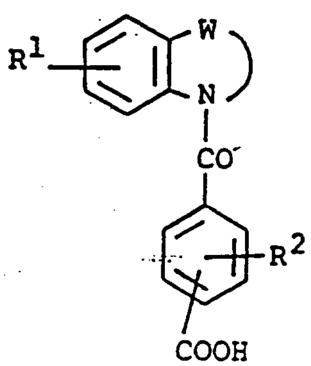

(5)

wherein $R^1$, $R^2$, and W are as defined above, with a compound of the formula (6):

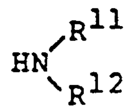

(6)

wherein $R^{11}$ and $R^{12}$ are as defined above.

**4.** A process for preparing a compound of the formula (1d)

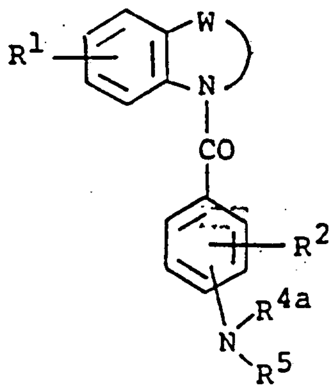

(1d)

wherein $R^1$, $R^2$, $R^5$ and W are as defined in claim 1, and $R^{4a}$ is a $C_1$-$C_6$ alkyl which comprises reacting a compound of the formula (7)

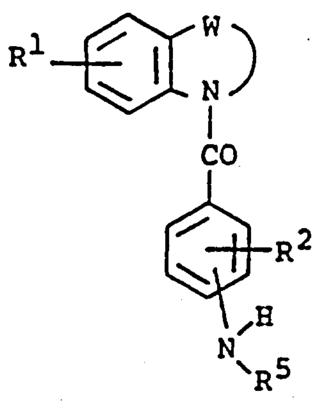

(7)

wherein $R^1$, $R^2$, $R^5$ and W are as defined above, with a compound of the formula (8) or (9):

$R^{4a}X$ (8) or

$R^{17}COR^{18}$ (9)

wherein $R^{4a}$ is a $C_1$-$C_6$ alkyl, X is a halogen atom, and $R^{17}$ and $R^{18}$ are each hydrogen atom or a $C_1$-$C_6$ alkyl.

**5.** A process for preparing a compound of the formula (1e)

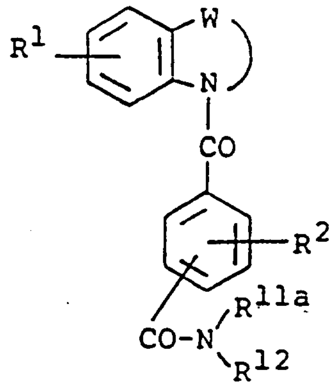

(1e)

wherein $R^1$, $R^2$, $R^{12}$ and W are as defined in claim 1, and $R^{11a}$ is $C_1$-$C_6$ alkyl which comprises reacting a compound of the formula (10):

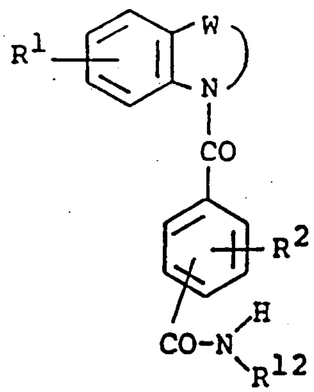

(10)

wherein $R^1$, $R^2$, $R^{12}$, and W are as defined above, with a compound of the formula (11) or (9):

$$R^{11a}X \qquad (11)\ \text{or}$$

$$R^{17}COR^{18} \qquad (9)$$

wherein $R^{11a}$ is a $C_1$-$C_6$ alkyl, and X, $R^{17}$ and $R^{18}$ are as defined above.

**6.** A process for preparing a compound of the formula (1f)

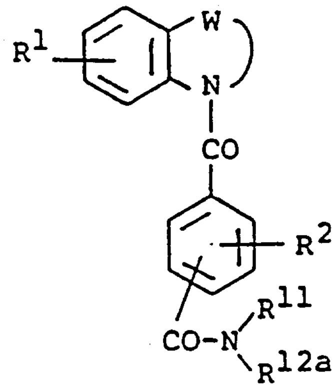

(1f)

wherein $R^1$, $R^2$, $R^{11}$, and W are as defined in claim 1, and $R^{12a}$ is $C_3$-$C_8$ cycloalkyl which comprises reacting a compound of the formula (12):

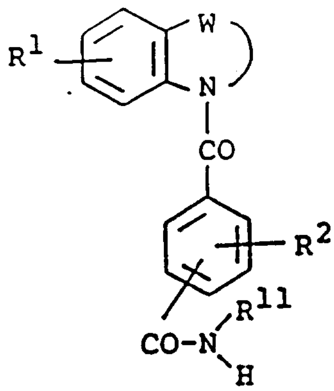

(12)

wherein $R^1$, $R^2$, $R^{11}$, and W are as defined above, with a compound of the formula (13):

$$R^{12a}X \qquad (13)$$

wherein $R^{12a}$ is a $C_3$-$C_8$ cycloalkyl and X is halogen.

7. A process for preparing a compound of the formula (1cc)

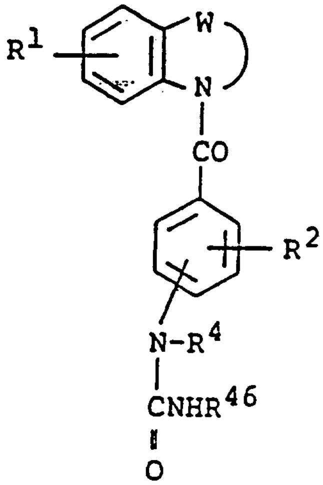

(1cc)

wherein $R^1$, $R^2$, $R^4$, and W are as defined in claim 1, and $R^{46}$ is a phenyl having optionally a $C_1$-$C_6$ alkyl substituent which comprises reacting a compound of the formula (2b):

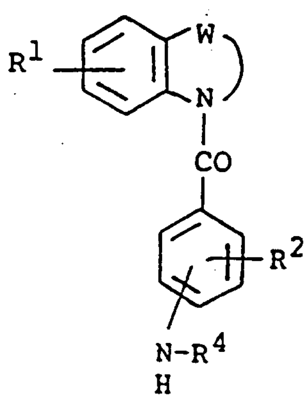

(2b)

wherein $R^1$, $R^2$, $R^4$, and W are as defined above, with a compound of the formula (38):

$$R^{46}N{=}C{=}O \quad (38)$$

wherein $R^{46}$ is as defined above.

**8.** A process for preparing a compound of the formula (1dd)

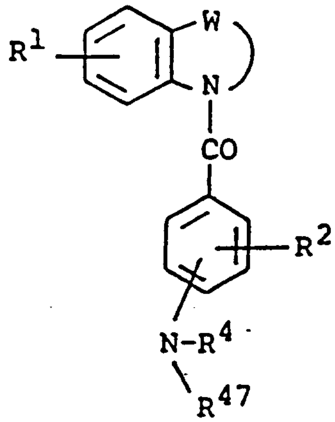

(1dd)

wherein $R^1$, $R^2$, $R^4$, and W are as defined in claim 1, and $R^{47}$ is a phenylsulfonyl which has optionally a substituent selected from a halogen atom and a $C_1$-$C_6$ alkyl on the phenyl ring, or quinolylsulfonyl, and X is halogen which comprises reacting a compound of the formula (2b):

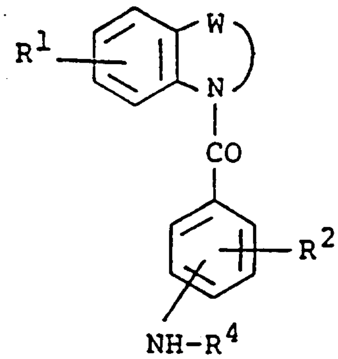

(2b)

wherein $R^1$, $R^2$, $R^4$, and W are as defined above, with a compound of the formula (39):

$$R^{47}X \quad (39)$$

wherein $R^{47}$ is as defined above.

9. A process for preparing a compound of the formula (1hh)

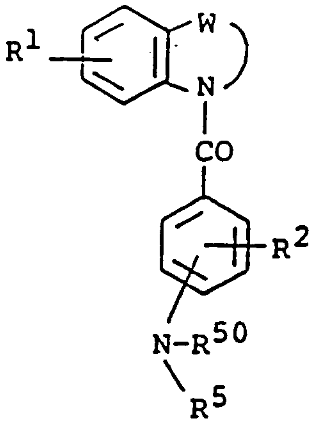

(1hh)

wherein $R^1$, $R^2$, $R^5$, and W are as defined in claim 1, and $R^{50}$ is a benzoyl having optionally a halogen substituent on the phenyl ring,

which comprises reacting a compound of the formula (7):

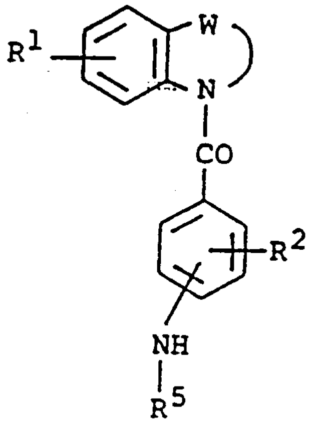

(7)

wherein $R^1$, $R^2$, $R^5$, and W are as defined above, with a compound of the formula (42):

$$R^{50}OH \quad (42)$$

wherein $R^{50}$ is as defined above.

10. The process according to claims 1 to 9, wherein $R^1$ in the formula (1) is hydrogen atom, or a salt thereof.

11. The process according to claims 1 to 9, wherein $R^1$ in the formula (1) is a halogen atom, and a salt thereof.

12. The process according to claims 1 to 9, wherein $R^1$ in the formula (1) is a $C_1$-$C_6$ alkyl, an amino having optionally a $C_1$-$C_6$ alkyl substituent, or a $C_1$-$C_6$ alkoxy, and a salt thereof.

13. The process according to claim 10, wherein $R^2$ is hydrogen atom, and a salt thereof.

14. The compound according to claim 10, wherein $R^2$ is a halogen atom, a $C_1$-$C_6$ alkoxy, or a $C_1$-$C_6$ alkyl, and a salt thereof.

15. The process according to claim 10, wherein $R^2$ is a phenyl-$C_1$-$C_6$ alkoxy; hydroxy; an amino having optionally a $C_1$-$C_6$ alkyl substituent; a carbamoyl-substituted $C_1$-$C_6$ alkoxy; an amino-substituted $C_1$-$C_6$ alkoxy having optionally a $C_1$-$C_6$ alkyl substituent; or a benzoyloxy having optionally a halogen substituent on the phenyl ring thereof, and a salt thereof.

**16.** The process according to claim 11, wherein $R^2$ is hydrogen atom, and a salt thereof.

**17.** The process according to claim 11, wherein $R^2$ is a halogen atom, a $C_1$-$C_6$ alkoxy, or a $C_1$-$C_6$ alkyl, and a salt thereof.

**18.** The process according to claim 11, wherein $R^2$ is a phenyl-$C_1$-$C_6$ alkoxy; hydroxy; an amino having optionally a $C_1$-$C_6$ alkyl substituent; a carbamoyl-substituted $C_1$-$C_6$ alkoxy; an amino-substituted $C_1$-$C_6$ alkoxy having optionally a $C_1$-$C_6$ alkyl substituent; or a benzoyloxy having optionally a halogen substituent on the phenyl ring thereof, and a salt thereof.

**19.** The process according to claim 12, wherein $R^2$ is hydrogen atom, and a salt thereof.

**20.** The process according to claim 12, wherein $R^2$ is a halogen atom, a $C_1$-$C_6$ alkoxy, or a $C_1$-$C_6$ alkyl, and a salt thereof.

**21.** The process according to claim 12, wherein $R^2$ is a phenyl-$C_1$-$C_6$ alkoxy; hydroxy; an amino having optionally a $C_1$-$C_6$ alkyl substituent; a carbamoyl-substituted $C_1$-$C_6$ alkoxy; an amino-substituted $C_1$-$C_6$ alkoxy having optionally a $C_1$-$C_6$ alkyl substituent; or a benzoyloxy having optionally a halogen substituent on the phenyl ring thereof, and a salt thereof.

**22.** The process according to claim 13, wherein $R^3$ is a group of the formula: $-NR^4R^5$ ($R^4$ and $R^5$ are as defined in claim 1), and a salt thereof.

**23.** The process according to claim 13, wherein $R^3$ is a group of the formula: $-CO-NR^{11}R^{12}$ ($R^{11}$ and $R^{12}$ are as defined in claim 1), and a salt thereof.

**24.** The process according to claim 14, wherein $R^3$ is a group of the formula: $-NR^4R^5$ ($R^4$ and $R^5$ are as defined in claim 1), and a salt thereof.

**25.** The process according to claim 14, wherein $R^3$ is a group of the formula: $-CO-NR^{11}R^{12}$ ($R^{11}$ and $R^{12}$ are as defined in claim 1), and a salt thereof.

**26.** The process according to claim 16, wherein $R^3$ is a group of the formula: $-NR^4R^5$ ($R^4$ and $R^5$ are as defined in claim 1), and a salt thereof.

**27.** The process according to claim 16, wherein $R^3$ is a group of the formula: $-CO-NA^{11}R^{12}$ ($R^{11}$ and $R^{12}$ are as defined in claim 1), and a salt thereof.

**28.** The process according to claim 17, wherein $R^3$ is a group of the formula: $-NR^4R^5$ ($R^4$ and $R^5$ are as defined in claim 1), and a salt thereof.

**29.** The process according to claim 17, wherein $R^3$ is a group of the formula: $-CO-NR^{11}R^{12}$ ($R^{11}$ and $R^{12}$ are as defined in claim 1), and a salt thereof.

**30.** The process according to claim 22, wherein $R^4$ is hydrogen atom, and $R^5$ is a group of the formula:

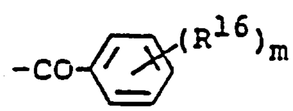

(wherein $R^{16}$ and m are as defined in claim 1), and a salt thereof.

**31.** The process according to claim 22, wherein $R^4$ is hydrogen atom and $R^5$ is a phenyl-$C_1$-$C_6$ alkoxycarbonyl, a $C_1$-$C_6$ alkanoyl a phenyl-$C_2$-$C_6$ alkanoyl, a $C_3$-$C_8$ cycloalkyl-$C_2$-$C_6$ alkanoyl, a $C_3$-$C_8$ cycloalkylcarbonyl, tricyclo[3.3.1.1]decanylcarbonyl, naphthylcarbonyl, pyridylcarbonyl, furoyl, thenoyl, a phenoxy-$C_2$-$C_6$ alkanoyl which phenyl ring has optionally 1 to 3 substituents selected from a $C_1$-$C_6$ alkyl, a $C_1$-$C_6$ alkoxy and an amino having optionally a $C_1$-$C_6$ alkanoyl substituent, a phthalimido-substituted $C_2$-$C_6$ alkanoyl, a $C_1$-$C_6$ alkoxycarbony-$C_2$-$C_6$ alkanoyl, a carboxy-$C_2$-$C_6$ alkanoyl, a naphthyloxy-$C_2$-$C_6$ alkanoyl, a halogen-substituted $C_1$-$C_6$ alkanoyl, a group

of the formula:

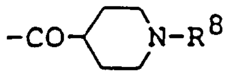

(wherein $R^8$ is hydrogen atom, a $C_1$-$C_6$ alkyl, a phenyl-$C_1$-$C_6$ alkoxycarbonyl, a carbamoyl-$C_1$-$C_6$ alkyl, an amino-$C_1$-$C_6$ alkanoyl having optionally a $C_1$-$C_6$ alkyl substituent, or a $C_1$-$C_6$ alkanoyl), an anilinocarbonyl which has optionally a $C_1$-$C_6$ alkyl substituent on the phenyl ring, phenoxycarbonyl, a phenylsulfonyl which has optionally a substituent selected from a halogen atom and a $C_1$-$C_6$ alkyl on the phenyl ring, quinolylsulfonyl, or a group of the formula:

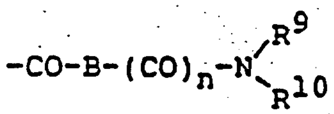

(wherein B is a $C_1$-$C_6$ alkylene, n is an integer of 0 or 1, and $R^9$ and $R^{10}$ are the same or different and are each hydrogen atom, a $C_1$-$C_6$ alkyl having optionally a hydroxy substituent, a $C_3$-$C_8$ cycloalkyl, a phenyl-$C_1$-$C_6$ alkyl, a $C_1$-$C_6$ alkanoyl, a $C_2$-$C_6$ alkenyl, a phenoxy-$C_1$-$C_6$ alkyl, a phenyl which has optionally 1 to 3 substituents selected from an amino-$C_1$-$C_6$ alkyl having optionally a $C_1$-$C_6$ alkanoyl substituent, a $C_1$-$C_6$ alkyl, a $C_1$-$C_6$ alkoxy and a halogen atom, a phthalimido-substituted $C_1$-$C_6$ alkyl, an amino-$C_1$-$C_6$ alkyl having optionally a $C_1$-$C_6$ alkanoyl substituent, a $C_2$-$C_6$ alkynyl, or an amino-$C_1$-$C_6$ alkyl having optionally a $C_1$-$C_6$ alkyl substituent, or $R^9$ and $R^{10}$ may bind together with nitrogen atom to which they bond to form a 5- or 6-membered saturated heterocyclic group with or without being intervened with nitrogen or oxygen atom wherein the heterocylic group has optionally a substituent selected from a $C_1$-$C_6$ alkyl, a $C_1$-$C_6$ alkoxycarbonyl and piperidinyl), and a salt thereof.

**32.** The process according to claim 22, wherein $R^4$ is a $C_1$-$C_6$ alkyl, and a salt thereof.

**33.** The process according to claim 24, wherein $R^4$ is hydrogen atom, and $R^5$ is a group of the formula:

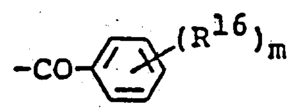

(wherein $R^{16}$ and m are as defined in claim 1), and a salt thereof.

**34.** The process according to claim 24, wherein $R^4$ is hydrogen atom and $R^5$ is a phenyl-$C_1$-$C_6$ alkoxycarbonyl, a $C_1$-$C_6$ alkanoyl, a phenyl-$C_1$-$C_6$ alkanoyl, a $C_3$-$C_8$ cycloalkyl-$C_2$-$C_6$ alkanoyl, a $C_3$-$C_8$ cycloalkylcarbonyl, tricyclo[3.3.1.1]decanylcarbonyl, naphthylcarbonyl, pyridylcarbonyl, furoyl, thenoyl, a phenoxy-$C_2$-$C_6$ alkanoyl which phenyl ring has optionally 1 to 3 substituents selected from a $C_1$-$C_6$ alkyl, a $C_1$-$C_6$ alkoxy and an amino having optionally a $C_1$-$C_6$ alkanoyl substituent, a phthalimido-substituted $C_2$-$C_6$ alkanoyl, a $C_1$-$C_6$ alkoxycarbonyl-$C_2$-$C_6$ alkanoyl, a carboxy-$C_2$-$C_6$ alkanoyl, a naphthyloxy-$C_2$-$C_6$ alkanoyl, a halogen-substituted $C_1$-$C_6$ alkanoyl, a group of the formula:

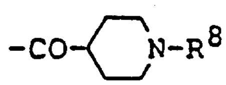

(wherein $R^8$ is hydrogen atom, a $C_1$-$C_6$ alkyl, a phenyl-$C_1$-$C_6$ alkoxycarbonyl, a carbamoyl-$C_1$-$C_6$ alkyl, an amino-$C_1$-$C_6$ alkanoyl having optionally a $C_1$-$C_6$ alkyl substituent, or a $C_1$-$C_6$ alkanoyl), an anilinocarbonyl which has optionally a $C_1$-$C_6$ alkyl substituent on the phenyl ring, phenoxycarbonyl, a phenylsulfonyl which has optionally a substituent selected from a halogen atom and a $C_1$-$C_6$ alkyl on the phenyl ring, quinolylsulfonyl, or a group of the formula:

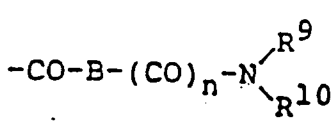

(wherein B is a $C_2$-$C_6$ alkylene, n is an integer of 0 or 1, and $R^9$ and $R^{10}$ are the same or different and are each hydrogen atom, a $C_1$-$C_6$ alkyl having optionally a hydroxy substituent, a $C_3$-$C_8$ cycloalkyl, a phenyl-$C_1$-$C_6$ alkyl, a $C_1$-$C_6$ --alkanoyl, a $C_2$-$C_6$ alkenyl, a phenoxy-$C_1$-$C_6$ alkyl, a phenyl which has optionally 1 to 3 substituents selected from an amino-$C_1$-$C_6$ alkyl having optionally a $C_1$-$C_6$ alkanoyl substituent, a $C_1$-$C_6$ alkyl, a $C_1$-$C_6$ alkoxy and a halogen atom, a phthalimido-substituted $C_1$-$C_6$ alkyl, an amino-$C_1$-$C_6$ alkyl having optionally a $C_1$-$C_6$ alkanoyl substituent, a $C_2$-$C_6$ alkynyl, or an amino-$C_1$-$C_6$ alkyl having optionally a $C_1$-$C_6$ alkyl substituent, or $R^9$ and $R^{10}$ may bind together with nitrogen atom to which they bond to form a 5- or 6-membered saturated heterocyclic group with or without being intervened with nitrogen or oxygen atom wherein the heterocylic group has optionally a substituent selected from a $C_1$-$C_6$ alkyl, a $C_1$-$C_6$ alkoxycarbonyl and piperidinyl), and a salt thereof.

**35.** The process according to claim 24, wrhein $R^4$ is a $C_1$-$C_6$ alkyl, and a salt thereof.

**36.** The process according to claim 15, wherein $R^4$ is hydrogen atom, and $R^5$ is a group of the formula:

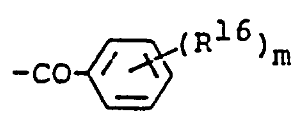

(wherein $R^{16}$ and m are as defined in claim 1), and a salt thereof.

**37.** The process according to claim 5 15, 18 to 21, 25 to 27, wherein $R^4$ is hydrogen atom and $R^5$ is as defined in claim 31.

**38.** The process according to claim 15, wrhein $R^4$ is a $C_1$-$C_6$ alkyl, and a salt thereof.

**39.** The process according to claim 26, wherein $R^4$ is hydrogen atom, and $R^5$ is a group of the formula:

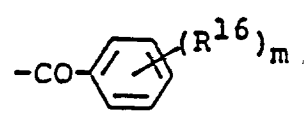

(wherein $R^{16}$ and m are as defined in claim 1), and a salt thereof.

**40.** The process according to claim 26, wrhein $R^4$ is a $C_1$-$C_6$ alkyl, and a salt thereof.

**41.** The process according to claim 28, wherein $R^4$ is hydrogen atom, and $R^5$ is a group of the formula:

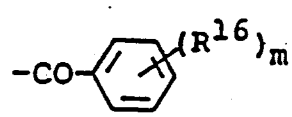

(wherein $R^{16}$ and m are as defined in claim 1), and a salt thereof.

**42.** The process according to claim 28, wrhein $R^4$ is a $C_1$-$C_6$ alkyl, and a salt thereof.

**43.** The process according to claim 18, wherein $R^4$ is hydrogen atom, and $R^5$ is a group of the formula:

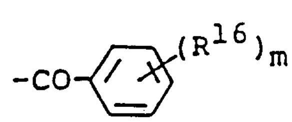

(wherein $R^{16}$ and m are as defined in claim 1), and a salt thereof.

**44.** The process according to claim 18, wrhein $R^4$ is a $C_1$-$C_6$ alkyl, and a salt thereof.

**45.** The process according to claim 17, wherein $R^4$ is hydrogen atom, and $R^5$ is a group of the formula:

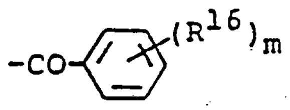

(wherein $R^{16}$ and m are as defined in claim 1), and a salt thereof.

**46.** The process according to claim 19, wherein $R^4$ is a $C_1$-$C_6$ alkyl, and a salt thereof.

**47.** The process according to claim 20, wherein $R^4$ is hydrogen atom, and $R^5$ is a group of the formula:

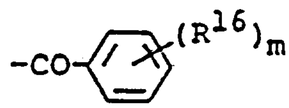

(wherein $R^{16}$ and m are as defined in claim 1), and a salt thereof.

**48.** The process according to claim 20, wherein $R^4$ is a $C_1$-$C_6$ alkyl, and a salt thereof.

**49.** The process according to claim 21, wherein $R^4$ is hydrogen atom, and $R^5$ is a group of the formula:

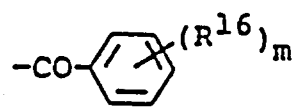

(wherein $R^{16}$ and m are as defined in claim 1), and a salt thereof.

**50.** The process according to claim 21, wherein $R^4$ is a $C_1$-$C_6$ alkyl, and a salt thereof.

**51.** The process according to claim 30, wherein $R^{16}$ is a halogen atom, or a $C_1$-$C_6$ alkyl having optionally a substituent selected from a halogen atom and hydroxy, and a salt thereof.

**52.** The process according to claim 33, wherein $R^{16}$ is a halogen atom, or a $C_1$-$C_6$ alkyl having optionally a substituent selected from a halogen atom and hydroxy, and a salt thereof.

**53.** The process according to claim 39, wherein $R^{16}$ is a halogen atom, or a $C_1$-$C_6$ alkyl having optionally a substituent selected from a halogen atom and hydroxy, and a salt thereof.

**54.** The process according to claim 42, wherein $R^{16}$ is a halogen atom, or a $C_1$-$C_6$ alkyl having optionally a substituent selected from a halogen atom and hydroxy, and a salt thereof.

**55.** The process according to claims 1 to 9, wherein W is a group of the formula: $-(CH_2)_p-$ wherein p is an integer of 3 to 5, and further said $-(CH_2)_p-$ group has optionally 1 to 3 substituents as defined in claim 1.

**56.** The process according to claims 1 to 9, wherein W is a group of the formula: $-CH=CH-(CH_2)_q-$ wherein q is an integer of 1 to 3, and further said $-CH=CH-(CH_2)_q-$ group has optionally 1 to 3 substituents selected from a $C_1$-$C_6$ alkyl having optionally a hydroxy substituent, and an amino-$C_1$-$C_6$ alkyl having optionally a substituent selected from a $C_1$-$C_6$ alkyl and a $C_1$-$C_6$ alkanoyl, and a salt thereof.

**57.** The process according to claim 55, wherein W is a group of the formula: $-(CH_2)_p-$ (p is an integer of 3 to 5) and said $-(CH_2)_p-$ group has optionally 1 to 3 substituents as defined in claim 1.

**58.** The process according to claim 57, wherein p in the group: $-(CH_2)_p-$ is 3 and the group has no substituent, and a salt thereof.

**59.** The process according to claim 57, wherein p in the group: $-(CH_2)_p-$ is 3 and the group has a substituent of a group of the formula:

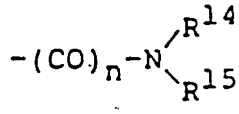

(wherein $R^{14}$, $R^{15}$, and n is as defined above), and a salt thereof.

**60.** The process according to claim 57, wherein p in the group: $-(CH_2)_p-$ is 4 and the group has no substituent, and a salt thereof.

**61.** The process according to claim 57, wherein p in the group: $-(CH_2)_p-$ is 4 and the group has a substituent of a group of the formula:

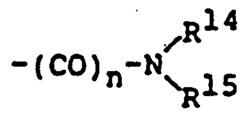

(wherein $R^{14}$, $R^{15}$, and n is as defined above), and a salt thereof.

**62.** The process according to claim 57, wherein p in the group: $-(CH_2)_p-$ is 5, and a salt thereof.

**63.** The process according to claim 56, wherein q in the group: $-CH=CH-(CH_2)_q-$ is 1, and a salt thereof.

**64.** The process according to claim 56, wherein q in the group: $-CH=CH-(CH_2)_q-$ is 2, and a salt thereof.

**65.** The process according to claim 56, wherein q in the group: $-CH=CH=(CH_2)_q-$ is 3, and a salt thereof.

**66.** The process according to claim 59 or 61, wherein n in the substituent:

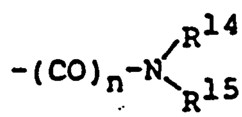

is 0, and $R^{14}$ and $R^{15}$ are the same or different and are each hydrogen atom, a $C_1$-$C_6$ alkyl, or a $C_3$-$C_8$ cycloalkyl, and a salt thereof.

**67.** The process according to claim 64 wherein the heterocyclic group of the formula:

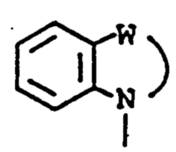

is 2,3-dihydro-1H-benzazepine, and a salt thereof.

**68.** The process according to claims 1 to 9 for the manufacture of

1-[4-(2-Methylbenzoylamino)benzoyl]-4-methyl-2,3,4,5-tetrahydro-1H-1,4-benzodiazepine,
5-Dimethylamino-1-[4-(2-methylbenzoylamino)benzoyl]-2,3,4,5-tetrahydro-1H-benzazepine,
5-Dimethylamino-1-[2-chloro-4-(2-methylbenzoylamino)benzoyl]-2,3,4,5-tetrahydro-1H-benzazepine,
5-Methylamino-1-[2-chloro-4-(2-methylbenzoylamino)benzoyl]-2,3,4,5-tetrahydro-1H-benzazepine,
5-Cyclopropylamino-1-[2-chloro-4-(2-methylbenzoylamino)benzoyl]-2,3,4,5-tetrahydro-1H-benzazepine,

5-Cyclopropylamino-1-[2-chloro-4-(2-chlorobenzoylamino)benzoyl]-2,3,4,5-tetrahydro-1H-benzazepine,
5-Dimethylamino-1-[2-methyl-4-(2-methylbenzoylamino)benzoyl]-2,3,4,5-tetrahydro-1H-benzazepine,
4-Dimethylamino-1-[3-methoxy-4-(2-methylbenzoylamino)benzoyl]-1,2,3,4-tetrahydroquinoline,
7-Chloro-5-methylamino-1-[4-(2-methylbenzoylamino)benzoyl]-2,3,4,5-tetrahydro-1H-benzazepine,
7-Chloro-5-methylamino-1-[4-(2-chlorobenzoylamino)benzoyl]-2,3,4,5-tetrahydro-1H-benzazepine,
5-Hydroxysulfonyloxyimino-1-[2-chloro-4-(2-methylbenzoylamino)benzoyl]-2,3,4,5-tetrahydro-1H-benzazepine.

**69.** The process according to claims 1 to 9 for the manufacture of compounds of the formulae

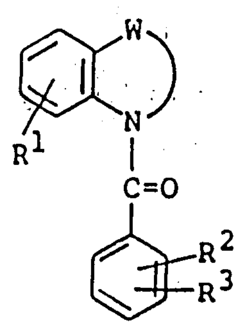

in which

a)

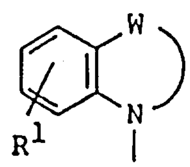

represents

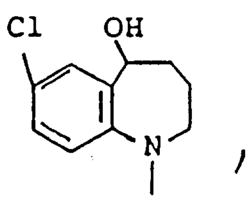

$R^2$ represents 2-$OCH_3$ and
$R^3$ represents

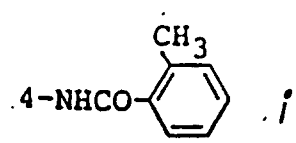

and

b)

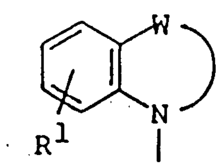

represents

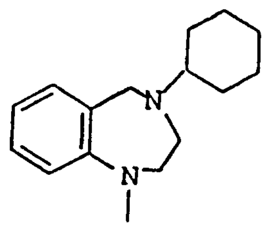

$R^2$ represents H and
$R^3$ represents

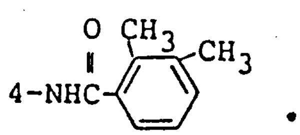

**70.** Process according to claims 1 to 9 for the manufacture of a benzoheterocyclic compound of the formula:

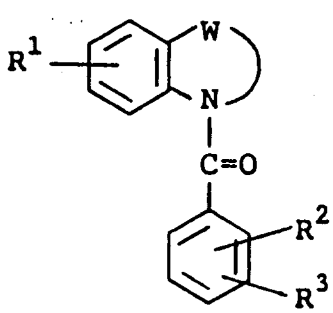

wherein $R^1$, $R^2$ and $R^3$ are as defined in claim 1, and the heterocyclic group of the formula:

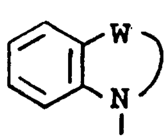

is a member selected from the group consisting of 3,4-dihydro-2H-1,4-benzoxazinyl, 1,2,3,5-tetrahydro-4,1-benzoxazepinyl, 1,2,3,4-tetrahydroquinoxalinyl, 1,2,3,4,5,6-hexahydro-1-5-benzodiazocinyl, 5-methyl-1,2,3,4,5,6-hexahydro-1,5-benzodiazocinyl, 4-methyl-1,2,3,4-tetrahydroquinoxalinyl, 1,2,3,4-tetrahydro-5,1-benzoxazepinyl, 3,4-dihydro-2H-1,4-benzothiazinyl, 2,3,4,5-tetrahydro-1,5-benzothiazepinyl, 1,2,3,5-tetrahydro-4,1-benzothiazepinyl,4-ethyl-1,2,3,4-tetrahydroquinoxalinyl, 4-propyl-1,2,3,4-tetrahydroquinoxalinyl, 4-butyl-1,2,3,4-tetrahydroquinoxalinyl, 4-pentyl-1,2,3,4-tetrahydroquinoxalinyl, 4-hexyl-1,2,3,4-tetrahydroquinoxalinyl, 2,3,4,5-tetrahydro-1H-1,4-benzodiazopinyl, 4-methyl-2,3,4,5-tetrahydro-1H-1,4-benzodiazepinyl, 4-ethyl-2,3,4,5-tetrahydro-1H-1,4-benzodiazepinyl,4-propyl-2,3,4,5-tetrahydro-1H-1,4-benzodiazepinyl, 4-butyl-2,3,4,5-tetrahydro-1H-1,4-benzodiazepinyl, 4-pentyl-2,3,4,5-tetrahydro-1H-1,4-benzodiazepinyl, 4-hexyl-2,3,4,5-tetrahydro-1H-1,4-benzodiazepinyl, 2,3,4,5-tetrahydro-1H-1,5-benzodiazepinyl, 5-methyl-2,3,4,5-tetrahydro-1H-1,5-benzodiazepinyl, 5-ethyl-2,3,4,5-tetrahydro-1H-1,5-benzodiazepinyl,5-propyl-2,3,4,5-tetrahydro-1H-1,5-benzodiazepinyl, 5-butyl-2,3,4,5-tetrahydro-1H-1,5-benzodiazepinyl, 5-pentyl-2,3,4,5-tetrahydro-1H-1,5-benzodiazepinyl, 5-hexyl-2,3,4,5-tetrahydro-1H-1,5-benzodiazepinyl, 3,4-dihydro-1-oxo-2H-1,4-benzothiazepinyl, 3,4-dihydro-1,1-dioxo-2H-1,4-benzothiazepinyl, 1-oxo-2,3,4,5-tetrahydro-1,5-benzothiazepinyl, 1,1-dioxo-2,3,4,5-tetrahydro-1,5-benzothiazepinyl, 4-oxo-1,2,3,5-tetrahydro-4,1-benzothiazepinyl, and 4,4-dioxo-1,2,3,5-tetrahydro-4,1-benzothiazepinyl,
and a salt thereof

71. Process for the production of a vasopressin antagonistic composition which comprises as an active ingredient a compound of the formula (1) prepared according to the process of claims 1 to 9, or a pharmaceutically acceptable salt thereof by mixing it with a pharmaceutically acceptable carrier or diluent.

## Patentansprüche

### Patentansprüche für folgende Vertragsstaaten : CH, DE, DK, FR, GB, IT, LI, NL, SE

1. Benzoheterocyclische Verbindung der folgenden Formel:

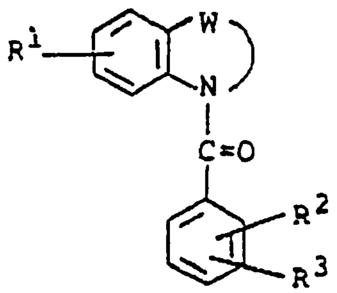

(1)

worin $R^1$ ein Wasserstoffatom, ein Halogenatom, $C_1$-$C_6$-Alkyl, Amino mit gegebenenfalls einem $C_1$-$C_6$-Alkylsubstituenten oder $C_1$-$C_6$-Alkoxy ist,
$R^2$ ein Wasserstoffatom, ein Halogenatom, $C_1$-$C_6$-Alkoxy, Phenyl($C_1$-$C_6$)alkoxy, Hydroxy, $C_1$-$C_6$-Alkyl, Amino mit gegebenenfalls einem $C_1$-$C_6$-Alkylsubstituenten, carbamoylsubstituiertes $C_1$-$C_6$-Alkoxy, aminosubstituiertes $C_1$-$C_6$-Alkoxy mit gegebenenfalls einem $C_1$-$C_6$-Alkylsubstituenten oder Benzoyloxy ist, das gegebenenfalls einen Halogensubstituenten am Phenylring besitzt,
$R^3$ eine Gruppe der Formel

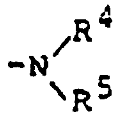

oder eine Gruppe der Formel

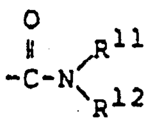

ist,
$R^4$ ein Wasserstoffatom, Benzoyl, das gegebenenfalls einen Halogensubstituenten am Phenylring besitzt, oder $C_1$-$C_6$-Alkyl ist,

$R^5$ (a) eine Gruppe der Formel

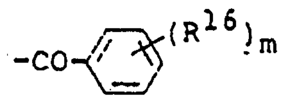

[worin $R^{16}$ (i) ein Halogenatom, (ii) $C_1$-$C_6$-Alkyl, das gegebenenfalls einen Substituenten besitzt, der aus einem Halogenatom und Hydroxy ausgewählt ist, (iii) Hydroxy, (iv) $C_1$-$C_6$-Alkoxy, (v) $C_1$-$C_6$-Alkanoyloxy, (vi) $C_1$-$C_6$-Alkylthio, (vii) $C_1$-$C_6$-Alkanoyl, (viii) Carboxy, (ix) $C_1$-$C_6$-Alkoxycarbonyl, (x) Cyano, (xi) Nitro, (xii) Amino, das gegebenenfalls einen Substituenten besitzt, der aus $C_1$-$C_6$-Alkyl und $C_1$-$C_6$-Alkanoyl ausgewählt ist, (xiii) Phenyl, (xiv) $C_3$-$C_8$-Cycloalkyl, (xv) $C_2$-$C_6$-alkanoyloxysubstituiertes $C_1$-$C_6$-Alkoxy, (xvi) carboxysubstituiertes $C_1$-$C_6$-Alkoxy, (xvii) halogensubstituiertes $C_1$-$C_6$-Alkoxy, (xviii) carbamoylsubstituiertes $C_1$-$C_6$-Alkoxy, (xix) hydroxysubstituiertes $C_1$-$C_6$-Alkoxy, (xx) $C_1$-$C_6$-alkoxycarbonylsubstituiertes $C_1$-$C_6$-Alkoxy, (xxi) phthalimidosubstituiertes $C_1$-$C_6$-Alkoxy, (xxii) Aminocarbonyl-$C_1$-$C_6$-alkoxy mit einem $C_1$-$C_6$-Alkylsubstituenten oder (xxiii) eine Gruppe der Formel

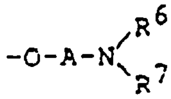

ist
(A ist Alkylen mit bis zu 6 Kohlenstoffatomen und
$R^6$ und $R^7$ sind gleich oder verschieden und sind jeweils ein Wasserstoffatom, $C_1$-$C_6$-Alkyl mit gegebenenfalls einem Hydroxysubstituenten, $C_1$-$C_6$-Alkanoyl oder Benzoyl oder
$R^6$ und $R^7$ können sich zusammen mit dem Stickstoffatom, an das sie binden, unter Bilden einer 5- oder 6-gliedrigen, gesättigten, heterocyclischen Gruppe verbinden, die durch ein Stickstoff- oder Sauerstoffatom unterbrochen ist oder nicht, wobei die heterocyclische Gruppe gegebenenfalls einen Substituenten besitzt, der aus Piperidinyl und $C_1$-$C_6$-Alkyl ausgewählt ist),
und m eine ganze Zahl von 0 bis 3 ist],

(b) Phenyl-$C_1$-$C_6$-alkoxycarbonyl,
(c) $C_1$-$C_6$-Alkanoyl,
(d) Phenyl-$C_2$-$C_6$-alkanoyl,
(e) $C_3$-$C_8$-Cycloalkyl-$C_2$-$C_6$-alkanoyl,
(f) $C_3$-$C_8$-Cyloalkylcarbonyl,
(g) Tricyclo[3.3.1.1]decanylcarbonyl,
(h) Naphthylcarbonyl,
(i) Pyridylcarbonyl,
(j) Furoyl,
(k) Thenoyl,
(l) Phenoxy-$C_2$-$C_6$-alkanoyl, wobei der Phenylring gegebenenfalls 1 bis 3 Substituenten besitzt, die aus $C_1$-$C_6$-Alkyl, $C_1$-$C_6$-Alkoxy und Amino mit gegebenenfalls einem $C_1$-$C_6$-Alkanoylsubstituenten ausgewählt sind,
(m) phthalimidosubstituiertes $C_2$-$C_6$-Alkanoyl,
(o) $C_1$-$C_6$-Alkoxycarbonyl-$C_2$-$C_6$-alkanoyl,
(p) Carboxy-$C_2$-$C_6$-alkanoyl,
(q) Naphthyloxy-$C_2$-$C_6$-alkanoyl,
(r) halogensubstituiertes $C_1$-$C_6$-Alkanoyl,
(s) eine Gruppe der Formel

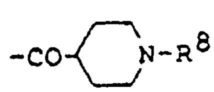

(worin $R^8$ (i) ein Wasserstoffatom, (ii) $C_1$-$C_6$-Alkyl, (iii) Phenyl-$C_1$-$C_6$-alkoxycarbonyl, (iv) Carbamoyl-$C_1$-$C_6$-alkyl, (v) Amino-$C_2$-$C_6$-alkanoyl mit gegebenenfalls einem $C_1$-$C_6$-Alkylsubstituenten oder (vi) $C_1$-$C_6$-Alkanoyl ist),

(t) Anilinocarbonyl, das gegebenenfalls einen $C_1$-$C_6$-Alkylsubstituenten am Phenylring besitzt,

(u) Phenoxycarbonyl,

(v) Phenylsulfonyl, das gegebenenfalls einen Substituenten besitzt, der aus einem Halogenatom und $C_1$-$C_6$-Alkyl am Phenylring ausgewählt ist,

(w) Chinolylsulfonyl oder

(x) eine Gruppe der Formel

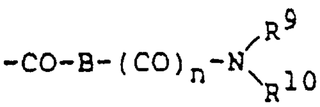

ist, worin B $C_1$-$C_6$-Alkylen ist, n eine ganze Zahl 0 oder 1 ist und $R^9$ und $R^{10}$ gleich oder verschieden sind und jeweils (i) ein Wasserstoffatom, (ii) Alkyl mit bis zu 6 Kohlenstoffatomen mit gegebenenfalls einem Hydroxysubstituenten, (iii) $C_3$-$C_8$-Cycloalkyl, (iv) Phenyl-$C_1$-$C_6$-alkyl, (v) $C_1$-$C_6$-Alkanoyl, (vi) Alkenyl mit bis zu 6 Kohlenstoffatomen, (vii) Phenoxy-$C_1$-$C_6$-alkyl, (viii) Phenyl, das gegebenenfalls 1 bis 3 Substituenten besitzt, die aus Amino-$C_1$-$C_6$-alkyl mit gegebenenfalls einem $C_1$-$C_6$-Alkanoylsubstituenten, $C_1$-$C_6$-Alkyl, $C_1$-$C_6$-Alkoxy und einem Halogenatom ausgewählt sind, (ix) phthalimidosubstituiertes $C_1$-$C_6$-Alkyl, (x) Amino-$C_1$-$C_6$-alkyl mit gegebenenfalls einem $C_1$-$C_6$-Alkanoylsubstituenten, (xi) Alkinyl mit bis zu 6 Kohlenstoffatomen oder (xii) Amino-$C_1$-$C_6$-alkyl mit gegebenenfalls einem $C_1$-$C_6$-Alkylsubstituenten, oder $R^9$ und $R^{10}$ sich zusammen mit dem Stickstoffatom, an das sie binden, unter Bilden einer 5- oder 6-gliedrigen, gesättigten, heterocyclischen Gruppe verbinden, die durch ein Stickstoff- oder Sauerstoffatom unterbrochen ist oder nicht, wobei die heterocyclische Gruppe gegebenenfalls einen Substituenten besitzt, der aus $C_1$-$C_6$-Alkyl, $C_1$-$C_6$-Alkoxycarbonyl und Piperidinyl ausgewählt ist),

$R^{11}$ ein Wasserstoffatom oder $C_1$-$C_6$-Alkyl ist,

$R^{12}$ $C_3$-$C_8$-Cycloalkyl oder Phenyl ist, das gegebenenfalls 1 bis 3 Substituenten besitzt, die aus $C_1$-$C_6$-Alkoxy, $C_1$-$C_6$-Alkyl und einem Halogenatom ausgewählt sind,

W eine Gruppe der Formel $-(CH_2)_p-$ (p ist eine ganze Zahl 3 bis 5) oder eine Gruppe der Formel $-CH=CH-(CH_2)_q-$ ist (q ist eine ganze Zahl 1 bis 3) oder worin die heterocyclische Gruppe der Formel

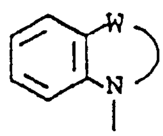

2,3,4,5-Tetrahydro-1H-1,4-benzodiazepin ist,

und weiter die $-(CH_2)_p-$Gruppe gegebenenfalls 1 bis 3 Substituenten besitzt, die aus (i) $C_1$-$C_6$-Alkyl mit gegebenenfalls einem Hydroxysubstituenten, (ii) $C_1$-$C_6$-Alkoxycarbonyl, (iii) Carboxy, (iv) Hydroxy, (v) Oxo, (vi) $C_1$-$C_6$-Alkanoyloxy mit gegebenenfalls einem Halogensubstituenten, (vii) Amino-$C_1$-$C_6$-alkyl mit gegebenenfalls einem Substituenten, der aus $C_1$-$C_6$-Alkyl und $C_1$-$C_6$-Alkanoyl ausgewählt ist, (viii) $C_2$-$C_6$-alkanoyloxysubstituiertem $C_1$-$C_6$-Alkyl, (ix) $C_1$-$C_6$-Alkylsulfonyloxy-$C_1$-$C_6$-alkyl, (x) Azido-$C_1$-$C_6$-alkyl, (xi) einer Gruppe der Formel $-O-CH_2-$ (die an die Gruppe $-(CH_2)_p-$ oder $-CH=CH-(CH_2)_q-$ in Spiroform gebunden ist), (xii) Aminocarbonyloxy mit gegebenenfalls einem $C_1$-$C_6$-Alkylsubstituenten, (xiii) $C_1$-$C_6$-Alkoxy, (xiv) $C_1$-$C_6$-alkoxycarbonylsubstituiertem $C_1$-$C_6$-Alkoxy, (xv) carboxysubstituiertem $C_1$-$C_6$-Alkoxy, (xvi) Aminocarbonyl-$C_1$-$C_6$-alkoxy mit gegebenenfalls einem $C_1$-$C_6$-Alkylsubstituenten, (xvii) Amino-$C_1$-$C_6$-alkoxymit gegebenenfalls einem aus $C_1$-$C_6$-Alkyl und $C_1$-$C_6$-Alkanoyl ausgewählten Substituenten, (xviii) phthalimidosubstituiertem $C_1$-$C_6$-Alkoxy, (xix) Hydroxyimino, (xx) $C_1$-$C_6$-Alkanoyloxyimino, (xxi) $C_1$-$C_6$-Alkyliden, (xxii) einem Halogenatom, (xxiii) Azido, (xxvi) Hydrazino, (xxvii) Pyrrolyl, (xxviii) Amino-$C_1$-$C_6$-alkanoyloxy mit gegebenenfalls einem $C_1$-$C_6$-Alkylsubstituenten, (xxix) einer Gruppe der Formel

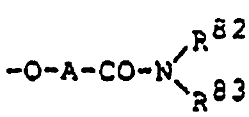

(A ist wie vorstehend definiert und $R^{82}$ und $R^{83}$ sind gleich oder verschieden und sind jeweils ein Wasserstoffatom, $C_1$-$C_6$-Alkyl, carbamoylsubstituiertes $C_1$-$C_6$-Alkyl, hydroxysubstituiertes $C_1$-$C_6$-Alkyl oder Pyridyl-$C_1$-$C_6$-alkyl,

oder $R^{82}$ und $R^{83}$ können sich zusammen mit dem Stickstoffatom, an das sie binden, unter Bilden einer 5- oder 6-gliedrigen, gesättigten, heterocyclischen Gruppe verbinden, die durch ein Stickstoff-, Sauerstoff- oder Schwefelatom unterbrochen ist oder nicht, wobei die heterocyclische Gruppe gegebenenfalls einen Substituenten besitzt, der aus Oxo, $C_1$-$C_6$-Alkyl, $C_1$-$C_6$-Alkanoyl und Carbamoyl ausgewählt ist), und (xxx) einer Gruppe der Formel

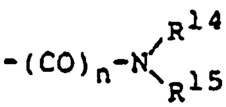

ausgewählt ist (worin n wie vorstehend definiert ist und $R^{14}$ und $R^{15}$ gleich oder verschieden sind und jeweils (i) ein Wasserstoffatom, (ii) $C_1$-$C_6$-Alkyl, (iii) Alkenyl mit bis zu 6 Kohlenstoffatomen, (iv) $C_1$-$C_6$-Alkanoyl, (v) $C_3$-$C_8$-Cycloalkyl, (vi) oxiranylsubstituiertes $C_1$-$C_6$-Alkyl, (vii) $C_1$-$C_6$-Alkyl mit 1 bis 2 Substituenten, die aus $C_1$-$C_6$-Alkoxy, Hydroxy und einer Aminogruppe mit gegebenenfalls einem $C_1$-$C_6$-Alkylsubstituenten ausgewählt sind, (viii) Phenyl-$C_1$-$C_6$-alkyl, (ix) Pyridyl-$C_1$-$C_6$-alkyl, (x) $C_1$-$C_6$-Alkylsulfonyl, (xi) Benzoyl, (xii) $C_1$-$C_6$-Alkoxycarbonyl, (xiii) Anilinocarbonyl, (xiv) Aminocarbonyl mit gegebenenfalls einem $C_1$-$C_6$-Alkylsubstituenten, (xv) cyanosubstituiertes $C_1$-$C_6$-Alkyl, (xvi) $C_1$-$C_6$-alkoxycarbonylsubstituiertes $C_1$-$C_6$-Alkyl, (xvii) carbamoylsubstituiertes $C_1$-$C_6$-Alkyl, (xviii) carboxysubstituiertes $C_1$-$C_6$-Alkyl, (xix) tetrahydropyranyloxysubstituiertes $C_1$-$C_6$-Alkyl, (xx) $C_2$-$C_6$-alkanoyloxysubstituiertes $C_1$-$C_6$-Alkyl, (xxi) Piperidinyl mit gegebenenfalls einem Phenyl-$C_1$-$C_6$-alkylsubstituenten am Piperidinylring, (xxii) halogensubstituiertes $C_2$-$C_6$-Alkanoyl, (xxiii) imidazolylsubstituiertes $C_2$-$C_6$-Alkanoyl, (xxiv) Amino-$C_2$-$C_6$-alkanoyl mit gegebenenfalls einem Substituenten, der aus $C_1$-$C_6$-Alkyl und $C_1$-$C_6$-Alkoxycarbonyl ausgewählt ist, (xxv) Aminocarbonyl-$C_1$-$C_6$-alkyl mit gegebenenfalls einem $C_1$-$C_6$-Alkylsubstituenten oder (xxvi) Phenyl-$C_1$-$C_6$-alkoxycarbonyl sind oder $R^{14}$ und $R^{15}$ können sich zusammen mit dem Stickstoffatom, an das sie binden, unter Bilden einer 5- oder 6-gliedrigen, gesättigten, heterocyclischen Gruppe verbinden, die durch Stickstoff oder Sauerstoff unterbrochen ist oder nicht, wobei die heterocyclische Gruppe gegebenenfalls einen Substituenten besitzen kann, der aus $C_1$-$C_6$-Alkyl, Phenyl-$C_1$-$C_6$-alkyl oder $C_1$-$C_6$-Alkanoyl ausgewählt ist und weiter die $-CH=CH-(CH_2)_q$-Gruppe gegebenenfalls 1 bis 3 Substituenten besitzt, die aus

(i) $C_1$-$C_6$-Alkyl mit gegebenenfalls einem Hydroxysubstituenten und
(ii) Amino-$C_1$-$C_6$-alkyl mit gegebenenfalls einem Substituenten ausgewählt sind, der aus $C_1$-$C_6$-Alkyl und $C_1$-$C_6$-Alkanoyl ausgewählt ist,

und ein Salz derselben.

**2.** Verbindung gemäß Anspruch 1, wobei $R^1$ in der Formel (1) ein Wasserstoffatom ist, oder ein Salz derselben.

**3.** Verbindung gemäß Anspruch 1, wobei $R^1$ in der Formel (1) ein Halogenatom ist, und ein Salz derselben.

**4.** Verbindung gemäß Anspruch 1, wobei $R^1$ in der Formel (1) $C_1$-$C_6$-Alkyl, Amino mit gegebenenfalls einem $C_1$-$C_6$-Alkylsubstituenten oder $C_1$-$C_6$-Alkoxy ist, und ein Salz derselben.

**5.** Verbindung gemäß Anspruch 2, wobei $R^2$ ein Wasserstoffatom ist, und ein Salz derselben.

**6.** Verbindung gemäß Anspruch 2, wobei $R^2$ ein Halogenatom, $C_1$-$C_6$-Alkoxy oder $C_1$-$C_6$-Alkyl ist, und ein Salz derselben.

**7.** Verbindung gemäß Anspruch 2, wobei $R^2$ Phenyl-$C_1$-$C_6$-alkoxy, Hydroxy, Amino mit gegebenenfalls einem $C_1$-$C_6$-Alkylsubstituenten, carbamoylsubstituiertes $C_1$-$C_6$-Alkoxy, aminosubstituiertes $C_1$-$C_6$-Alkoxy mit gegebenenfalls einem $C_1$-$C_6$-Alkylsubstituenten oder Benzoyloxy mit gegebenenfalls einem Halogensubstituenten an dessen Phenylring ist, und ein Salz derselben.

**8.** Verbindung gemäß Anspruch 3, wobei $R^2$ ein Wasserstoffatom ist, und ein Salz derselben.

**9.** Verbindung gemäß Anspruch 3, wobei $R^2$ ein Halogenatom, $C_1$-$C_6$-Alkoxy oder $C_1$-$C_6$-Alkyl ist, und ein Salz derselben.

**10.** Verbindung gemäß Anspruch 3, wobei $R^2$ Phenyl-$C_1$-$C_6$-alkoxy, Hydroxy, Amino mit gegebenenfalls einem $C_1$-$C_6$-Alkylsubstituenten, carbamoylsubstituiertes $C_1$-$C_6$-Alkoxy, aminosubstituiertes $C_1$-$C_6$-Alkoxy mit gegebenenfalls

einem $C_1$-$C_6$-Alkylsubstituenten oder Benzoyloxy mit gegebenenfalls einem Halogensubstituenten an dessen Phenylring ist, und ein Salz derselben.

**11.** Verbindung gemäß Anspruch 4, wobei $R^2$ ein Wasserstoffatom ist, und ein Salz derselben.

**12.** Verbindung gemäß Anspruch 4, wobei $R^2$ ein Halogenatom, $C_1$-$C_6$-Alkoxy oder $C_1$-$C_6$-Alkyl ist, und ein Salz derselben.

**13.** Verbindung gemäß Anspruch 4, wobei $R^2$ Phenyl-$C_1$-$C_6$-alkoxy, Hydroxy, Amino mit gegebenenfalls einem $C_1$-$C_6$-Alkylsubstituenten, carbamoylsubstituiertes $C_1$-$C_6$-Alkoxy, aminosubstituiertes $C_1$-$C_6$-Alkoxy mit gegebenenfalls einem $C_1$-$C_6$-Alkylsubstituenten oder Benzoyloxy mit gegebenenfalls einem Halogensubstituenten an dessen Phenylring ist, und ein Salz derselben.

**14.** Verbindung gemäß Anspruch 5, wobei $R^3$ eine Gruppe der Formel $-NR^4R^5$ ist ($R^4$ und $R^5$ sind wie in Anspruch 1 definiert), und ein Salz derselben.

**15.** Verbindung gemäß Anspruch 5, wobei $R^3$ eine Gruppe der Formel $-CO-NR^{11}R^{12}$ ist ($R^{11}$ und $R^{12}$ sind wie in Anspruch 1 definiert), und ein Salz derselben.

**16.** Verbindung gemäß Anspruch 6, wobei $R^3$ eine Gruppe der Formel $-NR^4R^5$ ist ($R^4$ und $R^5$ sind wie in Anspruch 1 definiert), und ein Salz derselben.

**17.** Verbindung gemäß Anspruch 6, wobei $R^3$ eine Gruppe der Formel $-CO-NR^{11}R^{12}$ ist ($R^{11}$ und $R^{12}$ sind wie in Anspruch 1 definiert), und ein Salz derselben.

**18.** Verbindung gemäß Anspruch 8, wobei $R^3$ eine Gruppe der Formel $-NR^4R^5$ ist ($R^4$ und $R^5$ sind wie in Anspruch 1 definiert), und ein Salz derselben.

**19.** Verbindung gemäß Anspruch 8, wobei $R^3$ eine Gruppe der Formel $-CO-NR^{11}R^{12}$ ist ($R^{11}$ und $R^{12}$ sind wie in Anspruch 1 definiert), und ein Salz derselben.

**20.** Verbindung gemäß Anspruch 9, wobei $R^3$ eine Gruppe der Formel $-NR^4R^5$ ist ($R^4$ und $R^5$ sind wie in Anspruch 1 definiert), und ein Salz derselben.

**21.** Verbindung gemäß Anspruch 9, wobei $R^3$ eine Gruppe der Formel $-CO-NR^{11}R^{12}$ ist ($R^{11}$ und $R^{12}$ sind wie in Anspruch 1 definiert), und ein Salz derselben.

**22.** Verbindung gemäß Anspruch 14, wobei $R^4$ ein Wasserstoffatom ist und $R^5$ eine Gruppe der Formel

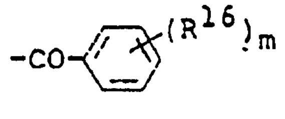

ist (wobei $R^{16}$ und m wie in Anspruch 1 definiert sind), und ein Salz derselben.

**23.** Verbindung gemäß Anspruch 14, wobei $R^4$ ein Wasserstoffatom ist und $R^5$ Phenyl-$C_1$-$C_6$-alkoxycarbonyl, $C_1$-$C_6$-Alkanoyl, Phenyl-$C_2$-$C_6$-alkanoyl, $C_3$-$C_8$-Cycloalkyl-$C_2$-$C_6$-alkanoyl, $C_3$-$C_8$-Cycloalkylcarbonyl, Tricyclo[3.3.1.1]decanylcarbonyl, Naphthylcarbonyl, Pyridylcarbonyl, Furoyl, Thenoyl, Phenoxy-$C_2$-$C_6$-alkanoyl, dessen Phenylring gegebenenfalls 1 bis 3 Substituenten besitzt, die aus $C_1$-$C_6$-Alkyl, $C_1$-$C_6$-Alkoxy und Amino mit gegebenenfalls einem $C_1$-$C_6$-Alkanoylsubstituenten ausgewählt sind, phthalimidosubstituiertes $C_2$-$C_6$-Alkanoyl, $C_1$-$C_6$-Alkoxycarbonyl, $C_2$-$C_6$-Alkanoyl, Carboxy-$C_2$-$C_6$-alkanoyl, Naphthyloxy-$C_2$-$C_6$-alkanoyl, halogensubstituiertes $C_1$-$C_6$-Alkanoyl, eine Gruppe der Formel

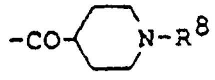

(worin $R^8$ ein Wasserstoffatom, $C_1$-$C_6$-Alkyl, Phenyl-$C_1$-$C_6$-alkoxycarbonyl, Carbamoyl-$C_1$-$C_6$-alkyl, Amino-$C_1$-$C_6$-alkanoyl mit gegebenenfalls einem $C_1$-$C_6$-Alkylsubstituenten oder $C_1$-$C_6$-Alkanoyl ist), Anilinocarbonyl, das gegebenenfalls einen $C_1$-$C_6$-Alkylsubstituenten am Phenylring besitzt, Phenoxycarbonyl, Phenylsulfonyl, das gegebenenfalls einen Substituenten besitzt, der aus einem Halogenatom und $C_1$-$C_6$-Alkyl am Phenylring ausgewählt ist, Chinolylsulfonyl oder eine Gruppe der Formel

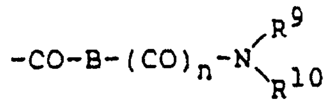

ist (worin B $C_1$-$C_6$-Alkylen ist, n eine ganze Zahl 0 oder 1 ist und $R^9$ und $R^{10}$ gleich oder verschieden sind und jeweils ein Wasserstoffatom, $C_1$-$C_6$-Alkyl mit gegebenenfalls einem Hydroxysubstituenten, $C_3$-$C_8$-Cycloalkyl, Phenyl-$C_1$-$C_6$-alkyl, $C_1$-$C_6$-Alkanoyl, $C_2$-$C_6$-Alkenyl, Phenoxy-$C_1$-$C_6$-alkyl, Phenyl, das gegebenenfalls 1 bis 3 Substituenten besitzt, die aus Amino-$C_1$-$C_6$-alkyl mit gegebenenfalls einem $C_1$-$C_6$-Alkanoylsubstituenten, $C_1$-$C_6$-Alkyl, $C_1$-$C_6$-Alkoxy und einem Halogenatom ausgewählt sind, phthalimidosubstituiertes $C_1$-$C_6$-Alkyl, Amino-$C_1$-$C_6$-alkyl mit gegebenenfalls einem $C_1$-$C_6$-Alkanoylsubstituenten, $C_2$-$C_6$-Alkinyl oder Amino-$C_1$-$C_6$-alkyl mit gegebenenfalls einem $C_1$-$C_6$-Alkylsubstituenten sind, oder $R^9$ und $R^{10}$ sich zusammen mit dem Stickstoffatom, an das sie binden, unter Bilden einer 5- oder 6-gliedrigen, gesättigten, heterocyclischen Gruppe verbinden können, die durch ein Stickstoff- oder Sauerstoffatom unterbrochen ist oder nicht, wobei die heterocyclische Gruppe gegebenenfalls einen Substituenten besitzt, der aus $C_1$-$C_6$-Alkyl, $C_1$-$C_6$-Alkoxycarbonyl und Piperidinyl ausgewählt ist), und ein Salz derselben.

**24.** Verbindung gemäß Anspruch 14, wobei $R^4$ $C_1$-$C_6$-Alkyl ist, und ein Salz derselben.

**25.** Verbindung gemäß Anspruch 16, wobei $R^4$ ein Wasserstoff ist und $R^5$ eine Gruppe der Formel

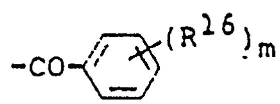

ist (worin $R^{16}$ und m wie in Anspruch 1 definiert sind), und ein Salz derselben.

**26.** Verbindung gemäß Anspruch 16, wobei $R^4$ ein Wasserstoffatom ist und $R^5$ Phenyl-$C_1$-$C_6$-alkoxycarbonyl, $C_1$-$C_6$-Alkanoyl, Phenyl-$C_1$-$C_6$-alkanoyl, $C_3$-$C_8$-Cycloalkyl-$C_2$-$C_6$-alkanoyl, $C_3$-$C_8$-Cycloalkylcarbonyl, Tricyclo[3.3.1.1]decanylcarbonyl, Naphthylcarbonyl, Pyridylcarbonyl, Furoyl, Thenoyl, Phenoxy-$C_2$-$C_6$-alkanoyl, dessen Phenylring gegebenenfalls 1 bis 3 Substituenten besitzt, die aus $C_1$-$C_6$-Alkyl, $C_1$-$C_6$-Alkoxy und Amino mit gegebenenfalls einem $C_1$-$C_6$-Alkanoylsubstituenten ausgewählt sind, phthalimidosubstituiertes $C_2$-$C_6$-Alkanoyl, $C_1$-$C_6$-Alkoxycarbonyl-$C_2$-$C_6$-alkanoyl, Carboxy-$C_2$-$C_6$-alkanoyl, Naphthyloxy-$C_2$-$C_6$-alkanoyl, halogensubstituiertes $C_1$-$C_6$-Alkanoyl, eine Gruppe der Formel

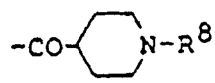

(worin $R^8$ ein Wasserstoffatom, $C_1$-$C_6$-Alkyl, Phenyl-$C_1$-$C_6$-alkoxycarbonyl, Carbamoyl-$C_1$-$C_6$-alkyl, Amino-$C_1$-$C_6$-alkanoyl mit gegebenenfalls einem $C_1$-$C_6$-Alkylsubstituenten oder $C_1$-$C_6$-Alkanoyl ist), Anilinocarbonyl, das gegebenenfalls einen $C_1$-$C_6$-Alkylsubstituenten am Phenylring besitzt, Phenoxycarbonyl, Phenylsulfonyl, das gegebenenfalls einen Substituenten besitzt, der aus einem Halogenatom und $C_1$-$C_6$-Alkyl am Phenylring ausgewählt ist, Chinolylsulfonyl, oder eine Gruppe der Formel

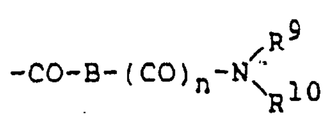

ist (worin B $C_2$-$C_6$-Alkylen ist, n eine ganze Zahl 0 oder 1 ist und $R^9$ und $R^{10}$ gleich oder verschieden sind und jeweils ein Wasserstoffatom, $C_1$-$C_6$-Alkyl mit gegebenenfalls einem Hydroxysubstituenten, $C_3$-$C_8$-Cycloalkyl, Phenyl-$C_1$-$C_6$-alkyl, $C_1$-$C_6$-Alkanoyl, $C_2$-$C_6$-Alkenyl, Phenoxy-$C_1$-$C_6$-alkyl, Phenyl, das gegebenenfalls 1 bis 3 Substituenten besitzt,dieaus Amino-$C_1$-$C_6$-alkyl mit gegebenenfalls einem $C_1$-$C_6$-Alkanoylsubstituenten, $C_1$-$C_6$-Alkyl, $C_1$-$C_6$-Alkoxy und einem Halogenatom ausgewählt sind, phthalimidosubstituiertes $C_1$-$C_6$-Alkyl, Amino-$C_1$-$C_6$-alkyl mit gegebenenfalls einem $C_1$-$C_6$-Alkanoylsubstituenten, $C_2$-$C_6$-Alkinyl oder Amino-$C_1$-$C_6$-alkyl mit gegebenenfalls einem $C_1$-$C_6$-Alkylsubstituenten sind oder $R^9$ und $R^{10}$ sich zusammen mit dem Stickstoffatom, an das sie binden, unter Bilden einer 5- oder 6-gliedrigen, gesättigten, heterocyclischen Gruppe verbinden können, die durch ein Stickstoff- oder Sauerstoffatom unterbrochen ist oder nicht, wobei die heterocyclische Gruppe gegebenenfalls einen Substituenten besitzt, der aus $C_1$-$C_6$-Alkyl, $C_1$-$C_6$-Alkoxycarbonyl und Piperidinyl ausgewählt ist), und ein Salz derselben.

**27.** Verbindung gemäß Anspruch 16, wobei $R^4$ $C_1$-$C_6$-Alkyl ist, und ein Salz derselben.

**28.** Verbindung gemäß Anspruch 7, wobei $R^4$ ein Wasserstoffatom ist und $R^5$ eine Gruppe der Formel

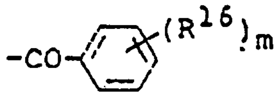

ist (worin $R^{16}$ und m wie in Anspruch 1 definiert sind), und ein Salz derselben.

**29.** Verbindung gemäß Anspruch 7, 10 bis 13, 18 und 20, wobei $R^4$ ein Wasserstoffatom ist und $R^5$ wie in Anspruch 23 definiert ist.

**30.** Verbindung gemäß Anspruch 7, wobei $R^4$ $C_1$-$C_6$-Alkyl ist, und ein Salz derselben.

**31.** Verbindung gemäß Anspruch 18, wobei $R^4$ ein Wasserstoffatom ist und $R^5$ eine Gruppe der Formel

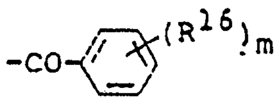

ist (wobei $R^{16}$ und m wie in Anspruch 1 definiert sind), und ein Salz derselben.

**32.** Verbindung gemäß Anspruch 18, wobei $R^4$ $C_1$-$C_6$-Alkyl ist, und ein Salz derselben.

**33.** Verbindung gemäß Anspruch 20, wobei $R^4$ ein Wasserstoffatom ist, und $R^5$ eine Gruppe der Formel

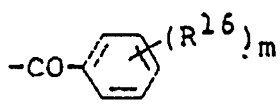

ist (worin $R^{16}$ und m wie in Anspruch 1 definiert sind), und ein Salz derselben.

**34.** Verbindung gemäß Anspruch 20, wobei $R^4$ $C_1$-$C_6$-Alkyl ist, und ein Salz derselben.

**35.** Verbindung gemäß Anspruch 10, wobei $R^4$ ein Wasserstoffatom ist und $R^5$ eine Gruppe der Formel

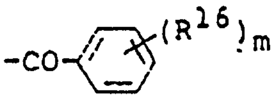

ist (worin $R^{16}$ und m wie in Anspruch 1 definiert sind), und ein Salz derselben.

**36.** Verbindung gemäß Anspruch 10, wobei $R^4$ $C_1$-$C_6$-Alkyl ist, und ein Salz derselben.

**37.** Verbindung gemäß Anspruch 11, wobei $R^4$ ein Wasserstoffatom ist und $R^5$ eine Gruppe der Formel

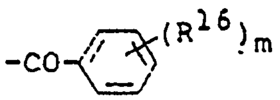

ist (worin $R^{16}$ und m wie in Anspruch 1 definiert sind), und ein Salz derselben.

**38.** Verbindung gemäß Anspruch 11, wobei $R^4$ $C_1$-$C_6$-Alkyl ist, und ein Salz derselben.

**39.** Verbindung gemäß Anspruch 12, wobei $R^4$ ein Wasserstoffatom ist und $R^5$ eine Gruppe der Formel

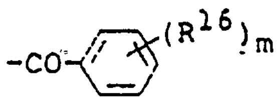

ist (worin $R^{16}$ und m wie in Anspruch 1 definiert sind), und ein Salz derselben.

**40.** Verbindung gemäß Anspruch 12, wobei $R^4$ $C_1$-$C_6$-Alkyl ist, und ein Salz derselben.

**41.** Verbindung gemäß Anspruch 13, wobei $R^4$ ein Wasserstoffatom ist und $R^5$ eine Gruppe der Formel

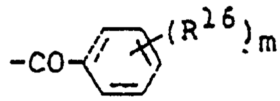

ist (worin $R^{16}$ und m wie in Anspruch 1 definiert sind), und ein Salz derselben.

**42.** Verbindung gemäß Anspruch 13, wobei $R^4$ $C_1$-$C_6$-Alkyl ist, und ein Salz derselben.

**43.** Verbindung gemäß Anspruch 22, wobei $R^{16}$ ein Halogenatom oder $C_1$-$C_6$-Alkyl mit gegebenenfalls einem Substituenten ist, der aus einem Halogenatom und Hydroxy ausgewählt ist, und ein Salz derselben.

**44.** Verbindung gemäß Anspruch 25, wobei $R^{16}$ ein Halogenatom oder $C_1$-$C_6$-Alkyl mit gegebenenfalls einem Substituenten ist, der aus einem Halogenatom und Hydroxy ausgewählt ist, und ein Salz derselben.

**45.** Verbindung gemäß Anspruch 31, wobei $R^{16}$ ein Halogenatom oder $C_1$-$C_6$-Alkyl mit gegebenenfalls einem Substituenten ist, der aus einem Halogenatom und Hydroxy ausgewählt ist, und ein Salz derselben.

**46.** Verbindung gemäß Anspruch 34, wobei $R^{16}$ ein Halogenatom oder $C_1$-$C_6$-Alkyl mit gegebenenfalls einem Substituenten ist, der aus einem Halogenatom und Hydroxy ausgewählt ist, und ein Salz derselben.

**47.** Verbindung gemäß Anspruch 1, wobei W eine Gruppe der Formel $-(CH_2)_p-$ ist, worin p eine ganze Zahl 3 bis 5 ist und die $-(CH_2)_p$-Gruppe gegebenenfalls weiter 1 bis 3 in Anspruch 1 definierte Substituenten besitzt.

**48.** Verbindung gemäß Anspruch 1, wobei W eine Gruppe der Formel $-CH=CH-(CH_2)_q-$ ist, worin q eine ganze Zahl 1 bis 3 ist und die $-CH=CH-(CH_2)_q$-Gruppe gegebenenfalls weiter 1 bis 3 Substituenten besitzt, die aus $C_1$-$C_6$-Alkyl mit gegebenenfalls einem Hydroxysubstituenten und Amino-$C_1$-$C_6$-alkyl mit gegebenenfalls einem Substituenten ausgewählt sind, deraus $C_1$-$C_6$-Alkyl und $C_1$-$C_6$-Alkanoyl ausgewählt ist, und ein Salz derselben.

**49.** Verbindung gemäß Anspruch 47, wobei W eine Gruppe der Formel $-(CH_2)_p-$ ist (p ist eine ganze Zahl 3 bis 5) und die $-(CH_2)_p$-Gruppe gegebenenfalls 1 bis 3 in Anspruch 1 definierte Substituenten besitzt.

**50.** Verbindung gemäß Anspruch 49, wobei p in der Gruppe $-(CH_2)_p-$ 3 ist und die Gruppe keinen Substituenten besitzt, und ein Salz derselben.

**51.** Verbindung gemäß Anspruch 49, wobei p in der Gruppe $-(CH_2)_p-$ 3 ist und die Gruppe einen Substituenten einer Gruppe der Formel

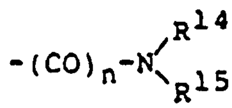

besitzt (worin $R^{14}$, $R^{15}$ und n wie vorstehend definiert sind), und ein Salz derselben.

**52.** Verbindung gemäß Anspruch 49, wobei p in der Gruppe $-(CH_2)_p-$ 4 ist und die Gruppe keinen Substituenten besitzt, und ein Salze derselben.

**53.** Verbindung gemäß Anspruch 49, wobei p in der Gruppe $-(CH_2)_p-$ 4 ist und die Gruppe einen Substituenten einer Gruppe der Formel

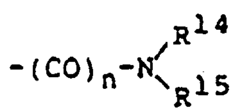

besitzt (worin $R^{14}$, $R^{15}$ und n wie vorstehend definiert sind), und ein Salz derselben.

**54.** Verbindung gemäß Anspruch 49, wobei p in der Gruppe $-(CH_2)_p-$ 5 ist, und ein Salz derselben.

**55.** Verbindung gemäß Anspruch 48, wobei q in der Gruppe $-CH=CH-(CH_2)_q-$ 1 ist, und ein Salz derselben.

**56.** Verbindung gemäß Anspruch 48, wobei q in der Gruppe $-CH=CH-(CH_2)_q-$ 2 ist, und ein Salz derselben.

**57.** Verbindung gemäß Anspruch 48, wobei q in der Gruppe $-CH=CH-(CH_2)_q-$ 3 ist, und ein Salz derselben.

**58.** Verbindung gemäß Anspruch 51 oder 53, wobei n in dem Substituenten

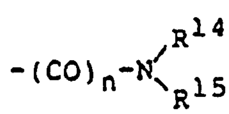

0 ist und $R^{14}$ und $R^{15}$ gleich oder verschieden sind und jeweils ein Wasserstoffatom, $C_1$-$C_6$-Alkyl oder $C_3$-$C_8$-Cycloalkyl sind, und ein Salz derselben.

**59.** Verbindung gemäß Anspruch 56, wobei die heterocyclische Gruppe der Formel

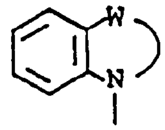

2,3-Dihydro-1H-benzazepin ist, und ein Salz derselben.

**60.** 1-[4-(2-Methylbenzoylamino)benzoyl]-4-methyl-2,3,4,5-tetrahydro-1H-1,4-benzodiazepin.

**61.** 5-Dimethylamino-1-[4-(2-methylbenzoylamino)benzoyl]-2,3,4,5-tetrahydro-1H-benzazepin.

**62.** 5-Dimethylamino-1-[2-chlor-4-(2-methylbenzoylamino)benzoyl]-2,3,4,5-tetrahydro-1H-benzazepin.

**63.** 5-Methylamino-1-[2-chlor-4-(2-methylbenzoylamino)benzoyl]-2,3,4,5-tetrahydro-1H-benzazepin.

**64.** 5-Cyclopropylamino-1-[2-chlor-4-(2-methylbenzoylamino)benzoyl]-2,3,4,5-tetrahydro-1H-benzazepin.

**65.** 5-Cyclopropylamino-1-[2-chlor-4-(2-chlorbenzoylamino)benzoyl]-2,3,4,5-tetrahydro-1H-benzazepin.

**66.** 5-Dimethylamino-1-[2-methyl-4-(2-methylbenzoylamino)benzoyl]-2,3,4,5-tetrahydro-1H-benzazepin.

**67.** 4-Dimethylamino-1-[3-methoxy-4-(2-methylbenzoylamino)benzoyl]-1,2,3,4-tetrahydrochinolin.

**68.** 7-Chlor-5-methylamino-1-[4-(2-methylbenzoylamino)benzoyl]-2,3,4,5-tetrahydro-1H-benzazepin.

**69.** 7-Chlor-5-methylamino-1-[4-(2-chlorbenzoylamino)benzoyl]-2,3,4,5-tetrahydro-1H-benzazepin.

**70.** Vasopressin-antagonistische Zusammensetzung, die als aktiven Bestandteil eine in Anspruch 1 angegebene Verbindung der Formel (1) oder ein pharmazeutisch annehmbares Salz derselben im Gemisch mit einem pharmazeutisch annehmbaren Träger oder Verdünnungsmittel umfaßt.

**71.** Verfahren zum Herstellen einer in Anspruch 1 angegebenen Verbindung der Formel (1), welches das Umsetzen einer Verbindung der Formel (2):

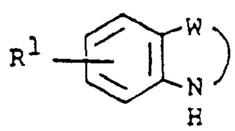

(2)

worin $R^1$ und W gleich wie vorstehend definiert sind, mit einer Verbindung der Formel (3) umfaßt:

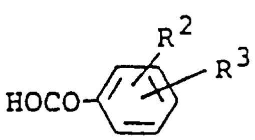

(3)

worin $R^2$ und $R^3$ gleich wie vorstehend definiert sind.

**72.** Verfahren zum Herstellen einer Verbindung der Formel

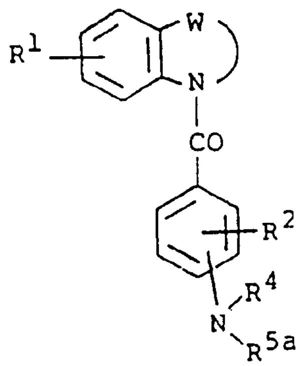

(1b)

worin $R^1$, $R^2$, $R^4$ und W wie in Anspruch 1 definiert sind, worin $R^{5a}$ gleich wie in Anspruch 1 definiertes $R^5$ ist, mit Ausnahme, daß Anilinocarbonyl mit gegebenenfalls einem $C_1$-$C_6$-Alkylsubstituenten am Phenylring, Phenylsulfonyl mit gegebenenfalls einem Substituenten, der aus einem Halogenatom und $C_1$-$C_6$-Alkyl am Phenylring ausgewählt ist, und Chinolinsulfonyl ausgenommen sind, welches das Umsetzen einer Verbindung der Formel (2b):

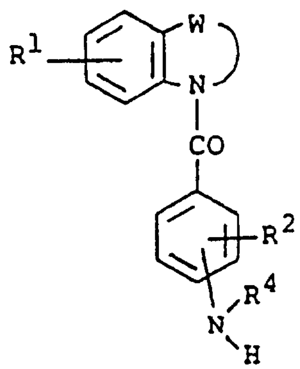

(2b)

worin $R^1$, $R^2$, $R^4$ und W wie vorstehend definiert sind, mit einer Verbindung der Formel (4) umfaßt:

$R^{5a}OH$ (4)

**73.** Verfahren zum Herstellen einer Verbindung der Formel (1c):

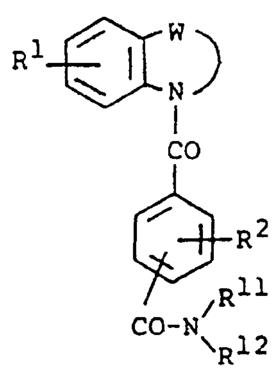

(1c)

worin $R^1$, $R^2$, $R^{11}$, $R^{12}$ und W wie in Anspruch 1 definiert sind,

welches das Umsetzen einer Verbindung der Formel (5):

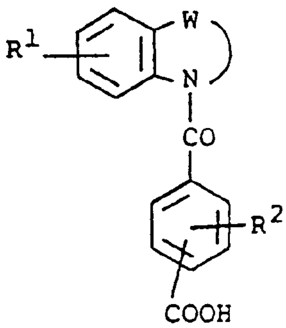

(5)

worin $R^1$, $R^2$ und W wie vorstehend definiert sind, mit einer Verbindung der Formel (6) umfaßt:

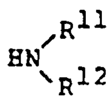

(6)

worin $R^{11}$ und $R^{12}$ wie vorstehend definiert sind.

**74.** Verfahren zur Herstellung einer Verbindung der Formel (1d):

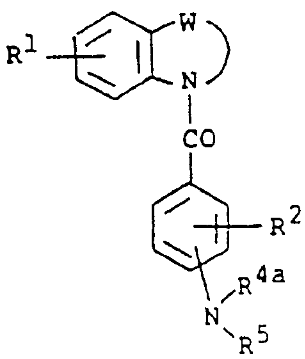

(1d)

worin $R^1$, $R^2$, $R^5$ und W wie in Anspruch 1 definiert sind, und $R^{4a}$ $C_1$-$C_6$-Alkyl ist, welches das Umsetzen einer Verbindung der Formel (7):

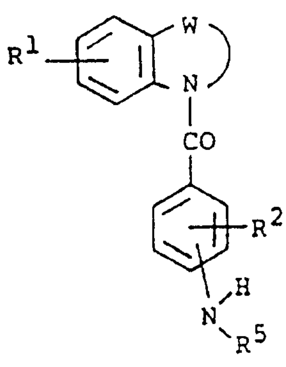

(7)

worin $R^1$, $R^2$, $R^5$ und W wie vorstehend definiert sind, mit einer Verbindung der Formel (8) oder (9) umfaßt:

$R^{4a}X$ (8) oder

$R^{17}COR^{18}$ (9)

worin $R^{4a}$ $C_1$-$C_6$-Alkyl ist, X ein Halogenatom ist und $R^{17}$ und $R^{18}$ jeweils ein Wasserstoffatom oder $C_1$-$C_6$-Alkyl sind.

**75.** Verfahren zum Herstellen einer Verbindung der Formel (1e)

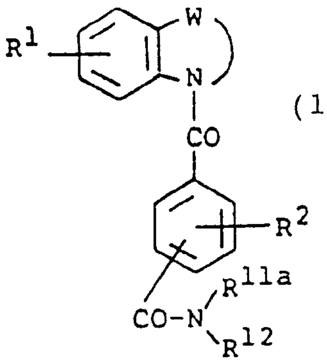

(1e)

worin $R^1$, $R^2$, $R^{12}$ und W wie in Anspruch 1 definiert sind und $R^{11a}$ $C_1$-$C_6$-Alkyl ist, welches das Umsetzen einer Verbindung der Formel (10):

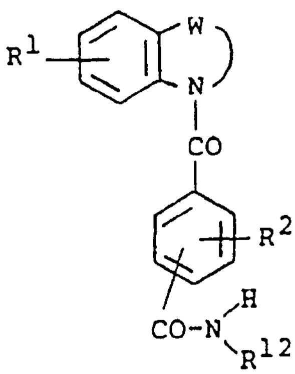

(10)

worin $R^1$, $R^2$, $R^{12}$ und W wie vorstehend definiert sind, mit einer Verbindung der Formel (11) oder (9) umfaßt:

$R^{11a}X$ (11) oder

$R^{17}COR^{18}$ (9)

worin $R^{11a}$ $C_1$-$C_6$-Alkyl ist und X, $R^{17}$ und $R^{18}$ wie vorstehend definiert sind.

**76.** Verfahren zum Herstellen einer Verbindung der Formel (1f):

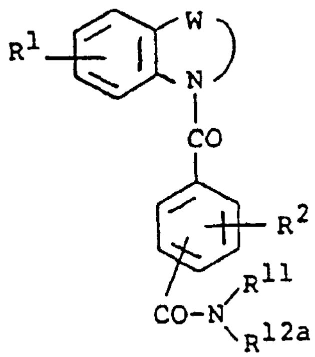

(1f)

worin $R^1$, $R^2$, $R^{11}$ und W wie in Anspruch 1 definiert sind und $R^{12a}$ $C_3$-$C_8$-Cycloalkyl ist, welches das Umsetzen einer Verbindung der Formel (12):

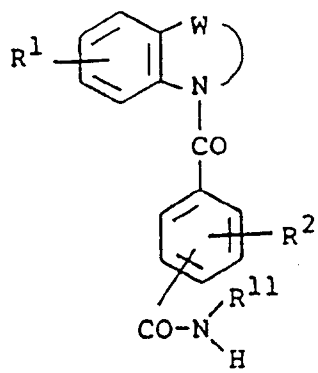

(12)

worin $R^1$, $R^2$, $R^{11}$ und W wie vorstehend definiert sind, mit einer Verbindung der Formel (13) umfaßt:

$$R^{12a}X \qquad (13)$$

worin $R^{12a}$ $C_3$-$C_8$-Cycloalkyl ist und X Halogen ist.

**77.** Verfahren zum Herstellen einer Verbindung der Formel (1cc):

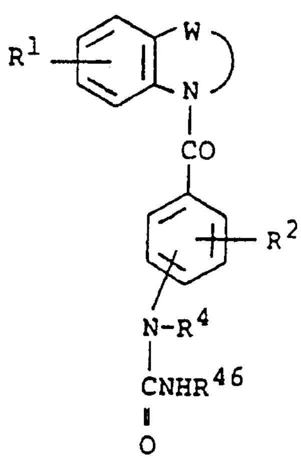

(1cc)

worin $R^1$, $R^2$, $R^4$ und W wie in Anspruch 1 definiert sind und $R^{46}$ Phenyl mit gegebenenfalls einem $C_1$-$C_6$-Alkylsub-

stituenten ist, welches das Umsetzen einer Verbindung der Formel (2b):

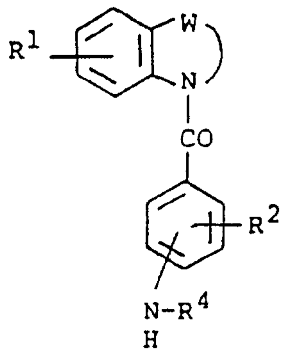

(2b)

worin $R^1$, $R^2$, $R^4$ und W wie vorstehend definiert sind, mit einer Verbindung der Formel (38) umfaßt:

$$R^{46}N{=}C{=}O \quad (38)$$

worin $R^{46}$ wie vorstehend definiert ist.

**78.** Verfahren zum Herstellen einer Verbindung (1dd)

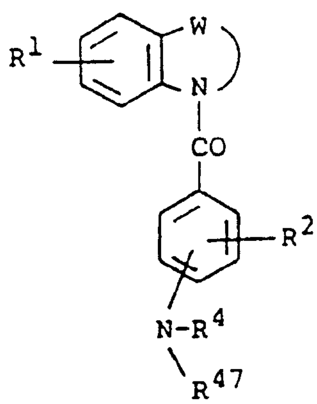

(1dd)

worin $R^1$, $R^2$, $R^4$ und W wie in Anspruch 1 definiert sind und $R^{47}$ Phenylsulfonyl, das gegebenenfalls einen Substituenten besitzt, der aus einem Halogenatom und $C_1$-$C_6$-Alkyl am Phenylring ausgewählt ist, oder Chinolinsulfonyl ist und X Halogen ist, welches das Umsetzen einer Verbindung der Formel (2b):

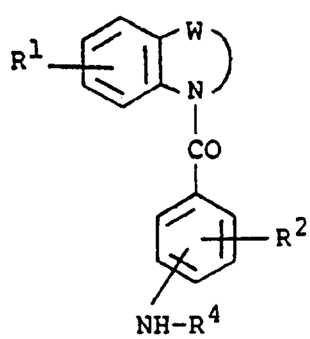

(2b)

worin $R^1$, $R^2$, $R^4$ und W wie vorstehend definiert sind, mit einer Verbindung der Formel (39) umfaßt:

$$R^{47}X \quad (39)$$

worin $R^{47}$ wie vorstehend definiert ist.

**79.** Verfahren zum Herstellen einer Verbindung der Formel (1hh):

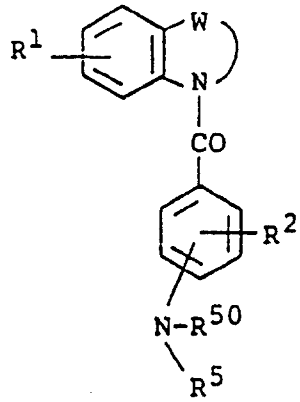

(1hh)

worin $R^1$, $R^2$, $R^5$ und W wie in Anspruch 1 definiert sind und $R^{50}$ Benzoyl mit gegebenenfalls einem Halogensubstituenten am Phenylring ist, welches das Umsetzen einer Verbindung der Formel (7):

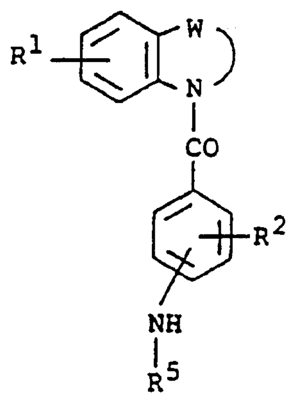

(7)

worin $R^1$, $R^2$, $R^5$ und W wie vorstehend definiert sind, mit einer Verbindung der Formel (42) umfaßt:

$$R^{50}OH \quad (42)$$

worin $R^{50}$ wie vorstehend definiert ist.

**80.** 5-Hydroxysulfonyloxyimino-1-[2-chlor-4-(2-methylbenzoylamino)benzoyl]-2,3,4,5-tetrahydro-1H-benzazepin.

**81.** 7-Fluor-5-hydroxy-1-[2-chlor-4-(2-methylbenzoylamino)benzoyl]-2,3,4,5-tetrahydro-1H-benzazepin.

**82.** 7-[4-(2-Chlorbenzoylamino)benzoyl]-1-methyl-1,2,3,4a,5,6,7,11b-octahydro-3-oxo[1]benzazepino[4,5-b][1,4]oxazin.

**83.** Verbindungen der Formeln:

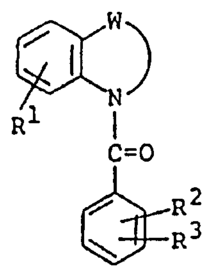

worin

a)

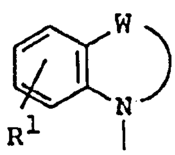 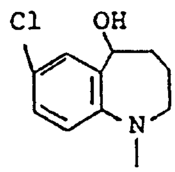

darstellt,

$R^2$ 2-$OCH_3$ darstellt und

$R^3$

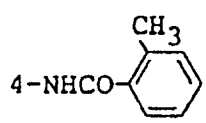

darstellt und

b)

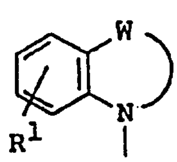 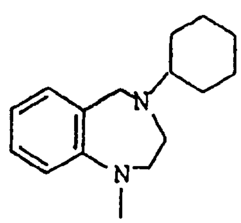

darstellt,

$R^2$ H darstellt und

$R^3$

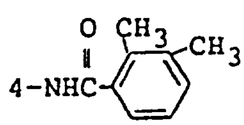

darstellt.

**84.** Benzoheterocyclische Verbindung der Formel:

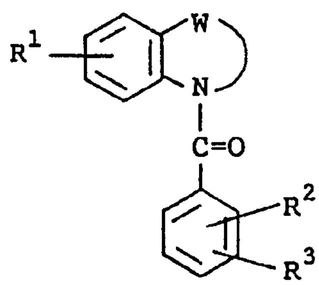

worin $R^1$, $R^2$ und $R^3$ wie in Anspruch 1 definiert sind und die heterocyclische Gruppe der Formel

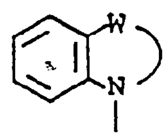

ein aus der Gruppe ausgewähltes Mitglied ist, die aus 3,4-Dihydro-2H-1,4-benzoxazinyl, 1,2,3,5-Tetrahydro-4,1-benzoxazepinyl, 1,2,3,4-Tetrahydrochinoxalinyl, 1,2,3,4,5,6-Hexahydro-1,5-benzodiazocinyl, 5-Methyl-1,2,3,4,5,6-Hexahydro-1,5-benzodiazocinyl, 4-Methyl-1,2,3,4-Tetrahydrochinoxalinyl, 1,2,3,4-Tetrahydro-5,1-benzoxazepinyl, 3,4-Dihydro-2H-1,4-benzothiazinyl, 2,3,4,5-Tetrahydro-1,5-benzothiazepinyl, 1,2,3,5-Tetrahydro-4,1-benzothiazepinyl, 4-Ethyl-1,2,3,4-tetrahydrochinoxalinyl, 4-Propyl-1,2,3,4-tetrahydrochinoxalinyl, 4-Butyl-1,2,3,4-tetrahydrochinoxalinyl, 4-Pentyl-1,2,3,4-tetrahydrochinoxalinyl, 4-Hexyl-1,2,3,4-tetrahydrochinoxalinyl, 2,3,4,5-Tetrahydro-1H-1,4-benzodiazepinyl, 4-Methyl-2,3,4,5-tetrahydro-1H-1,4-benzodiazepinyl, 4-Ethyl-2,3,4,5-tetrahydro-1H-1,4-benzodiazepinyl, 4-Propyl-2,3,4,5-tetrahydro-1H-1,4-benzodiazepinyl, 4-Butyl-2,3,4,5-tetrahydro-1H-1,4-benzodiazepinyl, 4-Pentyl-2,3,4,5-tetrahydro-1H-1,4-benzodiazepinyl, 4-Hexyl-2,3,4,5-tetrahydro-1H-1,4-benzodiazepinyl, 2,3,4,5-Tetrahydro-1H-1,5-benzodiazepinyl, 5-Methyl-2,3,4,5-tetrahydro-1H-1,5-benzodiazepinyl, 5-Ethyl-2,3,4,5-tetrahydro-1H-1,5-benzodiazepinyl, 5-Propyl-2,3,4,5-tetrahydro-1H-1,5-benzodiazepinyl, 5-Butyl-2,3,4,5-tetrahydro-1H-1,5-benzodiazepinyl, 5-Pentyl-2,3,4,5-tetrahydro-1H-1,5-benzodiazepinyl, 5-Hexyl-2,3,4,5-tetrahydro-1H-1,5-benzodiazepinyl, 3,4-Dihydro-1-oxo-2H-1,4-benzothiazepinyl, 3,4-Dihydro-1,1-dioxo-2H-1,4-benzothiazepinyl, 1-Oxo-2,3,4,5-tetrahydro-1,5-benzothiazepinyl, 1,1-Dioxo-2,3,4,5-tetrahydro-1,5-benzothiazepinyl, 4-Oxo-1,2,3,5-tetrahydro-4,1-benzothiazepinyl und 4,4-Dioxo-1,2,3,5-tetrahydro-4,1-benzothiazepinyl besteht, und ein Salz derselben.

**Patentansprüche für folgenden Vertragsstaat: ES**

**1.** Verfahren zum Herstellen einer benzoheterocyclischen Verbindung der folgenden Formel:

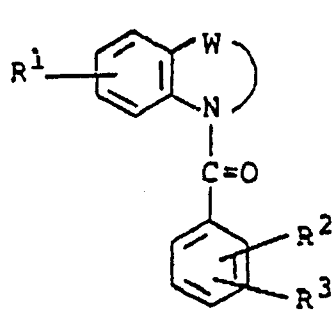

(1)

worin $R^1$ ein Wasserstoffatom, ein Halogenatom, $C_1$-$C_6$-Alkyl, Amino mit gegebenenfalls einem $C_1$-$C_6$-Alkylsubstituenten oder $C_1$-$C_6$-Alkoxy ist,
$R^2$ ein Wasserstoffatom, ein Halogenatom, $C_1$-$C_6$-Alkoxy, Phenyl($C_1$-$C_6$)alkoxy, Hydroxy, $C_1$-$C_6$-Alkyl, Amino mit gegebenenfalls einem $C_1$-$C_6$-Alkylsubstituenten, carbamoylsubstituiertes $C_1$-$C_6$-Alkoxy, aminosubstituiertes $C_1$-$C_6$-Alkoxy mit gegebenenfalls einem $C_1$-$C_6$-Alkylsubstituenten oder Benzoyloxy ist, das gegebenenfalls einen Halo-

gensubstituenten am Phenylring besitzt,
$R^3$ eine Gruppe der Formel

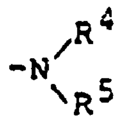

oder eine Gruppe der Formel

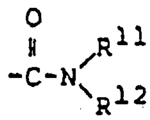

ist,
$R^4$ ein Wasserstoffatom, Benzoyl, das gegebenenfalls einen Halogensubstituenten am Phenylring besitzt, oder $C_1$-$C_6$-Alkyl ist,
$R^5$ (a) eine Gruppe der Formel

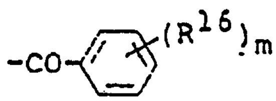

[worin $R^{16}$ (i) ein Halogenatom, (ii) $C_1$-$C_6$-Alkyl, das gegebenenfalls einen Substituenten besitzt, der aus einem Halogenatom und Hydroxy ausgewählt ist, (iii) Hydroxy, (iv) $C_1$-$C_6$-Alkoxy, (v) $C_1$-$C_6$-Alkanoyloxy, (vi) $C_1$-$C_6$-Alkylthio, (vii) $C_1$-$C_6$-Alkanoyl, (viii) Carboxy, (ix) $C_1$-$C_6$-Alkoxycarbonyl, (x) Cyano, (xi) Nitro, (xii) Amino, das gegebenenfalls einen Substituenten besitzt, der aus $C_1$-$C_6$-Alkyl und $C_1$-$C_6$-Alkanoyl ausgewählt ist, (xiii) Phenyl, (xiv) $C_3$-$C_8$-Cycloalkyl, (xv) $C_2$-$C_6$-alkanoyloxysubstituiertes $C_1$-$C_6$-Alkoxy, (xvi) carboxysubstituiertes $C_1$-$C_6$-Alkoxy, (xvii) halogensubstituiertes $C_1$-$C_6$-Alkoxy, (xviii) carbamoylsubstituiertes $C_1$-$C_6$-Alkoxy, (xix) hydroxysubstituiertes $C_1$-$C_6$-Alkoxy, (xx) $C_1$-$C_6$-alkoxycarbonylsubstituiertes $C_1$-$C_6$-Alkoxy, (xxi) phthalimidosubstituiertes $C_1$-$C_6$-Alkoxy, (xxii) Aminocarbonyl-$C_1$-$C_6$-alkoxy mit einem $C_1$-$C_6$-Alkylsubstituenten oder (xxiii) eine Gruppe der Formel

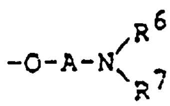

ist
(A ist Alkylen mit bis zu 6 Kohlenstoffatomen und $R^6$ und $R^7$ sind gleich oder verschieden und sind jeweils ein Wasserstoffatom, $C_1$-$C_6$-Alkyl mit gegebenenfalls einem Hydroxysubstituenten, $C_1$-$C_6$-Alkanoyl oder Benzoyl oder $R^6$ und $R^7$ können sich zusammen mit dem Stickstoffatom, an das sie binden, unter Bilden einer 5- oder 6-gliedrigen, gesättigten, heterocyclischen Gruppe verbinden, die durch ein Stickstoff- oder Sauerstoffatom unterbrochen ist oder nicht, wobei die heterocyclische Gruppe gegebenenfalls einen Substituenten besitzt, der aus Piperidinyl und $C_1$-$C_6$-Alkyl ausgewählt ist),
und m eine ganze Zahl von 0 bis 3 ist],

(b) Phenyl-$C_1$-$C_6$-alkoxycarbonyl,
(c) $C_1$-$C_6$-Alkanoyl,
(d) Phenyl-$C_2$-$C_6$-alkanoyl,
(e) $C_3$-$C_8$-Cycloalkyl-$C_2$-$C_6$-alkanoyl,
(f) $C_3$-$C_8$-Cyloalkylcarbonyl,
(g) Tricyclo[3.3.1.1]decanylcarbonyl,
(h) Naphthylcarbonyl,

(i) Pyridylcarbonyl,
(j) Furoyl,
(k) Thenoyl,
(l) Phenoxy-$C_2$-$C_6$-alkanoyl, wobei der Phenylring gegebenenfalls 1 bis 3 Substituenten besitzt, die aus $C_1$-$C_6$-Alkyl, $C_1$-$C_6$-Alkoxy und Amino mit gegebenenfalls einem $C_1$-$C_6$-Alkanoylsubstituenten ausgewählt sind,
(m) phthalimidosubstituiertes $C_2$-$C_6$-Alkanoyl,
(o) $C_1$-$C_6$-Alkoxycarbonyl-$C_2$-$C_6$-alkanoyl,
(p) Carboxy-$C_2$-$C_6$-alkanoyl,
(q) Naphthyloxy-$C_2$-$C_6$-alkanoyl,
(r) halogensubstituiertes $C_1$-$C_6$-Alkanoyl,
(s) eine Gruppe der Formel

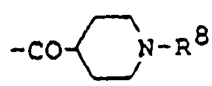

(worin $R^8$ (i) ein Wasserstoffatom, (ii) $C_1$-$C_6$-Alkyl, (iii) Phenyl-$C_1$-$C_6$-alkoxycarbonyl, (iv) Carbamoyl-$C_1$-$C_6$-alkyl, (v) Amino-$C_2$-$C_6$-alkanoyl mit gegebenenfalls einem $C_1$-$C_6$-Alkylsubstituenten oder (vi) $C_1$-$C_6$-Alkanoyl ist),
(t) Anilinocarbonyl, das gegebenenfalls einen $C_1$-$C_6$-Alkylsubstituenten am Phenylring besitzt,
(u) Phenoxycarbonyl,
(v) Phenylsulfonyl, das gegebenenfalls einen Substituenten besitzt, der aus einem Halogenatom und $C_1$-$C_6$-Alkyl am Phenylring ausgewählt ist,
(w) Chinolylsulfonyl oder
(x) eine Gruppe der Formel

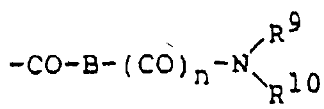

ist, worin B $C_1$-$C_6$-Alkylen ist, n eine ganze Zahl 0 oder 1 ist und $R^9$ und $R^{10}$ gleich oder verschieden sind und jeweils (i) ein Wasserstoffatom, (ii) Alkyl mit bis zu 6 Kohlenstoffatomen mit gegebenenfalls einem Hydroxysubstituenten, (iii) $C_3$-$C_8$-Cycloalkyl, (iv) Phenyl-$C_1$-$C_6$-alkyl, (v) $C_1$-$C_6$-Alkanoyl, (vi) Alkenyl mit bis zu 6 Kohlenstoffatomen, (vii) Phenoxy-$C_1$-$C_6$-alkyl, (viii) Phenyl, das gegebenenfalls 1 bis 3 Substituenten besitzt, die aus Amino-$C_1$-$C_6$-alkyl mit gegebenenfalls einem $C_1$-$C_6$-Alkanoylsubstituenten, $C_1$-$C_6$-Alkyl, $C_1$-$C_6$-Alkoxy und einem Halogenatom ausgewählt sind, (ix) phthalimidosubstituiertes $C_1$-$C_6$-Alkyl, (x) Amino-$C_1$-$C_6$-alkyl mit gegebenenfalls einem $C_1$-$C_6$-Alkanoylsubstituenten, (xi) Alkinyl mit bis zu 6 Kohlenstoffatomen oder (xii) Amino-$C_1$-$C_6$-alkyl mit gegebenenfalls einem $C_1$-$C_6$-Alkylsubstituenten, oder $R^9$ und $R^{10}$ sich zusammen mit dem Stickstoffatom, an das sie binden, unter Bilden einer 5- oder 6-gliedrigen, gesättigten, heterocyclischen Gruppe verbinden, die durch ein Stickstoff- oder Sauerstoffatom unterbrochen ist oder nicht, wobei die heterocyclische Gruppe gegebenenfalls einen Substituenten besitzt, der aus $C_1$-$C_6$-Alkyl, $C_1$-$C_6$-Alkoxycarbonyl und Piperidinyl ausgewählt ist),

$R^{11}$ ein Wasserstoffatom oder $C_1$-$C_6$-Alkyl ist,
$R^{12}$ $C_3$-$C_8$-Cycloalkyl oder Phenyl ist, das gegebenenfalls 1 bis 3 Substituenten besitzt, die aus $C_1$-$C_6$-Alkoxy, $C_1$-$C_6$-Alkyl und einem Halogenatom ausgewählt sind,
W eine Gruppe der Formel $-(CH_2)_p-$ (p ist eine ganze Zahl 3 bis 5) oder eine Gruppe der Formel $-CH=CH-(CH_2)_q-$ ist (q ist eine ganze Zahl 1 bis 3) oder worin die heterocyclische Gruppe der Formel

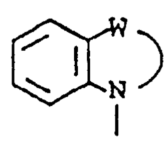

2,3,4,5-Tetrahydro-1H-1,4-benzodiazepin ist,
und weiter die $-(CH_2)_p-$Gruppe gegebenenfalls 1 bis 3 Substituenten besitzt, die aus (i) $C_1$-$C_6$-Alkyl mit gegebenen-

falls einem Hydroxysubstituenten, (ii) $C_1$-$C_6$-Alkoxycarbonyl, (iii) Carboxy, (iv) Hydroxy, (v) Oxo, (vi) $C_1$-$C_6$-Alkanoyloxy mit gegebenenfalls einem Halogensubstituenten, (vii) Amino-$C_1$-$C_6$-alkyl mit gegebenenfalls einem Substituenten, deraus $C_1$-$C_6$-Alkyl und $C_1$-$C_6$-Alkanoyl ausgewählt ist, (viii) $C_2$-$C_6$-alkanoyloxysubstituiertem $C_1$-$C_6$-Alkyl, (ix) $C_1$-$C_6$-Alkylsulfonyloxy-$C_1$-$C_6$-alkyl, (x) Azido-$C_1$-$C_6$-alkyl, (xi) einer Gruppe der Formel -O-$CH_2$- (die an die Gruppe -$(CH_2)_p$- oder -CH=CH-$(CH_2)_q$- in Spiroform gebunden ist), (xii) Aminocarbonyloxy mit gegebenenfalls einem $C_1$-$C_6$-Alkylsubstituenten, (xiii) $C_1$-$C_6$-Alkoxy, (xiv) $C_1$-$C_6$-alkoxycarbonylsubstituiertem $C_1$-$C_6$-Alkoxy, (xv) carboxysubstituiertem $C_1$-$C_6$-Alkoxy, (xvi) Aminocarbonyl-$C_1$-$C_6$-alkoxy mit gegebenenfalls einem $C_1$-$C_6$-Alkylsubstituenten, (xvii) Amino-$C_1$-$C_6$-alkoxy mit gegebenenfalls einem aus $C_1$-$C_6$-Alkyl und $C_1$-$C_6$-Alkanoyl ausgewählten Substituenten, (xviii) phthalimidosubstituiertem $C_1$-$C_6$-Alkoxy, (xix) Hydroxyimino, (xx) $C_1$-$C_6$-Alkanoyloxyimino, (xxi) $C_1$-$C_6$-Alkyliden, (xxii) einem Halogenatom, (xxiii) Azido, (xxvi) Hydrazino, (xxvii) Pyrrolyl, (xxviii) Amino-$C_1$-$C_6$-alkanoyloxy mit gegebenenfalls einem $C_1$-$C_6$-Alkylsubstituenten, (xxix) einer Gruppe der Formel

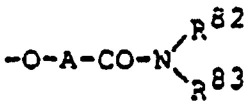

(A ist wie vorstehend definiert und $R^{82}$ und $R^{83}$ sind gleich oder verschieden und sind jeweils ein Wasserstoffatom, $C_1$-$C_6$-Alkyl, carbamoylsubstituiertes $C_1$-$C_6$-Alkyl, hydroxysubstituiertes $C_1$-$C_6$-Alkyl oder Pyridyl-$C_1$-$C_6$-alkyl, oder $R^{82}$ und $R^{83}$ können sich zusammen mit dem Stickstoffatom, an das sie binden, unter Bilden einer 5- oder 6-gliedrigen, gesättigten, heterocyclischen Gruppe verbinden, die durch ein Stickstoff-, Sauerstoff- oder Schwefelatom unterbrochen ist oder nicht, wobei die heterocyclische Gruppe gegebenenfalls einen Substituenten besitzt, der aus Oxo, $C_1$-$C_6$-Alkyl, $C_1$-$C_6$-Alkanoyl und Carbamoyl ausgewählt ist), und (xxx) einer Gruppe der Formel

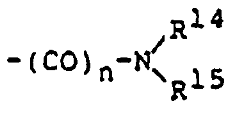

ausgewählt ist (worin n wie vorstehend definiert ist und $R^{14}$ und $R^{15}$ gleich oder verschieden sind und jeweils (i) ein Wasserstoffatom, (ii) $C_1$-$C_6$-Alkyl, (iii) Alkenyl mit bis zu 6 Kohlenstoffatomen, (iv) $C_1$-$C_6$-Alkanoyl, (v) $C_3$-$C_8$-Cycloalkyl, (vi) oxiranylsubstituiertes $C_1$-$C_6$-Alkyl, (vii) $C_1$-$C_6$-Alkyl mit 1 bis 2 Substituenten, die aus $C_1$-$C_6$-Alkoxy, Hydroxy und einer Aminogruppe mit gegebenenfalls einem $C_1$-$C_6$-Alkylsubstituenten ausgewählt sind, (viii) Phenyl-$C_1$-$C_6$-alkyl, (ix) Pyridyl-$C_1$-$C_6$-alkyl, (x) $C_1$-$C_6$-Alkylsulfonyl, (xi) Benzoyl, (xii) $C_1$-$C_6$-Alkoxycarbonyl, (xiii) Anilinocarbonyl, (xiv) Aminocarbonyl mit gegebenenfalls einem $C_1$-$C_6$-Alkylsubstituenten, (xv) cyanosubstituiertes $C_1$-$C_6$-Alkyl, (xvi) $C_1$-$C_6$-alkoxycarbonylsubstituiertes $C_1$-$C_6$-Alkyl, (xvii) carbamoylsubstituiertes $C_1$-$C_6$-Alkyl, (xviii) carboxysubstituiertes $C_1$-$C_6$-Alkyl, (xix) tetrahydropyranyloxysubstituiertes $C_1$-$C_6$-Alkyl, (xx) $C_2$-$C_6$-alkanoyloxysubstituiertes $C_1$-$C_6$-Alkyl, (xxi) Piperidinyl mit gegebenenfalls einem Phenyl-$C_1$-$C_6$-alkylsubstituenten am Piperidinylring, (xxii) halogensubstituiertes $C_2$-$C_6$-Alkanoyl, (xxiii) imidazolylsubstituiertes $C_2$-$C_6$-Alkanoyl, (xxiv) Amino-$C_2$-$C_6$-alkanoyl mit gegebenenfalls einem Substituenten, der aus $C_1$-$C_6$-Alkyl und $C_1$-$C_6$-Alkoxycarbonyl ausgewählt ist, (xxv) Aminocarbonyl-$C_1$-$C_6$-alkyl mit gegebenenfalls einem $C_1$-$C_6$-Alkylsubstituenten oder (xxvi) Phenyl-$C_1$-$C_6$-alkoxycarbonyl sind oder $R^{14}$ und $R^{15}$ können sich zusammen mit dem Stickstoffatom, an das sie binden, unter Bilden einer 5- oder 6-gliedrigen, gesättigten, heterocyclischen Gruppe verbinden, die durch Stickstoff oder Sauerstoff unterbrochen ist oder nicht, wobei die heterocyclische Gruppe gegebenenfalls einen Substituenten besitzen kann, der aus $C_1$-$C_6$-Alkyl, Phenyl-$C_1$-$C_6$-alkyl oder $C_1$-$C_6$-Alkanoyl ausgewählt ist und weiter die -CH=CH-$(CH_2)_q$-Gruppe gegebenenfalls 1 bis 3 Substituenten besitzt, die aus

(i) $C_1$-$C_6$-Alkyl mit gegebenenfalls einem Hydroxysubstituenten und
(ii) Amino-$C_1$-$C_6$-alkyl mit gegebenenfalls einem Substituenten ausgewählt sind, der aus $C_1$-$C_6$-Alkyl und $C_1$-$C_6$-Alkanoyl ausgewählt ist,

und eines Salzes derselben,
welches das Umsetzen einer Verbindung der Formel (2):

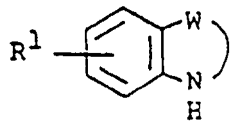

(2)

worin $R^1$ und W gleich wie vorstehend definiert sind, mit einer Verbindung der Formel (3) umfaßt:

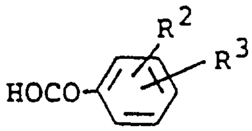

(3)

worin $R^2$ und $R^3$ gleich wie vorstehend definiert sind.

**2.** Verfahren zum Herstellen einer Verbindung der Formel

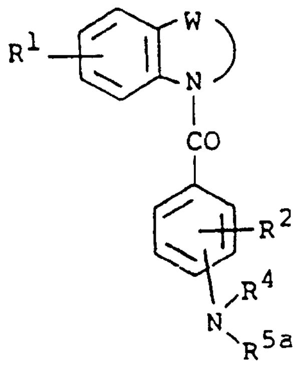

(1b)

worin $R^1$, $R^2$, $R^4$ und W wie in Anspruch 1 definiert sind, worin $R^{5a}$ gleich wie in Anspruch 1 definiertes $R^5$ ist, mit Ausnahme, daß Anilinocarbonyl mit gegebenenfalls einem $C_1$-$C_6$-Alkylsubstituenten am Phenylring, Phenylsulfonyl mit gegebenenfalls einem Substituenten, der aus einem Halogenatom und $C_1$-$C_6$-Alkyl am Phenylring ausgewählt ist, und Chinolinsulfonyl ausgenommen sind, welches das Umsetzen einer Verbindung der Formel (2b):

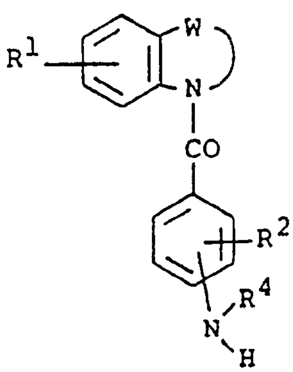

(2b)

worin $R^1$, $R^2$, $R^4$ und W wie vorstehend definiert sind, mit einer Verbindung der Formel (4) umfaßt:

$$R^{5a}OH \qquad (4)$$

**3.** Verfahren zum Herstellen einer Verbindung der Formel (1c):

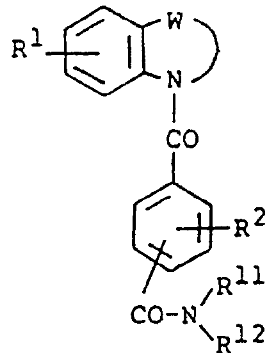

(1c)

worin $R^1$, $R^2$, $R^{11}$, $R^{12}$ und W wie in Anspruch 1 definiert sind, welches das Umsetzen einer Verbindung der Formel (5):

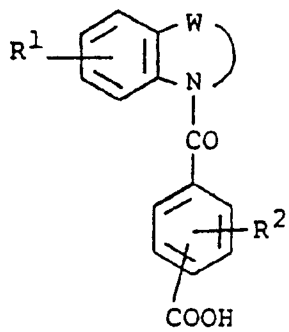

(5)

worin $R^1$, $R^2$ und W wie vorstehend definiert sind, mit einer Verbindung der Formel (6) umfaßt:

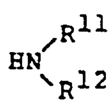

(6)

worin $R^{11}$ und $R^{12}$ wievorstehend definiert sind.

**4.** Verfahren zur Herstellung einer Verbindung der Formel (1d):

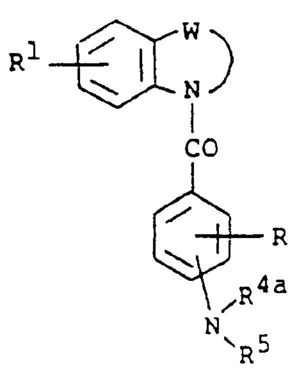

(1d)

worin $R^1$, $R^2$, $R^5$ und W wie in Anspruch 1 definiert sind, und $R^{4a}$ $C_1$-$C_6$-Alkyl ist, welches das Umsetzen einer

Verbindung der Formel (7):

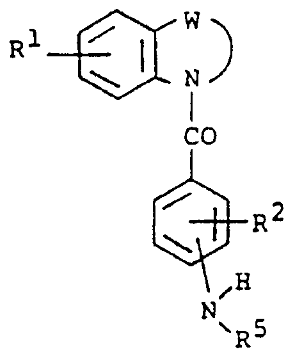

(7)

worin $R^1$, $R^2$, $R^5$ und W wie vorstehend definiert sind, mit einer Verbindung der Formel (8) oder (9) umfaßt:

$R^{4a}X$ (8) oder

$R^{17}COR^{18}$ (9)

worin $R^{4a}$ $C_1$-$C_6$-Alkyl ist, X ein Halogenatom ist und $R^{17}$ und $R^{18}$ jeweils ein Wasserstoffatom oder $C_1$-$C_6$-Alkyl sind.

**5.** Verfahren zum Herstellen einer Verbindung der Formel (1e)

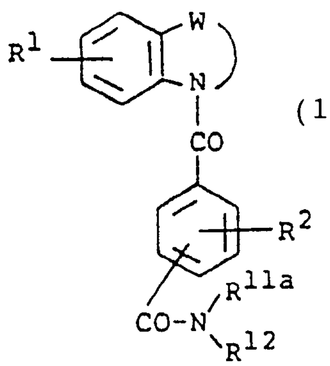

(1e)

worin $R^1$, $R^2$, $R^{12}$ und W wie in Anspruch 1 definiert sind und $R^{11a}$ $C_1$-$C_6$-Alkyl ist, welches das Umsetzen einer Verbindung der Formel (10):

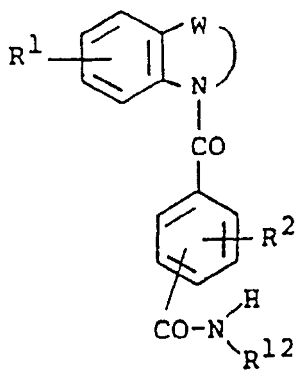

(10)

worin $R^1$, $R^2$, $R^{12}$ und W wie vorstehend definiert sind, mit einer Verbindung der Formel (11) oder (9) umfaßt:

$$R^{11a}X \qquad (11) \text{ oder}$$

$$R^{17}COR^{18} \qquad (9)$$

worin $R^{11a}$ $C_1$-$C_6$-Alkyl ist und X, $R^{17}$ und $R^{18}$ wie vorstehend definiert sind.

**6.** Verfahren zum Herstellen einer Verbindung der Formel (1f):

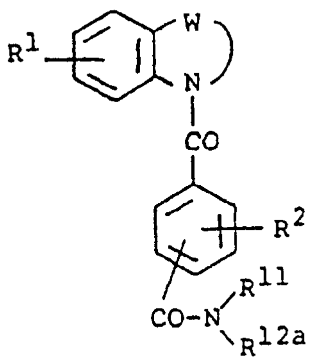

(1f)

worin $R^1$, $R^2$, $R^{11}$ und W wie in Anspruch 1 definiert sind und $R^{12a}$ $C_3$-$C_8$-Cycloalkyl ist, welches das Umsetzen einer Verbindung der Formel (12):

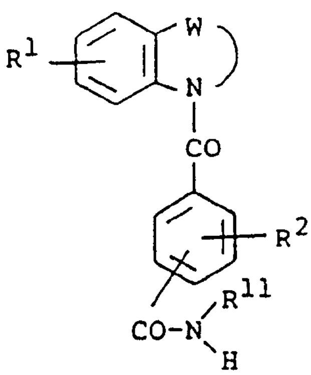

(12)

worin $R^1$, $R^2$, $R^{11}$ und W wie vorstehend definiert sind, mit einer Verbindung der Formel (13) umfaßt:

$$R^{12a}X \qquad (13)$$

worin $R^{12a}$ $C_3$-$C_8$-Cycloalkyl ist und X Halogen ist.

7. Verfahren zum Herstellen einer Verbindung der Formel (1cc):

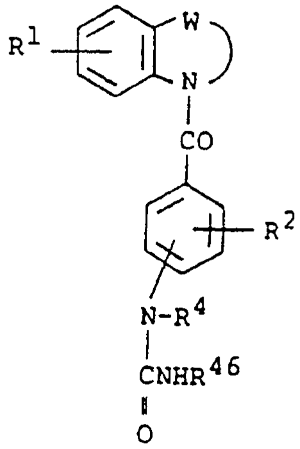

(1cc)

worin $R^1$, $R^2$, $R^4$ und W wie in Anspruch 1 definiert sind und $R^{46}$ Phenyl mit gegebenenfalls einem $C_1$-$C_6$-Alkylsubstituenten ist, welches das Umsetzen einer Verbindung der Formel (2b):

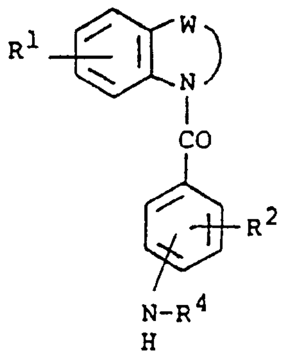

(2b)

worin $R^1$, $R^2$, $R^4$ und W wie vorstehend definiert sind, mit einer Verbindung der Formel (38) umfaßt:

$$R^{46}N=C=O \qquad (38)$$

worin $R^{46}$ wie vorstehend definiert ist.

8. Verfahren zum Herstellen einer Verbindung (1dd)

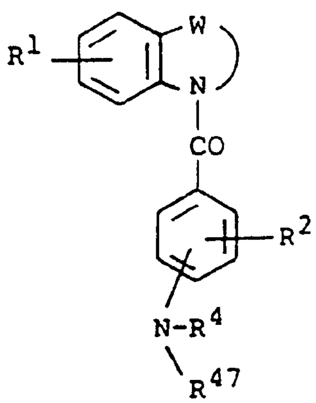

(1dd)

worin $R^1$, $R^2$, $R^4$ und W wie in Anspruch 1 definiert sind und $R^{47}$ Phenylsulfonyl, das gegebenenfalls einen Substituenten besitzt, der aus einem Halogenatom und $C_1$-$C_6$-Alkyl am Phenylring ausgewählt ist, oder Chinolinsulfonyl ist und X Halogen ist, welches das Umsetzen einer Verbindung der Formel (2b):

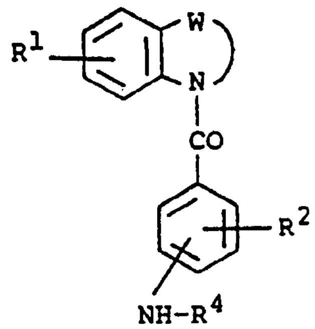

(2b)

worin $R^1$, $R^2$, $R^4$ und W wie vorstehend definiert sind, mit einer Verbindung der Formel (39) umfaßt:

$R^{47}X$ (39)

worin $R^{47}$ wie vorstehend definiert ist.

**9.** Verfahren zum Herstellen einer Verbindung der Formel (1hh):

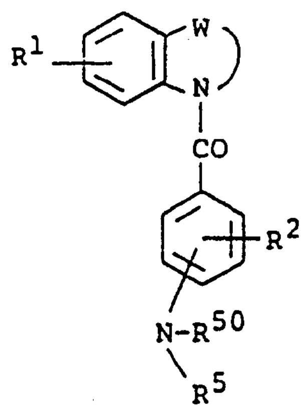

(1hh)

worin $R^1$, $R^2$, $R^5$ und W wie in Anspruch 1 definiert sind und $R^{50}$ Benzoyl mit gegebenenfalls einem Halogensubstituenten am Phenylring ist, welches das Umsetzen einer Verbindung der Formel (7):

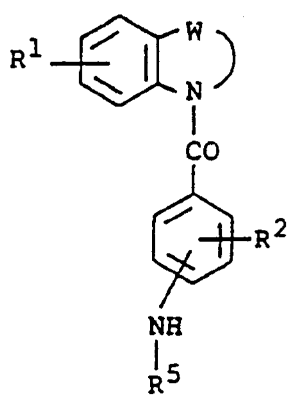

(7)

worin $R^1$, $R^2$, $R^5$ und W wie vorstehend definiert sind, mit einer Verbindung der Formel (42) umfaßt:

$$R^{50}OH \quad (42)$$

worin $R^{50}$ wie vorstehend definiert ist.

**10.** Verfahren gemäß Anspruch 1 bis 9, wobei $R^1$ in der Formel (1) ein Wasserstoffatom ist, oder ein Salz derselben.

**11.** Verfahren gemäß Anspruch 1 bis 9, wobei $R^1$ in der Formel (1) ein Halogenatom ist, und ein Salz derselben.

**12.** Verfahren gemäß Anspruch 1 bis 9, wobei $R^1$ in der Formel (1) $C_1$-$C_6$-Alkyl, Amino mit gegebenenfalls einem $C_1$-$C_6$-Alkylsubstituenten oder $C_1$-$C_6$-Alkoxy ist, und ein Salz derselben.

**13.** Verfahren gemäß Anspruch 10, wobei $R^2$ ein Wasserstoffatom ist, und ein Salz derselben.

**14.** Verfahren gemäß Anspruch 10, wobei $R^2$ ein Halogenatom, $C_1$-$C_6$-Alkoxy oder $C_1$-$C_6$-Alkyl ist, und ein Salz derselben.

**15.** Verfahren gemäß Anspruch 10, wobei $R^2$ Phenyl-$C_1$-$C_6$-alkoxy, Hydroxy, Amino mit gegebenenfalls einem $C_1$-$C_6$-Alkylsubstituenten, carbamoylsubstituiertes $C_1$-$C_6$-Alkoxy, aminosubstituiertes $C_1$-$C_6$-Alkoxy mit gegebenenfalls einem $C_1$-$C_6$-Alkylsubstituenten oder Benzoyloxy mit gegebenenfalls einem Halogensubstituenten an dessen Phenylring ist, und ein Salz derselben.

**16.** Verfahren gemäß Anspruch 11, wobei $R^2$ ein Wasserstoffatom ist, und ein Salz derselben.

**17.** Verfahren gemäß Anspruch 11, wobei $R^2$ ein Halogenatom, $C_1$-$C_6$-Alkoxy oder $C_1$-$C_6$-Alkyl ist, und ein Salz derselben.

**18.** Verfahren gemäß Anspruch 11, wobei $R^2$ Phenyl-$C_1$-$C_6$-alkoxy, Hydroxy, Amino mit gegebenenfalls einem $C_1$-$C_6$-Alkylsubstituenten, carbamoylsubstituiertes $C_1$-$C_6$-Alkoxy, aminosubstituiertes $C_1$-$C_6$-Alkoxy mit gegebenenfalls einem $C_1$-$C_6$-Alkylsubstituenten oder Benzoyloxy mit gegebenenfalls einem Halogensubstituenten an dessen Phenylring ist, und ein Salz derselben.

**19.** Verfahren gemäß Anspruch 12, wobei $R^2$ ein Wasserstoffatom ist, und ein Salz derselben.

**20.** Verfahren gemäß Anspruch 12, wobei $R^2$ ein Halogenatom, $C_1$-$C_6$-Alkoxy oder $C_1$-$C_6$-Alkyl ist, und ein Salz derselben.

**21.** Verfahren gemäß Anspruch 12, wobei $R^2$ Phenyl-$C_1$-$C_6$-alkoxy, Hydroxy, Amino mit gegebenenfalls einem $C_1$-$C_6$-Alkylsubstituenten, carbamoylsubstituiertes $C_1$-$C_6$-Alkoxy, aminosubstituiertes $C_1$-$C_6$-Alkoxy mit gegebenenfalls einem $C_1$-$C_6$-Alkylsubstituenten oder Benzoyloxy mit gegebenenfalls einem Halogensubstituenten an dessen Phenylring ist, und ein Salz derselben.

**22.** Verfahren gemäß Anspruch 13, wobei $R^3$ eine Gruppe der Formel $-NR^4R^5$ ist ($R^4$ und $R^5$ sind wie in Anspruch 1 definiert), und ein Salz derselben.

**23.** Verfahren gemäß Anspruch 13, wobei $R^3$ eine Gruppe der Formel $-CO-NR^{11}R^{12}$ ist ($R^{11}$ und $R^{12}$ sind wie in Anspruch 1 definiert), und ein Salz derselben.

**24.** Verfahren gemäß Anspruch 14, wobei $R^3$ eine Gruppe der Formel $-NR^4R^5$ ist ($R^4$ und $R^5$ sind wie in Anspruch 1 definiert), und ein Salz derselben.

**25.** Verfahren gemäß Anspruch 14, wobei $R^3$ eine Gruppe der Formel $-CO-NR^{11}R^{12}$ ist ($R^{11}$ und $R^{12}$ sind wie in Anspruch 1 definiert), und ein Salz derselben.

**26.** Verfahren gemäß Anspruch 16, wobei $R^3$ eine Gruppe der Formel $-NR^4R^5$ ist ($R^4$ und $R^5$ sind wie in Anspruch 1 definiert), und ein Salz derselben.

**27.** Verfahren gemäß Anspruch 16, wobei $R^3$ eine Gruppe der Formel $-CO-NR^{11}R^{12}$ ist ($R^{11}$ und $R^{12}$ sind wie in Anspruch 1 definiert), und ein Salz derselben.

**28.** Verfahren gemäß Anspruch 17, wobei $R^3$ eine Gruppe der Formel $-NR^4R^5$ ist ($R^4$ und $R^5$ sind wie in Anspruch 1 definiert), und ein Salz derselben.

**29.** Verfahren gemäß Anspruch 17, wobei $R^3$ eine Gruppe der Formel $-CO-NR^{11}R^{12}$ ist ($R^{11}$ und $R^{12}$ sind wie in Anspruch 1 definiert), und ein Salz derselben.

**30.** Verfahren gemäß Anspruch 22, wobei $R^4$ ein Wasserstoffatom ist und $R^5$ eine Gruppe der Formel

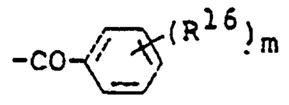

ist (wobei $R^{16}$ und m wie in Anspruch 1 definiert sind), und ein Salz derselben.

**31.** Verfahren gemäß Anspruch 22, wobei $R^4$ ein Wasserstoffatom ist und $R^5$ Phenyl-$C_1$-$C_6$-alkoxycarbonyl, $C_1$-$C_6$-Alkanoyl, Phenyl-$C_2$-$C_6$-alkanoyl, $C_3$-$C_8$-Cycloalkyl-$C_2$-$C_6$-alkanoyl, $C_3$-$C_8$-Cycloalkylcarbonyl, Tricyclo[3.3.1.1]decanylcarbonyl, Naphthylcarbonyl, Pyridylcarbonyl, Furoyl, Thenoyl, Phenoxy-$C_2$-$C_6$-alkanoyl, dessen Phenylring gegebenenfalls 1 bis 3 Substituenten besitzt, die aus $C_1$-$C_6$-Alkyl, $C_1$-$C_6$-Alkoxy und Amino mit gegebenenfalls einem $C_1$-$C_6$-Alkanoylsubstituenten ausgewählt sind, phthalimidosubstituiertes $C_2$-$C_6$-Alkanoyl, $C_1$-$C_6$-Alkoxycarbonyl, $C_2$-$C_6$-Alkanoyl, Carboxy-$C_2$-$C_6$-alkanoyl, Naphthyloxy-$C_2$-$C_6$-alkanoyl, halogensubstituiertes $C_1$-$C_6$-Alkanoyl, eine Gruppe der Formel

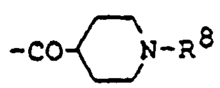

(worin $R^8$ ein Wasserstoffatom, $C_1$-$C_6$-Alkyl, Phenyl-$C_1$-$C_6$-alkoxycarbonyl, Carbamoyl-$C_1$-$C_6$-alkyl, Amino-$C_1$-$C_6$-alkanoyl mit gegebenenfalls einem $C_1$-$C_6$-Alkylsubstituenten oder $C_1$-$C_6$-Alkanoyl ist), Anilinocarbonyl, das gegebenenfalls einen $C_1$-$C_6$-Alkylsubstituenten am Phenylring besitzt, Phenoxycarbonyl, Phenylsulfonyl, das gegebenenfalls einen Substituenten besitzt, der aus einem Halogenatom und $C_1$-$C_6$-Alkyl am Phenylring ausgewählt ist, Chinolylsulfonyl oder eine Gruppe der Formel

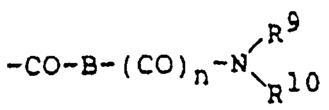

ist (worin B $C_1$-$C_6$-Alkylen ist, n eine ganze Zahl 0 oder 1 ist und $R^9$ und $R^{10}$ gleich oder verschieden sind und jeweils ein Wasserstoffatom, $C_1$-$C_6$-Alkyl mit gegebenenfalls einem Hydroxysubstituenten, $C_3$-$C_8$-Cycloalkyl, Phenyl-$C_1$-$C_6$-alkyl, $C_1$-$C_6$-Alkanoyl, $C_2$-$C_6$-Alkenyl, Phenoxy-$C_1$-$C_6$-alkyl, Phenyl, das gegebenenfalls 1 bis 3 Substituenten besitzt, die aus Amino-$C_1$-$C_6$-alkyl mit gegebenenfalls einem $C_1$-$C_6$-Alkanoylsubstituenten, $C_1$-$C_6$-Alkyl, $C_1$-$C_6$-Alkoxy und einem Halogenatom ausgewählt sind, phthalimidosubstituiertes $C_1$-$C_6$-Alkyl, Amino-$C_1$-$C_6$-alkyl mit gegebenenfalls einem $C_1$-$C_6$-Alkanoylsubstituenten, $C_2$-$C_6$-Alkinyl oder Amino-$C_1$-$C_6$-alkyl mit gegebenenfalls einem $C_1$-$C_6$-Alkylsubstituenten sind, oder $R^9$ und $R^{10}$ sich zusammen mit dem Stickstoffatom, an das sie binden, unter Bilden einer 5- oder 6-gliedrigen, gesättigten, heterocyclischen Gruppe verbinden können, die durch ein Stickstoff- oder Sauerstoffatom unterbrochen ist oder nicht, wobei die heterocyclische Gruppe gegebenenfalls einen Substituenten besitzt, der aus $C_1$-$C_6$-Alkyl, $C_1$-$C_6$-Alkoxycarbonyl und Piperidinyl ausgewählt ist), und ein Salz derselben.

**32.** Verfahren gemäß Anspruch 22, wobei $R^4$ $C_1$-$C_6$-Alkyl ist, und ein Salz derselben.

**33.** Verfahren gemäß Anspruch 24, wobei $R^4$ ein Wasserstoff ist und $R^5$ eine Gruppe der Formel

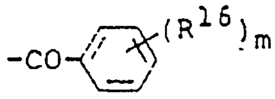

ist (worin $R^{16}$ und m wie in Anspruch 1 definiert sind), und ein Salz derselben.

**34.** Verfahren gemäß Anspruch 24, wobei $R^4$ ein Wasserstoffatom ist und $R^5$ Phenyl-$C_1$-$C_6$-alkoxycarbonyl, $C_1$-$C_6$-Alkanoyl, Phenyl-$C_1$-$C_6$-alkanoyl, $C_3$-$C_8$-Cycloalkyl-$C_2$-$C_6$-alkanoyl, $C_3$-$C_8$-Cycloalkylcarbonyl, Tricyclo[3.3.1.1]decanylcarbonyl, Naphthylcarbonyl, Pyridylcarbonyl, Furoyl, Thenoyl, Phenoxy-$C_2$-$C_6$-alkanoyl, dessen Phenylring gegebenenfalls 1 bis 3 Substituenten besitzt, die aus $C_1$-$C_6$-Alkyl, $C_1$-$C_6$-Alkoxy und Amino mit gegebenenfalls einem $C_1$-$C_6$-Alkanoylsubstituenten ausgewählt sind, phthalimidosubstituiertes $C_2$-$C_6$-Alkanoyl, $C_1$-$C_6$-Alkoxycarbonyl-$C_2$-$C_6$-alkanoyl, Carboxy-$C_2$-$C_6$-alkanoyl, Naphthyloxy-$C_2$-$C_6$-alkanoyl, halogensubstituiertes $C_1$-$C_6$-Alkanoyl, eine Gruppe der Formel

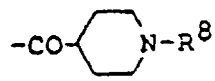

(worin $R^8$ ein Wasserstoffatom, $C_1$-$C_6$-Alkyl, Phenyl-$C_1$-$C_6$-alkoxycarbonyl, Carbamoyl-$C_1$-$C_6$-alkyl, Amino-$C_1$-$C_6$-alkanoyl mit gegebenenfalls einem $C_1$-$C_6$-Alkylsubstituenten oder $C_1$-$C_6$-Alkanoyl ist), Anilinocarbonyl, das gegebenenfalls einen $C_1$-$C_6$-Alkylsubstituenten am Phenylring besitzt, Phenoxycarbonyl, Phenylsulfonyl, das gegebenenfalls einen Substituenten besitzt, der aus einem Halogenatom und $C_1$-$C_6$-Alkyl am Phenylring ausgewählt ist, Chinolylsulfonyl, oder eine Gruppe der Formel

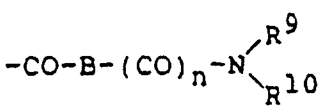

ist (worin B $C_2$-$C_6$-Alkylen ist, n eine ganze Zahl 0 oder 1 ist und $R^9$ und $R^{10}$ gleich oder verschieden sind und jeweils ein Wasserstoffatom, $C_1$-$C_6$-Alkyl mit gegebenenfalls einem Hydroxysubstituenten, $C_3$-$C_8$-Cycloalkyl, Phenyl-$C_1$-$C_6$-alkyl, $C_1$-$C_6$-Alkanoyl, $C_2$-$C_6$-Alkenyl, Phenoxy-$C_1$-$C_6$-alkyl, Phenyl, das gegebenenfalls 1 bis 3 Substituenten besitzt, die aus Amino-$C_1$-$C_6$-alkyl mit gegebenenfalls einem $C_1$-$C_6$-Alkanoylsubstituenten, $C_1$-$C_6$-Alkyl, $C_1$-$C_6$-Alkoxy und einem Halogenatom ausgewählt sind, phthalimidosubstituiertes $C_1$-$C_6$-Alkyl, Amino-$C_1$-$C_6$-alkyl mit gegebenenfalls einem $C_1$-$C_6$-Alkanoylsubstituenten, $C_2$-$C_6$-Alkinyl oder Amino-$C_1$-$C_6$-alkyl mit gegebenenfalls einem $C_1$-$C_6$-Alkylsubstituenten sind oder $R^9$ und $R^{10}$ sich zusammen mit dem Stickstoffatom, an das sie binden, unter Bilden einer 5- oder 6-gliedrigen, gesättigten, heterocyclischen Gruppe verbinden können, die durch ein Stickstoff- oder Sauerstoffatom unterbrochen ist oder nicht, wobei die heterocyclische Gruppe gegebenenfalls einen Substituenten besitzt, der aus $C_1$-$C_6$-Alkyl, $C_1$-$C_6$-Alkoxycarbonyl und Piperidinyl ausgewählt ist), und ein Salz derselben.

**35.** Verfahren gemäß Anspruch 24, wobei $R^4$ $C_1$-$C_6$-Alkyl ist, und ein Salz derselben.

**36.** Verfahren gemäß Anspruch 15, wobei $R^4$ ein Wasserstoffatom ist und $R^5$ eine Gruppe der Formel

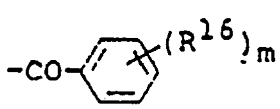

ist (worin $R^{16}$ und m wie in Anspruch 1 definiert sind), und ein Salz derselben.

**37.** Verfahren gemäß Anspruch 5, 15, 18 bis 21, 25 bis 27, wobei $R^4$ ein Wasserstoffatom ist und $R^5$ wie in Anspruch 31 definiert ist.

**38.** Verfahren gemäß Anspruch 15, wobei $R^4$ $C_1$-$C_6$-Alkyl ist, und ein Salz derselben.

**39.** Verfahren gemaß Anspruch 26, wobei $R^4$ ein Wasserstoffatom ist und $R^5$ eine Gruppe der Formel

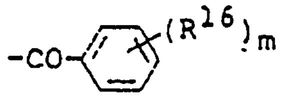

ist (wobei $R^{16}$ und m wie in Anspruch 1 definiert sind), und ein Salz derselben.

**40.** Verfahren gemaß Anspruch 26, wobei $R^4$ $C_1$-$C_6$-Alkyl ist, und ein Salz derselben.

**41.** Verfahren gemäß Anspruch 28, wobei $R^4$ ein Wasserstoffatom ist, und $R^5$ eine Gruppe der Formel

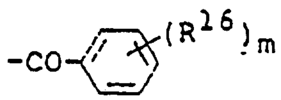

ist (worin $R^{16}$ und m wie in Anspruch 1 definiert sind), und ein Salz derselben.

**42.** Verfahren gemäß Anspruch 28, wobei $R^4$ $C_1$-$C_6$-Alkyl ist, und ein Salz derselben.

**43.** Verfahren gemäß Anspruch 18, wobei $R^4$ ein Wasserstoffatom ist und $R^5$ eine Gruppe der Formel

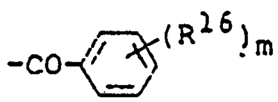

ist (worin $R^{16}$ und m wie in Anspruch 1 definiert sind), und ein Salz derselben.

**44.** Verfahren gemäß Anspruch 18, wobei $R^4$ $C_1$-$C_6$-Alkyl ist, und ein Salz derselben.

**45.** Verfahren gemäß Anspruch 17, wobei $R^4$ ein Wasserstoffatom ist und $R^5$ eine Gruppe der Formel

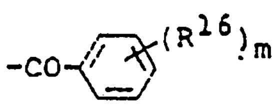

ist (worin $R^{16}$ und m wie in Anspruch 1 definiert sind), und ein Salz derselben.

**46.** Verfahren gemäß Anspruch 19, wobei $R^4$ $C_1$-$C_6$-Alkyl ist, und ein Salz derselben.

**47.** Verfahren gemäß Anspruch 20, wobei $R^4$ ein Wasserstoffatom ist und $R^5$ eine Gruppe der Formel

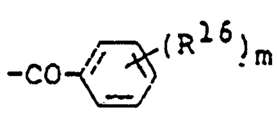

ist (worin $R^{16}$ und m wie in Anspruch 1 definiert sind), und ein Salz derselben.

**48.** Verfahren gemäß Anspruch 20, wobei $R^4$ $C_1$-$C_6$-Alkyl ist, und ein Salz derselben.

**49.** Verfahren gemäß Anspruch 21, wobei $R^4$ ein Wasserstoffatom ist und $R^5$ eine Gruppe der Formel

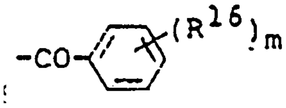

ist (worin $R^{16}$ und m wie in Anspruch 1 definiert sind), und ein Salz derselben.

**50.** Verfahren gemäß Anspruch 21, wobei $R^4$ $C_1$-$C_6$-Alkyl ist, und ein Salz derselben.

**51.** Verfahren gemäß Anspruch 30, wobei $R^{16}$ ein Halogenatom oder $C_1$-$C_6$-Alkyl mit gegebenenfalls einem Substituenten ist, der aus einem Halogenatom und Hydroxy ausgewählt ist, und ein Salz derselben.

**52.** Verfahren gemaß Anspruch 33, wobei $R^{16}$ ein Halogenatom oder $C_1$-$C_6$-Alkyl mit gegebenenfalls einem Substituenten ist, der aus einem Halogenatom und Hydroxy ausgewählt ist, und ein Salz derselben.

**53.** Verfahren gemäß Anspruch 39, wobei $R^{16}$ ein Halogenatom oder $C_1$-$C_6$-Alkyl mit gegebenenfalls einem Substituenten ist, der aus einem Halogenatom und Hydroxy ausgewählt ist, und ein Salz derselben.

**54.** Verfahren gemäß Anspruch 42, wobei $R^{16}$ ein Halogenatom oder $C_1$-$C_6$-Alkyl mit gegebenenfalls einem Substituenten ist, der aus einem Halogenatom und Hydroxy ausgewählt ist,und ein Salz derselben.

**55.** Verfahren gemäß Anspruch 1 bis 9, wobei W eine Gruppe der Formel $-(CH_2)_p-$ ist, worin p eineganze Zahl 3 bis 5 ist und die $-(CH_2)_p$-Gruppe gegebenenfalls weiter 1 bis 3 in Anspruch 1 definierte Substituenten besitzt.

**56.** Verfahren gemäß Anspruch 1 bis 9, wobei W eine Gruppe der Formel $-CH{=}CH-(CH_2)_q-$ ist, worin q eine ganze Zahl 1 bis 3 ist und die $-CH{=}CH(CH_2)_q$-Gruppe gegebenenfalls weiter 1 bis 3 Substituenten besitzt, die aus $C_1$-$C_6$-Alkyl mit gegebenenfalls einem Hydroxysubstituenten und Amino-$C_1$-$C_6$-alkyl mit gegebenenfalls einem Substituenten ausgewählt sind,der aus $C_1$-$C_6$-Alkyl und $C_1$-$C_6$-Alkanoyl ausgewählt ist, und ein Salz derselben.

**57.** Verfahren gemäß Anspruch 55, wobei W eine Gruppe der Formel $-(CH_2)_p-$ ist (p ist eine ganze Zahl 3 bis 5) und die $-(CH_2)_p$-Gruppe gegebenenfalls 1 bis 3 in Anspruch 1 definierte Substituenten besitzt.

**58.** Verfahren gemäß Anspruch 57, wobei p in der Gruppe $-(CH_2)_p-$ 3 ist und die Gruppe keinen Substituenten besitzt, und ein Salz derselben.

**59.** Verfahren gemäß Anspruch 57, wobei p in der Gruppe $-(CH_2)_p-$ 3 ist und die Gruppe einen Substituenten einer Gruppe der Formel

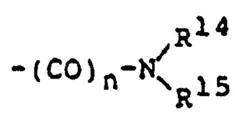

besitzt (worin $R^{14}$, $R^{15}$ und n wie vorstehend definiert sind), und ein Salz derselben.

**60.** Verfahren gemäß Anspruch 57, wobei p in der Gruppe $-(CH_2)_p-$ 4 ist und die Gruppe keinen Substituenten besitzt, und ein Salz derselben.

**61.** Verfahren gemäß Anspruch 57, wobei p in der Gruppe $-(CH_2)_p-$ 4 ist und die Gruppe einen Substituenten einer Gruppe der Formel

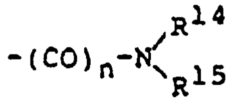

besitzt (worin $R^{14}$, $R^{15}$ und n wie vorstehend definiert sind), und ein Salz derselben.

**62.** Verfahren gemäß Anspruch 57, wobei p in der Gruppe $-(CH_2)_p-$ 5 ist, und ein Salz derselben.

**63.** Verfahren gemäß Anspruch 56, wobei q in der Gruppe $-CH=CH-(CH_2)_q-$ 1 ist, und ein Salz derselben.

**64.** Verfahren gemäß Anspruch 56, wobei q in der Gruppe $-CH=CH-(CH_2)_q-$ 2 ist, und ein Salz derselben.

**65.** Verfahren gemäß Anspruch 56, wobei q in der Gruppe $-CH=CH-(CH_2)_q-$ 3 ist, und ein Salz derselben.

**66.** Verfahren gemäß Anspruch 59 oder 61, wobei n in dem Substituenten

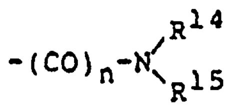

0 ist und $R^{14}$ und $R^{15}$ gleich oder verschieden sind und jeweils ein Wasserstoffatom, $C_1$-$C_6$-Alkyl oder $C_3$-$C_8$-Cycloalkyl sind, und ein Salz derselben.

**67.** Verfahren gemäß Anspruch 64, wobei die heterocyclische Gruppe der Formel

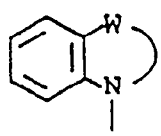

2,3-Dihydro-1H-benzazepin ist, und ein Salz derselben.

**68.** Verfahren gemäß Anspruch 1 bis 9 zur Herstellung von
1-[4-(2-Methylbenzoylamino)benzoyl]-4-methyl-2,3,4,5-tetrahydro-1H-1,4-benzodiazepin,
5-Dimethylamino-1-[4-(2-methylbenzoylamino)benzoyl]-2,3,4,5-tetrahydro-1H-benzazepin,
5-Dimethylamino-1-[2-chlor-4-(2-methylbenzoylamino)benzoyl]-2,3,4,5-tetrahydro-1H-benzazepin,
5-Methylamino-1-[2-chlor-4-(2-methylbenzoylamino)benzoyl]-2,3,4,5-tetrahydro-1H-benzazepin,
5-Cyclopropylamino-1-[2-chlor-4-(2-methylbenzoylamino)benzoyl]-2,3,4,5-tetrahydro-1H-benzazepin,
5-Cyclopropylamino-1-[2-chlor-4-(2-chlorbenzoylamino)benzoyl]-2,3,4,5-tetrahydro-1H-benzazepin,
5-Dimethylamino-1-[2-methyl-4-(2-methylbenzoylamino)benzoyl]-2,3,4,5-tetrahydro-1H-benzazepin,
4-Dimethylamino-1-[3-methoxy-4-(2-methylbenzoylamino)benzoyl]-1,2,3,4-tetrahydrochinolin,
7-Chlor-5-methylamino-1-[4-(2-methylbenzoylamino)benzoyl]-2,3,4,5-tetrahydro-1H-benzazepin,
7-Chlor-5-methylamino-1-[4-(2-chlorbenzoylamino)benzoyl]-2,3,4,5-tetrahydro-1H-benzazepin,
5-Hydroxysulfonyloxyimino-1-[2-chlor-4-(2-methylbenzoylamino)benzoyl]-2,3,4,5-tetrahydro-1H-benzazepin.

**69.** Verfahren gemäß Anspruch 1 bis 9 zur Herstellung von Verbindungen der Formeln

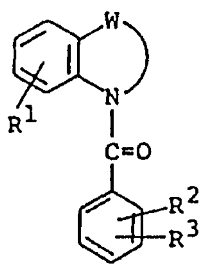

worin

a)

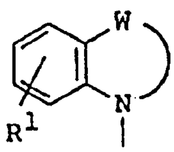

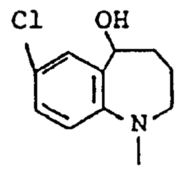

darstellt,

$R^2$ 2-$OCH_3$ darstellt und

$R^3$

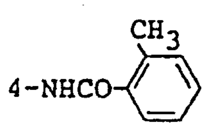

darstellt und

b)

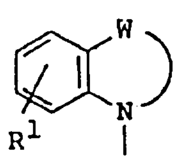

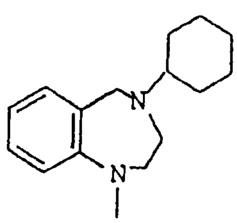

darstellt,

$R^2$ H darstellt und

$R^3$

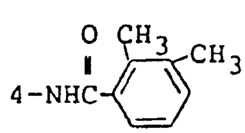

darstellt.

**70.** Verfahren gemäß Anspruch 1 bis 9 zur Herstellung einer benzoheterocyclischen Verbindung der Formel:

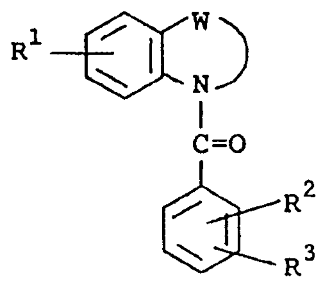

worin $R^1$, $R^2$ und $R^3$ wie in Anspruch 1 definiert sind und die heterocyclische Gruppe der Formel

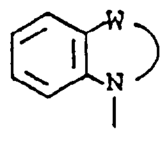

ein aus der Gruppe ausgewähltes Mitglied ist, die aus 3,4-Dihydro-2H-1,4-benzoxazinyl, 1,2,3,5-Tetrahydro-4,1-benzoxazepinyl, 1,2,3,4-Tetrahydrochinoxalinyl, 1,2,3,4,5,6-Hexahydro-1,5-benzodiazocinyl, 5-Methyl-1,2,3,4,5,6-Hexahydro-1,5-benzodiazocinyl, 4-Methyl-1,2,3,4-Tetrahydrochinoxalinyl, 1,2,3,4-Tetrahydro-5,1-benzoxazepinyl, 3,4-Dihydro-2H-1,4-benzothiazinyl, 2,3,4,5-Tetrahydro-1,5-benzothiazepinyl, 1,2,3,5-Tetrahydro-4,1-benzothiazepinyl, 4-Ethyl-1,2,3,4-tetrahydrochinoxalinyl, 4-Propyl-1,2,3,4-tetrahydrochinoxalinyl, 4-Butyl-1,2,3,4-tetrahydrochinoxalinyl, 4-Pentyl-1,2,3,4-tetrahydrochinoxalinyl, 4-Hexyl-1,2,3,4-tetrahydrochinoxalinyl, 2,3,4,5-Tetrahydro-1H-1,4-benzodiazepinyl, 4-Methyl-2,3,4,5-tetrahydro-1H-1,4-benzodiazepinyl, 4-Ethyl-2,3,4,5-tetrahydro-1H-1,4-benzodiazepinyl, 4-Propyl-2,3,4,5-tetrahydro-1H-1,4-benzodiazepinyl, 4-Butyl-2,3,4,5-tetrahydro-1H-1,4-benzodiazepinyl, 4-Pentyl-2,3,4,5-tetrahydro-1H-1,4-benzodiazepinyl, 4-Hexyl-2,3,4,5-tetrahydro-1H-1,4-benzodiazepinyl, 2,3,4,5-Tetrahydro-1H-1,5-benzodiazepinyl, 5-Methyl-2,3,4,5-tetrahydro-1H-1,5-benzodiazepinyl, 5-Ethyl-2,3,4,5-tetrahydro-1H-1,5-benzodiazepinyl, 5-Propyl-2,3,4,5-tetrahydro-1H-1,5-benzodiazepinyl, 5-Butyl-2,3,4,5-tetrahydro-1H-1,5-benzodiazepinyl, 5-Pentyl-2,3,4,5-tetrahydro-1H-1,5-benzodiazepinyl, 5-Hexyl-2,3,4,5-tetrahydro-1H-1,5-benzodiazepinyl, 3,4-Dihydro-1-oxo-2H-1,4-benzothiazepinyl, 3,4-Dihydro-1,1-dioxo-2H-1,4-benzothiazepinyl, 1-Oxo-2,3,4,5-tetrahydro-1,5-benzothiazepinyl, 1,1-Dioxo-2,3,4,5-tetrahydro-1,5-benzothiazepinyl, 4-Oxo-1,2,3,5-tetrahydro-4,1-benzothiazepinyl und 4,4-Dioxo-1,2,3,5-tetrahydro-4,1-benzothiazepinyl besteht, und ein Salz derselben.

**71.** Verfahren zur Herstellung einer vasopressin-antagonistischen Zusammensetzung, die als aktiven Bestandteil eine gemäß dem Verfahren von Anspruch 1 bis 9 hergestellte Verbindung der Formel (1) oder ein pharmazeutisch annehmbares Salz derselben umfaßt, durch ihr Mischen mit einem pharmazeutisch annehmbaren Träger oder Verdünnungsmittel.

## Revendications

### Revendications pour les Etats contractants suivants : CH, DE, DK FR, GB, IT, LI, NL, SE

**1.** Composé benzohétérocyclique de formule suivante :

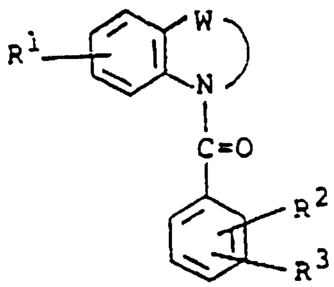

(1)

dans laquelle

$R^1$ est un atome d'hydrogène, un atome d'halogène, un groupe alkyle en $C_1$-$C_6$, un groupe amino ayant facultativement un substituant alkyle en $C_1$-$C_6$ ou un groupe alcoxy en $C_1$-$C_6$,

$R^2$ est un atome d'hydrogène, un atome d'halogène, un groupe alcoxy en $C_1$-$C_6$, un groupe phényl-alcoxy en $C_1$-$C_6$, un groupe hydroxy, un groupe alkyle en $C_1$-$C_6$, un groupe amino ayant faculativement un substituant alkyle en $C_1$-$C_6$, un groupe carbamoyl-alcoxy en $C_1$-$C_6$, un groupe amino-alcoxy en $C_1$-$C_6$ ayant facultativement un substituant alkyle en $C_1$-$C_6$ ou un groupe benzoyloxy qui a facultativement sur le noyau phényle un substituant halogène,

$R^3$ est un groupe de formule

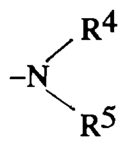

ou un groupe de formule

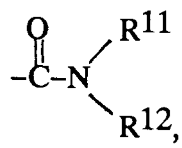

$R^4$ est un atome d'hydrogène, un groupe benzoyle qui a facultativement sur le noyau phényle un substituant halogène ou un groupe alkyle en $C_1$-$C_6$,

$R^5$ est (a) un groupe de formule

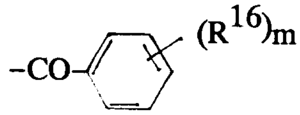

[où $R^{16}$ est (i) un atome d'halogène ; (ii) un groupe alkyle en $C_1$-$C_6$ qui a facultativement un substituant choisi parmi un atome d'halogène et un groupe hydroxy ; (iii) un groupe hydroxy ; (iv) un groupe alcoxy en $C_1$-$C_6$ ; (v) un groupe alcanoyloxy en $C_1$-$C_6$ ; (vi) un groupe alkylthio en $C_1$-$C_6$ ; (vii) un groupe alcanoyl en $C_1$-$C_6$ ; (viii) un groupe carboxy ; (ix) un groupe (alcoxy en $C_1$-$C_6$)carbonyle ; (x) un groupe cyano ; (xi) un groupe nitro ; (xii) un groupe amino qui a facultativement un substituant choisi parmi un groupe alkyle en $C_1$-$C_6$ et un groupe alcanoyle en $C_1$-$C_6$ ; (xiii) un

groupe phényle ; (xiv) un groupe cycloalkyle en $C_3$-$C_8$ ; (xv) un groupe (alcanoyloxy en $C_2$-$C_6$)-alcoxy en $C_1$-$C_6$ ; (xvi) un groupe carboxy-alcoxy en $C_1$-$C_6$ ; (xvii) un groupe halogéno-alcoxy en $C_1$-$C_6$ ; (xviii) un groupe carbamoyl-alcoxy en $C_1$-$C_6$ ; (xix) un groupe hydroxy-alcoxy en $C_1$-$C_6$ ; (xx) un groupe (alcoxy en $C_1$-$C_6$) carbonyl-alcoxy en $C_1$-$C_6$ ; (xxi) un groupe phtalimido-alcoxy en $C_1$-$C_6$ ; (xxii) un groupe aminocarbonyl-alcoxy en $C_1$-$C_6$ ayant un substituant alkyle en $C_1$-$C_6$ ; ou (xxiii) un groupe de formule

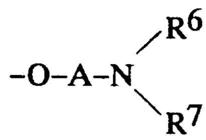

(A est un groupe alkylène jusqu'en $C_6$ et

$R^6$ et $R^7$ sont les mêmes ou différents et sont chacun un atome d'hydrogène, un groupe alkyle en $C_1$-$C_6$ ayant facultativement un substituant hydroxy, un groupe alcanoyle en $C_1$-$C_6$ ou un groupe benzoyle, ou bien

$R^6$ et $R^7$ peuvent être reliés ensemble peur former avec l'atome d'azote auquel ils sont fixés un groupe hétérocyclique saturé à 5 ou 6 chaînons avec ou sans atome d'azote ou d'oxygène intercalé, dans lequel le groupe hétérocyclique a facultativement un substituant choisi parmi un groupe pipéridinyle et un groupe alkyle en $C_1$-$C_6$); et m est un entier de 0 à 3],

(b) un groupe phényl-(alcoxy en $C_1$-$C_6$)carbonyle,
(c) un groupe alcanoyle en $C_1$-$C_6$,
(d) un groupe phényl-alcanoyle en $C_2$-$C_6$,
(e) un groupe (cycloalkyl en $C_3$-$C_8$)-alcanoyle en $C_2$-$C_6$,
(f) un groupe (cycloalkyl en $C_3$-$C_8$)carbonyle,
(g) un groupe tricyclo[3.3.1.1]décanylcarbonyle,
(h) un group naphtylcarbonyle,
(i) un groupe pyridylcarbonyle,
(j) un groupe furoyle,
(k) un groupe thénoyle,
(l) un groupe phénoxy-alcanoyle en $C_2$-$C_6$ dont le noyau phényle a facultativement 1 à 3 substituants choisis parmi un groupe alkyle en $C_1$-$C_6$, un groupe alcoxy en $C_1$-$C_6$ et un groupe amino ayant facultativement un substituant alcanoyle en $C_1$-$C_6$,
(m) un groupe phtalimido-alcanoyle en $C_2$-$C_6$,
(o) un groupe(alcoxy en $C_1$-$C_6$)carbonyl-alcanoyle en $C_2$-$C_6$,
(p) un groupe carboxy-alcanoyle en $C_2$-$C_6$,
(q) un groupe naphtyloxy-alcanoyle en $C_2$-$C_6$,
(r) un groupe halogénoalcanoyle en $C_1$-$C_6$,
(s) un groupe de formule

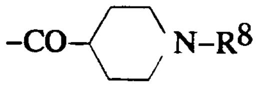

(dans laquelle $R^8$ est (i) un atome d'hydrogène, (ii) un groupe alkyle en $C_1$-$C_6$, (iii) un groupe phényl(alcoxy en $C_1$-$C_6$)carbonyle, (iv) un groupe carbamoyl-alkyle en $C_1$-$C_6$, (v) un groupe amino-alcanoyle en $C_2$-$C_6$ ayant facultativement un substituant alkyle en $C_1$-$C_6$ ou (vi) un groupe alcanoyle en $C_1$-$C_6$),
(t) un groupe anilinocarbonyle qui a facultativement sur le noyau phényle un substituant alkyle en $C_1$-$C_6$,
(u) un groupe phénoxycarbonyle,
(v) un groupe phénylsulfonyle qui a facultativement sur le noyau phényle un substituant choisi parmi un atome d'halogène et un groupe alkyle en $C_1$-$C_6$,
(w) un groupe quinolysulfonyle ou

(x) un groupe de formule

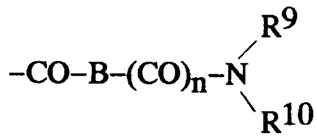

dans laquelle

B est un groupe alkylène en $C_1$-$C_6$, n est un entier égal à 0 ou 1 et $R^9$ et $R^{10}$ sont les mêmes ou différents et sont chacun (i) un atome d'hydrogène, (ii) un groupe alkyle jusqu'en $C_6$ ayant facultativement un substituant hydroxy, (iii) un groupe cycloalkyle en $C_3$-$C_8$, (iv) un groupe phényl-alkyle en $C_1$-$C_6$, (v) un groupe alcanoyle en $C_1$-$C_6$, (vi) un groupe alcényle jusqu'en $C_6$, (vii) un groupe phénoxy-alkyle en $C_1$-$C_6$, (viii) un groupe phényle qui a facultativement 1 à 3 substituants choisis parmi un groupe amino-alkyle en $C_1$-$C_6$ ayant facultativement un substituant alcanoyle en $C_1$-$C_6$, un groupe alkyle en $C_1$-$C_6$, un groupe alcoxy en $C_1$-$C_6$ et un atome d'halogène, (ix) un groupe phtalimido-alkyle en $C_1$-$C_6$, (x) un groupe amino-alkyle en $C_1$-$C_6$ ayant facultativement un substituant alcanoyle en $C_1$-$C_6$, (xi) un groupe alcynyle jusqu'en $C_6$ ou (xii) un groupe amino-alkyle en $C_1$-$C_6$ ayant facultativement un substituant alkyle en $C_1$-$C_6$, ou bien $R^9$ et $R^{10}$ peuvent être reliés ensemble pour former avec l'atome d'azote auquel ils sont liés un groupe hétérocyclique saturé à 5 ou 6 chaînons avec ou sans atome d'azote ou d'oxygène intercalé, où le groupe hétérocyclique a facultativement un substituant choisi parmi un groupe alkyle en $C_1$-$C_6$, un groupe (alcoxy en $C_1$-$C_6$)carbonyle et un groupe pipéridinyle,

$R^{11}$ est un atome d'hydrogène ou un groupe alkyle en $C_1$-$C_6$,

$R^{12}$ est un groupe cycloalkyle en $C_3$-$C_8$ ou un groupe phényle qui a facultativement 1 à 3 substituants choisis parmi un groupe alcoxy en $C_1$-$C_6$, un groupe alkyle en $C_1$-$C_6$ et un atome d'halogène,

W est un groupe de formule $-(CH_2)p-$ (p est un entier de 3 à 5) ou un groupe de formule $-CH=CH-(CH_2)_q-$ (q est entier de 1 à 3), ou dans lequel le groupe hétérocyclique de formule :

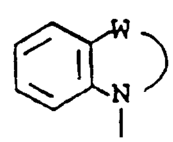

est un groupe 2,3,4,5-tétrahydro-1H-1,4-benzodiazépine

et en outre ledit groupe $-(CH_2)_p-$ ayant facultativement 1 à 3 substituants choisis parmi (i) un groupe alkyle en $C_1$-$C_6$ ayant facultativement un substituant hydroxy, (ii) un groupe (alcoxy en $C_1$-$C_6$)carbonyle, (iii) un groupe carboxy, (iv) un groupe hydroxy, (v) un reste oxo, (vi) un groupe alcanoyloxy en $C_1$-$C_6$ ayant facultativement un substituant halogène, (vii) un groupe amino-alkyle en $C_1$-$C_6$ ayant facultativement un substituant choisi parmi un groupe alkyle en $C_1$-$C_6$ et un groupe alcanoyle en $C_1$-$C_6$, (viii) un groupe (alcanoyloxy en $C_2$-$C_6$)-alkyle en $C_1$-$C_6$, (ix) un groupe (alkyl en $C_1$-$C_6$)-sulfonyloxy-alkyle en $C_1$-$C_6$, (x) un groupe azido-alkyle en $C_1$-$C_6$, (xi) un groupe de formule $-O-CH_2-$ (qui est relié au groupe $-(CH_2)_p-$ ou $CH=CH-(CH_2)_q-$ de manière à être sous la forme spiro), (xii) un groupe aminocarbonyloxy ayant facultativement un substituant alkyle en $C_1$-$C_6$, (xiii) un groupe alcoxy en $C_1$-$C_6$, (xiv) un groupe (alcoxy en $C_1$-$C_6$)carbonyl-alcoxy en $C_1$-$C_6$, (xv) un groupe carboxy-alcoxy en $C_1$-$C_6$, (xvi) un groupe aminocarbonyl-alcoxy en $C_1$-$C_6$ ayant facultativement un substituant alkyle en $C_1$-$C_6$, (xvii) un groupe amino-alcoxy en $C_1$-$C_6$ ayant facultativement un substituant choisi parmi un groupe alkyle en $C_1$-$C_6$ et un groupe alcanoyle en $C_1$-$C_6$, (xviii) un groupe phtalimido-alcoxy en $C_1$-$C_6$, (xix) un groupe hydroxyimino, (xx) un groupe (alcanoyloxy en $C_1$-$C_6$)imino, (xxi) un groupe alkylidène en $C_1$-$C_6$, (xxii) un atome d'halogène, (xxiii) un groupe azido, (xxvi) un groupe hydrazino, (xxvii) un groupe pyrrolyle, (xxviii) un groupe amino-alcanoyloxy en $C_1$-$C_6$ ayant facultativement un substituant alkyle en $C_1$-$C_6$, (xxix) un groupe de formule

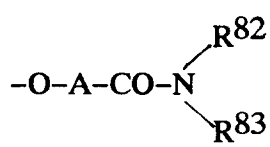

(A est défini comme comme ci-dessus et

$R^{82}$ et $R^{83}$ sont les mêmes ou différents et sont chacun un atome d'hydrogène, un groupe alkyle en $C_1$-$C_6$, un groupe carbamoyl-alkyle en $C_1$-$C_6$, un groupe hydroxy-alkyle en $C_1$-$C_6$ ou un groupe pyridyl-alkyle en $C_1$-$C_6$,

ou bien $R^{82}$ et $R^{83}$ peuvent être reliés ensemble pour former avec l'atome d'azote auquel ils sont liés un groupe hétérocyclique saturé à 5 ou 6 chaînons avec ou sans atome d'azote d'oxygène ou de soufre intercalé, dans lequel le groupe hétérocyclique a facultativement un substituant choisi parmi un reste oxo, un groupe alkyle en $C_1$-$C_6$, un groupe alcanoyle en $C_1$-$C_6$ et un groupe carbamoyle), et

(xxx) un groupe de fomule

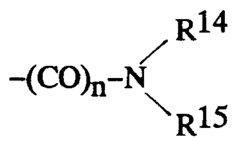

(dans laquelle n est défini comme ci-dessus et $R^{14}$ et $R^{15}$ sont les mêmes ou différents et sont chacun (i) un atome d'hydrogène, (ii) un groupe alkyle en $C_1$-$C_6$, (iii) un groupe alcényle jusqu'en $C_6$, (iv) un groupe alcanoyle en $C_1$-$C_6$, (v) un groupe cycloalkyle en $C_3$-$C_8$, (vi) un groupe oxiranyl-alkyle en $C_1$-$C_6$, (vii) un groupe alkyle en $C_1$-$C_6$ ayant 1 à 2 substituants choisis parmi un groupe alcoxy en $C_1$-$C_6$, un groupe hydroxy et un groupe amino ayant facultativement un substituant alkyle en $C_1$-$C_6$, (viii) un groupe phényl-alkyle en $C_1$-$C_6$, (ix) un groupe pyridyl-alkyle en $C_1$-$C_6$, (x) un groupe alkylsulfonyle en $C_1$-$C_6$, (xi) un groupe benzoyle, (xii) un groupe (alcoxy en $C_1$-$C_6$)carbonyle, (xiii) un groupe anilinocarbonyle, (xiv) un groupe aminocarbonyle ayant facultativement un substituant alkyle en $C_1$-$C_6$, (xv) un groupe cyano-alkyle en $C_1$-$C_6$, (xvi) un groupe (alcoxy en $C_1$-$C_6$)carbonyl-alkyle en $C_1$-$C_6$, (xvii) un groupe carbamoyl-alkyle en $C_1$-$C_6$, (xviii) un groupe carboxy-alkyle en $C_1$-$C_6$, (xix) un groupe tétrahydropyranyloxy-alkyle en $C_1$-$C_6$, (xx) un groupe (alcanoyloxy en $C_2$-$C_6$)-alkyle en $C_1$-$C_6$, (xxi) un groupe pipéridinyle ayant facultativement sur le noyau pipéridinyle un substituant phényl-alkyle en $C_1$-$C_6$, (xxii) un groupe halogéno-alcanoyle en $C_2$-$C_6$, (xxiii) un groupe imidazolyl-alcanoyle en $C_2$-$C_6$, (xxiv) un groupe amino-alcanoyle en $C_2$-$C_6$ ayant facultativement un substituant choisi parmi un groupe alkyle en $C_1$-$C_6$ et un groupe (alcoxy en $C_1$-$C_6$-carbonyle), (xxv) un groupe amino-carbonyl-alkyle en $C_1$-$C_6$ ayant facultativement un substituant alkyle en $C_1$-$C_6$ ou (xxvi) un groupe phényl-(alcoxy en $C_1$-$C_6$)-carbonyle, ou bien

$R^{14}$ et $R^{15}$ peuvent être reliés ensemble pour former avec l'atome d'azote auquel ils sont liés un groupe hétérocyclique saturé à 5 ou 6 chaînons avec ou sans atome d'azote ou d'oxygène intercalé, dans lequel le groupe hétérocyclique peut facultativement avoir un substituant choisi parmi un groupe alkyle en $C_1$-$C_6$, un groupe phényl-alkyle en $C_1$-$C_6$ ou un groupe alcanoyle en $C_1$-$C_6$) et en outre ledit groupe $-CH=CH-(CH_2)_q-$ ayant facultativement 1 à 3 substituants choisis parmi

(i) un groupe alkyle en $C_1$-$C_6$ ayant facultativement un substituant hydroxy et
(ii) un groupe amino-alkyle en $C_1$-$C_6$ ayant facultativement un substituant choisi parmi un groupe alkyle en $C_1$-$C_6$ et un groupe alcanoyle en $C_1$-$C_6$

et un de ses sels.

2. Composé selon la revendication 1, dans lequel $R^1$ dans la formule (1) est un atome d'hydrogène, et un de ses sels.

3. Composé selon la revendication 1, dans lequel $R^1$ dans la formule (1) est un atome d'halogène, et un de ses sels.

4. Composé selon la revendication 1, dans lequel $R^1$ dans la formule (1) est un groupe alkyle en $C_1$-$C_6$, un groupe amino ayant facultativement un substituant alkyle en $C_1$-$C_6$ ou un groupe alcoxy en $C_1$-$C_6$, et un de ses sels.

5. Composé selon la revendication 2, dans lequel $R^2$ est un atome d'hydrogène, et un de ses sels.

6. Composé selon la revendication 2, dans lequel $R^2$ est un atome d'halogène, un groupe alcoxy en $C_1$-$C_6$, ou un groupe alkyle en $C_1$-$C_6$ et un de ses sels.

7. Composé selon la revendication 2, dans lequel $R^2$ est un groupe phényl-alcoxy en $C_1$-$C_6$ ; un groupe hydroxy ; un groupe amino ayant facultativement un substituant alkyle en $C_1$-$C_6$ ; un groupe carbamoyl-alcoxy en $C_1$-$C_6$ ; un groupe amino-alcoxy en $C_1$-$C_6$ ayant facultativement un substituant alkyle en $C_1$-$C_6$ ; ou un groupe benzoyloxy ayant facultativement sur son noyau phényle un substituant halogène, et un de ses sels.

8. Composé selon la revendication 3, dans lequel $R^2$ est un atome d'hydrogène, et un de ses sels.

9. Composé selon la revendication 3, dans lequel $R^2$ est un atome d'halogène, un groupe alcoxy en $C_1$-$C_6$, ou un groupe alkyle en $C_1$-$C_6$ et un de ses sels.

10. Composé selon la revendication 3, dans lequel $R^2$ est un groupe phényl-alcoxy en $C_1$-$C_6$ ; un groupe hydroxy ; un groupe amino ayant facultativement un substituant alkyle en $C_1$-$C_6$ ; un groupe carbamoyl-alcoxy en $C_1$-$C_6$ ; un groupe amino-alcoxy en $C_1$-$C_6$ ayant facultativement un substituant alkyle en $C_1$-$C_6$ ; ou un groupe benzoyloxy ayant facultativement sur son noyau phényle un substituant halogène, et un de ses sels.

11. Composé selon la revendication 4, dans lequel $R^2$ est un atome d'hydrogène et un de ses sels.

12. Composé selon la revendication 4, dans lequel $R^2$ est un atome d'halogène, un groupe alcoxy en $C_1$-$C_6$ ou un groupe alkyle en $C_1$-$C_6$ et un de ses sels.

13. Composé selon la revendication 4, dans lequel $R^2$ est un groupe phényl-alcoxy en $C_1$-$C_6$ ; un groupe hydroxy ; un groupe amino ayant facultativement un substituant alkyle en $C_1$-$C_6$ ; un groupe carbamoyl-alcoxy en $C_1$-$C_6$ ; un groupe amino-alcoxy en $C_1$-$C_6$ ayant facultativement un substituant alkyle en $C_1$-$C_6$ ; ou un groupe benzoyloxy ayant facultativement sur son noyau phényle un substituant halogène, et un de ses sels.

14. Composé selon la revendication 5, dans lequel $R^3$ est un groupe de formule $-NR^4R^5$ ($R^4$ et $R^5$ sont tels que définis dans la revendication 1) et un de ses sels.

15. Composé selon la revendication 5, dans lequel $R^3$ est un groupe de formule $-CO-NR^{11}R^{12}$ ($R^{11}$ et $R^{12}$ sont tels que définis dans la revendication 1) et un de ses sels.

16. Composé selon la revendication 6, dans lequel $R^3$ est un groupe de formule $-NR^4R^5$ ($R^4$ et $R^5$ sont tels que définis dans la revendication 1) et un de ses sels.

17. Composé selon la revendication 6, dans lequel $R^3$ est un groupe de formule $-CO-NR^{11}R^{12}$ ($R^{11}$ et $R^{12}$ sont tels que définis dans la revendication 1) et un de ses sels.

18. Composé selon la revendication 8, dans lequel $R^3$ est un groupe de formule $-NR^4R^5$ ($R^4$ et $R^5$ sont tels que définis dans la revendication 1), et un de ses sels.

19. Composé selon la revendication 8, dans lequel $R^3$ est un groupe de formule $-CO-NR^{11}R^{12}$ ($R^{11}$ et $R^{12}$ sont tels que définis dans la revendication 1) et un de ses sels.

20. Composé selon la revendication 9, dans lequel $R^3$ est un groupe de formule $-NR^4R^5$ ($R^4$ et $R^5$ sont tels que définis dans la revendication 1) et un de ses sels.

21. Composé selon la revendication 9, dans lequel $R^3$ est un groupe de formule $-CO-NR^{11}R^{12}$ ($R^{11}$ et $R^{12}$ sont tels que définis dans la revendication 1) et un de ses sels.

22. Composé selon la revendication 14, dans lequel $R^4$ est un atome d'hydrogène et $R^5$ est un groupe de formule :

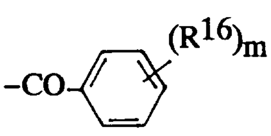

(dans laquelle $R^{16}$ et m sont tels que définis dans la revendication 1) et un de ses sels.

23. Composé selon la revendication 14, dans lequel $R^4$ est un atome d'hydrogène et $R^5$ est un groupe phényl-(alcoxy en $C_1$-$C_6$)carbonyle, un groupe alcanoyle en $C_1$-$C_6$, un groupe phényl-alcanoyle en $C_2$-$C_6$, un groupe (cycloalkyl en $C_3$-$C_8$)-alcanoyle en $C_2$-$C_6$, un groupe (cycloalkyl en $C_3$-$C_8$)carbonyle, un groupe tricyclo[3.3.1.1]décanylcarbonyle, un groupe naphtylcarbonyle, un groupe pyridylcarbonyle, un groupe furoyle, un groupe thénoyle, un groupe phénoxy-alcanoyle en $C_2$-$C_6$ dont le noyau phényle a facultativement 1 à 3 substituants choisis parmi un groupe

alkyle en $C_1$-$C_6$, un groupe alcoxy en $C_1$-$C_6$ et un groupe amino ayant facultativement un substituant alcanoyle en $C_1$-$C_6$, un groupe phtalimido-alcanoyle en $C_2$-$C_6$, un groupe (alcoxy en $C_1$-$C_6$)carbonyl-alcanoyle en $C_2$-$C_6$, un groupe carboxy-alcanoyle en $C_2$-$C_6$, un groupe naphtyloxy-alcanoyle en $C_2$-$C_6$, un groupe halogéno-alcanoyle en $C_1$-$C_6$, un groupe de formule

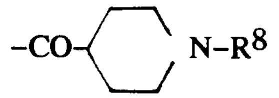

(dans laquelle $R^8$ est un atome d'hydrogène, un groupe alkyle en $C_1$-$C_6$, un groupe phényl-(alcoxy en $C_1$-$C_6$)carbonyle, un groupe carbamoyl-alkyle en $C_1$-$C_6$, un groupe amino-alcanoyle en $C_1$-$C_6$ ayant facultativement un substituant alkyle en $C_1$-$C_6$, ou un groupe alcanoyle en $C_1$-$C_6$), un groupe anilinocarbonyle qui a facultativement sur le noyau phényle un substituant alkyle en $C_1$-$C_6$, un groupe phénoxycarbonyle, un groupe phénylsulfonyle qui a facultativement sur le noyau phényle un substituant choisi parmi un atome d'halogène et un groupe alkyle en $C_1$-$C_6$, un groupe quinolylsulfonyle ou un groupe de formule

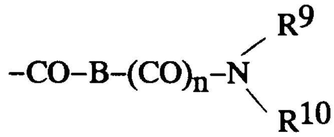

(dans laquelle B est un groupe alkylène en $C_1$-$C_6$, n est un entier égal à 0 ou 1 et $R^9$ et $R^{10}$ sont les mêmes ou différents et sont chacun un atome d'hydrogène, un groupe alkyle en $C_1$-$C_6$ ayant facultativement un substituant hydroxy, un groupe cycloalkyle en $C_3$-$C_8$, un groupe phényl-alkyle en $C_1$-$C_6$, un groupe alcanoyle en $C_1$-$C_6$, un groupe alcényle en $C_2$-$C_6$, un groupe phénoxy-alkyle en $C_1$-$C_6$, un groupe phényle qui a facultativement 1 à 3 substituants choisis parmi un groupe amino-alkyle en $C_1$-$C_6$ ayant facultativement un substituant alcanoyle en $C_1$-$C_6$, un groupe alkyle en $C_1$-$C_6$, un groupe alcoxy en $C_1$-$C_6$ et un atome d'halogène, un groupe phtalimido-alkyle en $C_1$-$C_6$, un groupe amino-alkyle en $C_1$-$C_6$ ayant facultativement un substituant alcanoyle en $C_1$-$C_6$, un groupe alcynyle en $C_2$-$C_6$ ou un groupe amino-alkyle en $C_1$-$C_6$ ayant facultativement un substituant alkyle en $C_1$-$C_6$, ou bien $R^9$ et $R^{10}$ peuvent être reliés ensemble pour former avec l'atome d'azote auquel ils sont liés un groupe hétérocyclique saturé à 5 ou 6 chaînons avec ou sans atome d'azote ou d'oxygène intercalé, dans lequel le groupe hétérocyclique a facultativement un substituant choisi parmi un groupe alkyle en $C_1$-$C_6$, un groupe (alcoxy en $C_1$-$C_6$)carbonyle et un groupe pipéridinyle), et un de ses sels.

**24.** Composé selon la revendication 14, dans lequel $R^4$ est un groupe alkyle en $C_1$-$C_6$, et un de ses sels.

**25.** Composé selon la revendication 16, dans lequel $R^4$ est un atome d'hydrogène et $R^5$ est un groupe de formule

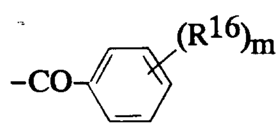

(dans laquelle $R^{16}$ et m sont tels que définis dans la revendication 1, et un de ses sels.

**26.** Composé selon la revendication 16, dans lequel $R^4$ est un atome d'hydrogène et $R^5$ est un groupe phényl-(alcoxy en $C_1$-$C_6$)carbonyle, un groupe alcanoyle en $C_1$-$C_6$, un groupe phényl-alcanoyle en $C_1$-$C_6$, un groupe (cycloalkyl en $C_3$-$C_8$)-alcanoyle en $C_2$-$C_6$, un groupe (cycloalkyl en $C_3$-$C_8$)carbonyle, un groupe tricyclo[3.3.1.1]décanylcarbonyle, un groupe naphtylcarbonyle, un groupe pyridylcarbonyle, un groupe furoyle, un groupe thénoyle, un groupe phénoxy-alcanoyle en $C_2$-$C_6$ dont le noyau phényle a facultativement 1 à 3 substituants choisis parmi un groupe alkyle en $C_1$-$C_6$, un groupe alcoxy en $C_1$-$C_6$ et un groupe amino ayant facultativement un substituant alcanoyle en $C_1$-$C_6$, un groupe phtalimido-alcanoyle en $C_2$-$C_6$, un groupe (alcoxy en $C_1$-$C_6$)carbonyl-alcanoyle en $C_2$-$C_6$, un groupe carboxy-alcanoyle en $C_2$-$C_6$, un groupe naphtyloxy-alcanoyle en $C_2$-$C_6$, un groupe halogéno-alcanoyle en $C_1$-$C_6$, un

groupe de formule

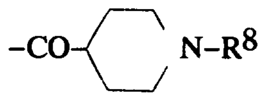

(dans laquelle $R^8$ est un atome d'hydrogène, un groupe alkyle en $C_1$-$C_6$, un groupe phényl-(alcoxy en $C_1$-$C_6$)carbonyle, un groupe carbamoyl-alkyle en $C_1$-$C_6$, un groupe amino-alcanoyle en $C_1$-$C_6$ ayant facultativement un substituant alkyle en $C_1$-$C_6$ ou un groupe alcanoyle en $C_1$-$C_6$), un groupe anilinocarbonyle qui a facultativement sur le noyau phényle un substituant alkyle en $C_1$-$C_6$, un groupe phénoxycarbonyle, un groupe phénylsulfonyle qui a facultativement sur le noyau phényle un substituant choisi parmi un atome d'halogène et un groupe alkyle en $C_1$-$C_6$, un groupe quinolysulfonyle ou un groupe de formule

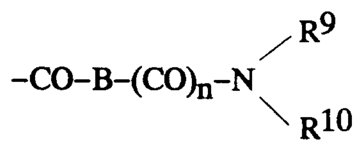

(dans laquelle B est un groupe alkylène en $C_2$-$C_6$, n est un entier de 0 ou 1 et $R^9$ et $R^{10}$ sont les mêmes ou différents et sont chacun un atome d'hydrogène, un groupe alkyle en $C_1$-$C_6$ ayant facultativement un substituant hydroxy, un groupe cycloalkyle en $C_3$-$C_8$, un groupe phényl-alkyle en $C_1$-$C_6$, un groupe alcanoyle en $C_1$-$C_6$, un groupe alcényle en $C_2$-$C_6$, un groupe phénoxy-alkyle en $C_1$-$C_6$, un groupe phényle qui a facultativement 1 à 3 substituants choisis parmi un groupe amino-alkyle en $C_1$-$C_6$ ayant facultativement un substituant alcanoyle en $C_1$-$C_6$, un groupe alkyle en $C_1$-$C_6$, un groupe alcoxy en $C_1$-$C_6$ et un atome d'halogène, un groupe phtalimido-alkyle en $C_1$-$C_6$, un groupe amino-alkyle en $C_1$-$C_6$ ayant facultativement un substituant alcanoyle en $C_1$-$C_6$, un groupe alcynyle en $C_2$-$C_6$ ou un groupe amino-alkyle en $C_1$-$C_6$ ayant facultativement un substituant alkyle en $C_1$-$C_6$, ou bien $R^9$ et $R^{10}$ peuvent être reliés ensemble pour former avec l'atome d'azote auquel ils sont liés un groupe hétérocyclique saturé à 5 ou 6 chaînons avec ou sans atome d'azote ou d'oxygène intercalé, dans lequel le groupe hétérocyclique a facultativement un substituant choisi parmi un groupe alkyle en $C_1$-$C_6$, un groupe (alcoxy en $C_1$-$C_6$)carbonyle et un groupe pipéridinyle), et un de ses sels.

**27.** Composé selon la revendication 16, dans lequel $R^4$ est un groupe alkyle en $C_1$-$C_6$, et un de ses sels.

**28.** Composé selon la revendication 7, dans lequel $R^4$ est un atome d'hydrogène et $R^5$ est un groupe de formule

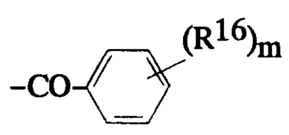

(dans laquelle $R^{16}$ et m sont tels que définis dans la revendication 1, et un de ses sels.

**29.** Composé selon les revendications 7, 10 à 13, 18 et 20 dans lequel $R^4$ est un atome d'hydrogène et $R^5$ est tel que défini à la revendication 23.

**30.** Composé selon la revendication 7, dans lequel $R^4$ est un groupe alkyle en $C_1$-$C_6$, et un de ses sels.

**31.** Composé selon la revendication 18, dans lequel $R^4$ est un atome d'hydrogène et $R^5$ est un groupe de formule

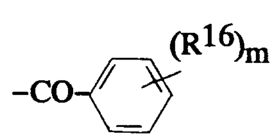

(dans laquelle $R^{16}$ et m sont tels que définis à la revendication 1), et un de ses sels.

**32.** Composé selon la revendication 18, dans lequel $R^4$ est un groupe alkyle en $C_1$-$C_6$, et un de ses sels.

**33.** Composé selon la revendication 20, dans lequel $R^4$ est un atome d'hydrogène et $R^5$ est un groupe de formule

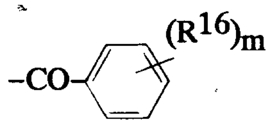

(dans laquelle $R^{16}$ et m sont tels que définis dans la revendication 1), et un de ses sels.

**34.** Composé selon la revendication 20, dans lequel $R^4$ est un groupe alkyle en $C_1$-$C_6$, et un de ses sels.

**35.** Composé selon la revendication 10, dans lequel $R^4$ est un atome d'hydrogène et $R^5$ est un groupe de formule

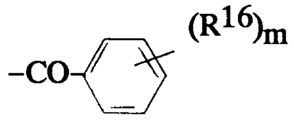

(dans laquelle $R^{16}$ et m sont tels que définis dans la revendication 1), et un de ses sels.

**36.** Composé selon la revendication 10, dans lequel $R^4$ est un groupe alkyle en $C_1$-$C_6$, et un de ses sels.

**37.** Composé selon la revendication 11, dans lequel $R^4$ est un atome d'hydrogène et $R^5$ est un groupe de formule

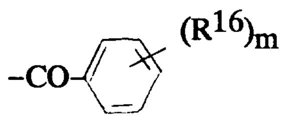

(dans laquelle $R^{16}$ et m sont tels que définis dans la revendication 1), et un de ses sels.

**38.** Composé selon la revendication 11, dans lequel $R^4$ est un groupe alkyle en $C_1$-$C_6$, et un de ses sels.

**39.** Composé selon la revendication 12, dans lequel $R^4$ est un atome d'hydrogène et $R^5$ est un groupe de formule

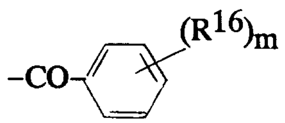

(dans laquelle $R^{16}$ et m sont tels que définis dans la revendication 1), et un de ses sels.

**40.** Composé selon la revendication 12, dans lequel $R^4$ est un groupe alkyle en $C_1$-$C_6$, et un de ses sels.

**41.** Composé selon la revendication 13, dans lequel $R^4$ est un atome d'hydrogène et $R^5$ est un groupe de formule

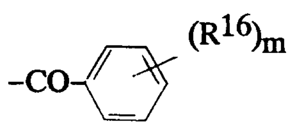

(dans laquelle $R^{16}$ et m sont tels que définis dans la revendication 1), et un de ses sels.

**42.** Composé selon la revendication 13, dans lequel $R^4$ est un groupe alkyle en $C_1$-$C_6$, et un de ses sels.

**43.** Composé selon la revendication 22, dans lequel $R^{16}$ est un atome d'halogène ou un groupe alkyle en $C_1$-$C_6$ ayant facultativement un substituant choisi parmi un atome d'halogène et un groupe hydroxy, et un de ses sels.

**44.** Composé selon la revendication 25, dans lequel $R^{16}$ est un atome d'halogène ou un groupe alkyle en $C_1$-$C_6$ ayant facultativement un substituant choisi parmi un atome d'halogène et un groupe hydroxy, et un de ses sels.

**45.** Composé selon la revendication 31, dans lequel $R^{16}$ est un atome d'halogène ou un groupe alkyle en $C_1$-$C_6$ ayant facultativement un substituant choisi parmi un atome d'halogène et un groupe hydroxy, et un de ses sels.

**46.** Composé selon la revendication 34, dans lequel $R^{16}$ est un atome d'halogène ou un groupe alkyle en $C_1$-$C_6$ ayant facultativement un substituant choisi parmi un atome d'halogène et un groupe hydroxy, et un de ses sels.

**47.** Composé selon la revendication 1, dans lequel W est un groupe de formule $-(CH_2)_p-$ dans laquelle p est un entier de 3 à 5 et en outre ledit groupe $-(CH_2)_p-$ a facultativement 1 à 3 substituants tels que définis dans la revendication 1.

**48.** Composé selon la revendication 1, dans lequel W est un groupe de formule $-CH=CH-(CH_2)_q-$ dans laquelle q est un entier de 1 à 3 et en outre ledit groupe $-CH=CH-(CH_2)_q-$ a facultativement 1 à 3 substituants choisis parmi un groupe alkyle en $C_1$-$C_6$ ayant facultativement un substituant hydroxy, et un groupe amino-alkyle en $C_1$-$C_6$ ayant facultativement un substituant choisi parmi un groupe alkyle en $C_1$-$C_6$ et un groupe alcanoyle en $C_1$-$C_6$, et un de ses sels.

**49.** Composé selon la revendication 47, dans lequel W est un groupe de formule $-(CH_2)_p-$ (p est un entier de 3 à 5) et ledit groupe $-(CH_2)_p-$ a facultativement 1 à 3 substituants tels que définis dans la revendication 1.

**50.** Composé selon la revendication 40, dans lequel p dans le groupe $-(CH_2)_p-$ est égal à 3 et le groupe n'a pas de substituant, et un de ses sels.

**51.** Composé selon la revendication 49, dans lequel p dans le groupe $-(CH_2)_p-$ est égal à 3 et le groupe est substitué par un groupe de formule

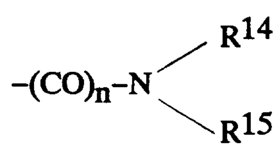

(dans laquelle $R^{14}$, $R^{15}$ et n sont tels que définis ci-dessus), et un de ses sels.

**52.** Composé selon la revendication 49, dans lequel p dans le groupe $-(CH_2)_p$ est égal à 4 et le groupe n'a pas de substituant, et un de ses sels.

**53.** Composé selon la revendication 49, dans lequel p dans le groupe $-(CH_2)_p-$ est égal à 4 et le groupe est substitué par un groupe de formule

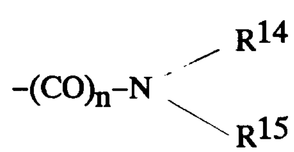

(dans laquelle $R^{14}$, $R^{15}$ et n sont tels que définis ci-dessus), et un de ses sels.

**54.** Composé selon la revendication 49, dans lequel p dans le groupe $-(CH_2)_p-$ est égal à 5, et un de ses sels.

**55.** Composé selon la revendication 48, dans lequel q dans le groupe $-CH=CH-(CH_2)_q-$ est égal à 1, et un de ses sels.

**56.** Composé selon la revendication 48, dans lequel q dans le groupe $-CH=CH-(CH_2)_q-$ est égal à 2, et un de ses sels.

**57.** Composé selon la revendication 48, dans lequel q dans le groupe $-CH=CH-(CH_2)_q-$ est égal à 3, et un de ses sels.

**58.** Composé selon la revendication 51 ou 53, dans lequel n dans le substituant

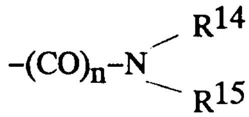

est égal à 0 et $R^{14}$ et $R^{15}$ sont les mêmes ou différents et sont chacun un atome d'hydrogène, un groupe alkyle en $C_1$-$C_6$ ou un groupe cycloalkyle en $C_3$-$C_8$, et un de ses sels.

**59.** Composé selon la revendication 56, dans lequel le groupe hétéro cyclique de formule

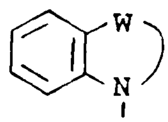

est un groupe 2,3-dihydro-1H-benzazépine, et un de ses sels.

**60.** La 1-[4-(2-méthylbenzoylamino)benzoyl]-4-méthyl-2,3,4,5-tétrahydro-1H-1,4-benzodiazépine.

**61.** La 5-diméthylamino-1-[4-(2-méthylbenzoylamino)benzoyl]-2,3,4,5-tétrahydro-1H-benzazépine.

**62.** La 5-diméthylamino-1-[2-chloro-4-(2-méthylbenzoylamino)benzoyl]-2,3,4,5-tétrahydro-1H-benzazépine.

**63.** La 5-méthylamino-1-[2-chloro-4-(2-méthylbenzoylamino)benzoyl]-2,3,4,5-tétrahydro-1H-benzazépine.

**64.** La 5-cyclopropylamino-1-[2-chloro-4-(2-méthylbenzoylamino)benzoyl]-2,3,4,5-tétrahydro-1H-benzazépine.

**65.** La 5-cyclopropylamino-1-[2-chloro-4-(2-chlorobenzoylamino)benzoyl]-2,3,4,5-tétrahydro-1H-benzazépine.

**66.** La 5-diméthylamino-1-[2-méthyl-4-(2-méthylbenzoylamino)benzoyl]-2,3,4,5-tétrahydro-1H-benzazépine.

**67.** La 4-diméthylamino-1-[3-méthoxy-4-(2-méthylbenzoylamino)benzoyl]-1,2,3,4-tétrahydroquinoléine.

**68.** La 7-chloro-5-méthylamino-1-[4-(2-méthylbenzoylamino)benzoyl]-2,3,4,5-tétrahydro-1H-benzazépine.

**69.** La 7-chloro-5-méthylamino-1-[4-(2-chlorobenzoylamino)benzoyl]-2,3,4,5-tétrahydro-1H-benzazépine.

**70.** Composition antagoniste de la vasopressine, qui comprend comme ingrédient actif un composé de formule (1) selon la revendication 1, ou un de ses sels acceptables en pharmacie, en mélange avec un support ou diluant acceptable en pharmacie.

**71.** Procédé pour préparer un composé de formule (1) selon la revendication 1, qui comprend la réaction d'un composé de formule (2) :

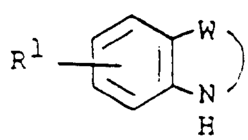 (2)

dans laquelle

$R^1$ et W sont tels que définis dans la revendication 1 avec un composé de formule (3) :

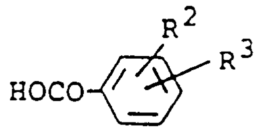

(3)

dans laquelle
$R^2$ et $R^3$ sont tels que définis dans la revendication 1.

**72.** Procédé pour préparer un composé de formule

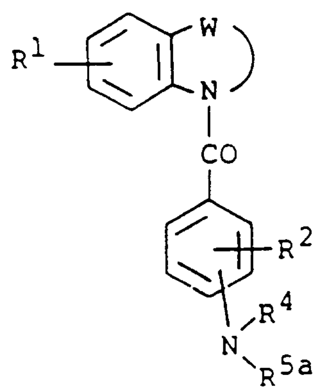

(1b)

dans laquelle
$R^1$, $R^2$, $R^4$ et W sont tels que définis dans la revendication 1, dans laquelle $R^{5a}$ est le même que $R^5$ tel que défini dans la revendication 1, sauf qu'un groupe anilinocarbonyle ayant facultativement sur le noyau phényle un groupe alkyle en $C_1$-$C_6$, un groupe phénylsulfonyle ayant facultativement sur le noyau phényle un substituant choisi parmi un atome d'halogène et un groupe alkyle en $C_1$-$C_6$ et un groupe quinolylsulfonyle sont exclus, qui comprend la réaction d'un composé de formule (2b) :

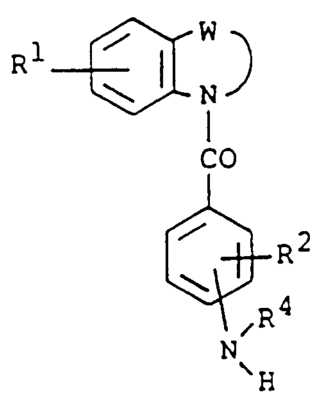

(2b)

dans laquelle
$R^1$, $R^2$, $R^4$ et W sont tels que définis ci-dessus, avec un composé de formule (4) :

$$R^{5a}OH \quad (4)$$

**73.** Procédé pour préparer un composé de formule (1c) :

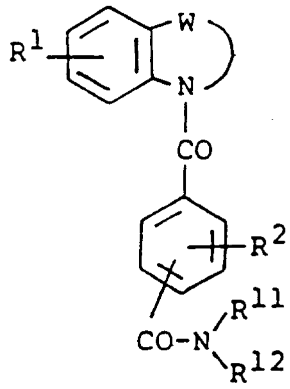

(1c)

dans laquelle

$R^1$, $R^2$, $R^{11}$, $R^{12}$ et W sont tels que définis dans la revendication 1, qui comprend la réaction d'un composé de formule (5) :

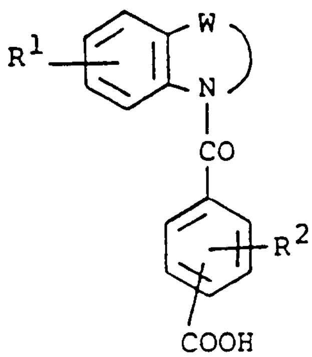

(5)

dans laquelle

$R^1$, $R^2$ et W sont tels que définis ci-dessus, avec un composé de formule (6) :

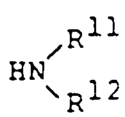

(6)

dans laquelle

$R^{11}$ et $R^{12}$ sont tels que définis ci-dessus.

**74.** Procédé pour préparer un composé de formule (1d) :

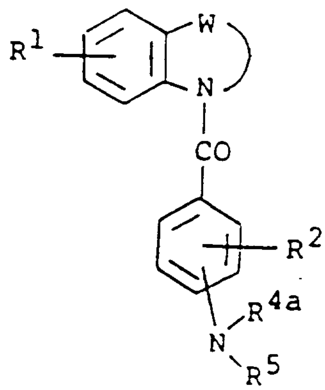

(1d)

dans laquelle

$R^1$, $R^2$, $R^5$ et W sont tels que définis dans la revendication 1, et $R^{4a}$ est un groupe alkyle en $C_1$-$C_6$, qui comprend la réaction d'un composé de formule (7) :

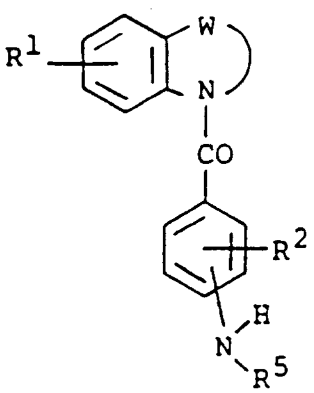

(7)

dans laquelle

$R^1$, $R^2$, $R^5$ et W sont tels que définis ci-dessus, avec un composé de formule (8) ou (9) :

$$R^{4a}X \quad (8)$$

ou

$$R^{17}COR^{18} \quad (9)$$

dans lesquelles

dans lesquelles

$R^{4a}$ est un groupe alkyle en $C_1$-$C_6$, X est un atome d'halogène et $R^{17}$ et $R^{18}$ sont chacun un atome d'hydrogène ou un groupe alkyle en $C_1$-$C_6$.

**75.** Procédé pour préparer un composé de formule (1e) :

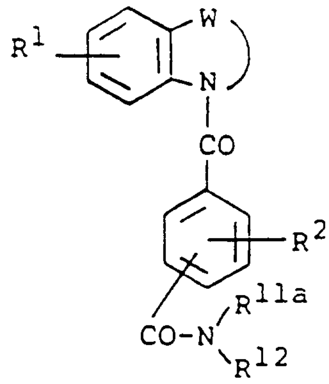

(1e)

dans laquelle

$R^1$, $R^2$, $R^{12}$ et W sont tels que définis dans la revendication 1, et $R^{11a}$ est un groupe alkyle en $C_1$-$C_6$, qui comprend la réaction d'un composé de formule (10) :

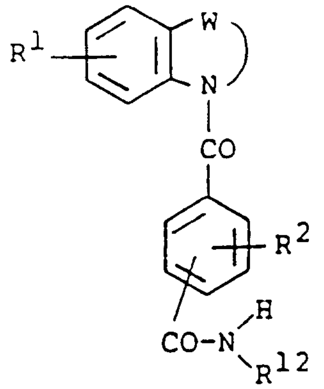

(10)

dans laquelle

$R^1$, $R^2$, $R^{12}$ et W sont tels que définis ci-dessus, avec un composé de formule (11) ou (9) :

$$R^{11a}X \qquad (11)$$

ou

$$R^{17}COR^{18} \qquad (9)$$

dans lesquelles

$R^{11a}$ est un groupe alkyle en $C_1$-$C_6$ et X, $R^{17}$ et $R^{18}$ sont tels que définis ci-dessus.

**76.** Procédé pour préparer un composé de formule (1f) :

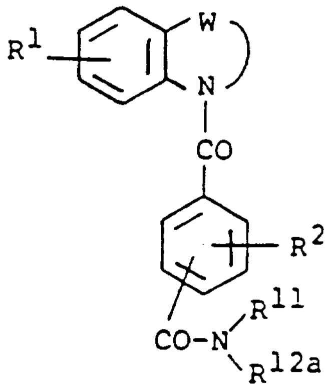

(1f)

dans laquelle

$R^1$, $R^2$, $R^{11}$ et W sont tels que définis dans la revendication 1, et $R^{12a}$ est un groupe cycloalkyle en $C_3$-$C_8$, qui comprend la réaction d'un composé de formule (12) :

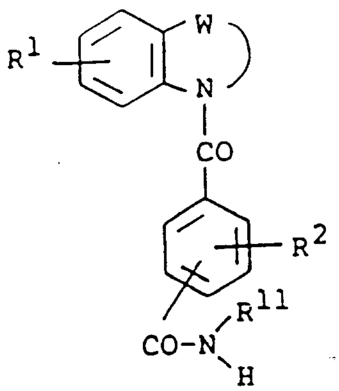

(12)

dans laquelle

$R^1$, $R^2$, $R^{11}$ et W sont tels que définis ci-dessus, avec un composé de formule (13) :

$$R^{12a}X \qquad (13)$$

dans laquelle

$R^{12a}$ est un groupe cycloalkyle en $C_3$-$C_8$ et X est un halogène.

**77.** Procédé pour préparer un composé de formule (1cc)

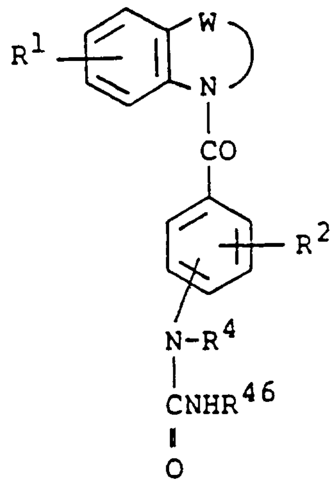

(1cc)

dans laquelle

$R^1$, $R^2$, $R^4$ et W sont tels que définis dans la revendication 1, et $R^{46}$ est un groupe phényle ayant facultativement un substituant alkyle en $C_1$-$C_6$, qui comprend la réaction d'un composé de formule (2b) :

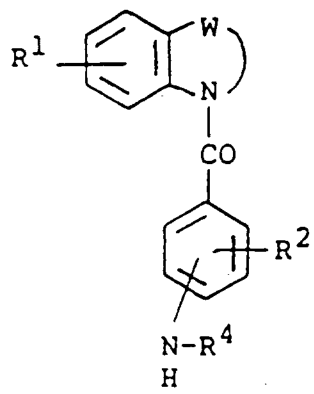

(2b)

dans laquelle

$R^1$, $R^2$, $R^4$ et W sont tels que définis ci-dessus, avec un composé de formule (38) :

$$R^{46}N{=}C{=}O \qquad (38)$$

dans laquelle

$R^{46}$ est tel que défini ci-dessus.

**78.** Procédé pour préparer un composé de formule (1dd) :

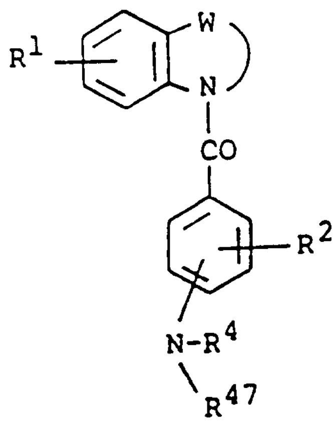

(1dd)

dans laquelle

$R^1$, $R^2$, $R^4$ et W sont tels que définis dans la revendication 1, et $R^{47}$ est un groupe phénylsulfonyle ayant facultativement sur le noyau phényle un substituant choisi parmi un atome d'halogène et un groupe alkyle en $C_1$-$C_6$ ou un groupe quinolylsulfonyle et X est un halogène, qui comprend la réaction d'un composé de formule (2b) :

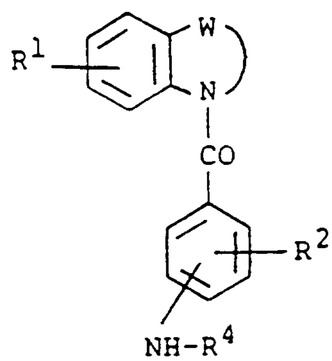

(2b)

dans laquelle

$R^1$, $R^2$, $R^4$ et W sont tels que définis ci-dessus, avec un composé de formule (39) :

$$R^{47}X \qquad (39)$$

dans laquelle

$R^{47}$ est tel que défini ci-dessus.

**79.** Procédé pour préparer un composé de formule (1 hh)

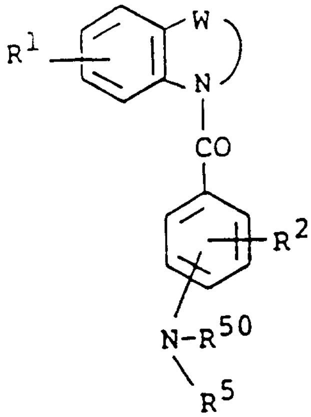

(1hh)

dans laquelle
$R^1$, $R^2$, $R^5$ et W sont tels que définis dans la revendication 1, et $R^{50}$ est un groupe benzoyle ayant facultativement sur le noyau phényle un substituant halogène, qui comprend la réaction d'un composé de formule (7)

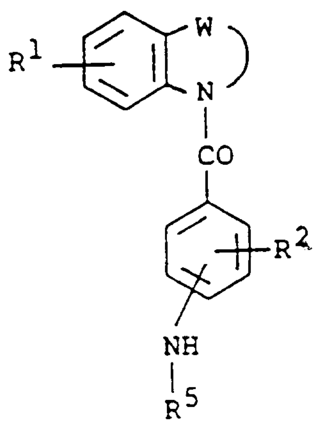

(7)

dans laquelle
$R^1$, $R^2$, $R^5$ et W sont tels que définis ci-dessus avec un composé de formule (42)

$R^{50}OH$ (42)

dans laquelle
$R^{50}$ est tel que défini ci-dessus.

**80.** La 5-hydroxysulfonyloxyimino-1-[2-chloro-4-(2-méthylbenzoylamino)benzoyl]-2,3,4,5-tétrahydro-1H-benzazépine.

**81.** La 7-fluoro-5-hydroxy-1-[2-chloro-4-(2-méthylbenzoylamino)benzoyl]-2,3,4,5-tétrahydro-1H-benzazépine.

**82.** La 7-[4-(2-chlorobenzoylamino)benzoyl]-1-méthyl-1,2,3,4a,5,6,7,11b-octahydro-3-oxo[1]benzazépino[4,5-b][1,4]oxazine.

**83.** Composés de formule :

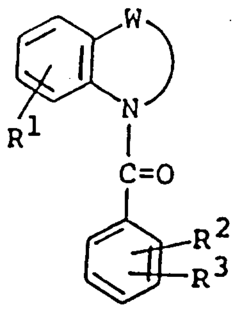

dans laquelle

a)

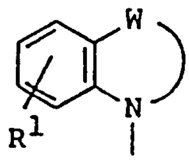

représente

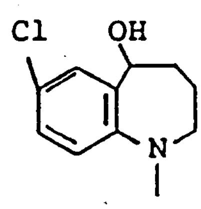

$R^2$ représente 2-$OCH_3$ et
$R^3$ représente

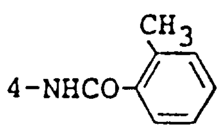 ;

et

b)

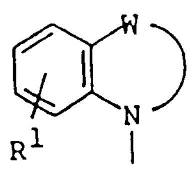

représente

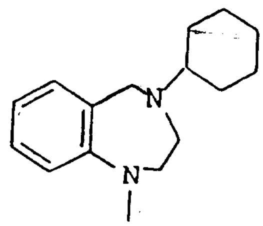

$R^2$ représente H et
$R^3$ représente

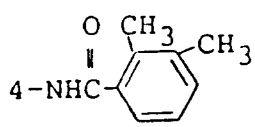

**84.** Composé benzohétérocyclique de formule :

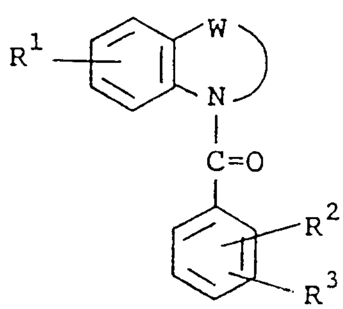

dans laquelle
$R^1$, $R^2$ et $R^3$ sont tels que définis dans la revendication 1 et le groupe hétérocyclique de formule :

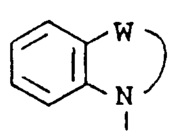

est choisi parmi les groupes 3,4-dihydro-2H-1,4-benzoxazinyle, 1,2,3,5-tétrahydro-4,1-benzoxazépinyle, 1,2,3,4-tétrahydroquinoxalinyle, 1,2,3,4,5,6-hexahydro-1,5-benzodiazocinyle, 5-méthyl-1,2,3,4,5,6-hexahydro-1,5-benzodiazocinyle, 4-méthyl-1,2,3,4-tétahydroquinoxalinyle, 1,2,3,4-tétrahydro-5,1-benzoxazépinyle, 3,4-dihydro-2H-1,4-benzothiazinyle, 2,3,4,5-tétrahydro-1,5-benzothiazépinyle, 1,2,3,5-tétrahydro-4,1-benzothiazépinyle, 4-éthyl-1,2,3,4-tétrahydroquinoxalinyle, 4-propyl-1,2,3,4-tétrahydroquinoxalinyle, 4-butyl-1,2,3,4-tétrahydroquinoxalinyle,

4-pentyl-1,2,3,4-tétrahydroquinoxalinyle, 4-hexyl-1,2,3,4-tétrahydroquinoxalinyle, 2,3,4,5-tétrahydro-1H-1,4-benzodiazépinyle, 4-méthyl-2,3,4,5-tétrahydro-1H-1,4-benzodiazépinyle, 4-éthyl-2,3,4,5-tétrahydro-1H-1,4-benzodiazépinyle, 4-propyl-2,3,4,5-tetrahydro-1H-1,4-benzodiazépinyle, 4-butyl-2,3,4,5-tétrahydro-1H-1,4-benzodiazépinyle, 4-pentyl-2,3,4,5-tétrahydro-1H-1,4-benzodiazépinyle, 4-hexyl-2,3,4,5-tétrahydro-1H-1,4-benzodiazépinyle, 2,3,4,5-tétrahydro-1H-1,5-benzodiazépinyle, 5-méthyl-2,3,4,5-tétrahydro-1H-1,5-benzodiazépinyle, 5-éthyl-2,3,4,5-tétrahydro-1H-1,5-benzodiazépinyle, 5-propyl-2,3,4,5-tétrahydro-1H-1,5-benzodiazépinyle, 5-butyl-2,3,4,5-tétrahydro-1H-1,5-benzodiazépinyle, 5-pentyl-2,3,4,5-tétrahydro-1H-1,5-benzodiazépinyle, 5-hexyl-2,3,4,5-tétrahydro-1H-1,5-benzodiazépinyle, 3,4-dihydro-1-oxo-2H-1,4-benzothiazépinyle, 3,4-dihydro-1,1-dioxo-2H-1,4-benzothiazépinyle, 1-oxo-2,3,4,5-tétrahydro-1,5-benzothiazépinyle, 1,1-dioxo-2,3,4,5-tétrahydro-1,5-benzothiazépinyle, 4-oxo-1,2,3,4,5-tétrahydro-4,1-benzothiazépinyle et 4,4-dioxo-1,2,3,5-tétrahydro-4,1-benzothiazépinyle, et un de ses sels.

**Revendications pour l'Etat contractant suivant : ES**

1. Procédé dur préparer un composé benzohétérocylique, de formule suivante :

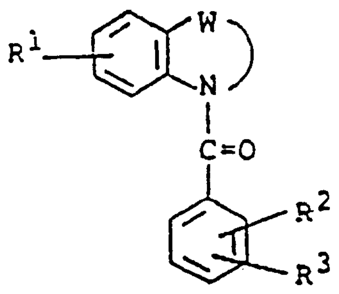

(1)

dans laquelle

$R^1$ est un atome d'hydrogène, un atome d'halogène, un groupe alkyle en $C_1$-$C_6$, un groupe amino ayant facultativement un substituant alkyle en $C_1$-$C_6$ ou un groupe alcoxy en $C_1$-$C_6$,

$R^2$ est un atome d'hydrogène, un atome d'halogène, un groupe alcoxy en $C_1$-$C_6$, un groupe phényl-alcoxy en $C_1$-$C_6$, un groupe hydroxy, un groupe alkyle en $C_1$-$C_6$, un groupe amino ayant faculativement un substituant alkyle en $C_1$-$C_6$, un groupe carbamoyl-alcoxy en $C_1$-$C_6$, un groupe amino-alcoxy en $C_1$-$C_6$ ayant facultativement un substituant alkyle en $C_1$-$C_6$ ou un groupe benzoyloxy qui a facultativement sur le noyau phényle un substituant halogène,

$R^3$ est un groupe de formule

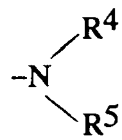

ou un groupe de formule

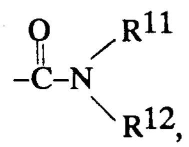

$R^4$ est un atome d'hydrogène, un groupe benzoyle qui a facultativement sur le noyau phényle un substituant halogène ou un groupe alkyle en $C_1$-$C_6$,

$R^5$ est (a) un groupe de formule

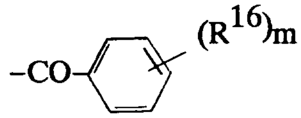

[où $R^{16}$ est (i) un atome d'halogène ; (ii) un groupe alkyle en $C_1$-$C_6$ qui a facultativement un substituant choisi parmi un atome d'halogène et un groupe hydroxy ; (iii) un groupe hydroxy ; (iv) un groupe alcoxy en $C_1$-$C_6$ ; (v) un groupe alcanoyloxy en $C_1$-$C_6$ ; (vi) un groupe alkylthio en $C_1$-$C_6$ ; (vii) un groupe alcanoyle en $C_1$-$C_6$ ; (viii) un groupe carboxy ; (ix) un groupe (alcoxy en $C_1$-$C_6$)carbonyle ; (x) un groupe cyano ; (xi) un groupe nitro ; (xii) un groupe amino qui a facultativement un substituant choisi parmi un groupe alkyle en $C_1$-$C_6$ et un groupe alcanoyle en $C_1$-$C_6$ ; (xiii) un groupe phényle; (xiv) un groupe cycloalkyle en $C_3$-$C_8$ ; (xv) un groupe (alcanoyloxy en $C_2$-$C_6$)-alcoxy en $C_1$-$C_6$ ; (xvi) un groupe carboxy-alcoxy en $C_1$-$C_6$ ; (xvii) un groupe halogéno-alcoxy en $C_1$-$C_6$ ; (xviii) un groupe carbamoyl-alcoxy en $C_1$-$C_6$ ; (xix) un groupe hydroxy-alcoxy en $C_1$-$C_6$ ; (xx) un groupe (alcoxy en $C_1$-$C_6$) carbonyl-alcoxy en $C_1$-$C_6$ ; (xxi) un groupe phtalimido-alcoxy en $C_1$-$C_6$ ; (xxii) un groupe aminocarbonyl-alcoxy en $C_1$-$C_6$ ayant un substituant alkyle en $C_1$-$C_6$ ; ou (xxiii) un groupe de formule

$$-O-A-N\begin{matrix}R^6\\R^7\end{matrix}$$

(A est un groupe alkylène jusqu'en $C_6$ et

$R^6$ et $R^7$ sont les mêmes ou différents et sont chacun un atome d'hydrogène, un groupe alkyle en $C_1$-$C_6$ ayant facultativement un substituant hydroxy, un groupe alcanoyle en $C_1$-$C_6$ ou un groupe benzoyle, ou bien

$R^6$ et $R^7$ peuvent être reliés ensemble pour former avec l'atome d'azote auquel ils sont fixés un groupe hétérocyclique saturé à 5 ou 6 chaînons avec ou sans atome d'azote ou d'oxygène intercalé, dans lequel le groupe hétérocyclique a facultativement un substituant choisi parmi un groupe pipéridinyle et un groupe alkyle en $C_1$-$C_6$) ; et m est un entier de 0 à 3],

(b) un groupe phényl-(alcoxy en $C_1$-$C_6$)carbonyle,
(c) un groupe alcanoyle en $C_1$-$C_6$,
(d) un groupe phényl-alcanoyle en $C_2$-$C_6$,
(e) un groupe (cycloalkyl en $C_3$-$C_8$)-alcanoyle en $C_2$-$C_6$,
(f) un groupe (cycloalkyl en $C_3$-$C_8$)carbonyle,
(g) un groupe tricyclo[3.3.1.1]décanylcarbonyle,
(h) un group naphtylcarbonyle,
(i) un groupe pyridylcarbonyle,
(j) un groupe furoyle,
(k) un groupe thénoyle,
(l) un groupe phénoxy-alcanoyle en $C_2$-$C_6$ dont le noyau phényle a facultativement 1 à 3 substituants choisis parmi un groupe alkyle en $C_1$-$C_6$, un groupe alcoxy en $C_1$-$C_6$ et un groupe amino ayant facultativement un substituant alcanoyle en $C_1$-$C_6$,
(m) un groupe phtalimido-alcanoyle en $C_2$-$C_6$,
(o) un groupe (alcoxy en $C_1$-$C_6$)carbonyl-alcanoyle en $C_2$-$C_6$,
(p) un groupe carboxy-alcanoyle en $C_2$-$C_6$,
(q) un groupe naphtyloxy-alcanoyle en $C_2$-$C_6$,
(r) un groupe halogénoalcanoyle en $C_1$-$C_6$,
(s) un groupe de formule

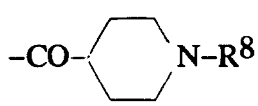

(dans laquelle $R^8$ est (i) un atome d'hydrogène, (ii) un groupe alkyle en $C_1$-$C_6$, (iii) un groupe phényl-(alcoxy en

$C_1$-$C_6$)carbonyle, (iv) un groupe carbamoyl-alkyle en $C_1$-$C_6$, (v) un groupe amino-alcanoyle en $C_2$-$C_6$ ayant facultativement un substituant alkyle en $C_1$-$C_6$ ou (vi) un groupe alcanoyle en $C_1$-$C_6$),

(t) un groupe anilinocarbonyle qui a facultativement sur le noyau phényle un substituant alkyle en $C_1$-$C_6$,

(u) un groupe phénoxycarbonyle,

(v) un groupe phénylsulfonyle qui a facultativement sur le noyau phényle un substituant choisi parmi un atome d'halogène et un groupe alkyle en $C_1$-$C_6$,

(w) un groupe quinolysulfonyle ou

(x) un groupe de formule

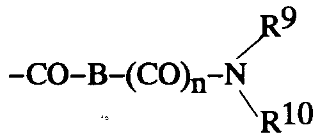

dans laquelle

B est un groupe alkylène en $C_1$-$C_6$, n est un entier égal à 0 ou 1 et $R^9$ et $R^{10}$ sont les mêmes ou différents et sont chacun (i) un atome d'hydrogène, (ii) un groupe alkyle jusqu'en $C_6$ ayant facultativement un substituant hydroxy, (iii) un groupe cycloalkyle en $C_3$-$C_8$, (iv) un groupe phényl-alkyle en $C_1$-$C_6$, (v) un groupe alcanoyle en $C_1$-$C_6$, (vi) un groupe alcényle jusqu'en $C_6$, (vii) un groupe phénoxy-alkyle en $C_1$-$C_6$, (viii) un groupe phényle qui a facultativement 1 à 3 substituants choisis parmi un groupe amino-alkyle en $C_1$-$C_6$ ayant facultativement un substituant alcanoyle en $C_1$-$C_6$, un groupe alkyle en $C_1$-$C_6$, un groupe alcoxy en $C_1$-$C_6$ et un atome d'halogène, (ix) un groupe phtalimido-alkyle en $C_1$-$C_6$, (x) un groupe amino-alkyle en $C_1$-$C_6$ ayant facultativement un substituant alcanoyle en $C_1$-$C_6$, (xi) un groupe alcynyle jusqu'en $C_6$ ou (xii) un groupe amino-alkyle en $C_1$-$C_6$ ayant facultativement un substituant aklyle en $C_1$-$C_6$, ou bien $R^9$ et $R^{10}$ peuvent être reliés ensemble pour former avec l'atome d'azote auquel ils sont liés un groupe hétérocyclique saturé à 5 ou 6 chaînons avec ou sans atome d'azote ou d'oxygène intercalé, où le groupe hétérocyclique a facultativement un substituant choisi parmi un groupe alkyle en $C_1$-$C_6$, un groupe (alcoxy en $C_1$-$C_6$)carbonyle et un groupe pipéridinyle,

$R^{11}$ est un atome d'hydrogène ou un groupe alkyle en $C_1$-$C_6$,

$R^{12}$ est un groupe cycloalkyle en $C_3$-$C_8$ ou un groupe phényle qui a facultativement 1 à 3 substituants choisis parmi un groupe alcoxy en $C_1$-$C_6$, un groupe alkyle en $C_1$-$C_6$ et un atome d'halogène,

W est un groupe de formule $-(CH_2)p-$ (p est un entier de 3 à 5) ou un groupe de formule $-CH=CH-(CH_2)_q-$ (q est un entier de 1 à 3), ou dans lequel le groupe hétérocyclique de formule :

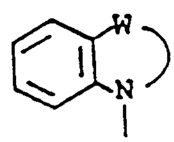

est un groupe 2,3,4,5-tétrahydro-1H-1,4-benzodiazépine,

et en outre ledit groupe $-(CH_2)_p-$ ayant facultativement 1 à 3 substituants choisis parmi (i) un groupe alkyle en $C_1$-$C_6$ ayant facultativement un substituant hydroxy, (ii) un groupe (alcoxy en $C_1$-$C_6$)carbonyle, (iii) un groupe carboxy, (iv) un groupe hydroxy, (v) un reste oxo, (vi) un groupe alcanoyloxy en $C_1$-$C_6$ ayant facultativement un substituant halogène, (vii) un groupe amino-alkyle en $C_1$-$C_6$ ayant facultativement un substituant choisi parmi un groupe alkyle en $C_1$-$C_6$ et un groupe alcanoyle en $C_1$-$C_6$, (viii) un groupe (alcanoyloxy en $C_2$-$C_6$)-alkyle en $C_1$-$C_6$, (ix) un groupe (alkyl en $C_1$-$C_6$)-sulfonyloxy-alkyle en $C_1$-$C_6$, (x) un groupe azido-alkyle en $C_1$-$C_6$, (xi) un groupe de formule $-O-CH_2-$ (qui est relié au groupe $-(CH_2)_p-$ ou $-CH=CH-(CH_2)_q-$ de manière à être sous la forme spiro), (xii) un groupe aminocarbonyloxy ayant facultativement un substituant alkyle en $C_1$-$C_6$, (xiii) un groupe alcoxy en $C_1$-$C_6$, (xiv) un groupe (alcoxy en $C_1$-$C_6$)carbonyl-alcoxy en $C_1$-$C_6$, (xv) un groupe carboxy-alcoxy en $C_1$-$C_6$, (xvi) un groupe aminocarbonyl-alcoxy en $C_1$-$C_6$ ayant facultativement un substituant alkyle en $C_1$-$C_6$, (xvii) un groupe amino-alcoxy en $C_1$-$C_6$ ayant facultativement un substituant choisi parmi un groupe alkyle en $C_1$-$C_6$ et un groupe alcanoyle en $C_1$-$C_6$, (xviii) un groupe phtalimido-alcoxy en $C_1$-$C_6$, (xix) un groupe hydroxyimino, (xx) un groupe (alcanoyloxy en $C_1$-$C_6$)imino, (xxi) un groupe alkylidène en $C_1$-$C_6$, (xxii) un atome d'halogène, (xxiii) un groupe azido, (xxvi) un groupe hydrazino, (xxvii) un groupe pyrrolyle, (xxviii) un groupe amino-alcanoyloxy en $C_1$-$C_6$ ayant facultativement

un substituant alkyle en $C_1$-$C_6$, (xxix) un groupe de formule

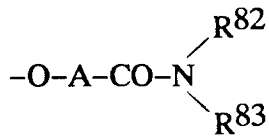

(A est défini comme comme ci-dessus et

$R^{82}$ et $R^{83}$ sont les mêmes ou différents et sont chacun un atome d'hydrogène, un groupe alkyle en $C_1$-$C_6$, un groupe carbamoyl-alkyle en $C_1$-$C_6$, un groupe hydroxy-alkyle en $C_1$-$C_6$ ou un groupe pyridyl-alkyle en $C_1$-$C_6$,

ou bien $R^{82}$ et $R^{83}$ peuvent être reliés ensemble pour former avec l'atome d'azote auquel ils sont liés un groupe hétérocyclique saturé à 5 ou 6 chaînons avec ou sans atome d'azote d'oxygène ou de soufre intercalé, dans lequel le groupe hétérocyclique a facultativement un substituant choisi parmi un reste oxo, un groupe alkyle en $C_1$-$C_6$, un groupe alcanoyle en $C_1$-$C_6$ et un groupe carbamoyle), et

(xxx) un groupe de fomule

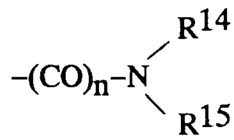

(dans laquelle n est défini comme ci-dessus et $R^{14}$ et $R^{15}$ sont les mêmes ou différents et sont chacun (i) un atome d'hydrogène, (ii) un groupe alkyle en $C_1$-$C_6$, (iii) un groupe alcényle jusqu'en $C_6$, (iv) un groupe alcanoyle en $C_1$-$C_6$, (v) un groupe cycloalkyle en $C_3$-$C_8$, (vi) un groupe oxiranyl-alkyle en $C_1$-$C_6$, (vii) un groupe alkyle en $C_1$-$C_6$ ayant 1 à 2 substituants choisis parmi un groupe alcoxy en $C_1$-$C_6$, un groupe hydroxy et un groupe amino ayant facultativement un substituant alkyle en $C_1$-$C_6$, (viii) un groupe phényl-alkyle en $C_1$-$C_6$, (ix) un groupe pyridyl-alkyle en $C_1$-$C_6$, (x) un groupe alkylsulfonyle en $C_1$-$C_6$, (xi) un groupe benzoyle, (xii) un groupe (alcoxy en $C_1$-$C_6$)carbonyle, (xiii) un groupe anilinocarbonyle, (xiv) un groupe aminocarbonyle ayant facultativement un substituant alkyle en $C_1$-$C_6$, (xv) un groupe cyano-alkyle en $C_1$-$C_6$, (xvi) un groupe (alcoxy en $C_1$-$C_6$)carbonyl-alkyle en $C_1$-$C_6$, (xvii) un groupe carbamoyl-alkyle en $C_1$-$C_6$, (xviii) un groupe carboxy-alkyle en $C_1$-$C_6$, (xix) un groupe tétrahydropyranyloxy-alkyle en $C_1$-$C_6$, (xx) un groupe (alcanoyloxy en $C_2$-$C_6$)-alkyle en $C_1$-$C_6$, (xxi) un groupe pipéridinyle ayant facultativement sur le noyau pipéridinyle un substituant phényl-alkyle en $C_1$-$C_6$, (xxii) un groupe halogéno-alcanoyle en $C_2$-$C_6$, (xxiii) un groupe imidazolyl-alcanoyle en $C_2$-$C_6$, (xxiv) un groupe amino-alcanoyle en $C_2$-$C_6$ ayant facultativement un substituant choisi parmi un groupe alkyle en $C_1$-$C_6$ et un groupe (alcoxy en $C_1$-$C_6$)-carbonyle, (xxv) un groupe amino-carbonyl-alkyle en $C_1$-$C_6$ ayant facultativement un substituant alkyle en $C_1$-$C_6$ ou (xxvi) un groupe phényl-(alcoxy en $C_1$-$C_6$)carbonyle, ou bien

$R^{14}$ et $R^{15}$ peuvent être reliés ensemble pour former avec l'atome d'azote auquel ils sont liés un groupe hétérocyclique saturé à 5 ou 6 chaînons avec ou sans atome d'azote ou d'oxygène intercalé, dans lequel le groupe hétérocyclique peut facultativement avoir un substituant choisi parmi un groupe alkyle en $C_1$-$C_6$, un groupe phényl-alkyle en $C_1$-$C_6$ ou un groupe alcanoyle en $C_1$-$C_6$) et en outre le:lit groupe $-CH=CH-(CH_2)_q-$ ayant facultativement 1 à 3 substituants choisis parmi

(i) un groupe alkyle en $C_1$-$C_6$ ayant facultativement un substituant hydroxy et
(ii) un groupe amino-alkyle en $C_1$-$C_6$ ayant facultativement un substituant choisi parmi un groupe alkyle en $C_1$-$C_6$ et un groupe alcanoyle en $C_1$-$C_6$

et d'un de ses sels,
qui comprend la réaction d'un composé de formule (2)

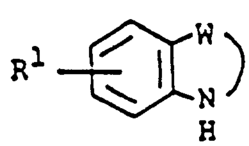

(2)

dans laquelle

$R^1$ et W sont tels que définis ci-dessus avec un composé de formule (3) :

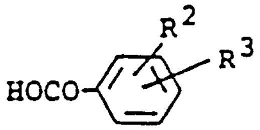

(3)

dans laquelle

$R^2$ et $R^3$ sont tels que définis ci-dessus.

**2.** Procédé pour préparer un composé de formule

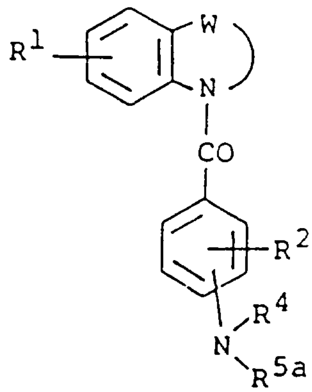

(1b)

dans laquelle

$R^1$, $R^2$, $R^4$ et W sont tels que définis dans la revendication 1, dans laquelle $R^{5a}$ est le même que $R^5$ tel que défini dans la revendication 1, sauf qu'un groupe anilinocarbonyle ayant facultativement sur le noyau phényle un groupe alkyle en $C_1$-$C_6$, un groupe phénylsulfonyle ayant facultativement sur le noyau phényle un substituant choisi parmi un atome d'halogène et un groupe alkyle en $C_1$-$C_6$ et un groupe quinolylsulfonyle sont exclus, qui comprend la réaction d'un composé de formule (2b) :

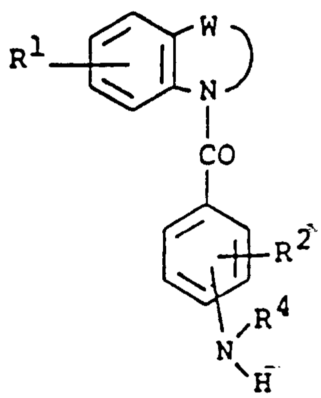

(2b)

dans laquelle

$R^1$, $R^2$, $R^4$ et W sont tels que définis ci-dessus, avec un composé de formule (4) :

$$R^{5a}OH \qquad (4)$$

3. Procédé peur préparer un composé de formule (1c) :

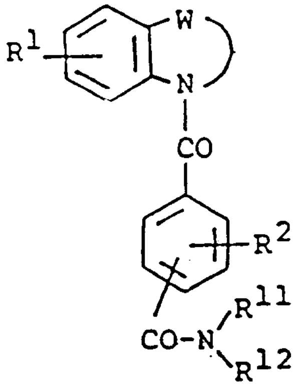

(1c)

dans laquelle

$R^1$, $R^2$, $R^{11}$, $R^{12}$ et W sont tels que définis dans la revendication 1, qui comprend la réaction d'un composé de formule (5) :

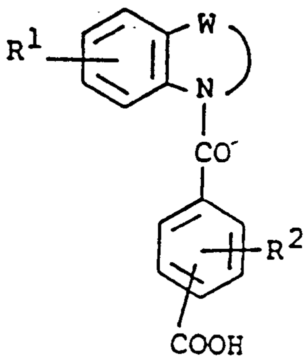

(5)

dans laquelle

$R^1$, $R^2$ et W sont tels que définis ci-dessus, avec un composé de formule (6) :

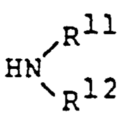

(6)

dans laquelle

$R^{11}$ et $R^{12}$ sont tels que définis ci-dessus.

**4.** Procédé pour préparer un composé de formule (1d) :

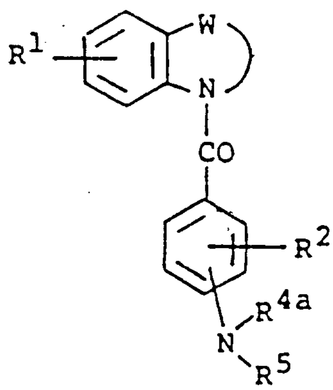

(1d)

dans laquelle

$R^1$, $R^2$, $R^5$ et W sont tels que définis dans la revendication 1, et $R^{4a}$ est un groupe alkyle en $C_1$-$C_6$, qui comprend la réaction d'un composé de formule (7) :

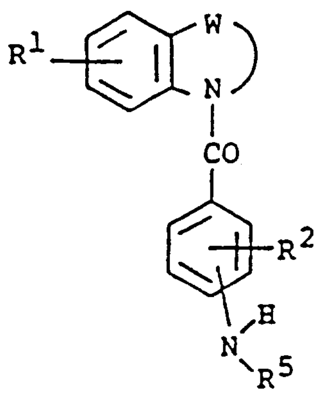

(7)

dans laquelle

$R^1$, $R^2$, $R^5$ et W sont tels que définis ci-dessus, avec un composé de formule (8) ou (9) :

$$R^{4a}X \quad (8)$$

ou

$$R^{17}COR^{18} \quad (9)$$

dans lesquelles

$R^{4a}$ est un groupe alkyle en $C_1$-$C_6$, X est un atome d'halogène et $R^{17}$ et $R^{18}$ sont chacun un atome d'hydrogène ou un groupe alkyle en $C_1$-$C_6$.

**5.** Procédé pour préparer un composé de formule (1e) :

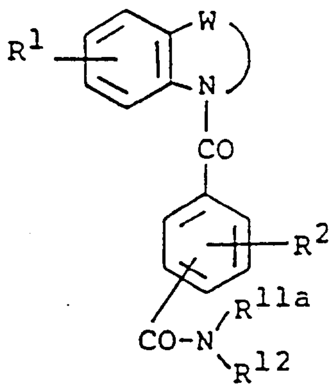

(1e)

dans laquelle

$R^1$, $R^2$, $R^{12}$ et W sont tels que définis dans la revendication 1, et $R^{11a}$ est un groupe alkyle en $C_1$-$C_6$, qui comprend la réaction d'un composé de formule (10) :

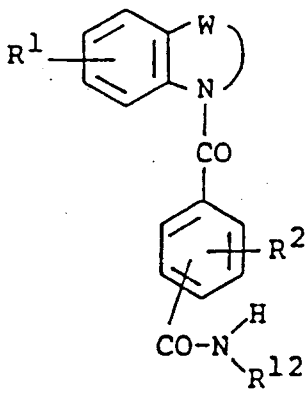

(10)

dans laquelle

$R^1$, $R^2$, $R^{12}$ et W sont tels que définis ci-dessus, avec un composé de formule (11) ou (9) :

$$R^{11a}X \qquad (11)$$

ou

$$R^{17}COR^{18} \qquad (9)$$

dans lesquelles

$R^{11a}$ est un groupe alkyle en $C_1$-$C_6$ et X, $R^{17}$ et $R^{18}$ sont tels que définis ci-dessus.

**6.** Procédé pour préparer un composé de formule (1f) :

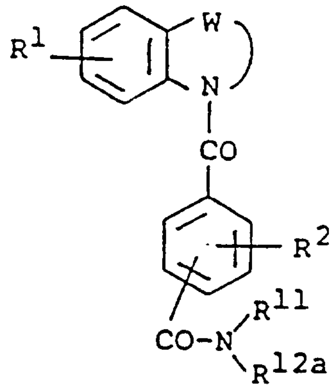

(1f)

dans laquelle

$R^1$, $R^2$, $R^{11}$ et W sont tels que définis dans la revendication 1, et $R^{12a}$ est un groupe cycloalkyle en $C_3$-$C_8$, qui comprend la réaction d'un composé de formule (12) :

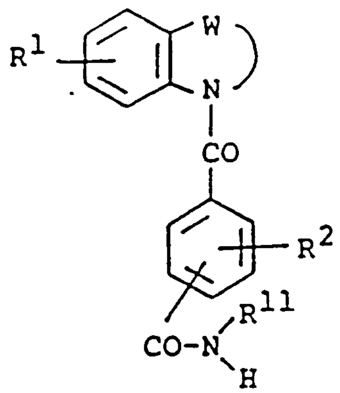

(12)

dans laquelle

$R^1$, $R^2$, $R^{11}$ et W sont tels que définis ci-dessus, avec un composé de formule (13) :

$$R^{12a}X \qquad (13)$$

dans laquelle

$R^{12a}$ est un groupe cycloalkyle en $C_3$-$C_8$ et X est un halogène.

7. Procédé pour préparer un composé de formule (1cc)

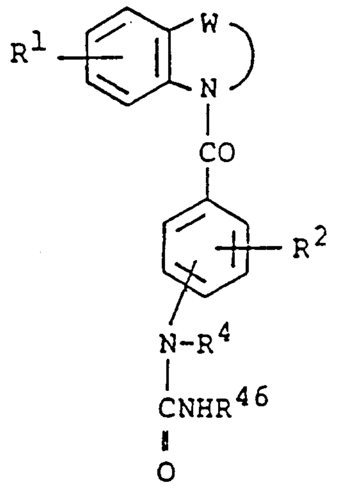

(1cc)

dans laquelle

$R^1$, $R^2$, $R^4$ et W sont tels que définis dans la revendication 1, et $R^{46}$ est un groupe phényle ayant facultativement un substituant alkyle en $C_1$-$C_6$, qui comprend la réaction d'un composé de formule (2b) :

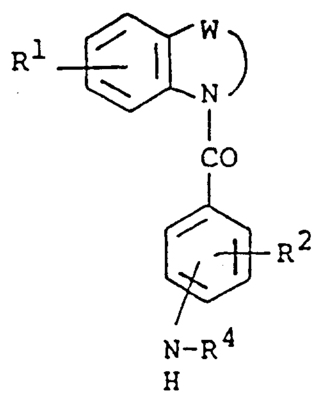

(2b)

dans laquelle

$R^1$, $R^2$, $R^4$ et W sont tels que définis ci-dessus, avec un composé de formule (38) :

$$R^{46}N=C=O \quad (38)$$

dans laquelle

$R^{46}$ est tel que défini ci-dessus.

**8.** Procédé pour préparer un composé de formule (1dd) :

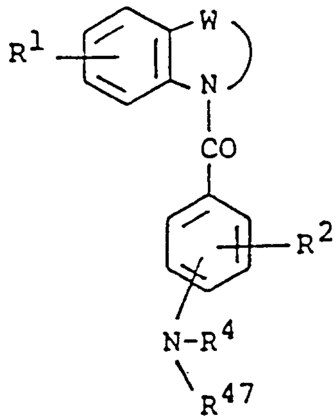

(1dd)

dans laquelle

$R^1$, $R^2$, $R^4$ et W sont tels que définis dans la revendication 1, et $R^{47}$ est un groupe phénylsulfonyle ayant facultativement sur le noyau phényle un substituant choisi parmi un atome d'halogène et un groupe alkyle en $C_1$-$C_6$, ou un groupe quinolylsulfonyle et X est un halogène, qui comprend la réaction d'un composé de formule (2b) :

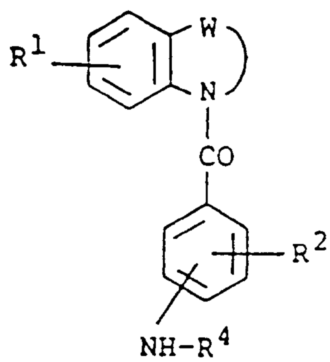

(2b)

dans laquelle

$R^1$, $R^2$, $R^4$ et W sont tels que définis ci-dessus, avec un composé de formule (39) :

$$R^{47}X \qquad (39)$$

dans laquelle

$R^{47}$ est tel que défini ci-dessus.

**9.** Procédé pour préparer un composé de formule (1hh)

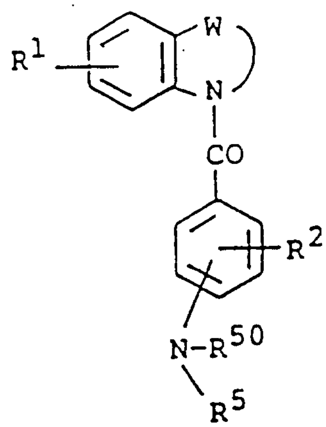

(1hh)

dans laquelle

$R^1$, $R^2$, $R^5$ et W sont tels que définis dans la revendication 1, et $R^{50}$ est un groupe benzoyle ayant facultativement sur le noyau phényle un substituant halogène, qui comprend la réaction d'un composé de formule (7) :

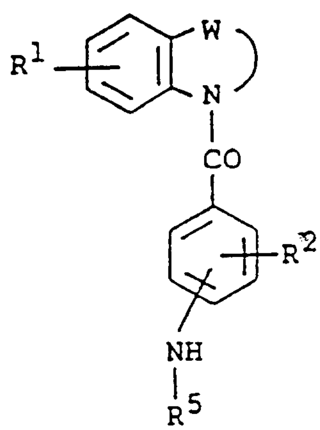

(7)

dans laquelle

$R^1$, $R^2$, $R^5$ et W sont tels que définis ci-dessus, avec un composé de formule (42) :

$R^{50}OH$ (42)

dans laquelle

$R^{50}$ est tel que défini ci-dessus.

**10.** Procédé selon les revendications 1 à 9, dans lequel $R^1$ dans la formule (1) est un atome d'hydrogène, et un de ses sels.

**11.** Procédé selon les revendications 1 à 9, dans lequel $R^1$ dans la formule (1) est un atome d'halogène, et un de ses sels.

**12.** Procédé selon les revendications 1 à 9, dans lequel $R^1$ dans la formule (1) est un groupe alkyle en $C_1$-$C_6$, un groupe amino ayant facultativement un substituant alkyle en $C_1$-$C_6$ ou un groupe alcoxy en $C_1$-$C_6$, et un de ses sels.

**13.** Procédé selon la revendication 10, dans lequel $R^2$ est un atome d'hydrogène, et un de ses sels.

**14.** Composé selon la revendication 10, dans lequel $R^2$ est un atome d'halogène, un groupe alcoxy en $C_1$-$C_6$, ou un groupe alkyle en $C_1$-$C_6$ et un de ses sels.

15. Composé selon la revendication 10, dans lequel $R^2$ est un groupe phényl-alcoxy en $C_1$-$C_6$ ; un groupe hydroxy ; un groupe amino ayant facultativement un substituant alkyle en $C_1$-$C_6$ ; un groupe carbamoyl-alcoxy en $C_1$-$C_6$ ; un groupe amino-alcoxy en $C_1$-$C_6$ ayant facultativement un substituant alkyle en $C_1$-$C_6$ ; ou un groupe benzoyloxy ayant facultativement sur son noyau phényle un substituant halogène, et un de ses sels.

16. Procédé selon la revendication 11, dans lequel $R^2$ est un atome d'hydrogène, et un de ses sels.

17. Procédé selon la revendication 11, dans lequel $R^2$ est un atome d'halogène, un groupe alcoxy en $C_1$-$C_6$, ou un groupe alkyle en $C_1$-$C_6$ et un de ses sels.

18. Procédé selon la revendication 11, dans lequel $R^2$ est un groupe phényl-alcoxy en $C_1$-$C_6$ ; un groupe hydroxy ; un groupe amino ayant facultativement un substituant alkyle en $C_1$-$C_6$ ; un groupe carbamoyl-alcoxy en $C_1$-$C_6$ ; un groupe amino-alcoxy en $C_1$-$C_6$ ayant facultativement un substituant alkyle en $C_1$-$C_6$ ; ou un groupe benzoyloxy ayant facultativement sur son noyau phényle un substituant halogène, et un de ses sels.

19. Procédé selon la revendication 12, dans lequel $R^2$ est un atome d'hydrogène et un de ses sels.

20. Procédé selon la revendication 12, dans lequel $R^2$ est un atome d'halogène, un groupe alcoxy en $C_1$-$C_6$ ou un groupe alkyle en $C_1$-$C_6$ et un de ses sels.

21. Procédé selon la revendication 12, dans lequel $R^2$ est un groupe phényl-alcoxy en $C_1$-$C_6$ ; un groupe hydroxy ; un groupe amino ayant facultativement un substituant alkyle en $C_1$-$C_6$ ; un groupe carbamoyl-alcoxy en $C_1$-$C_6$ ; un groupe amino-alcoxy en $C_1$-$C_6$ ayant facultativement un substituant alkyle en $C_1$-$C_6$ ; ou un groupe benzoyloxy ayant facultativement sur son noyau phényle un substituant halogène, et un de ses sels.

22. Procédé selon la revendication 13, dans lequel $R^3$ est un groupe de formule $-NR^4R^5$ ($R^4$ et $R^5$ sont tels que définis dans la revendication 1) et un de ses sels.

23. Procédé selon la revendication 13, dans lequel $R^3$ est un groupe de formule $-CO-NR^{11}R^{12}$ ($R^{11}$ et $R^{12}$ sont tels que définis dans la revendication 1) et un de ses sels.

24. Procédé selon la revendication 14, dans lequel $R^3$ est un groupe de formule $-NR^4R^5$ ($R^4$ et $R^5$ sont tels que définis dans la revendication 1) et un de ses sels.

25. Procédé selon la revendication 14, dans lequel $R^3$ est un groupe de formule $-CO-NR^{11}R^{12}$ ($R^{11}$ et $R^{12}$ sont tels que définis dans la revendication 1) et un de ses sels.

26. Procédé selon la revendication 16, dans lequel $R^3$ est un groupe de formule $-NR^4R^5$ ($R^4$ et $R^5$ sont tels que définis dans la revendication 1), et un de ses sels.

27. Procédé selon la revendication 16, dans lequel $R^3$ est un groupe de formule $-CO-NR^{11}R^{12}$ ($R^{11}$ et $R^{12}$ sont tels que définis dans la revendication 1) et un de ses sels.

28. Procédé selon la revendication 17, dans lequel $R^3$ est un groupe de formule $-NR^4R^5$ ($R^4$ et $R^5$ sont tels que définis dans la revendication 1) et un de ses sels.

29. Procédé selon la revendication 17, dans lequel $R^3$ est un groupe de formule $-CO-NR^{11}R^{12}$ ($R^{11}$ et $R^{12}$ sont tels que définis dans la revendication 1) et un de ses sels.

30. Procédé selon la revendication 22, dans lequel $R^4$ est un atome d'hydrogène et $R^5$ est un groupe de formule :

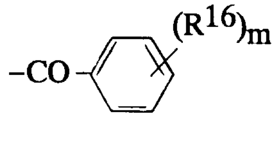

(dans laquelle $R^{16}$ et m sont tels que définis dans la revendication 1) et un de ses sels.

**31.** Procédé selon la revendication 22, dans lequel $R^4$ est un atome d'hydrogène et $R^5$ est un groupe phényl-(alcoxy en $C_1$-$C_6$)carbonyle, un groupe aleanoyle en $C_1$-$C_6$, un groupe phényl-alcanoyle en $C_2$-$C_6$, un groupe (cycloalkyl en $C_3$-$C_8$)-alcanoyle en $C_2$-$C_6$, un groupe (cycloalkyl en $C_3$-$C_8$)carbonyle, un groupe tricyclo[3.3.1.1]décanylcarbonyle, un groupe naphtylcarbonyle, un groupe pyridylcarbonyle, un groupe furoyle, un groupe thénoyle, un groupe phénoxy-alcanoyle en $C_2$-$C_6$ dont le noyau phényle a facultativement 1 à 3 substituants choisis parmi un groupe alkyle en $C_1$-$C_6$, un groupe alcoxy en $C_1$-$C_6$ et un groupe amino ayant facultativement un substituant alcanoyle en $C_1$-$C_6$, un groupe phtalimido-alcanoyle en $C_2$-$C_6$, un groupe (alcoxy en $C_1$-$C_6$)carbonyl-alcanoyle en $C_2$-$C_6$, un groupe carboxy-alcanoyle en $C_2$-$C_6$, un groupe naphtyloxy-alcanoyle en $C_2$-$C_6$, un groupe halogéno-alcanoyle en $C_1$-$C_6$, un groupe de formule

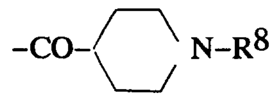

(dans laquelle $R^8$ est un atome d'hydrogène, un groupe alkyle en $C_1$-$C_6$, un groupe phényl-(alcoxy en $C_1$-$C_6$)carbonyle, un groupe carbamoyl-alkyle en $C_1$-$C_6$, un groupe amino-alcanoyle en $C_1$-$C_6$ ayant facultativement un substituant alkyle en $C_1$-$C_6$, ou un groupe alcanoyle en $C_1$-$C_6$), un groupe anilinocarbonyle qui a facultativement sur le noyau phényle un substituant alkyle en $C_1$-$C_6$, un groupe phénoxycarbonyle, un groupe phénylsulfonyle qui a facultativement sur le noyau phényle un substituant choisi parmi un atome d'halogène et un groupe alkyle en $C_1$-$C_6$, un groupe quinolylsulfonyle ou un groupe de formule

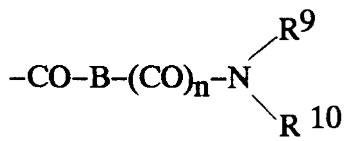

(dans laquelle B est un groupe alkylène en $C_1$-$C_6$, n est un entier égal à 0 ou 1 et $R^9$ et $R^{10}$ sont les mêmes ou différents et sont chacun un atome d'hydrogène, un groupe alkyle en $C_1$-$C_6$ ayant facultativement un substituant hydroxy, un groupe cycloalkyle en $C_3$-$C_8$, un groupe phényl-alkyle en $C_1$-$C_6$, un groupe alcanoyle en $C_1$-$C_6$, un groupe alcényle en $C_2$-$C_6$, un groupe phénoxy-alkyle en $C_1$-$C_6$, un groupe phényle qui a facultativement 1 à 3 substituants choisis parmi un groupe amino-alkyle en $C_1$-$C_6$ ayant facultativement un substituant alcanoyle en $C_1$-$C_6$, un groupe alkyle en $C_1$-$C_6$, un groupe alcoxy en $C_1$-$C_6$ et un atome d'halogène, un groupe phtalimido-alkyle en $C_1$-$C_6$, un groupe amino-alkyle en $C_1$-$C_6$ ayant facultativement un substituant alcanoyle en $C_1$-$C_6$, un groupe alcynyle en $C_2$-$C_6$ ou un groupe amino-alkyle en $C_1$-$C_6$ ayant facultativement un substituant alkyle en $C_1$-$C_6$, ou bien $R^9$ et $R^{10}$ peuvent être reliés ensemble pour former avec l'atome d'azote auquel ils sont liés un groupe hétérocyclique saturé à 5 ou 6 chaînons avec ou sans atome d'azote ou d'oxygène intercalé, dans lequel le groupe hétérocyclique a facultativement un substituant choisi parmi un groupe alkyle en $C_1$-$C_6$, un groupe (alcoxy en $C_1$-$C_6$)carbonyl et un groupe pipéridinyle), et un de ses sels.

**32.** Procédé selon la revendication 22, dans lequel $R^4$ est un groupe alkyle en $C_1$-$C_6$, et un de ses sels.

**33.** Procédé selon la revendication 24, dans lequel $R^4$ est un atome d'hydrogène et $R^5$ est un groupe de formule

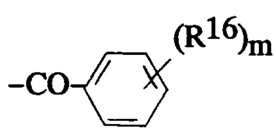

(dans laquelle $R^{16}$ et m sont tels que définis dans la revendication 1, et un de ses sels.

**34.** Procédé selon la revendication é', dans lequel $R^4$ est un atome d'hydrogène et $R^5$ est un groupe phényl-(alcoxy en $C_1$-$C_6$)carbonyle, un groupe alcanoyle en $C_1$-$C_6$, un groupe phényl-alcanoyle en $C_1$-$C_6$, un groupe (cycloalkyl en $C_3$-$C_8$)-alcanoyle en $C_2$-$C_6$, un groupe (cycloalkyl en $C_3$-$C_8$)carbonyle, un groupe tricyclo[3.3.1.1]décanylcarbonyle, un groupe naphtylcarbonyle, un groupe pyridylcarbonyle, un groupe furoyle, un groupe thénoyle, un groupe phénoxy-alcanoyle en $C_2$-$C_6$ dont le noyau phényle a facultativement 1 à 3 substituants choisis parmi un groupe alkyle en

$C_1$-$C_6$, un groupe alcoxy en $C_1$-$C_6$ et un groupe amino ayant facultativement un substituant alcanoyle en $C_1$-$C_6$, un groupe phtalimido-alcanoyle en $C_2$-$C_6$, un groupe (alcoxy en $C_1$-$C_6$)carbonyl-alcanoyle en $C_2$-$C_6$, un groupe carboxy-alcanoyle en $C_2$-$C_6$, un groupe naphtyloxy-alcanoyle en $C_2$-$C_6$, un groupe halogéno-alcanoyle en $C_1$-$C_6$, un groupe de formule

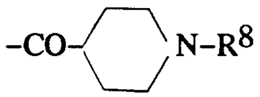

(dans laquelle $R^8$ est un atome d'hydrogène, un groupe alkyle en $C_1$-$C_6$, un groupe phényl-(alcoxy en $C_1$-$C_6$)carbonyle, un groupe carbamoyl-alkyle en $C_1$-$C_6$, un groupe amino-alcanoyle en $C_1$-$C_6$ ayant facultativement un substituant alkyle en $C_1$-$C_6$ ou un groupe alcanoyle en $C_1$-$C_6$), un groupe anilinocarbonyle qui a facultativement sur le noyau phényle un substituant alkyle en $C_1$-$C_6$, un groupe phénoxycarbonyle, un groupe phénylsulfonyle qui a facultativement sur le noyau phényle un substituant choisi parmi un atome d'halogène et un groupe alkyle en $C_1$-$C_6$, un groupe quinolysulfonyle ou un groupe de formule

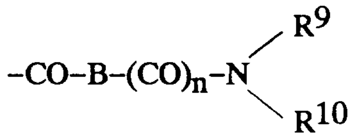

(dans laquelle B est un groupe alkylène en $C_2$-$C_6$, n est un entier de 0 ou 1 et $R^9$ et $R^{10}$ sont les mêmes ou différents et sont chacun un atome d'hydrogène, un groupe alkyle en $C_1$-$C_6$ ayant facultativement un substituant hydroxy, un groupe cycloalkyle en $C_3$-$C_8$, un groupe phényl-alkyle en $C_1$-$C_6$, un groupe alcanoyle en $C_1$-$C_6$, un groupe alcényle en $C_2$-$C_6$, un groupe phénoxy-alkyle en $C_1$-$C_6$, un groupe phényle qui a facultativement 1 à 3 substituants choisis parmi un groupe amino-alkyle en $C_1$-$C_6$ ayant facultativement un substituant alcanoyle en $C_1$-$C_6$, un groupe alkyle en $C_1$-$C_6$, un groupe alcoxy en $C_1$-$C_6$ et un atome d'halogène, un groupe phtalimido-alkyle en $C_1$-$C_6$, un groupe amino-alkyle en $C_1$-$C_6$ ayant facultativement un substituant alcanoyle en $C_1$-$C_6$, un groupe alcynyle en $C_2$-$C_6$ ou un groupe amino-alkyle en $C_1$-$C_6$ ayant facultativement un substituant alkyle en $C_1$-$C_6$, ou bien $R^9$ et $R^{10}$ peuvent être reliés ensemble dur former avec l'atome d'azote auquel ils sont liés un groupe hétérocyclique saturé à 5 ou 6 chaînons avec ou sans atome d'azote ou d'oxygène intercalé, dans lequel le groupe hétérocyclique a facultativement un substituant choisi parmi un groupe alkyle en $C_1$-$C_6$, un groupe (alcoxy en $C_1$-$C_6$)carbonyle et un groupe pipéridinyle), et un de ses sels.

**35.** Procédé selon la revendication 24, dans lequel $R^4$ est un groupe alkyle en $C_1$-$C_6$ et un de ses sels.

**36.** Procédé selon la revendication 15, dans lequel $R^4$ est un atome d'hydrogène et $R^5$ est un groupe de formule

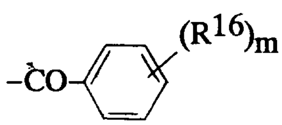

(dans laquelle $R^{16}$ et m sont tels que définis dans la revendication 1, et un de ses sels.

**37.** Procédé selon les revendications 15, 18 à 21, 25 à 27, dans lequel $R^4$ est un atome d'hydrogène et $R^5$ est tel que défini à la revendication 31.

**38.** Procédé selon la revendication 15, dans lequel $R^4$ est un groupe alkyle en $C_1$-$C_6$, et un de ses sels.

39. Procédé selon la revendication 26, dans lequel $R^4$ est un atome d'hydrogène et $R^5$ est un groupe de formule

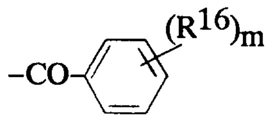

(dans laquelle $R^{16}$ et m sont tels que définis à la revendication 1), et un de ses sels.

40. Procédé selon la revendication 26, dans lequel $R^4$ est un groupe alkyle en $C_1$-$C_6$, et un de ses sels.

41. Procédé selon la revendication 28, dans lequel $R^4$ est un atome d'hydrogène et $R^5$ est un groupe de formule

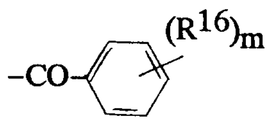

(dans laquelle $R^{16}$ et m sont tels que définis dans la revendication 1), et un de ses sels.

42. Procédé selon la revendication 28, dans lequel $R^4$ est un groupe alkyle en $C_1$-$C_6$, et un de ses sels.

43. Procédé selon la revendication 18, dans lequel $R^4$ est un atome d'hydrogène et $R^5$ est un groupe de formule

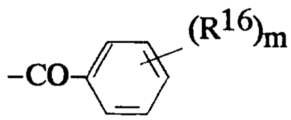

(dans laquelle $R^{16}$ et m sont tels que définis dans la revendication 1), et un de ses sels.

44. Procédé selon la revendication 18, dans lequel $R^4$ est un groupe alkyle en $C_1$-$C_6$, et un de ses sels.

45. Procédé selon la revendication 17, dans lequel $R^4$ est un atome d'hydrogène et $R^5$ est un groupe de formule

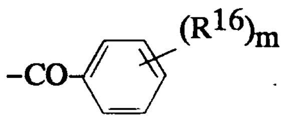

(dans laquelle $R^{16}$ et m sont tels que définis dans la revendication 1), et un de ses sels.

46. Procédé selon la revendication 19, dans lequel $R^4$ est un groupe alkyle en $C_1$-$C_6$, et un de ses sels.

47. Procédé selon la revendication 20, dans lequel $R^4$ est un atome d'hydrogène et $R^5$ est un groupe de formule

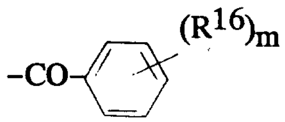

(dans laquelle $R^{16}$ et m sont tels que définis dans la revendication 1), et un de ses sels.

48. Procédé selon la revendication 20, dans lequel $R^4$ est un groupe alkyle en $C_1$-$C_6$, et un de ses sels.

**49.** Procédé selon la revendication 21, dans lequel $R^4$ est un atome d'hydrogène et $R^5$ est un groupe de formule

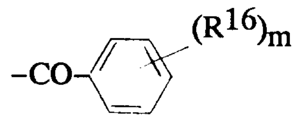

(dans laquelle $R^{16}$ et m sont tels que définis dans la revendication 1), et un de ses sels.

**50.** Procédé selon la revendication 21, dans lequel $R^4$ est un groupe alkyle en $C_1$-$C_6$ et un de ses sels.

**51.** Procédé selon la revendication 30, dans lequel $R^{16}$ est un atome d'halogène ou un groupe alkyle en $C_1$-$C_6$ ayant facultativement un substituant choisi parmi un atome d'halogène et un groupe hydroxy, et un de ses sels.

**52.** Procédé selon la revendication 33, dans lequel $R^{16}$ est un atome d'halogène ou un groupe alkyle en $C_1$-$C_6$ ayant facultativement un substituant choisi parmi un atome d'halogène et un groupe hydroxy, et un de ses sels.

**53.** Procédé selon la revendication 39, dans lequel $R^{16}$ est un atome d'halogène ou un groupe alkyle en $C_1$-$C_6$ ayant facultativement un substituant choisi parmi un atome d'halogène et un groupe hydroxy, et un de ses sels.

**54.** Procédé selon la revendication 42, dans lequel $R^{16}$ est un atome d'halogène ou un groupe alkyle en $C_1$-$C_6$ ayant facultativement un substituant choisi parmi un atome d'halogène et un groupe hydroxy, et un de ses sels.

**55.** Procédé selon les revendications 1 à 9, dans lequel W est un groupe de formule $-(CH_2)_p-$ dans laquelle p est un entier de 3 à 5 et en outre ledit groupe $-(CH_2)_p-$ a facultativement 1 à 3 substituants tels que définis dans la revendication 1.

**56.** Procédé selon les revendications 1 à 9, dans lequel W est un groupe de formule $-CH=CH-(CH_2)_q-$ dans laquelle q est un entier de 1 à 3, et en outre ledit groupe $-CH=CH-(CH_2)_q-$ a facultativement 1 à 3 substituants choisis parmi un groupe alkyle en $C_1$-$C_6$ ayant facultativement un substituant hydroxy, et un groupe amino-alkyle en $C_1$-$C_6$ ayant facultativement un substituant choisi parmi un groupe alkyle en $C_1$-$C_6$ et un groupe alcanoyle en $C_1$-$C_6$, et un de ses sels.

**57.** Procédé selon la revendication 55, dans lequel W est un groupe de formule $-(CH_2)_p-$ (p est un entier de 3 à 5) et ledit groupe $-(CH_2)_p-$ a facultativement 1 à 3 substituants tels que définis dans la revendication 1.

**58.** Procédé selon la revendication 57, dans lequel p dans le groupe $-(CH_2)_p-$ est égal à 3 et le groupe n'a pas de substituant, et un de ses sels.

**59.** Procédé selon la revendication 57, dans lequel p dans le groupe $-(CH_2)_p-$ est égal à 3 et le groupe est substitué par un groupe de formule

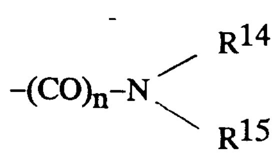

(dans laquelle $R^{14}$, $R^{15}$ et n sont tels que définis ci-dessus), et un de ses sels.

**60.** Procédé selon la revendication 57, dans lequel p dans le groupe $-(CH_2)_p$ est égal à 4 et le groupe n'a pas de substituant, et un de ses sels.

**61.** Procédé selon la revendication 57, dans lequel p dans le groupe $-(CH_2)_p-$ est égal à 4 et le groupe est substitué par un groupe de formule

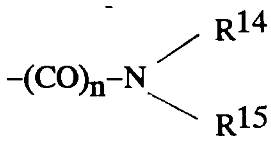

(dans laquelle $R^{14}$, $R^{15}$ et n sont tels que définis ci-dessus), et un de ses sels.

**62.** Procédé selon la revendication 57, dans lequel p dans le groupe $-(CH_2)_p-$ est égal à 5, et un de ses sels.

**63.** Procédé selon la revendication 56, dans lequel q dans le groupe $-CH=CH-(CH_2)_q-$ est égal à 1, et un de ses sels.

**64.** Procédé selon la revendication 56, dans lequel q dans le groupe $-CH=CH-(CH_2)_q-$ est égal à 2, et un de ses sels.

**65.** Procédé selon la revendication 56, dans lequel q dans le groupe $-CH=CH(CH_2)_q-$ est égal à 3, et un de ses sels.

**66.** Procédé selon la revendication 59 ou 61, dans lequel n dans le substituant

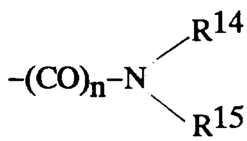

est égal à 0 et $R^{14}$ et $R^{15}$ sont les mêmes ou différents et sont chacun un atome d'hydrogène, un groupe alkyle en $C_1$-$C_6$ ou un groupe cycloalkyle en $C_3$-$C_8$, et un de ses sels.

**67.** Procédé selon la revendication 64, dans lequel le groupe hétéro cyclique de formule

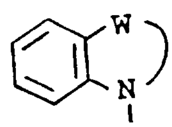

est un groupe 2,3-dihydro-1H-benzazépine, et un de ses sels.

**68.** Procédé selon les revendications 1 à 9, pour fabriquer
la 1-[4-(2-méthylbenzoylamino)benzoyl]-4-méthyl-2,3,4,5-tétrahydro-1H-1,4-benzodiazépine,
la 5-diméthylamino-1-[4-(2-méthylbenzoylamino)benzoyl]-2,3,4,5-tétrahydro-1H-benzazépine,
la 5-diméthylamino-1-[2-chloro-4-(2-méthylbenzoylamino)benzoyl]-2,3,4,5-tétrahydro-1H-benzazépine,
la 5-méthylamino-1-[2-chloro-4-(2-méthylbenzoylamino)benzoyl]-2,3,4,5-tétrahydro-1H-benzazépine,
la 5-cyclopropylamino-1-[2-chloro-4-(2-méthyl-benzoylamino)benzoyl]-2,3,4,5-tétrahydro-1H-benzazépine,
la 5-cyclopropylamino-1-[2-chloro-4-(2-chlorobenzoylamino)benzoyl]-2,3,4,5-tétrahydro-1H-benzazépine,
la 5-diméthylamino-1-[2-méthyl-4-(2-méthylbenzoylamino)benzoyl]-2,3,4,5-tétrahydro-1H-benzazépine,
la 4-diméthylamino-1-[3-méthoxy-4-(2-méthylbenzoylamino)benzoyl]-1,2,3,4-tétrahydroquinoléine,
la 7-chloro-5-méthylamino-1-[4-(2-méthylbenzoylamino)benzoyl]-2,3,4,5-tétrahydro-1H-benzazépine,
la 7-chloro-5-méthylamino-1-[4-(2-chlorobenzoylamino)benzoyl]-2,3,4,5-tétrahydro-1H-benzazépine et
la 5-hydroxysulfonyloxyimino-1-[2-chloro-4-(2-méthylbenzoylamino)benzoyl-2,3,4,5-tétrahydro-1H-benzazépine.

**69.** Procédé selon les revendications 1 à 9, pour la fabrication de composés de formule :

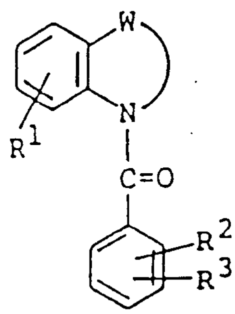

dans laquelle

a)

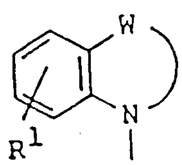

représente

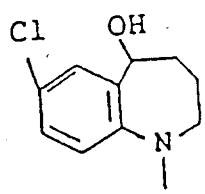

$R^2$ représente 2-$OCH_3$ et
$R^3$ représente

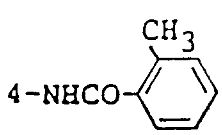

et

b)

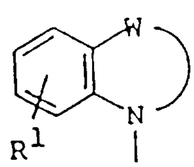

représente

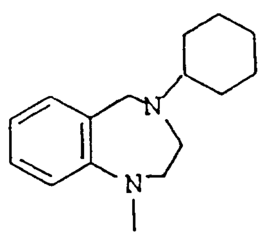

R² représente H et
R³ représente

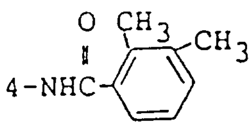

**70.** Procédé selon les revendications 1 à 9, pour la fabrication d'un composé benzohétérocyclique de formule :

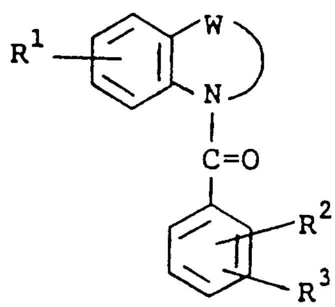

dans laquelle

$R^1$, $R^2$ et $R^3$ sont tels que définis dans la revendication 1 et le groupe hétérocyclique de formule :

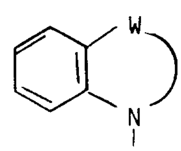

est choisi parmi les groupes 3,4-dihydro-2H-1,4-benzoxazinyle, 1,2,3,5-tétrahydro-4,1-benzoxazépinyle, 1,2,3,4-tétrahydroquinoxalinyle, 1,2,3,4,5,6-hexahydro-1,5-benzodiazocinyle, 5-méthyl-1,2,3,4,5,6-hexahydro-1,5-benzodiazocinyle, 4-méthyl-1,2,3,4-tétrahydroquinoxalinyle, 1,2,3,4-tétrahydro-5,1-benzoxazépinyle, 3,4-dihydro-2H-1,4-benzothiazinyle, 2,3,4,5-tétrahydro-1,5-benzothiazépinyle, 1,2,3,5-tétrahydro-4,1-benzothiazépinyle, 4-éthyl-1,2,3,4-tétrahydroquinoxalinyle, 4-propyl-1,2,3,4-tétrahydroquinoxalinyle, 4-butyl-1,2,3,4-tétrahydroquinoxalinyle, 4-pentyl-1,2,3,4-tétrahydroquinoxalinyle, 4-hexyl-1,2,3,4-tétrahydroquinoxalinyle, 2,3,4,5-tétrahydro-1H-1,4-benzodiazépinyle, 4-méthyl-2,3,4,5-tétrahydro-1H-1,4-benzodiazépinyle, 4-éthyl-2,3,4,5-tétrahydro-1H-1,4-benzodiazépinyle, 4-propyl-2,3,4,5-tétrahydro-1H-1,4-benzodiazépinyle, 4-butyl-2,3,4,5-tétrahydro-1H-1,4-benzodiazépinyle, 4-pentyl-2,3,4,5-tétrahydro-1H-1,4-benzodiazépinyle, 4-hexyl-2,3,4,5-tétrahydro-1H-1,4-benzodiazépinyle, 2,3,4,5-tétrahydro-1H-1,5-benzodiazépinyle, 5-méthyl-2,3,4,5-tétrahydro-1H-1,5-benzodiazépinyle, 5-éthyl-2,3,4,5-tétrahydro-1H-1,5-benzodiazépinyle, 5-propyl-2,3,4,5-tétrahydro-1H-1,5-benzodiazépinyle, 5-butyl-2,3,4,5-tétrahydro-1H-1,5-benzodiazépinyle, 5-pentyl-2,3,4,5-tétrahydro-1H-1,5-benzodiazépinyle, 5-hexyl-2,3,4,5-tétrahydro-1H-1,5-benzodiazépinyle, 3,4-dihydro-1-oxo-2H-1,4-benzothiazépinyle, 3,4-dihydro-1,1-dioxo-2H-1,4-benzothiazépinyle, 1-oxo-2,3,4,5-tétrahydro-1,5-benzothiazépinyle, 1,1-dioxo-2,3,4,5-tétrahydro-1,5-benzothiazépinyle, 4-oxo-1,2,3,4,5-tétrahydro-4,1-benzothiazépinyle, et 4,4-dioxo-1,2,3,5-tétrahydro-4,1-benzothiazépinyle, et d'un de ses sels.

**71.** Procédé pour la production d'une composition antagoniste de la vasopressine, qui comprend comme ingrédient actif un composé de formule (1) préparé par le procédé selon les revendications 1 à 9, ou un de ses sels acceptables en pharmacie, par mélange avec un support ou diluant acceptable en pharmacie.

Fig. 1

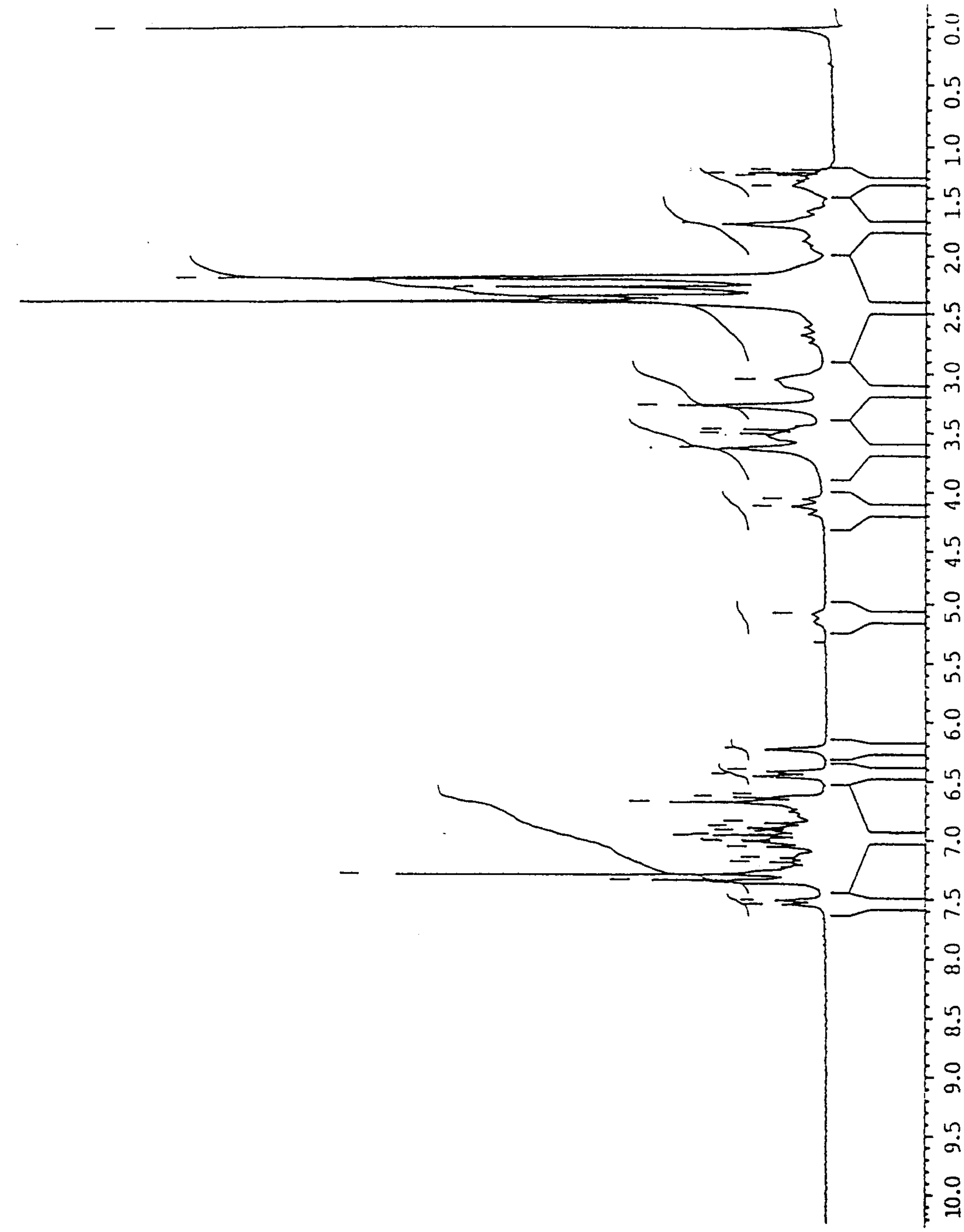

Fig. 2

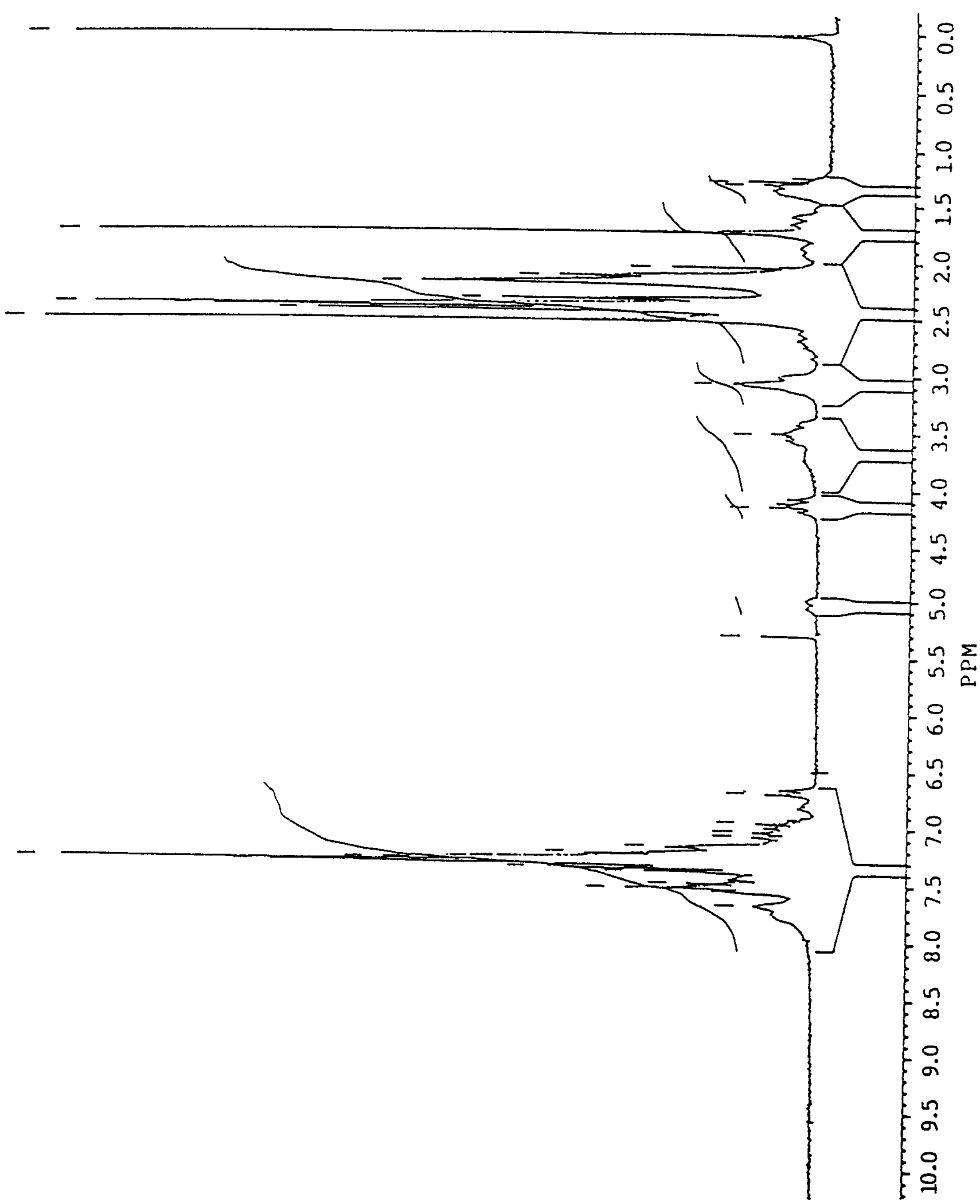

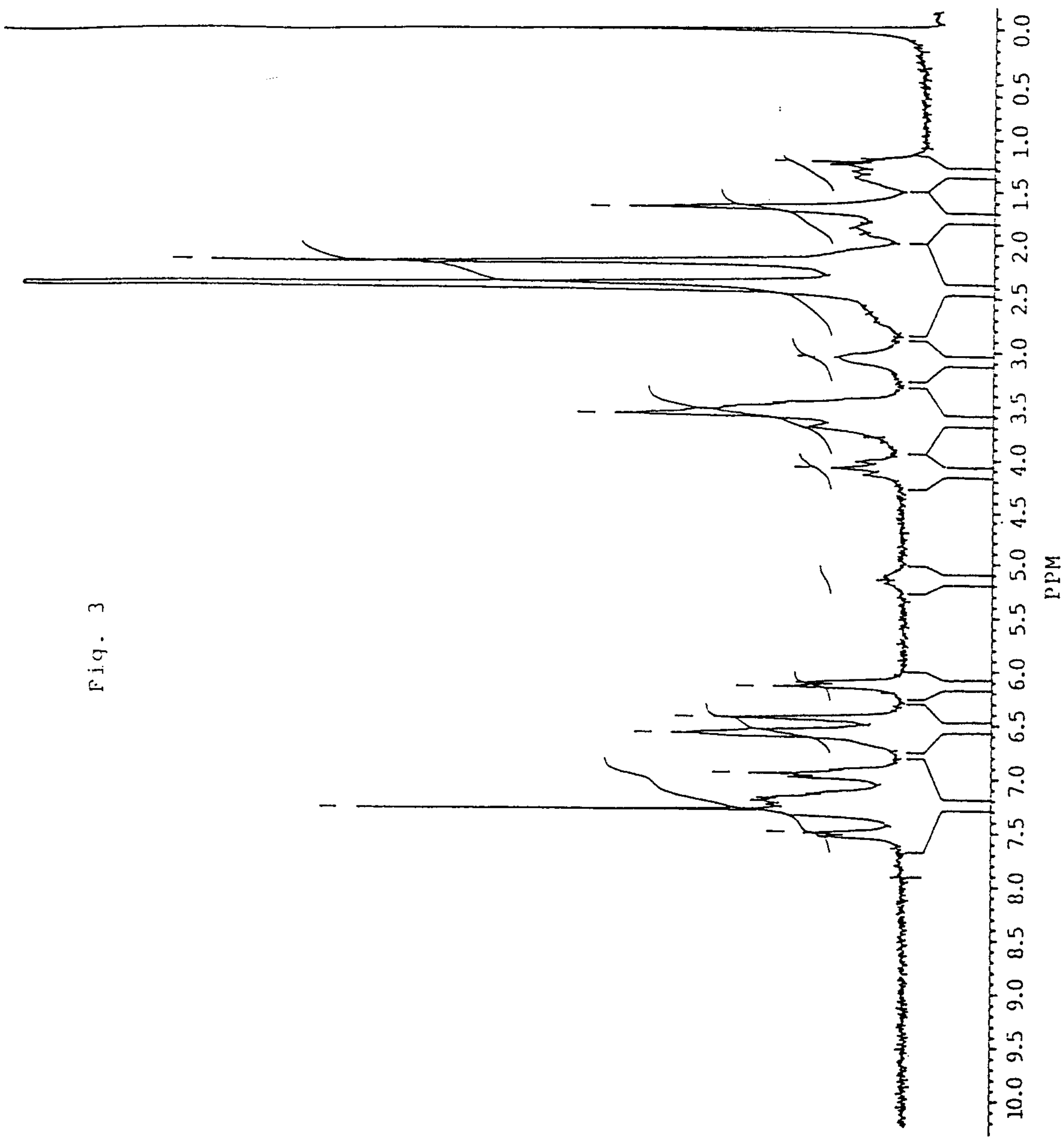


Fig. 3

Fig. 4